(19) Europäisches Patentamt / European Patent Office / Office européen des brevets

(11) **EP 4 024 495 A2**

(12) **EUROPEAN PATENT APPLICATION**

(43) Date of publication:
**06.07.2022 Bulletin 2022/27**

(21) Application number: **22150222.2**

(22) Date of filing: **04.01.2022**

(51) International Patent Classification (IPC):
***H01L 51/54*** *(2006.01)* ***C07C 13/00*** *(2006.01)*
***C07C 15/00*** *(2006.01)* ***C07C 211/54*** *(2006.01)*
***C07F 5/02*** *(2006.01)* ***C07F 15/00*** *(2006.01)*
***H01L 51/00*** *(2006.01)* ***H01L 51/50*** *(2006.01)*

(52) Cooperative Patent Classification (CPC):
**H01L 51/006; C07C 13/00; C07C 15/00; C07F 5/02; C07F 15/0086; H01L 51/0052; H01L 51/0054; H01L 51/0055; H01L 51/0067; H01L 51/0071; H01L 51/0072; H01L 51/008; H01L 51/0087; H01L 51/5004; H01L 51/5028;**

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.01.2021 KR 20210001063**
**03.01.2022 KR 20220000511**

(71) Applicant: **Samsung Electronics Co., Ltd.**
**Gyeonggi-do 16677 (KR)**

(72) Inventors:
- **IHN, Sooghang**
  **16678 Suwon-si (KR)**
- **KWON, Eunsuk**
  **16678 Suwon-si (KR)**
- **MARUYAMA, Yusuke**
  **16678 Suwon-si (KR)**
- **KIM, Sangmo**
  **16678 Suwon-si (KR)**
- **KIM, Jong Soo**
  **16678 Suwon-si (KR)**
- **NAM, Sungho**
  **16678 Suwon-si (KR)**
- **LEE, Hasup**
  **16678 Suwon-si (KR)**
- **CHWAE, Jun**
  **16678 Suwon-si (KR)**

(74) Representative: **Elkington and Fife LLP**
**Prospect House**
**8 Pembroke Road**
**Sevenoaks, Kent TN13 1XR (GB)**

(54) **COMPOSITION AND ORGANIC LIGHT-EMITTING DEVICE INCLUDING THE SAME**

(57) A composition comprising a first compound, a second compound, and a third compound, wherein the first compound, the second compound, and the third compound are different from each other, the first compound satisfies one of Condition 1 and Condition 2 as described herein, and the second compound includes a compound represented by Formula 1:

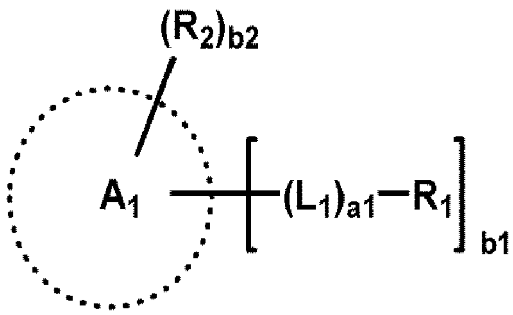

wherein ring $A_1$ is a condensed cyclic group in which 3 or more cyclic groups are condensed with each other, and the 3 or more cyclic groups are each a $C_5$-$C_{30}$ carbocyclic group or a $C_1$-$C_{30}$ heterocyclic group; a1 is an integer from 1 to 5; b1 is an integer from 3 to 10; and $L_1$, $R_1$, and $R_2$ are as described herein.

EP 4 024 495 A2

FIG. 1

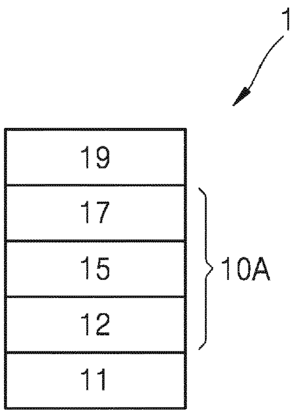


(52) Cooperative Patent Classification (CPC): (Cont.)
H01L 2251/5384; H01L 2251/552

**Description**

FIELD OF THE INVENTION

One or more embodiments relate to compositions and organic light-emitting devices including the same.

BACKGROUND OF THE INVENTION

Organic light-emitting devices (OLEDs) are self-emission devices that produce full-color images. Relative to other electronic devices, OLEDs can provide full color images that have wide viewing angles, high contrast ratios, short response times, and excellent characteristics in terms of luminance, driving voltage, and response speed.

For example, an organic light-emitting device includes an anode, a cathode, and an organic layer located between the anode and the cathode, wherein the organic layer includes an emission layer. A hole transport region may be located between the anode and the emission layer, and an electron transport region may be located between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transition from an excited state to a ground state to thereby generate light, for example, visible light.

SUMMARY OF THE INVENTION

One or more embodiments include a composition and an organic light-emitting device including the same.

Additional aspects will be set forth in part in the description, which follows and, in part, will be apparent from the description, or may be learned by practice of the presented embodiments of the disclosure.

Provided is a composition including a first compound, a second compound and a third compound,

the first compound, the second compound and the third compound are different from each other,
the first compound satisfies one of Condition 1 and Condition 2, and
the second compound includes a compound represented by Formula 1

Condition 1
the first compound contains transition metal.

Condition 2

the difference between the triplet energy level of the first compound and the singlet energy level of the first compound is 0.4 eV or less, and the first compound emits delayed fluorescence

Formula 1

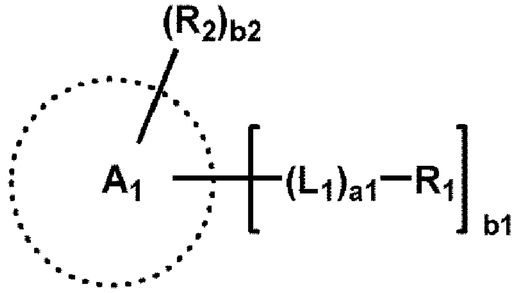

Ring $A_1$ in Formula 1 may be a condensed cyclic group in which 3 or more cyclic groups are condensed with each other, and the cyclic group may be a $C_5$-$C_{30}$ carbocyclic group or a $C_1$-$C_{30}$ heterocyclic group,
$L_1$ in Formula 1 may be a single bond, a $C_5$-$C_{30}$ carbocyclic group that is unsubstituted or substituted with at least one $R_{10a}$, or a $C_1$-$C_{30}$ heterocyclic group that is unsubstituted or substituted with at least one $R_{10a}$,
a1 in Formula 1 may be an integer from 1 to 5,
b1 in Formula 1 is an integer from 3 to 10, and
$R_1$ in Formula 1 may be a group represented by Formula 2 or Formula 3,

Formula 2

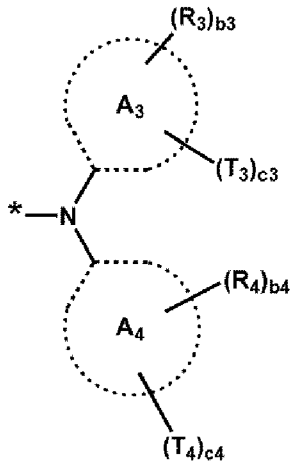

Formula 3

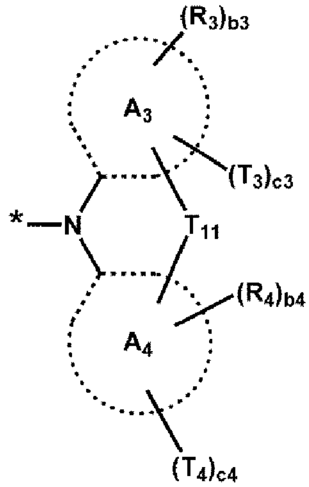

ring $A_3$ and ring $A_4$ in Formulae 2 and 3 may each independently be a $C_5$-$C_{30}$ carbocyclic group or a $C_1$-$C_{30}$ heterocyclic group,

$T_3$ and $T_4$ in Formulae 2 and 3 may each independently be a group represented by *-$C(R_5)(R_6)(R_7)$ or *-$Si(R_5)(R_6)(R_7)$,

c3 and c4 in Formulae 2 and 3 may each independently be an integer from 0 to 10, wherein, when c3 is 2 or more, two or more of $T_3$(s) may be identical to or different from each other, and when c4 is 2 or more, two or more of $T_4$(s) may be identical to or different from each other,

$T_{11}$ in Formula 3 may be a single bond, O, S, $N(R_8)$, $C(R_8)(R_9)$, or $Si(R_8)(R_9)$,

$R_2$ to $R_9$ and $R_{10a}$ in Formulae 1 to 3 may each independently be hydrogen, deuterium, -F, -Cl, -Br, -I, $-SF_5$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkyl heteroaryl group, a substituted or unsubstituted $C_2$-$C_{60}$ heteroaryl alkyl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_3)(Q_4)(Q_5)$, $-Ge(Q_3)(Q_4)(Q_5)$,$-B(Q_6)(Q_7)$, $-P(=O)(Q_8)(Q_9)$, $-P(=S)(Q_8)(Q_9)$, or $-P(Q_8)(Q_9)$,

b2 to b4 in Formula 1 to 3 may each independently be an integer from 0 to 10, wherein, when b2 is 2 or more, two or more of $R_2$(s) may be identical to or different from each other, when b3 is 2 or more, two or more of $R_3$(s) may be identical to or different from each other, and when b4 is 2 or more, two or more of $R_4$(s) may be identical to or different from each other,

a substituent of the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_1$-$C_{60}$ alkylthio group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_7$-$C_{60}$ alkyl aryl group, the substituted $C_7$-$C_{60}$ aryl alkyl group, the substituted $C_6$-$C_{60}$ aryloxy group, the substituted $C_6$-$C_{60}$ arylthio group, the substituted $C_1$-$C_{60}$ heteroaryl group, the substituted $C_2$-$C_{60}$ alkyl heteroaryl group, the substituted $C_2$-$C_{60}$ heteroaryl alkyl group, the substituted $C_1$-$C_{60}$ heteroaryloxy group, the substituted $C_1$-$C_{60}$ heteroarylthio group, the substituted monovalent non-aromatic condensed polycyclic group, and the substituted monovalent non-aromatic condensed heteropolycyclic group is

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a

salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group,

a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $-N(Q_{11})(Q_{12})$, $-Si(Q_{13})(Q_{14})(Q_{15})$, $-Ge(Q_{13})(Q_{14})(Q_{15})$,$-B(Q_{16})(Q_{17})$, $-P(=O)(Q_{18})(Q_{19})$, $-P(=S)(Q_8)(Q_9)$, $-P(Q_{18})(Q_{19})$, or a combination thereof,

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a $C_1$-$C_{60}$ heteroaryloxy group, a $C_1$-$C_{60}$ heteroarylthio group, a monovalent non-aromatic condensed polycyclic group, or a monovalent non-aromatic condensed heteropolycyclic group, each unsubstituted or substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_1$-$C_{60}$ alkylthio group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alky aryl group, a $C_7$-$C_{60}$ aryl alkyl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a $C_2$-$C_{60}$ heteroaryl alkyl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, $-N(Q_{21})(Q_{22})$, $-Si(Q_{23})(Q_{24})(Q_{25})$,$-Ge(Q_{23})(Q_{24})(Q_{25})$, $-B(Q_{26})(Q_{27})$, $-P(=O)(Q_{28})(Q_{29})$, $-P(=S)(Q_8)(Q_9)$, $-P(Q_{28})(Q_{29})$, or a combination thereof,

-N(Q31)(Q32), $-Si(Q_{33})(Q_{34})(Q_{35})$, $-Ge(Q_{33})(Q_{34})(Q_{35})$, $-B(Q_{36})(Q_{37})$,$-P(=O)(Q_{38})(Q_{39})$, $-P(=S)(Q_8)(Q_9)$, or $-P(Q_{38})(Q_{39})$, or

a combination thereof,

wherein $Q_1$ to $Q_9$, $Q_{11}$ to $Q_{19}$, $Q_{21}$ to $Q_{29}$, and $Q_{31}$ to $Q_{39}$ are each independently hydrogen, deuterium: -F: -Cl: -Br: -I: a hydroxyl group: a cyano group: a nitro group: an amidino group: a hydrazine group: a hydrazone group: a carboxylic acid group or a salt thereof: a sulfonic acid group or a salt thereof: a phosphoric acid group or a salt thereof: a $C_1$-$C_{60}$ alkyl group which is unsubstituted or substituted with at least one of deuterium, a $C_1$-$C_{60}$ alkyl group, a $C_6$-$C_{60}$ aryl group, or a combination thereof: a $C_2$-$C_{60}$ alkenyl group: a $C_2$-$C_{60}$ alkynyl group: a $C_1$-$C_{60}$ alkoxy group: a $C_1$-$C_{60}$ alkylthio group; a $C_3$-$C_{10}$ cycloalkyl group: a $C_1$-$C_{10}$ heterocycloalkyl group: a $C_3$-$C_{10}$ cycloalkenyl group: a $C_1$-$C_{10}$ heterocycloalkenyl group: a $C_6$-$C_{60}$ aryl group which is unsubstituted or substituted with at least one of deuterium, a $C_1$-$C_{60}$ alkyl group, a $C_6$-$C_{60}$ aryl group, or a combination thereof: a $C_6$-$C_{60}$ aryloxy group: a $C_6$-$C_{60}$ arylthio group: a $C_1$-$C_{60}$ heteroaryl group: a $C_2$-$C_{60}$ alkyl heteroaryl group; a $C_2$-$C_{60}$ heteroaryl alkyl group; a $C_1$-$C_{60}$ heteroaryloxy group; a $C_1$-$C_{60}$ heteroarylthio group; a monovalent non-aromatic condensed polycyclic group: or a monovalent non-aromatic condensed heteropolycyclic group.

According to one or more aspects, an organic light-emitting device includes

a first electrode,
a second electrode, and
an organic layer located between the first electrode and the second electrode,
wherein the organic layer includes an emission layer, and
wherein the organic layer includes the composition.

According to one or more aspects, an organic light-emitting device includes:

a first electrode,
a second electrode,
m light-emitting units located between the first electrode and the second electrode and including at least one emission layer, and
m-1 charge generation layers located between neighboring two light-emitting units of the m light-emitting units and including an n-type charge generation layer and a p-type charge generation layer,

wherein m is an integer of 2 or more,
a maximum emission wavelength of light emitted from at least one light-emitting unit of the m light-emitting units may be different from a maximum emission wavelength of light emitted from at least one light-emitting unit of the remaining light-emitting units, and
the at least one emission layer includes the composition.

According to one or more aspects, an organic light-emitting device includes:

a first electrode,
a second electrode, and
m emission layers located between the first electrode and the second electrode,
wherein m is an integer of 2 or more,
a maximum emission wavelength of light emitted from at least one emission layer of the m emission layers may be different from a maximum emission wavelength of light emitted from at least one emission layer of the remaining emission layers, and
at least one emission layer includes the composition.

BRIEF DESCRIPTION OF THE DRAWINGS

The above and other aspects, features, and advantages of certain embodiments of the disclosure will be more apparent from the following description taken in conjunction with the accompanying drawings, in which:

FIG. 1 shows a schematic cross-sectional view of an organic light-emitting device according to one or more embodiments;
FIG. 2 is a schematic cross-sectional view of an organic light-emitting device according to another embodiment;
FIG. 3 is a schematic cross-sectional view of an organic light-emitting device according to another embodiment;
FIG. 4 is a graph of external quantum efficiency (EQE, %) versus luminance (candela per square meter, cd/$m^2$) of the organic light-emitting device manufactured according to Example 1; and
FIG. 5 is a graph of luminance (%) versus time (hours, hr) of the organic light-emitting device manufactured according to Example 1;
FIG. 6 is a graph of EQE (%) versus luminance (cd/$m^2$) of the organic light-emitting device manufactured according to Comparative Example 1;
FIG. 7 is a graph of luminance (%) versus time (hr) of the organic light-emitting device manufactured according to Comparative Example 1;
FIG. 8 is a graph of EQE (%) versus luminance (cd/$m^2$) of the organic light-emitting device manufactured according to Example 2;
FIG. 9 is a graph of luminance (%) versus time (hr) of the organic light-emitting device manufactured according to Example 2;
FIG. 10 is a graph of EQE (%) versus luminance (cd/$m^2$) of the organic light-emitting device manufactured according to Comparative Example 2;
FIG. 11 is a graph of luminance (%) versus time (hr) of the organic light-emitting device manufactured according to Comparative Example 2;
FIG. 12 is a graph of EQE (%) versus luminance (cd/$m^2$) of the organic light-emitting device manufactured according to Example 3;
FIG. 13 is a graph of luminance (%) versus time (hr) of the organic light-emitting device manufactured according to Example 3;
FIG. 14 is a graph of EQE (%) versus luminance (cd/$m^2$) of the organic light-emitting device manufactured according to Comparative Example 3;
FIG. 15 is a graph of luminance (%) versus time (hr) of the organic light-emitting device manufactured according to Comparative Example 3;
FIG. 16 is a graph of measured relative HOD values of Example 1-1, Comparative Example 1, and Comparative Example 1-1; and
FIG. 17 is a graph of measured relative HOD values of Example 2-1, Comparative Example 2, and Comparative Example 2-1.

DETAILED DESCRIPTION OF THE EMBODIMENTS

Reference will now be made in detail to embodiments, examples of which are illustrated in the accompanying

drawings, wherein like reference numerals refer to like elements throughout the specification. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

The terminology used herein is for the purpose of describing one or more exemplary embodiments only and is not intended to be limiting. As used herein, the singular forms "a," "an," and "the" are intended to include the plural forms as well, unless the context clearly indicates otherwise. The term "or" means "and/or." It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

It will be understood that, although the terms first, second, third etc. may be used herein to describe various elements, components, regions, layers, and/or sections, these elements, components, regions, layers, and/or sections should not be limited by these terms. These terms are only used to distinguish one element, component, region, layer, or section from another element, component, region, layer, or section. Thus, a first element, component, region, layer, or section discussed below could be termed a second element, component, region, layer, or section without departing from the teachings of the present embodiments.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

It will be understood that when an element is referred to as being "on" another element, it can be directly in contact with the other element or intervening elements may be present therebetween. In contrast, when an element is referred to as being "directly on" another element, there are no intervening elements present.

Unless otherwise defined, all terms (including technical and scientific terms) used herein have the same meaning as commonly understood by one of ordinary skill in the art to which this general inventive concept belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10%, 5% of the stated value.

Composition

The composition includes a first compound, a second compound, and a third compound. The first compound, the second compound, and the third compound are the same as described herein.

The first compound, the second compound, and the third compound included in the composition are different from each other. That is, the composition may include three or more compounds that are different from each other.

The composition may satisfy Condition 3 below:

Condition 3

$$E_{HOMO_C1} - 0.4\ eV \leq E_{HOMO_C2} \leq E_{HOMO_C1} + 0.1\ eV$$

In Condition 3, $E_{HOMO_C1}$ is the highest occupied molecular orbital (HOMO) energy level value of the first compound, $E_{HOMO_C2}$ is the HOMO energy level value of the second compound, and eV is electron volts.

In this regard, the HOMO energy level value was measured using an atmospheric-pressure photoelectron spectroscopy device (manufactured by RIKEN KEIKI Co., Ltd.: AC3).

When the composition satisfies Condition 3, trapping of holes in the second compound is suppressed, and thus, direct formation of excitons due to electron-hole recombination in the second compound may be prevented. That is, it is possible to implement a mechanism in which excitons are first formed through a sensitizer (first compound), and then the excitons are moved to the second compound, which is the final luminous material, through energy transfer, and then light is emitted.

The composition may satisfy Condition 4 below:

Condition 4

$$R(HOD)_H / R(HOD)_0 \leq 1.07$$

In Condition 4, $R(HOD)_H$ is the relative HOD value of the composition including the first compound, the second compound, and the third compound, and $R(HOD)_0$ is the relative HOD value of the composition including the first compound and the third compound.

In this regard, the relative HOD value refers to the ratio of the applied voltage required to provide a specific current density to a single hole only device (HOD), and the relative HOD value is a value obtained by dividing the voltage applied to the HOD that is doped with the final luminous material (the second compound) by the voltage applied to the HOD that is not doped. The HOD structure may have, for example, the following structure, and the fabrication method therefor is the same as described herein:

ITO/HAT-CN (10 nm)/NPB (50 nm)/Test substance (40 nm)/NPB (10 nm)/Al

The first composition may satisfy one of Condition 1 or Condition 2:

Condition 1 The first compound contains transition metal

Condition 2 The difference between the triplet energy level of the first compound and the singlet energy level of the first compound is 0.4 eV or less, and the first compound may emit delayed fluorescence

According to one or more embodiments, the first compound may satisfy Condition 1.

For example, the first compound may include an organometallic compound represented by Formula 101:

Formula 101 $M_{11}(L_{11})n_{11}(L_{12})_{n12}$

wherein, in Formula 101,

$M_{11}$ may be a first-row transition metal of the Periodic Table of Elements, a second-row transition metal of the Periodic Table of Elements, or a third-row transition metal of the Periodic Table of Elements;
$L_{11}$ may be a ligand represented by one of Formulae 101-1 to 101-4;
$L_{12}$ may be a monodentate ligand or a bidentate ligand;
n11 may be 1,
n12 may be 0, 1, or 2;

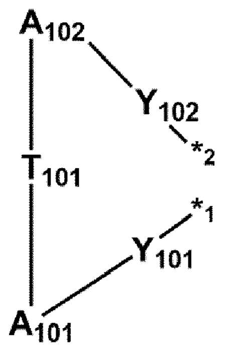

101-1

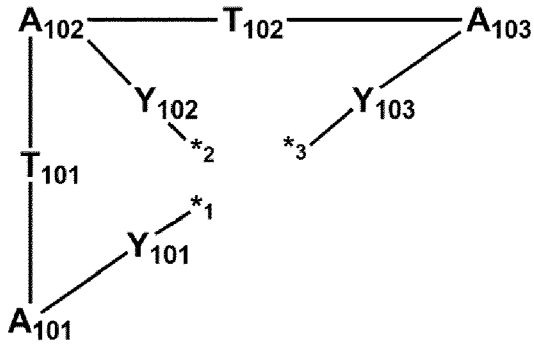

101-2

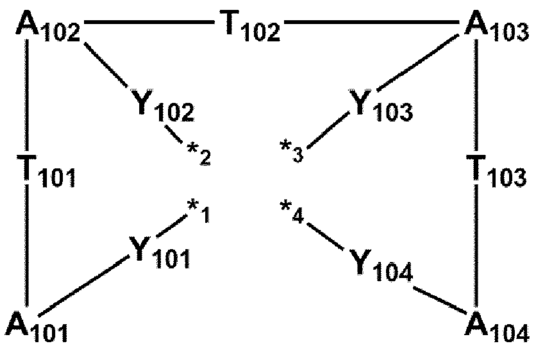

101-3

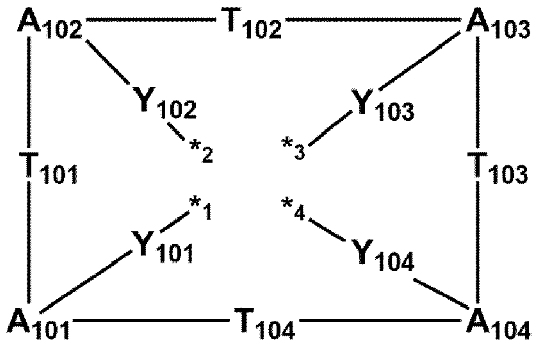

101-4

wherein, in Formulae 101-1 to 101-4,

$A_{101}$ to $A_{104}$ may each independently be a substituted or unsubstituted $C_5$-$C_{30}$ carbocyclic group, a substituted or unsubstituted $C_1$-$C_{30}$ heterocyclic group, or a non-cyclic group,

$Y_{101}$ to $Y_{104}$ may each independently be a chemical bond, O, S, $N(R_{91})$, $B(R_{91})$, $P(R_{91})$, or $C(R_{91})(R_{92})$,

$T_{101}$ to $T_{104}$ may each independently be a single bond, a double bond, $N(R_{93})$*', $B(R_{93})$, $P(R_{93})$, $C(R_{93})(R_{94})$, $Si(R_{93})(R_{94})$, $Ge(R_{93})(R_{94})$, S, Se, O, C(=O), S(=O), $S(=O)_2$, $-C(R_{93})=$, $=C(R_{93})$-, $C(R_{93})=C(R_{94})$, C(=S), or C=C,

a substituent of the substituted $C_6$-$C_{30}$ carbocyclic group, a substituent of the substituted $C_1$-$C_{30}$ heterocyclic group, and $R_{91}$ to $R_{94}$ may each independently be at least one of hydrogen, deuterium, -F, -Cl, -Br, -I, $-SF_5$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkyl heteroaryl group, a substituted or unsubstituted $C_2$-$C_{60}$ heteroaryl alkyl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-C(=O)(Q_{41})$, $-S(=O)(Q_{41})$, $-S(=O)_2(Q_{41})$, $-N(Q_{42})(Q_{43})$,-B(Q42)(Q43), $-Si(Q_{44})(Q_{45})(Q_{46})$, $-Ge(Q_{44})(Q_{45})(Q_{46})$, $-P(=O)(Q_{47})(Q_{48})$,$-P(=S)(Q_{47})(Q_{48})$, and $-P(Q_{47})(Q_{48})$, wherein each of a substituent of the substituted $C_5$-$C_{30}$ carbocyclic group and a substituent of substituted $C_1$-$C_{30}$ heterocyclic group may not be hydrogen,

$*_1$, $*_2$, $*_3$, and $*_4$ may each indicate a binding site to $M_{11}$, and

$Q_{41}$ to $Q_{48}$ may each independently be hydrogen, deuterium, -F, -Cl, -Br, -I, a hydroxyl group, a cyano group, a nitro group, an amidino group, a hydrazine group, a hydrazone group, a carboxylic acid group or a salt thereof, a sulfonic

acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_1$-$C_{60}$ alkylthio group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alkyl aryl group, a $C_7$-$C_{60}$ aryl alkyl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a $C_2$-$C_{60}$ heteroaryl alkyl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, a $C_1$-$C_{60}$ alkyl group that is substituted with at least one of deuterium, -F, a cyano group, a $C_1$-$C_{60}$ alkyl group, or a $C_6$-$C_{60}$ aryl group, and a $C_6$-$C_{60}$ aryl group that is substituted with deuterium, -F, a cyano group, a $C_1$-$C_{60}$ alkyl group, or a $C_6$-$C_{60}$ aryl group.

In one or more embodiments, the first compound may satisfy Condition 2.

For example, the first compound may include a thermally activated delayed fluorescence emitter represented by Formula 201 or 202 below:

Formula 201

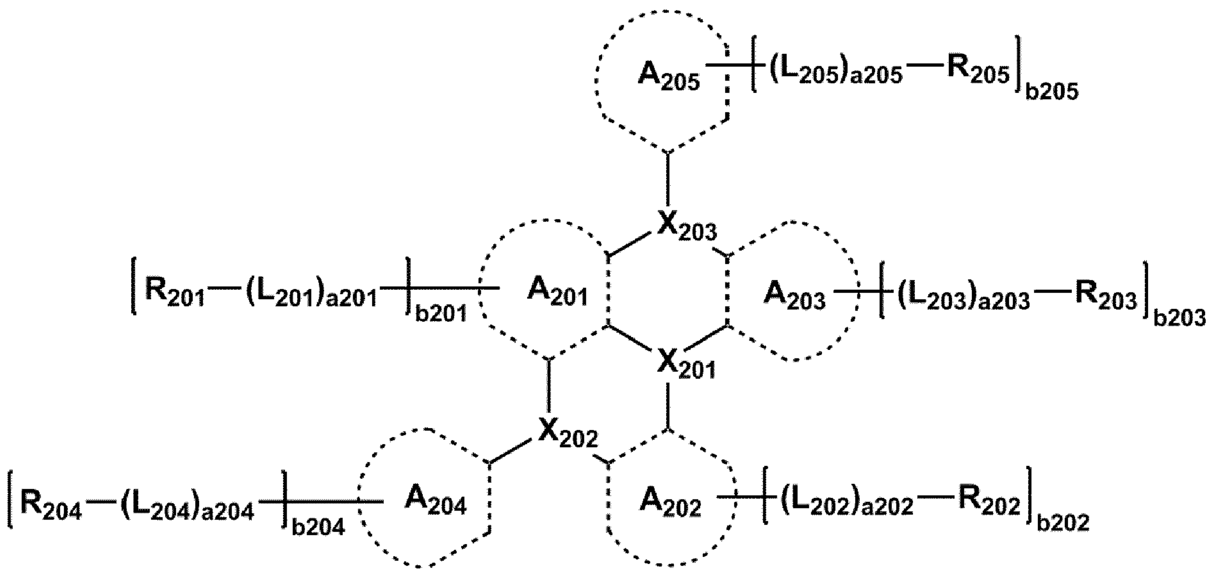

In Formula 201,

$X_{201}$ to $X_{203}$ may each independently be B or N,

$A_{201}$ to $A_{205}$ may each independently be a substituted or unsubstituted $C_5$-$C_{30}$ carbocyclic group or a substituted or unsubstituted $C_1$-$C_{30}$ heterocyclic group,

$L_{201}$ to $L_{205}$ may each independently be a single bond, a $C_5$-$C_{30}$ carbocyclic group that is unsubstituted or substituted with at least one $R_{200a}$, or a $C_1$-$C_{30}$ heterocyclic group that is unsubstituted or substituted with at least one $R_{200a}$,

a201 to a205 may each independently be an integer from 1 to 5,

$R_{201}$ to $R_{205}$, and $R_{200a}$ may each independently be hydrogen, deuterium, -F, -Cl, -Br, -I, $-SF_5$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkyl heteroaryl group, a substituted or unsubstituted $C_2$-$C_{60}$ heteroaryl alkyl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-N(Q_{51})(Q_{52})$, $-Si(Q_{53})(Q_{54})(Q_{55})$,$-Ge(Q_{53})(Q_{54})(Q_{55})$, $-B(Q_{56})(Q_{57})$, $-P(=O)(Q_{58})(Q_{69})$, $-P(=S)(Q_{58})(Q_{59})$, or$-P(Q_{58})(Q_{59})$,

b201 to b205 may each independently be an integer from 0 to 10, wherein, when b201 is 2 or more, two or more of $R_{201}$(s) may be identical to or different from each other, when b202 is 2 or more, two or more of $R_{202}$(s) may be identical to or different from each other, when b203 is 2 or more, two or more of $R_{203}$(s) may be identical to or different from each other, when b204 is 2 or more, two or more of $R_{204}$(s) may be identical to or different from each other, and when b205 is 2 or more, two or more of $R_{205}$(s) may be identical to or different from each other,

Formula 202

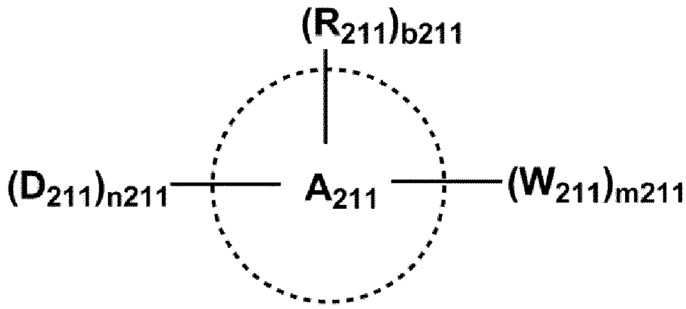

wherein, in Formula 202,

$A_{211}$ may be a substituted or unsubstituted $C_5$-$C_{30}$ carbocyclic group or a substituted or unsubstituted $C_1$-$C_{30}$ heterocyclic group,
$W_{211}$ may be an acceptor group,
$D_{211}$ may be a donor group,
m211 may be an integer from 1 to 4, and n211 may be an integer from 1 to 4;
$R_{211}$ may be hydrogen, deuterium, -F, -Cl, -Br, -I, $-SF_5$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkyl heteroaryl group, a substituted or unsubstituted $C_2$-$C_{60}$ heteroaryl alkyl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-N(Q_{51})(Q_{52})$, $-Si(Q_{53})(Q_{54})(Q_{55})$, $-Ge(Q_{53})(Q_{54})(Q_{55})$,$-B(Q_{56})(Q_{57})$, $-P(=O)(Q_{58})(Q_{69})$, $-P(=S)(Q_{58})(Q_{59})$, or $-P(Q_{58})(Q_{59})$, and a plurality of $R_{211}$(s) may optionally be bonded to form a substituted or unsubstituted $C_5$-$C_{30}$ carbocyclic group or a substituted or unsubstituted $C_1$-$C_{30}$ heterocyclic group,
a substituent of the substituted $C_5$-$C_{30}$ carbocyclic group, substituted $C_1$-$C_{30}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_1$-$C_{60}$ alkylthio group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_7$-$C_{60}$ alkyl aryl group, the substituted $C_7$-$C_{60}$ aryl alkyl group, the substituted $C_6$-$C_{60}$ aryloxy group, the substituted $C_6$-$C_{60}$ arylthio group, the substituted $C_1$-$C_{60}$ heteroaryl group, the substituted $C_2$-$C_{60}$ alkyl heteroaryl group, the substituted $C_2$-$C_{60}$ heteroaryl alkyl group, the substituted $C_1$-$C_{60}$ heteroaryloxy group, the substituted $C_1$-$C_{60}$ heteroarylthio group, the substituted monovalent non-aromatic condensed polycyclic group, and the substituted monovalent non-aromatic condensed heteropolycyclic group is:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;

a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with deuterium, -F, -Cl, -Br, -I, $-CD_3$,$-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a monovalent non-aromatic condensed heteropolycyclic group, $-N(Q_{61})(Q_{62})$, $-Si(Q_{63})(Q_{64})(Q_{65})$, $-Ge(Q_{63})(Q_{64})(Q_{65})$,-B(Q66)(Q67), $-P(=O)(Q_{58})(Q_{69})$, $-P(=S)(Q_{68})(Q_{69})$, $-P(Q_{68})(Q_{69})$, or a combination thereof;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a $C_1$-$C_{60}$ heteroaryloxy group, a $C_1$-$C_{60}$ heteroarylthio group, a monovalent non-aromatic condensed polycyclic group, or a monovalent non-aromatic condensed heteropolycyclic group, each unsubstituted or substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_1$-$C_{60}$ alkylthio group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alky aryl group, a $C_7$-$C_{60}$ aryl alkyl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a $C_2$-$C_{60}$ heteroaryl alkyl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, $-N(Q_{71})(Q_{72})$, $-Si(Q_{73})(Q_{74})(Q_{75})$,-Ge(Q73)(Q74)(Q75), $-B(Q_{76})(Q_{77})$, $-P(=O)(Q_{78})(Q_{79})$, $-P(=S)(Q_{78})(Q_{79})$,$-P(Q_{78})(Q_{79})$, or a combination thereof;

$-N(Q_{81})(Q_{82})$, $-Si(Q_{83})(Q_{84})(Q_{85})$, $-Ge(Q_{83})(Q_{84})(Q_{85})$, $-B(Q_{86})(Q_{87})$,$-P(=O)(Q_{88})(Q_{89})$, $-P(=S)(Q_{88})(Q_{89})$, or $-P(Q_{88})(Q_{89})$; or

a combination thereof,

wherein $Q_{51}$ to $Q_{59}$, $Q_{61}$ to $Q_{69}$, $Q_{71}$ to $Q_{79}$ and $Q_{81}$ to $Q_{89}$ are each independently: hydrogen; deuterium; -F; -Cl; -Br; -I; a hydroxyl group; a cyano group; a nitro group; an amidino group; a hydrazine group; a hydrazone group;

a carboxylic acid group or a salt thereof; a sulfonic acid group or a salt thereof; a phosphoric acid group or a salt thereof; a $C_1$-$C_{60}$ alkyl group which is unsubstituted or substituted with deuterium, a $C_1$-$C_{60}$ alkyl group, a $C_6$-$C_{60}$ aryl group, or a combination thereof; a $C_2$-$C_{60}$ alkenyl group; a $C_2$-$C_{60}$ alkynyl group; a $C_1$-$C_{60}$ alkoxy group; a $C_1$-$C_{60}$ alkylthio group, a $C_3$-$C_{10}$ cycloalkyl group; a $C_1$-$C_{10}$ heterocycloalkyl group; a $C_3$-$C_{10}$ cycloalkenyl group; a $C_1$-$C_{10}$ heterocycloalkenyl group; a $C_6$-$C_{60}$ aryl group which is unsubstituted or substituted with deuterium, a $C_1$-$C_{60}$ alkyl group, a $C_6$-$C_{60}$ aryl group, or a combination thereof; a $C_6$-$C_{60}$ aryloxy group; a $C_6$-$C_{60}$ arylthio group; a $C_1$-$C_{60}$ heteroaryl group; a $C_2$-$C_{60}$ alkyl heteroaryl group, a $C_2$-$C_{60}$ heteroaryl alkyl group, a $C_1$-$C_{60}$ heteroaryloxy group, a $C_1$-$C_{60}$ heteroarylthio group, a monovalent non-aromatic condensed polycyclic group; or a monovalent non-aromatic condensed heteropolycyclic group.

For example, $W_{211}$ may be a substituted or unsubstituted π electron-deficient nitrogen-free cyclic group, and $D_{211}$ may be:

-F, a cyano group, or a π electron-deficient nitrogen-containing cyclic group;

a $C_1$-$C_{60}$ alkyl group, an π-electron deficient nitrogen-containing cyclic group, or an π electron-deficient nitrogen-free cyclic group, each substituted with at least one of -F or a cyano group; or

an π-electron deficient nitrogen-containing cyclic group substituted with at least one of deuterium, a $C_1$-$C_{60}$ alkyl group, an π -electron deficient nitrogen-containing cyclic group, or an π electron-deficient nitrogen-free cyclic group, wherein the π electron-deficient nitrogen-free cyclic group may be a phenyl group, a heptalene group, an indene group, a naphthalene group, an azulene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, an isoindole group, an indole group, a furan group, a thiophene group, a benzofuran group, a benzothiophene group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, a triindolobenzene group, or a condensed cyclic group of two or more π electron-deficient nitrogen-free cyclic groups, and

the π electron-deficient nitrogen-containing cyclic group may be a cyclic group having at least one -N= moiety, and, for example, may be an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, an azacarbazole group, and a benzimidazolobenzimidazole group; or a condensed cyclic group in which two or more π electron-efficient nitrogen-containing cyclic groups are condensed with each other.

The first compound may be understood by referring to the description about a sensitizer as provided herein.

Second compound in composition

The second compound includes a compound represented by Formula 1:

Formula 1

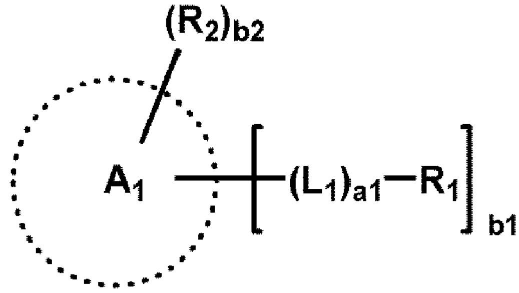

Ring $A_1$ in Formula 1 is a condensed cyclic group in which 3 or more cyclic groups are condensed with each other, and the cyclic group may be a $C_5$-$C_{30}$ carbocyclic group or a $C_1$-$C_{30}$ heterocyclic group.

In one or more embodiments, ring $A_1$ in Formula 1 may be an anthracene group, a phenalene group, a phenanthrene group, a tetracene group, a pyrene group, a chrysene group, a triphenylene group, a pentacene group, a perylene group, a fluoranthene group, a fluorene group, an acridine group, a phenanthridine group, a phenazine group, a phenoxazine group, a phenothiazine group, a xanthene group, a carbazole group, a dibenzofuran group, or a dibenzothiophene group.

For example, ring $A_1$ may be an anthracene group, a pyrene group, a chrysene group, or a perylene group.

$L_1$ in Formula 1 may be a single bond, a $C_5$-$C_{30}$ carbocyclic group that is unsubstituted or substituted with at least one $R_{10a}$, or a $C_1$-$C_{30}$ heterocyclic group that is unsubstituted or substituted with at least one $R_{10a}$.

In one or more embodiments, $L_1$ in Formula 1 may be a phenyl group, a heptalene group, an indene group, a naphthalene group, an azine group, a heptalene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, a furan group, a thiophene group, an isoindole group, an indole group, an indene group, a benzofuran group, a benzothiophene group, benzosilole group, a naphtho pyrrole group, a naphtho furan group, a naphtho thiophene group, a naphthosilole group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, a benzosilolocarbazole group, a triindolobenzene group, an acridine group, a dihydroacridine group, an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a benzoisoquinoline group, a phthalazine group, a naphthyridine group, a quinoxaline group, a benzoquinoxaline group, a quinazoline group, a cinnoline group, a phenanthridine group,

an acridine group, a phenanthroline group, a phenazine group, a benzimidazole group, an isobenzothiazole group, a benzoxazole group, a benzoisoxazole group, a triazole group, a tetrazole group, an oxadiazole group, a triazine group, a thiadiazole group, an imidazopyridine group, an imidazopyrimidine group, an azacarbazole group, an azadibenzofuran group, an azadibenzothiophene group, an azadibenzosilole group, a benzonaphthofuran group, a benzonaphthothiophene group, an (indolo)phenanthrene group, a (benzofurano)phenanthrene group, or a (benzothieno)phenanthrene group, each unsubstituted or substituted with at least one $R_{10a}$ as provided herein.

a1 in Formula 1 may be an integer from 1 to 5, wherein, when a1 is 2 or more, two or more of $L_1$(s) may be identical to or different from each other.

$R_1$ in Formula 1 may be a group represented by Formula 2 or Formula 3:

Formula 2

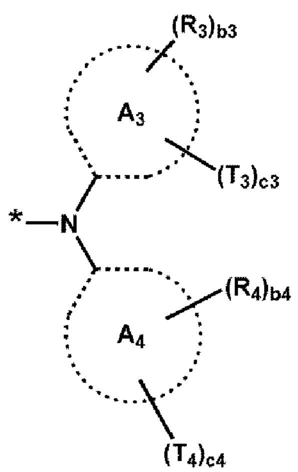

Formula 3

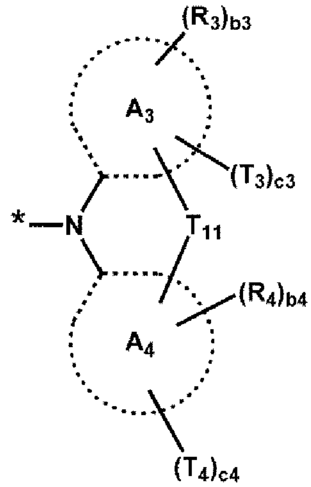

b1 in Formula 1 may be an integer from 3 to 10. For example, b1 may be an integer from 3 to 8. For example, b1 may be an integer from 3 to 6.

When b1 is 2 or more, two or more of $R_1$(s) may be identical to or different from each other.

Ring $A_3$ and ring $A_4$ in Formulae 2 and 3 may each independently be a $C_5$-$C_{30}$ carbocyclic group or a $C_1$-$C_{30}$ heterocyclic group.

In one or more embodiments, ring $A_3$ and ring $A_4$ may each independently be a phenyl group, a heptalene group, an indene group, a naphthalene group, an azulene group, a heptalene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, a furan group, a thiophene group, an isoindole group, an indole group, an indene group, a benzofuran group, a benzothiophene group, a benzosilole group, a naphthopyrrole group, a naphthofuran group, a naphthothiophene group, a naphthosilole group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, a benzosilolocarbazole group, a triindolobenzene group, an acridine group, a dihydroacridine group, an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a benzoisoquinoline group, a phthalazine group, a naphthyridine group, a quinoxaline group, a benzoquinoxaline group, a quinazoline group, a cinnoline group, a phenanthridine group, an acridine group, a phenanthroline group, a phenazine group, a benzimidazole group, an isobenzothiazole group, a benzoxazole group, a benzoisoxazole group, a triazole group, a tetrazole group, an oxadiazole group, a triazine group, a thiadiazole group, an imidazopyridine group, an imidazopyrimidine group, an azacarbazole group, an azadibenzofuran group, an azadibenzothiophene group, an azadibenzosilole group, a benzonaphthofuran group, a benzonaphthothiophene group, an (indolo)phenanthrene group, a (benzofurano)phenanthrene group, or a (benzothieno)phenanthrene group.

For example, ring $A_3$ and ring $A_4$ may each independently be a phenyl group, a naphthalene group, an anthracene group, a phenanthrene group, a triphenylene group, a fluorene group, dibenzosilole group, a carbazole group, a dibenzofuran group, or a dibenzothiophene group.

In Formulae 2 and 3, $T_3$ and $T_4$ may each independently be a group represented by *-C($R_5$)($R_6$)($R_7$) or *-Si($R_5$)($R_6$)($R_7$), and c3 and c4 may each independently be an integer from 0 to 10, wherein, when c3 is 2 or greater, two or more $T_3$(s) may be identical to or different from each other, and when c4 is 2 or greater, two or more $T_4$(s) may be identical to each other or different from each other.

In one or more embodiments, the sum of c3 and c4 in Formulae 2 and 3 may be 1 or greater.

For example, the sum of c3 and c4 in Formulae 2 and 3 may be an integer from 1 to 4.

$T_{11}$ in Formula 3 may be a single bond, O, S, N($R_8$), C($R_8$)($R_9$), or Si($R_8$)($R_9$). For example, $T_{11}$ may be a single bond.

$R_2$ to $R_9$ and $R_{10a}$ in Formulae 1 to 3 may each independently be hydrogen, deuterium, -F, -Cl, -Br, -I, $-SF_5$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkyl heteroaryl group, a substituted or unsubstituted $C_2$-$C_{60}$ heteroaryl alkyl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -N($Q_1$)($Q_2$), -Si($Q_3$)($Q_4$)($Q_5$), -Ge($Q_3$)($Q_4$)($Q_5$), -B($Q_6$)($Q_7$), -P(=O)($Q_8$)($Q_9$),-P(=S)($Q_8$)($Q_9$), or -P($Q_8$)($Q_9$).

b2 to b4 in Formula 1 to 3 may each independently be an integer from 0 to 10, wherein, when b2 is 2 or greater, two or more of $R_2$(s) may be identical to or different from each other, when b3 is 2 or greater, two or more of $R_3$(s) may be identical to or different from each other, and when b4 is 2 or greater, two or more of $R_4$(s) may be identical to or different from each other.

In one or more embodiments, $R_5$ to $R_7$ in Formulae 2 and 3 may each independently be $-CH_3$, $-CD_3$, $-CD_2H$, $-CDH_2$, a phenyl group, or a group represented by one of Formulae 9-1 to 9-19:

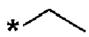
9-1

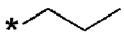
9-2

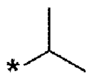
9-3

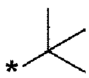
9-4

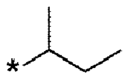
9-5

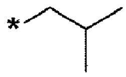
9-6

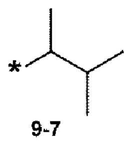
9-7

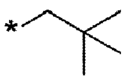
9-8

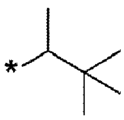
9-9

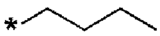
9-10

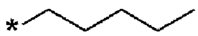
9-11

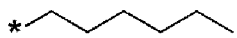
9-12

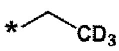
9-13

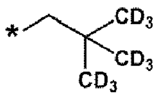
9-14

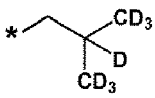
9-15

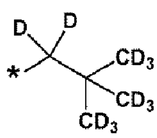
9-16

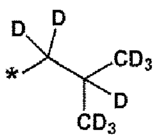
9-17

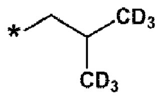
9-18

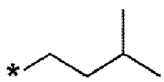
9-19

* in Formulae 9-1 to 9-19 indicates a binding site to a neighboring atom.

In one or more embodiments, the compound represented by Formula 1 may be represented by one of Formulae 1-1 to 1-4:

Formula 1-1

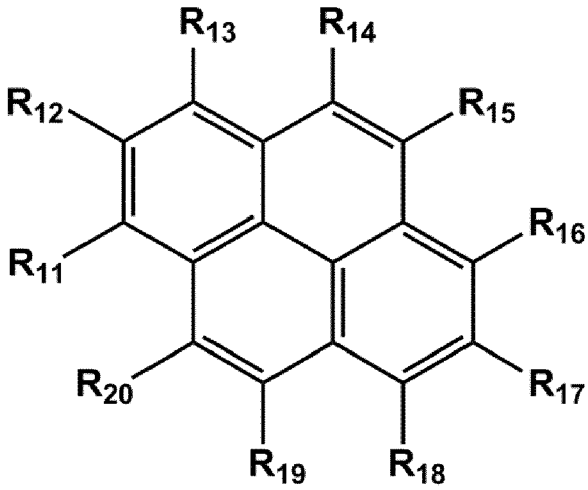

Formula 1-2

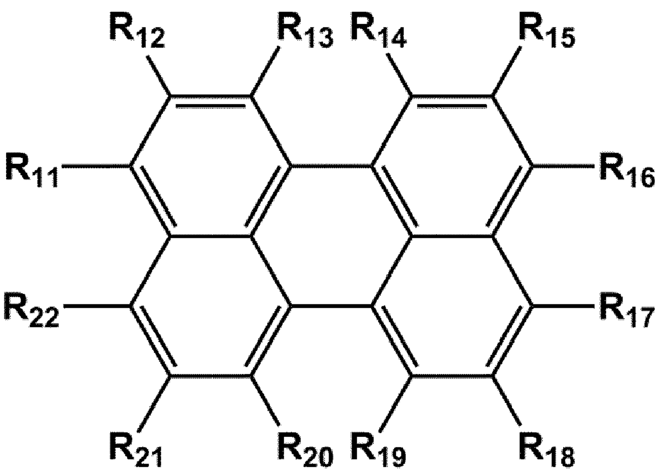

Formula 1-3

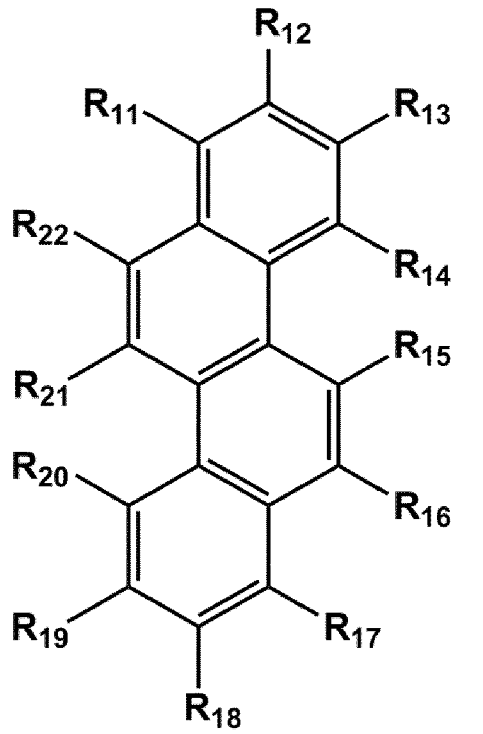

Formula 1-4

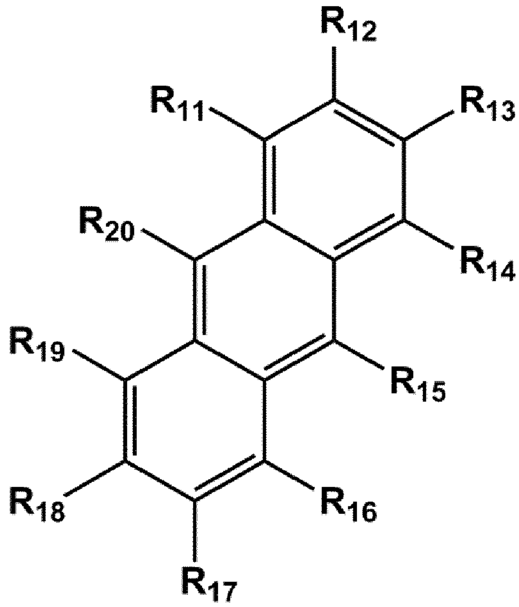

wherein, in Formulae 1-1 to 1-4,

$R_{11}$ to $R_{22}$ may each independently be a group represented by $*-(L_1)_{a1}-R_1$, hydrogen, deuterium, -F, -Cl, -Br, -I, $-SF_5$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkyl heteroaryl group, a substituted or unsubstituted $C_2$-$C_{60}$ heteroaryl alkyl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_3)(Q_4)(Q_5)$,$-Ge(Q_3)(Q_4)(Q_5)$, $-B(Q_6)(Q_7)$, $-P(=O)(Q_8)(Q_9)$, $-P(=S)(Q_8)(Q_9)$, or $-P(Q_8)(Q_9)$,

at least three of $R_{11}$ to $R_{20}$ in Formulae 1-1 and 1-4 may each independently be a group represented by $*-(L_1)_{a1}-R_1$,

at least three of $R_{11}$ to $R_{22}$ in Formulae 1-2 and 1-3 may each independently be a group represented by $*-(L_1)_{a1}-R_1$, and

the substituents of the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_1$-$C_{60}$ alkylthio group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_7$-$C_{60}$ alkyl aryl group, the substituted $C_7$-$C_{60}$ aryl alkyl group, the substituted $C_6$-$C_{60}$ aryloxy group, the substituted $C_6$-$C_{60}$ arylthio group, the substituted $C_1$-$C_{60}$ heteroaryl group, the substituted $C_2$-$C_{60}$ alkyl heteroaryl group, the substituted $C_2$-$C_{60}$ heteroaryl alkyl group, the substituted $C_1$-$C_{60}$ heteroaryloxy group, the substituted $C_1$-$C_{60}$ heteroarylthio group, the substituted monovalent non-aromatic condensed polycyclic group, and the substituted monovalent non-aromatic condensed heteropolycyclic group are the same as described above.

For example,

i) three or more of $R_{11}$, $R_{13}$, $R_{16}$, and $R_{18}$ in Formula 1-1 may each be a group represented by *-$(L_1)_{a1}$-$R_1$;
ii) three or more of $R_{12}$, $R_{15}$, $R_{18}$, and $R_{21}$ in Formula 1-2 may each be a group represented by *-$(L_1)_{a1}$-$R_1$;
iii) three or more of $R_{13}$, $R_{16}$, $R_{19}$, and $R_{22}$ in Formula 1-3 may each be a group represented by *-$(L_1)_{a1}$-$R_1$, and
iv) three or more of $R_{12}$, $R_{15}$, $R_{17}$, and $R_{20}$ in Formula 1-4 may each be a group represented by *-$(L_1)_{a1}$-R1.

In one or more embodiments, $R_1$ of Formula 1 may be a group represented by one of Formulae 2-1 to 2-42 and 3-1 to 3-36:

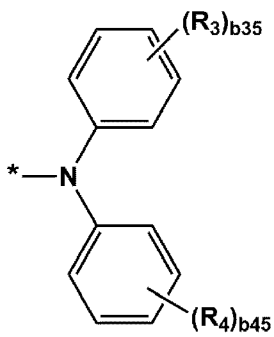

2-1

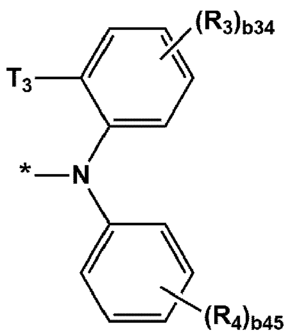

2-2

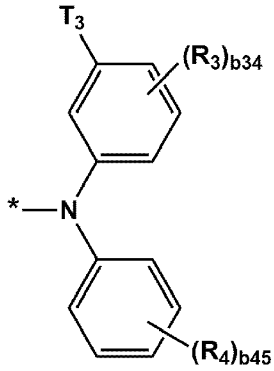

2-3

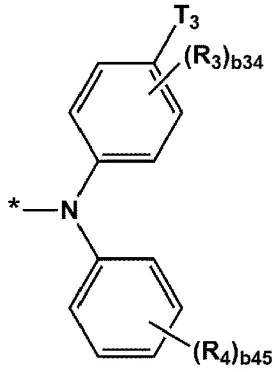

2-4

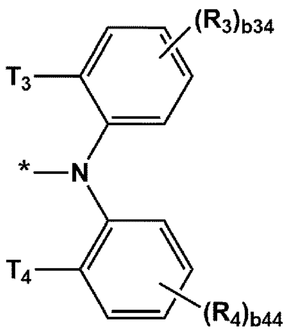

2-5

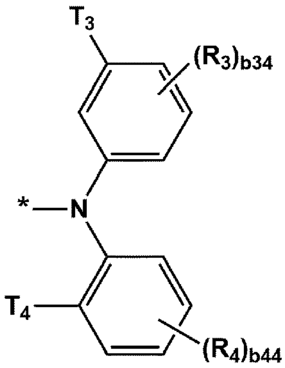

2-6

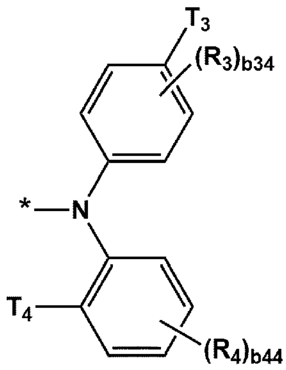

2-7

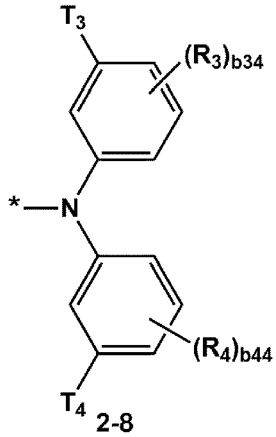

2-8

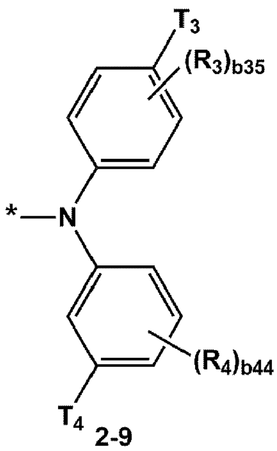

2-9

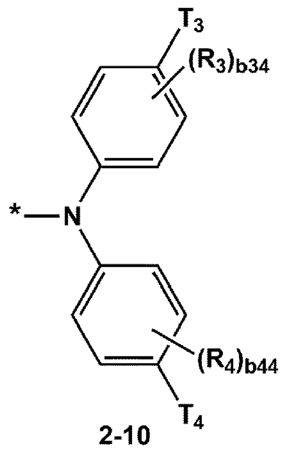

2-10

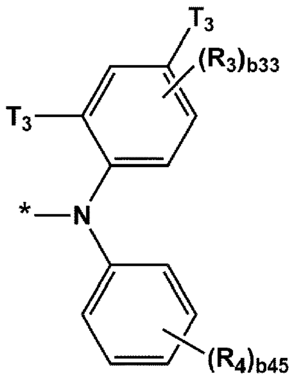

2-11

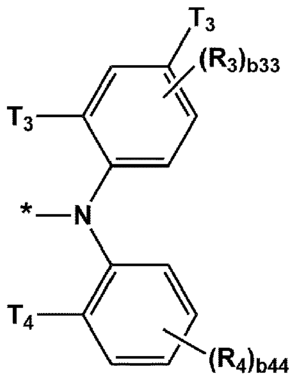

2-12

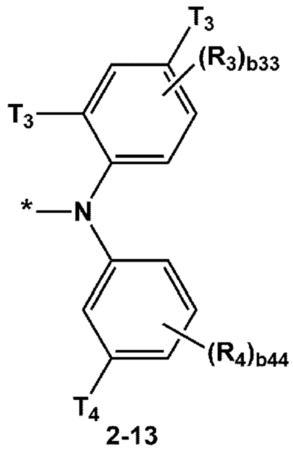

2-13

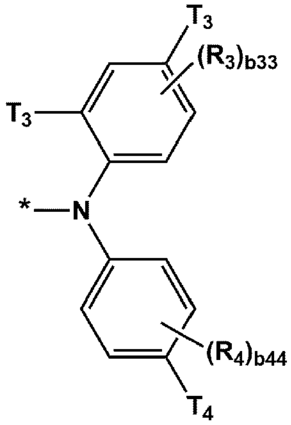

2-14

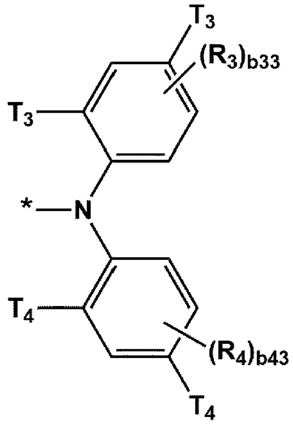

2-15

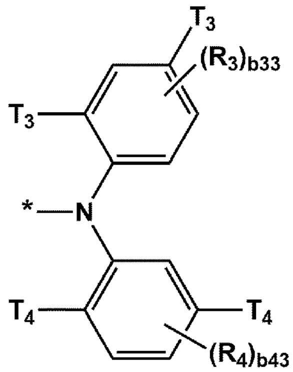

2-16

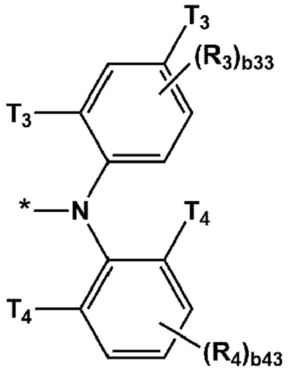

2-17

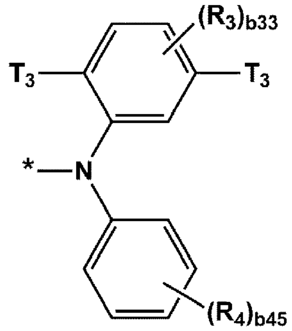

2-18

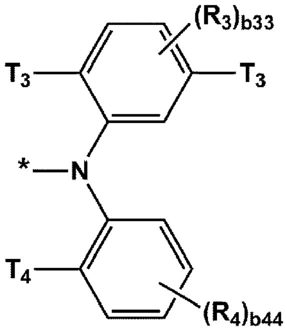

2-19

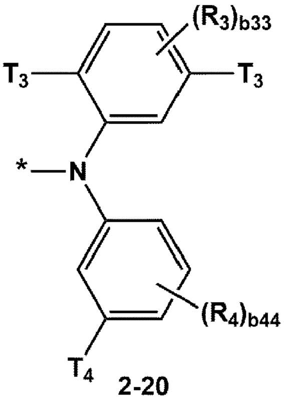

2-20

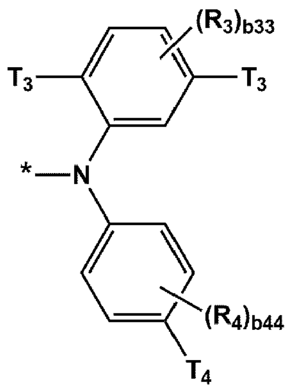

2-21

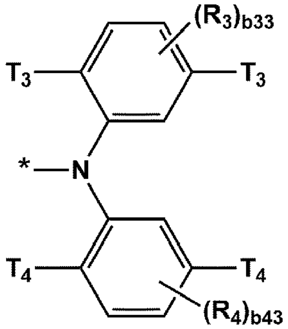

2-22

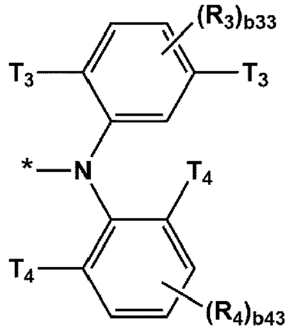

2-23

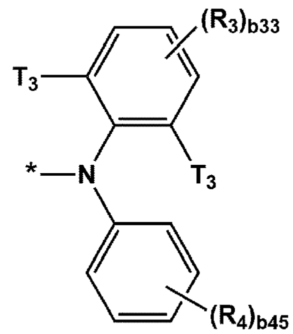

2-24

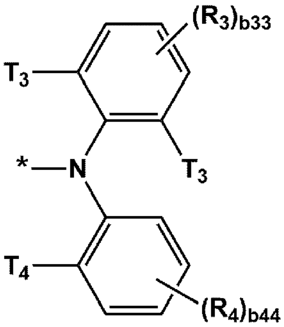
2-25

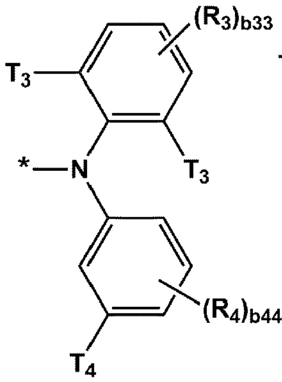
2-26

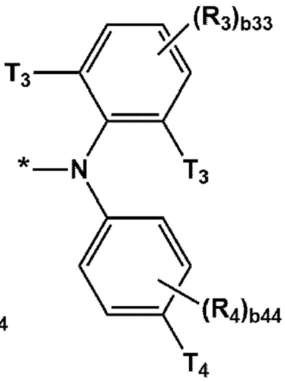
2-27

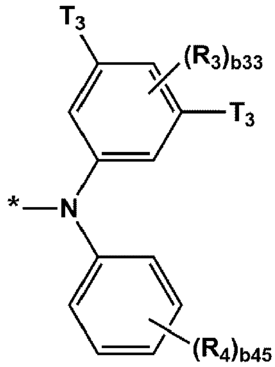
2-28

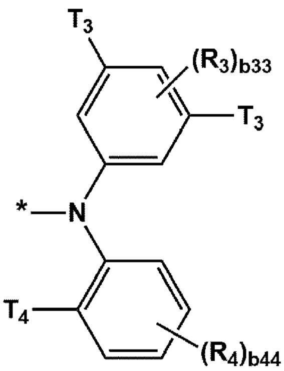
2-29

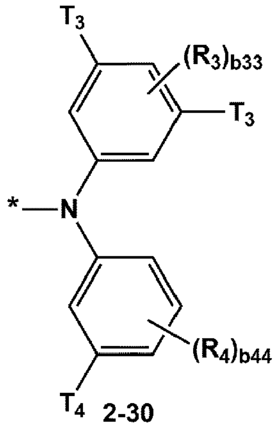
2-30

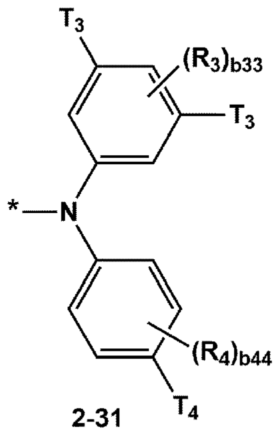
2-31

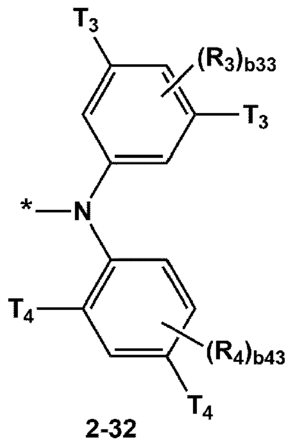
2-32

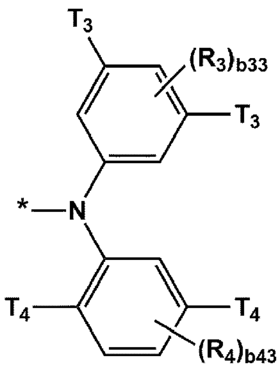
2-33

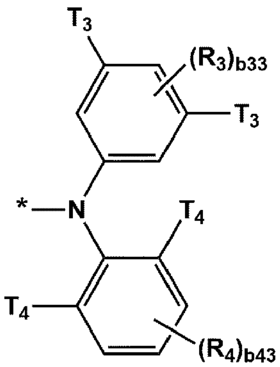
2-34

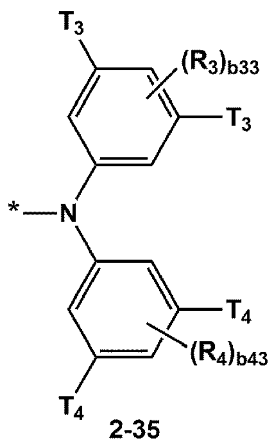
2-35

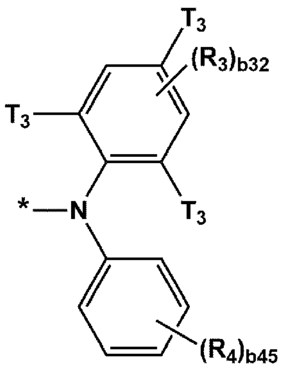
2-36

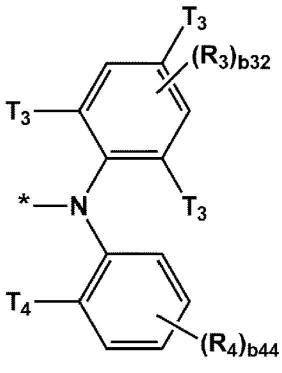

2-37

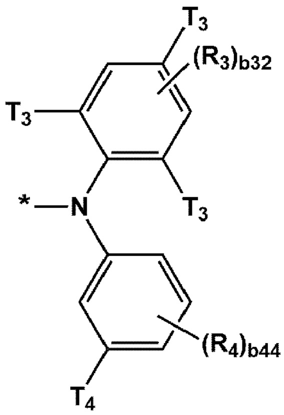

2-38

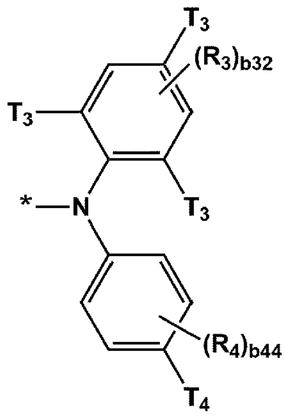

2-39

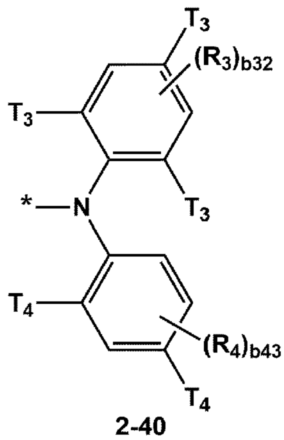

2-40

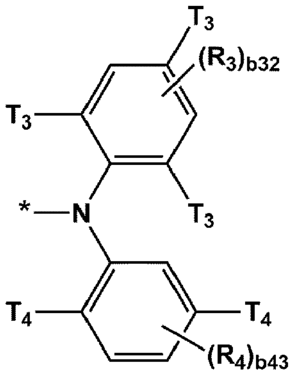

2-41

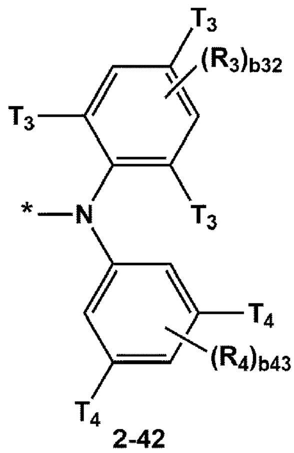

2-42

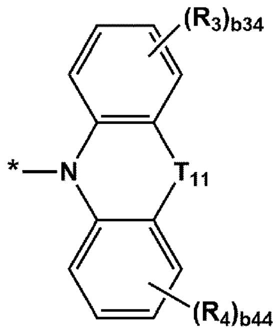

3-1

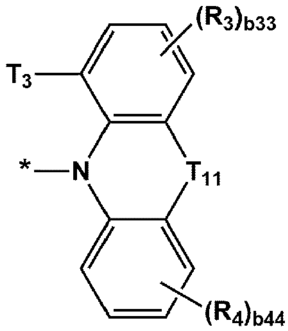

3-2

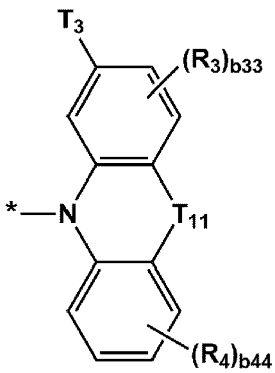

3-3

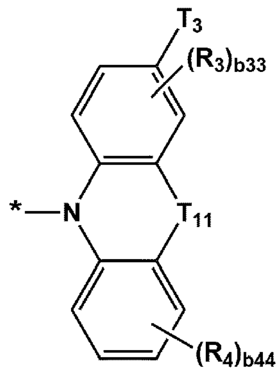

3-4

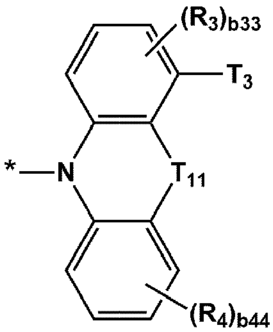

**3-5**

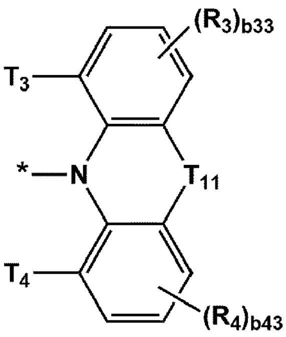

**3-6**

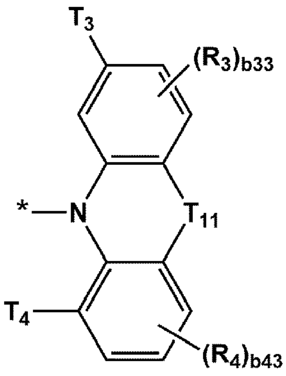

**3-7**

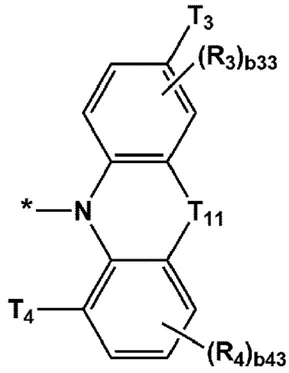

**3-8**

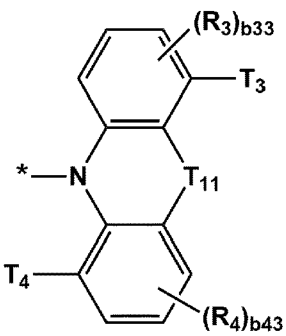

**3-9**

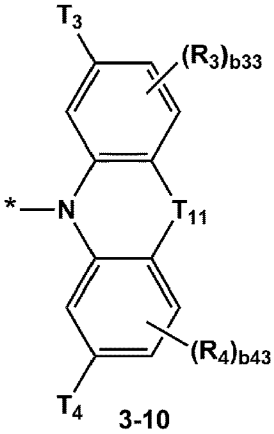

**3-10**

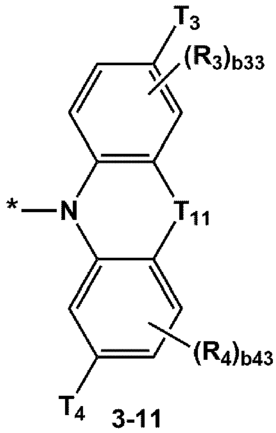

**3-11**

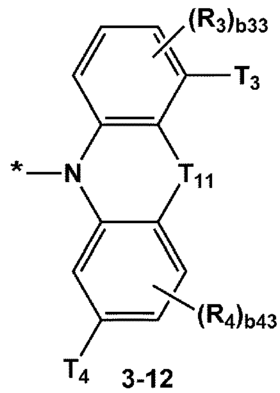

**3-12**

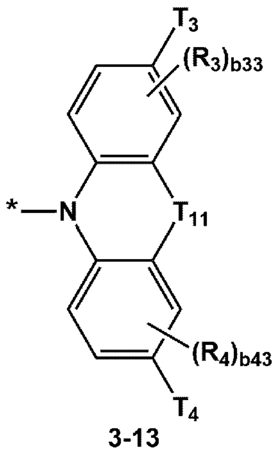

**3-13**

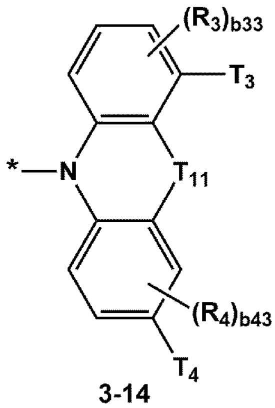

**3-14**

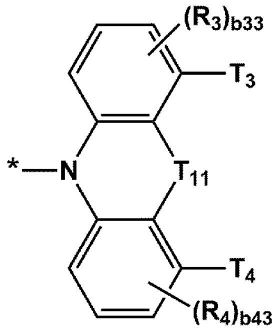

**3-15**

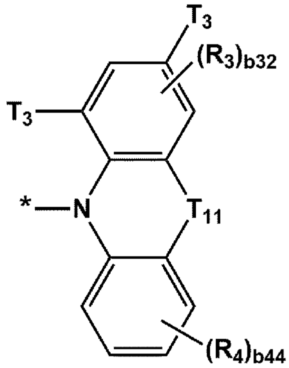

**3-16**

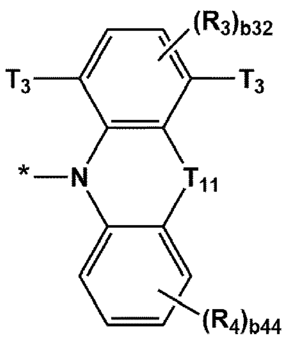

3-17

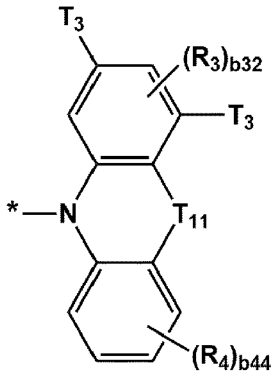

3-18

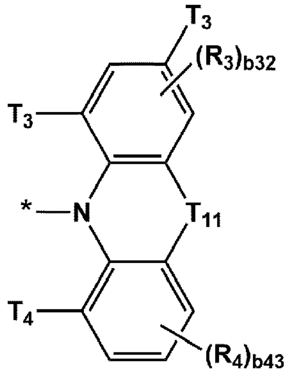

3-19

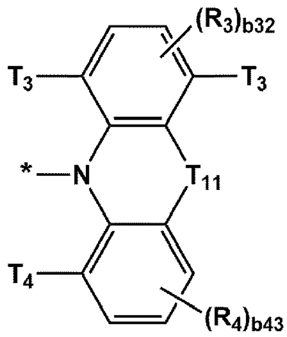

3-20

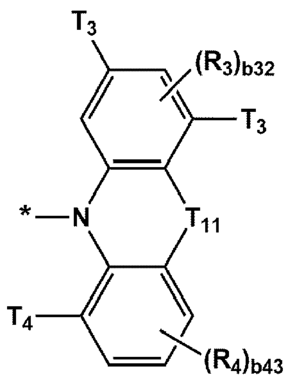

3-21

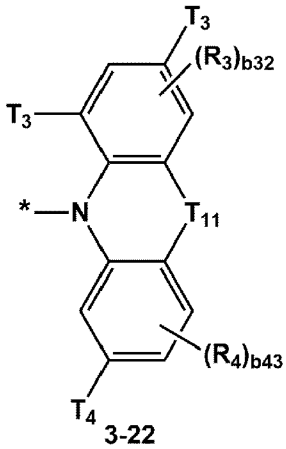

3-22

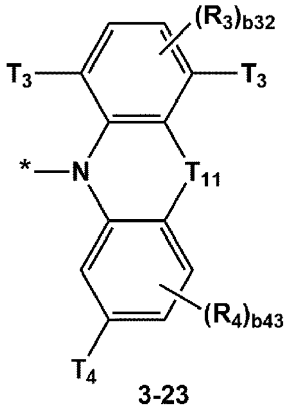

3-23

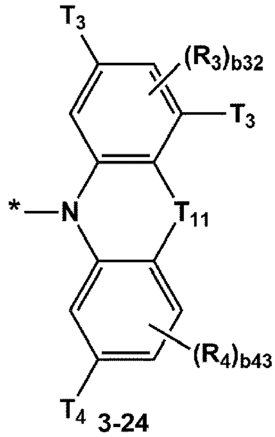

3-24

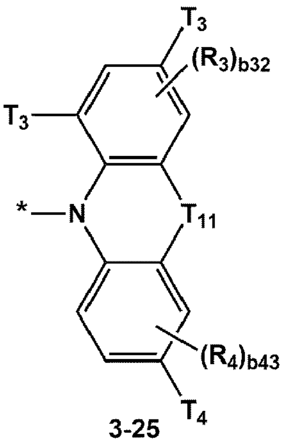

3-25

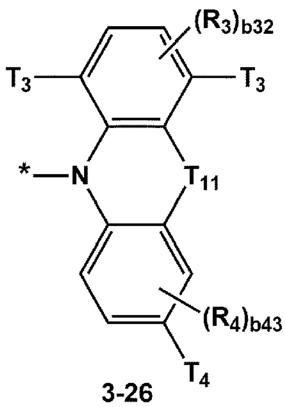

3-26

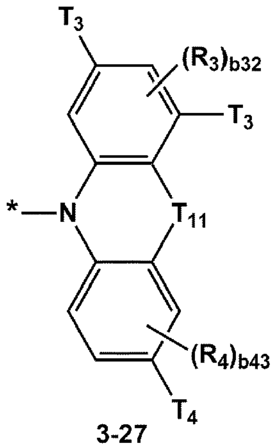

3-27

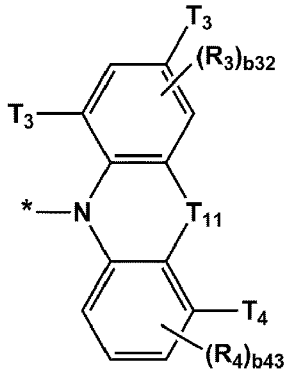

3-28

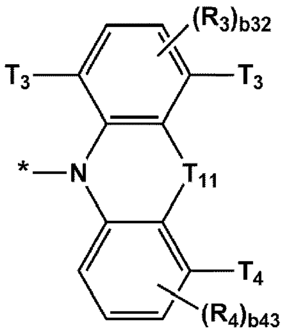

3-29

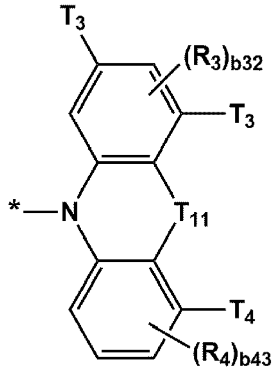

3-30

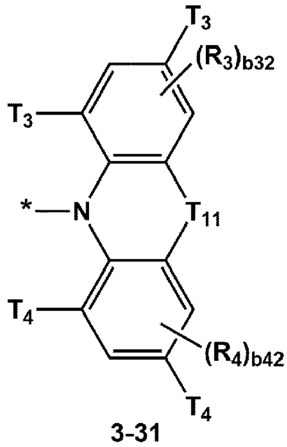

3-31

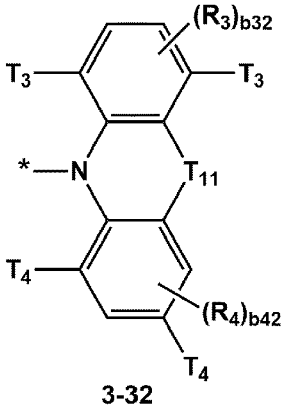

3-32

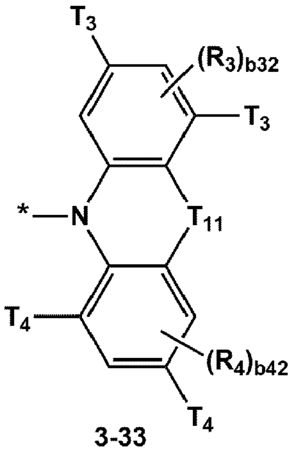

3-33

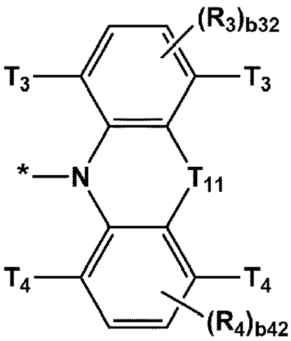

3-34

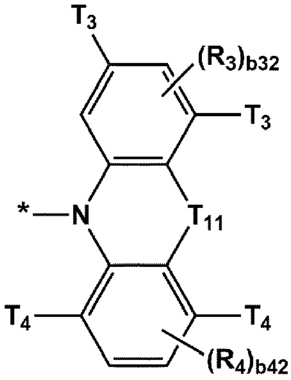

3-35

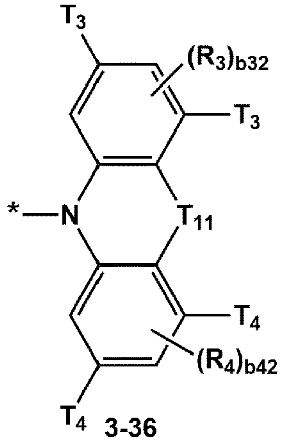

3-36

In Formulae 2-1 to 2-42 and 3-1 to 3-36,

$T_3$, $T_4$, $T_{11}$, $R_3$, and $R_4$ are the same as described above,
b32 and b42 may each independently be an integer from 0 to 2,
b33 and b43 may each independently be an integer from 0 to 3,
b34 and b44 may each independently be an integer from 0 to 4,
b35 and b45 may each independently be an integer from 0 to 5, and
* indicates a binding site to a neighboring atom.

In one or more embodiments, the second compound may be any one of Compounds FD1 to FD12:

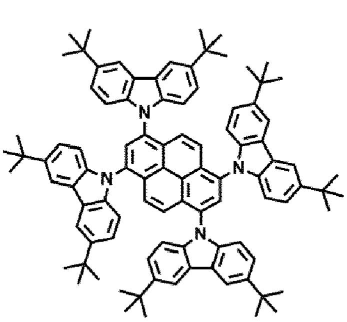

FD1

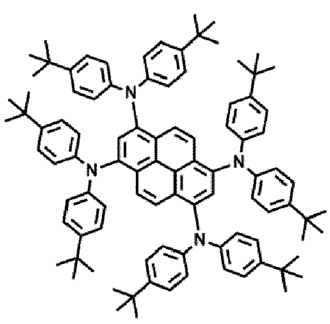

FD2

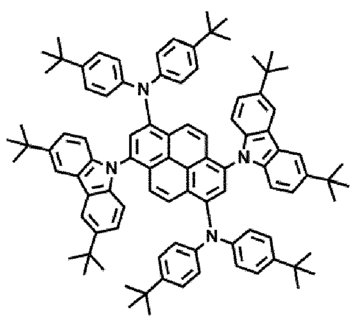

FD3

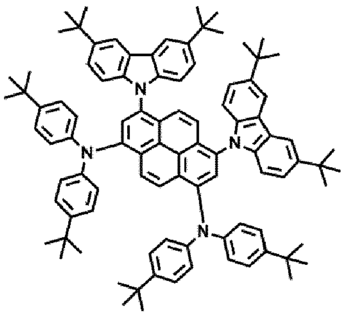

FD4

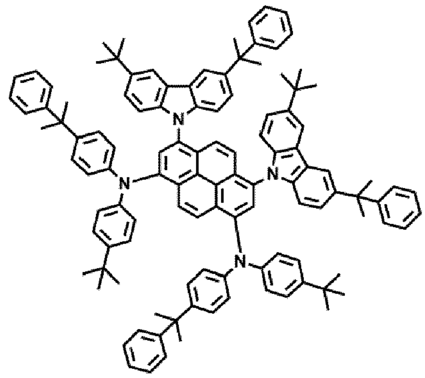

FD5

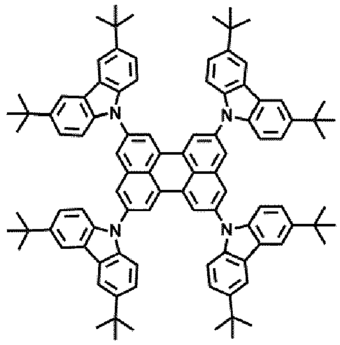

FD6

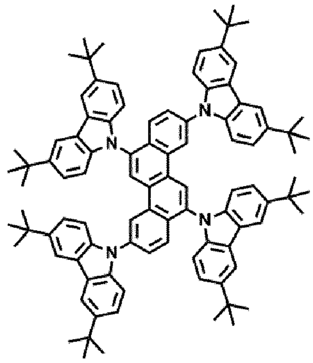

FD7

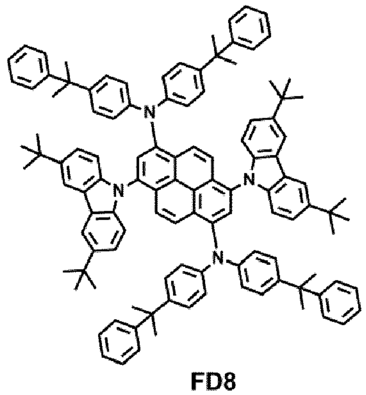

FD8

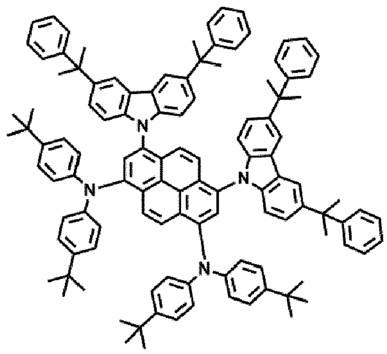

FD9

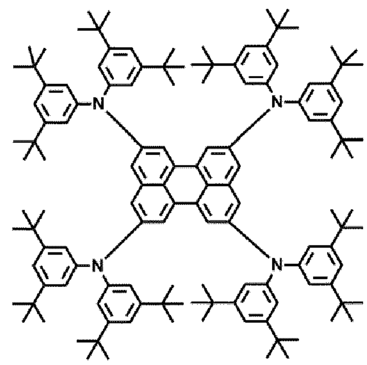

FD10

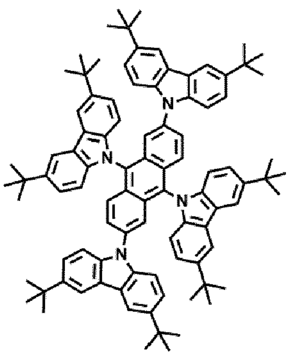

FD11

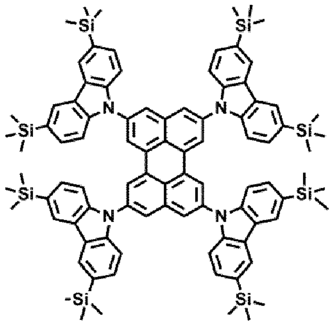

FD12

The second compound may emit blue light by having a condensed cyclic core in which three or more cyclic groups are condensed with each other.

Since the second compound includes three or more $R_1$ groups, a maximum spacing between a light-emitting portion of the sensitizer and a light-emitting portion of the dopant may be secured, suppressing dexter energy transfer.

Since the second compound may optionally contain one or more t-butyl substituents or trimethylsilyl (TMS) substituents, the maximum spacing between a light-emitting unit of the sensitizer and a light-emitting unit of the dopant may be secured, suppressing dexter energy transfer.

The second compound may be understood by referring to the description about a dopant to be provided herein.

Third compound in composition

The third compound includes a bipolar compound, an electron-transporting compound, a hole-transporting compound, or a combination thereof,

the electron-transporting compound includes at least one electron-transporting moiety,
the hole-transporting compound may not include the electron-transporting moiety, and
the electron-transporting moiety may be a cyano group, a π electron-deficient nitrogen-containing cyclic group, or a group represented by one of the following Formulae:

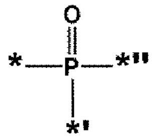 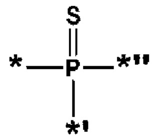 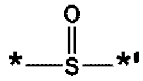 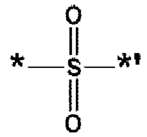

wherein *, *', and *" in the formulae above are each a binding site to a neighboring atom.

In one or more embodiments, the electron-transporting compound may include at least one π electron-deficient nitrogen-free cyclic group and at least one electron-transporting moiety,

the hole-transporting compound may include at least one π electron-deficient nitrogen-free cyclic group, and may not include an electron-transporting moiety, and
the electron-transporting moiety may be a cyano group or a π electron-deficient nitrogen-containing cyclic group.

For example, in one or more embodiments, the electron-deficient nitrogen-containing cyclic group may be an imidazole group, a pyrazole group, a thiazole group, an isothiazole group, an oxazole group, an isoxazole group, a pyridine group, a pyrazine group, a pyridazine group, a pyrimidine group, indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, phenanthroline group, phenazine group, a benzimidazole group, an isobenzothiazole group, a benzoxazole group, a benzoisoxazole group, a triazole group, a tetrazole group, an oxadiazole group, a triazine group, thiadiazole group, an imidazopyridine group, an imidazopyrimidine group, and an azacarbazole group; or a condensed cyclic group of two or more π electron-deficient nitrogen-containing cyclic groups, and
the π electron-deficient nitrogen-free cyclic group may be a phenyl group, a heptalene group, an indene group, a naphthalene group, an azulene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, an isoindole group, an indole group, a furan group, a thiophene group, a benzofuran group, a benzothiophene group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, a triindolobenzene group, or a condensed cyclic group of two or more π electron-deficient nitrogen-free cyclic groups.
For example, the electron-transporting compound includes

i) at least one of a cyano group, a pyrimidine group, a pyrazine group, and a triazine group and ii) a triphenylene group, and
the hole-transporting compound may include a carbazole group.

The third compound may be understood by referring to the description about a host as provided herein.

Description of FIG. 1

FIG. 1 is a schematic view of an organic light-emitting device 10 according to one or more embodiments. Hereinafter, a structure and a manufacturing method of an organic light-emitting device according to an example of the present disclosure will be described with reference to FIG. 1.
The organic light-emitting device 10 of FIG. 1 includes: the first electrode 11; the second electrode 19; and an organic layer 10A located between the first electrode 11 and the second electrode 19 and including the emission layer 15, wherein the organic layer 10A includes the composition described above.
In one or more embodiments, the composition may be included in the emission layer 15.
In one or more embodiments, the emission layer 15 may include a sensitizer, a dopant, and a host, wherein the sensitizer may include the first compound of the composition, the dopant may include the second compound of the

composition, and the host may include at least one third compound of the composition.

In one or more embodiments, the emission layer 15 may emit blue light.

In one or more embodiments, the organic layer 10A may include the hole transport region 12 located between the first electrode 11 and the emission layer 15, and the electron transport region 17 located between the emission layer 15 and the second electrode 19.

A substrate may be additionally located under the first electrode 11 or above the second electrode 19. For use as the substrate, any substrate that is used in organic light-emitting devices available in the art may be used, and the substrate may be a glass substrate or a transparent plastic substrate, each having excellent mechanical strength, thermal stability, transparency, surface smoothness, ease of handling, and water resistance.

First electrode 11

In one or more embodiments, the first electrode 11 may be formed by depositing or sputtering a material for forming the first electrode 11 on the substrate. The first electrode 11 may be an anode. The material for forming the first electrode 11 may be selected from materials with a high work function to facilitate hole injection.

The first electrode 11 may be a reflective electrode, a semi-transmissive electrode, or a transmissive electrode. When the first electrode 11 is a transmissive electrode, a material for forming a first electrode may be indium tin oxide (ITO), indium zinc oxide (IZO), tin oxide ($SnO_2$), zinc oxide (ZnO), or a combination thereof, but embodiments of the present disclosure are not limited thereto. In one or more embodiments, when the first electrode 110 is a semi-transmissive electrode or a reflective electrode, a material for forming the first electrode 110 may be magnesium (Mg), silver (Ag), aluminum (Al), aluminum-lithium (Al-Li), calcium (Ca), magnesium-indium (Mg-In), magnesium-silver (Mg-Ag), or a combination thereof, but embodiments of the present disclosure are not limited thereto.

The first electrode 11 may have a single-layered structure or a multi-layered structure including two or more layers.

Emission layer 15

The emission layer 15 may include a host, a dopant, and a sensitizer.

The emission layer 15 may emit fluorescent light. That is, the dopant may be a material that may emit fluorescent light. The emission layer 15, which emits the fluorescent light, is clearly distinguished from an emission layer of the related art that emits phosphorescent light.

In general, it is known that since triplet excitons stay long in an excited state, they influence the decrease in the lifespan of organic light-emitting devices. However, according to the present disclosure, the dopant is used to reduce the time during which the triplet excitons of the sensitizer remains in the excited state. Accordingly, an organic light-emitting device including the dopant may have a prolonged lifespan.

In one or more embodiments, the greater the number of triplet excitons the sensitizer has, the more excess energy is accumulated in the sensitizer, resulting in an increased number of hot excitons. That is, the amount of triplet excitons of the sensitizer is proportional to the amount of hot excitons. The hot excitons break down various chemical bonds of a compound included in the emission layer 15 and/or a compound existing at the boundary of the emission layer 15 to decompose the compound. Accordingly, the lifespan of organic light-emitting devices may be reduced. However, according to the present disclosure, by using dopants, the triplet excitons of the sensitizer may be quickly converted to singlet excitons of the dopant, ultimately reducing the amount of hot excitons and increasing the lifespan of an organic light-emitting device.

In this regard, "hot excitons" may be generated or increased by exciton-exciton annihilation due to an increase in the density of excitons in the emission layer 15, exciton-charge annihilation due to the charge imbalance in the emission layer 15, and/or radical ion pairs due to the delivery of electrons between a host and a dopant.

In addition, since the dopant emits fluorescent light, the formed organic light-emitting device may have high color purity. For example, as the singlet excitons in the excited state of the dopant at room temperature quickly switch to the ground state, the accumulation of singlet excitons in the state may be prevented and the lifespan of organic light-emitting devices may be improved.

In one or more embodiments, singlet and triplet excitons are formed at the host in the emission layer 15, and the singlet and triplet excitons formed in the host are transferred to the sensitizer and then to the dopant through Forster energy transfer (FRET). To obtain the high efficiency and long lifespan of the organic light-emitting device, hot excitons generated in the host of the emission layer 15 may be controlled and to this end, optimization of energy transfer is required.

In one or more embodiments, when the sensitizer is a thermally activated delayed fluorescence (TADF) emitter satisfying the condition of $\Delta E_{ST} \leq 0.4$ electron volts (eV), 25% of singlet excitons formed in the host is transferred to the sensitizer through FRET, and the energy of 75% of triplet excitons formed in the host is transferred to the singlet and the triplet of the sensitizer, and out of the energy, the energy transferred to the triplet is subjected to reverse intersystem

crossing to a singlet, and then the singlet energy of the sensitizer is transferred to the dopant through FRET.

Furthermore, in one or more embodiments, when the sensitizer is an organic metallic compound containing at least one type of meta from a first-row transition metal of the Periodic Table of Elements, a second-row transition metal of the Periodic Table of Elements, or a third-row transition metal of the Periodic Table of Elements, about 75% of triplet excitons formed in the host is transferred to the sensitizer through Dexter energy transfer, and the energy of about 25% of singlet excitons formed in the host is transferred to the singlet and triplet of the sensitizer, and the energy transferred to the singlet is subjected to ISC to triplet, and then the triplet energy of the sensitizer is transferred to the dopant through FRET.

Accordingly, by transferring all the singlet excitons and triplet excitons generated in the host of the emission layer 15 to the dopant, an organic light-emitting device having improved efficiency can be obtained. In addition, since an organic light-emitting device can be obtained with significantly reduced energy loss, the lifespan characteristics of the organic light-emitting device can be improved.

The amount of the sensitizer in the emission layer 15 may be from about 5 wt% to about 50 wt%. Within these ranges, it is possible to achieve effective energy transfer in the emission layer 15, and accordingly, an organic light-emitting device having high efficiency and long lifespan can be obtained.

The emission layer 15 may consist of the host, the dopant, and the sensitizer. That is, the emission layer 15 may not further include materials other than the host, the dopant, and the sensitizer.

A thickness of the emission layer 15 may be in a range of about 100 Å to about 1,000 Å, for example, about 200 Å to about 600 Å. When the thickness of the emission layer 15 is within these ranges, excellent light-emission characteristics may be obtained without a substantial increase in driving voltage.

Host in emission layer 15

The host may include at least one third compound.

The host may include no metal atoms or metal ions.

In one or more embodiments, the host may include one kind of host (i.e., a single host compound). When the host includes a single host, the single host may be a bipolar host, an electron-transporting host, or a hole-transporting host, as described herein.

In one or more embodiments, the host may include a mixture of two or more different hosts. For example, the host may be a mixture of an electron-transporting host and a hole-transporting host, a mixture of two types of electron-transporting hosts different from each other, or a mixture of two types of hole-transporting hosts different from each other. The electron-transporting host and the hole-transporting host may be understood by referring to the related description as provided herein.

In one or more embodiments, the host may include an electron-transporting host including at least one electron-transporting moiety and a hole-transporting host that is free of an electron-transporting moiety.

The electron-transporting moiety used herein may be a cyano group, a π electron-deficient nitrogen-containing cyclic group, or a group represented by one of the following Formulae:

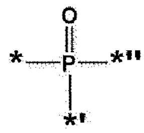 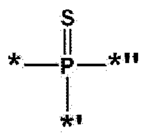 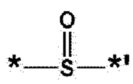 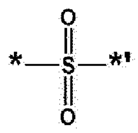

In the formulae, *, *', and *" are each binding sites to neighboring atoms.

In one or more embodiments, the electron-transporting host of the emission layer 15 may include at least one of a cyano group and a π electron-deficient nitrogen-containing cyclic group.

In one or more embodiments, the electron-transporting host in the emission layer 15 may include at least one cyano group.

In one or more embodiments, the electron-transporting host in the emission layer 15 may include at least one cyano group and at least one π electron deficient nitrogen-containing cyclic group.

In one or more embodiments, the host may include an electron-transporting host and a hole-transporting host, wherein the electron-transporting host may include at least one π electron-deficient nitrogen-free cyclic group and at least one electron-transporting moiety, and the hole-transporting host may include at least one π electron-deficient nitrogen-free cyclic group and may not include an electron-transporting moiety.

In one or more embodiments, the electron-transporting host may be a compound represented by Formula E-1, and

the hole-transporting host may be a compound represented by Formula H-1, but embodiments of the present disclosure are not limited thereto:

Formula E-1 $[Ar_{301}]_{xb11}-[(L_{301})_{xb1}-R_{301}]_{xb21}$

wherein, in Formula E-1,

$Ar_{301}$ may be a substituted or unsubstituted $C_5$-$C_{60}$ carbocyclic group or a substituted or unsubstituted $C_1$-$C_{60}$ heterocyclic group,

xb11 may be 1, 2, or 3,

$L_{301}$ may each independently be a single bond, a group represented by one of the following formulae, a substituted or unsubstituted $C_5$-$C_{60}$ carbocyclic group, or a substituted or unsubstituted $C_1$-$C_{60}$ heterocyclic group, and *, *' and *" in the following formulae are each a binding site to a neighboring atom,

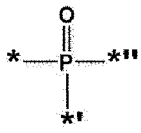 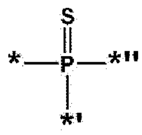 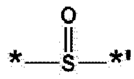 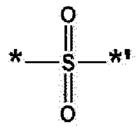

xb1 may be an integer from 1 to 5,

$R_{301}$ may be hydrogen, deuterium, -F, -Cl, -Br, -I, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkyl heteroaryl group, a substituted or unsubstituted $C_2$-$C_{60}$ heteroaryl alkyl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-Si(Q_{301})(Q_{302})(Q_{303})$, $-N(Q_{301})(Q_{302})$, $-B(Q_{301})(Q_{302})$,$-C(=O)(Q_{301})$, $-S(=O)_2(Q_{301})$, $-S(=O)(Q_{301})$, $-P(=O)(Q_{301})(Q_{302})$,$-P(=S)(Q_{301})(Q_{302})$,, or $P(Q_{301})(Q_{302})$,

xb21 may be an integer from 1 to 5,

$Q_{301}$ to $Q_{303}$ may each independently be a $C_1$-$C_{10}$ alkyl group, a $C_1$-$C_{10}$ alkoxy group, a phenyl group, a biphenyl group, a terphenyl group, or a naphthyl group, and

at least one of Condition A to Condition C may be satisfied:

Condition A $Ar_{301}$, $L_{301}$, and $R_{301}$ in Formula E-1 may each independently include a $\pi$ electron-deficient nitrogen-containing cyclic group

Condition B $L_{301}$ in Formula E-1 may be a group represented by one of the following groups:

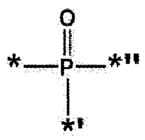 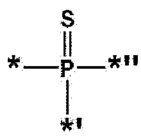 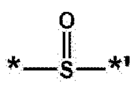 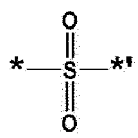

Condition C $R_{301}$ in Formula E-1 may be a cyano group, $-S(=O)_2(Q_{301})$, $-S(=O)(Q_{301})$, $-P(=O)(Q_{301})(Q_{302})$, or $-P(=S)(Q_{301})(Q_{302})$

Formula H-1 $Ar_{401}-(L_{401})_{xd1}-(Ar_{402})_{xd11}$

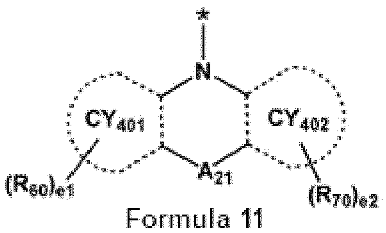

Formula 11

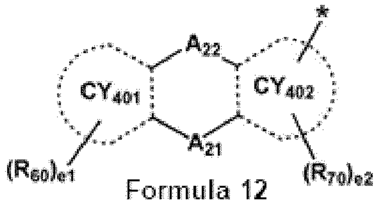

Formula 12

wherein, in Formulae H-1, 11, and 12,
$L_{401}$ may be:

a single bond; or
a phenyl group, a heptalene group, an indene group, a naphthalene group, an azulene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, an isoindole group, an indole group, a furan group, a thiophene group, a benzofuran group, a benzothiophene group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, or a triindolobenzene group, each unsubstituted or substituted with at least one of deuterium, a $C_1$-$C_{10}$ alkyl group, a $C_1$-$C_{10}$ alkoxy group, a phenyl group, a naphthyl group, a fluorenyl group, a carbazolyl group, a dibenzofuranyl group, a dibenzothiophenyl group, a triphenylenyl group, a biphenyl group, a terphenyl group, a quaterphenyl group, or-Si($Q_{401}$)($Q_{402}$)($Q_{403}$),
xd1 may be an integer from 1 to 10, wherein when xd1 is 2 or more, two or more of $L_{401}$(s) may be identical to or different from each other,
$Ar_{401}$ may be a group represented by one of Formulae 11 and 12,
$Ar_{402}$ may be a group represented by one of Formula 11 and 12, a phenyl group, a naphthyl group, a fluorenyl group, a carbazolyl group, a dibenzofuranyl group, a dibenzothiophenyl group, a biphenyl group, a terphenyl group, or a triphenylenyl group; or
a phenyl group, a naphthyl group, a fluorenyl group, a carbazolyl group, a dibenzofuranyl group, a dibenzothiophenyl group, a biphenyl group, a terphenyl group, and a triphenylenyl group, each substituted with at least one of deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a naphthyl group, a fluorenyl group, a carbazolyl group, a dibenzofuranyl group, a dibenzothiophenyl group, a biphenyl group, a terphenyl group, or a triphenylenyl group,
$CY_{401}$ and $CY_{402}$ may each independently be a phenyl group, a naphthalene group, a fluorene group, a carbazole group, a benzocarbazole group, an indolocarbazole group, a dibenzofuran group, a dibenzothiophene group, a dibenzosilole group, a benzonaphthofuran group, a benzonaphthothiophene group, or a benzonaphthosilole group,
$A_{21}$ may be a single bond, O, S, N($R_{51}$), C($R_{51}$)($R_{52}$), or Si($R_{51}$)($R_{52}$),
$A_{22}$ may be a single bond, O, S, N($R_{53}$), C($R_{53}$)($R_{54}$), or Si($R_{53}$)($R_{54}$),
at least one of $A_{21}$ and $A_{22}$ in Formula 12 is not a single bond,
$R_{51}$ to $R_{54}$, $R_{60}$, and $R_{70}$ may each independently be:

hydrogen, deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, or a $C_1$-$C_{20}$ alkoxy group;
a $C_1$-$C_{20}$ alkyl group or a $C_1$-$C_{20}$ alkoxy group, each substituted with at least one of deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a fluorenyl group, a carbazolyl group, a dibenzofuranyl group, or a dibenzothiophenyl group;
a $\pi$ electron-deficient nitrogen-free cyclic group (for example, a phenyl group, a naphthyl group, a fluorenyl group, a carbazolyl group, a dibenzofuranyl group, a dibenzothiophenyl group, a biphenyl

group, a terphenyl group, or a triphenylenyl group);

a π electron-deficient nitrogen-free cyclic group (for example, a phenyl group, a naphthyl group, a fluorenyl group, a carbazolyl group, a dibenzofuranyl group, a dibenzothiophenyl group, a biphenyl group, a terphenyl group, and a triphenylenyl group), each substituted with at least one of deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a naphthyl group, a fluorenyl group, a carbazolyl group, a dibenzofuranyl group, a dibenzothiophenyl group, or a biphenyl group; or

-Si($Q_{404}$)($Q_{405}$)($Q_{406}$),

e1 and e2 may each independently be an integer from 0 to 10,

$Q_{401}$ to $Q_{406}$ may each independently be hydrogen, deuterium, a hydroxyl group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a fluorenyl group, a carbazolyl group, a dibenzofuranyl group, a dibenzothiophenyl group, a biphenyl group, a terphenyl group, or a triphenylenyl group, and

* indicates a binding site to an adjacent atom.

In one or more embodiments, $Ar_{301}$ and $L_{301}$ in Formula E-1 may each independently be a phenyl group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, or an azacarbazole group, each unsubstituted or substituted with at least one of deuterium, -F, -Cl, -Br, -I, a hydroxyl group, a cyano group, a nitro group, an amidino group, a hydrazine group, a hydrazone group, a $C_1$-$C_{20}$ alkyl group, a $C_1$-$C_{20}$ alkoxy group, a phenyl group, a biphenyl group, a terphenyl group, a naphthyl group, a cyano-containing phenyl group, a cyano-containing biphenyl group, a cyano-containing terphenyl group, a cyano-containing naphthyl group, a pyridinyl group, a phenylpyridinyl group, a diphenylpyridinyl group, a biphenylpyridinyl group, a di(biphenyl)pyridinyl group, a pyrazinyl group, a phenylpyrazinyl group, a diphenylpyrazinyl group, a biphenylpyrazinyl group, a di(biphenyl)pyrazinyl group, a pyridazinyl group, a phenylpyridazinyl group, a diphenylpyridazinyl group, a biphenylpyridazinyl group, a di(biphenyl)pyridazinyl group, a pyrimidinyl group, a phenylpyrimidinyl group, a diphenylpyrimidinyl group, a biphenylpyrimidinyl group, a di(biphenyl)pyrimidinyl group, a triazinyl group, a phenyltriazinyl group, a diphenyltriazinyl group, a biphenyltriazinyl group, a di(biphenyl)triazinyl group, - Si($Q_{31}$)($Q_{32}$)($Q_{33}$), -N($Q_{31}$)($Q_{32}$), -B($Q_{31}$)($Q_{32}$), -C(=O)($Q_{31}$), -S(=O)$_2$($Q_{31}$), -P($Q_{31}$)($Q_{32}$), -P(=O)($Q_{31}$)($Q_{32}$), or -P(=S)($Q_{31}$)($Q_{32}$),

at least one of $L_{301}$(s) in the number of xb1 may each independently be an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, or an azacarbazole group, each unsubstituted or substituted with at least one of deuterium, -F, -Cl, -Br, - I, a hydroxyl group, a cyano group, a nitro group, an amidino group, a hydrazine group, a hydrazone group, a $C_1$-$C_{20}$ alkyl group, a $C_1$-$C_{20}$ alkoxy group, a phenyl group, a biphenyl group, a terphenyl group, a naphthyl group, a cyano group-containing phenyl group, a cyano group-containing biphenyl group, a cyano group-containing terphenyl group, a cyano group-containing naphthyl group, a pyridinyl group, a phenylpyridinyl group, a diphenylpyridinyl group, a biphenylpyridinyl group, a di(biphenyl)pyridinyl group, a pyrazinyl group, a phenylpyrazinyl group, a diphenylpyrazinyl group, a biphenylpyrazinyl group, a di(biphenyl)pyrazinyl group, a pyridazinyl group, a phenylpyridazinyl group, a diphenylpyridazinyl group, a biphenylpyridazinyl group, a di(biphenyl)pyridazinyl group, a pyrimidinyl group, a phenylpyrimidinyl group, a diphenylpyrimidinyl group, a biphenylpyrimidinyl group, a di(biphenyl)pyrimidinyl group, a triazinyl group, a phenyltriazinyl group, a diphenyltriazinyl group, a biphenyltriazinyl group, a di(biphenyl)triazinyl

group, $-Si(Q_{31})(Q_{32})(Q_{33})$, $-N(Q_{31})(Q_{32})$, $-B(Q_{31})(Q_{32})$, $-C(=O)(Q_{31})$, $-S(=O)_2(Q_{31})$, or-$P(=O)(Q_{31})(Q_{32})$, and $R_{301}$ may be hydrogen, deuterium, -F, -Cl, -Br, -I, a hydroxyl group, a cyano group, a nitro group, an amidino group, a hydrazine group, a hydrazone group, a $C_1$-$C_{20}$ alkyl group, a $C_1$-$C_{20}$ alkoxy group, a phenyl group, a biphenyl group, a terphenyl group, a quaterphenyl group, a naphthyl group, a cyano-containing phenyl group, a cyano-containing biphenyl group, a cyano-containing terphenyl group, a cyano-containing quaterphenyl group, a cyano-containing naphthyl group, a pyridinyl group, a phenylpyridinyl group, a diphenylpyridinyl group, a biphenylpyridinyl group, a di(biphenyl)pyridinyl group, a pyrazinyl group, a phenylpyrazinyl group, a diphenylpyrazinyl group, a biphenylpyrazinyl group, a di(biphenyl)pyrazinyl group, a pyridazinyl group, a phenylpyridazinyl group, a diphenylpyridazinyl group, a biphenylpyridazinyl group, a di(biphenyl)pyridazinyl group, a pyrimidinyl group, a phenylpyrimidinyl group, a diphenylpyrimidinyl group, a biphenylpyrimidinyl group, a di(biphenyl)pyrimidinyl group, a triazinyl group, a phenyltriazinyl group, a diphenyltriazinyl group, a biphenyltriazinyl group, a di(biphenyl)triazinyl group, $-Si(Q_{31})(Q_{32})(Q_{33})$, $-N(Q_{31})(Q_{32})$, $-B(Q_{31})(Q_{32})$, $-C(=O)(Q_{31})$, $-S(=O)_2(Q_{31})$, or-$P(=O)(Q_{31})$(Q32),
wherein $Q_{31}$ to $Q_{33}$ may each independently be a $C_1$-$C_{10}$ alkyl group, a $C_1$-$C_{10}$ alkoxy group, a phenyl group, a biphenyl group, a terphenyl group, or a naphthyl group, but embodiments of the present disclosure are not limited thereto.

In one or more embodiments,

$Ar_{301}$ may be a phenyl group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, or a dibenzothiophene group, each unsubstituted or substituted with at least one of deuterium, -F, -Cl, -Br, - I, a hydroxyl group, a cyano group, a nitro group, an amidino group, a hydrazine group, a hydrazone group, a $C_1$-$C_{20}$ alkyl group, a $C_1$-$C_{20}$ alkoxy group, a phenyl group, a biphenyl group, a terphenyl group, a naphthyl group, a cyano-containing phenyl group, a cyano-containing biphenyl group, a cyano-containing terphenyl group, a cyano-containing naphthyl group, a pyridinyl group, a phenylpyridinyl group, a diphenylpyridinyl group, a biphenylpyridinyl group, a di(biphenyl)pyridinyl group, a pyrazinyl group, a phenylpyrazinyl group, a diphenylpyrazinyl group, a biphenylpyrazinyl group, a di(biphenyl)pyrazinyl group, a pyridazinyl group, a phenylpyridazinyl group, a diphenylpyridazinyl group, a biphenylpyridazinyl group, a di(biphenyl)pyridazinyl group, a pyrimidinyl group, a phenylpyrimidinyl group, a diphenylpyrimidinyl group, a biphenylpyrimidinyl group, a di(biphenyl)pyrimidinyl group, a triazinyl group, a phenyltriazinyl group, a diphenyltriazinyl group, a biphenyltriazinyl group, a di(biphenyl)triazinyl group, $-Si(Q_{31})(Q_{32})(Q_{33})$, $-N(Q_{31})(Q_{32})$, $-B(Q_{31})(Q_{32})$, $-C(=O)(Q_{31})$, $-S(=O)_2(Q_{31})$, or-$P(=O)(Q_{31})(Q_{32})$; or
a group represented by one Formulae 5-1 to 5-3 or Formulae 6-1 to 6-33, and
$L_{301}$ may be a group represented by one of Formulae 5-1 to 5-3 and Formulae 6-1 to 6-33:

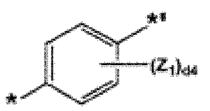
5-1
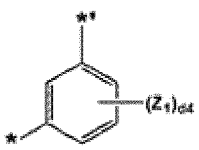
5-2
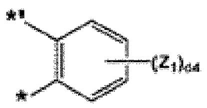
5-3

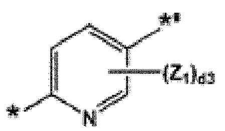
6-1
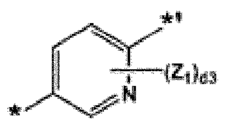
6-2
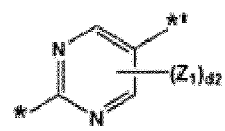
6-3
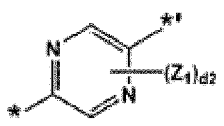
6-4

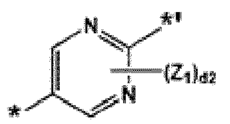
6-5
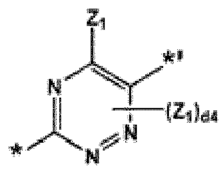
6-6
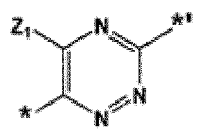
6-7
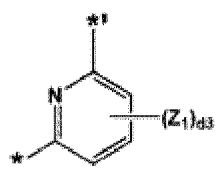
6-8

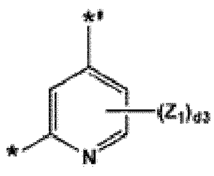
6-9

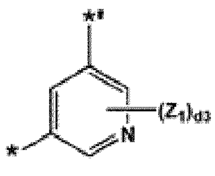
6-10

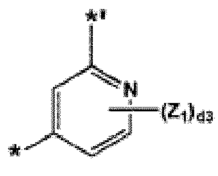
6-11

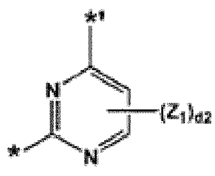
6-12

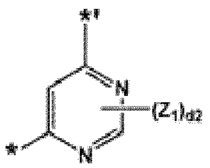
6-13

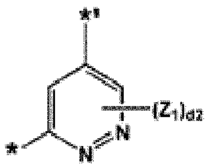
6-14

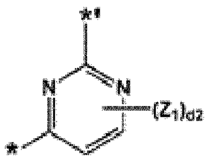
6-15

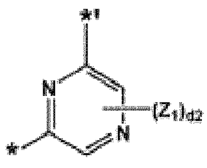
6-16

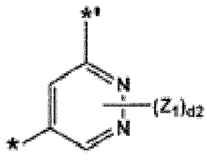
6-17

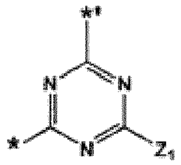
6-18

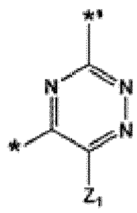
6-19

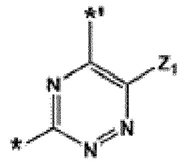
6-20

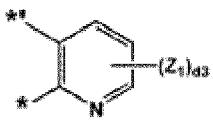
6-21

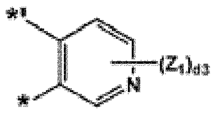
6-22

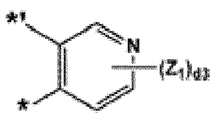
6-23

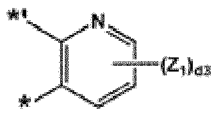
6-24

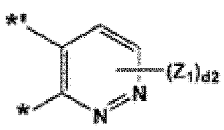
6-25

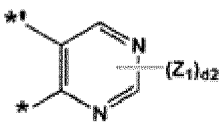
6-26

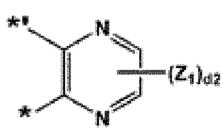
6-27

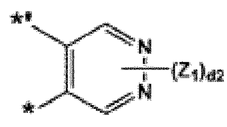
6-28

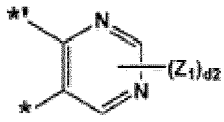
6-29

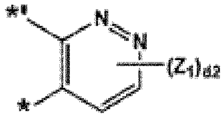
6-30

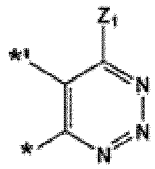
6-31

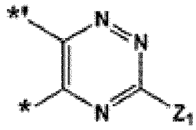
6-32

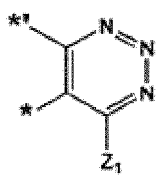
6-33

wherein, in Formulae 5-1 to 5-3 and 6-1 to 6-33,

$Z_1$ may be hydrogen, deuterium, -F, -Cl, -Br, -I, a hydroxyl group, a cyano group, a nitro group, an amidino group, a hydrazine group, a hydrazone group, a $C_1$-$C_{20}$ alkyl group, a $C_1$-$C_{20}$ alkoxy group, a phenyl group, a biphenyl group, a terphenyl group, a naphthyl group, a cyano-containing phenyl group, a cyano-containing biphenyl group,

a cyano-containing terphenyl group, a cyano-containing naphthyl group, a pyridinyl group, a phenylpyridinyl group, a diphenylpyridinyl group, a biphenylpyridinyl group, a di(biphenyl)pyridinyl group, a pyrazinyl group, a phenylpyrazinyl group, a diphenylpyrazinyl group, a biphenylpyrazinyl group, a di(biphenyl)pyrazinyl group, a pyridazinyl group, a phenylpyridazinyl group, a diphenylpyridazinyl group, a biphenylpyridazinyl group, a di(biphenyl)pyridazinyl group, a pyrimidinyl group, a phenylpyrimidinyl group, a diphenylpyrimidinyl group, a biphenylpyrimidinyl group, a di(biphenyl)pyrimidinyl group, a triazinyl group, a phenyltriazinyl group, a diphenyltriazinyl group, a biphenyltriazinyl group, a di(biphenyl)triazinyl group, $-Si(Q_{31})(Q_{32})(Q_{33})$, $-N(Q_{31})(Q_{32})$, $-B(Q_{31})(Q_{32})$, $-C(=O)(Q_{31})$, $-S(=O)_2(Q_{31})$, or-$P(=O)(Q_{31})(Q32)$,

d4 may be 0, 1, 2, 3, or 4,

d3 may be 0, 1, 2, or 3,

d2 may be 0, 1, or 2, and

* and *' each indicate a binding site to a neighboring atom,

wherein $Q_{31}$ to $Q_{33}$ are the same as described above.

In one or more embodiments, $L_{301}$ may be a group represented by one of Formulae 5-2, 5-3, or 6-8 to 6-33.

In one or more embodiments, $R_{301}$ may be a cyano group or a group represented by one of Formulae 7-1 to 7-18, and at least one of $Ar_{402}$(s) in the number of xd11 may be a group represented by one of Formulae 7-1 to 7-18, but embodiments of the present disclosure are not limited thereto:

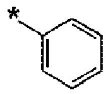
7-1
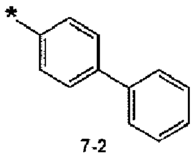
7-2
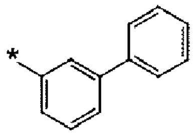
7-3
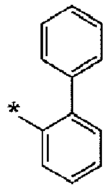
7-4
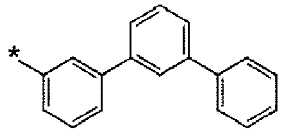
7-5

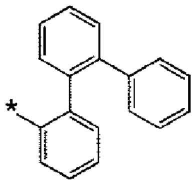
7-6
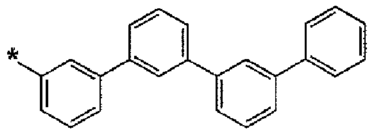
7-7
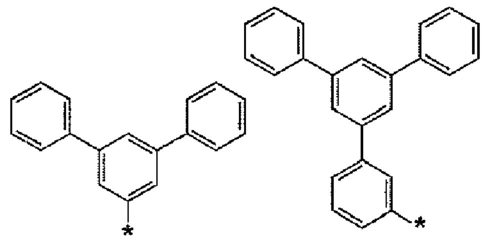
7-8
7-9

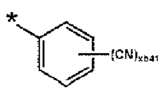
7-10
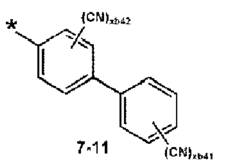
7-11
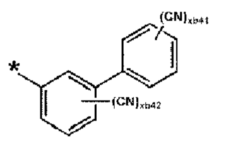
7-12
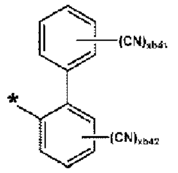
7-13

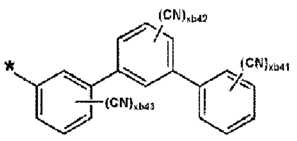
7-14
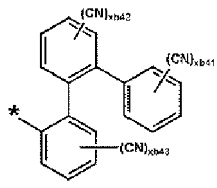
7-15
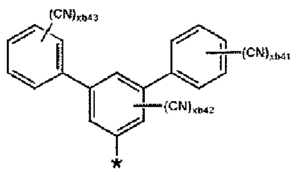
7-16

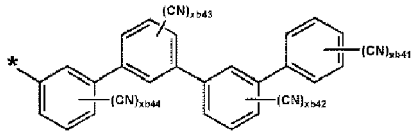

7-17

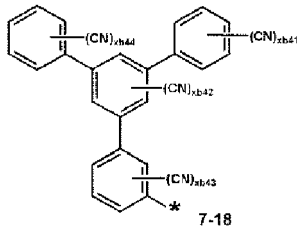

7-18

wherein, in Formulae 7-1 to 7-18,

xb41 to xb44 may each be 0, 1, or 2, wherein xb41 in Formula 7-10 is not 0, the sum of xb41 and xb42 in Formulae 7-11 to 7-13 is not 0, the sum of xb41, xb42, and xb43 in Formulae 7-14 to 7-16 is not 0, the sum of xb41, xb42, xb43, and xb44 in Formulae 7-17 and 7-18 is not 0, and * indicates a binding site to a neighboring atom.

Two or more $Ar_{301}$(s) in Formula E-1 may be identical to or different from each other, two or more of $L_{301}$(s) may be identical to or different from each other, two or more of $L_{401}$(s) in Formula H-1 may be identical to or different from each other, and two or more of $Ar_{402}$(s) in Formula H-1 may be identical to or different from each other.
In one or more embodiments, the electron-transporting host includes i) at least one of a cyano group, a pyrimidine group, a pyrazine group, and a triazine group and ii) a triphenylene group, and the hole-transporting host may include a carbazole group.
In one or more embodiments, the electron-transporting host may include at least one cyano group.
The electron-transporting host may be, for example, a compound from groups HE1 to HE7, but embodiments of the present disclosure are not limited thereto:

Group HE1

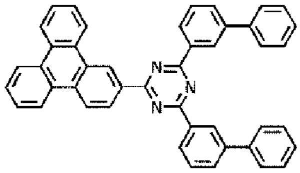
H-E1

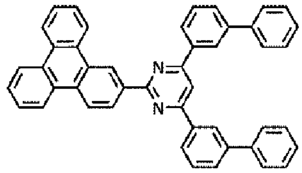
H-E2

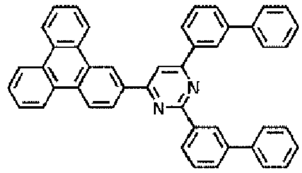
H-E3

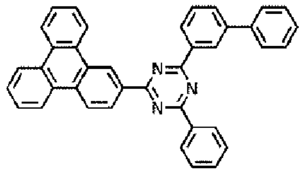
H-E4

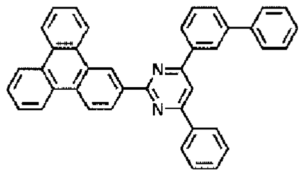
H-E5

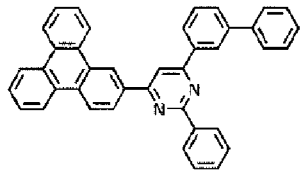
H-E6

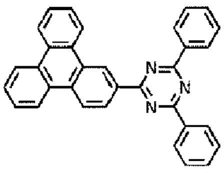
H-E7

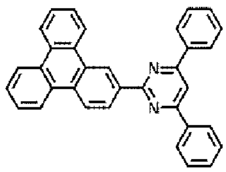
H-E8

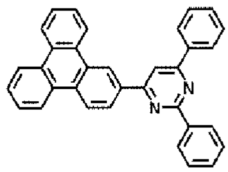
H-E9

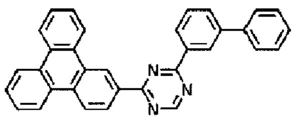
H-E10

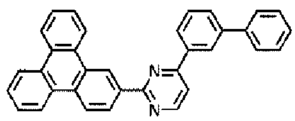
H-E11

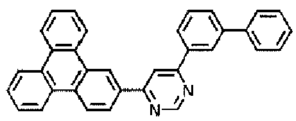
H-E12

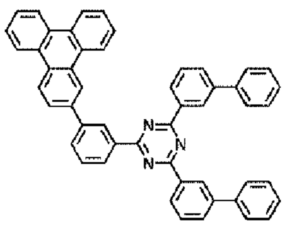
H-E13

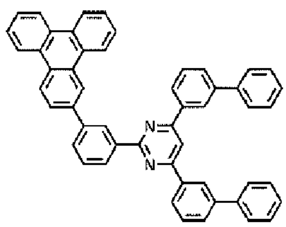
H-E14

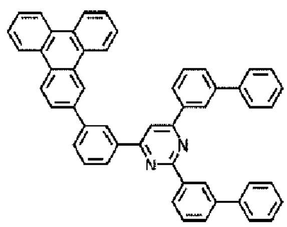
H-E15

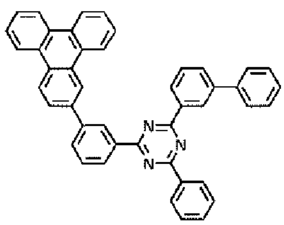
H-E16

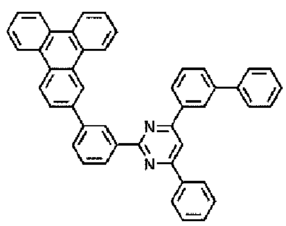
H-E17

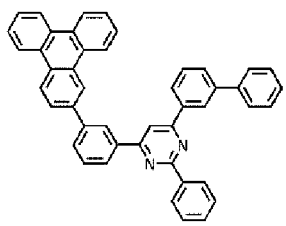
H-E18

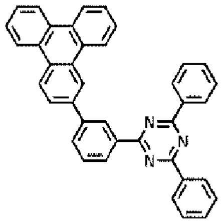

H-E19

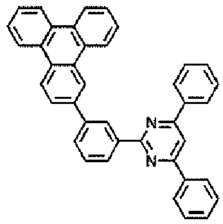

H-E20

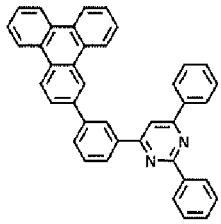

H-E21

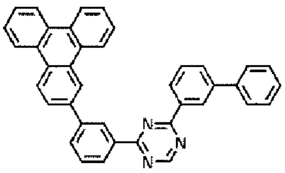

H-E22

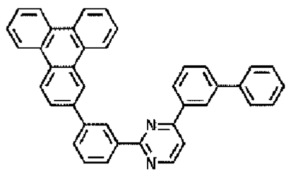

H-E23

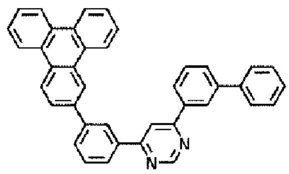

H-E24

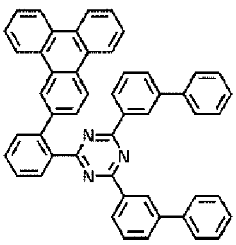

H-E25

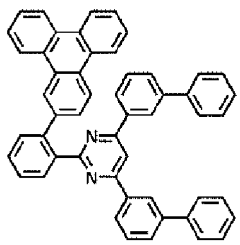

H-E26

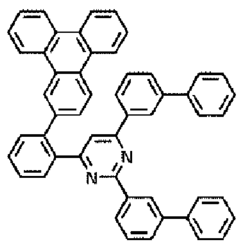

H-E27

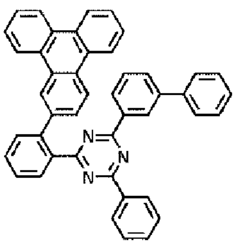

H-E28

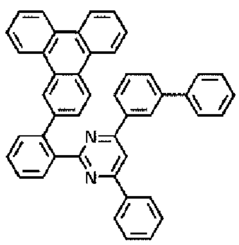

H-E29

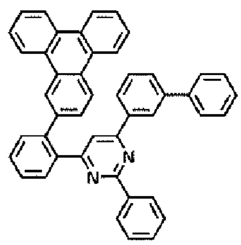

H-E30

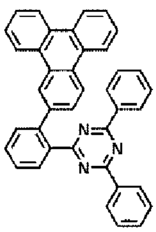

H-E31

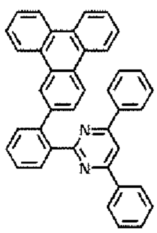

H-E32

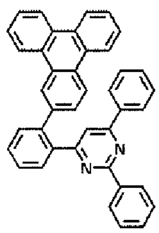

H-E33

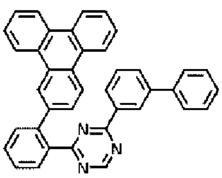

H-E34

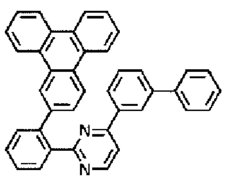

H-E35

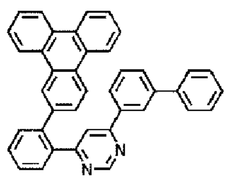

H-E36

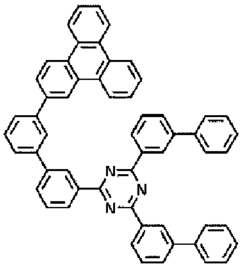

H-E37

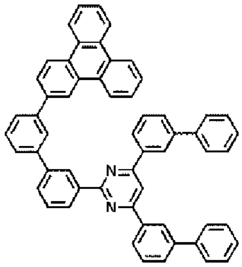

H-E38

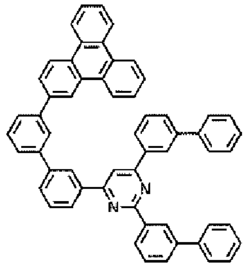

H-E39

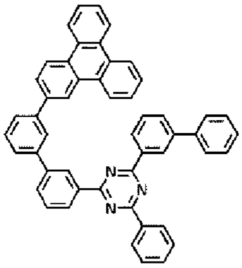

H-E40

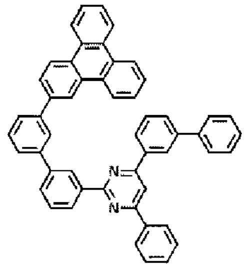

H-E41

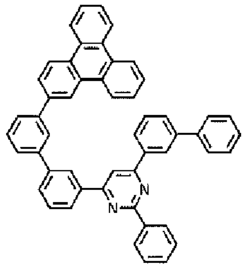

H-E42

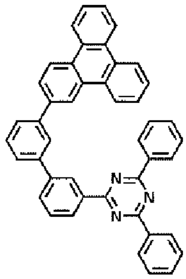

H-E43

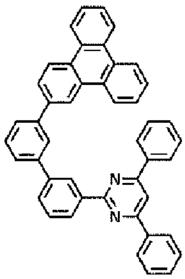

H-E44

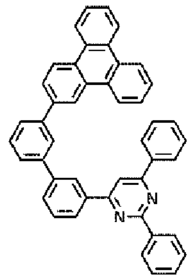

H-E45

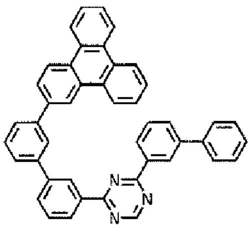

H-E46

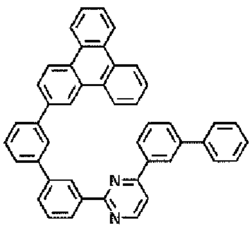

H-E47

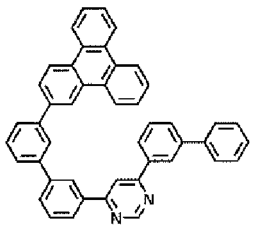

H-E48

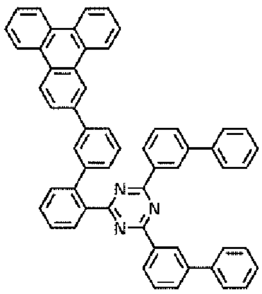
H-E49

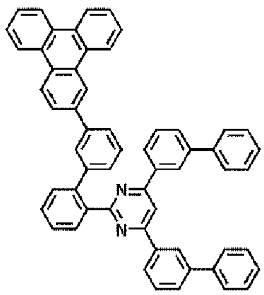
H-E50

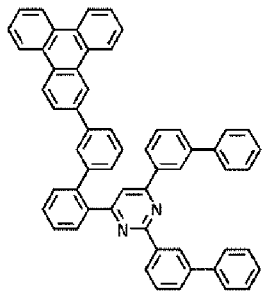
H-E51

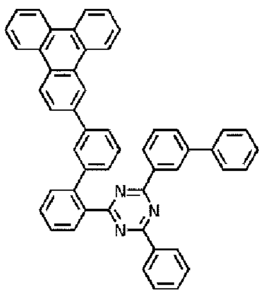
H-E52

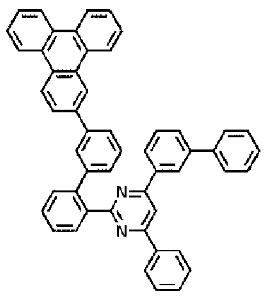
H-E53

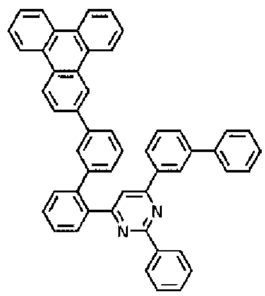
H-E54

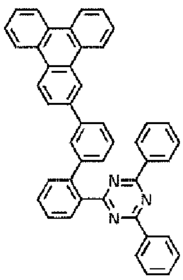
H-E55

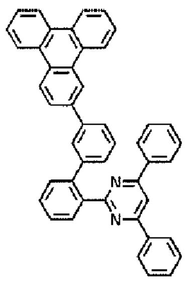
H-E56

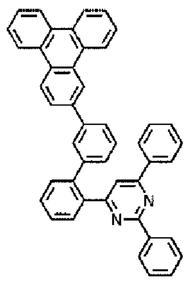
H-E57

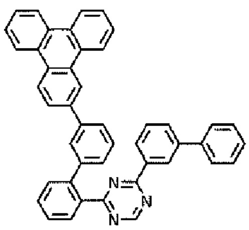
H-E58

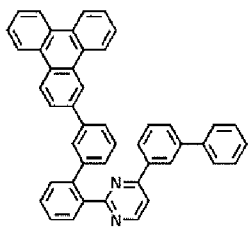
H-E59

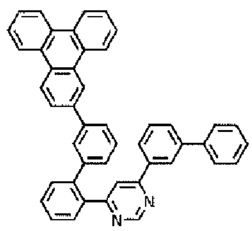
H-E60

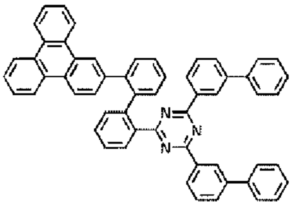

H-E61

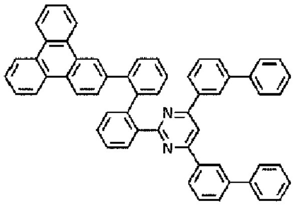

H-E62

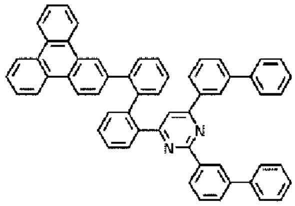

H-E63

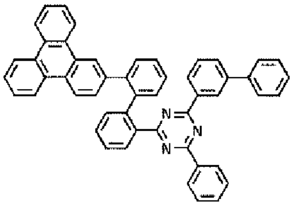

H-E64

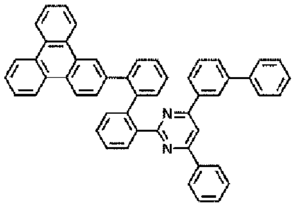

H-E65

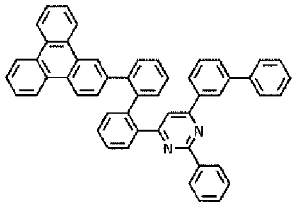

H-E66

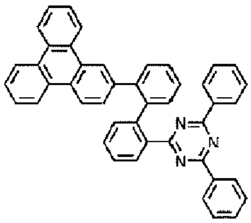

H-E67

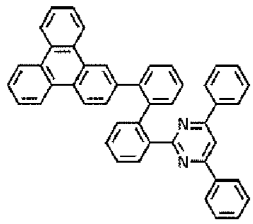

H-E68

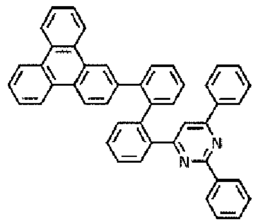

H-E69

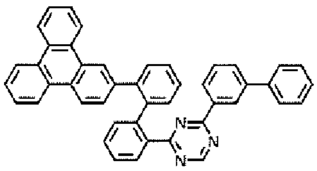

H-E70

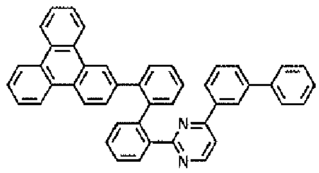

H-E71

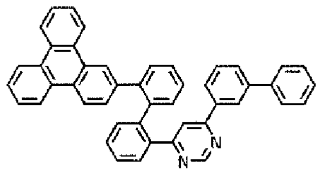

H-E72

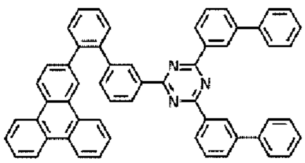

H-E73

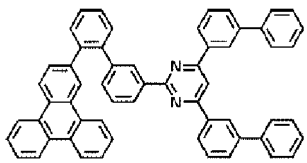

H-E74

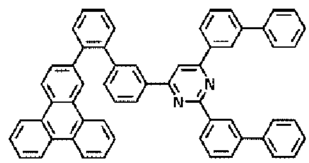

H-E75

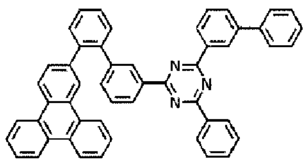

H-E76

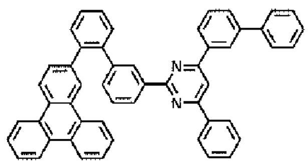

H-E77

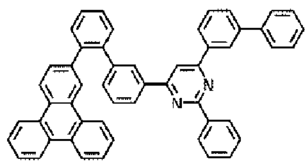

H-E78

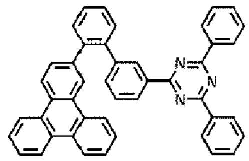
H-E79

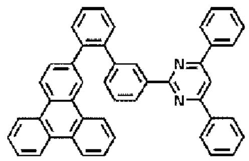
H-E80

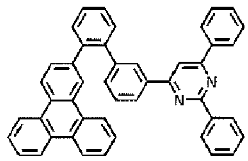
H-E81

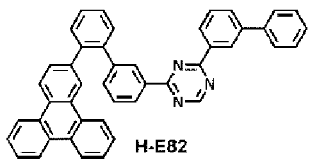
H-E82

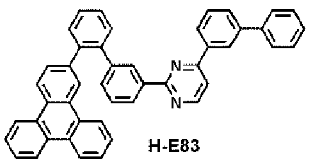
H-E83

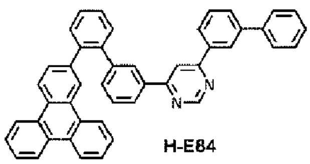
H-E84

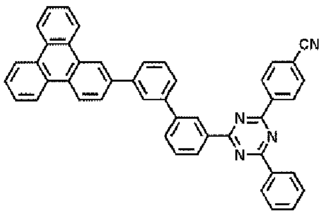
H-E(1)

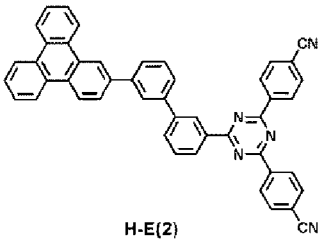
H-E(2)

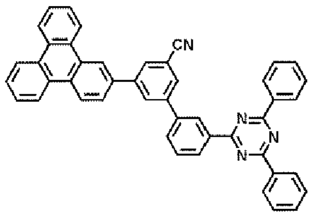
H-E(3)

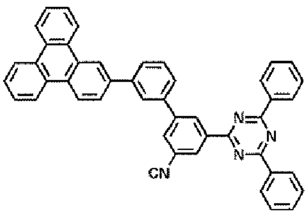
H-E(4)

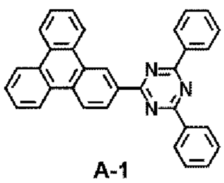
A-1

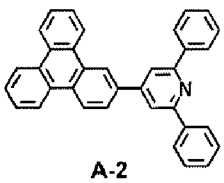
A-2

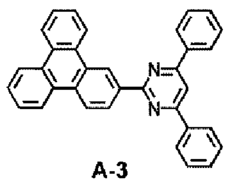
A-3

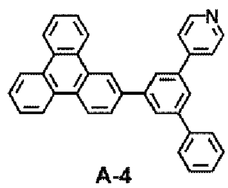
A-4

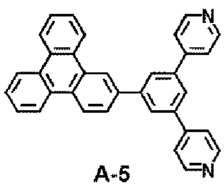
A-5

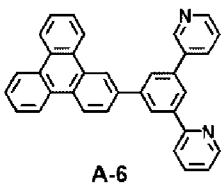
A-6

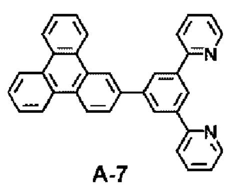
A-7

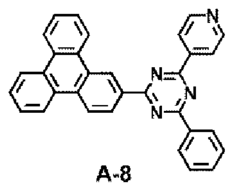
A-8

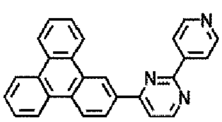
A-9

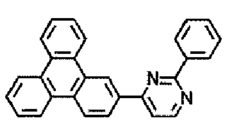
A-10

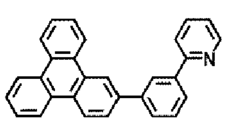
A-11

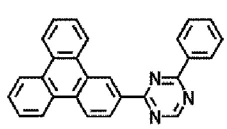
A-12

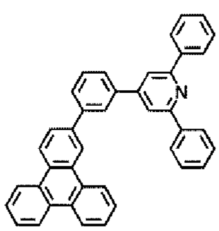
A-13

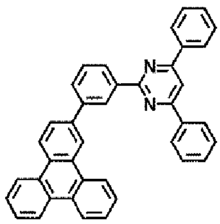
A-14

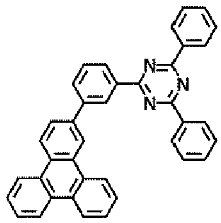
A-15

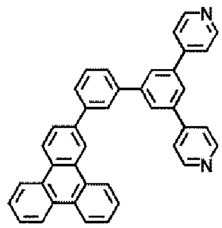
A-16

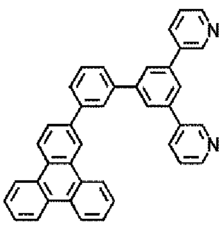
A-17

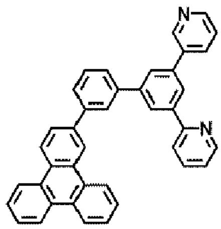
A-18

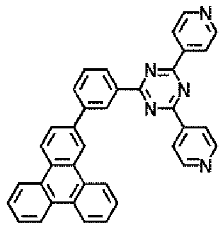
A-19

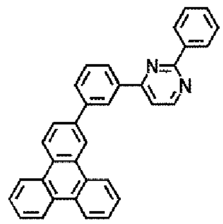
A-20

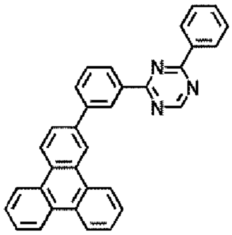
A-21

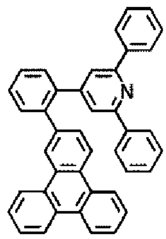
A-22

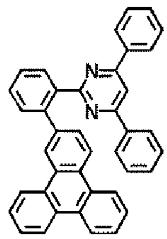
A-23

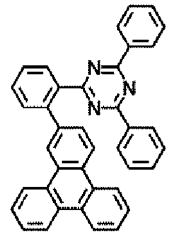
A-24

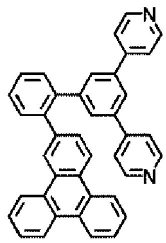
A-25

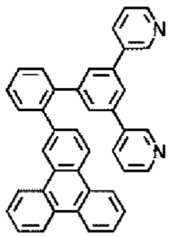
A-26

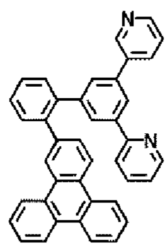
A-27

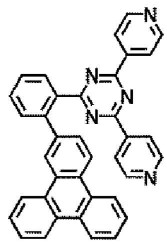
A-28

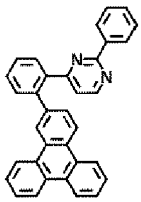

A-29

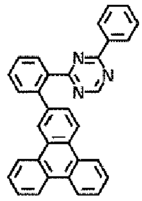

A-30

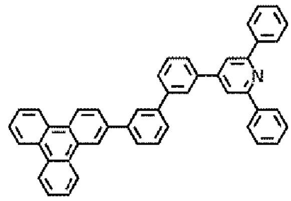

A-31

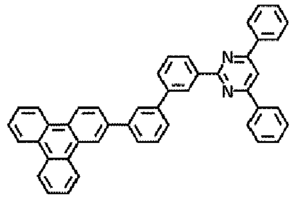

A-32

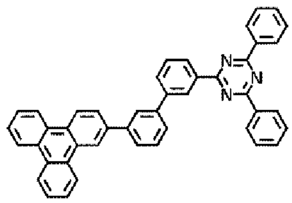

A-33

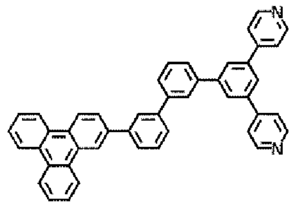

A-34

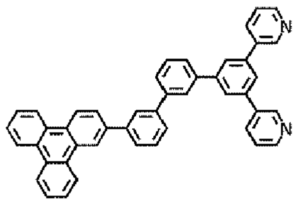

A-35

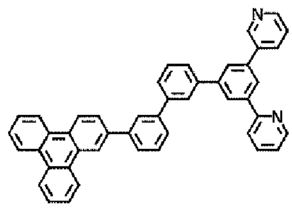

A-36

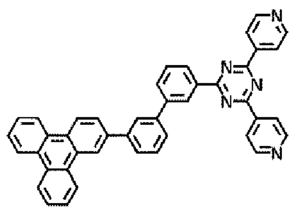

A-37

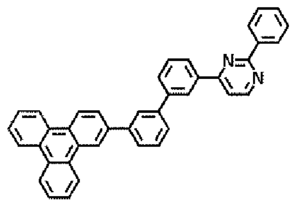

A-38

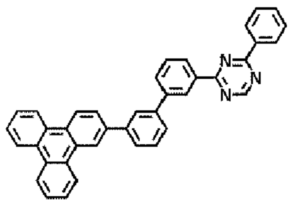

A-39

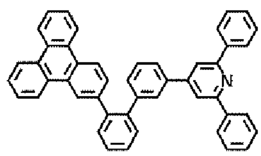

A-40

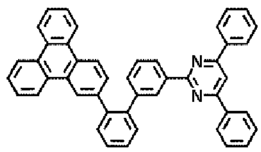

A-41

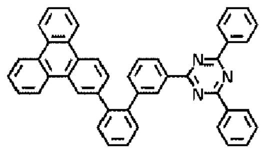

A-42

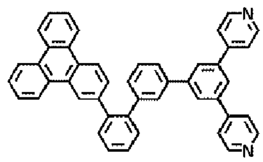

A-43

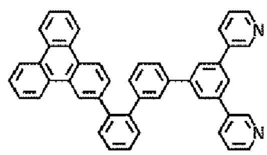

A-44

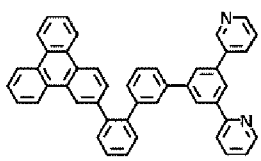

A-45

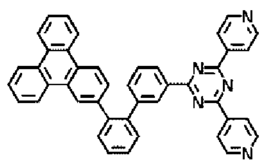

A-46

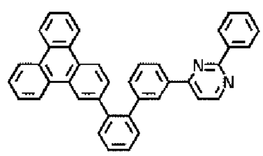

A-47

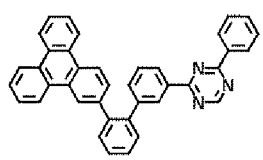

A-48

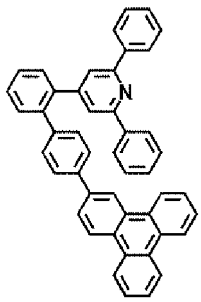

A-49

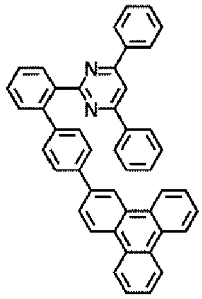

A-50

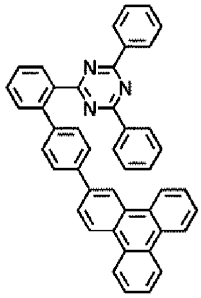

A-51

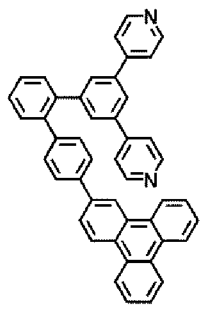

A-52

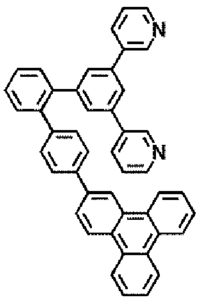

A-53

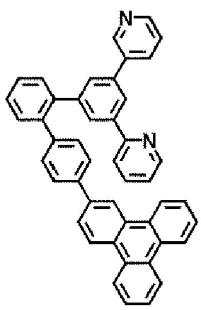

A-54

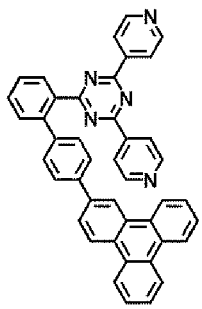

A-55

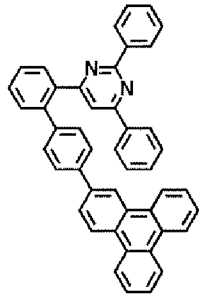

A-56

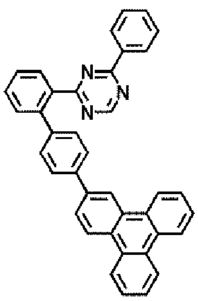

A-57

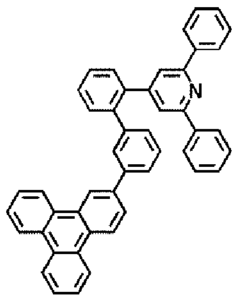

A-58

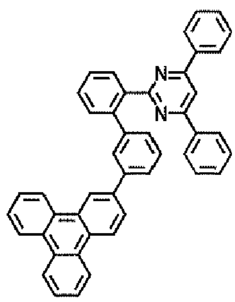

A-59

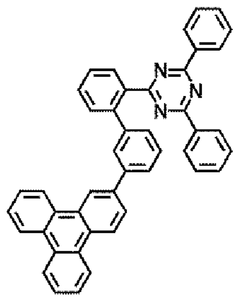

A-60

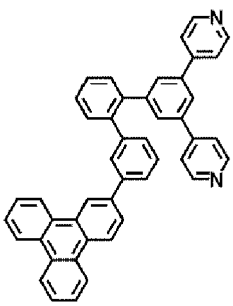

A-61

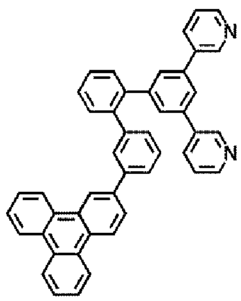

A-62

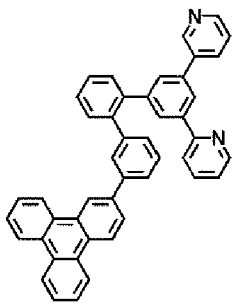

A-63

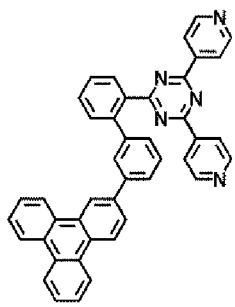

A-64

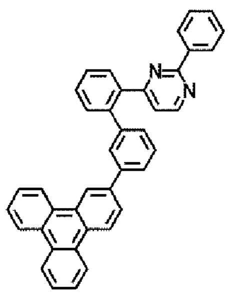

**A-65**

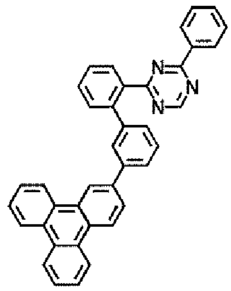

**A-66**

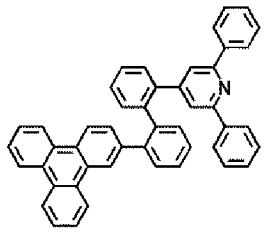

**A-67**

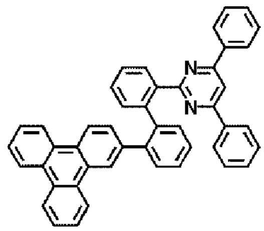

**A-68**

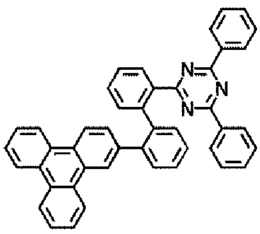

**A-69**

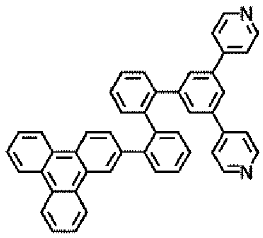

**A-70**

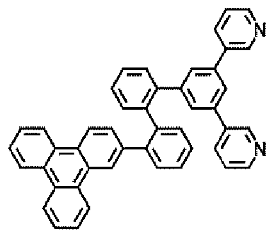

**A-71**

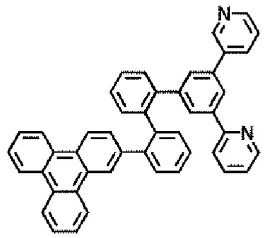

**A-72**

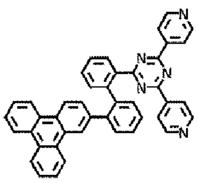

A-73

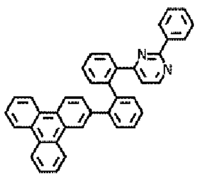

A-74

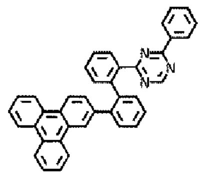

A-75

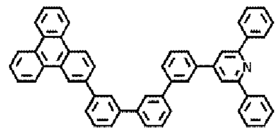

A-76

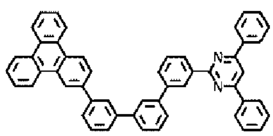

A-77

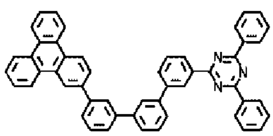

A-78

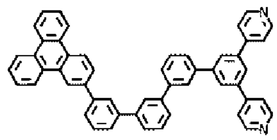

A-79

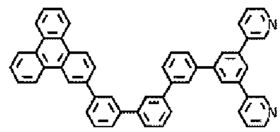

A-80

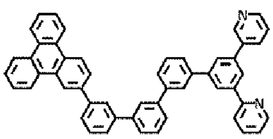

A-81

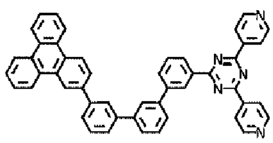

A-82

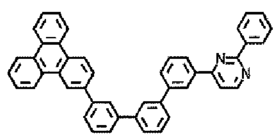

A-83

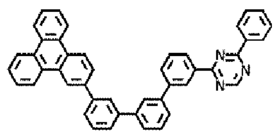

A-84

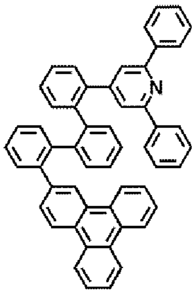
A-85

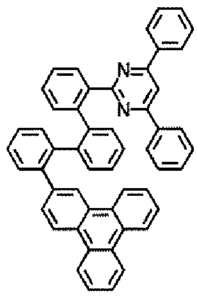
A-86

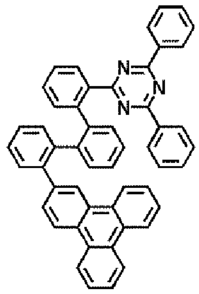
A-87

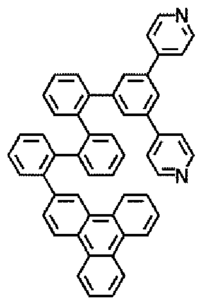
A-88

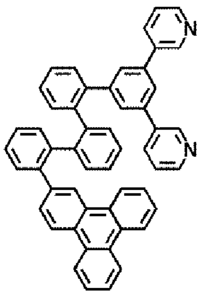
A-89

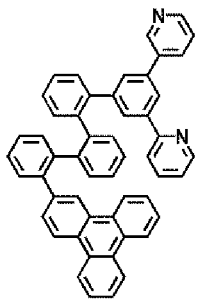
A-90

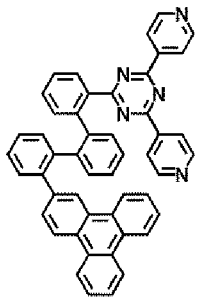
A-91

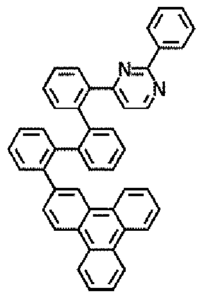
A-92

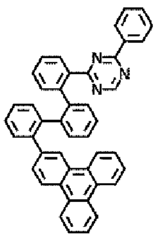
A-93

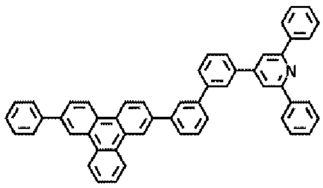
A-94

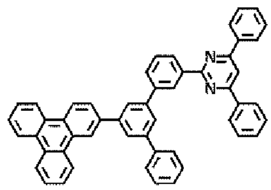
A-95

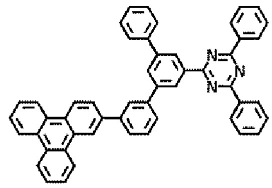
A-96

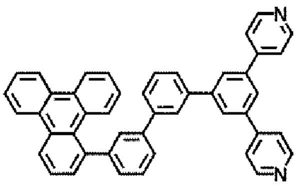

**A-97**

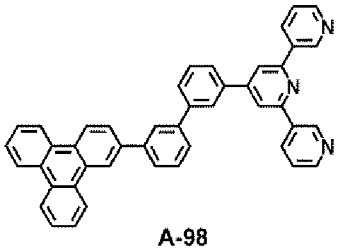

**A-98**

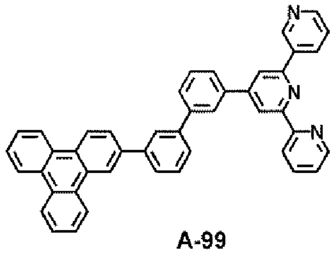

**A-99**

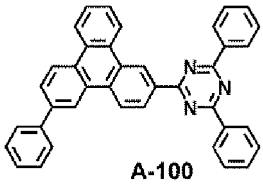

**A-100**

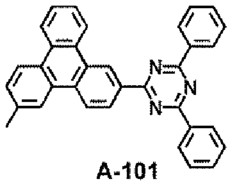

**A-101**

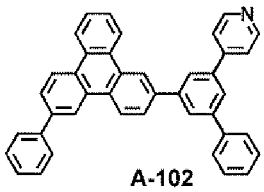

**A-102**

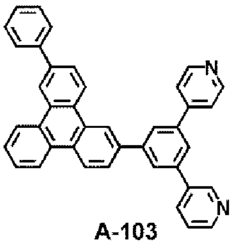

**A-103**

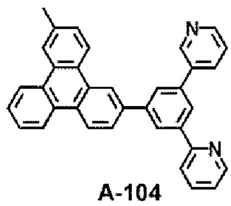

**A-104**

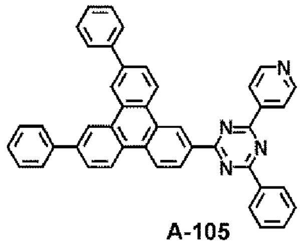

**A-105**

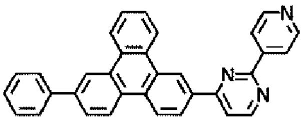

**A-106**

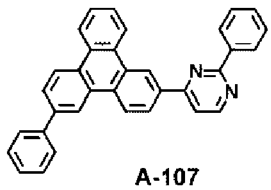

**A-107**

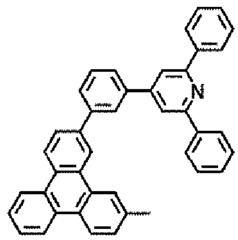

**A-108**

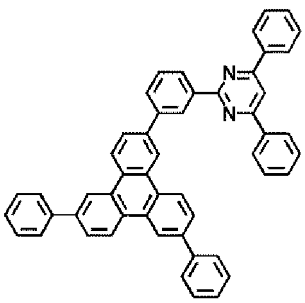

**A-109**

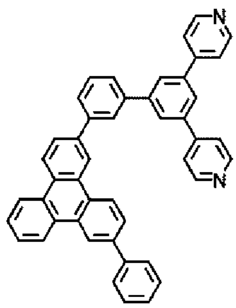

**A-110**

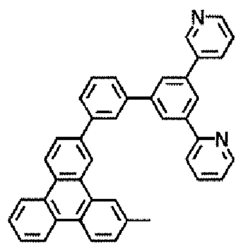

**A-111**

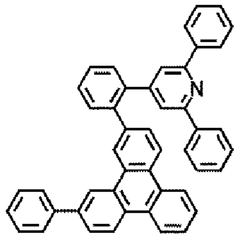
A-112

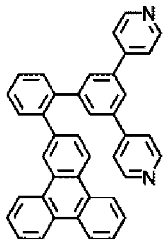
A-113

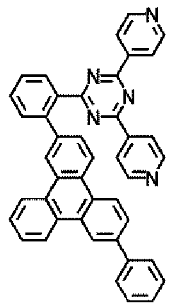
A-114

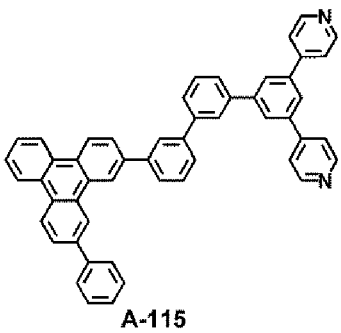
A-115

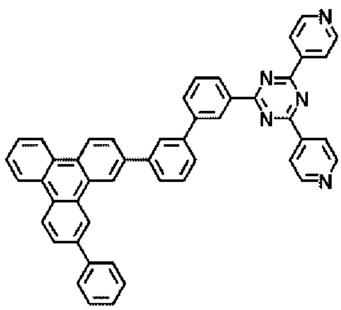
A-116

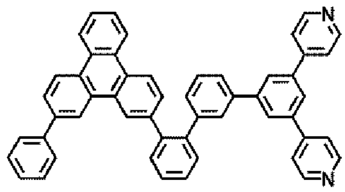
A-117

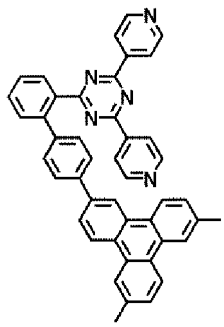
A-118

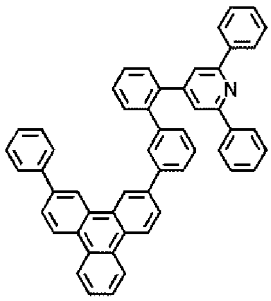
A-119

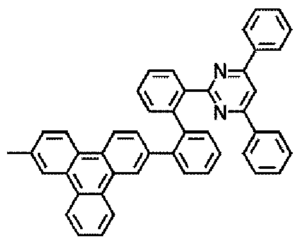
A-120

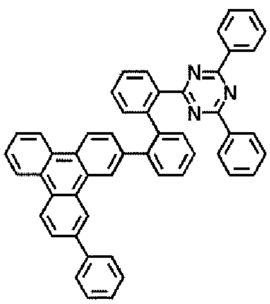
A-121

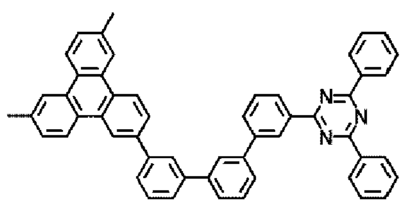
A-122

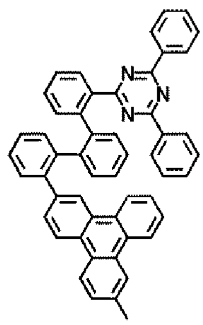
A-123

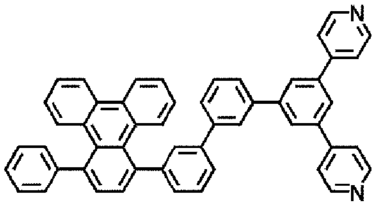

A-124

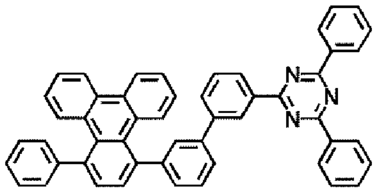

A-125

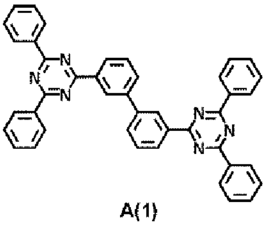

A(1)

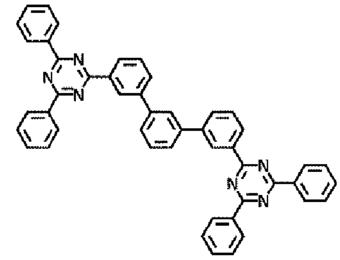

A(2)

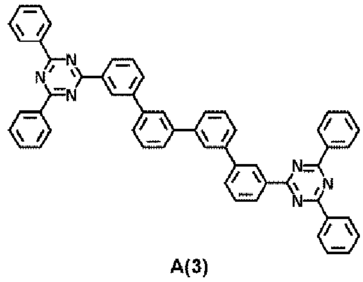

A(3)

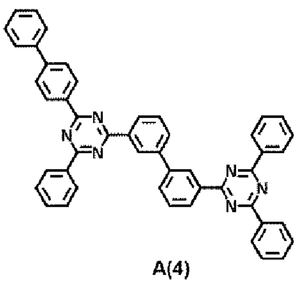

A(4)

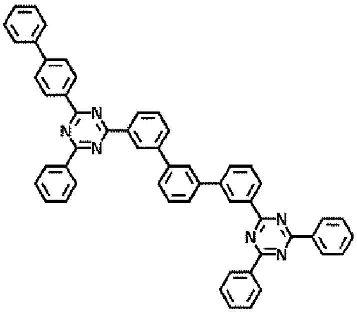

A(5)

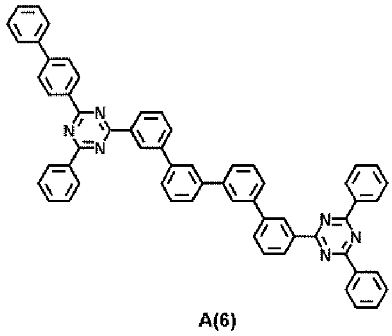

A(6)

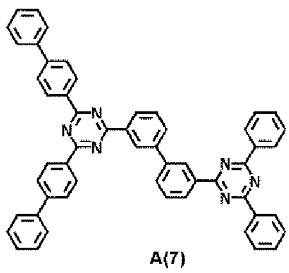

A(7)

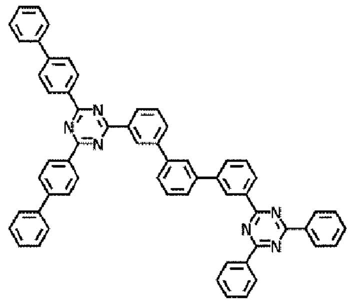

A(8)

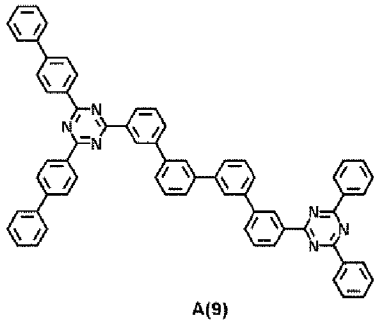

A(9)

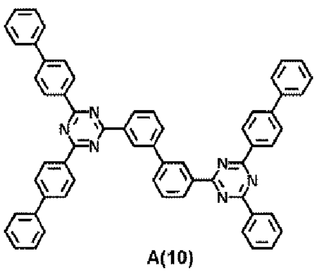

A(10)

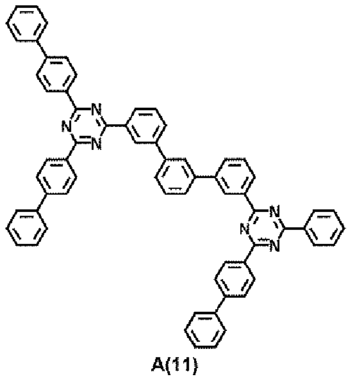

A(11)

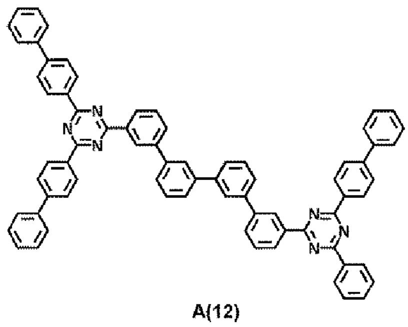

A(12)

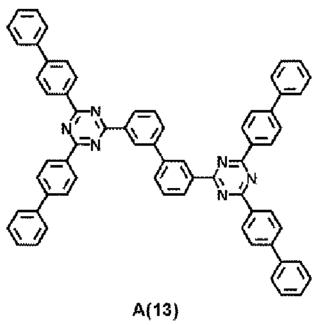

A(13)

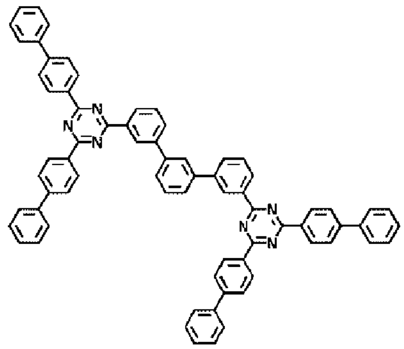

A(14)

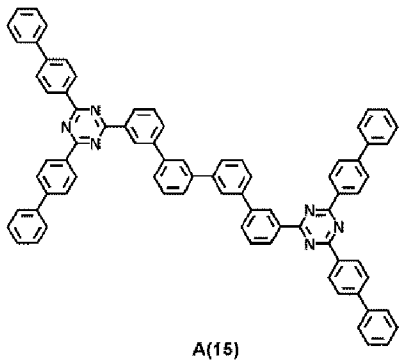

A(15)

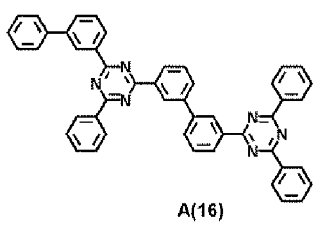

A(16)

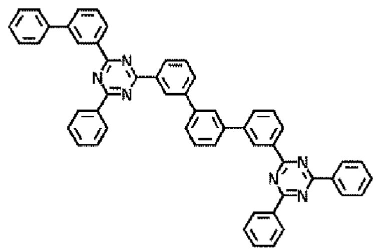

A(17)

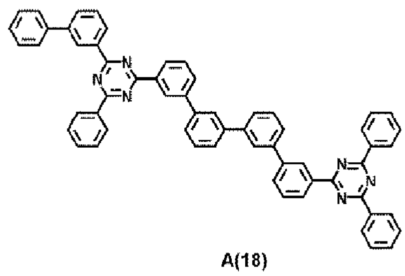

A(18)

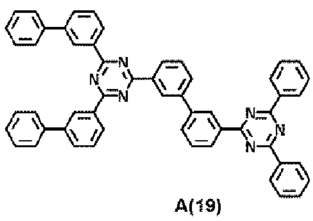

A(19)

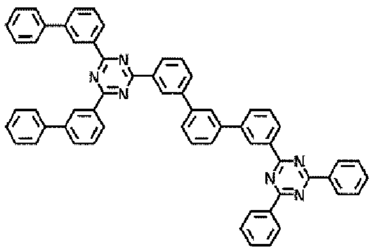

A(20)

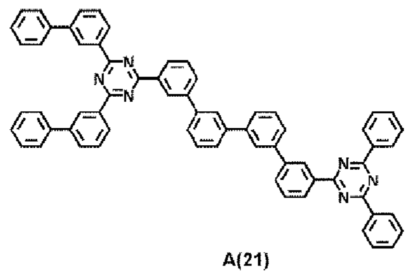

A(21)

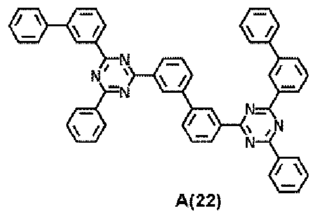

A(22)

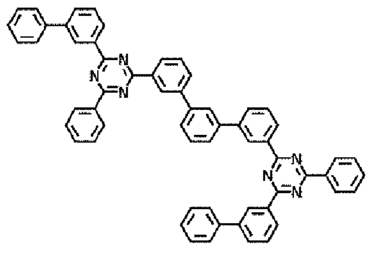

A(23)

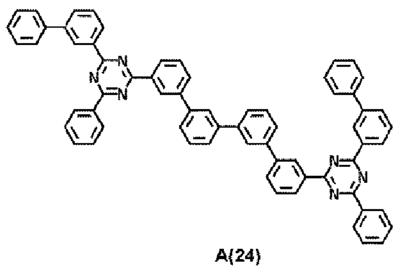

A(24)

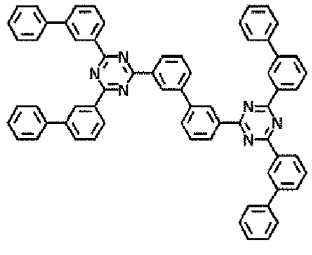

A(25)

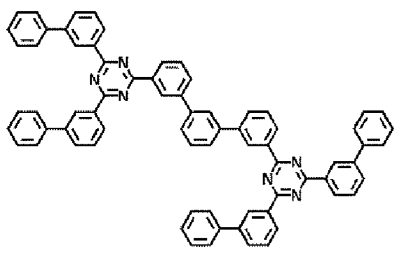

A(26)

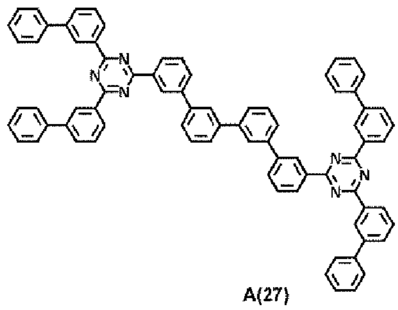

A(27)

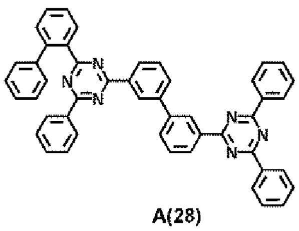

A(28)

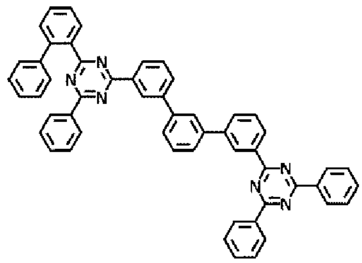

A(29)

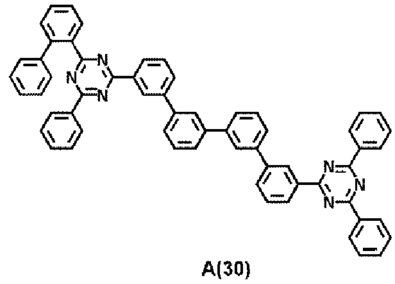

A(30)

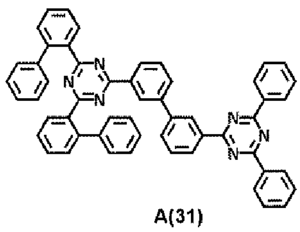

A(31)

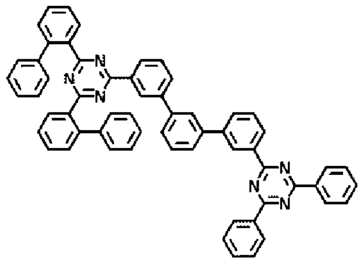

A(32)

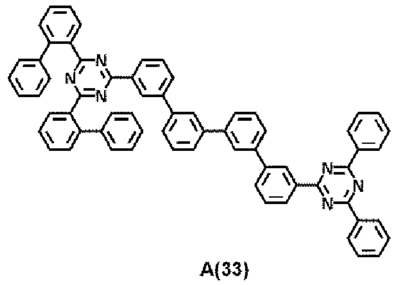

A(33)

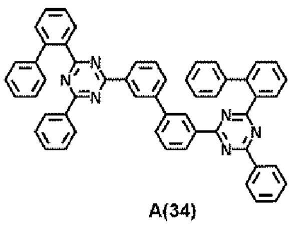

A(34)

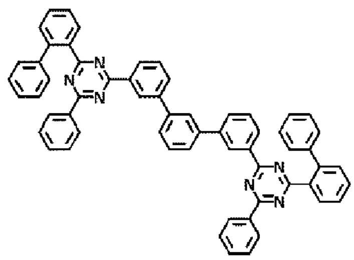

A(35)

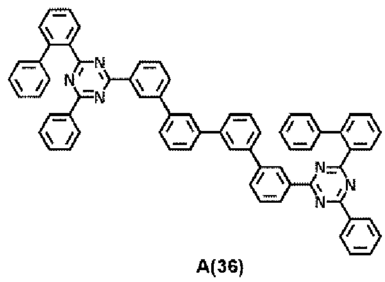

A(36)

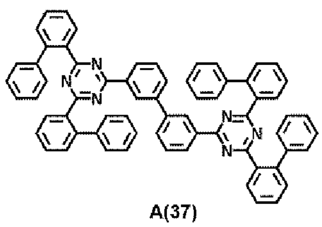

A(37)

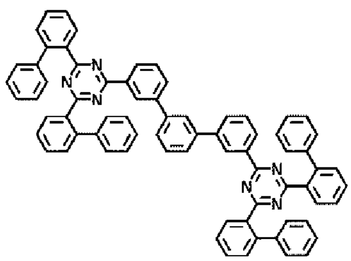

A(38)

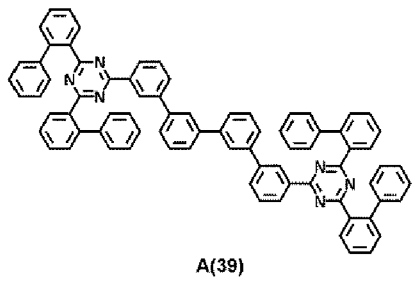

A(39)

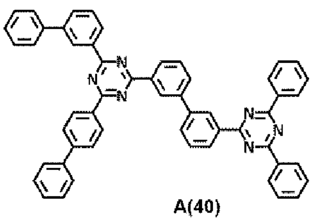

A(40)

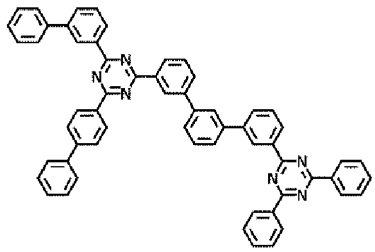

A(41)

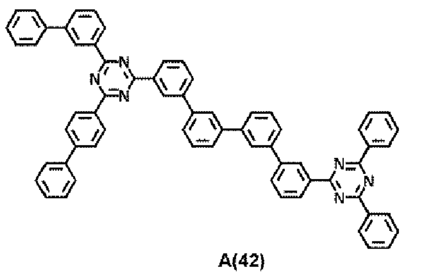

A(42)

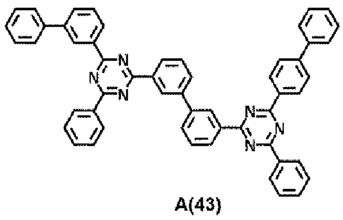

A(43)

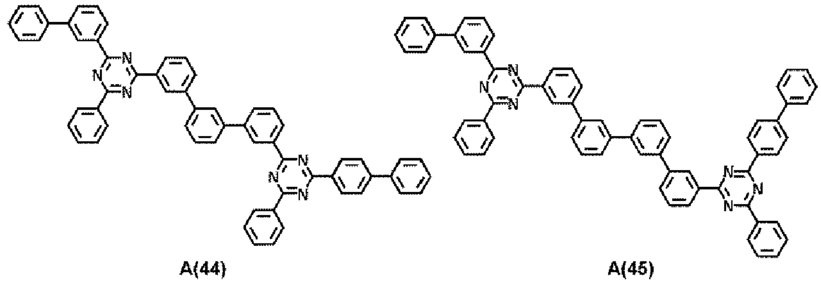

A(44)

A(45)

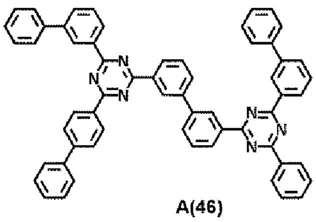

A(46)

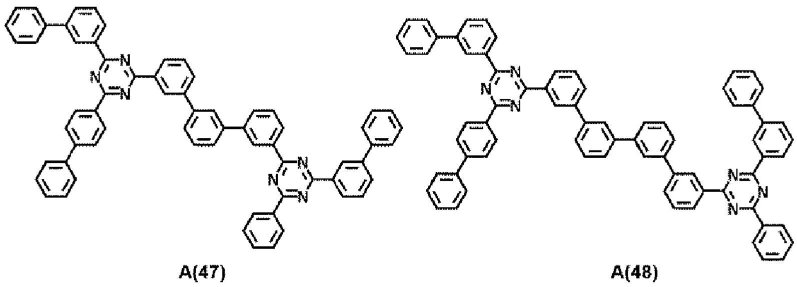

A(47)

A(48)

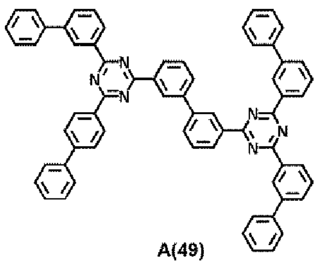

A(49)

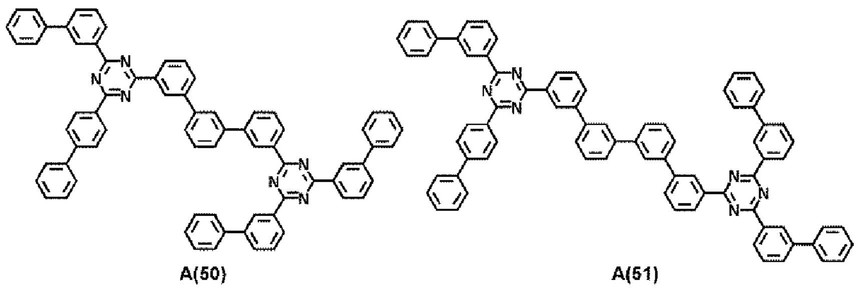

A(50)

A(51)

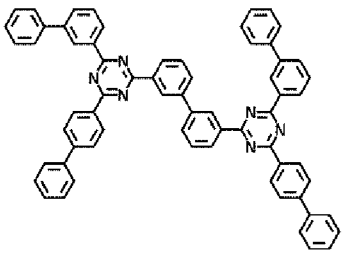
A(52)

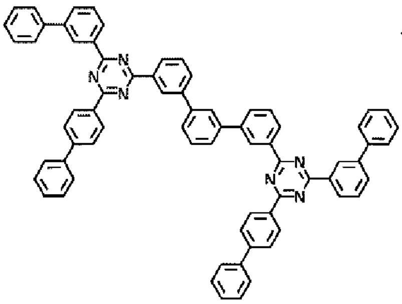
A(53)

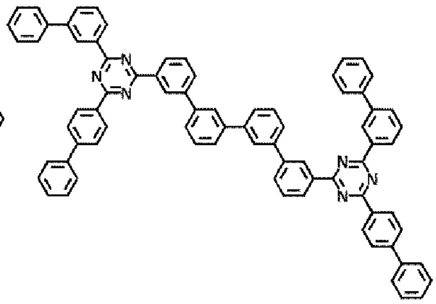
A(54)

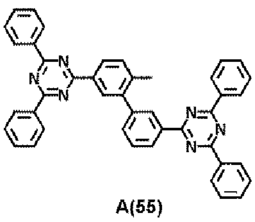
A(55)

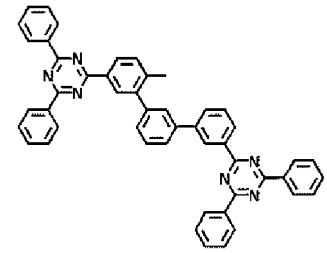
A(56)

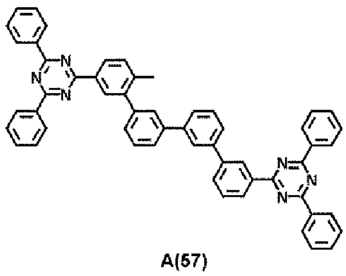
A(57)

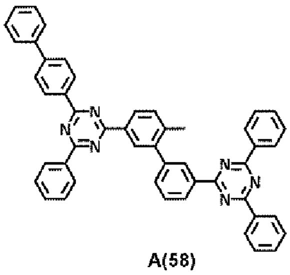
A(58)

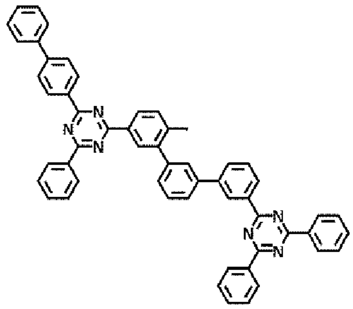
A(59)

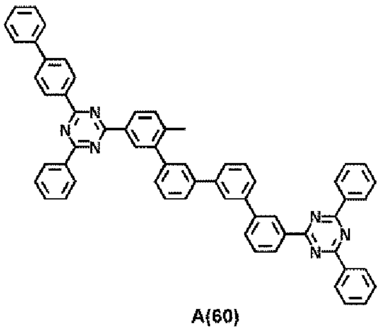
A(60)

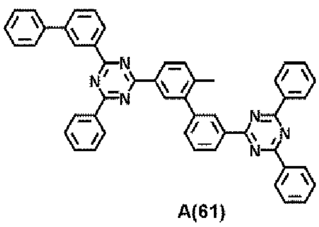
A(61)

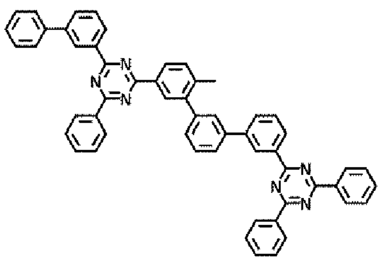
A(62)

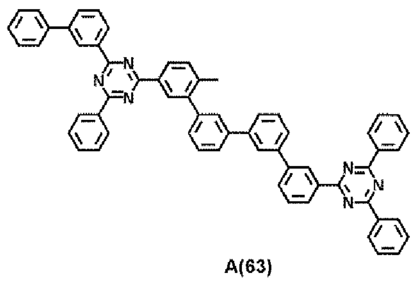
A(63)

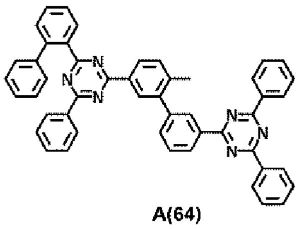
A(64)

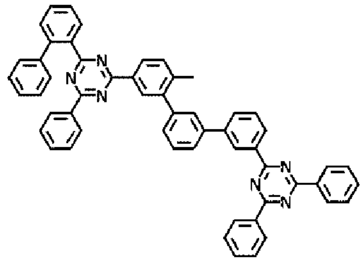
A(65)

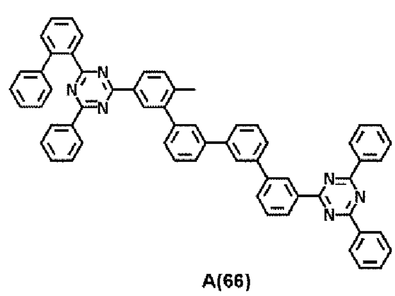
A(66)

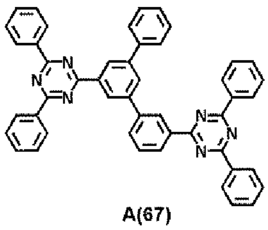
A(67)

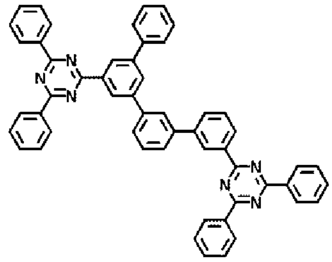
A(68)

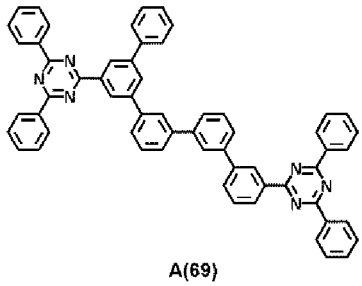
A(69)

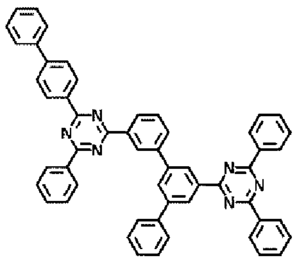
A(70)

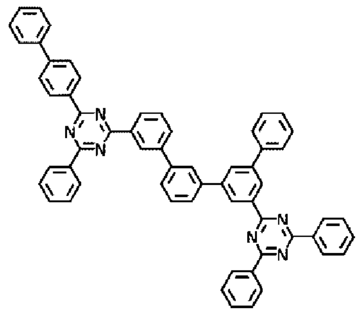
A(71)

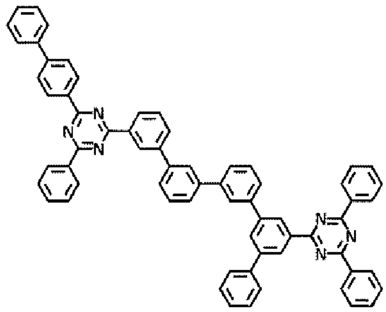
A(72)

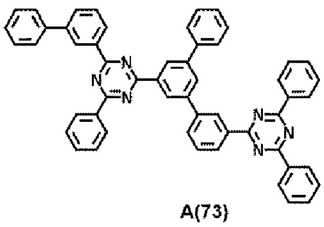
A(73)

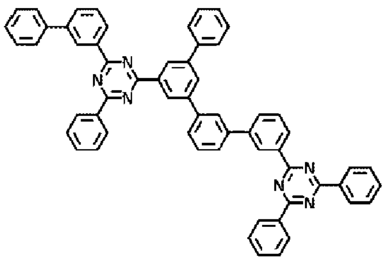
A(74)

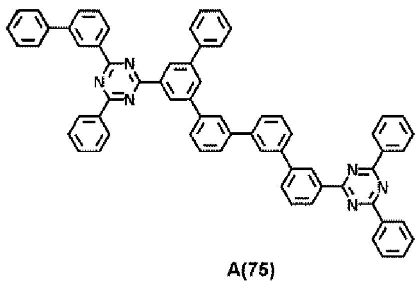
A(75)

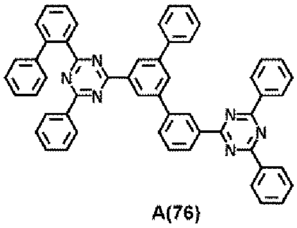
A(76)

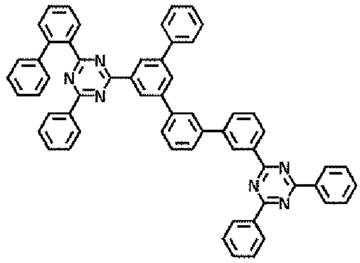
A(77)

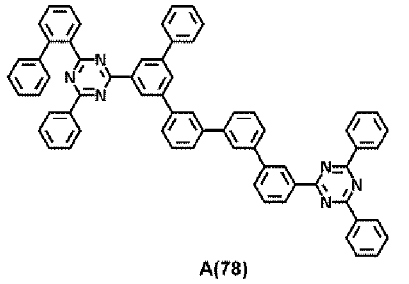
A(78)

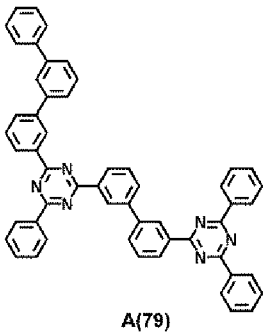
A(79)

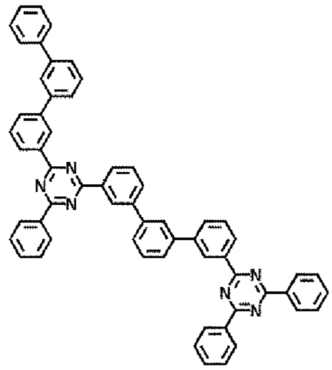
A(80)

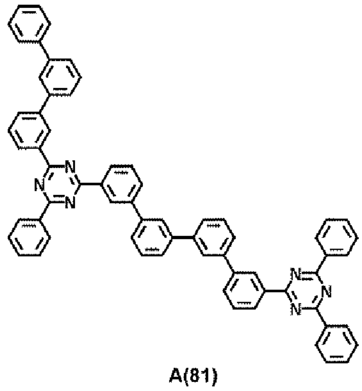
A(81)

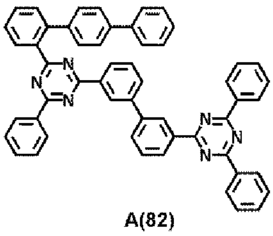
A(82)

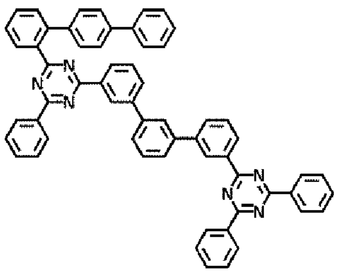
A(83)

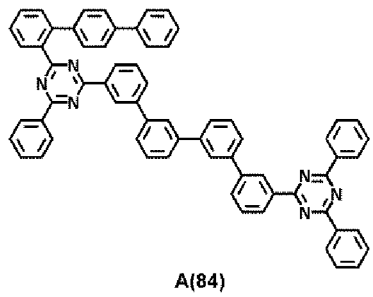
A(84)

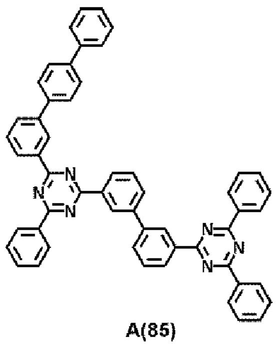
A(85)

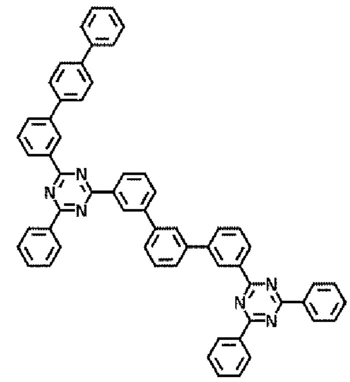
A(86)

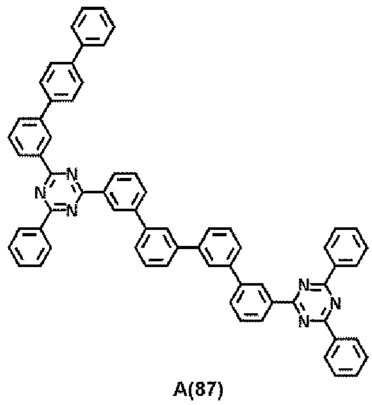
A(87)

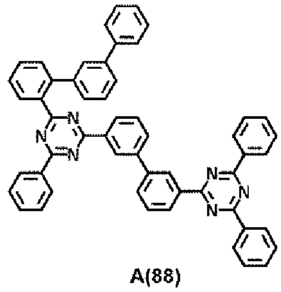

A(88)

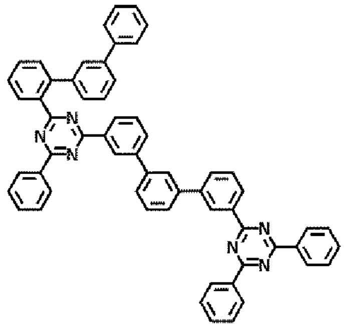

A(89)

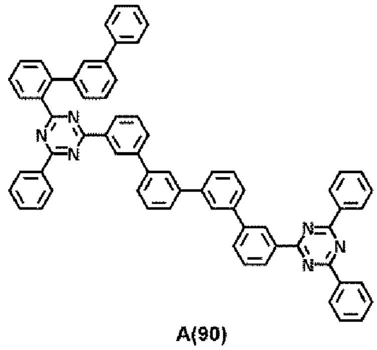

A(90)

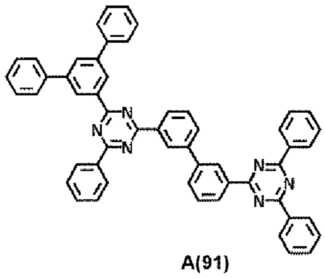

A(91)

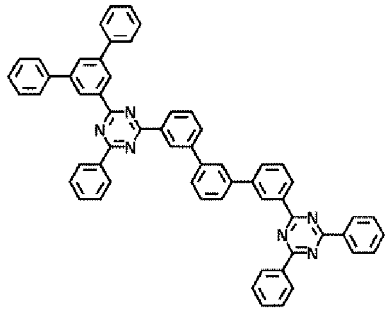

A(92)

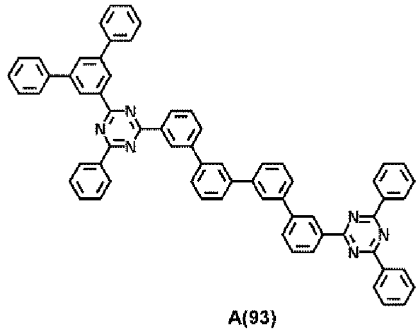

A(93)

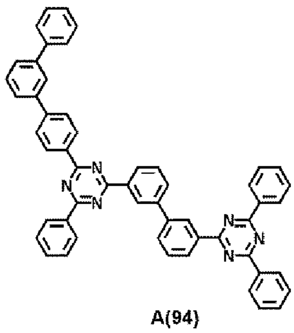

A(94)

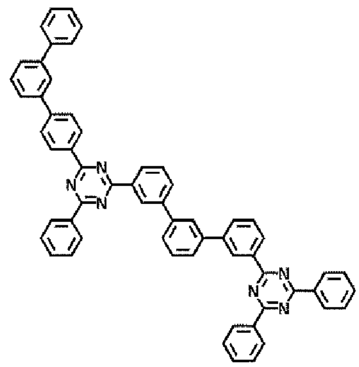

A(95)

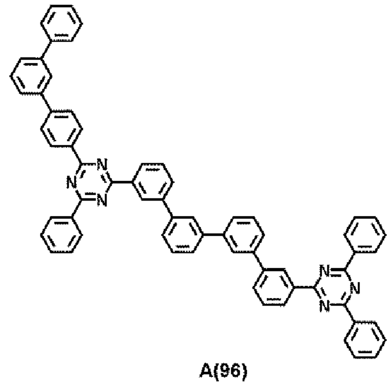

A(96)

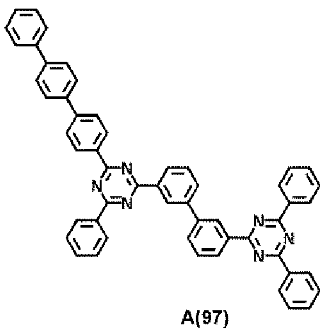

A(97)

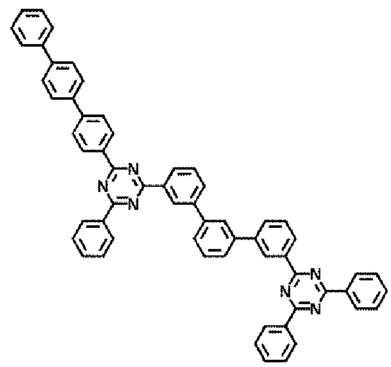

A(98)

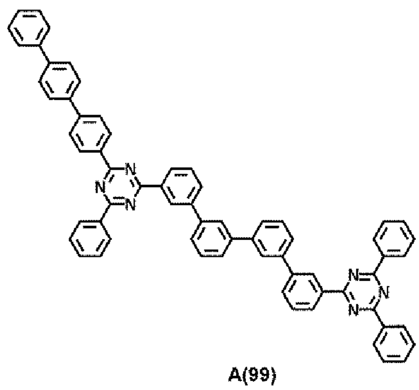

A(99)

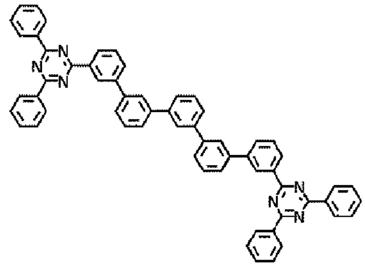
A(100)

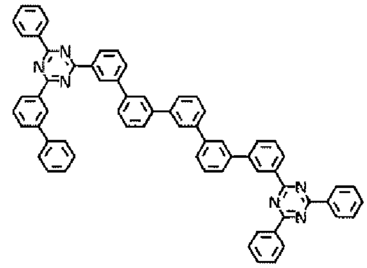
A(101)

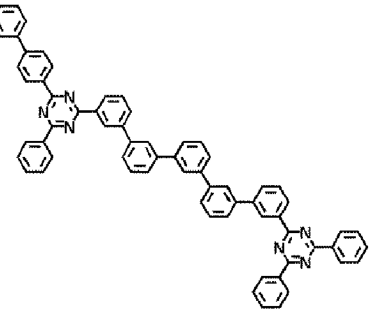
A(102)

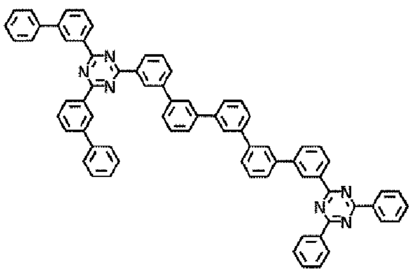
A(103)

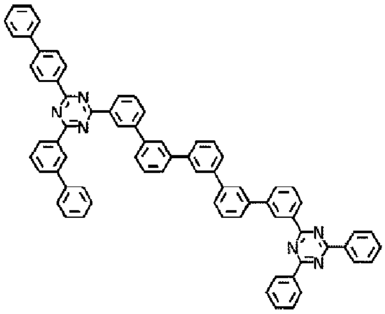
A(104)

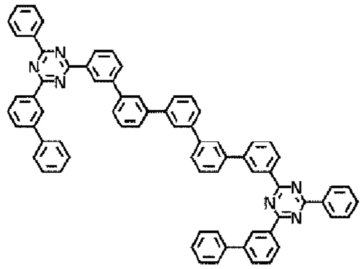
A(105)

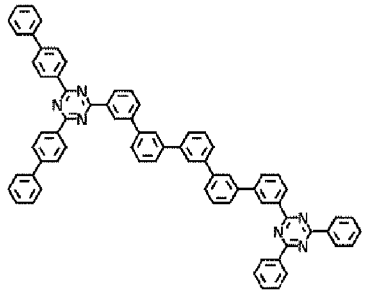
A(106)

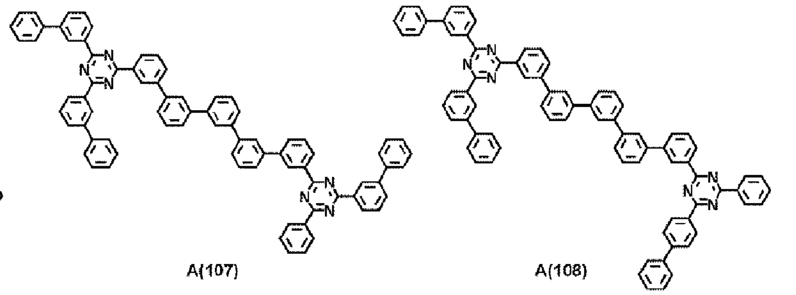
A(107)

A(108)

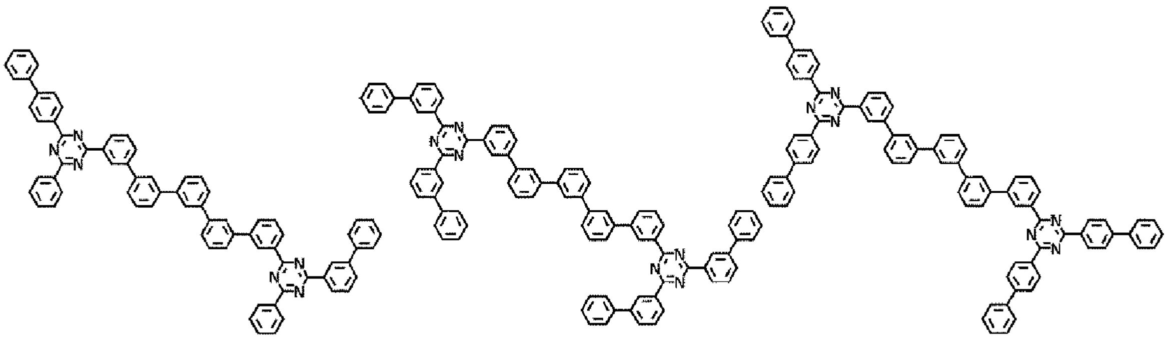
A(109)

A(110)

A(111)

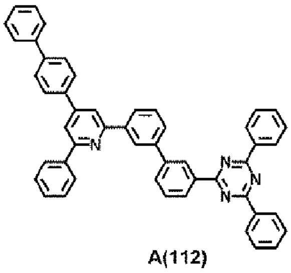
A(112)

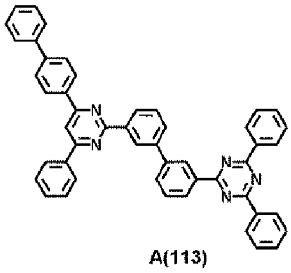
A(113)

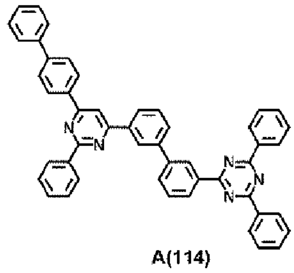
A(114)

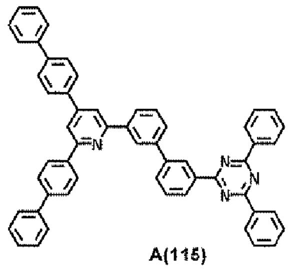
A(115)

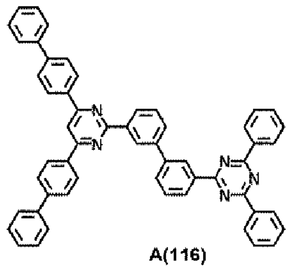
A(116)

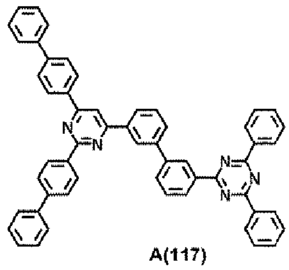
A(117)

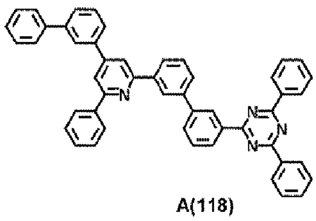
A(118)

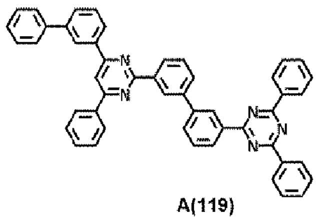
A(119)

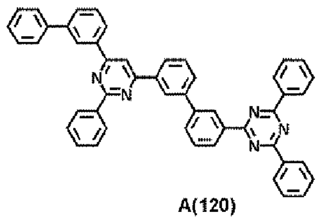
A(120)

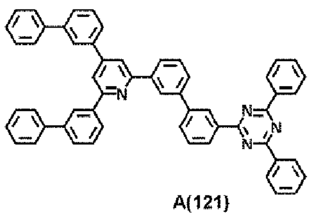
A(121)

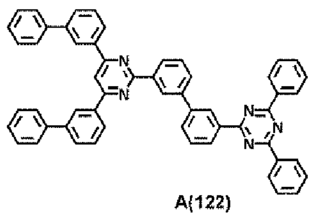
A(122)

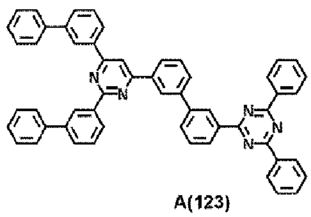
A(123)

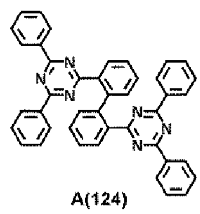
A(124)

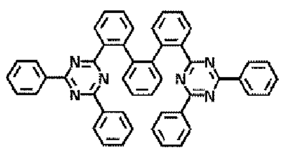
A(125)

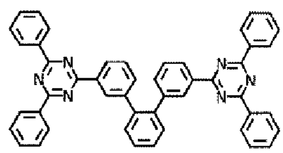
A(126)

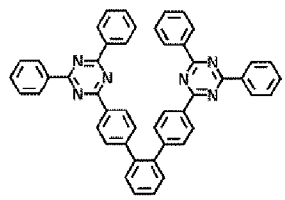
A(127)

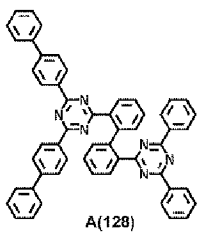
A(128)

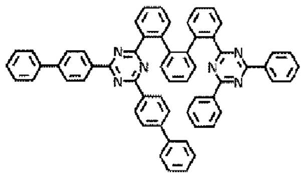
A(129)

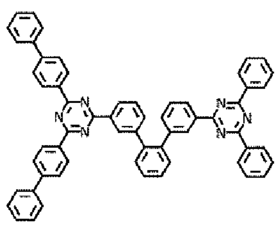
A(130)

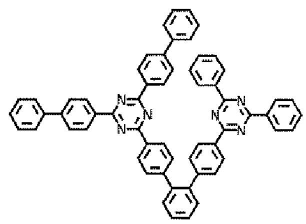
A(131)

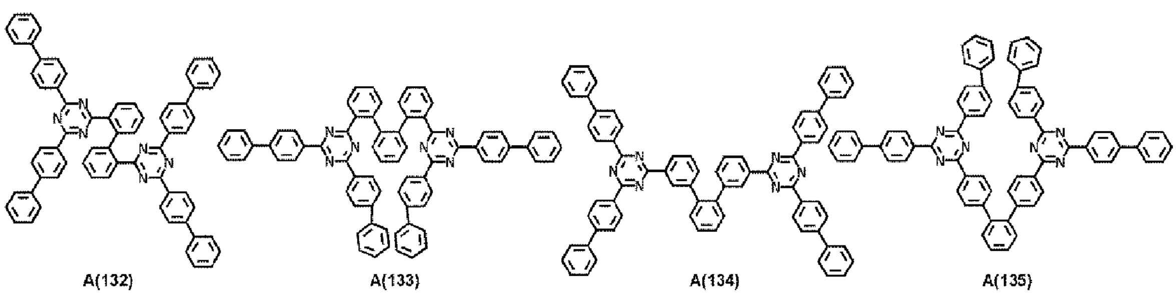
A(132) A(133) A(134) A(135)

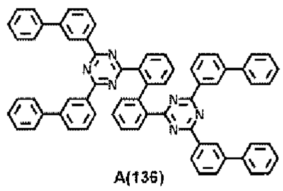
A(136)

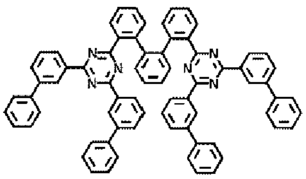
A(137)

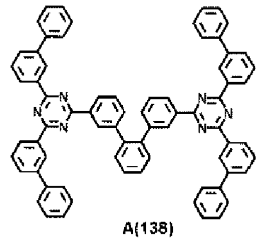
A(138)

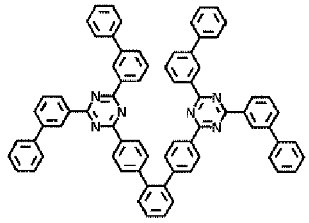
A(139)

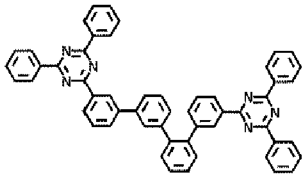
A(140)

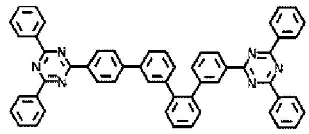
A(141)

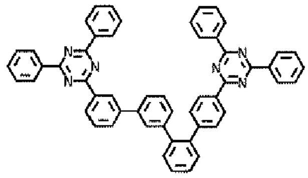
A(142)

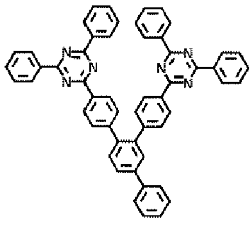
A(143)

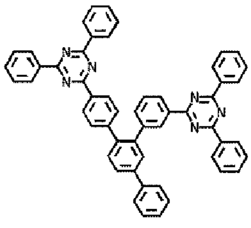
A(144)

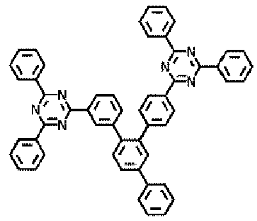
A(145)

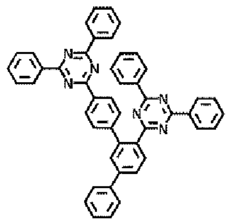
A(146)

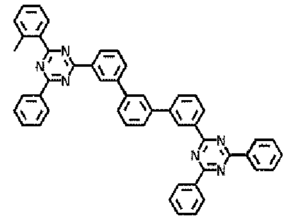
A(147)

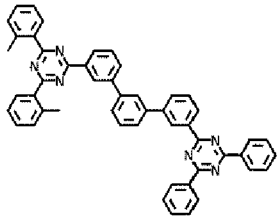
A(148)

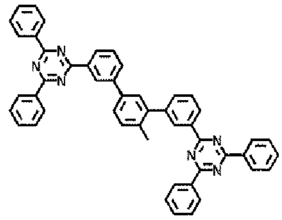
A(149)

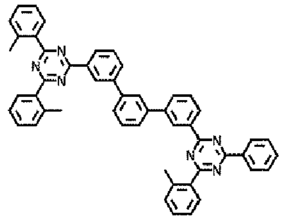
A(150)

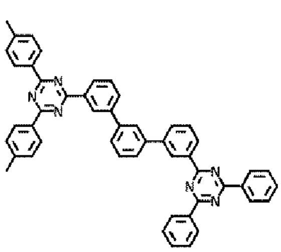
A(151)

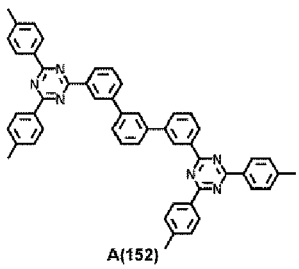
A(152)

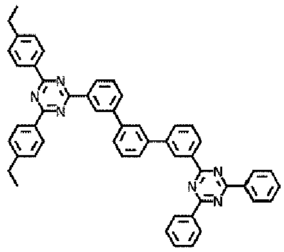
A(153)

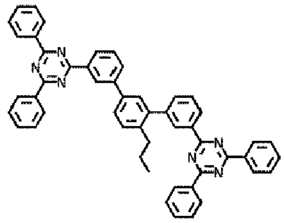
A(154)

Group HE2

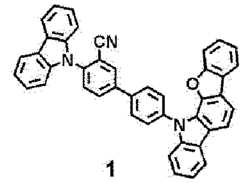
1

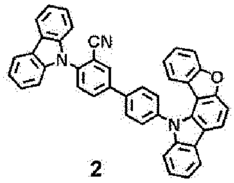
2

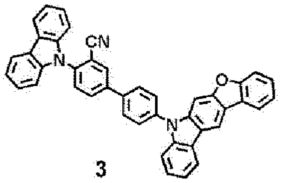
3

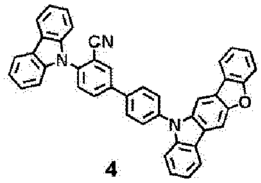
4

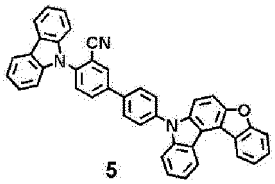
5

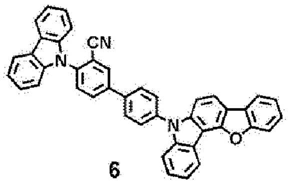
6

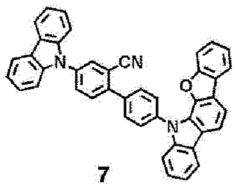
7

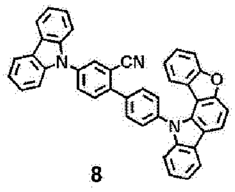
8

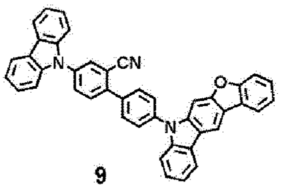
9

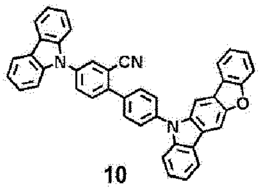
10

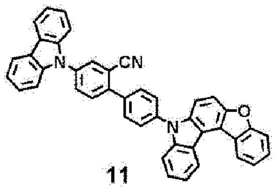
11

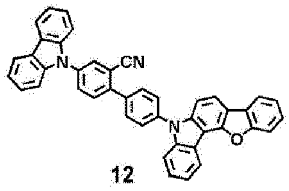
12

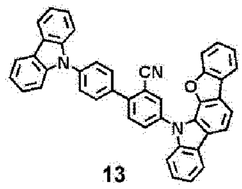
13

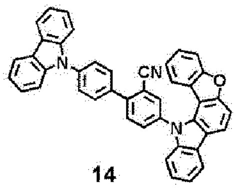
14

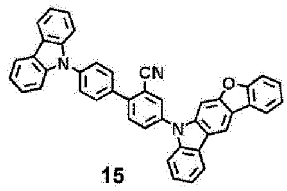
15

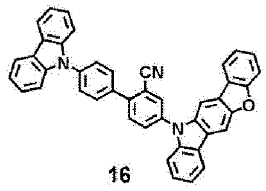
16

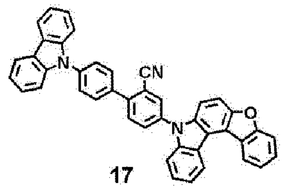
17

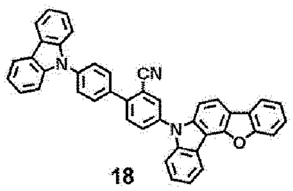
18

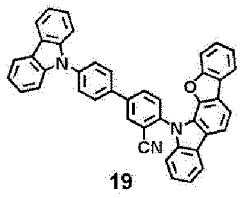
19

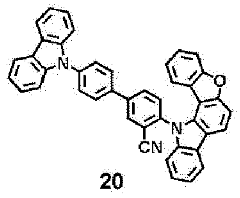
20

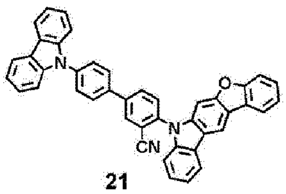
21

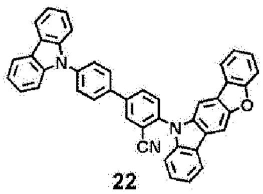
22

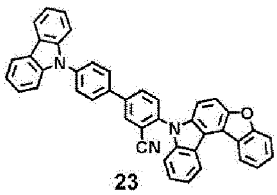
23

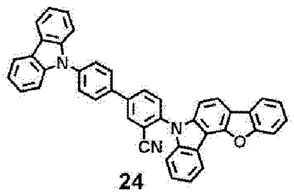
24

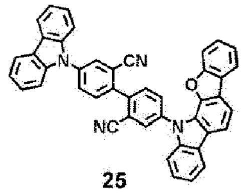
**25**

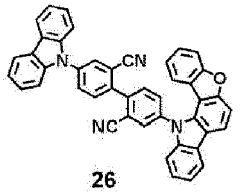
**26**

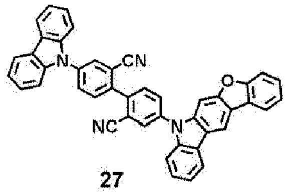
**27**

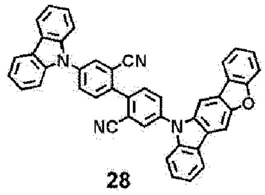
**28**

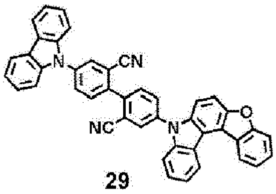
**29**

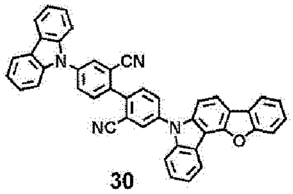
**30**

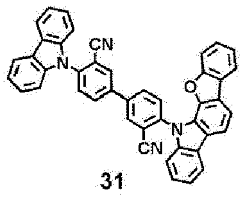
**31**

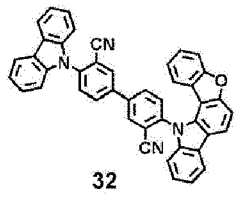
**32**

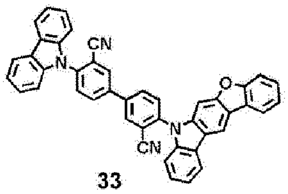
**33**

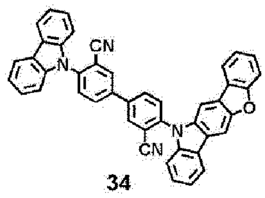
**34**

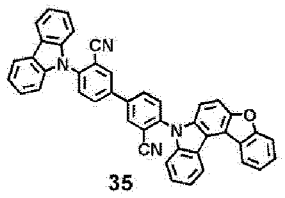
**35**

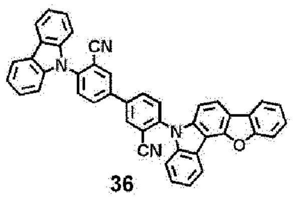
**36**

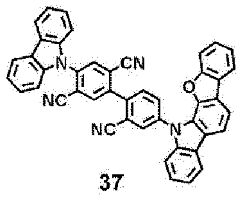
**37**

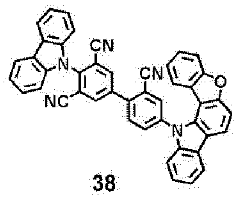
**38**

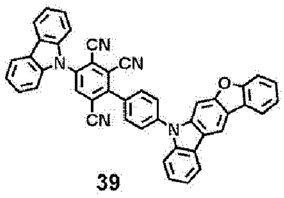
**39**

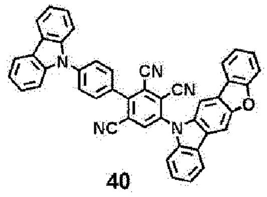
**40**

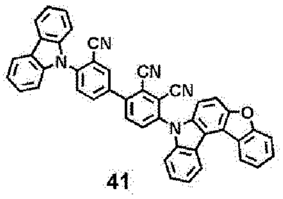
**41**

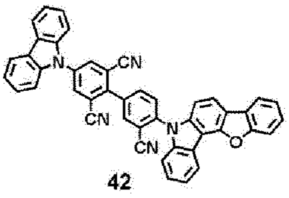
**42**

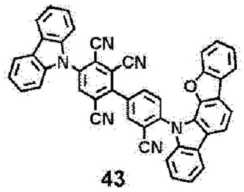
**43**

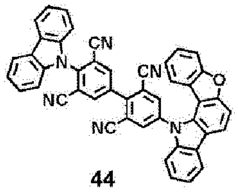
**44**

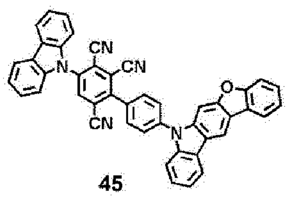
**45**

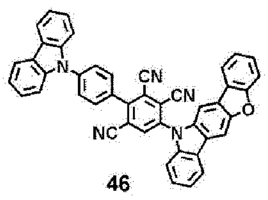
**46**

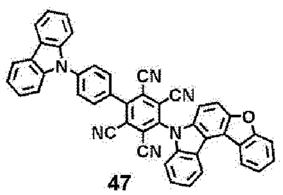
**47**

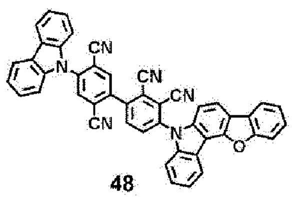
**48**

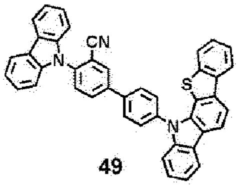
49

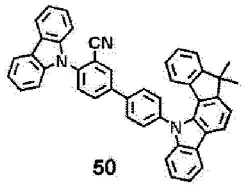
50

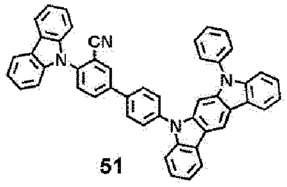
51

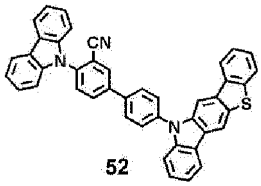
52

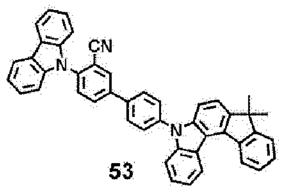
53

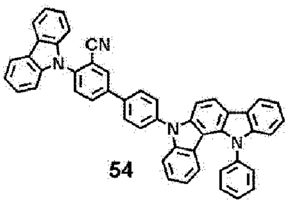
54

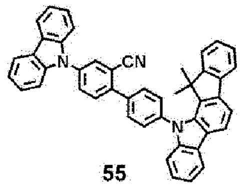
55

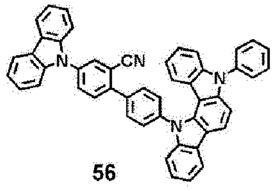
56

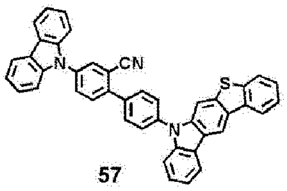
57

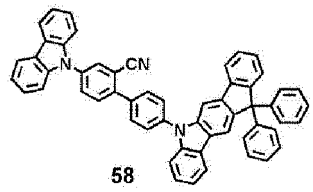
58

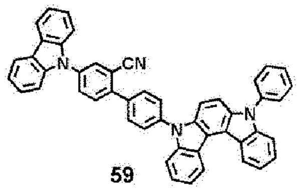
59

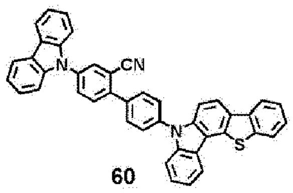
60

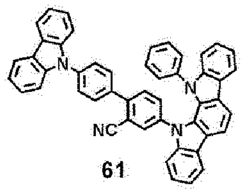
61

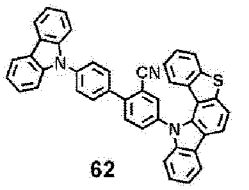
62

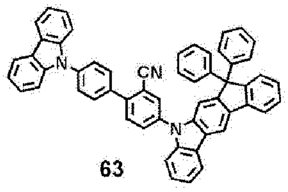
63

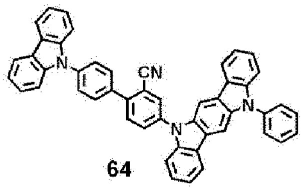
64

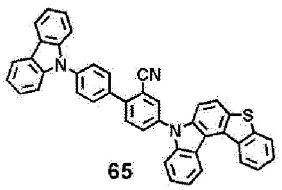
65

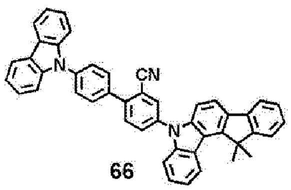
66

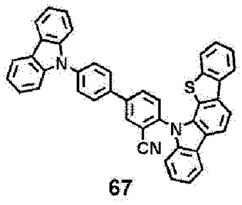
67

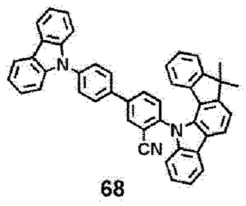
68

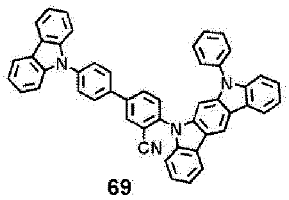
69

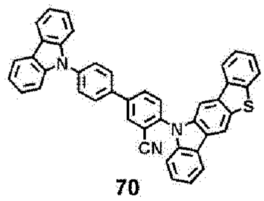
70

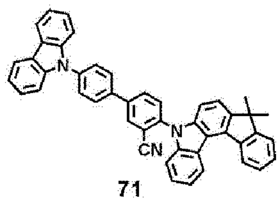
71

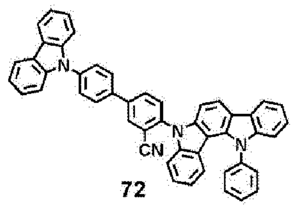
72

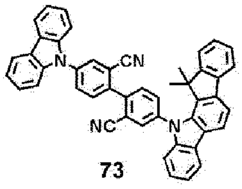
73
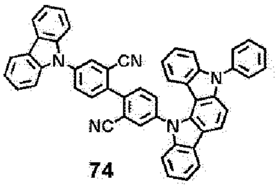
74
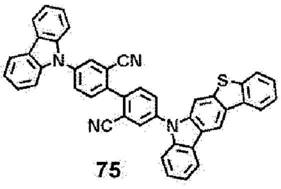
75
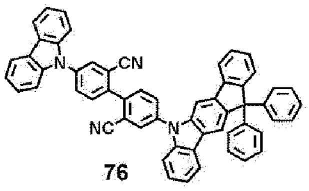
76

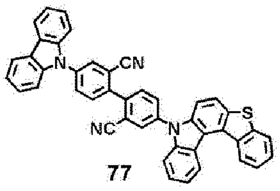
77
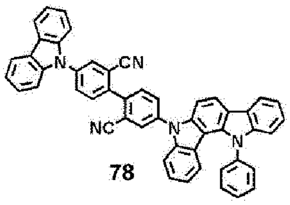
78
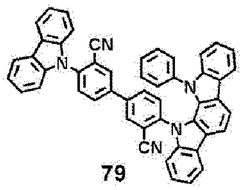
79
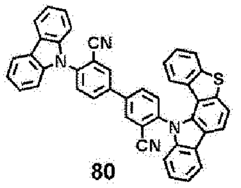
80

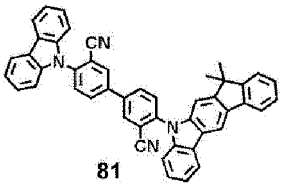
81
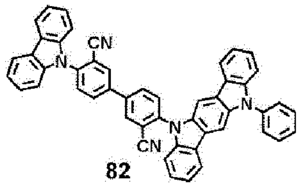
82
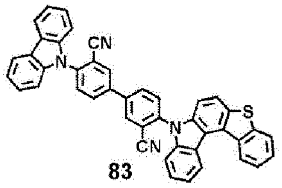
83
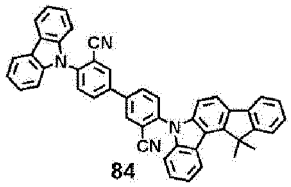
84

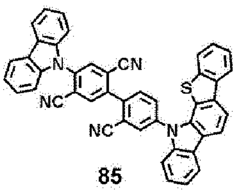
85
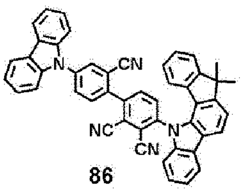
86
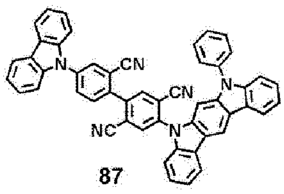
87
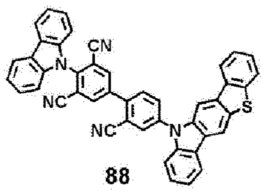
88

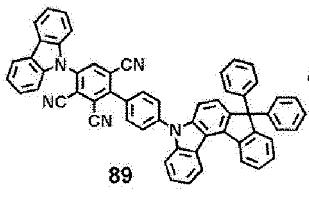
89
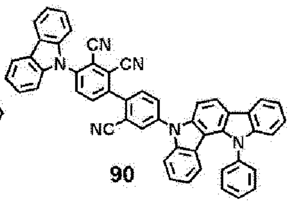
90
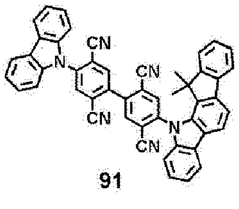
91
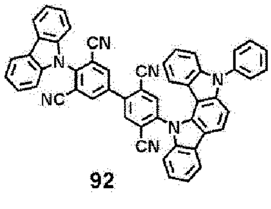
92

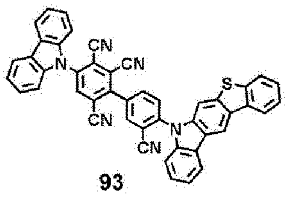
93
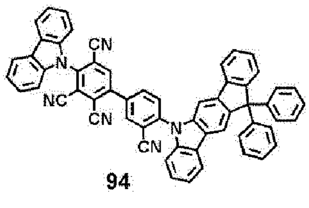
94
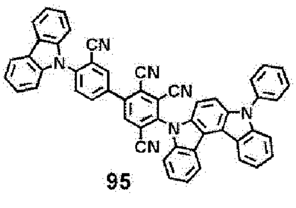
95
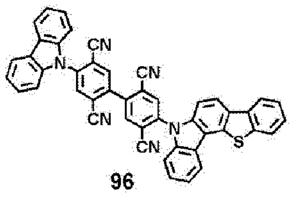
96

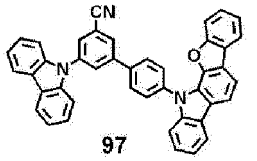
97

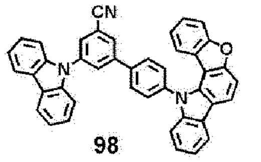
98

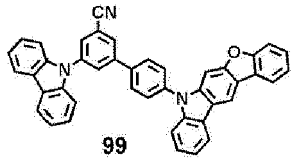
99

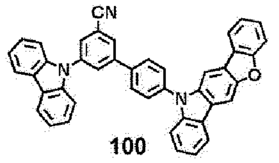
100

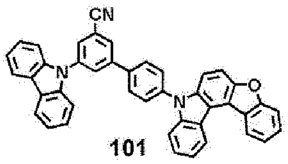
101

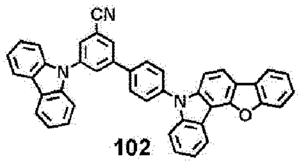
102

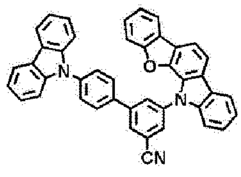
103

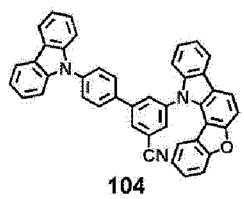
104

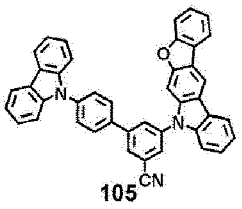
105

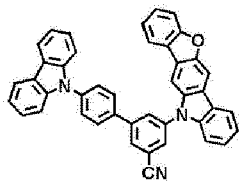
106

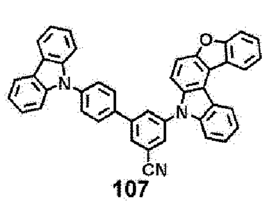
107

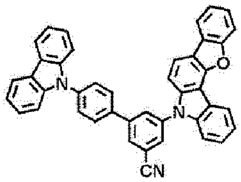
108

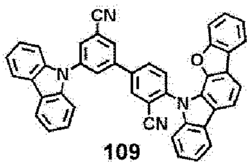
109

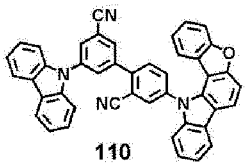
110

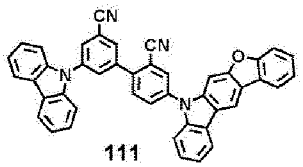
111

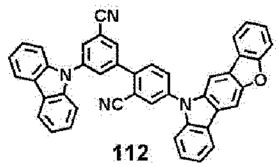
112

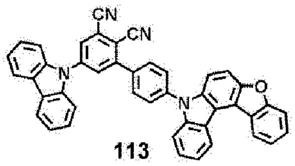
113

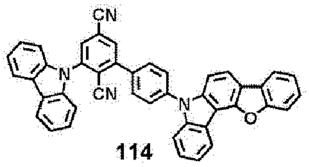
114

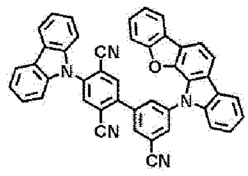
115

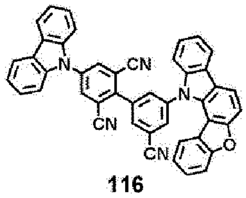
116

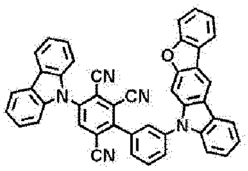
117

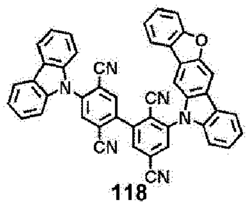
118

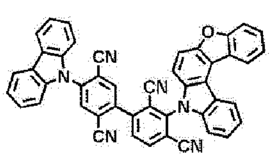
119

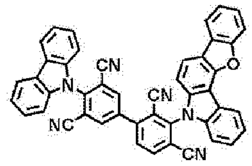
120

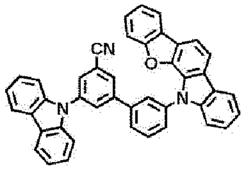
121

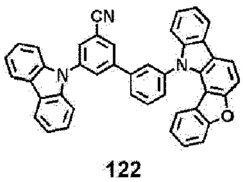
122

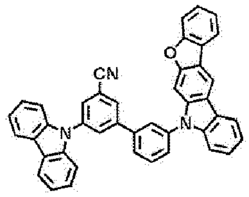
123

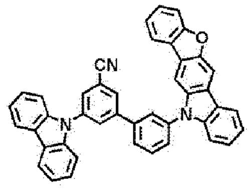
124

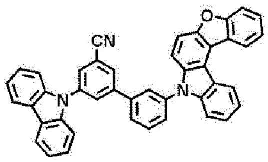
125

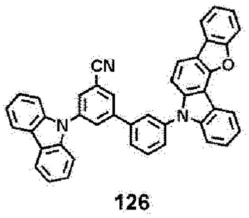
126

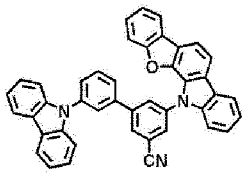
127

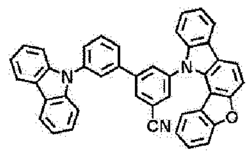
128

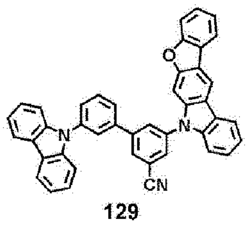
129

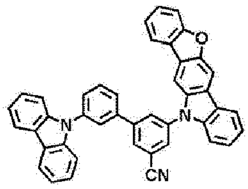
130

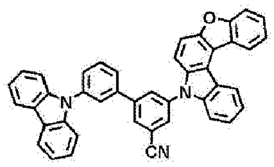
131

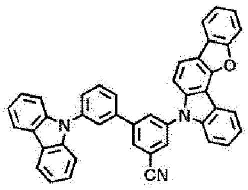
132

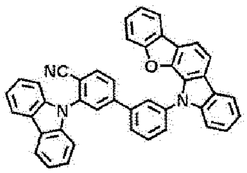
133

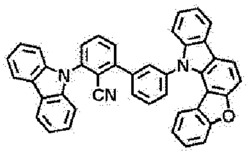
134

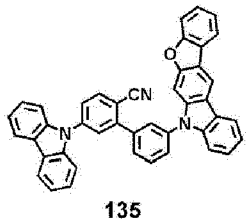
135

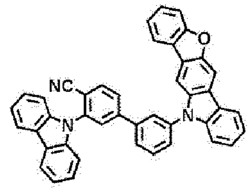
136

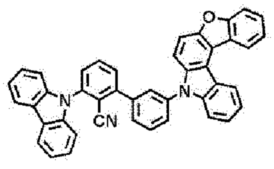
137

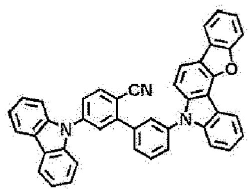
138

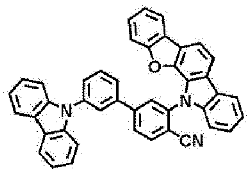
139

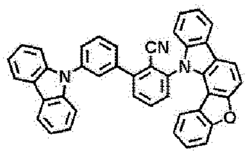
140

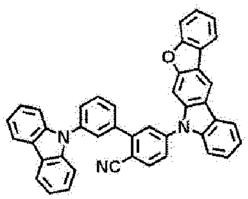
141

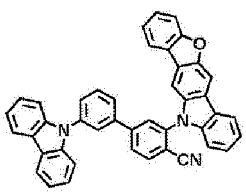
142

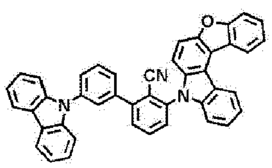
143

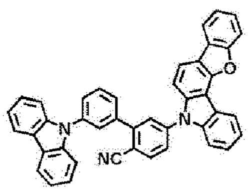
144

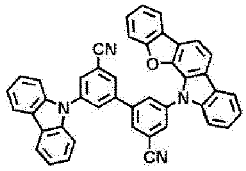
145

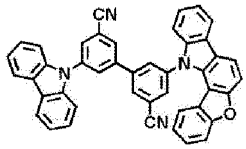
146

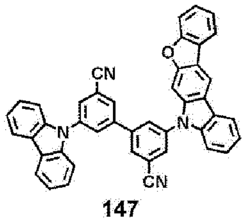
147

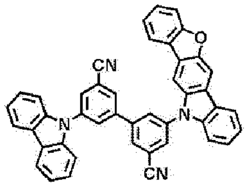
148

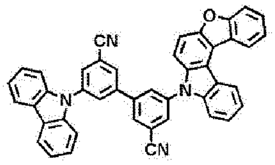
149

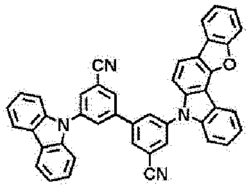
150

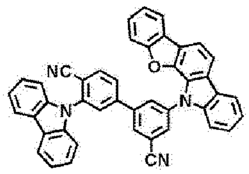
151

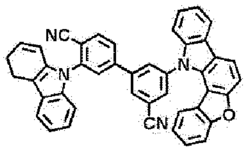
152

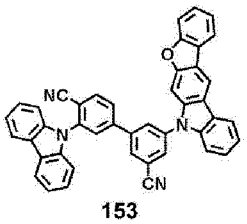
153

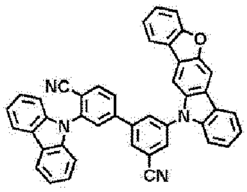
154

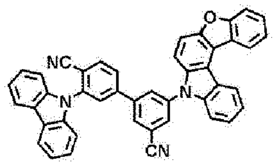
155

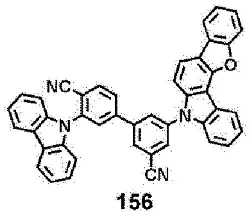
156

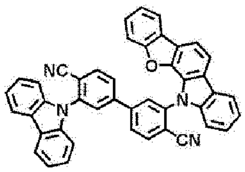
157

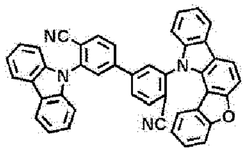
158

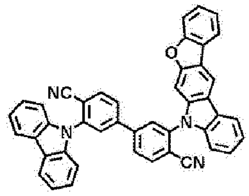
159

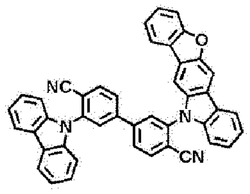
160

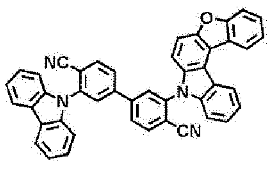
161

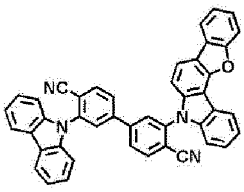
162

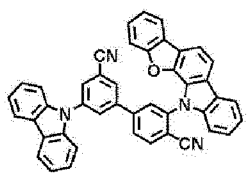
163

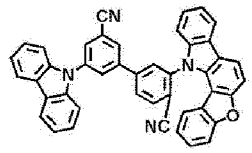
164

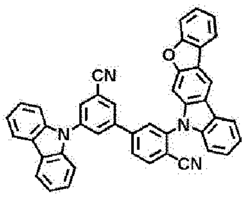
165

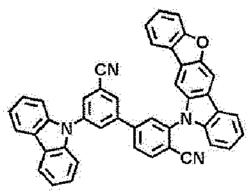
166

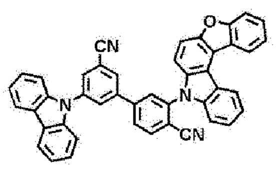
167

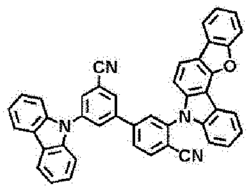
168

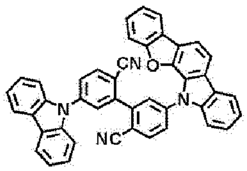
169

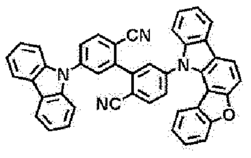
170

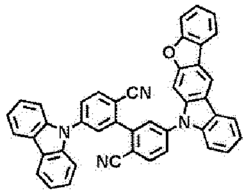
171

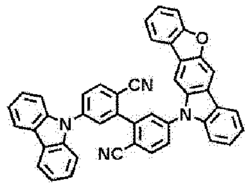
172

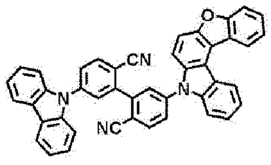
173

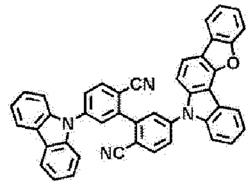
174

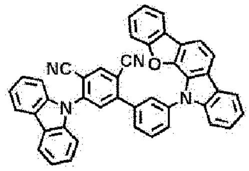
175

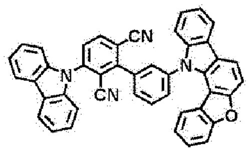
176

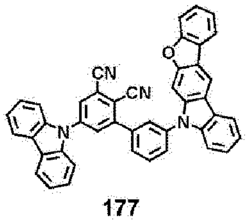
177

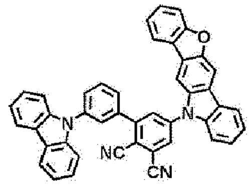
178

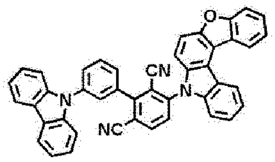
179

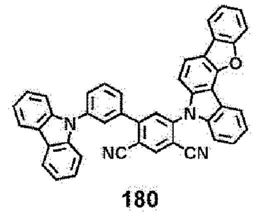
180

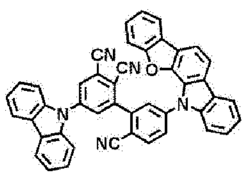
181

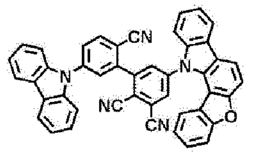
182

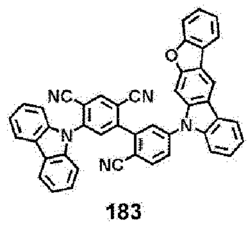
183

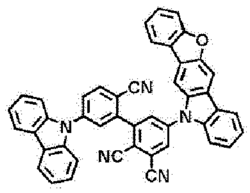
184

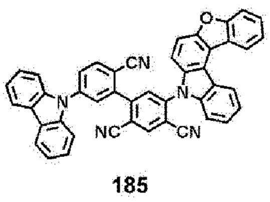
185

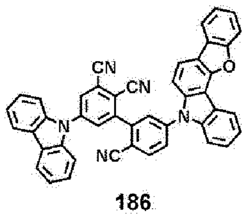
186

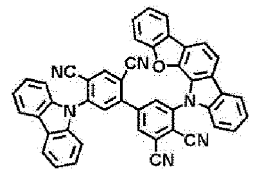
187

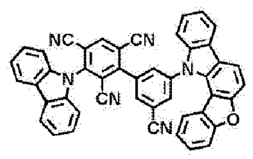
188

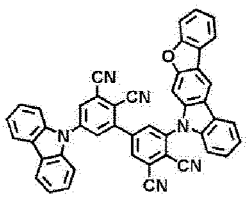
189

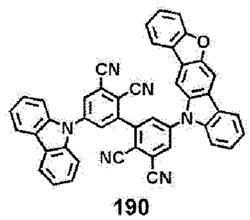
190

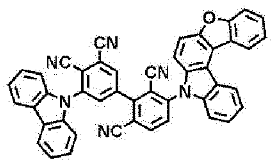
191

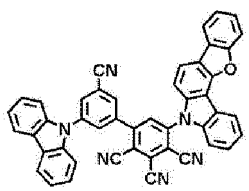
192

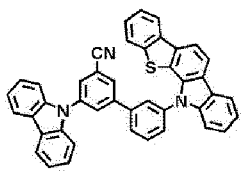
193

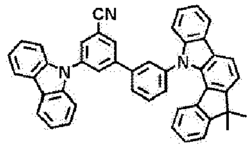
194

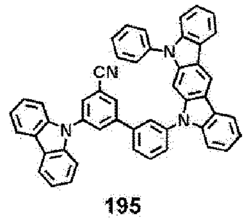
195

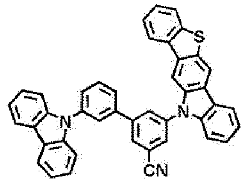
196

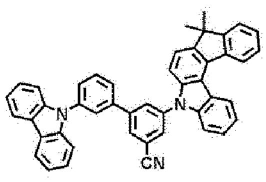
197

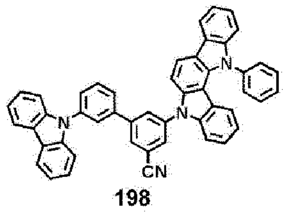
198

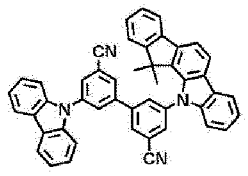
199

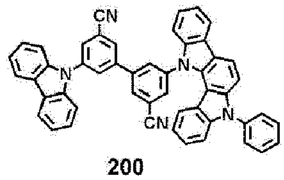
200

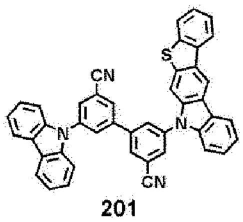
201

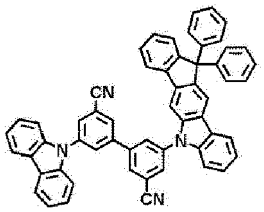
202

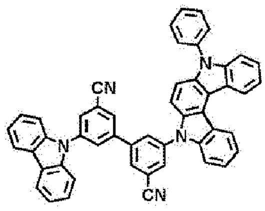
203

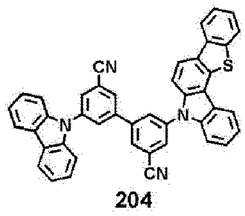
204

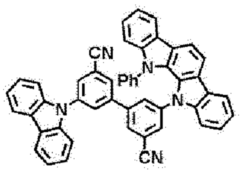
205

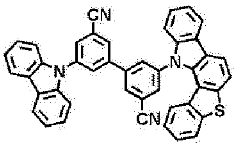
206

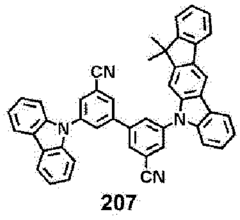
207

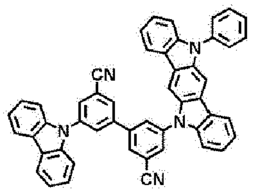
208

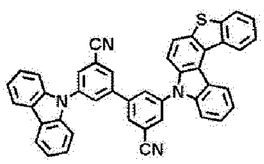
209

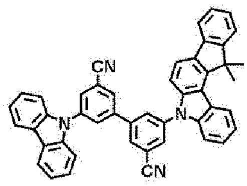
210

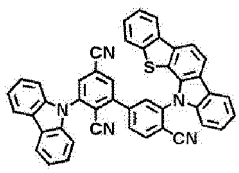
211

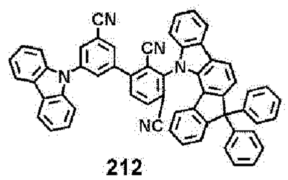
212

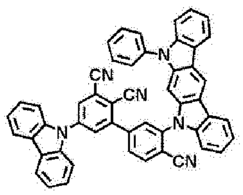
213

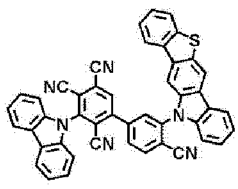
214

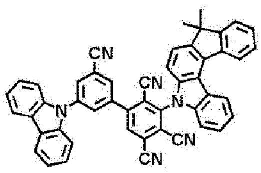
215

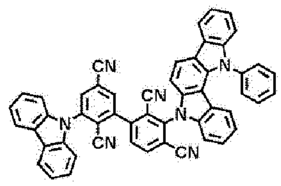
216

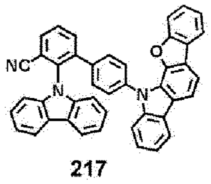
217
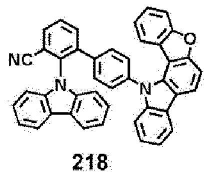
218
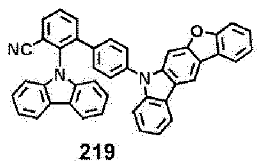
219
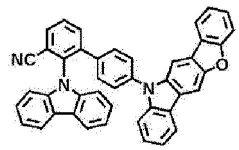
220

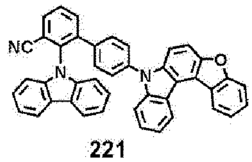
221
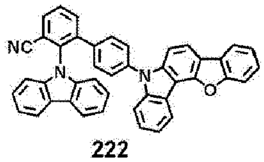
222
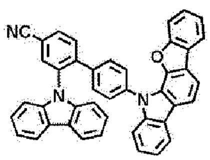
223
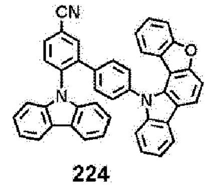
224

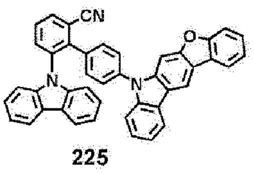
225
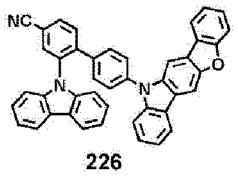
226
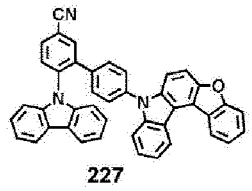
227
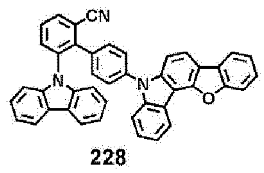
228

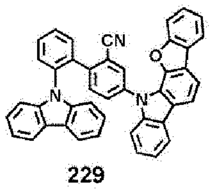
229
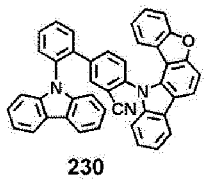
230
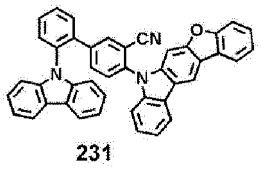
231
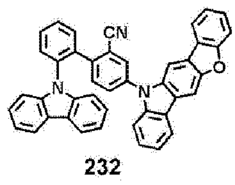
232

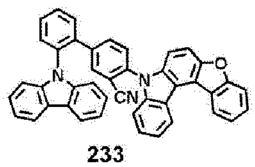
233
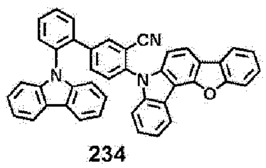
234
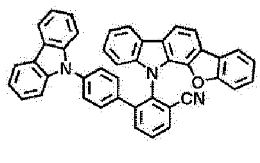
235
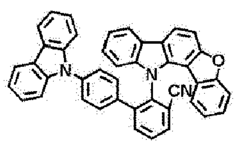
236

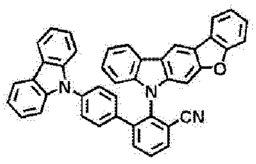
237
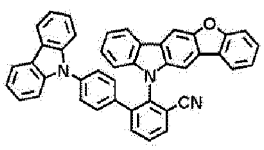
238
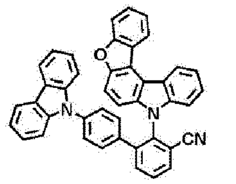
239
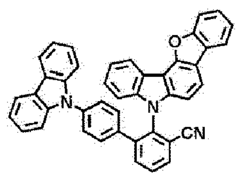
240

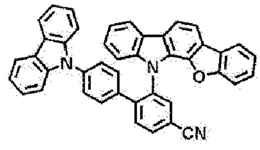
241

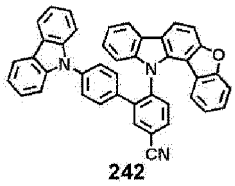
242

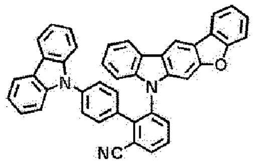
243

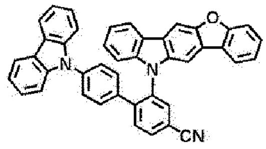
244

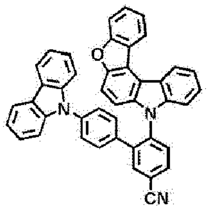
245

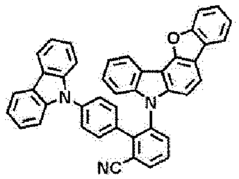
246

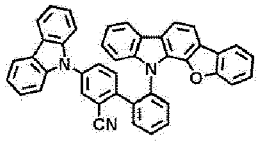
247

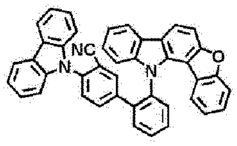
248

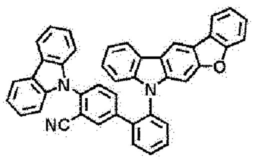
249

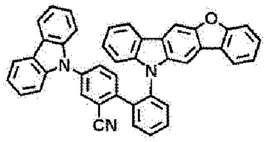
250

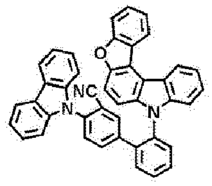
251

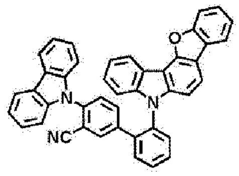
252

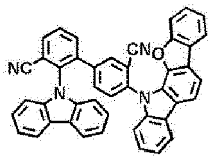
253

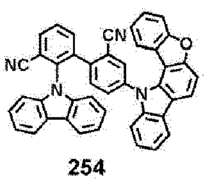
254

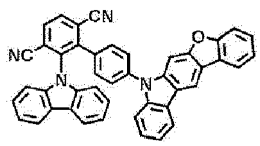
255

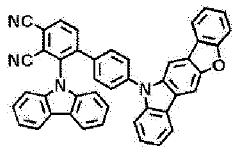
256

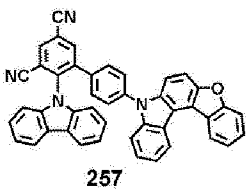
257

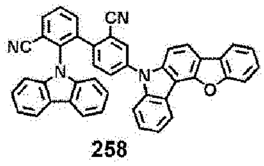
258

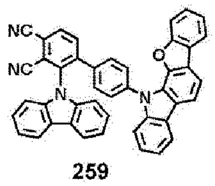
259

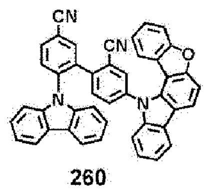
260

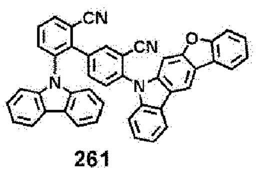
261

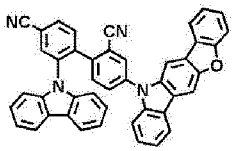
262

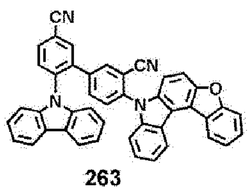
263

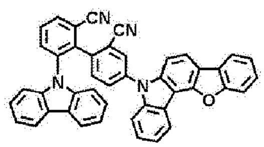
264

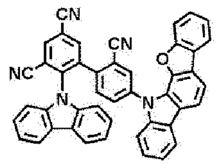
**265**

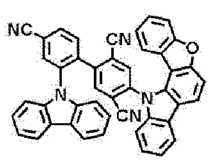
**266**

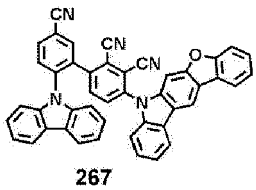
**267**

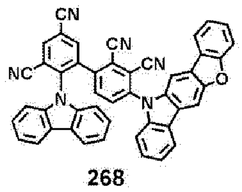
**268**

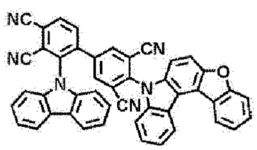
**269**

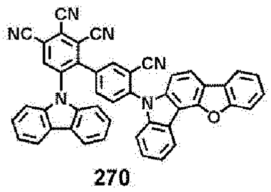
**270**

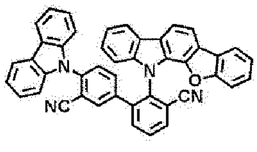
**271**

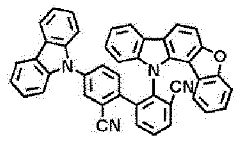
**272**

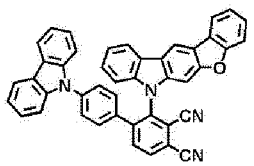
**273**

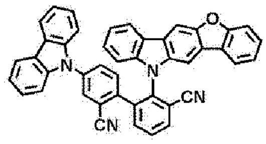
**274**

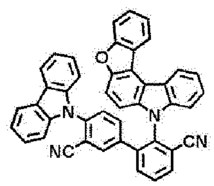
**275**

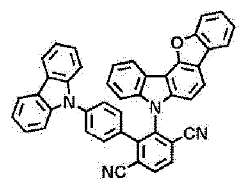
**276**

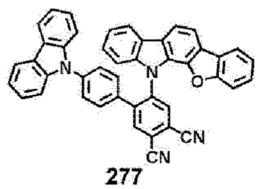
**277**

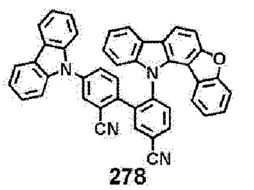
**278**

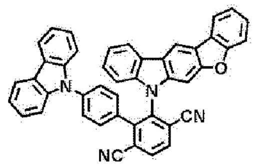
**279**

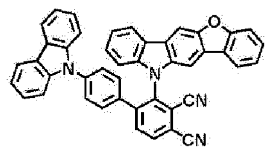
**280**

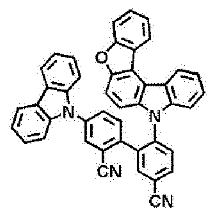
**281**

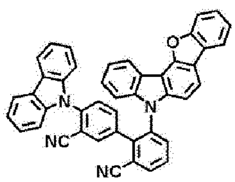
**282**

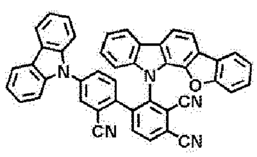
**283**

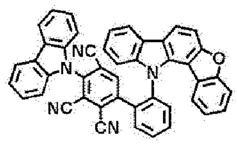
**284**

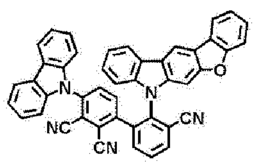
**285**

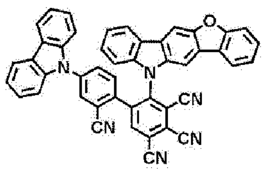
**286**

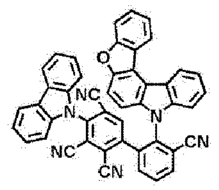
**287**

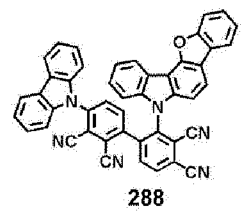
**288**

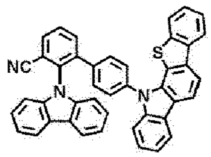
289
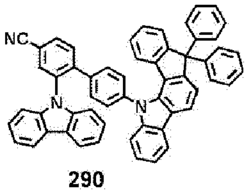
290
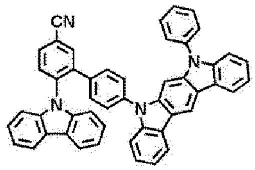
291
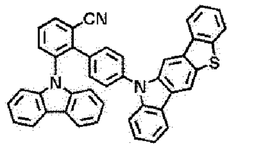
292

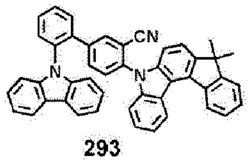
293
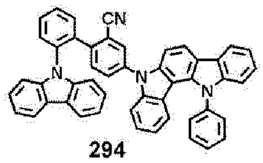
294
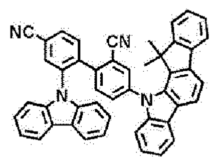
295
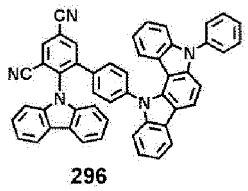
296

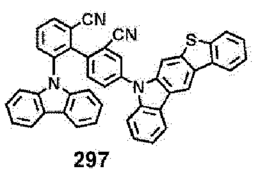
297
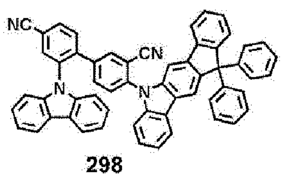
298
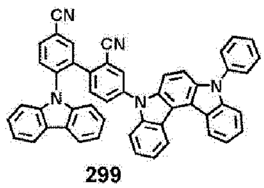
299
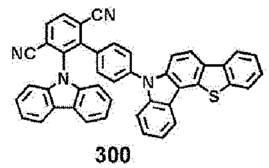
300

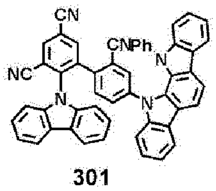
301
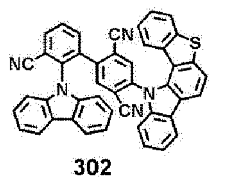
302
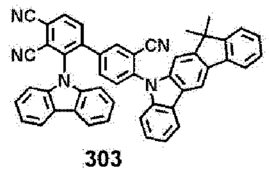
303
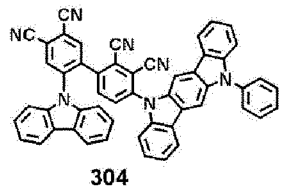
304

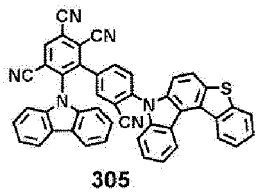
305
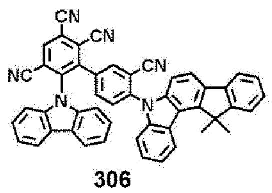
306
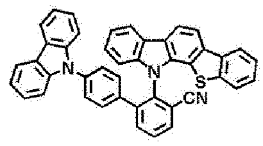
307
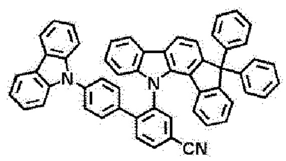
308

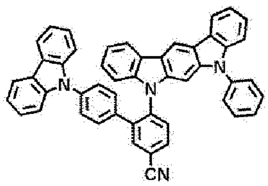
309
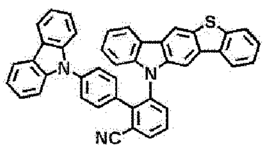
310
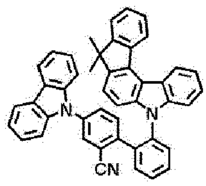
311
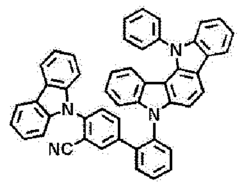
312

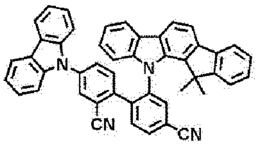

313

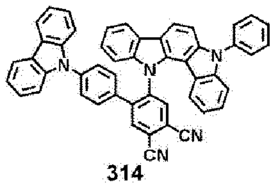

314

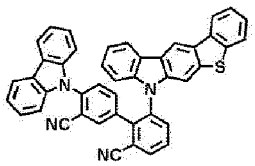

315

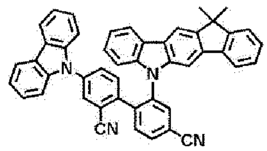

316

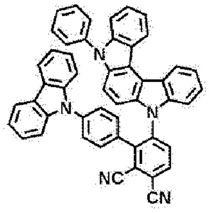

317

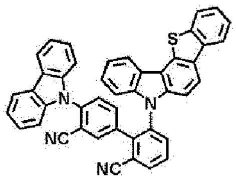

318

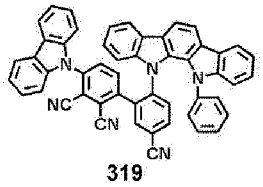

319

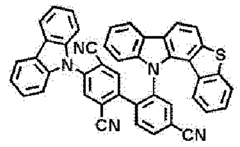

320

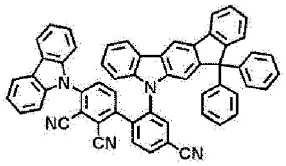

321

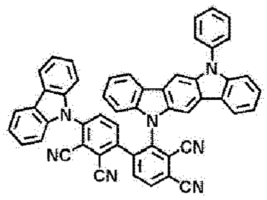

322

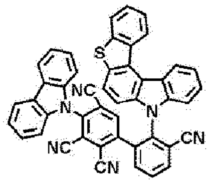

323

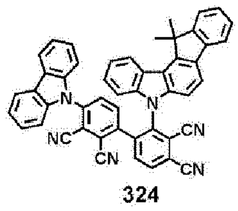

324

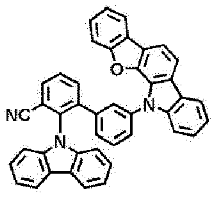

325

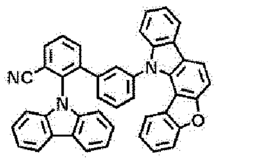

326

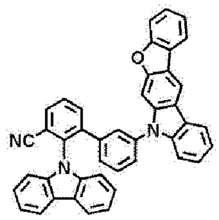

327

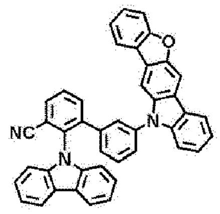

328

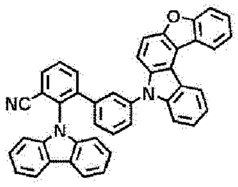

329

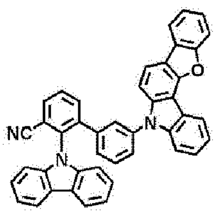

330

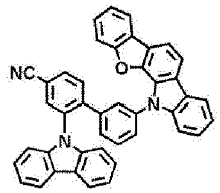

331

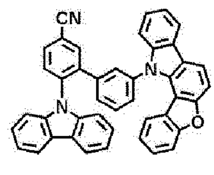

332

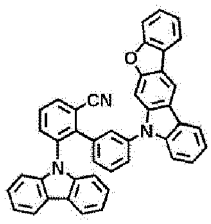

333

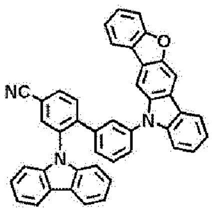

334

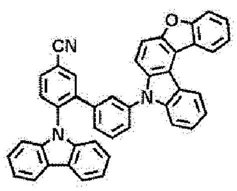

335

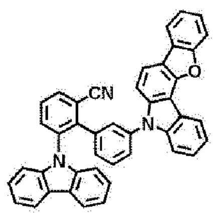

336

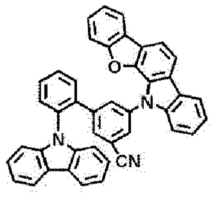
337

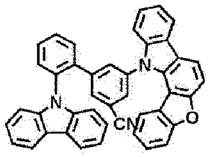
338

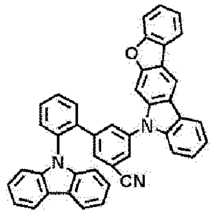
339

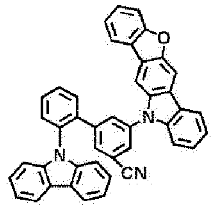
340

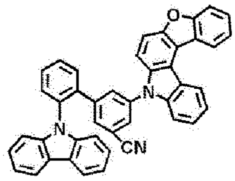
341

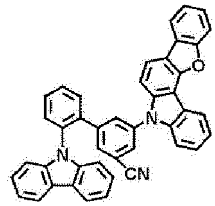
342

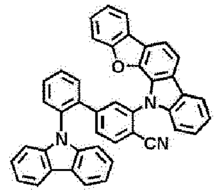
343

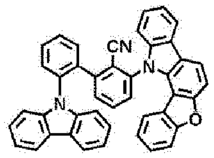
344

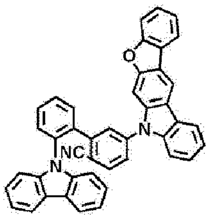
345

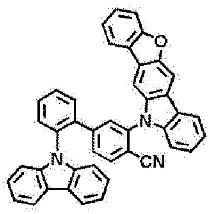
346

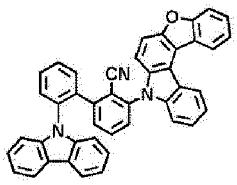
347

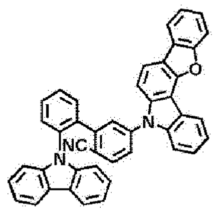
348

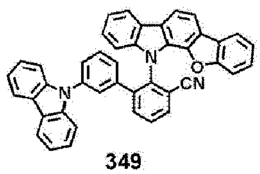
349

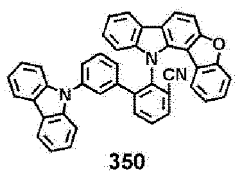
350

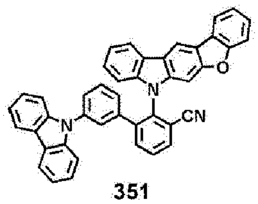
351

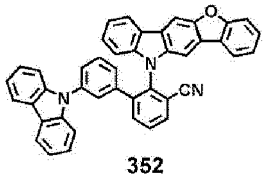
352

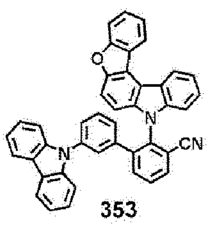
353

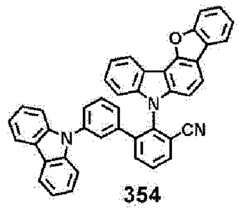
354

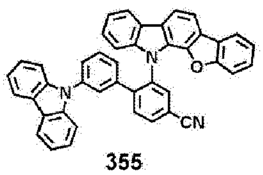
355

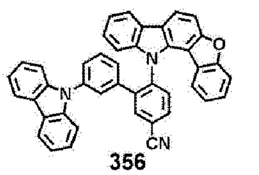
356

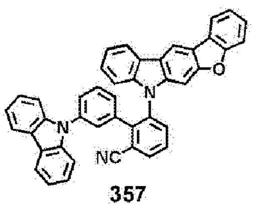
357

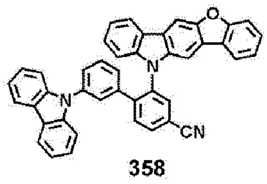
358

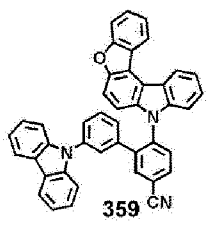
359

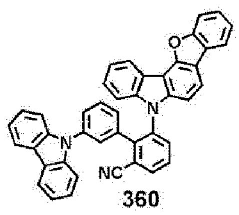
360

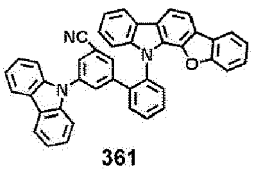
361
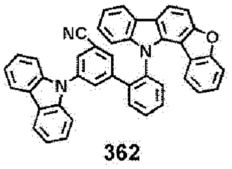
362
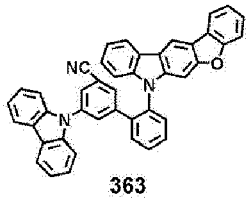
363
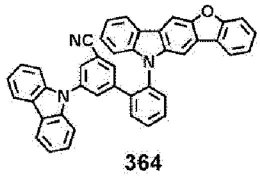
364

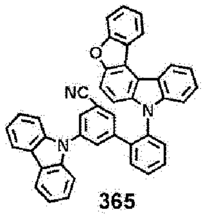
365
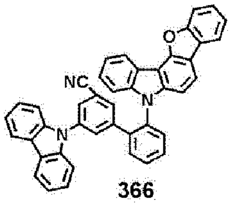
366
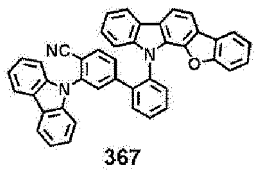
367
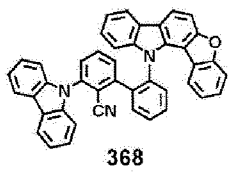
368

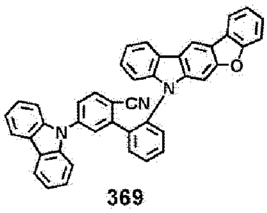
369
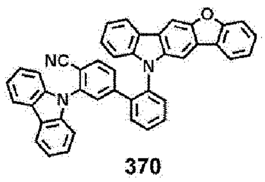
370
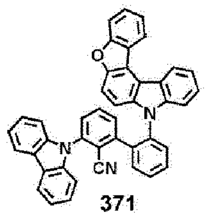
371
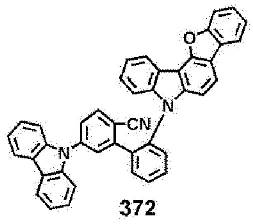
372

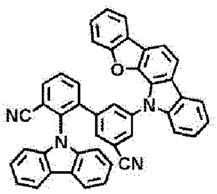
373
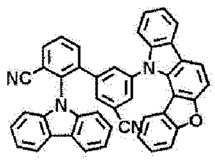
374
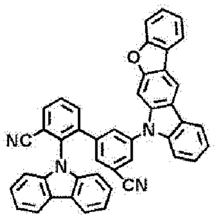
375
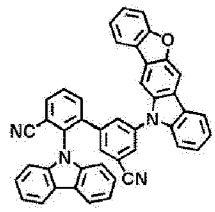
376

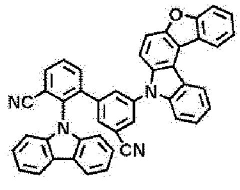
377
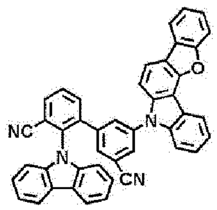
378
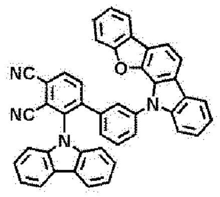
379
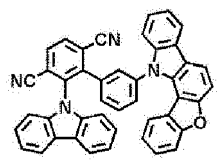
380

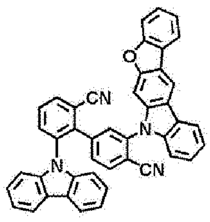
381
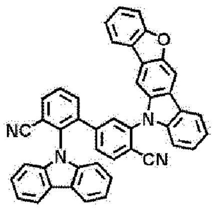
382
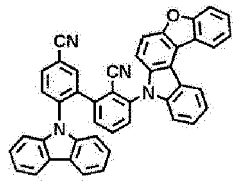
383
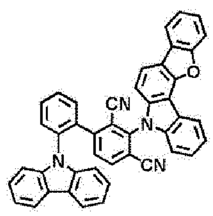
384

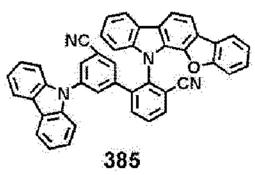
385
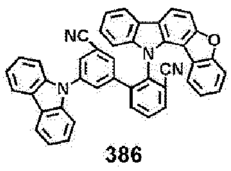
386
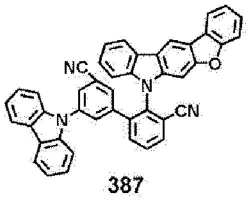
387
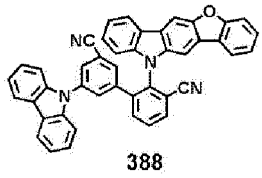
388

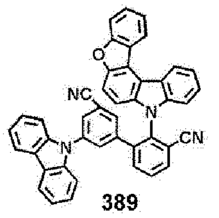
389
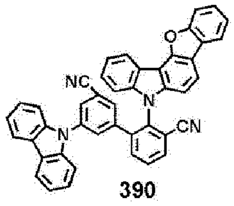
390
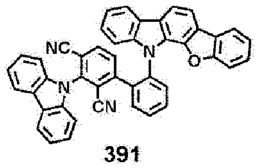
391
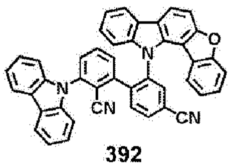
392

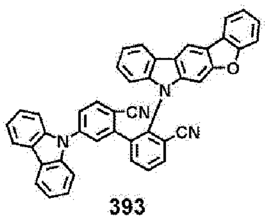
393
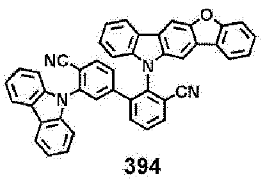
394
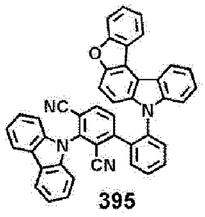
395
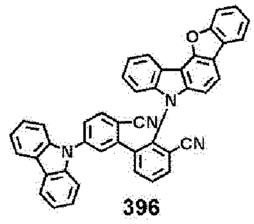
396

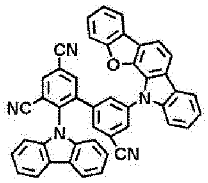
397
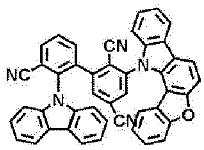
398
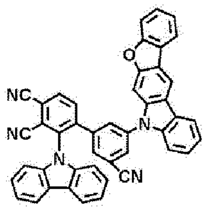
399
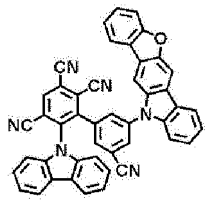
400

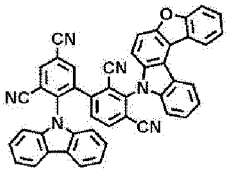
401
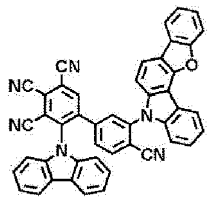
402
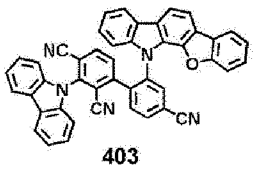
403
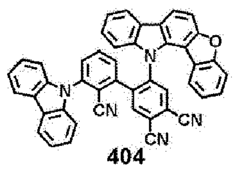
404

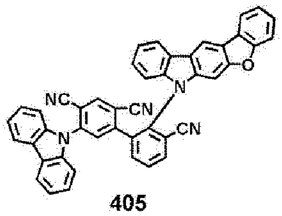
405
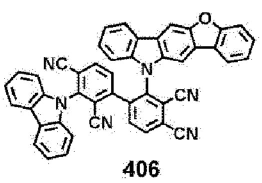
406
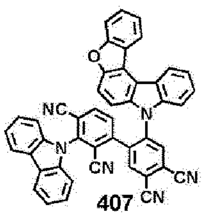
407
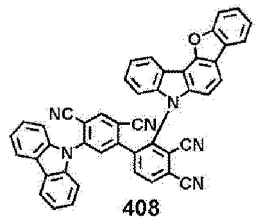
408

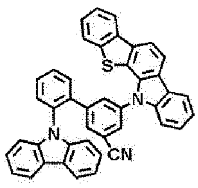

409

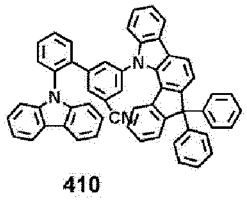

410

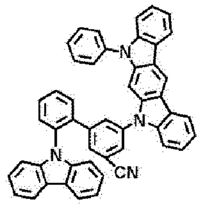

411

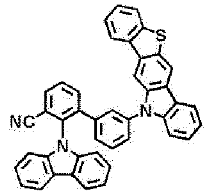

412

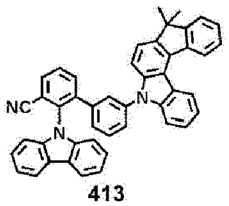

413

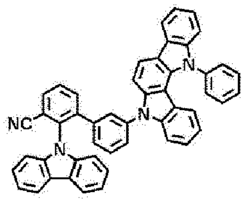

414

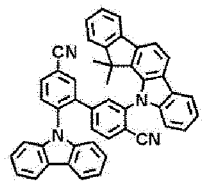

415

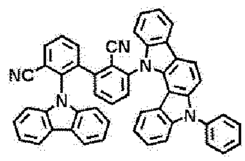

416

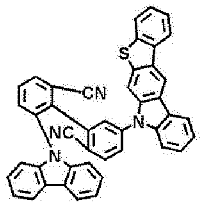

417

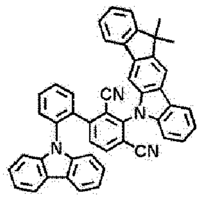

418

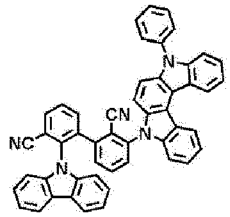

419

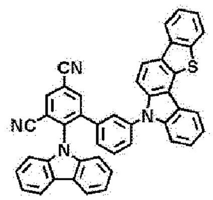

420

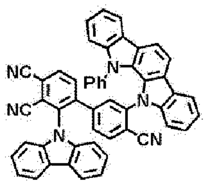

421

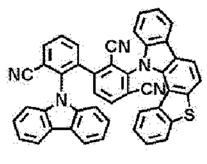

422

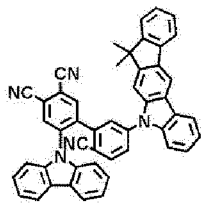

423

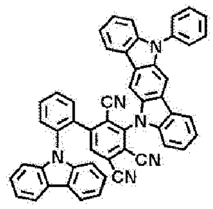

424

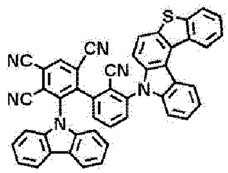

425

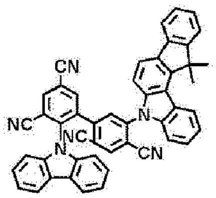

426

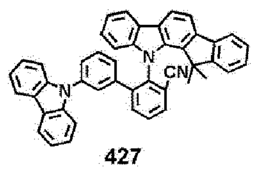

427

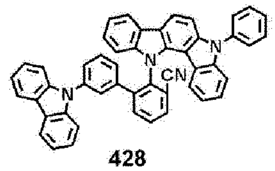

428

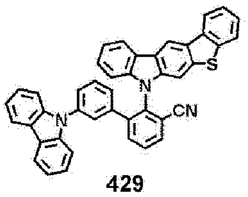

429

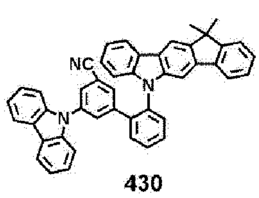

430

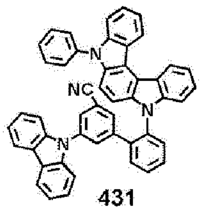

431

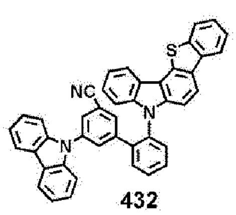

432

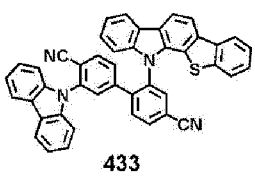
433
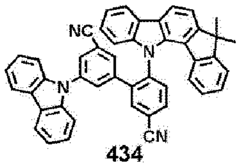
434
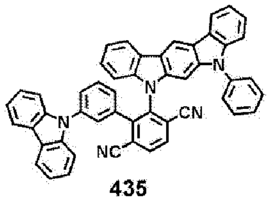
435
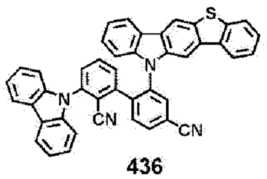
436

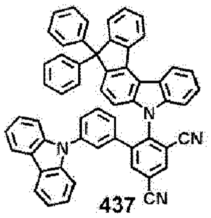
437
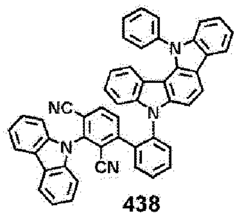
438
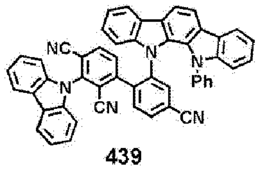
439
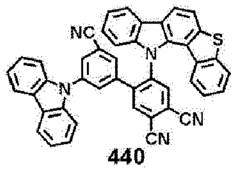
440

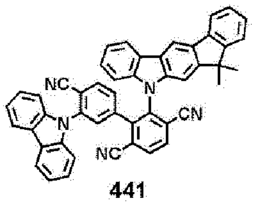
441
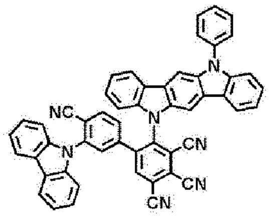
442
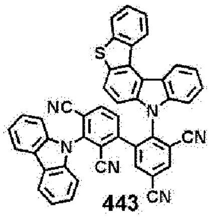
443
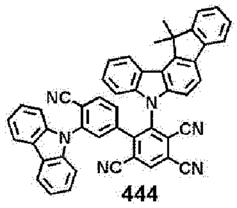
444

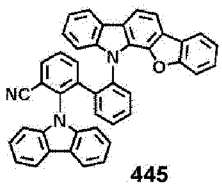
445
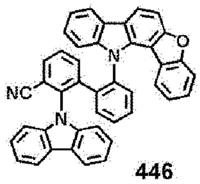
446
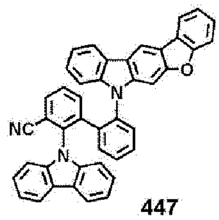
447
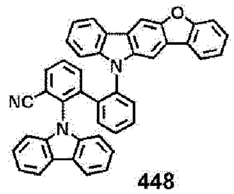
448

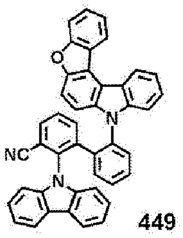
449
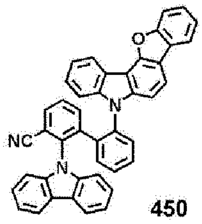
450
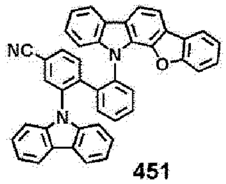
451
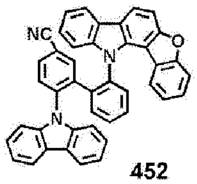
452

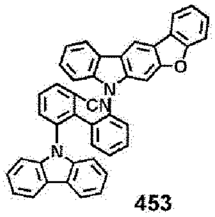
453
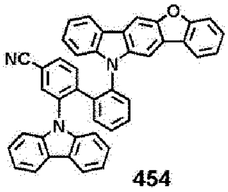
454
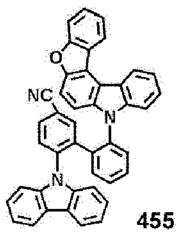
455
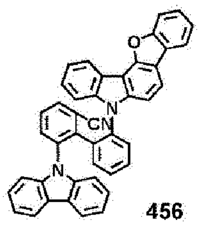
456

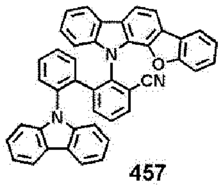
457

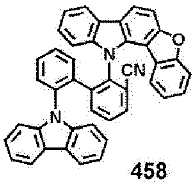
458

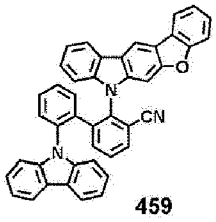
459

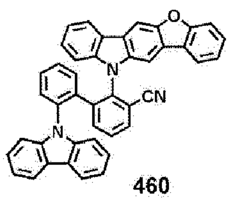
460

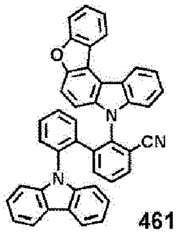
461

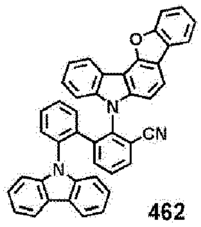
462

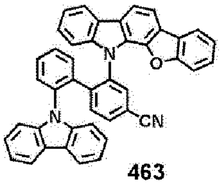
463

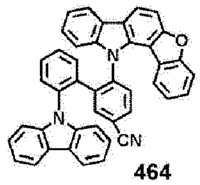
464

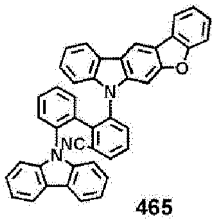
465

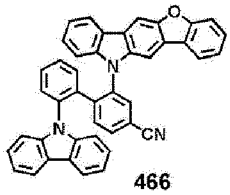
466

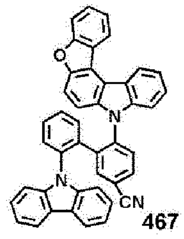
467

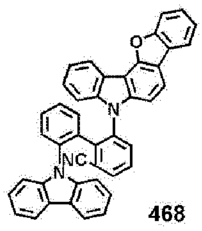
468

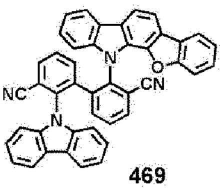
469

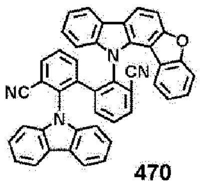
470

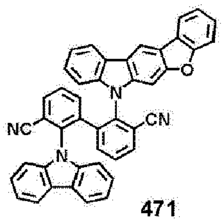
471

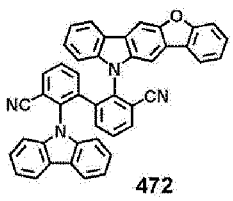
472

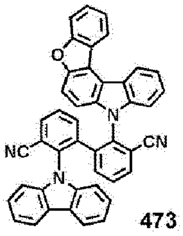
473

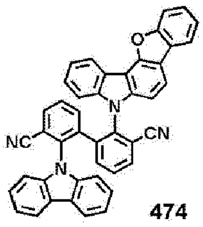
474

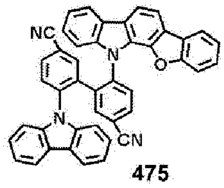
475

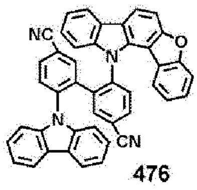
476

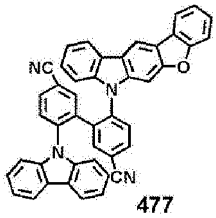
477

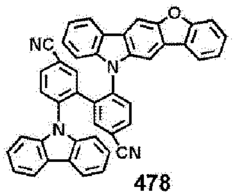
478

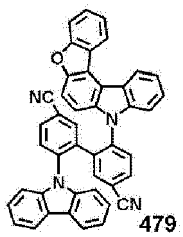
479

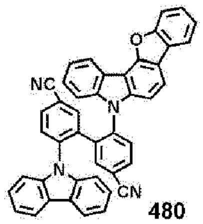
480

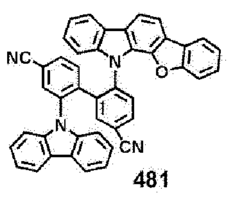
481

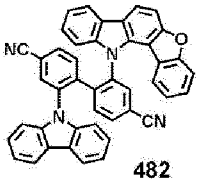
482

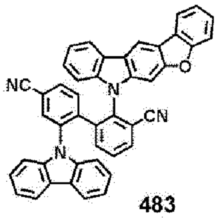
483

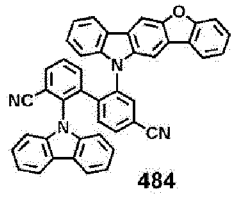
484

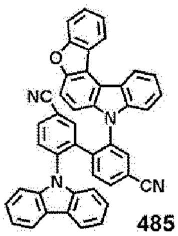
485

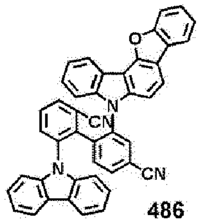
486

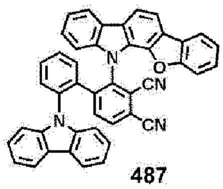
487

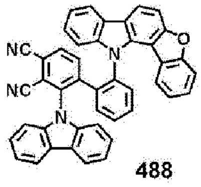
488

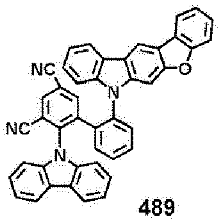
489

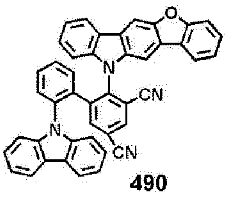
490

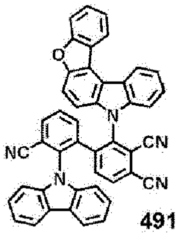
491

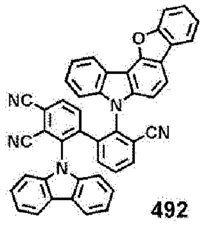
492

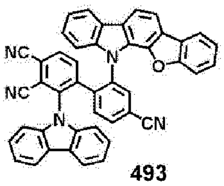
493

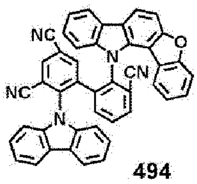
494

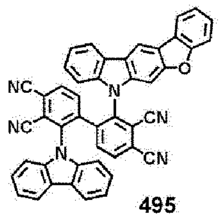
495

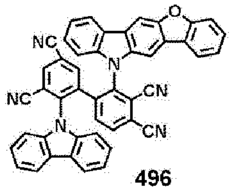
496

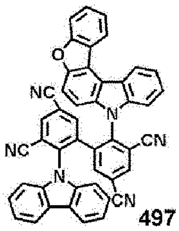
497

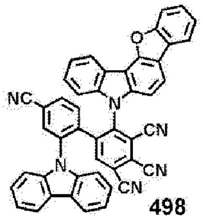
498

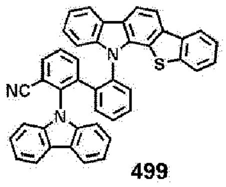
499

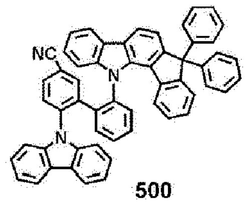
500

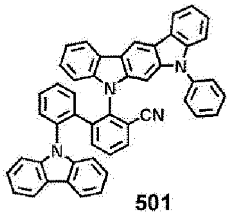
501

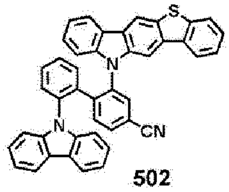
502

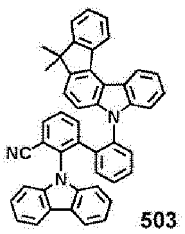
503

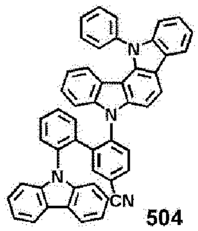
504

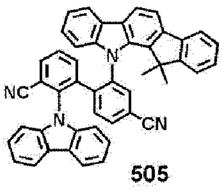
505

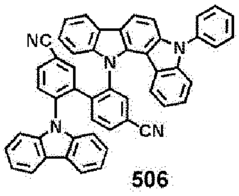
506

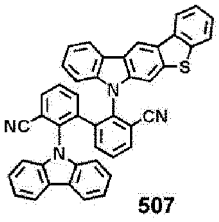
507

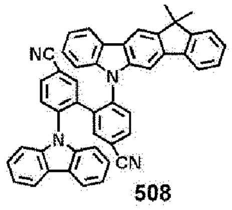
508

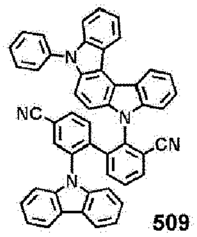
509

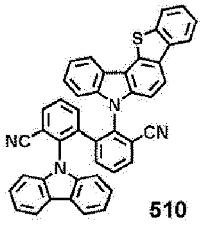
510

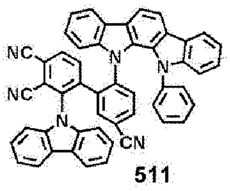
511

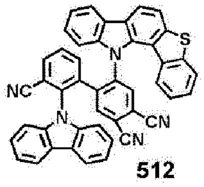
512

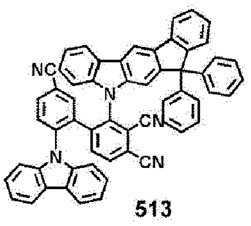
513

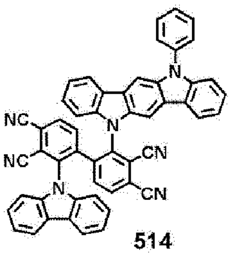
514

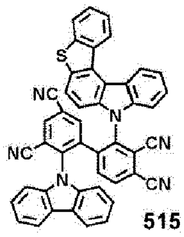
515

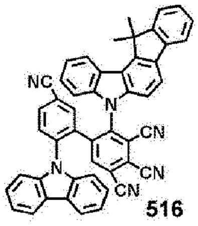
516

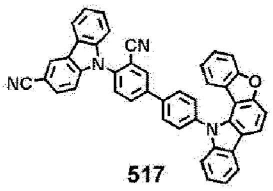
517

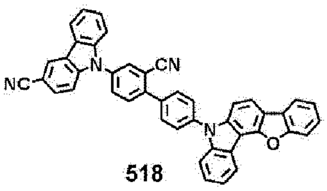
518

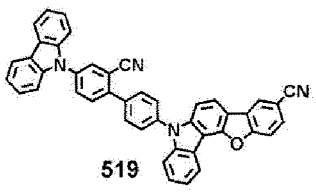
519

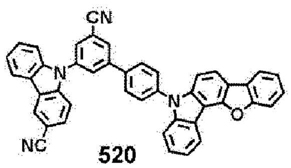
520

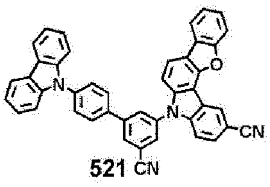
521

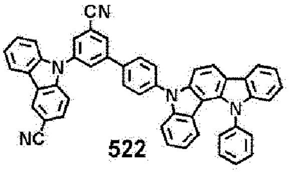
522

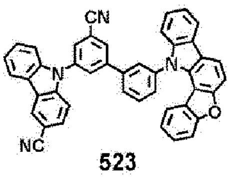
523

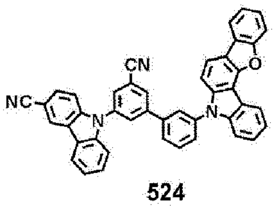
524

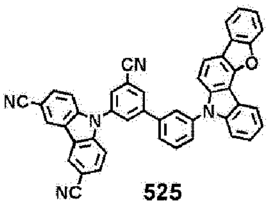
525

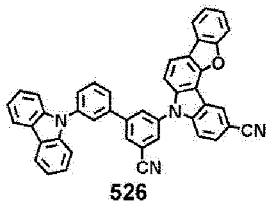
526

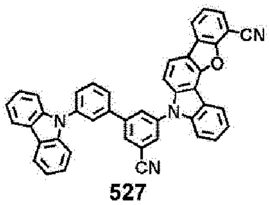
527

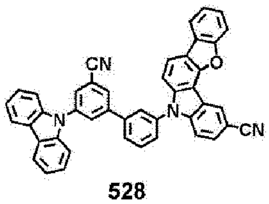
528

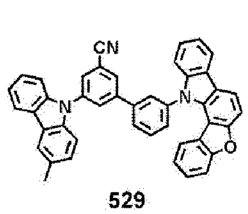
529

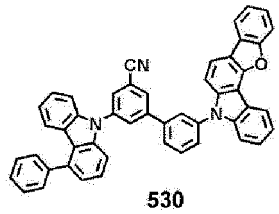
530

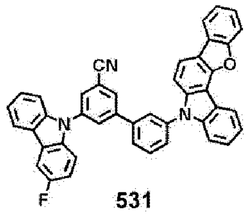
531

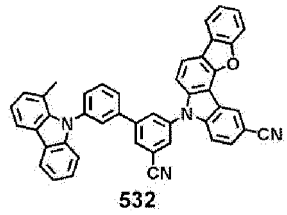
532

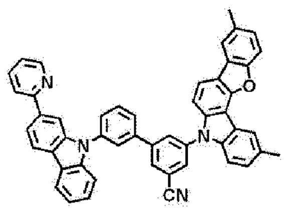
533

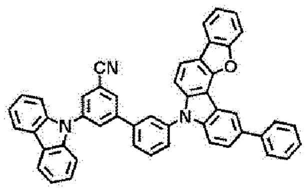
534

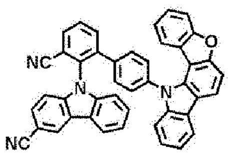
535

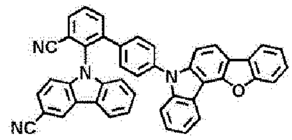
536

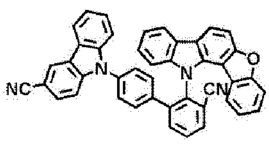
537

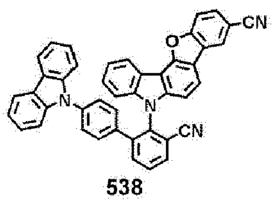
538

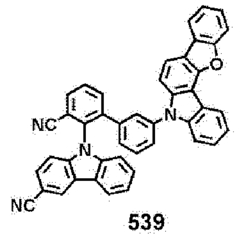
539

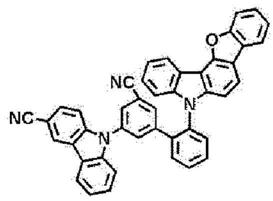
540

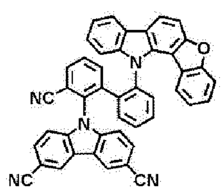
541

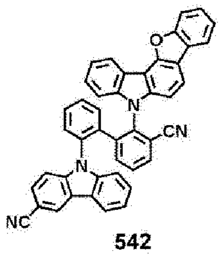
542

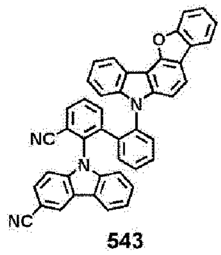
543

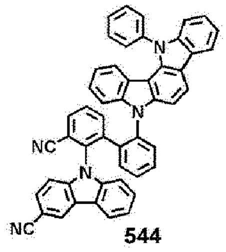
544

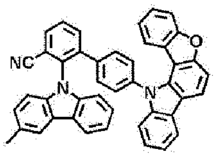
545

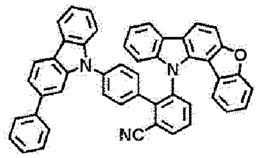
546

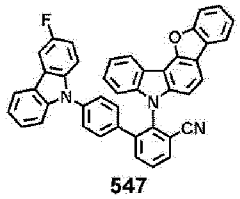
547

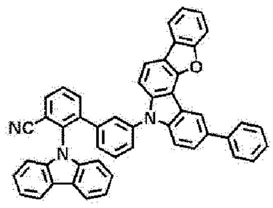
548

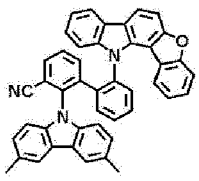
549

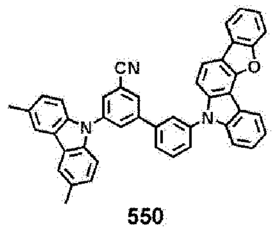
550

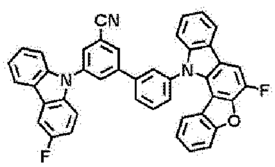
551

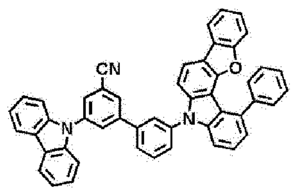
552

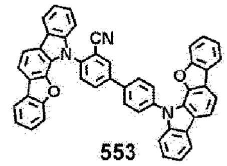 553 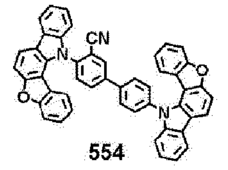 554 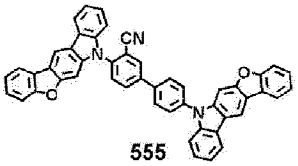 555 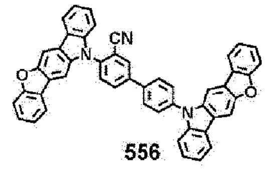 556

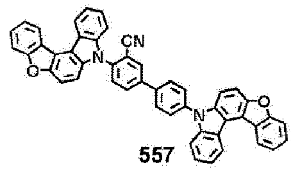 557 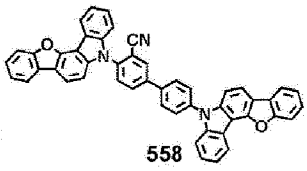 558 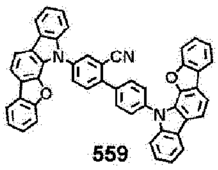 559 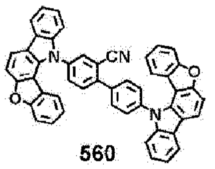 560

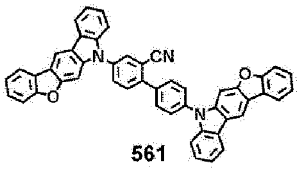 561 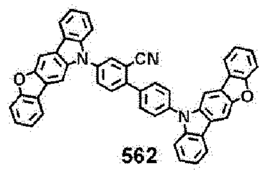 562 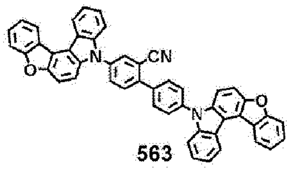 563 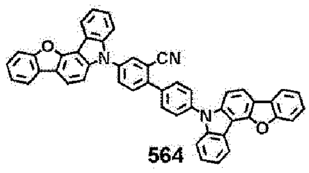 564

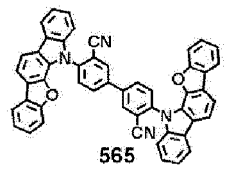 565 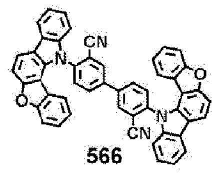 566 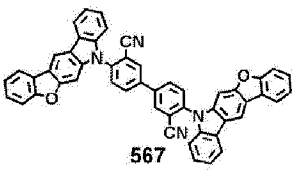 567 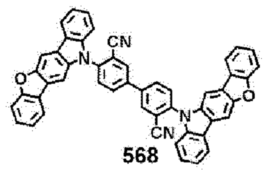 568

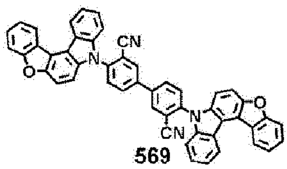 569 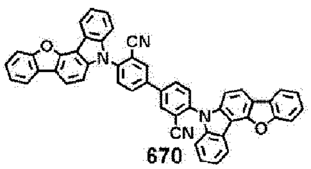 670 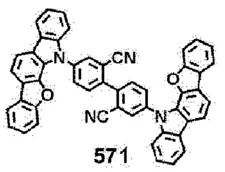 571 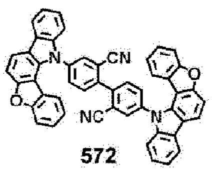 572

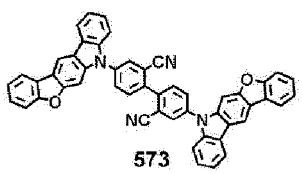 573 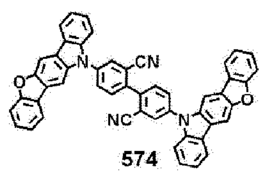 574 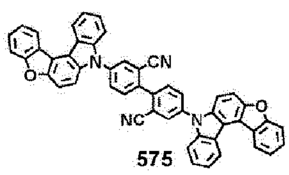 575 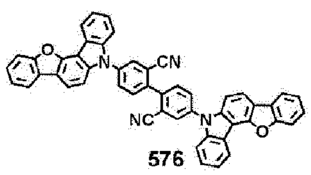 576

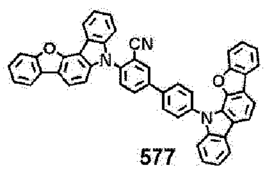 577 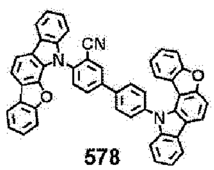 578 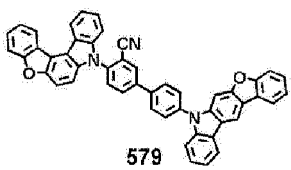 579 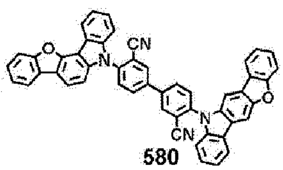 580

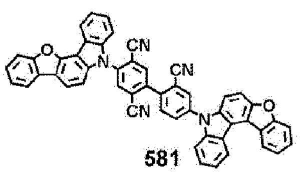 581 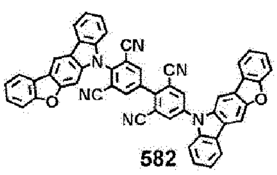 582 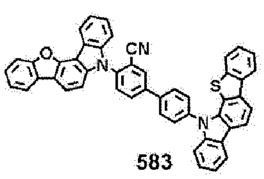 583 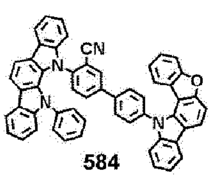 584

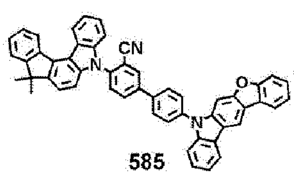 585 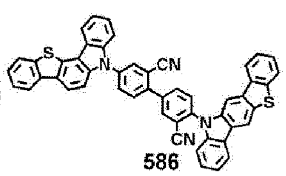 586 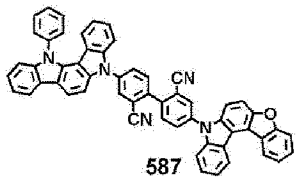 587 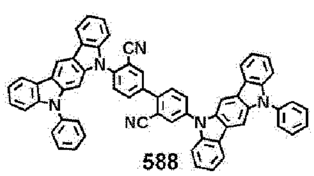 588

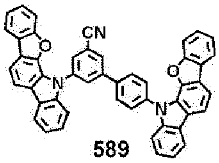
589
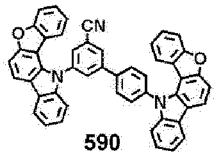
590
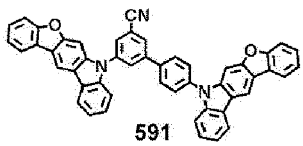
591
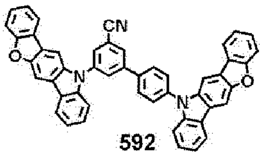
592

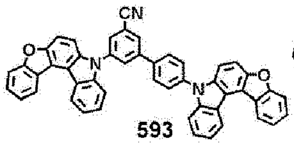
593
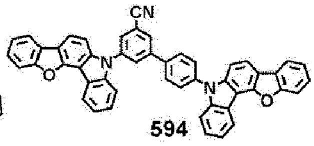
594
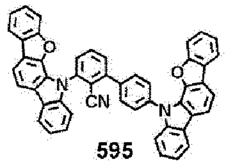
595
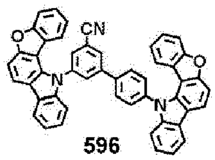
596

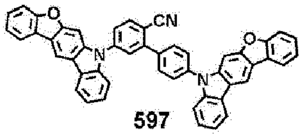
597
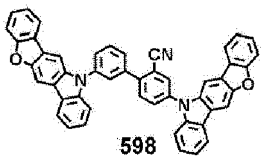
598
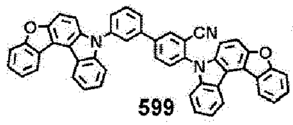
599
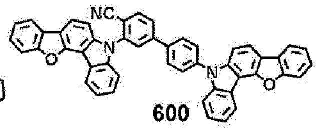
600

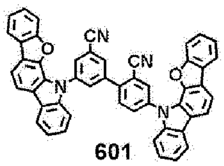
601
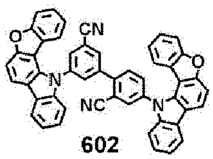
602
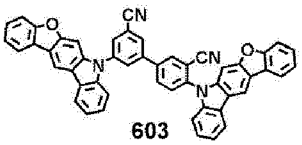
603
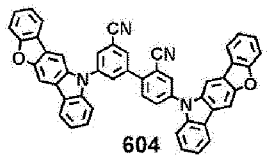
604

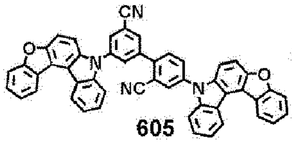
605
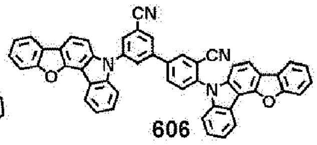
606
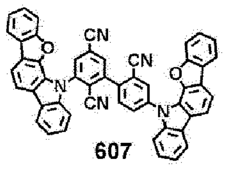
607
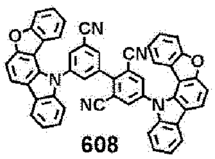
608

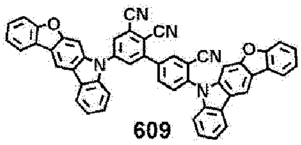
609
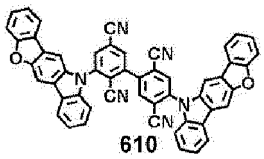
610
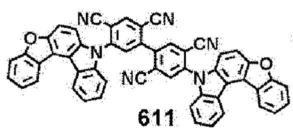
611
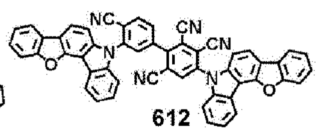
612

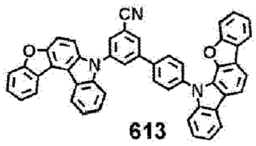
613
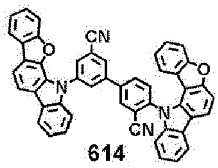
614
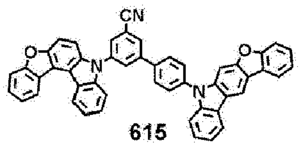
615
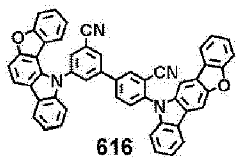
616

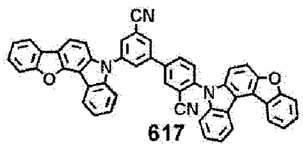
617
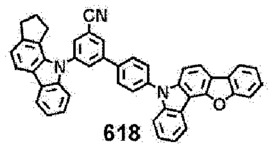
618
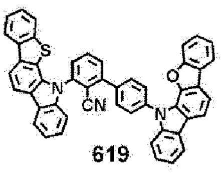
619
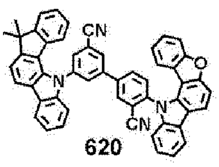
620

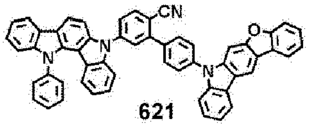
621
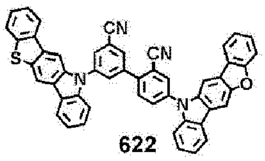
622
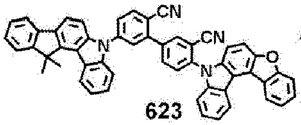
623
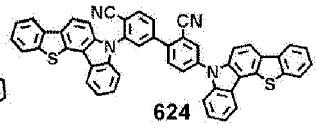
624

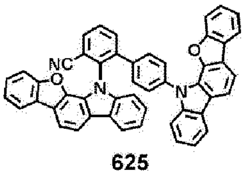
625
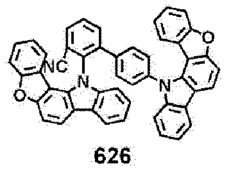
626
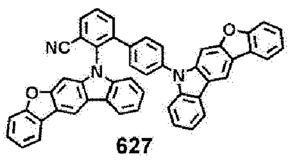
627
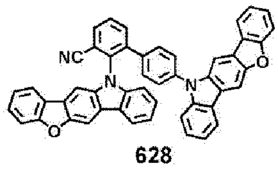
628

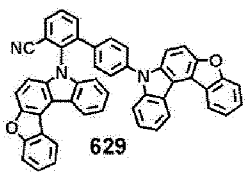
629
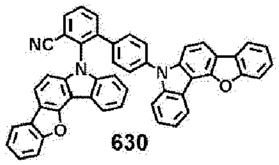
630
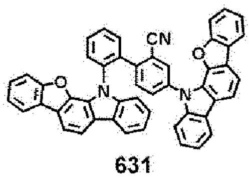
631
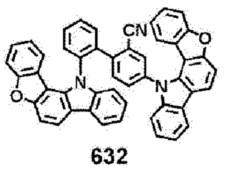
632

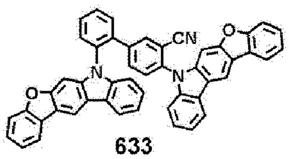
633
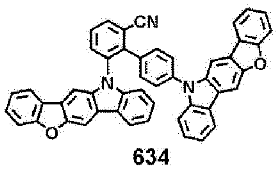
634
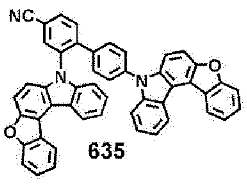
635
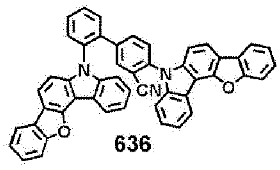
636

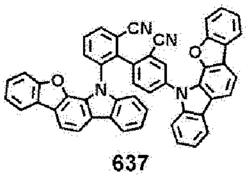
637
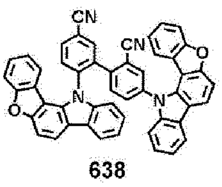
638
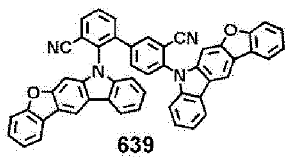
639
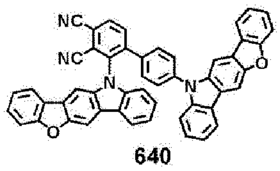
640

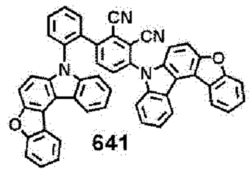
641
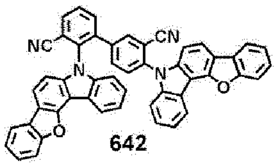
642
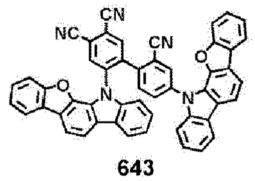
643
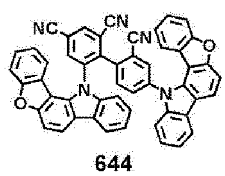
644

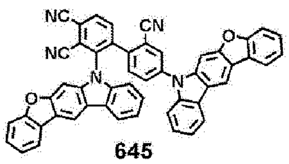
645
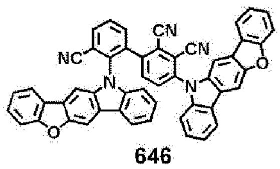
646
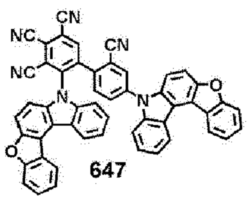
647
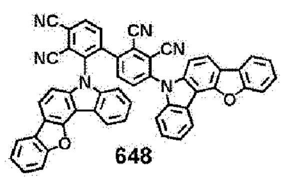
648

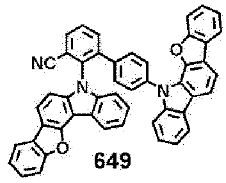
649
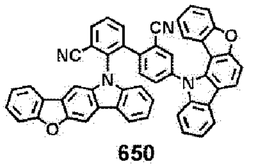
650
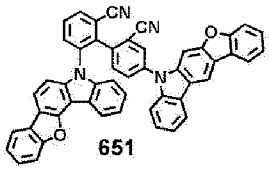
651
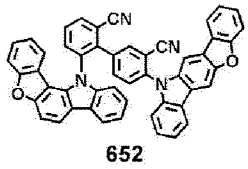
652

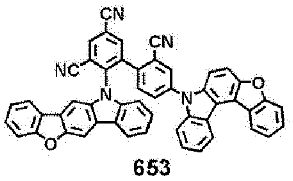
653
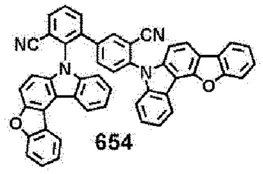
654
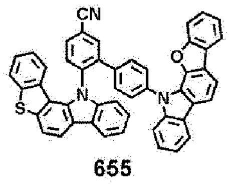
655
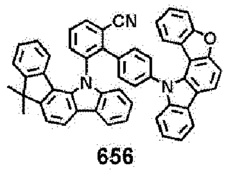
656

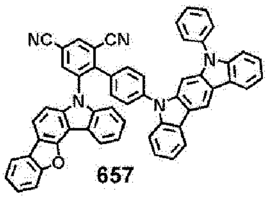
657
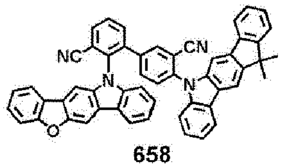
658
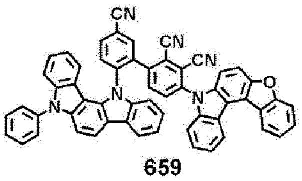
659
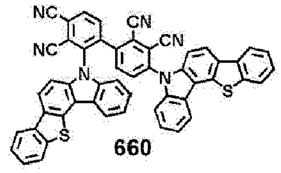
660

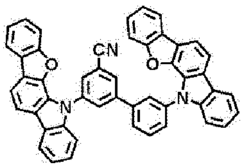
661
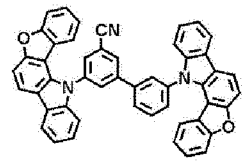
662
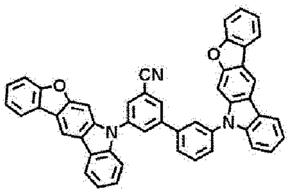
663
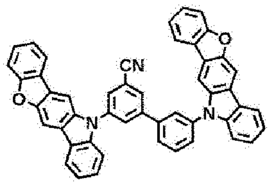
664

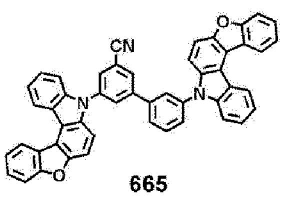
665
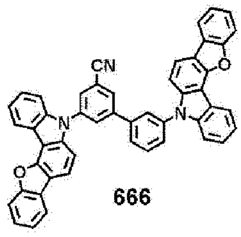
666
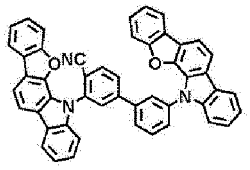
667
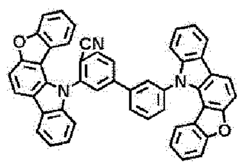
668

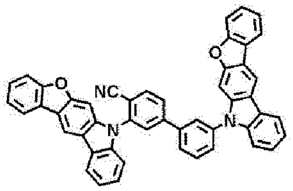
669
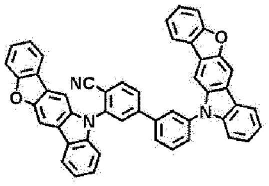
670
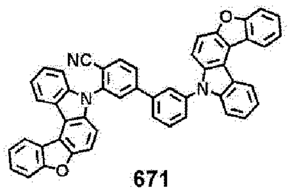
671
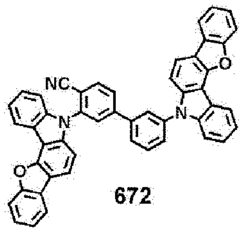
672

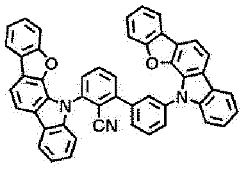
673

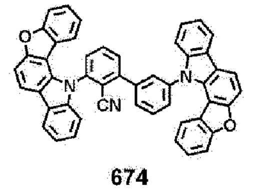
674

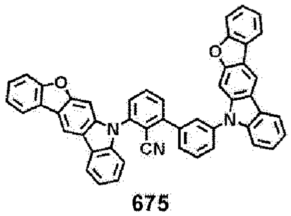
675

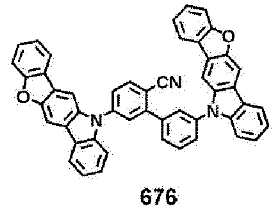
676

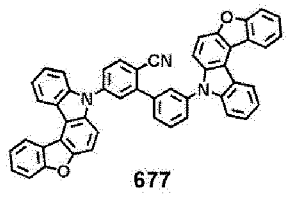
677

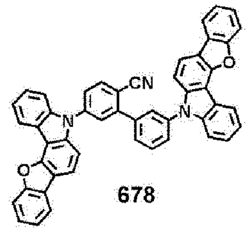
678

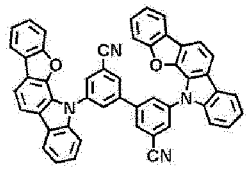
679

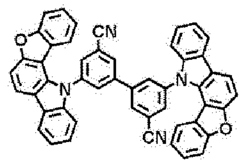
680

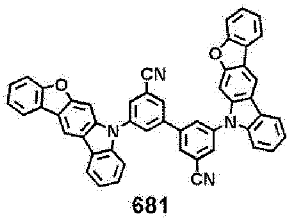
681

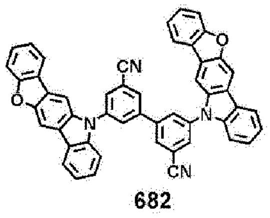
682

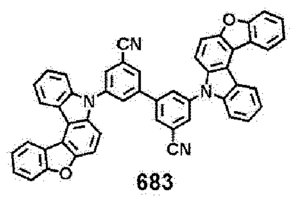
683

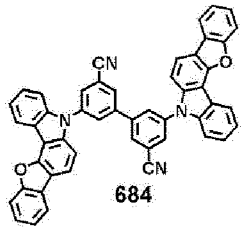
684

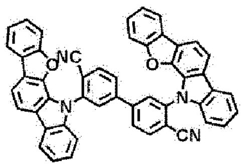
685

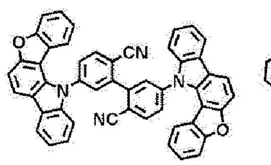
686

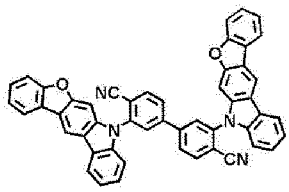
687

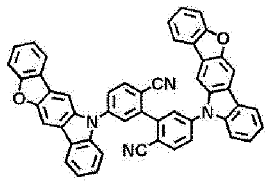
688

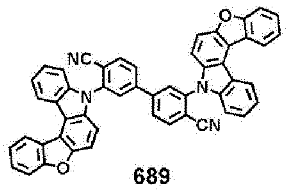
689

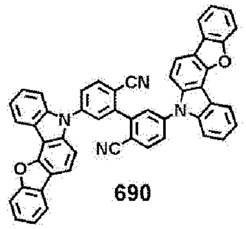
690

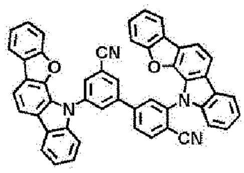
691

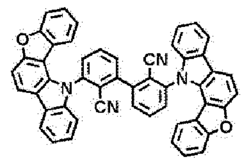
692

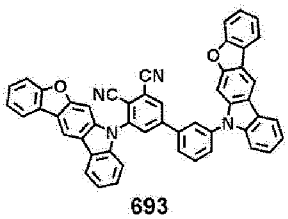
693

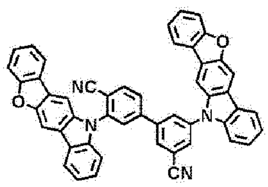
694

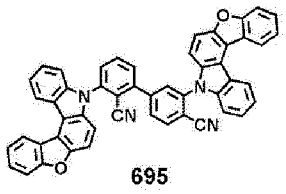
695

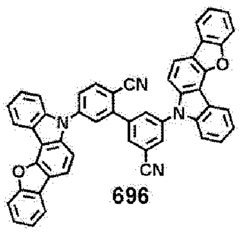
696

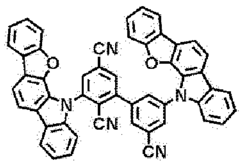
697

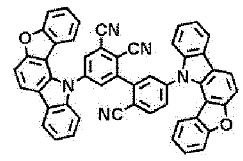
698

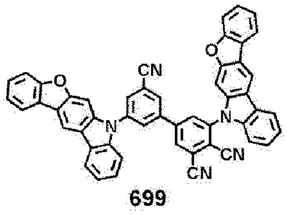
699

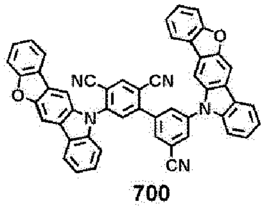
700

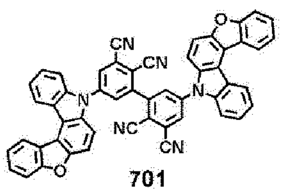
701

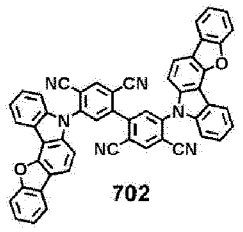
702

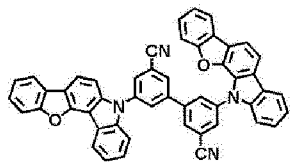
703

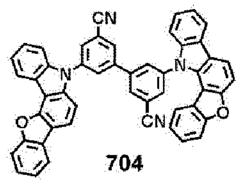
704

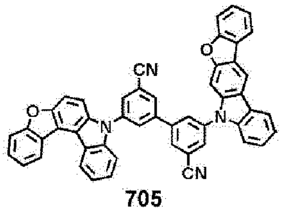
705

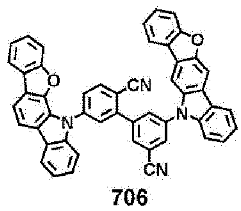
706

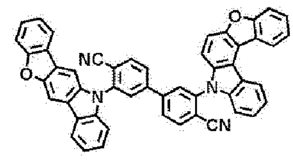
707

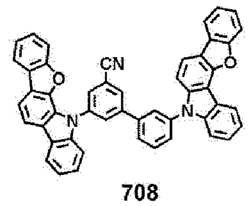
708

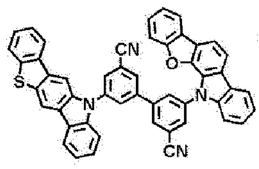
709

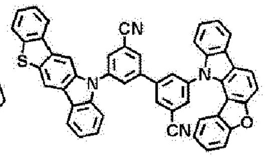
710

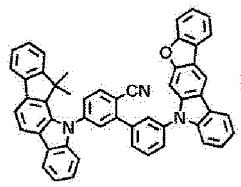
711

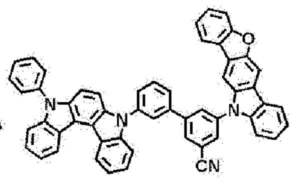
712

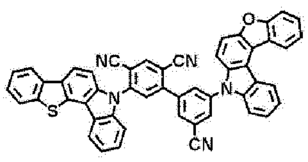
713

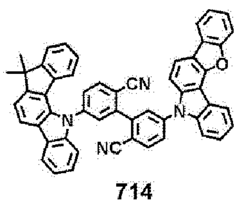
714

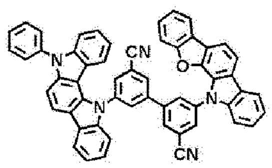
715

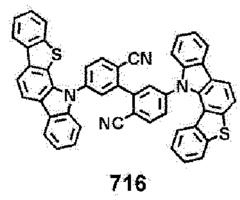
716

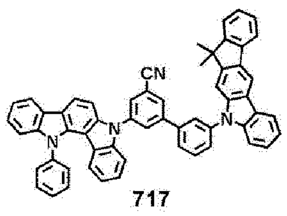
717

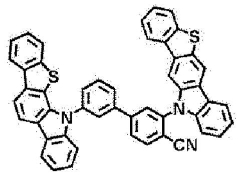
718

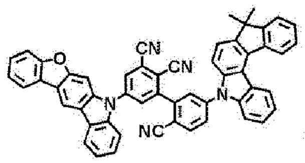
719

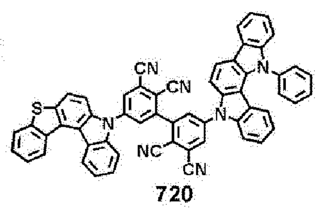
720

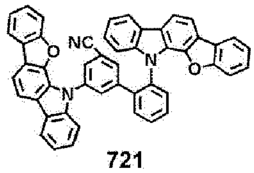
721
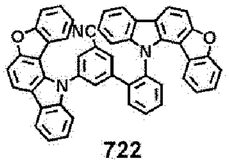
722
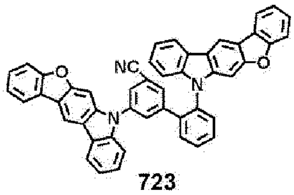
723
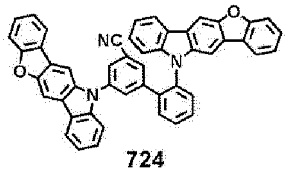
724

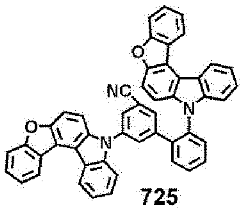
725
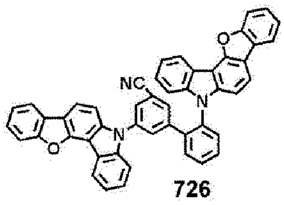
726
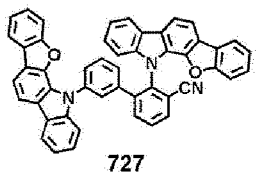
727
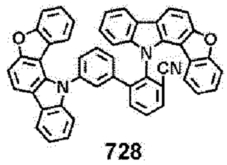
728

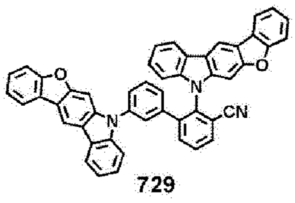
729
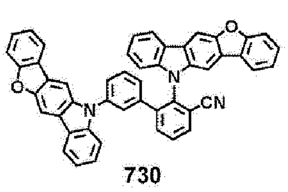
730
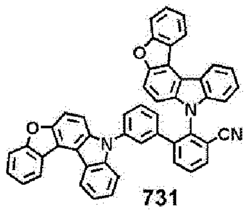
731
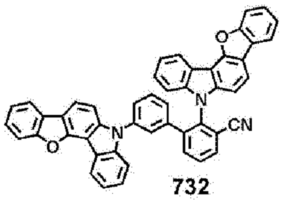
732

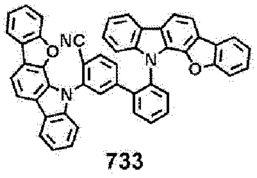
733
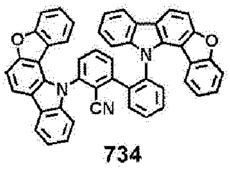
734
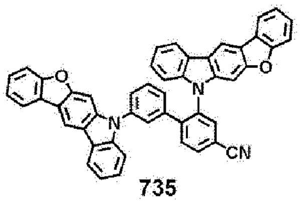
735
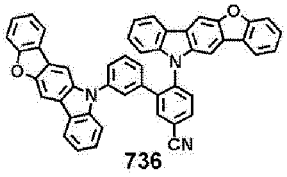
736

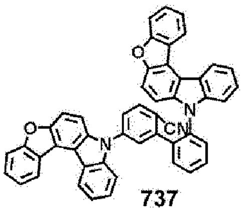
737
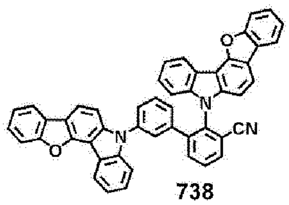
738
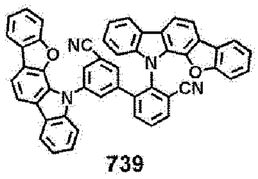
739
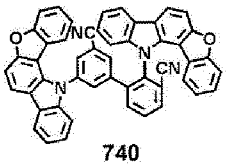
740

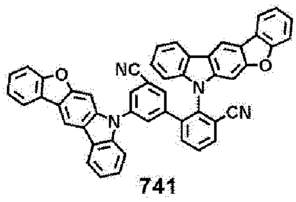
741
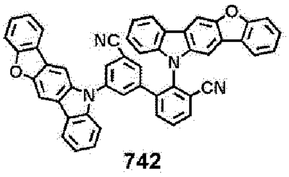
742
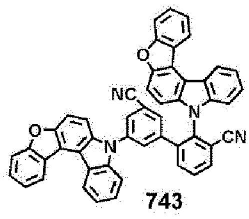
743
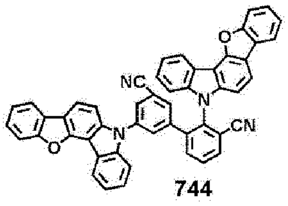
744

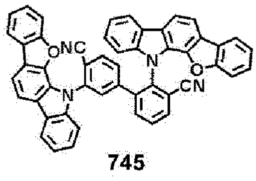
745

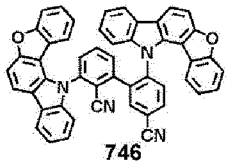
746

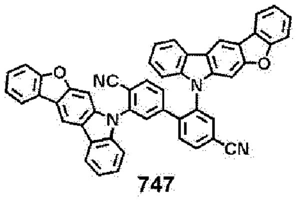
747

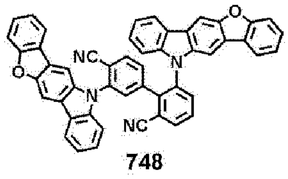
748

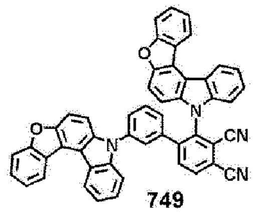
749

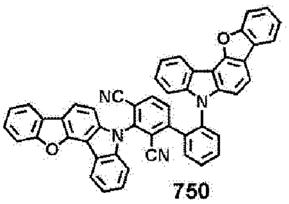
750

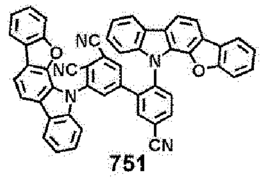
751

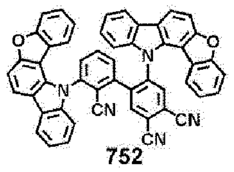
752

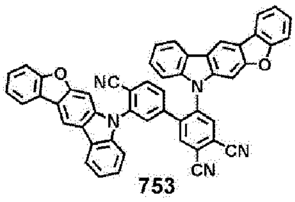
753

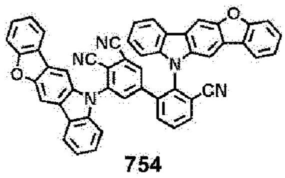
754

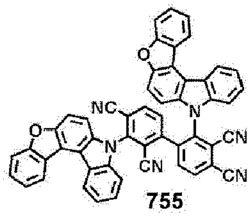
755

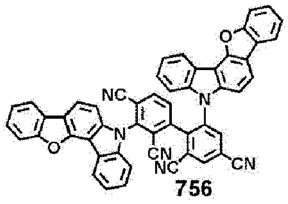
756

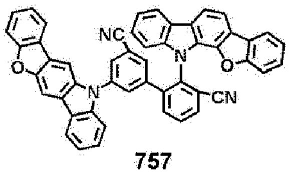
757

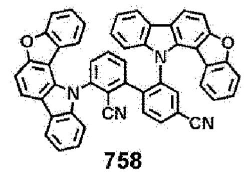
758

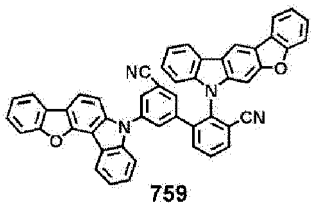
759

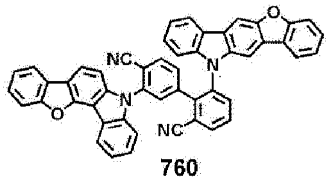
760

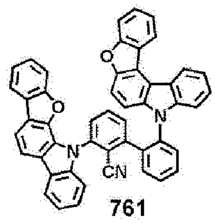
761

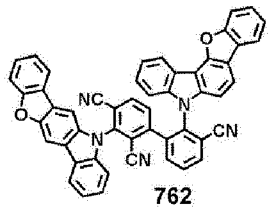
762

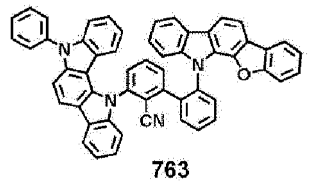
763

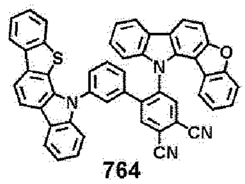
764

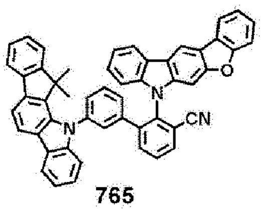
765

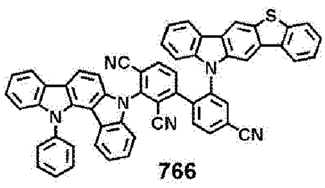
766

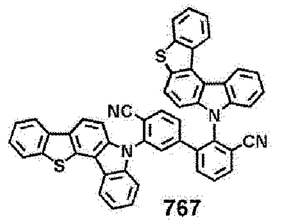
767

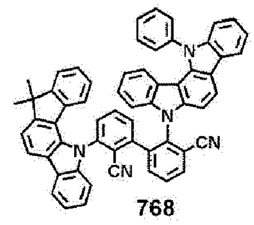
768

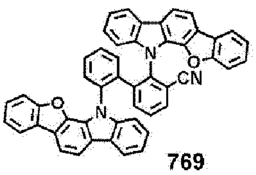
769

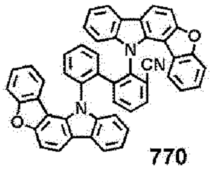
770

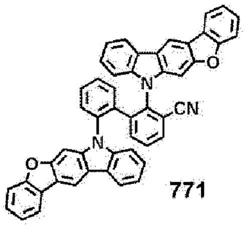
771

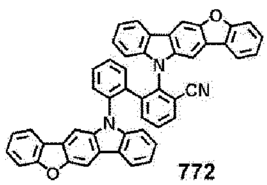
772

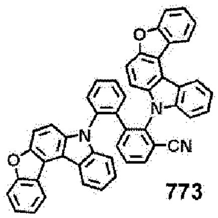
773

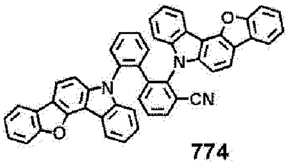
774

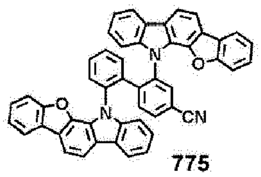
775

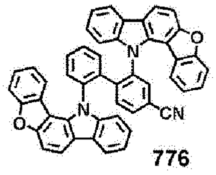
776

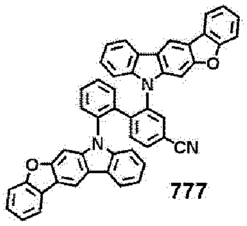
777

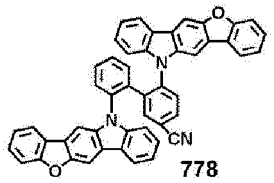
778

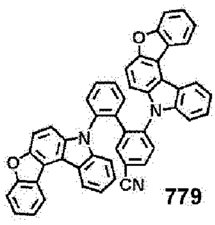
779

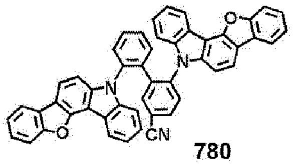
780

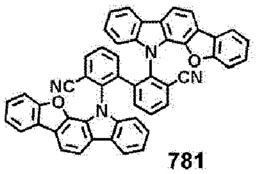
781

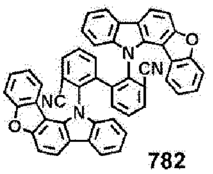
782

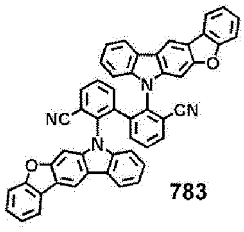
783

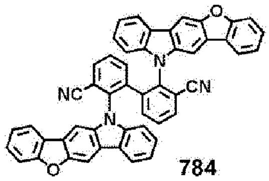
784

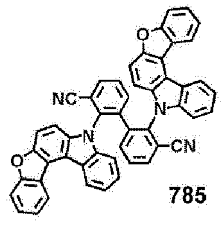
785

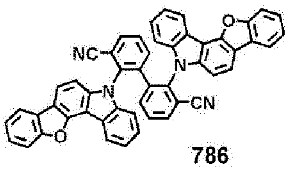
786

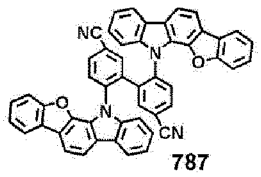
787

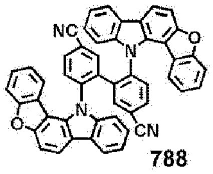
788

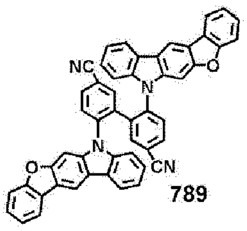
789

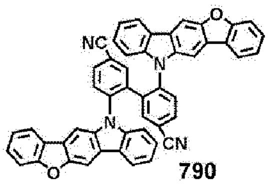
790

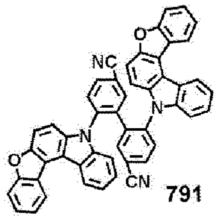
791

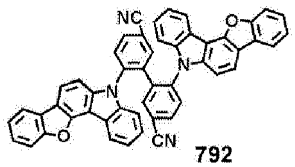
792

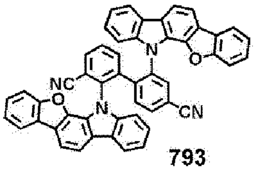
793
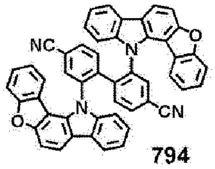
794
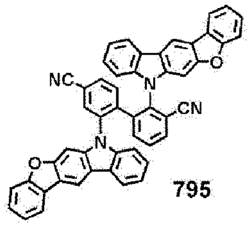
795
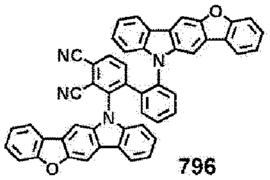
796

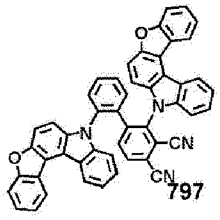
797
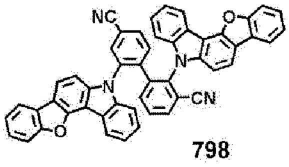
798
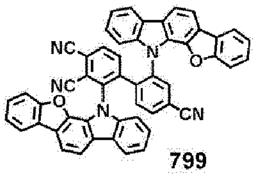
799
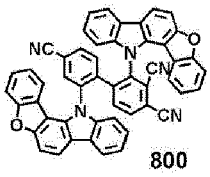
800

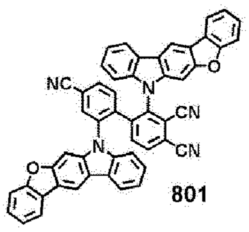
801
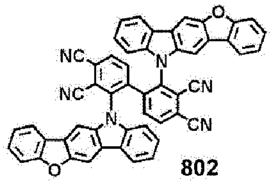
802
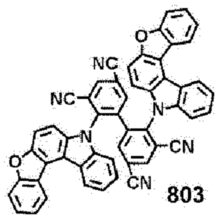
803
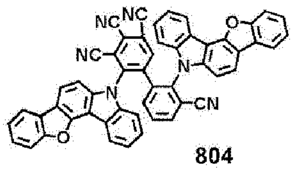
804

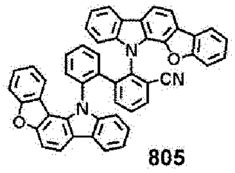
805
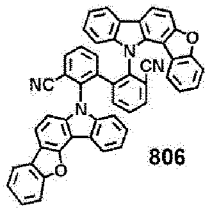
806
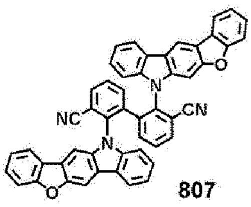
807
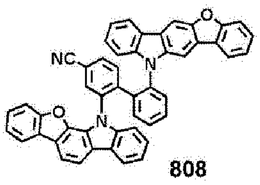
808

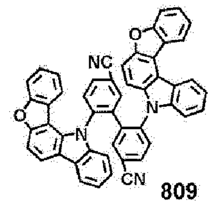
809
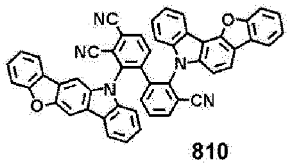
810
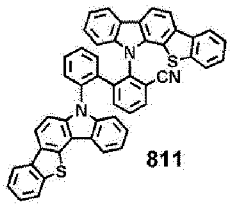
811
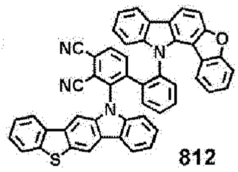
812

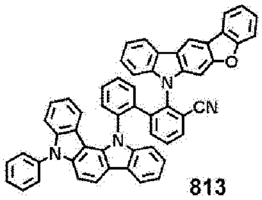
813
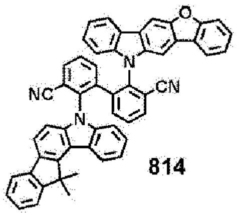
814
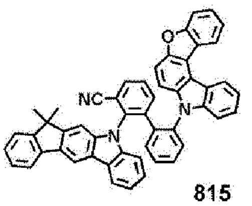
815
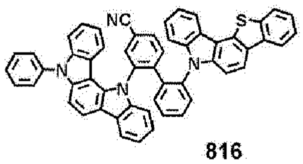
816

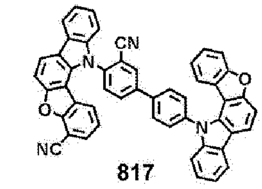
817

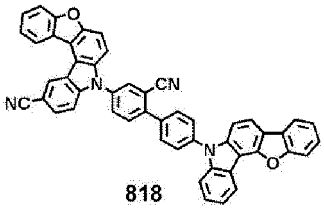
818

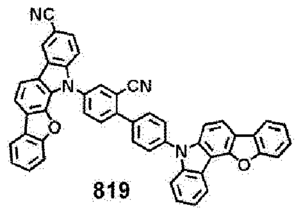
819

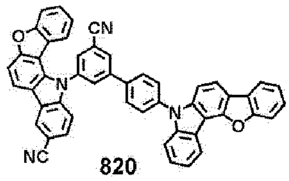
820

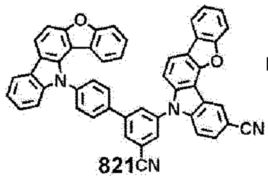
821

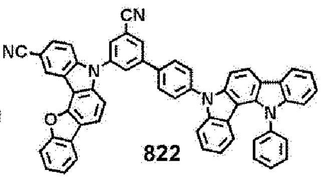
822

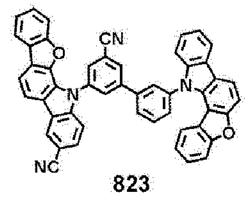
823

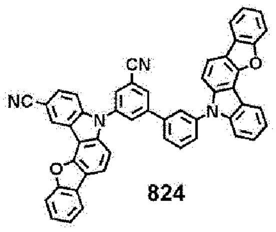
824

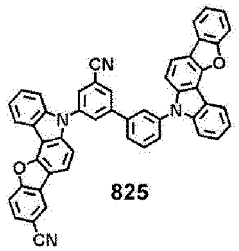
825

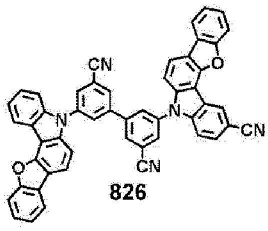
826

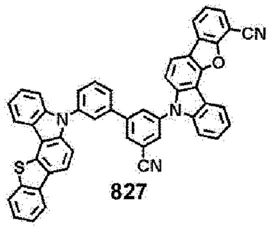
827

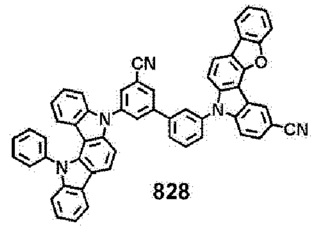
828

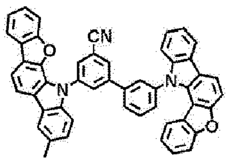
829

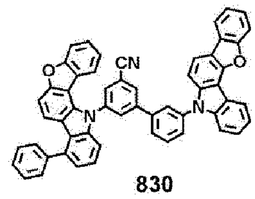
830

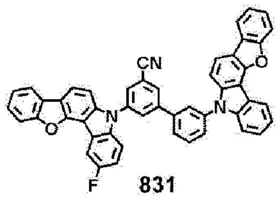
831

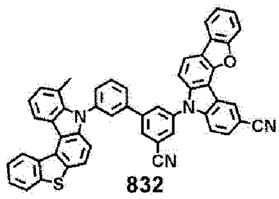
832

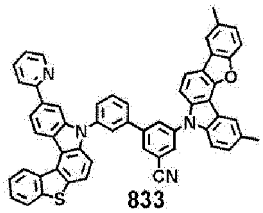
833

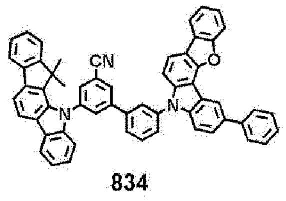
834

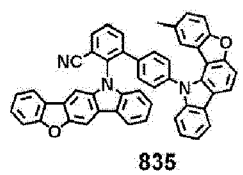
835

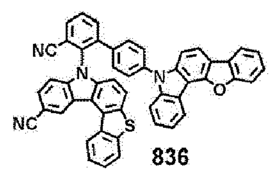
836

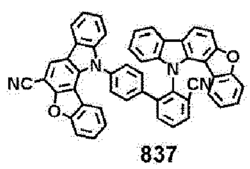
837

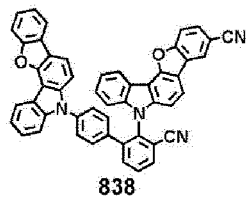
838

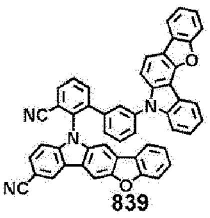
839

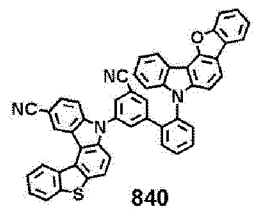
840

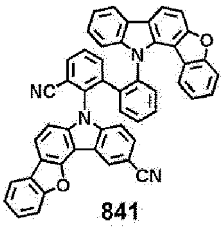
841

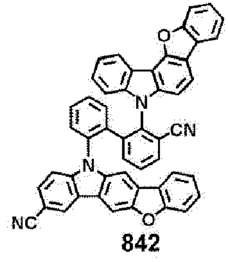
842

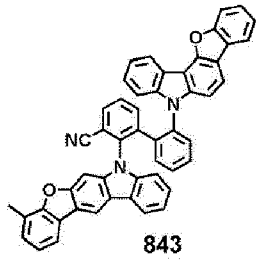
843

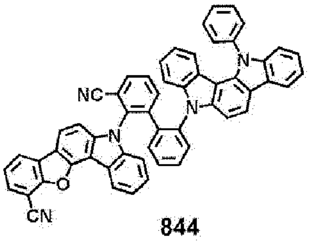
844

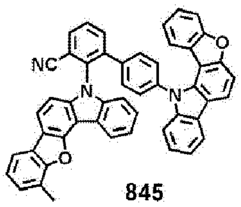
845

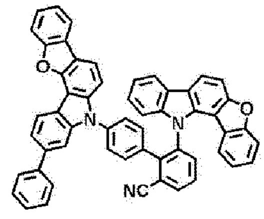
846

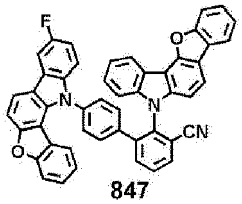
847

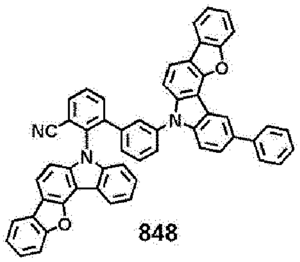
848

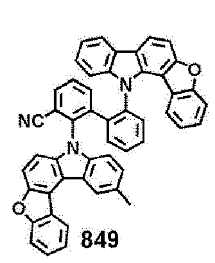
849

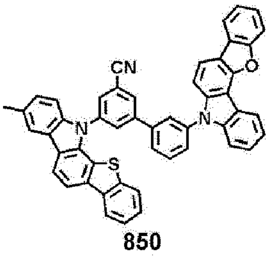
850

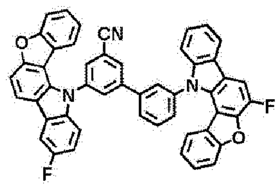
851

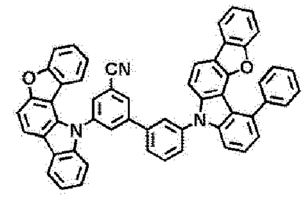
852

## Group HE3

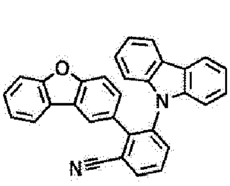
1

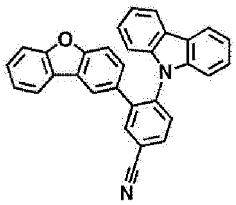
2

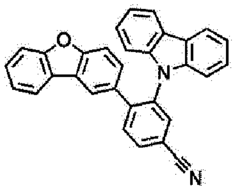
3

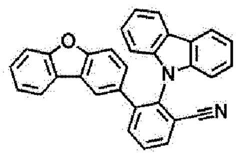
4

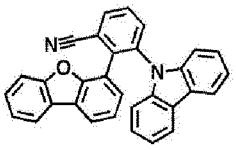
5

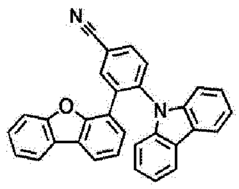
6

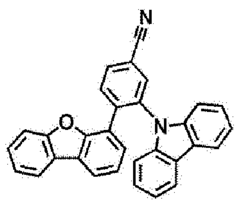
7

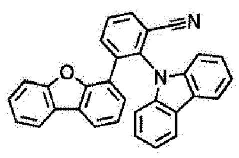
8

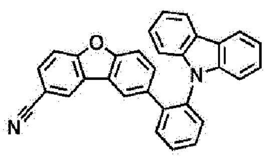
9

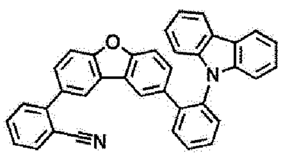
10

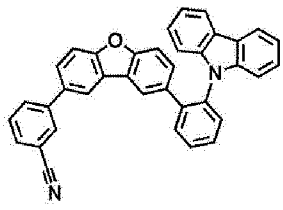
11

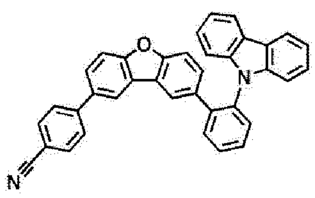
12

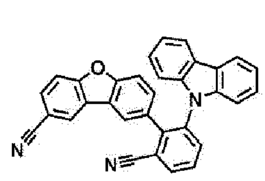
13

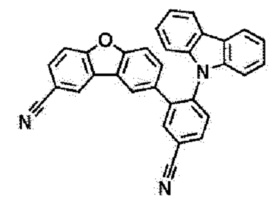
14

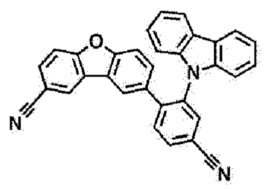
15

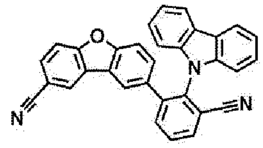
16

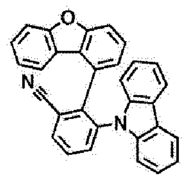
17

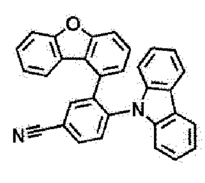
18

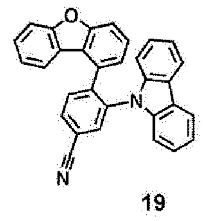
19

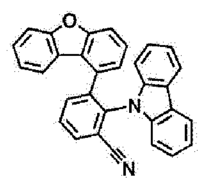
20

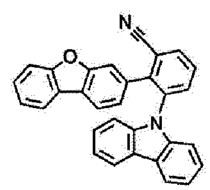
21

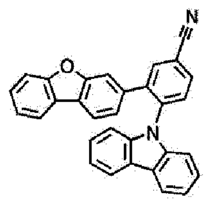
22

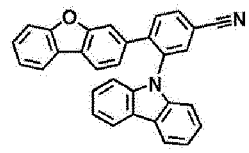
23

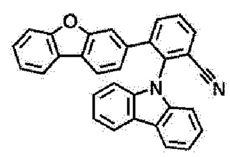
24

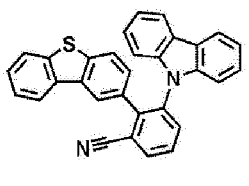
25

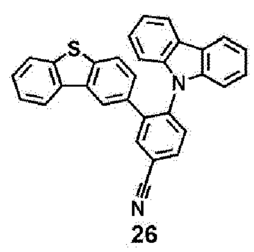
26

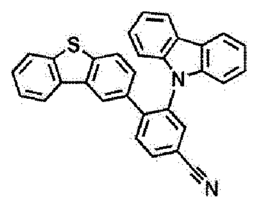
27

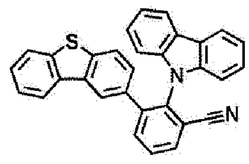
28

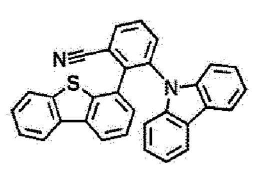
29

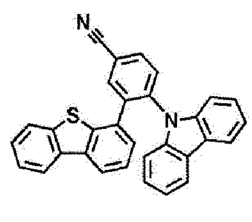
30

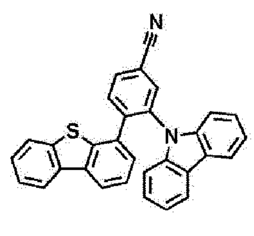
31

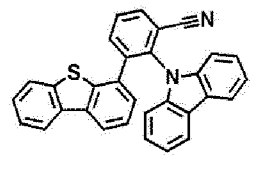
32

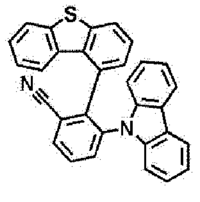
33

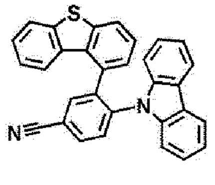
34

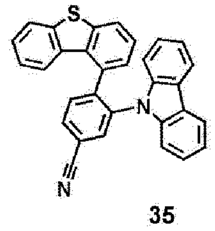
35

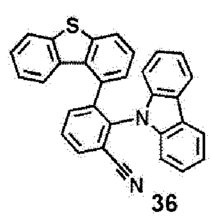
36

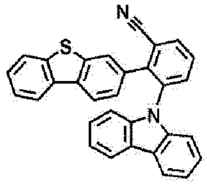
37

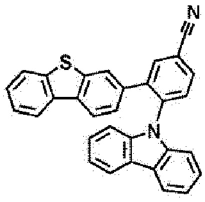
38

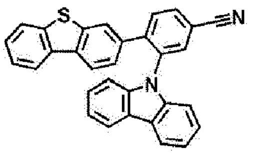
39

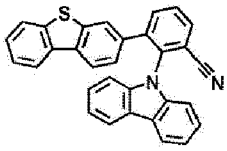
40

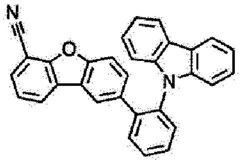
41

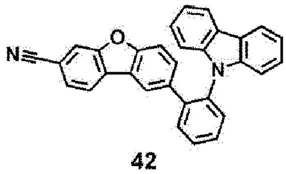
42

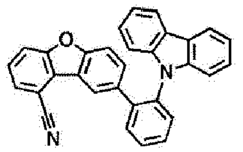
43

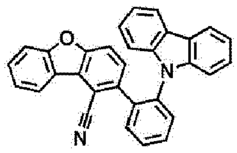
44

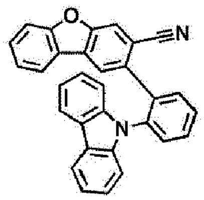
45

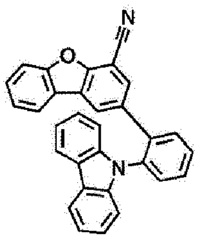
46

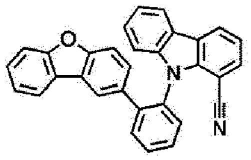
47

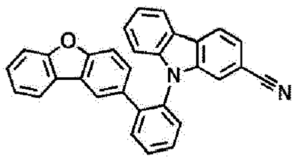
48

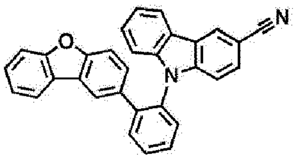
49

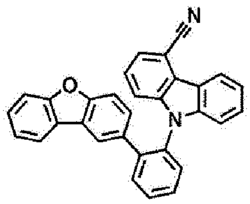
50

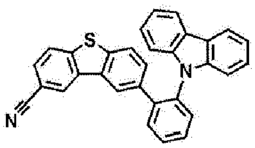
51

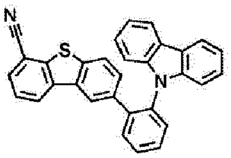
52

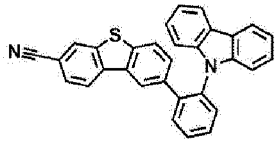
53

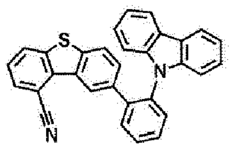
54

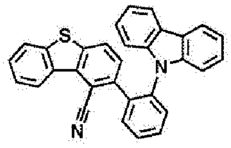
55

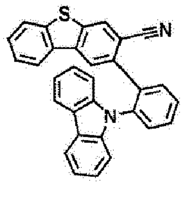
56

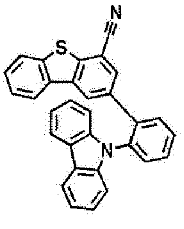
57

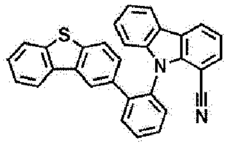
58

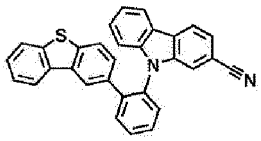

59

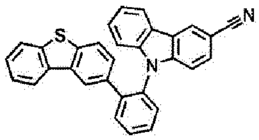

60

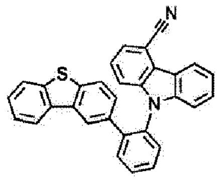

61

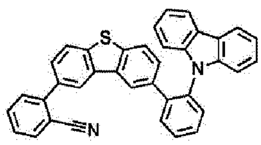

62

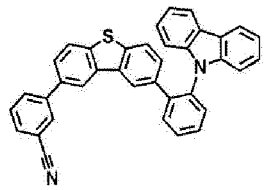

63

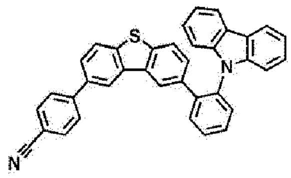

64

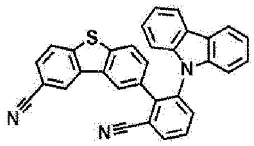

65

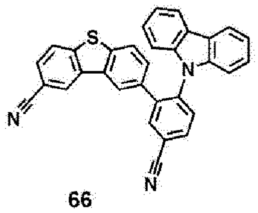

66

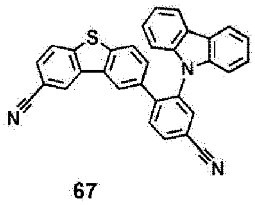

67

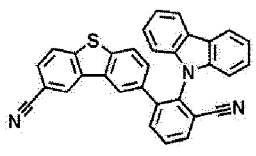

68

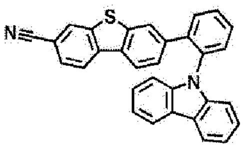

69

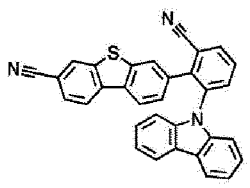

70

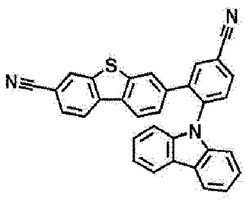

71

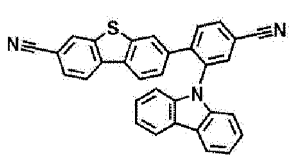

72

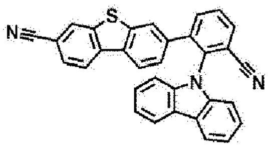

73

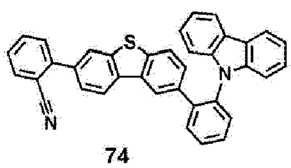

74

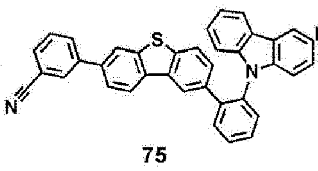

75

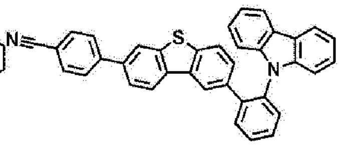

76

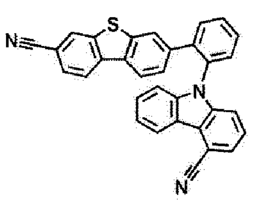

77

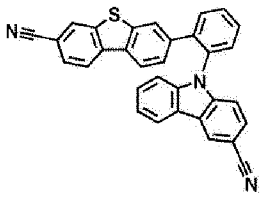

78

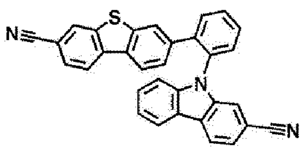

79

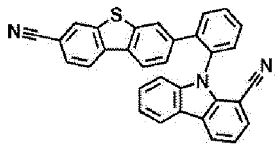

80

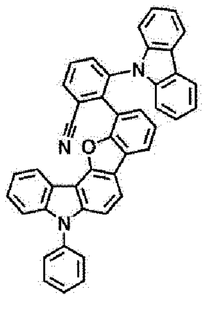

81

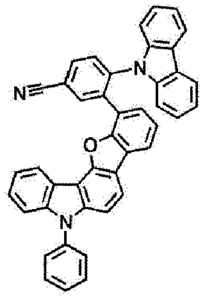

82

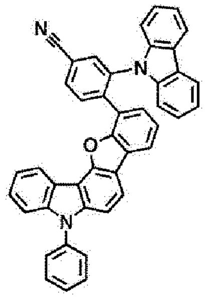

83

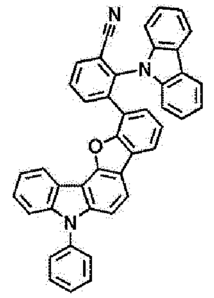

84

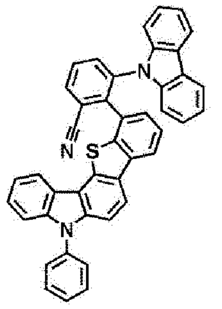

85

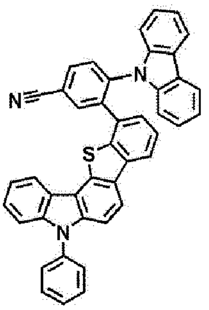

86

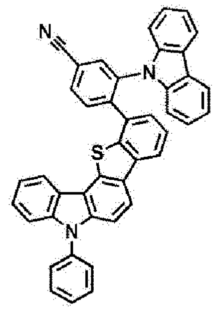

87

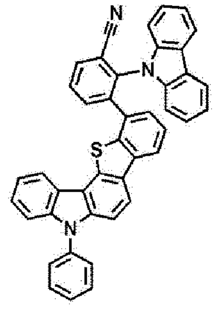

88

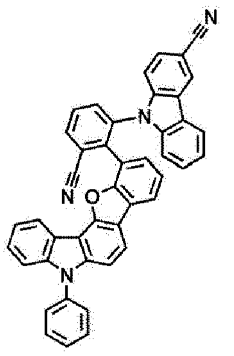

89

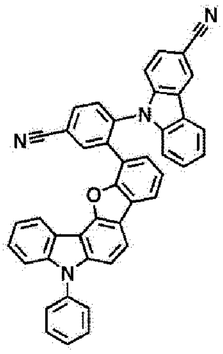

90

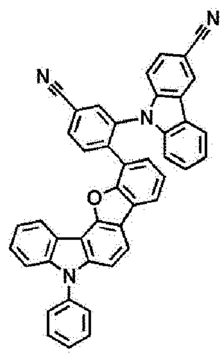

91

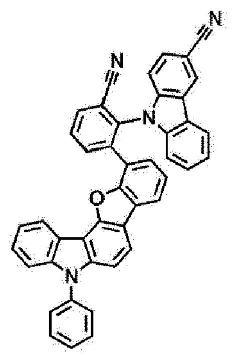

92

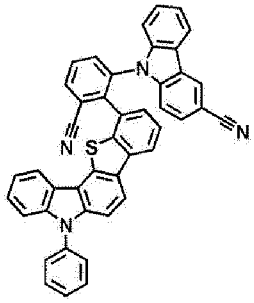

**93**

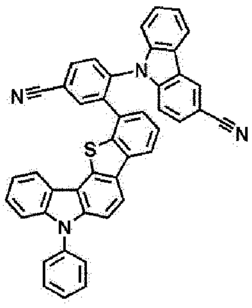

**94**

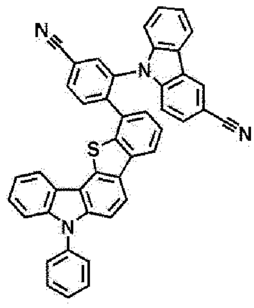

**95**

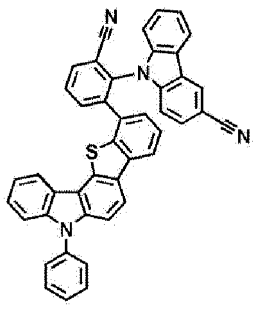

**96**

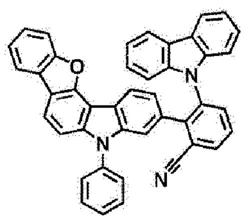

**97**

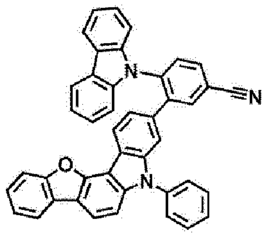

**98**

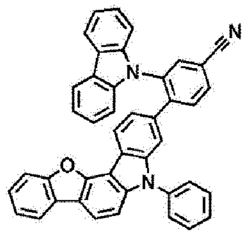

**99**

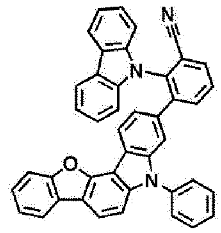

**100**

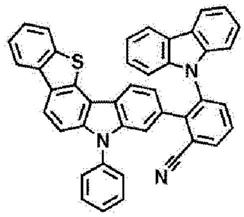

**101**

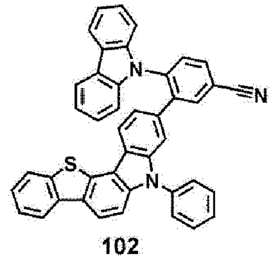

**102**

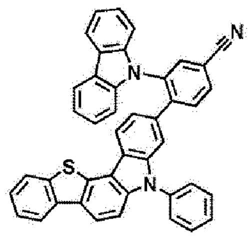

**103**

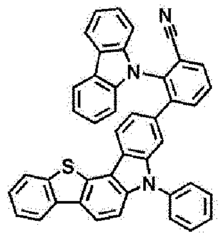

**104**

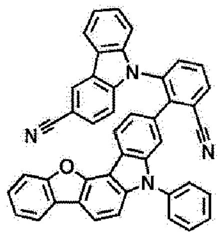

**105**

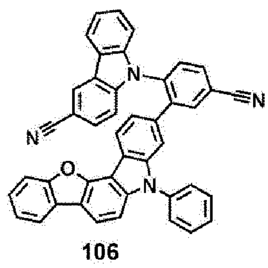

**106**

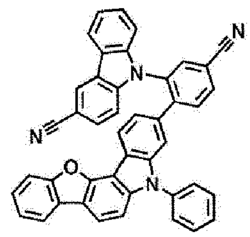

**107**

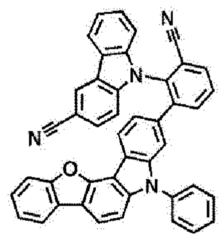

**108**

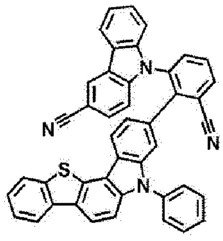
109

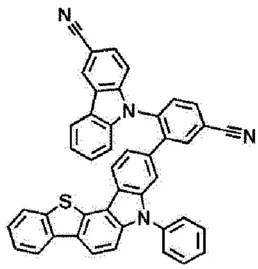
110

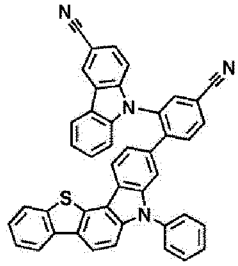
111

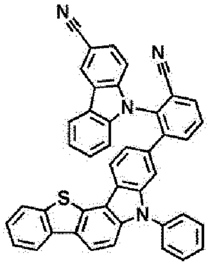
112

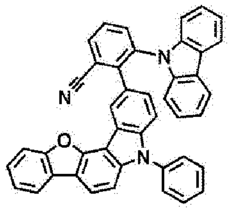
113

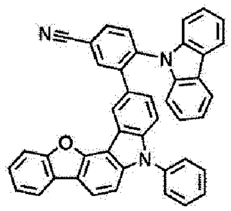
114

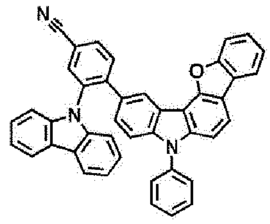
115

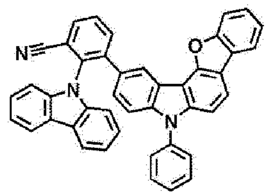
116

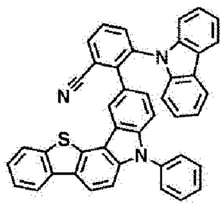
117

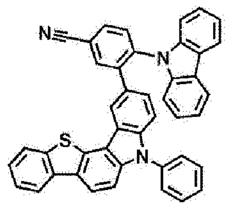
118

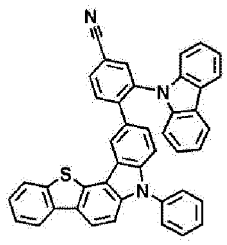
119

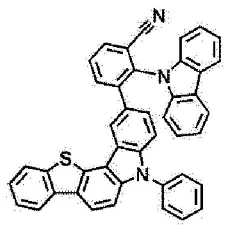
120

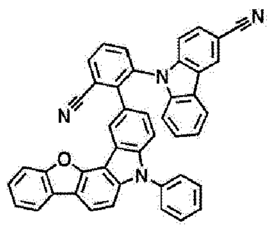
121

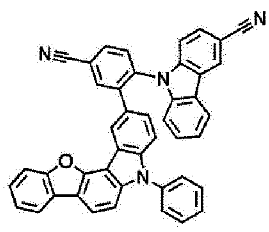
122

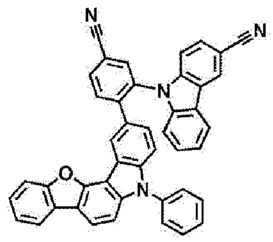
123

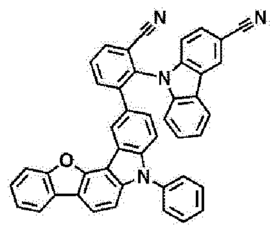
124

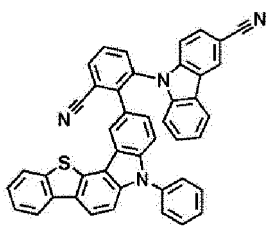
125

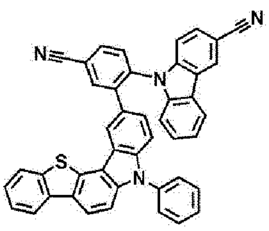
126

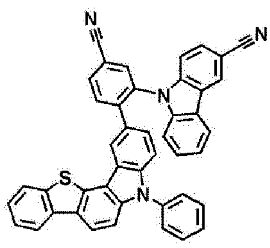
127

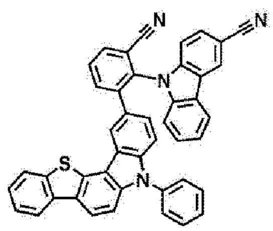
128

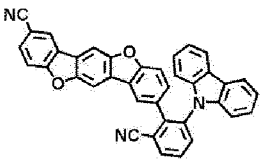
129

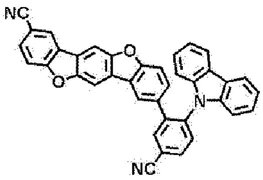
130

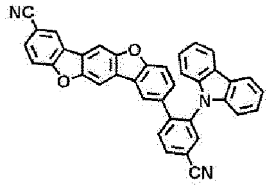
131

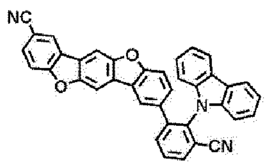
132

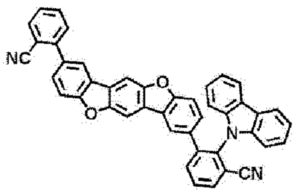
133

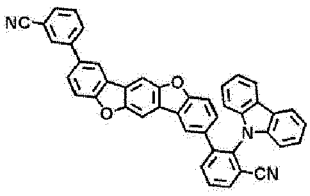
134

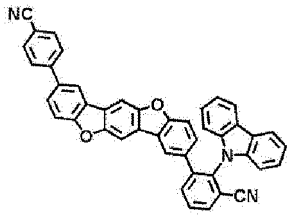
135

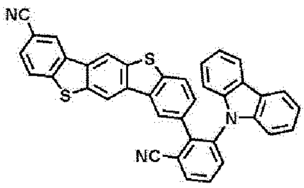
136

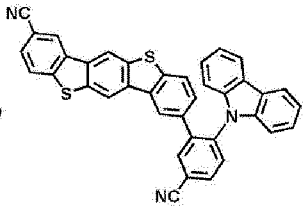
137

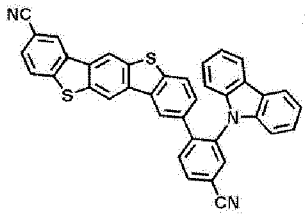
138

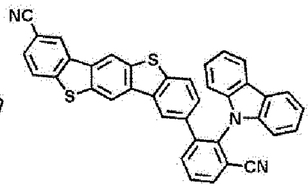
139

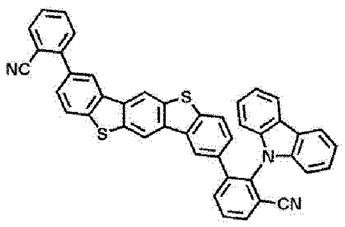
140

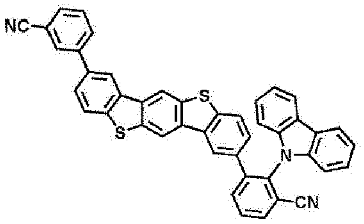
141

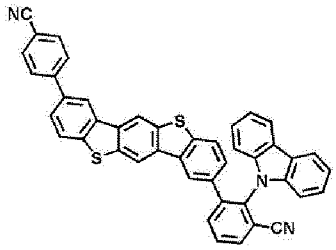
142

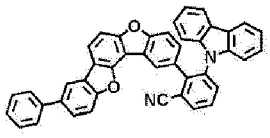
143

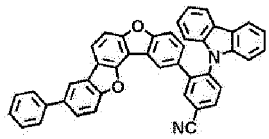
144

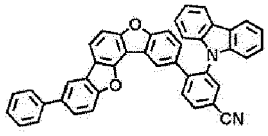
145

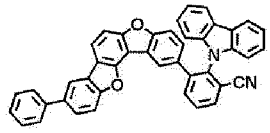
146

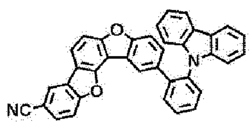
147

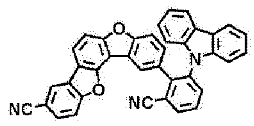
148

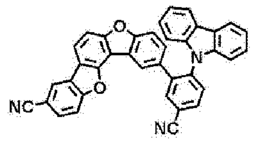
149

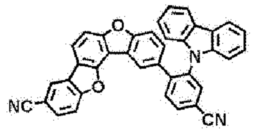
150

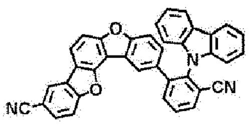
151

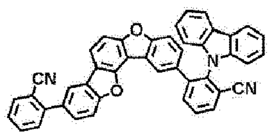
152

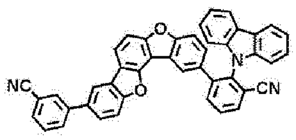
153

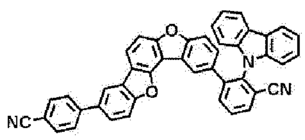
154

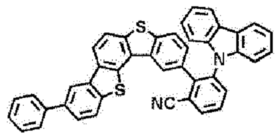
155

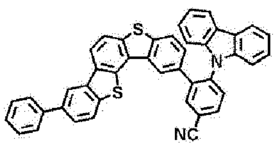
156

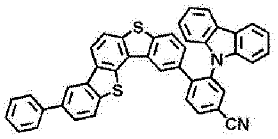
157

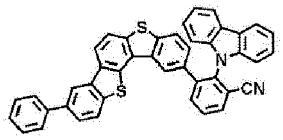
158

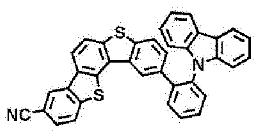
159

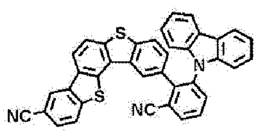
160

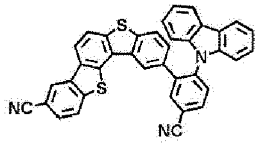
161

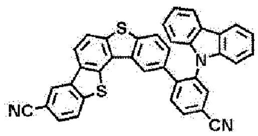
162

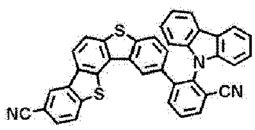
163

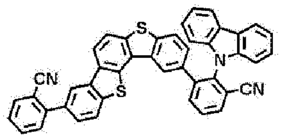
164

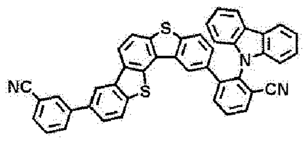
165

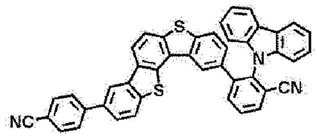
166

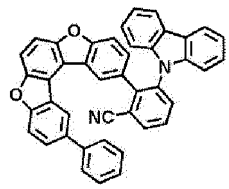
167
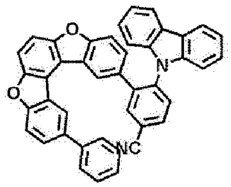
168
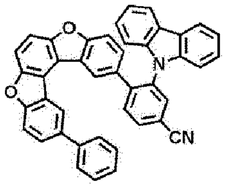
169
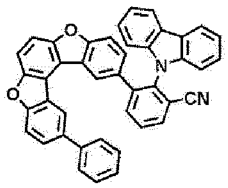
170

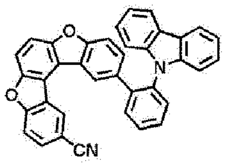
171
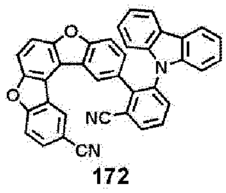
172
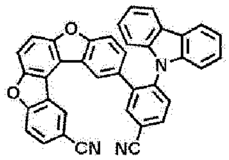
173
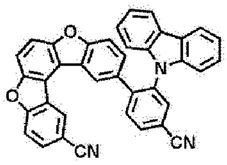
174

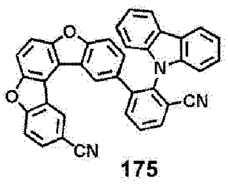
175
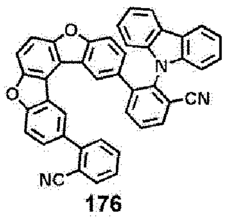
176
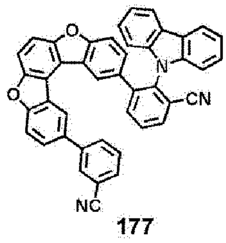
177
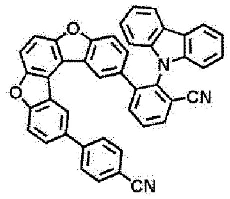
178

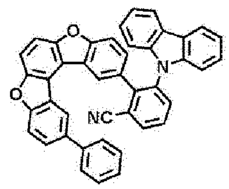
179
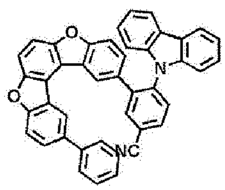
180
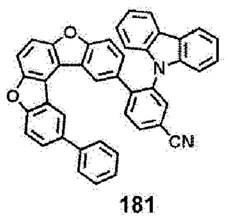
181
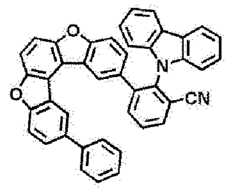
182

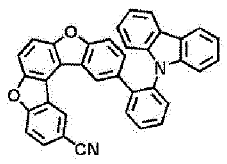
183
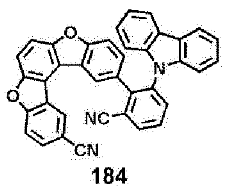
184
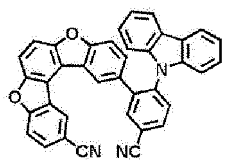
185
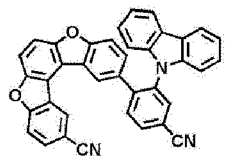
186

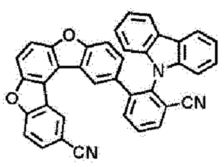
187
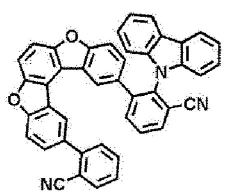
188
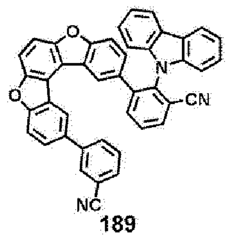
189
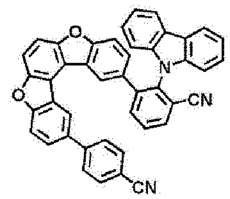
190

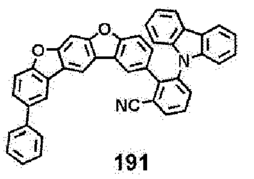
191

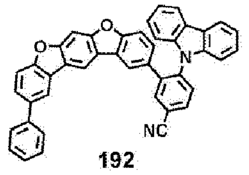
192

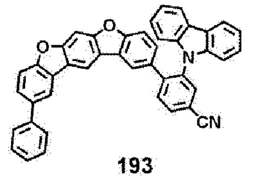
193

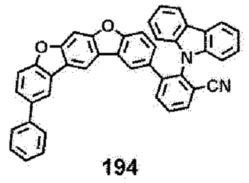
194

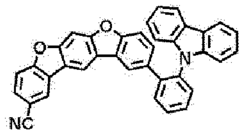
195

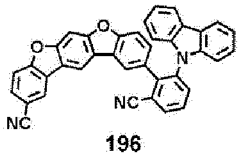
196

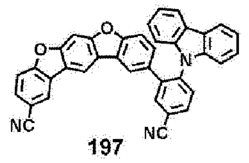
197

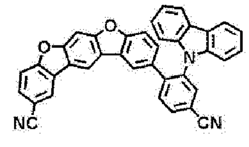
198

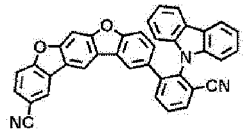
199

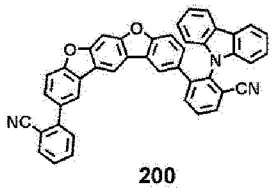
200

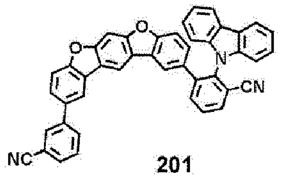
201

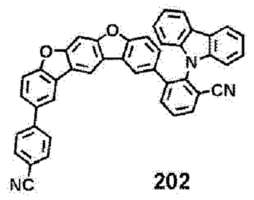
202

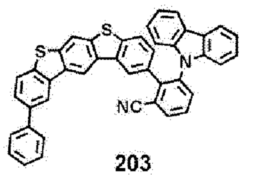
203

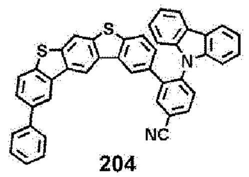
204

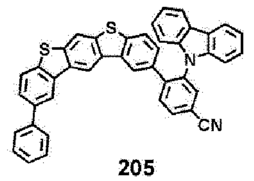
205

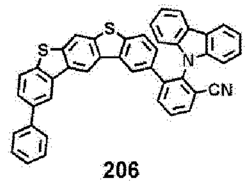
206

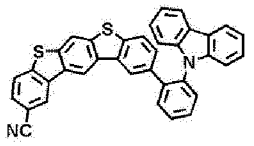
207

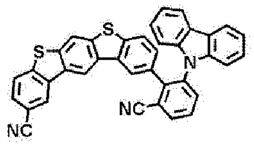
208

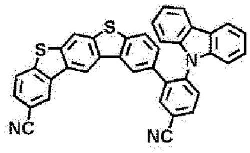
209

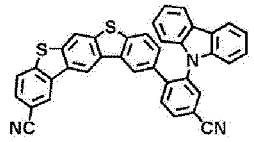
210

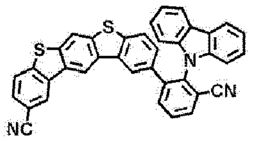
211

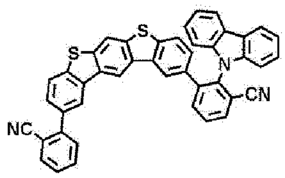
212

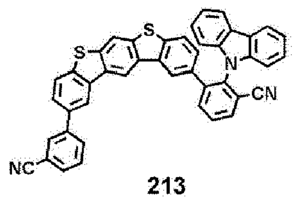
213

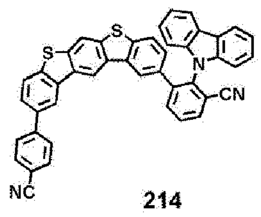
214

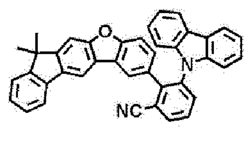
215

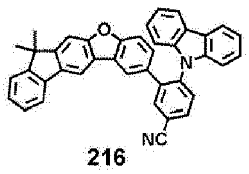
216

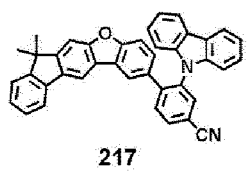
217

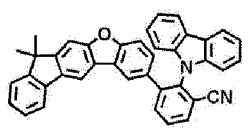
218

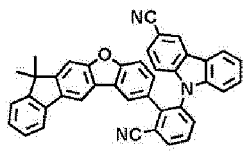
219

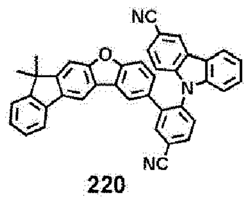
220

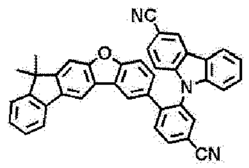
221

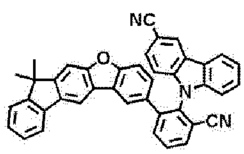
222

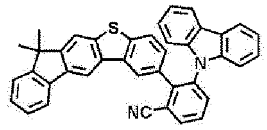

223

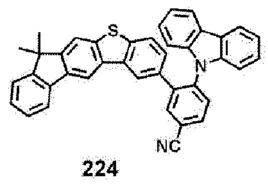

224

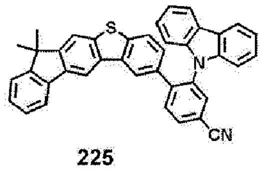

225

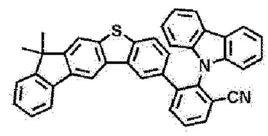

226

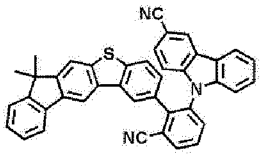

227

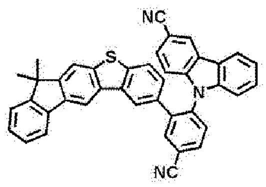

228

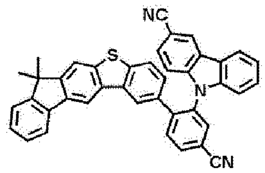

229

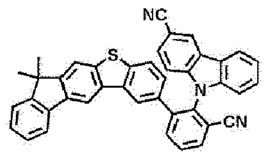

230

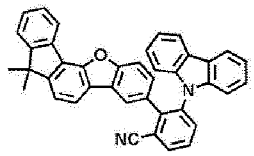

231

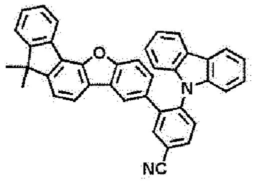

232

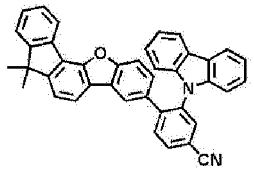

233

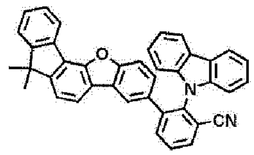

234

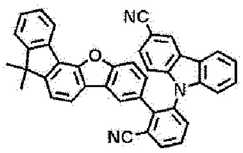

235

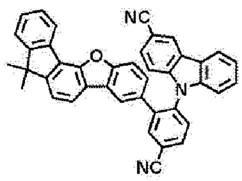

236

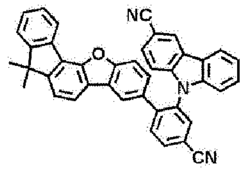

237

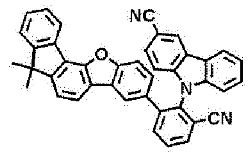

238

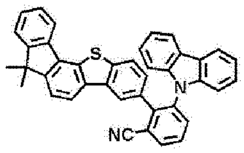

239

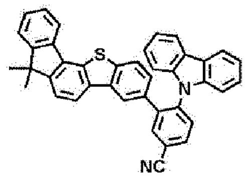

240

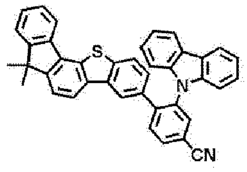

241

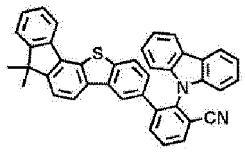

242

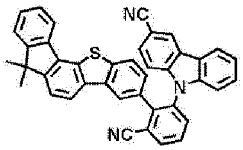

243

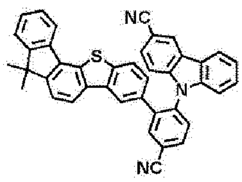

244

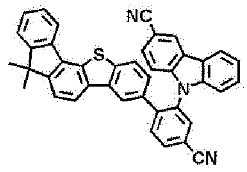

245

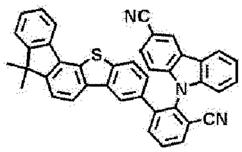

246

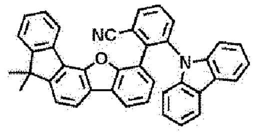
247
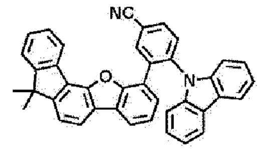
248
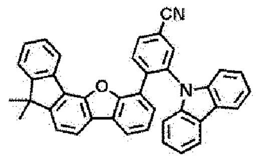
249
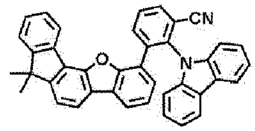
250

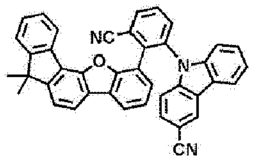
251
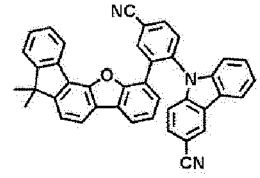
252
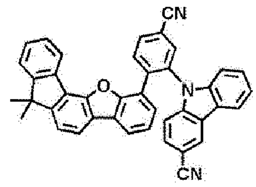
253
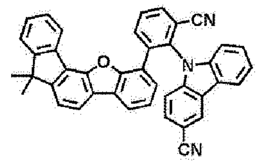
254

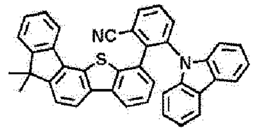
255
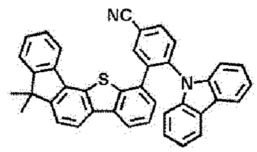
256
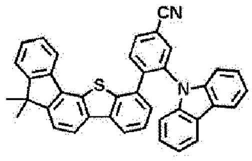
257
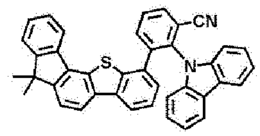
258

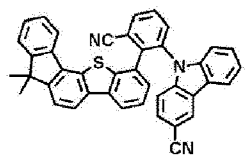
259
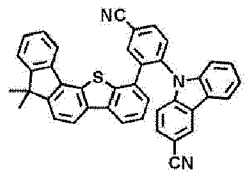
260
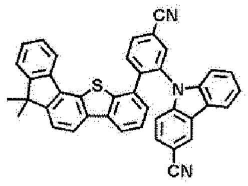
261
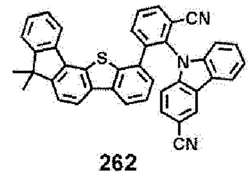
262

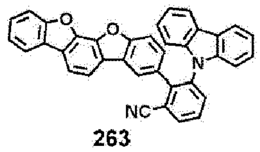
263
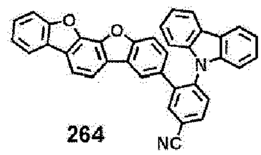
264
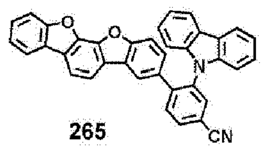
265
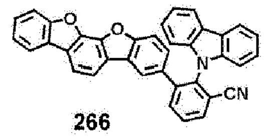
266

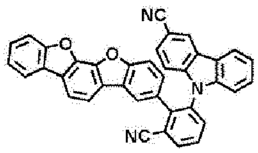
267
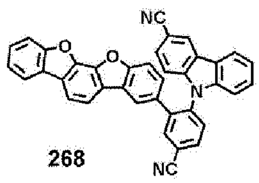
268
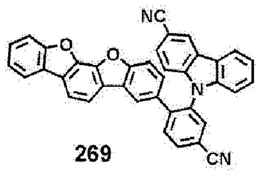
269
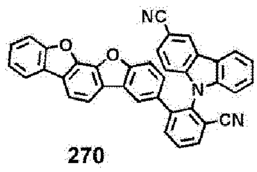
270

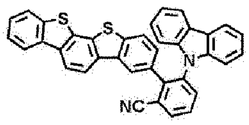
271

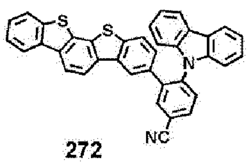
272

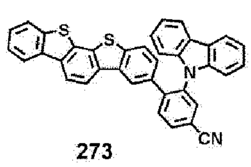
273

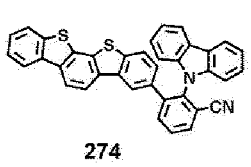
274

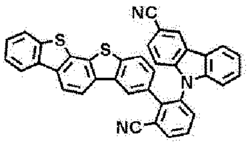
275

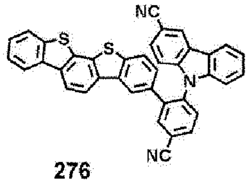
276

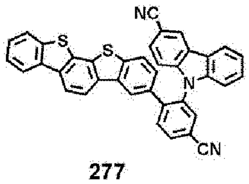
277

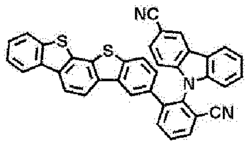
278

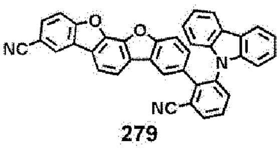
279

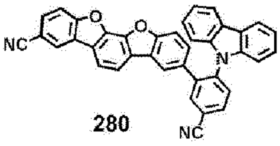
280

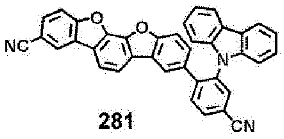
281

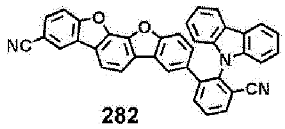
282

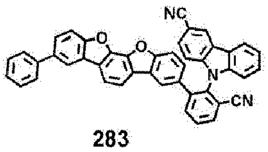
283

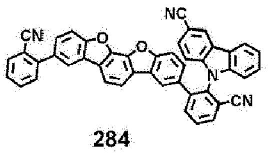
284

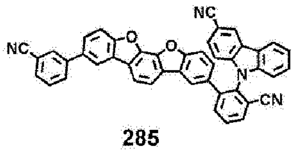
285

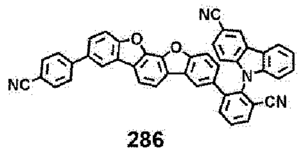
286

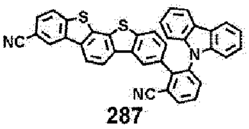
287

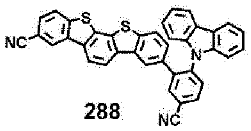
288

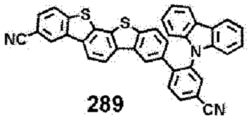
289

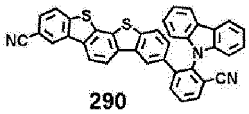
290

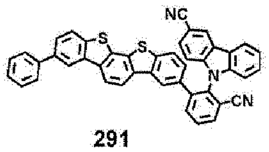
291

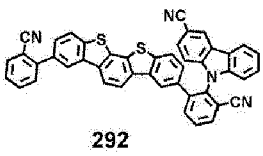
292

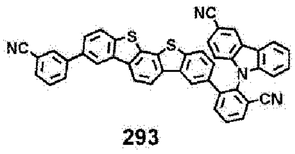
293

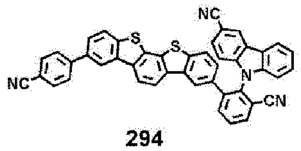
294

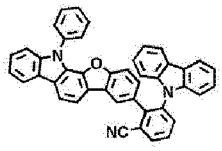
295

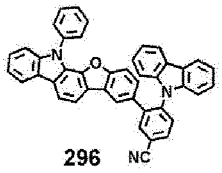
296

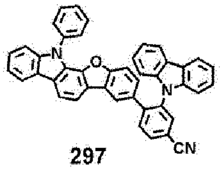
297

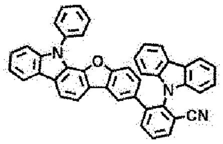
298

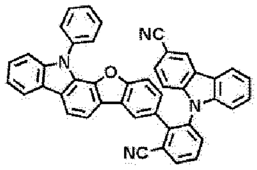

299

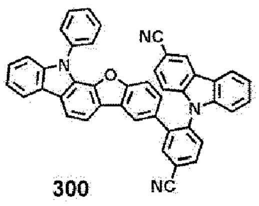

300

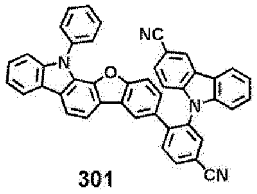

301

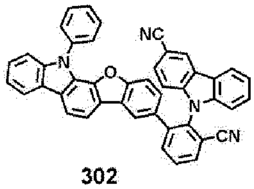

302

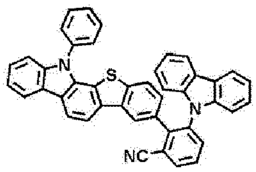

303

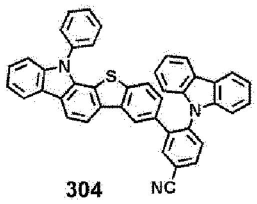

304

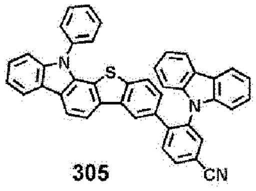

305

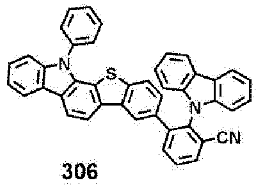

306

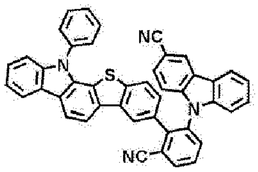

307

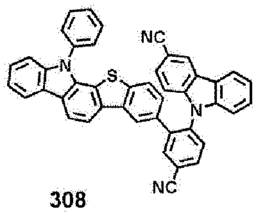

308

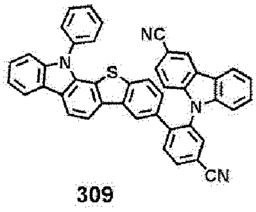

309

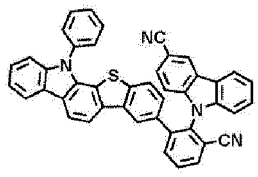

310

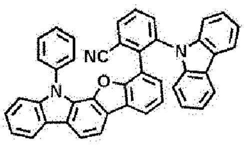

311

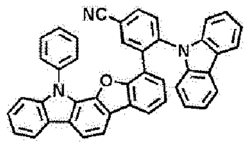

312

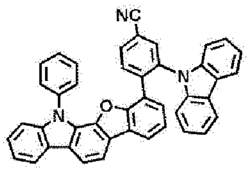

313

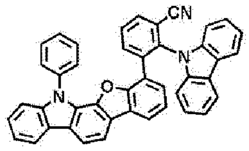

314

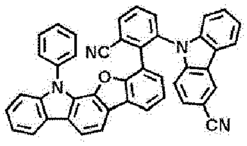

315

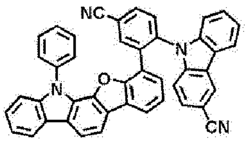

316

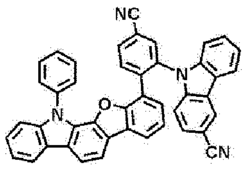

317

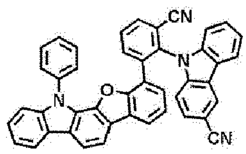

318

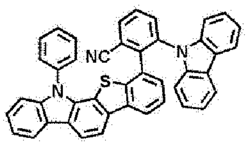

319

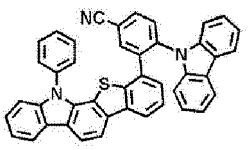

320

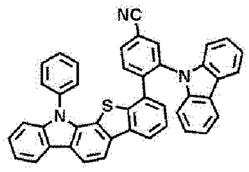

321

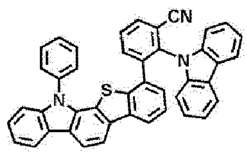

322

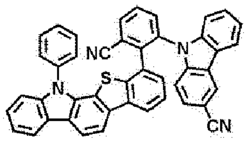
323
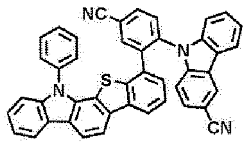
324
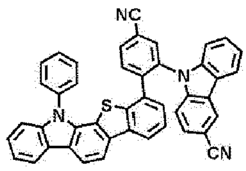
325
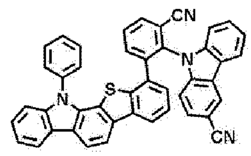
326

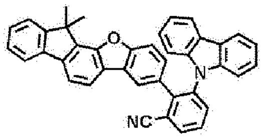
327
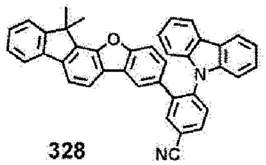
328
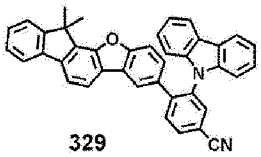
329
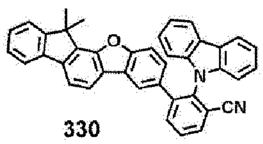
330

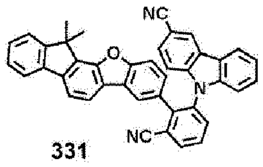
331
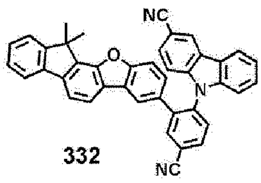
332
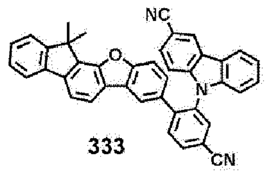
333
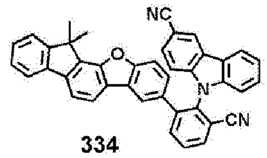
334

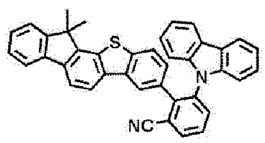
335
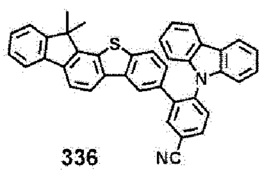
336
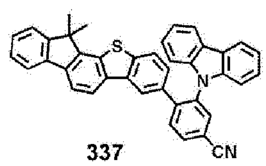
337
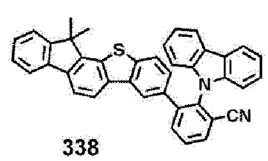
338

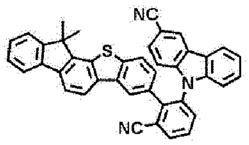
339
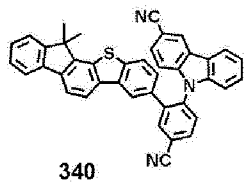
340
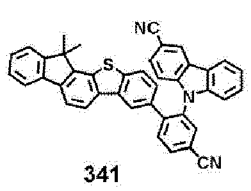
341
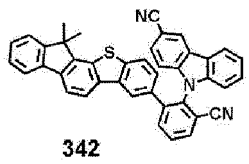
342

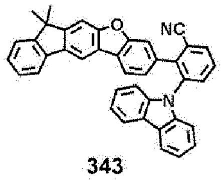
343
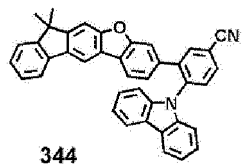
344
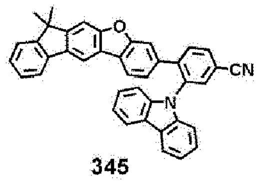
345
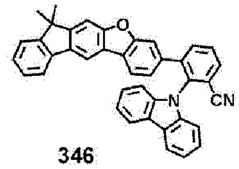
346

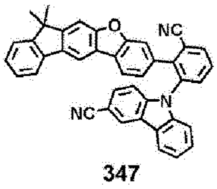
347
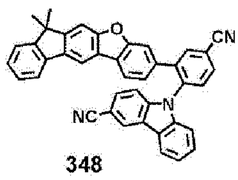
348
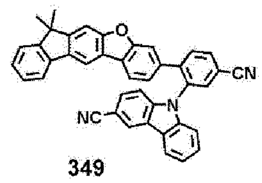
349
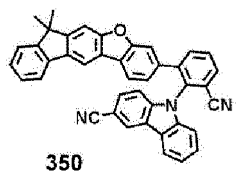
350

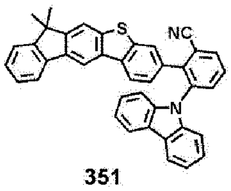
351

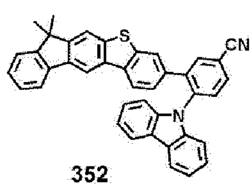
352

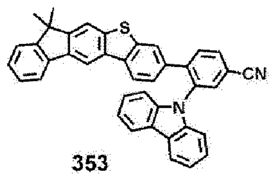
353

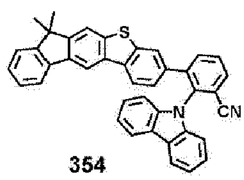
354

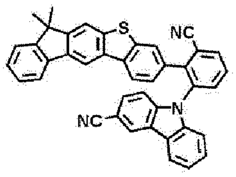
355

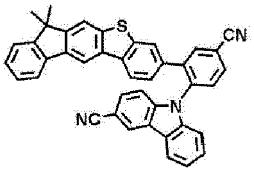
356

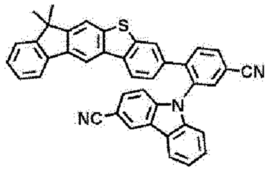
357

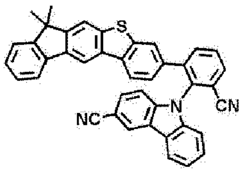
358

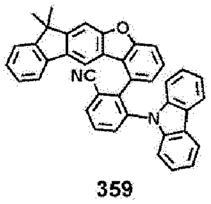
359

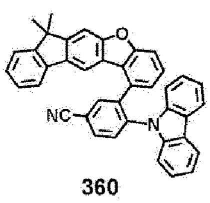
360

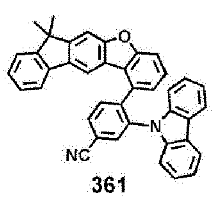
361

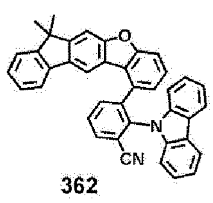
362

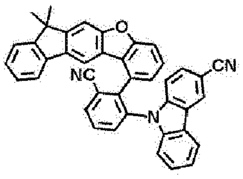
363

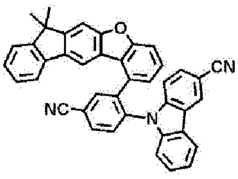
364

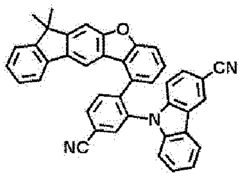
365

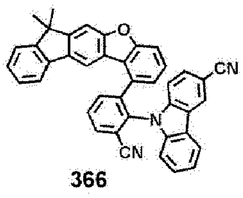
366

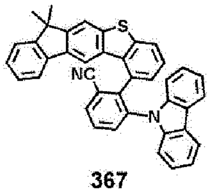
367

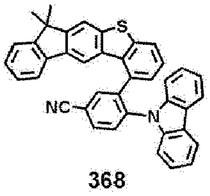
368

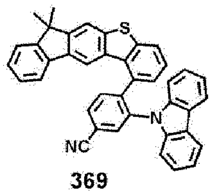
369

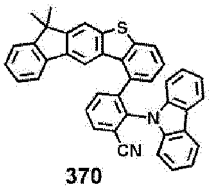
370

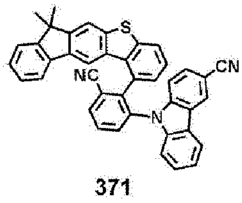
371

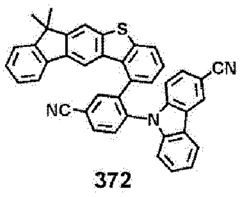
372

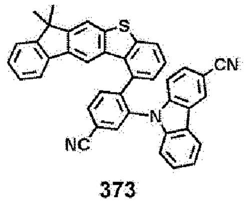
373

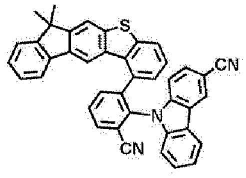
374

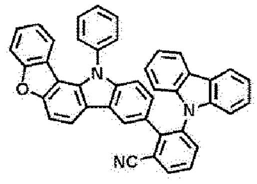
375

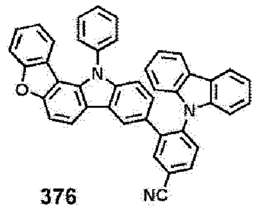
376

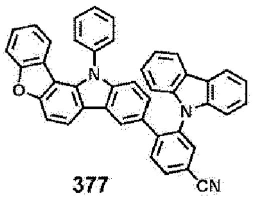
377

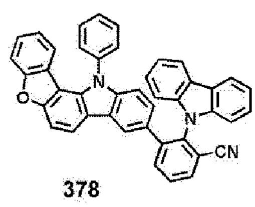
378

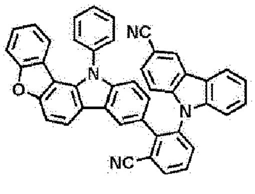
379

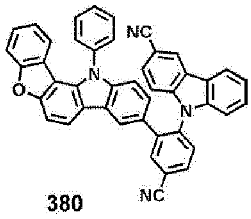
380

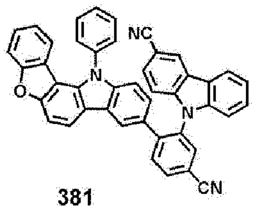
381

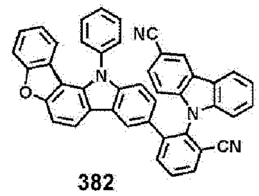
382

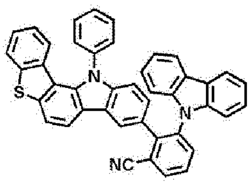
383

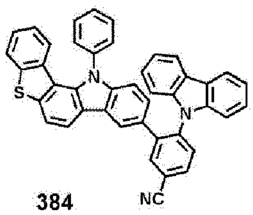
384

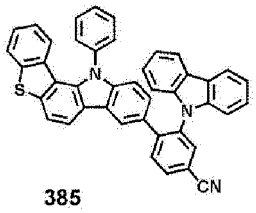
385

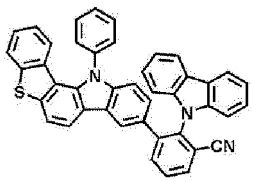
386

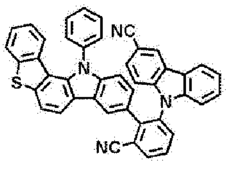
387

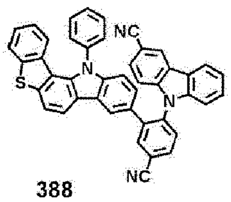
388

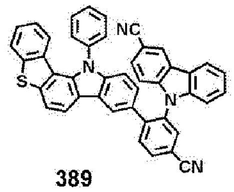
389

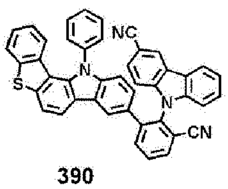
390

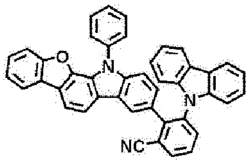
391

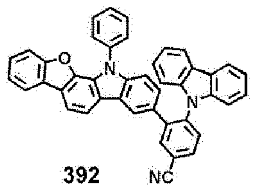
392

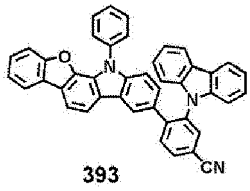
393

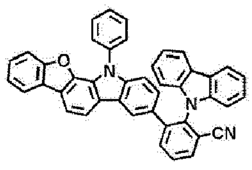
394

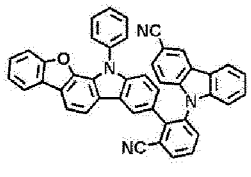
395

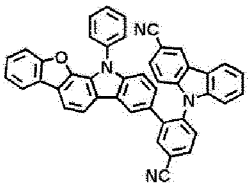
396

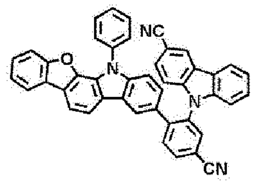
397

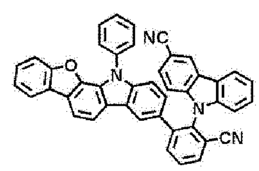
398

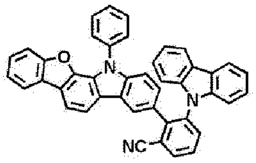
399

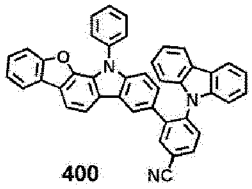
400

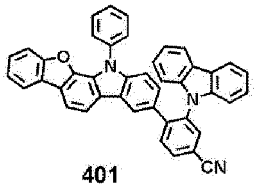
401

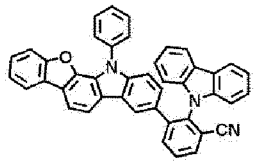
402

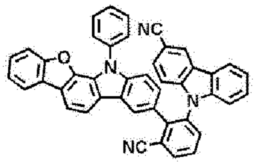
403

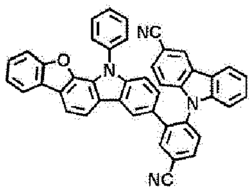
404

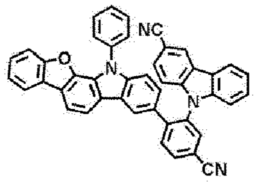
405

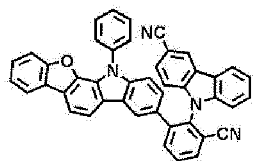
406

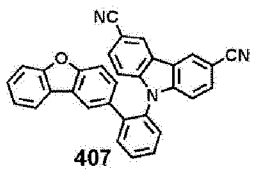
407

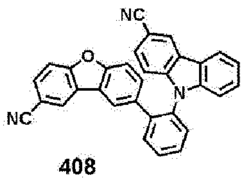
408

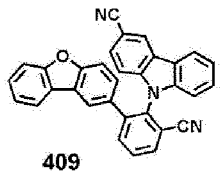
409

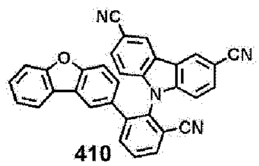
410

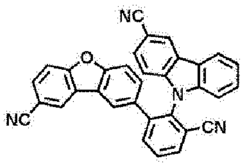
411

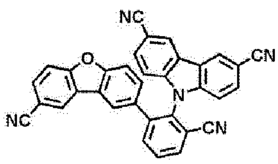
412

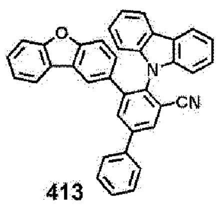
413

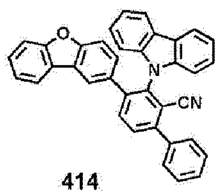
414

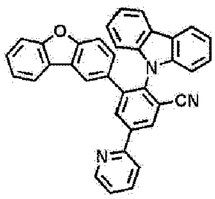
415

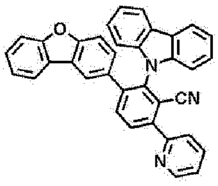
416

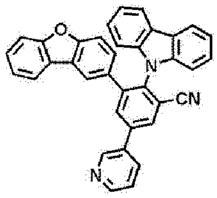
417

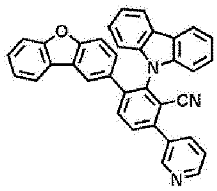
418

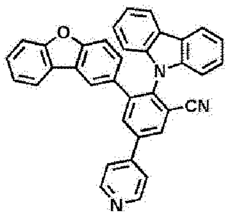
419

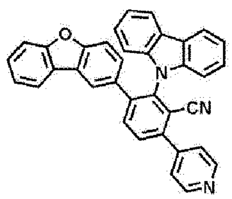
420

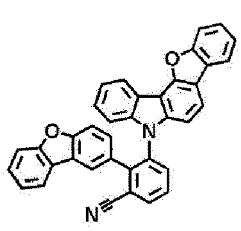

421

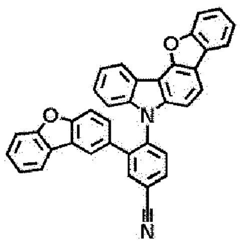

422

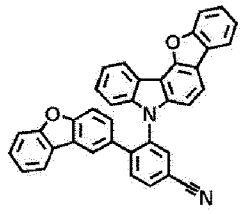

423

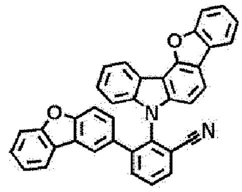

424

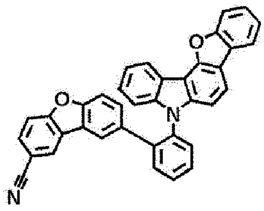

425

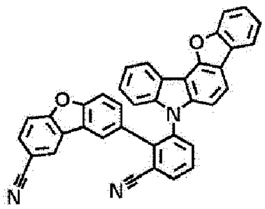

426

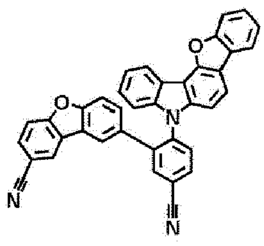

427

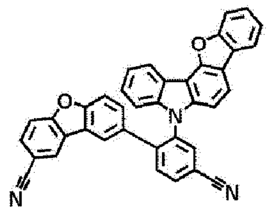

428

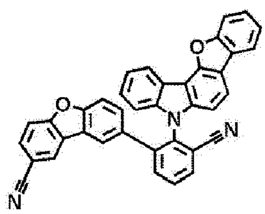

429

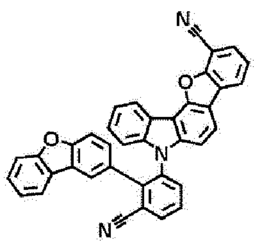

430

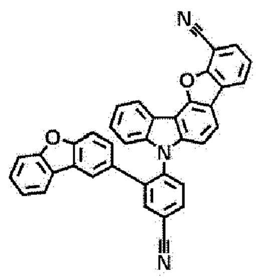

431

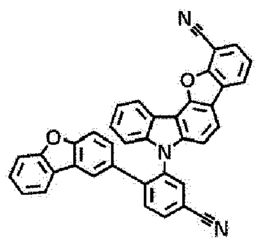

432

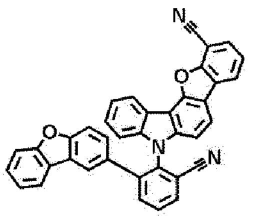
433

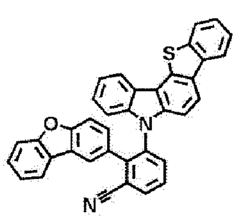
434

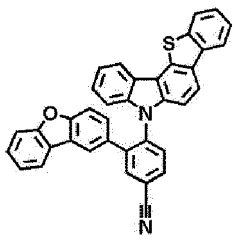
435

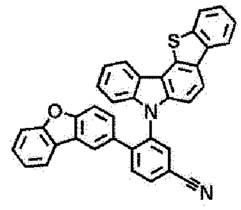
436

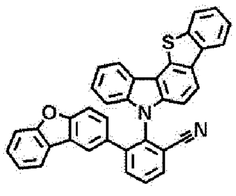
437

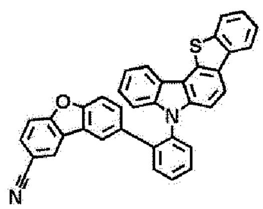
438

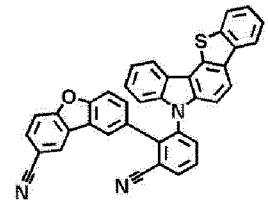
439

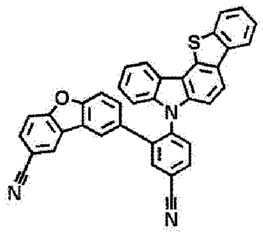
440

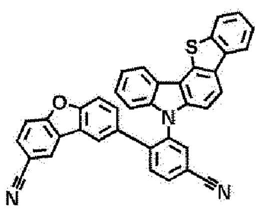
441

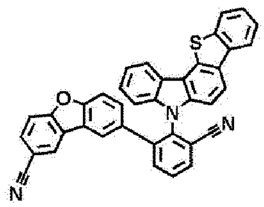
442

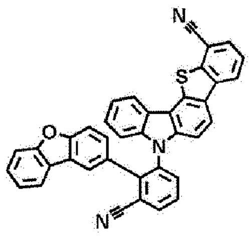
443

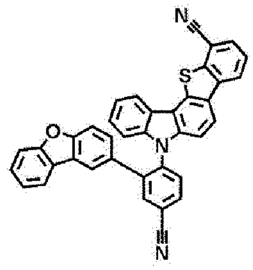
444

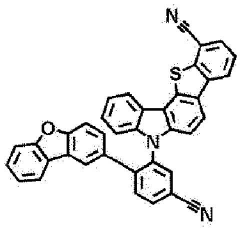
445

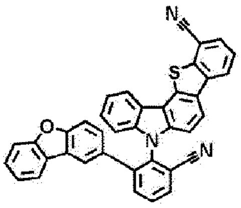
446

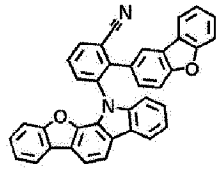
447

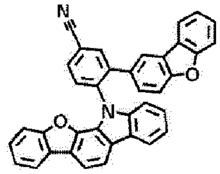
448

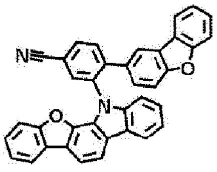
449

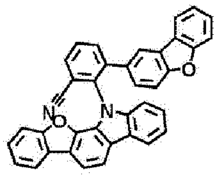
450

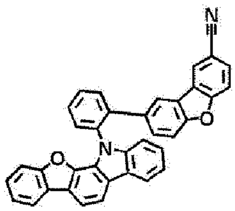
451

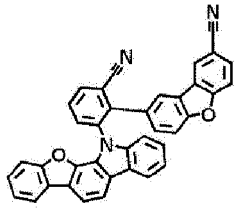
452

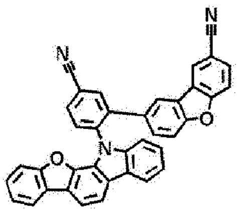
453

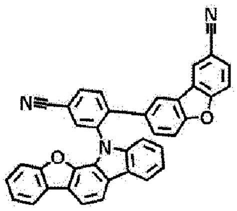
454

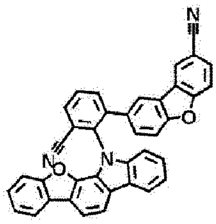
455

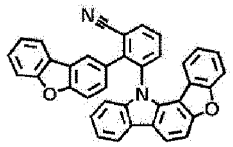
456

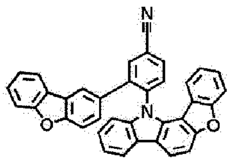
457

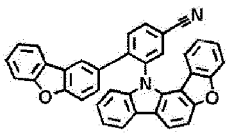
458

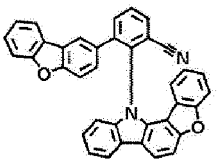
459

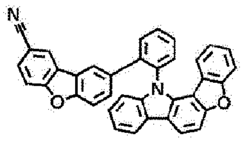
460

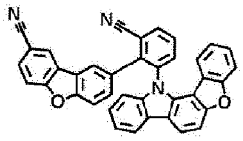
461

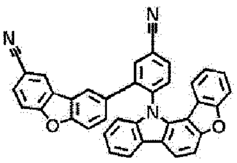
462

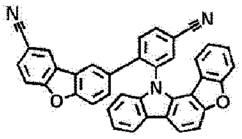
463

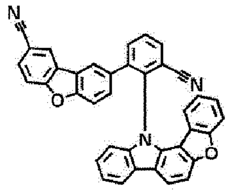
464

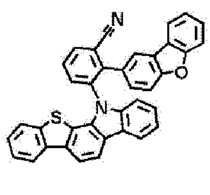
465

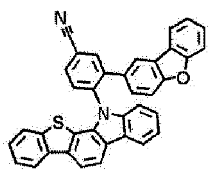
466

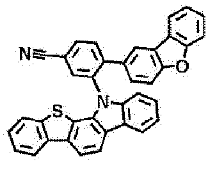
467

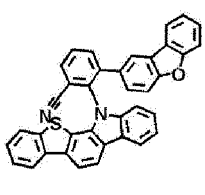
468

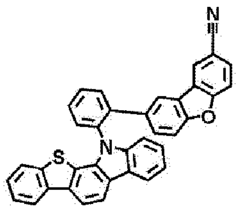

469

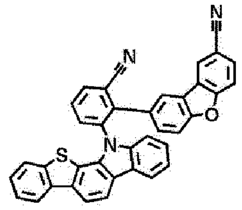

470

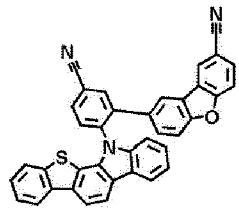

471

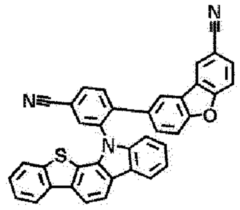

472

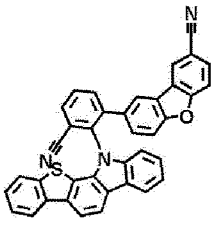

473

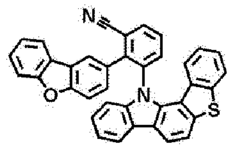

474

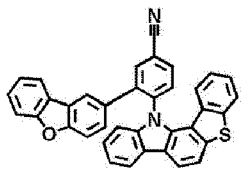

475

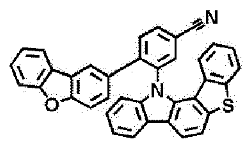

476

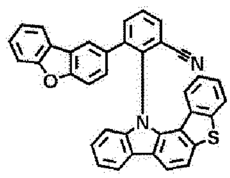

477

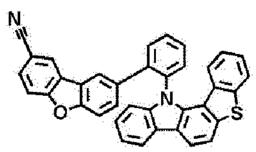

478

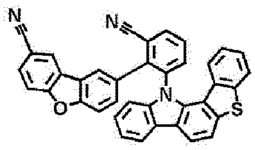

479

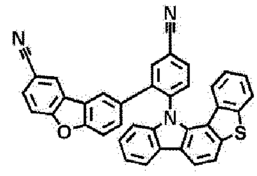

480

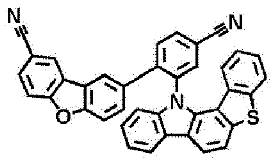

481

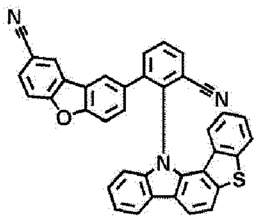

482

Group HE4

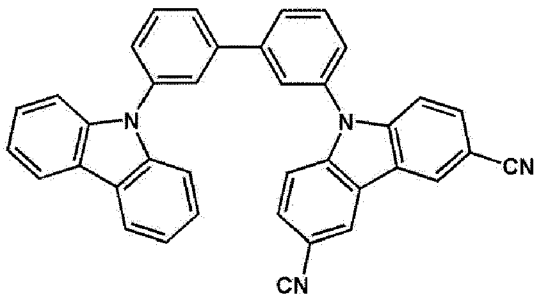

1

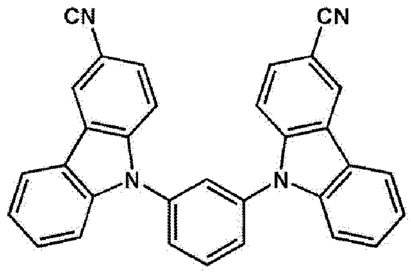

2

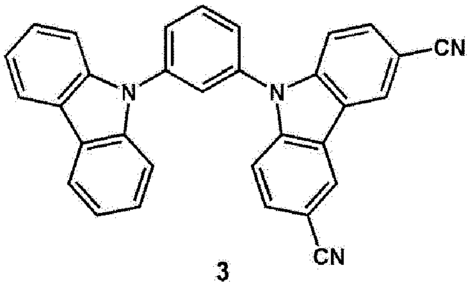

3

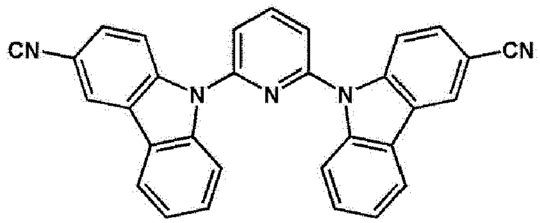

4

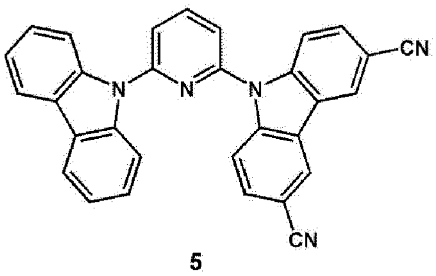

5

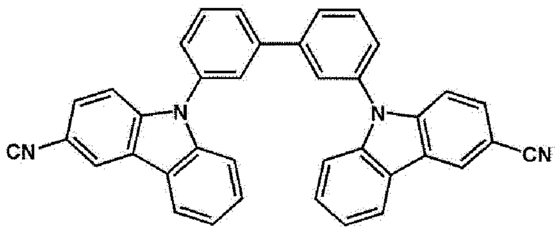

6

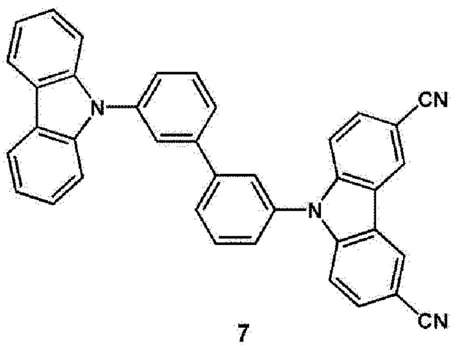

7

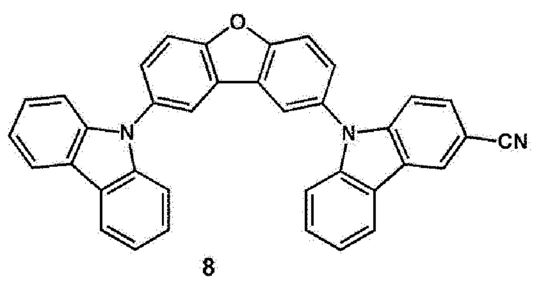

8

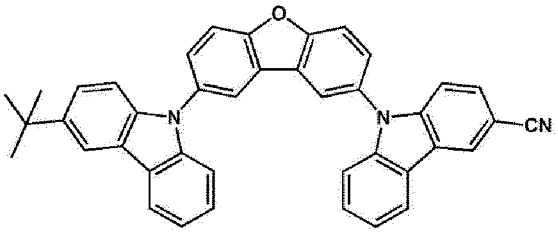

9

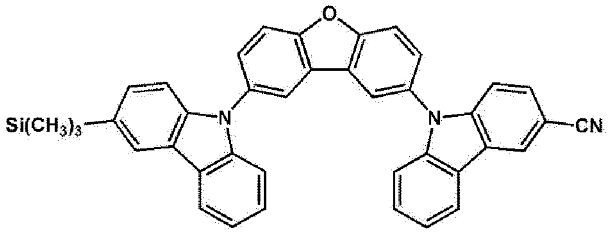

10

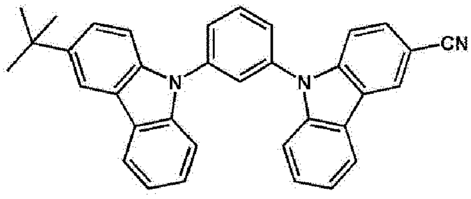

11

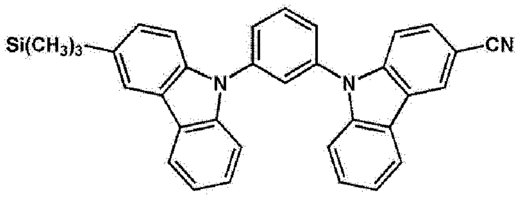

12

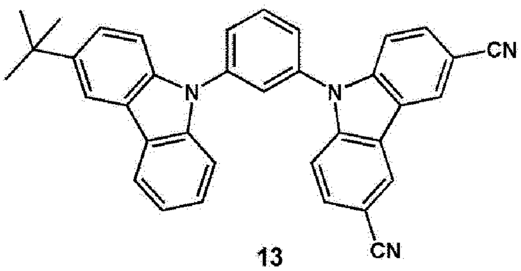

13

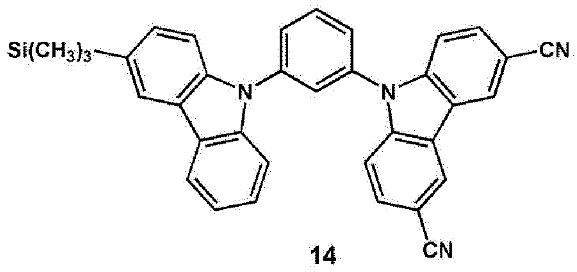

14

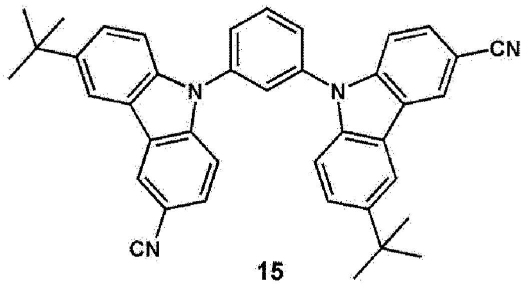

15

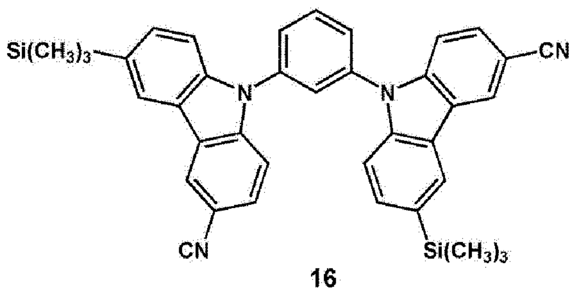

16

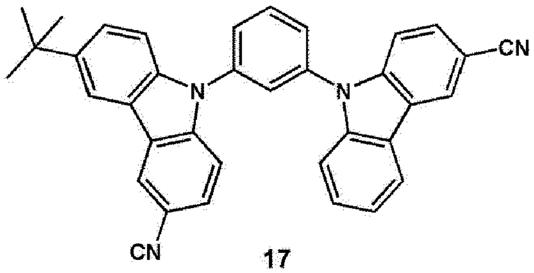

17

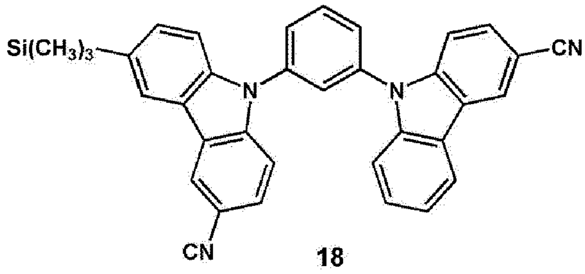

18

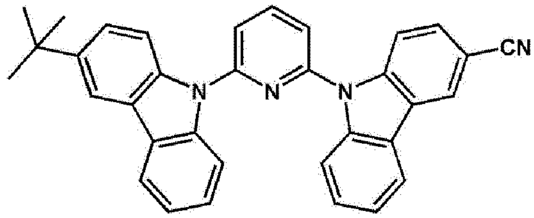

19

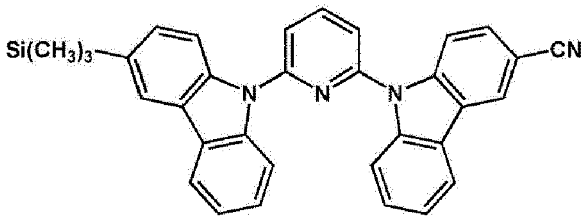

20

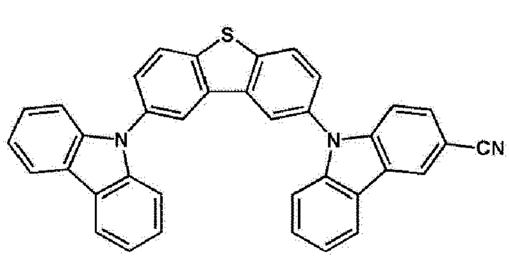

21

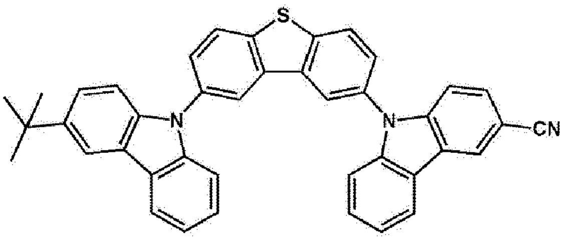

22

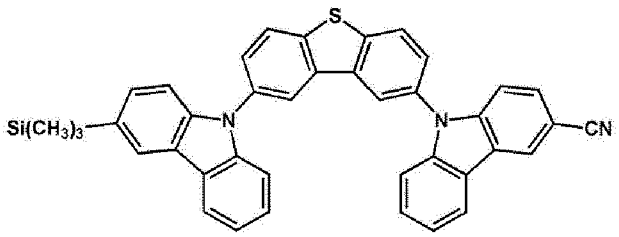

23

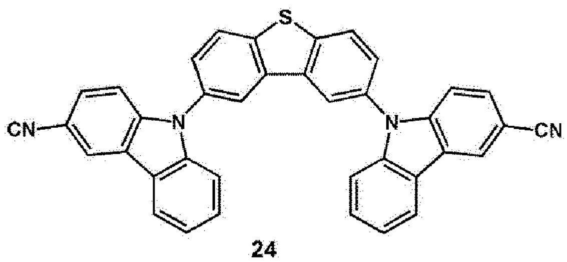

24

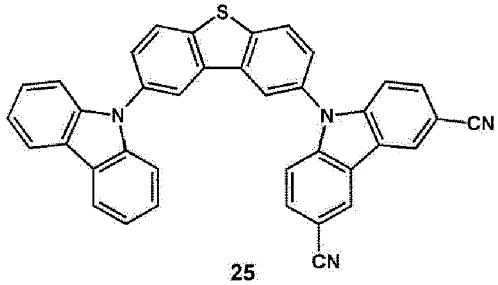

25

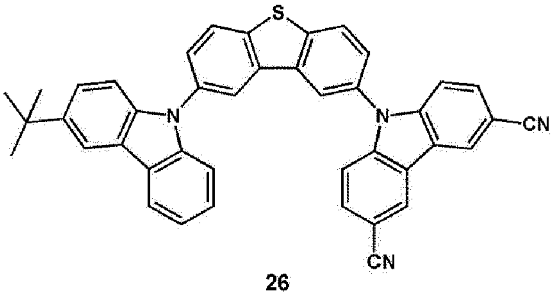

26

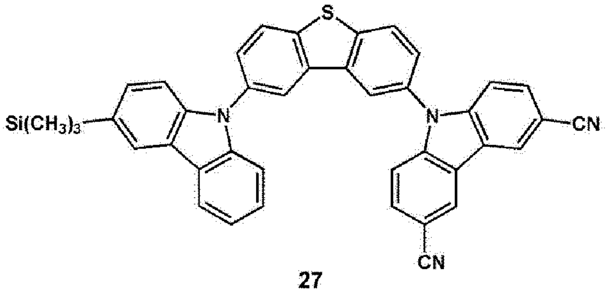

27

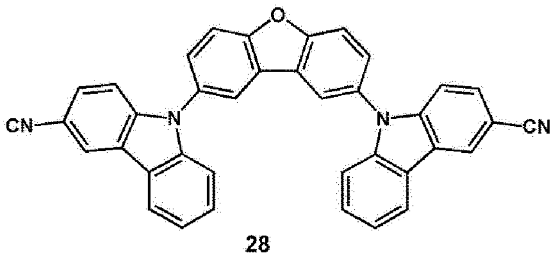

28

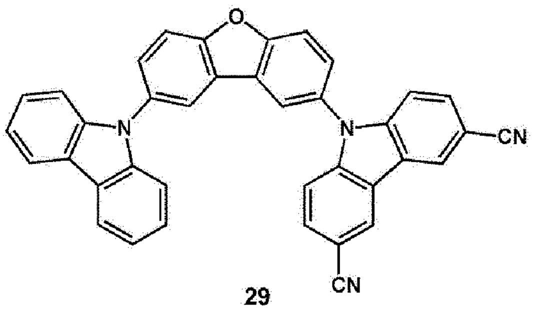

29

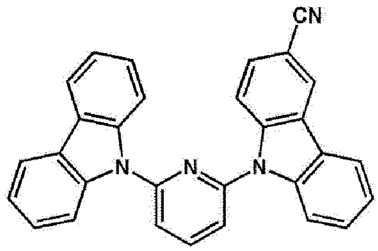

30

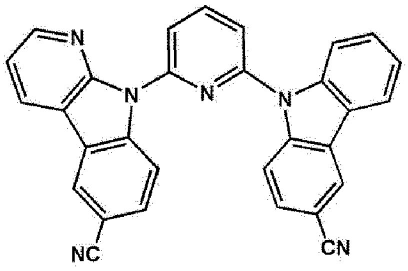

31

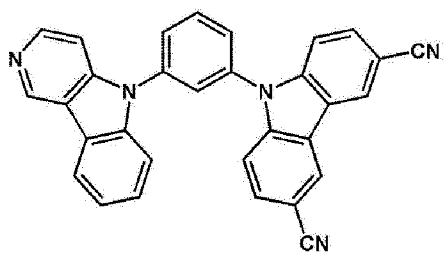

32

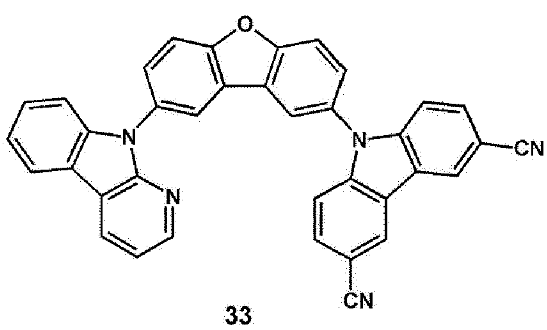

33

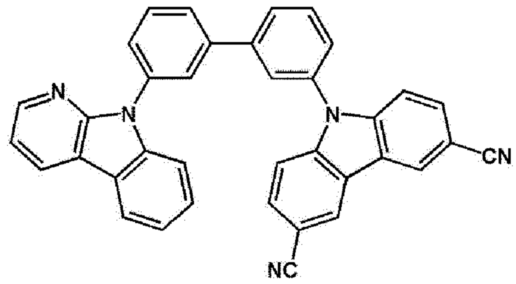

34

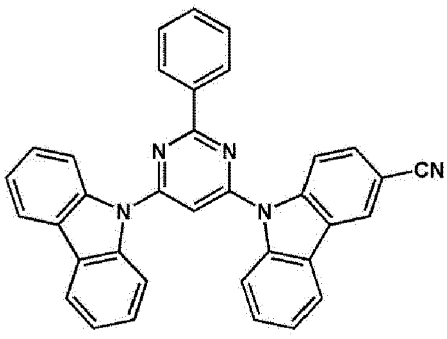

35

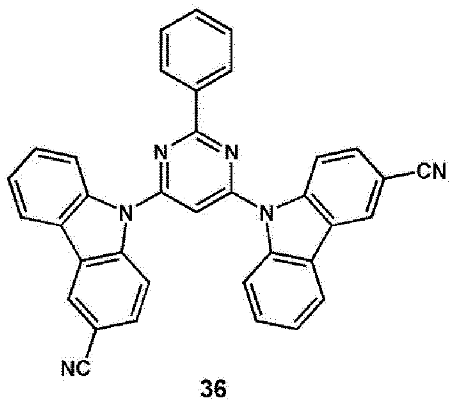

36

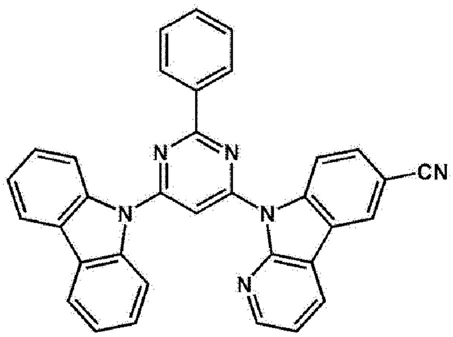

37

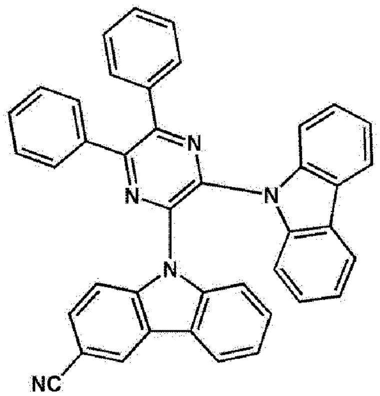

38

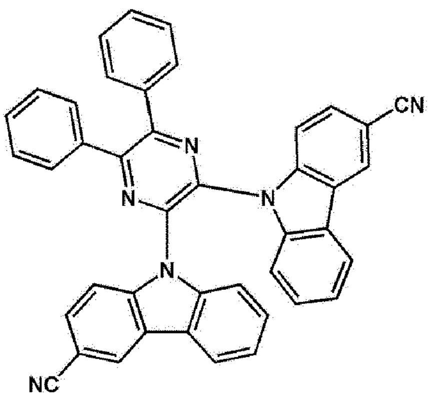

39

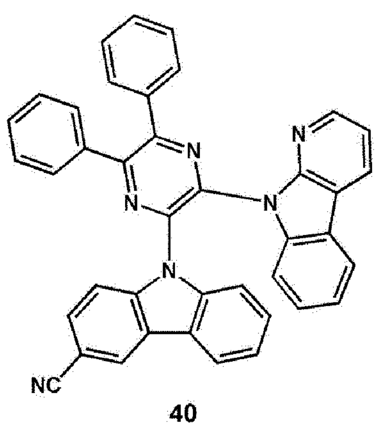

40

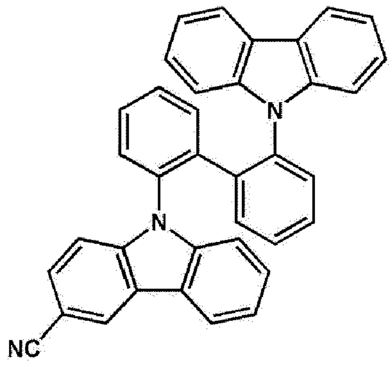

41

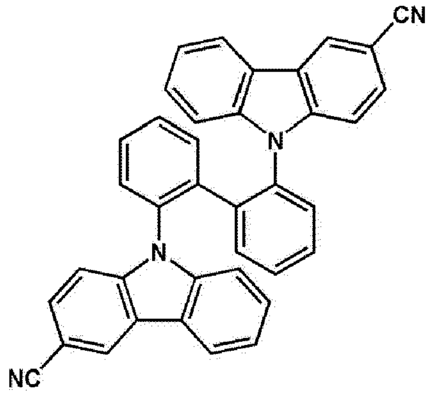

42

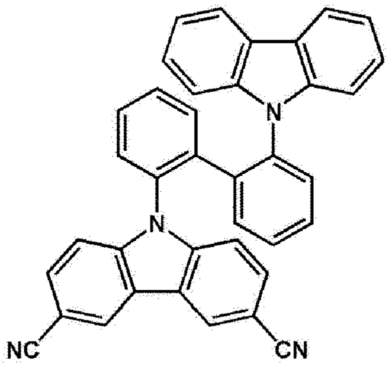

43

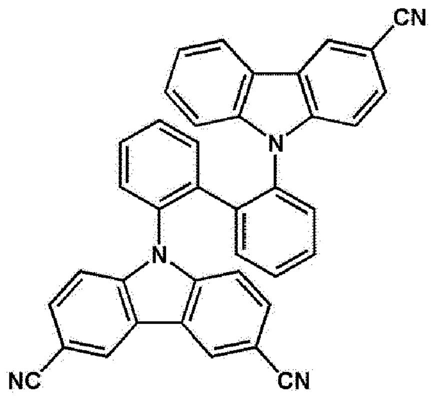

44

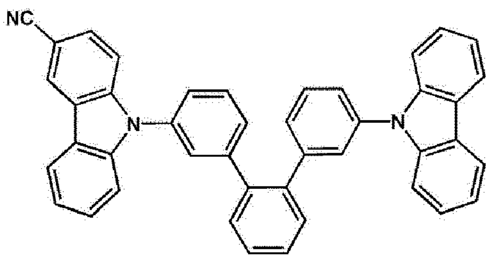

45

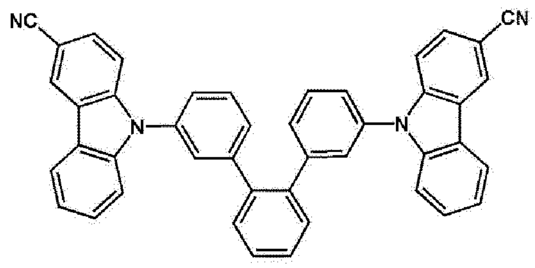

46

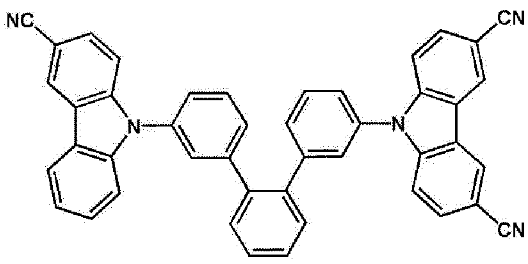

47

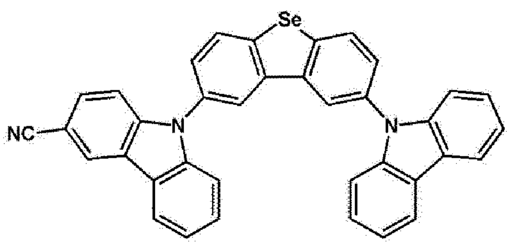

48

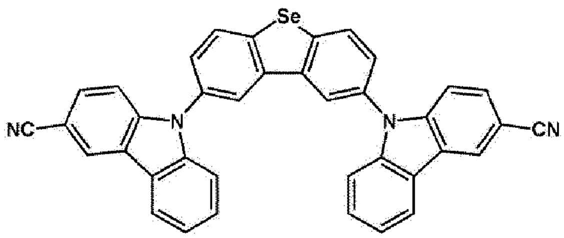

49

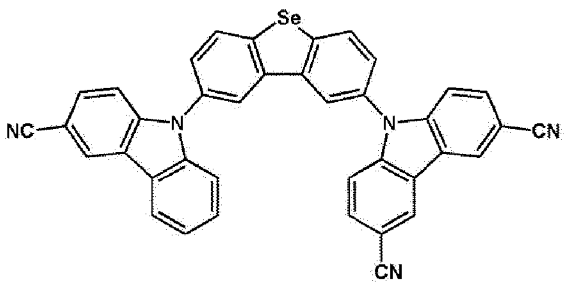

50

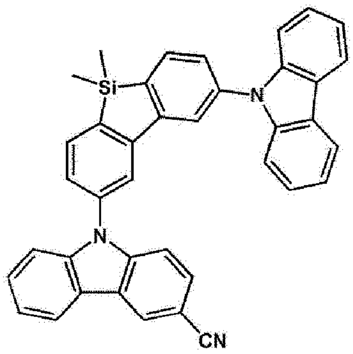

51

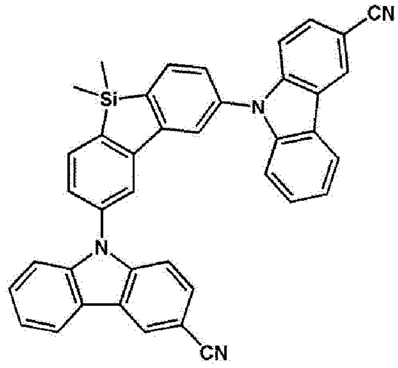

52

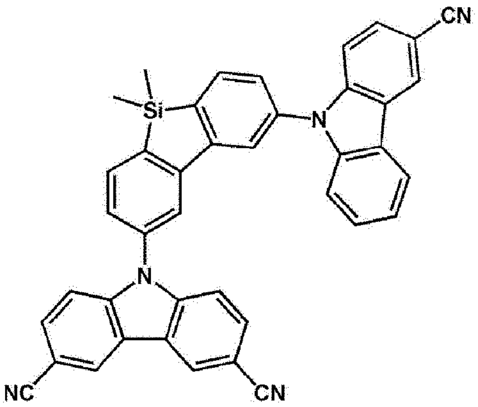

53

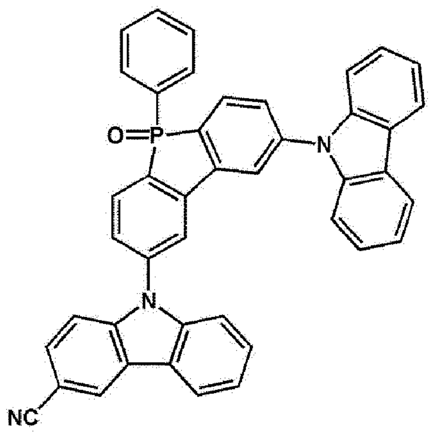

54

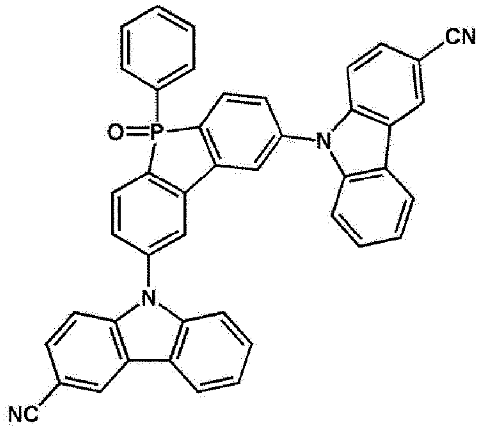

55

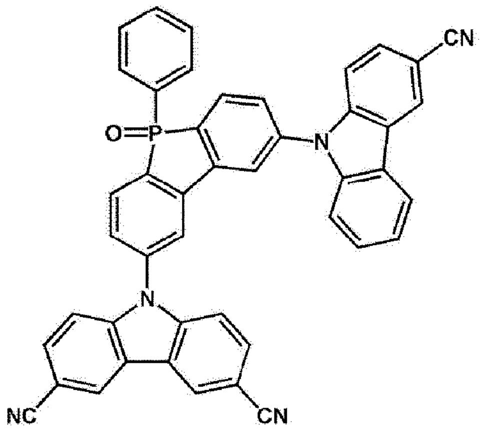

56

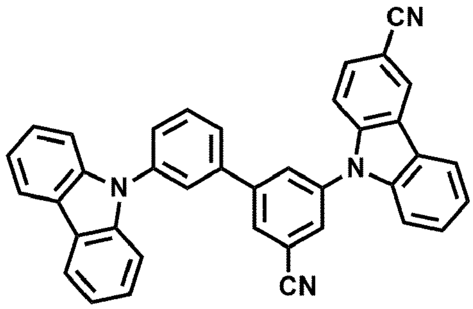

**57**

## Group HE5

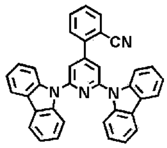
1

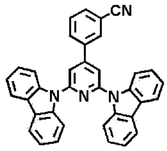
2

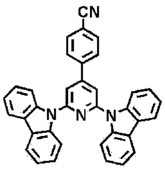
3

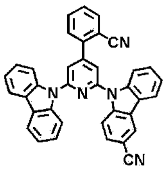
4

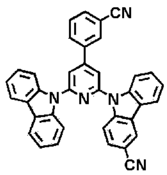
5

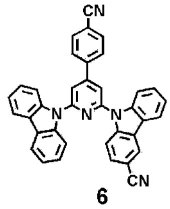
6

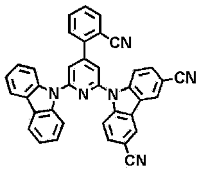
7

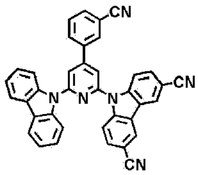
8

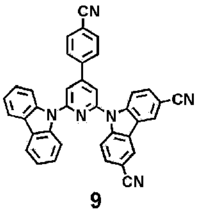
9

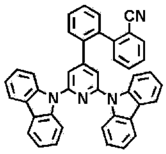
10

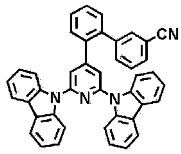
11

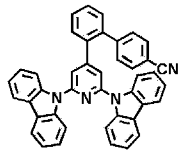
12

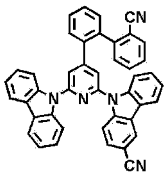
13

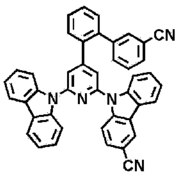
14

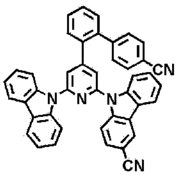
15

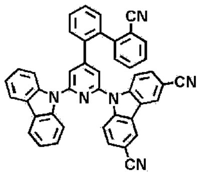
16

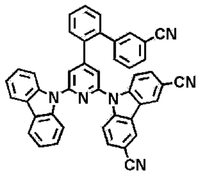
17

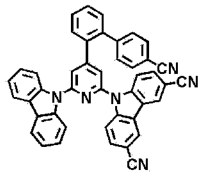
18

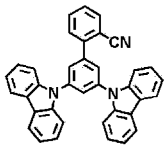
19

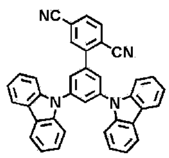
20

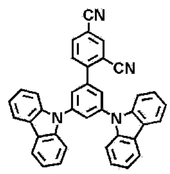
21

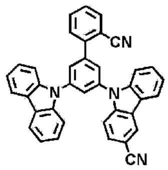
22

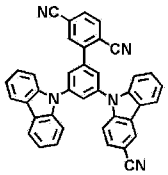
23

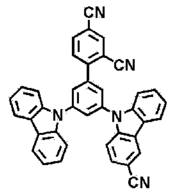
24

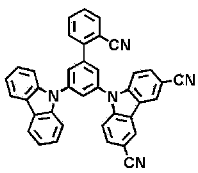
25

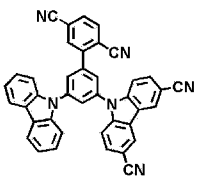
26

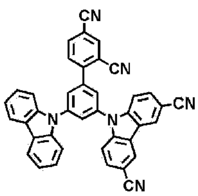
27

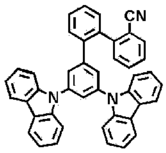
28

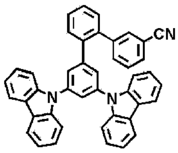
29

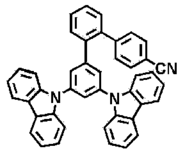
30

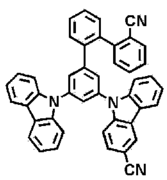
31

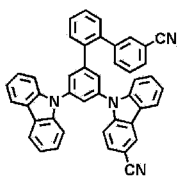
32

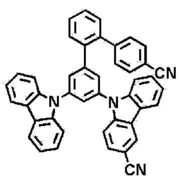
33

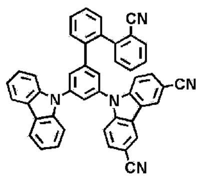
34

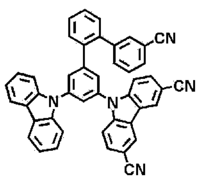
35

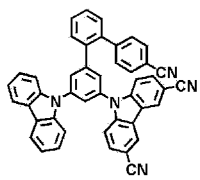
36

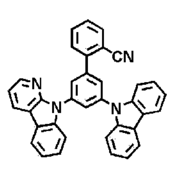 37 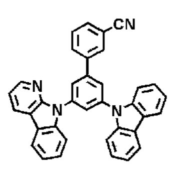 38 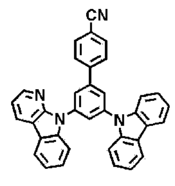 39 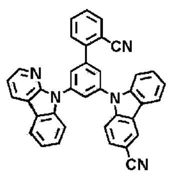 40 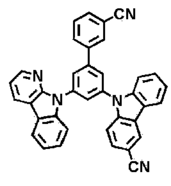 41 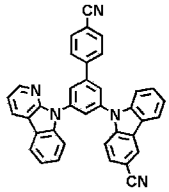 42

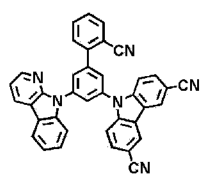 43 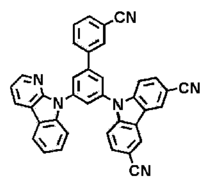 44 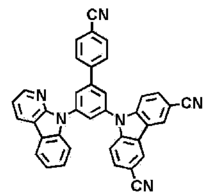 45 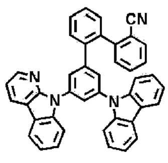 46 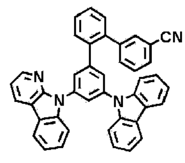 47 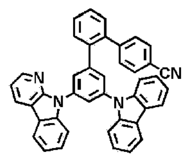 48

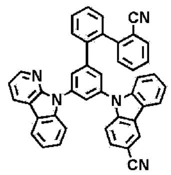 49 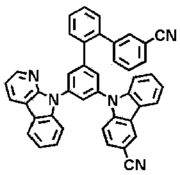 50 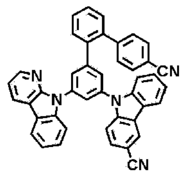 51 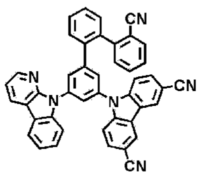 52 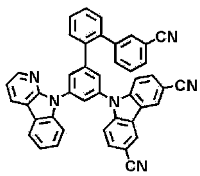 53 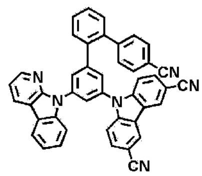 54

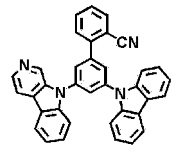 55 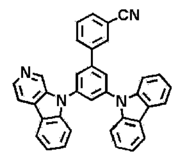 56 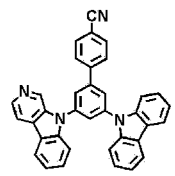 57 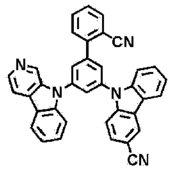 58 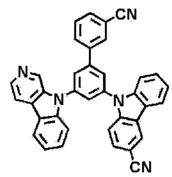 59 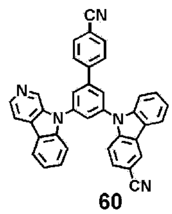 60

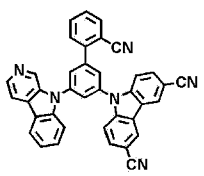 61 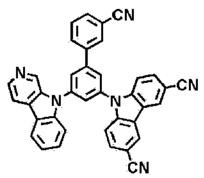 62 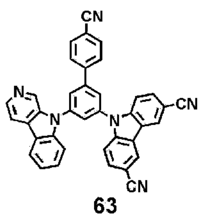 63 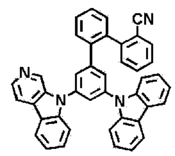 64 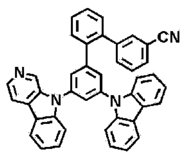 65 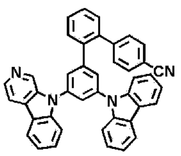 66

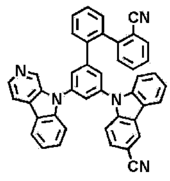 67 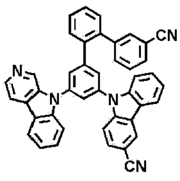 68 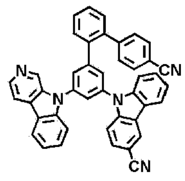 69 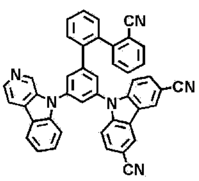 70 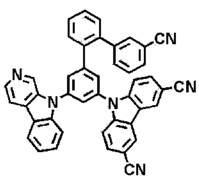 71 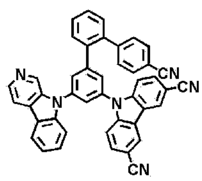 72

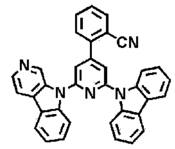
73
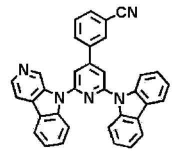
74
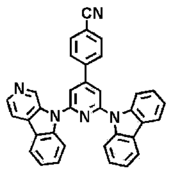
75
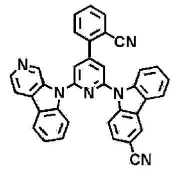
76
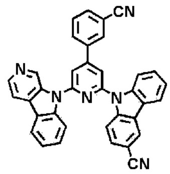
77
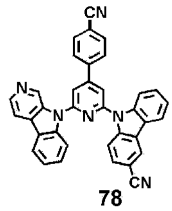
78

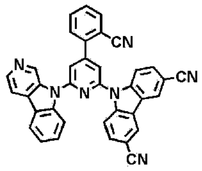
79
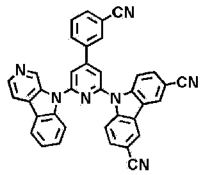
80
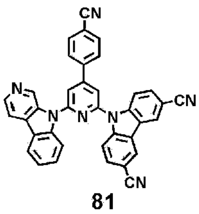
81
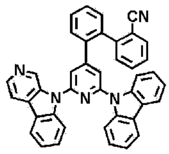
82
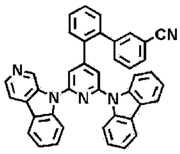
83
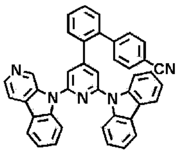
84

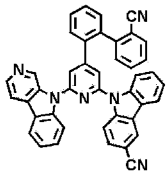
85
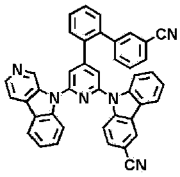
86
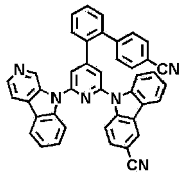
87
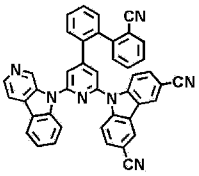
88
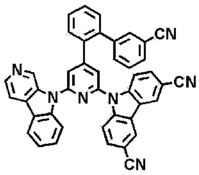
89
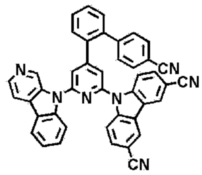
90

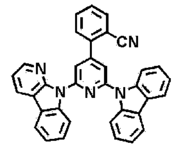
91
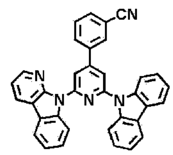
92
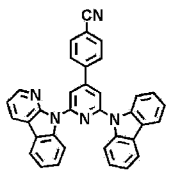
93
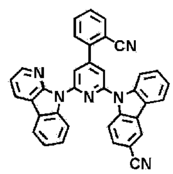
94
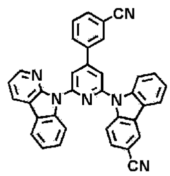
95
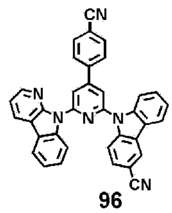
96

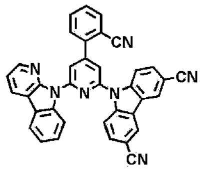
97
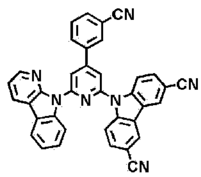
98
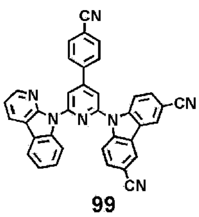
99
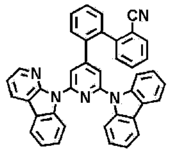
100
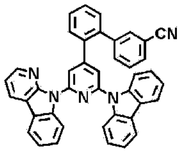
101
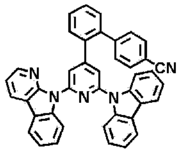
102

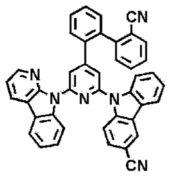
103
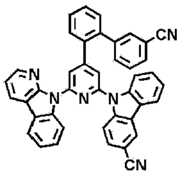
104
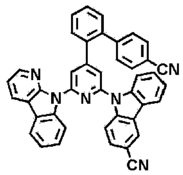
105
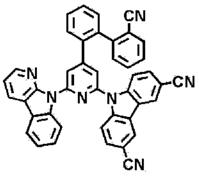
106
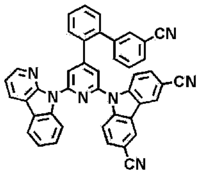
107
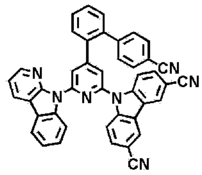
108

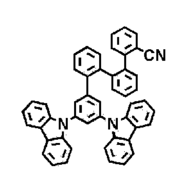
109
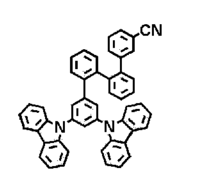
110
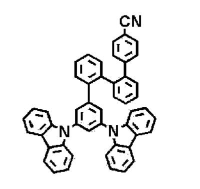
111
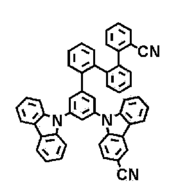
112
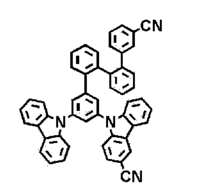
113
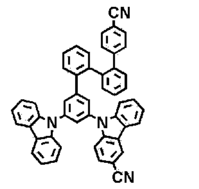
114

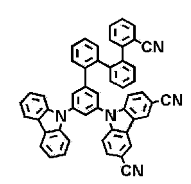
115
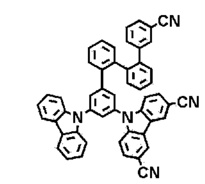
116
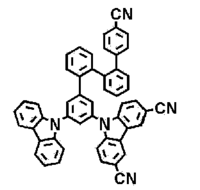
117
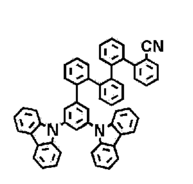
118
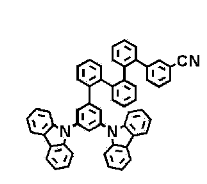
119
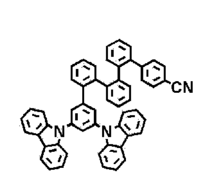
120

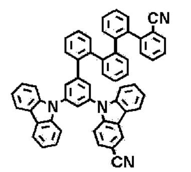
121
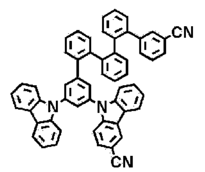
122
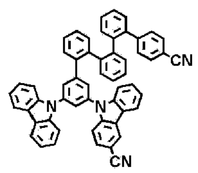
123
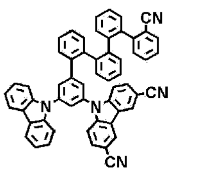
124
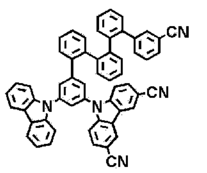
125
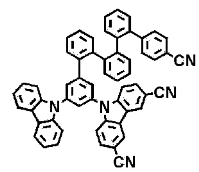
126

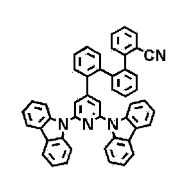
127
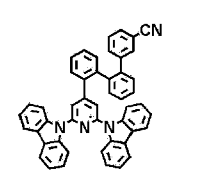
128
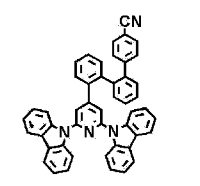
129
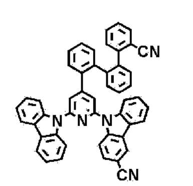
130
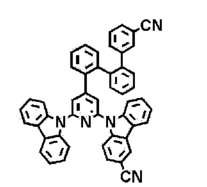
131
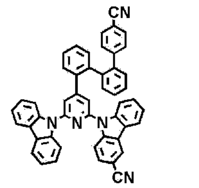
132

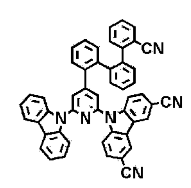
133
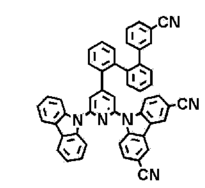
134
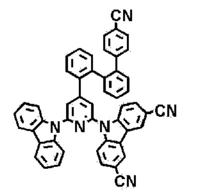
135
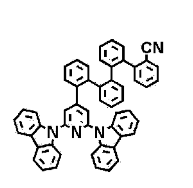
136
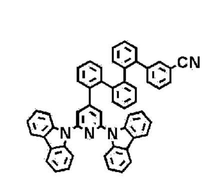
137
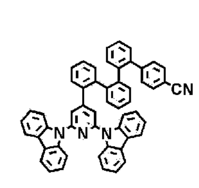
138

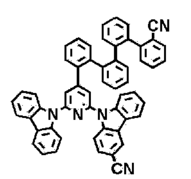
139
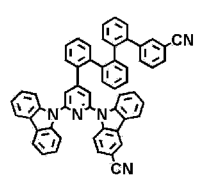
140
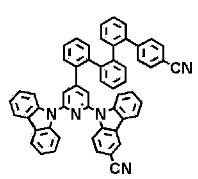
141
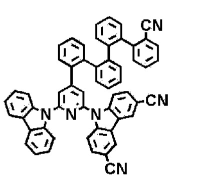
142
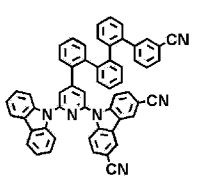
143
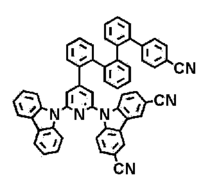
144

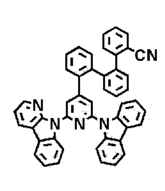
145
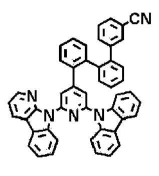
146
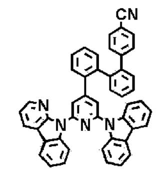
147
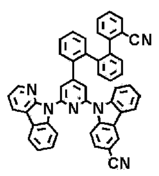
148
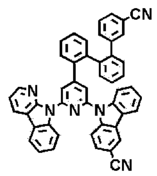
149
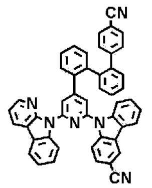
150

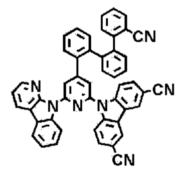
151
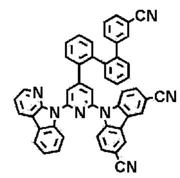
152
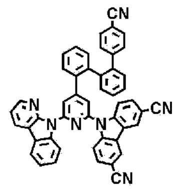
153
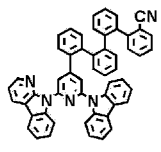
154
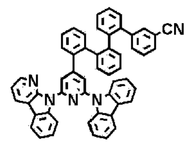
155
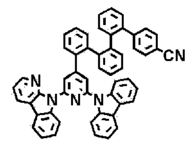
156

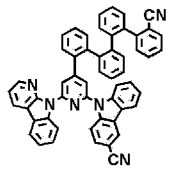
157
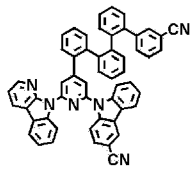
158
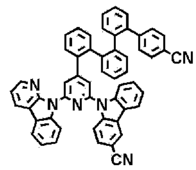
159
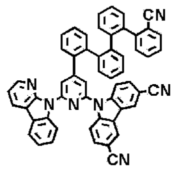
160
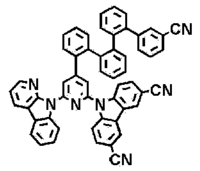
161
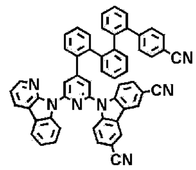
162

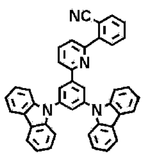
163
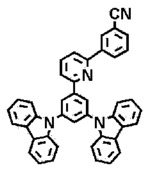
164
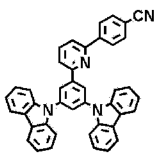
165
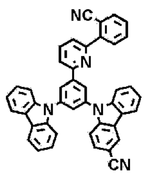
166
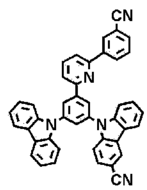
167
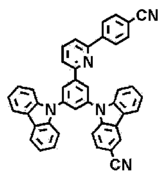
168

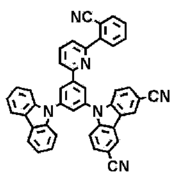
169
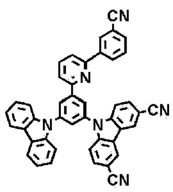
170
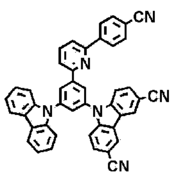
171
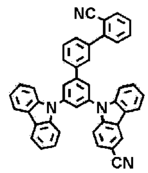
172
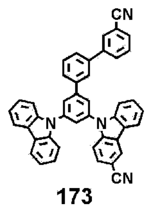
173
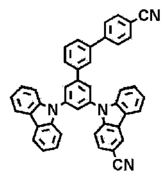
174

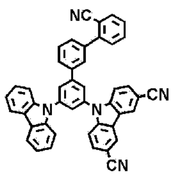
175
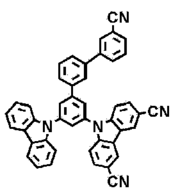
176
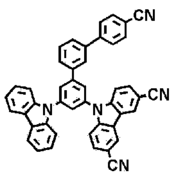
177
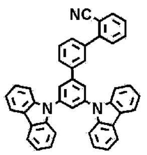
178
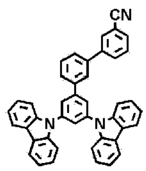
179
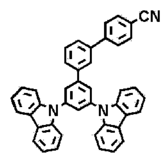
180

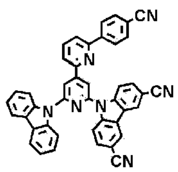
**181**

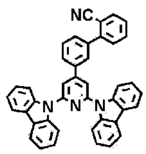
**182**

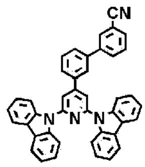
**183**

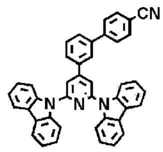
**184**

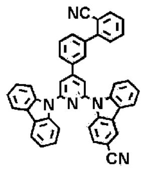
**185**

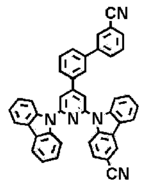
**186**

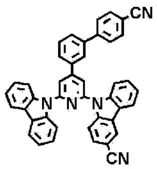
**187**

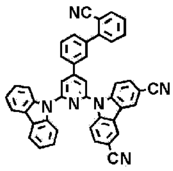
**188**

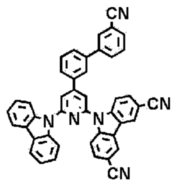
**189**

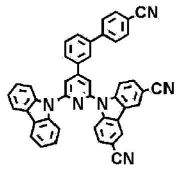
**190**

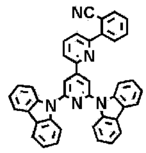
**191**

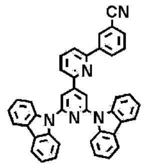
**192**

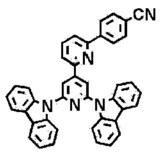
**193**

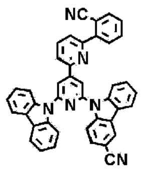
**194**

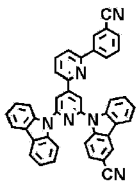
**195**

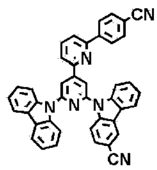
**196**

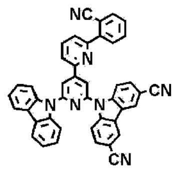
**197**

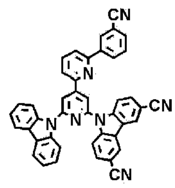
**198**

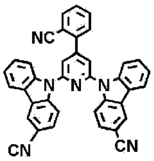
**199**

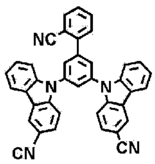
**200**

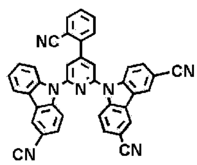
**201**

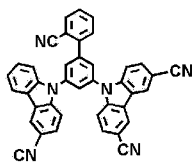
**202**

Group HE6

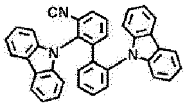
1
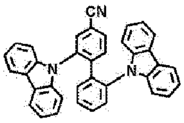
2
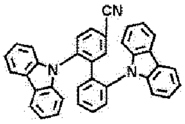
3
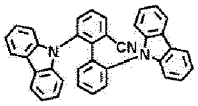
4
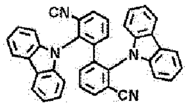
5

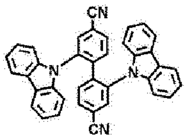
6
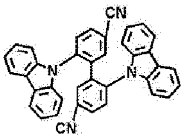
7
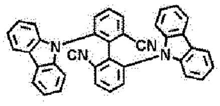
8
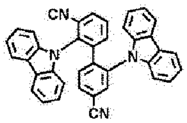
9
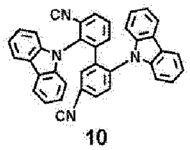
10

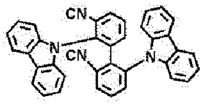
11
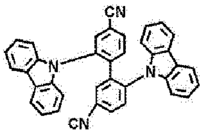
12
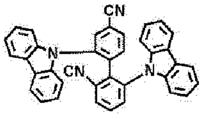
13
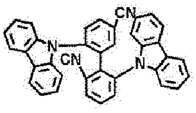
14
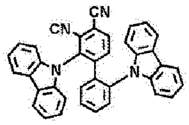
15

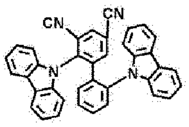
16
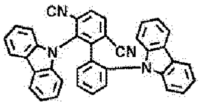
17
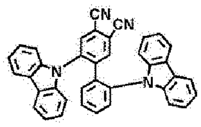
18
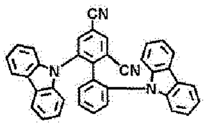
19
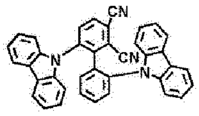
20

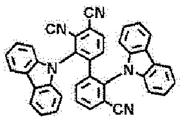
21
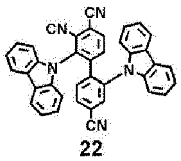
22
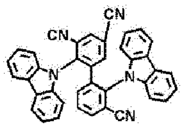
23
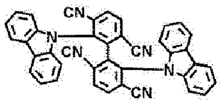
24
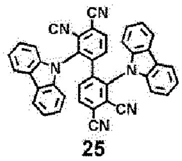
25

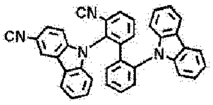
26
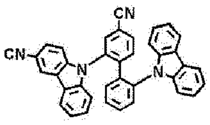
27
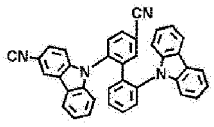
28
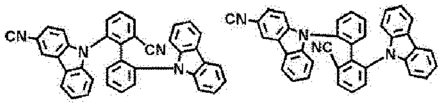
29
30

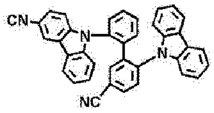
31
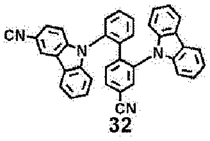
32
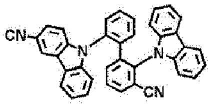
33
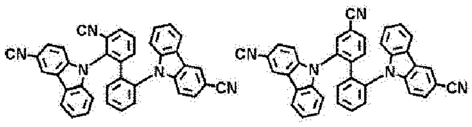
34
35

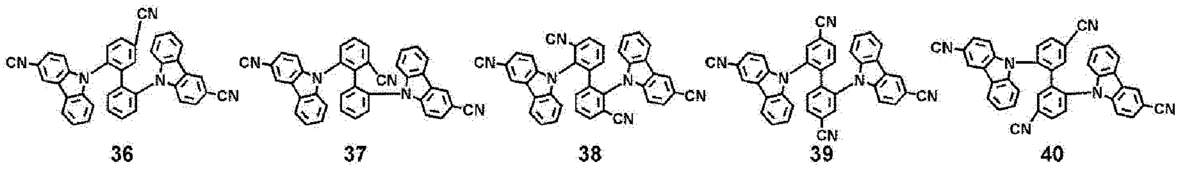
36
37
38
39
40

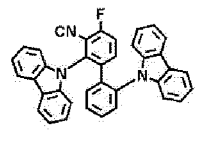
41

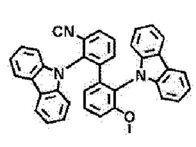
42

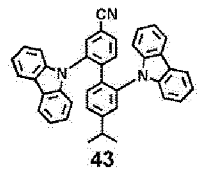
43

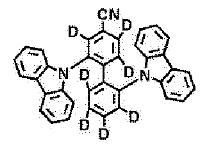
44

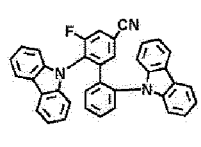
45

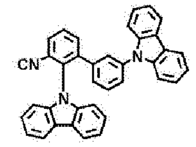
46

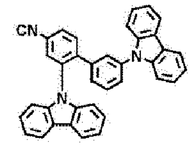
47

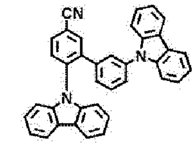
48

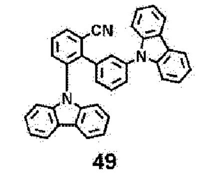
49

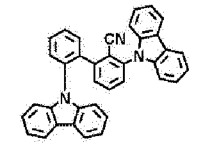
50

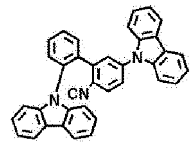
51

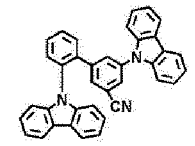
52

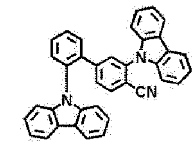
53

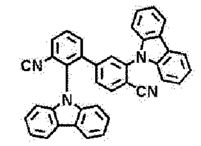
54

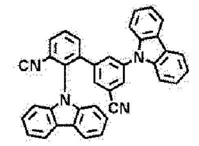
55

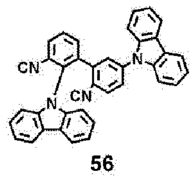
56

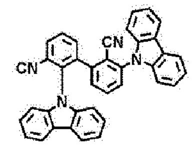
57

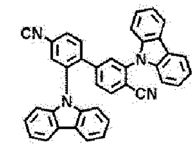
58

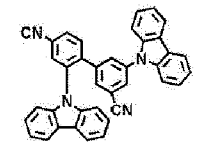
59

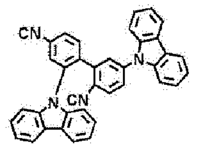
60

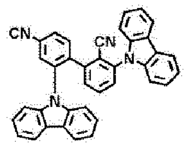
61

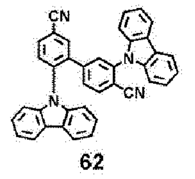
62

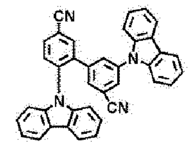
63

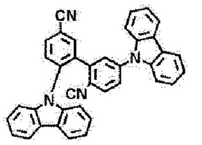
64

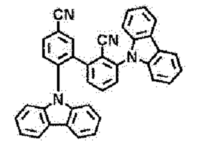
65

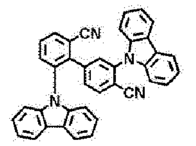
66

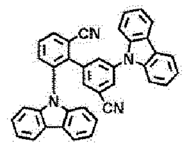
67

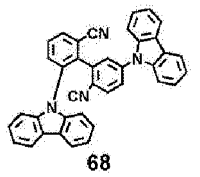
68

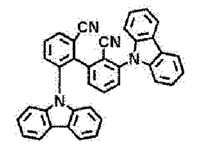
69

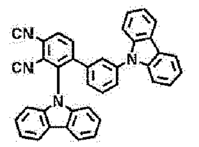
70

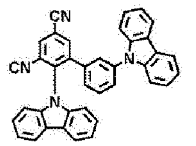
71

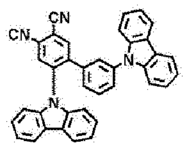
72

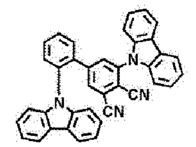
73

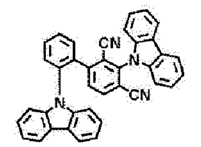
74

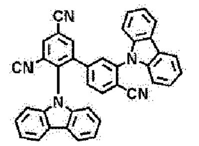
75

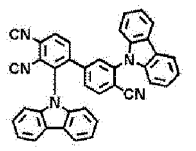
76

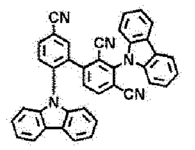
77

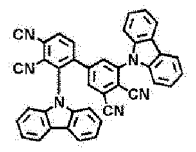
78

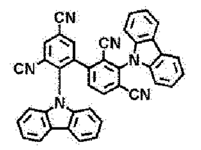
79

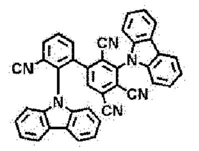
80

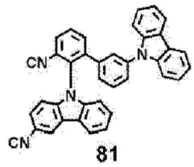
81
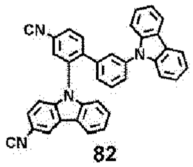
82
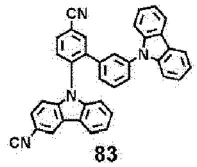
83
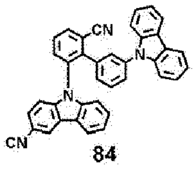
84
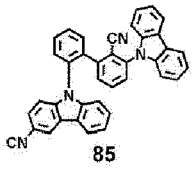
85

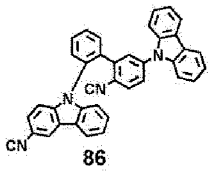
86
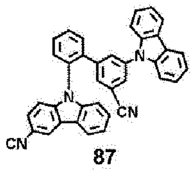
87
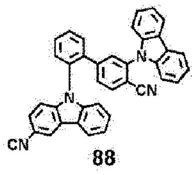
88
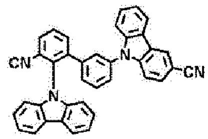
89
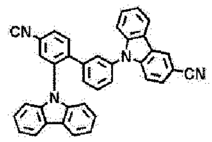
90

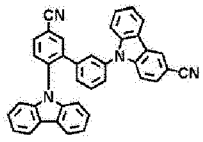
91
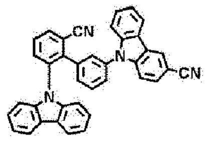
92
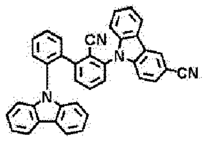
93
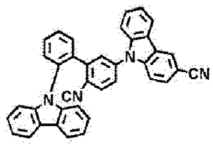
94
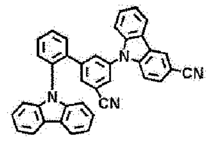
95

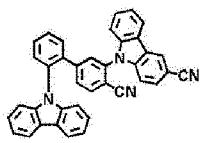
96
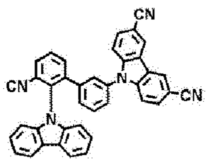
97
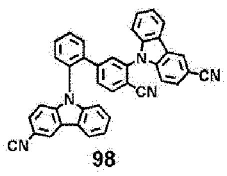
98
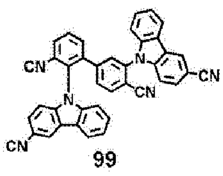
99
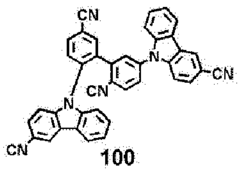
100

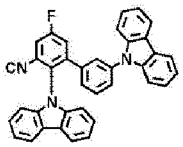
101
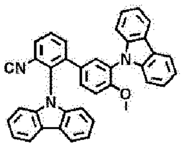
102
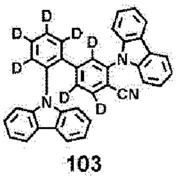
103
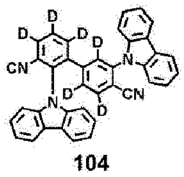
104
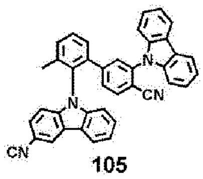
105

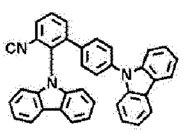
106
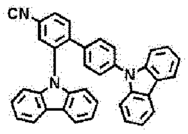
107
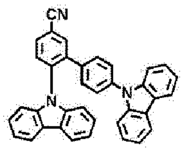
108
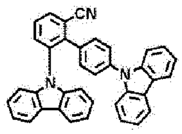
109
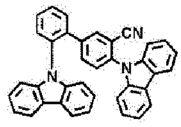
110

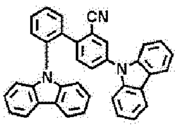
111
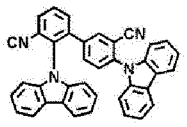
112
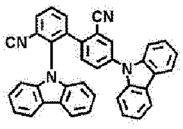
113
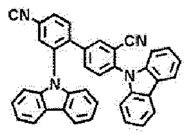
114
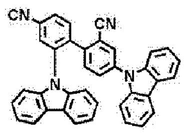
115

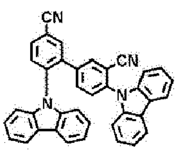
116
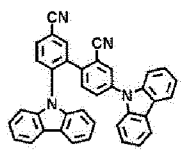
117
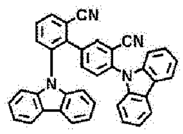
118
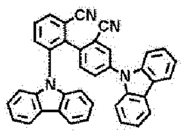
119
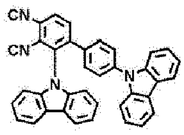
120

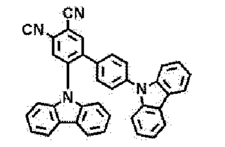
121

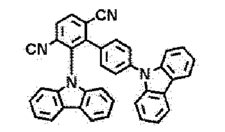
122

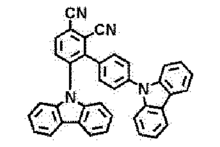
123

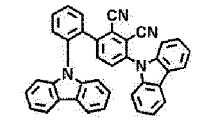
124

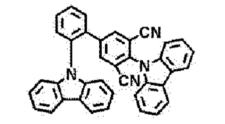
125

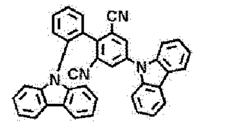
126

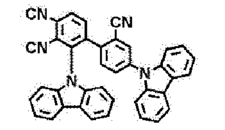
127

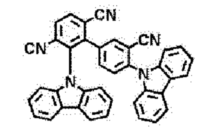
128

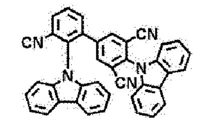
129

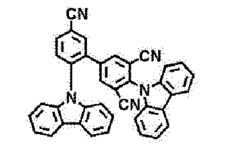
130

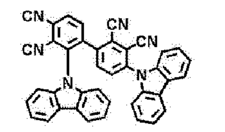
131

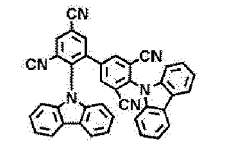
132

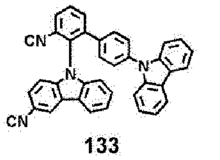
133

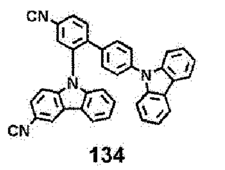
134

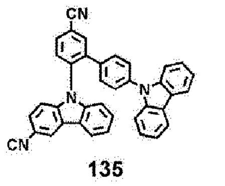
135

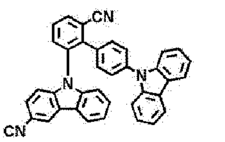
136

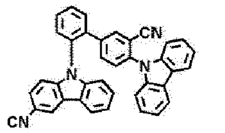
137

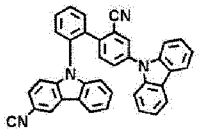
138

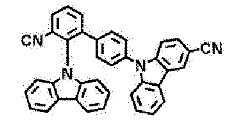
139

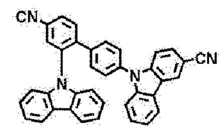
140

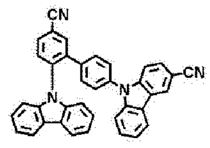
141

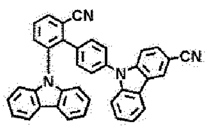
142

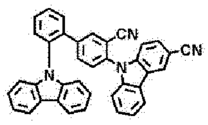
143

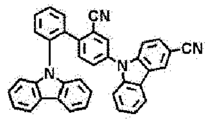
144

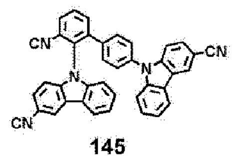
145

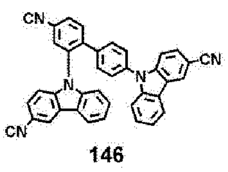
146

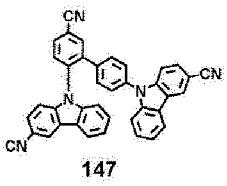
147

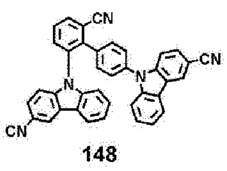
148

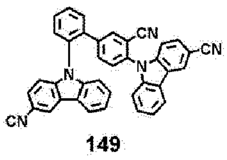
149

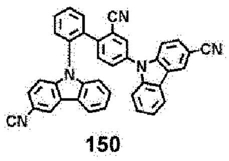
150

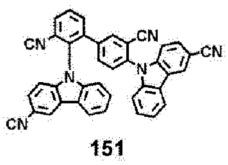
151

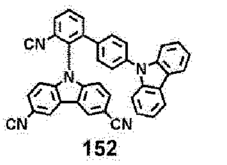
152

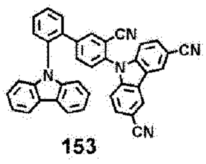
153

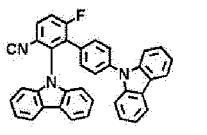
154

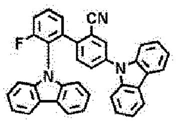
155

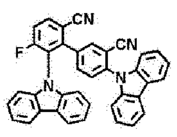
156

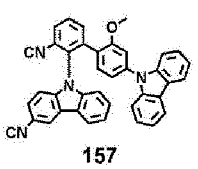
157

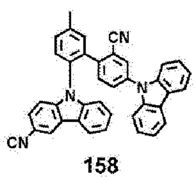
158

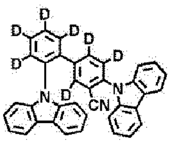
159

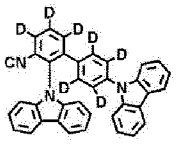
160

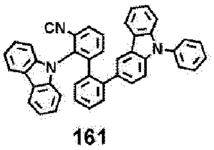
161
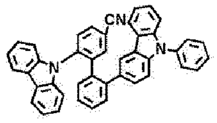
162
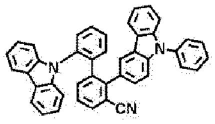
163
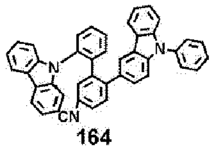
164
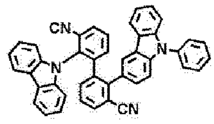
165
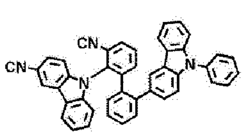
166
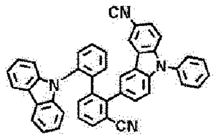
167
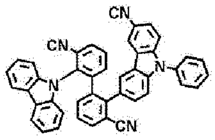
168
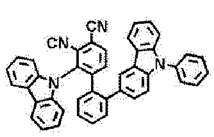
169
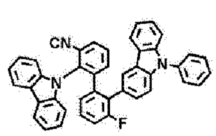
170
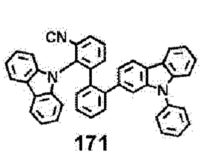
171
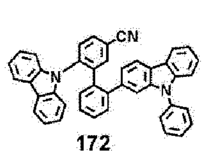
172
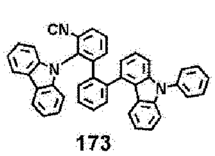
173
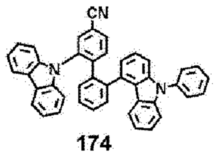
174
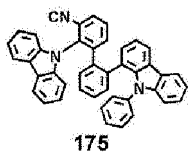
175
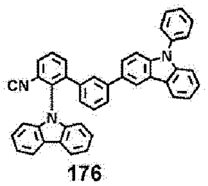
176
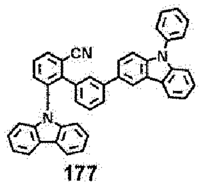
177
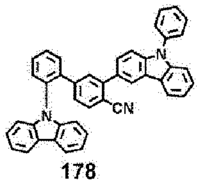
178
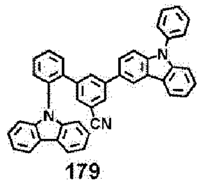
179
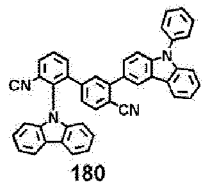
180
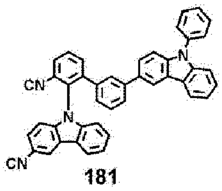
181
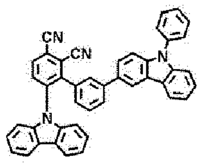
182
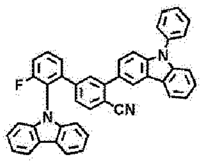
183
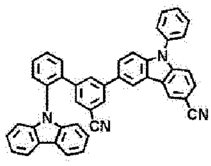
184
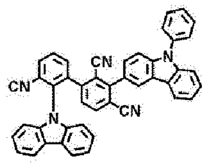
185
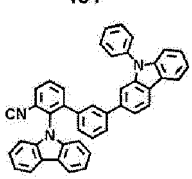
186
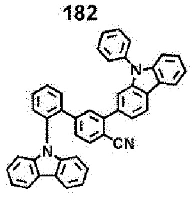
187
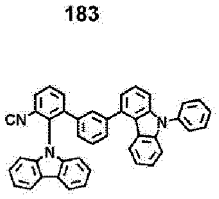
188
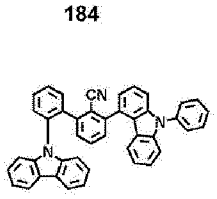
189
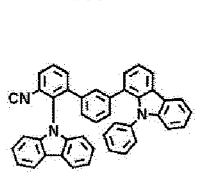
190
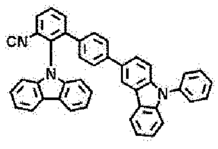
191
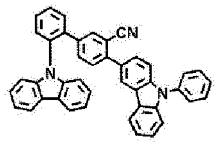
192
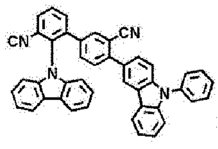
193
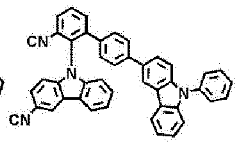
194
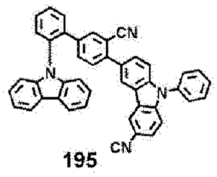
195
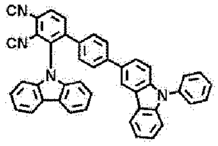
196
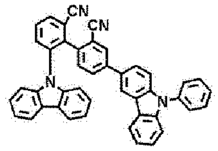
197
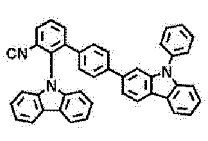
198
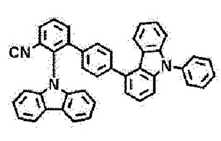
199
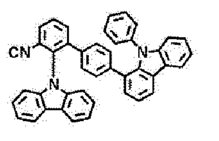
200

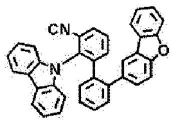
201

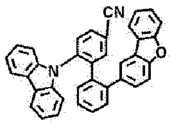
202

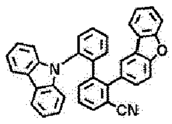
203

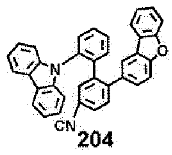
204

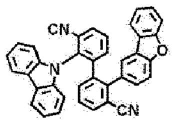
205

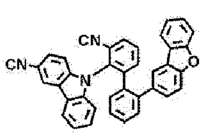
206

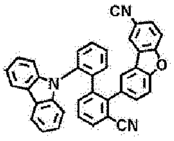
207

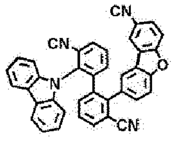
208

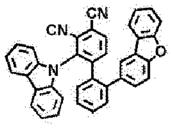
209

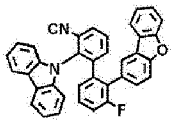
210

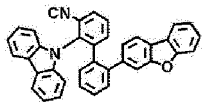
211

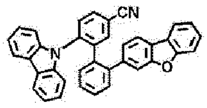
212

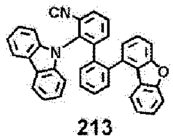
213

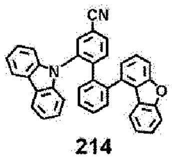
214

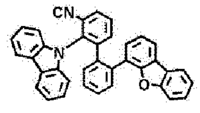
215

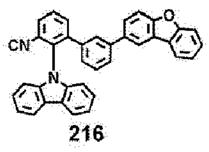
216

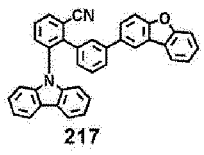
217

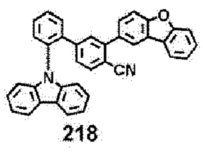
218

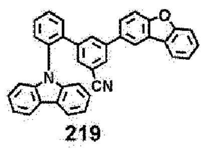
219

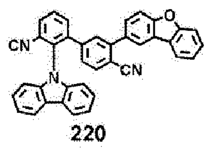
220

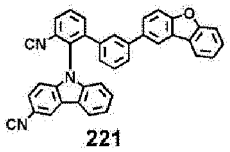
221

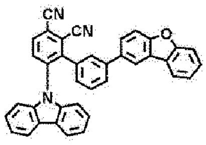
222

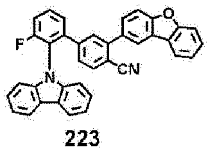
223

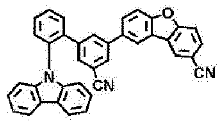
224

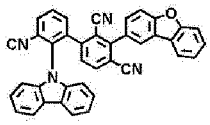
225

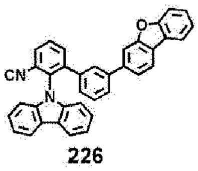
226

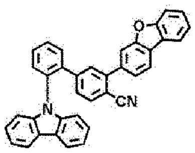
227

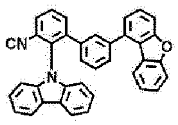
228

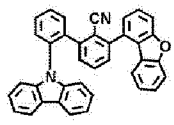
229

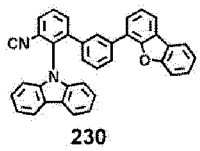
230

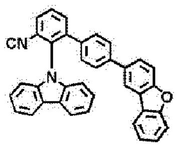
231

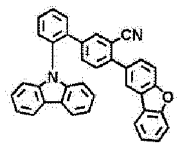
232

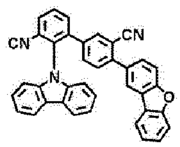
233

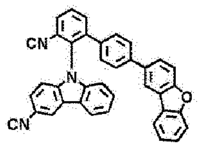
234

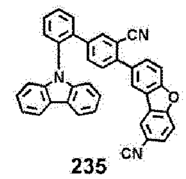
235

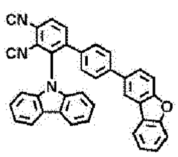
236

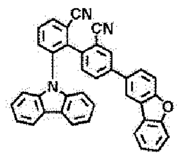
237

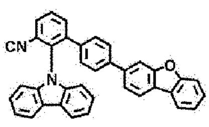
238

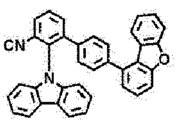
239

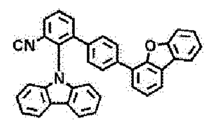
240

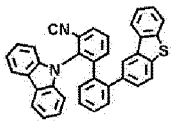
241
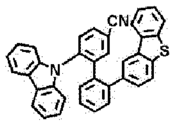
242
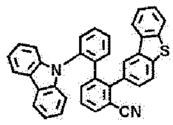
243
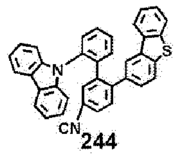
244
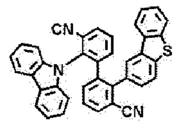
245

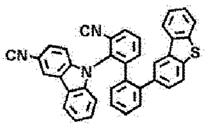
246
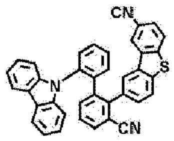
247
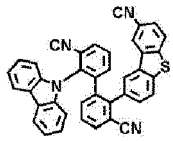
248
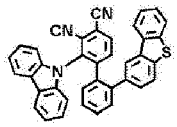
249
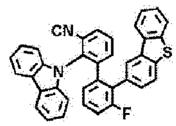
250

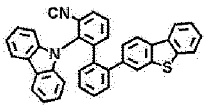
251
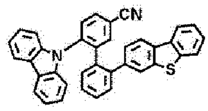
252
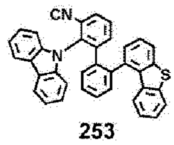
253
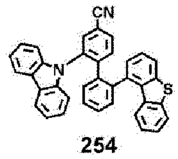
254
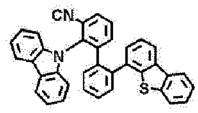
255

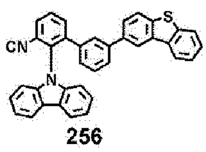
256
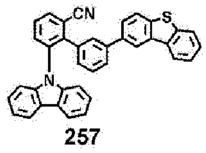
257
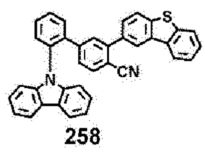
258
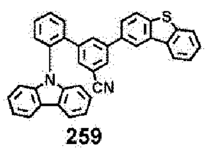
259
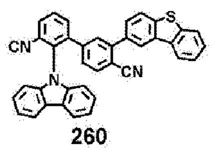
260

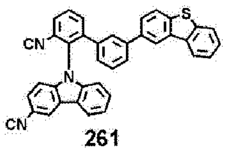
261
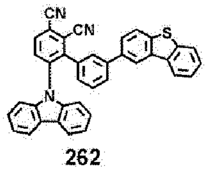
262
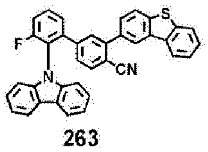
263
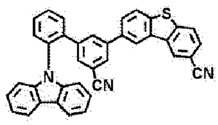
264
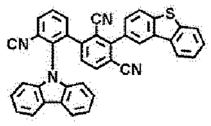
265

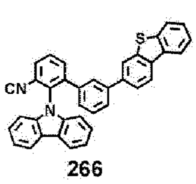
266
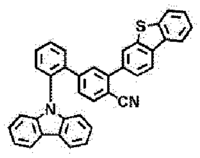
267
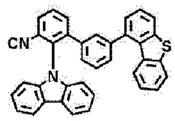
268
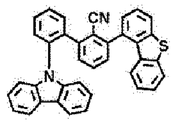
269
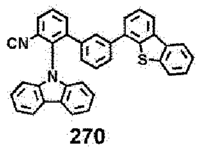
270

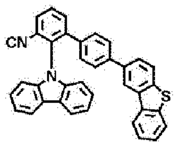
271
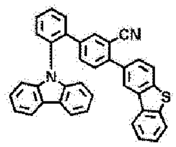
272
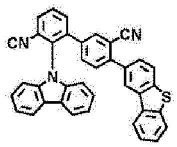
273
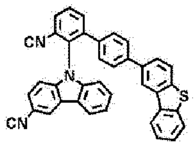
274
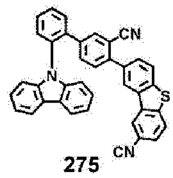
275

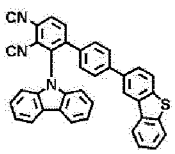
276
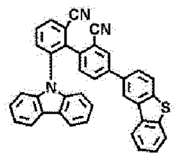
277
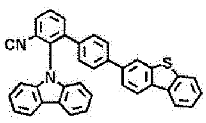
278
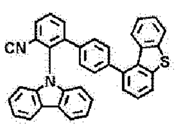
279
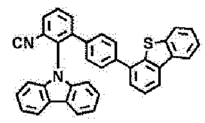
280

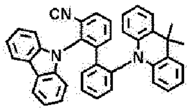
281
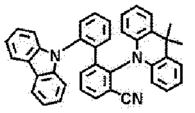
282
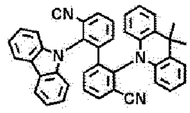
283
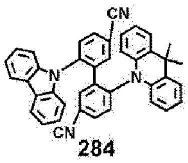
284
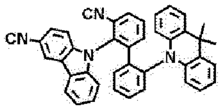
285

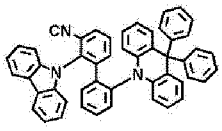
286
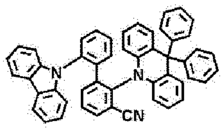
287
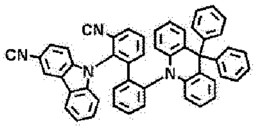
288
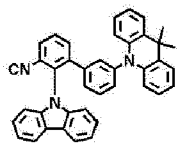
289
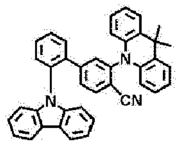
290

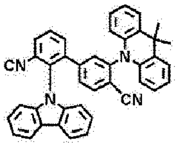
291
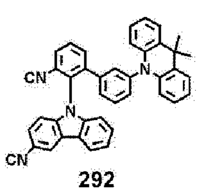
292
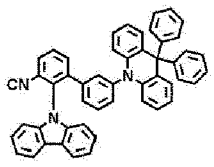
293
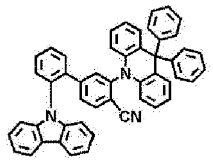
294
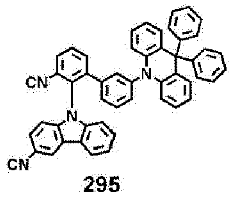
295

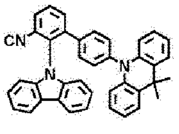
296
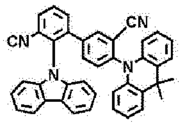
297
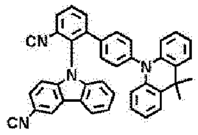
298
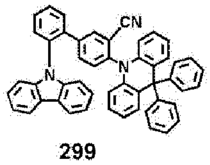
299
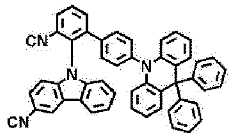
300

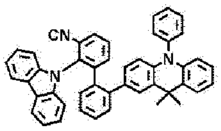
301
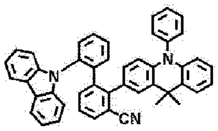
302
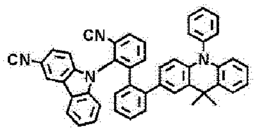
303
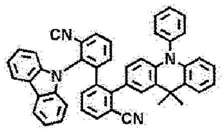
304
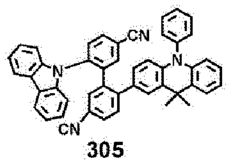
305

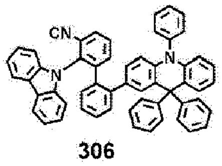
306
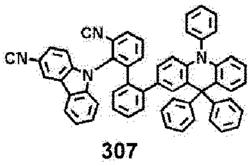
307
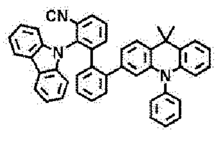
308
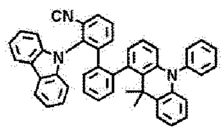
309
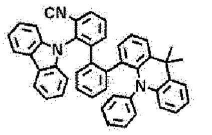
310

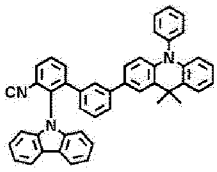
311
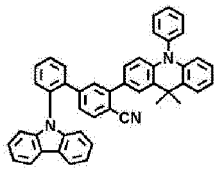
312
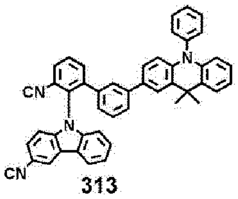
313
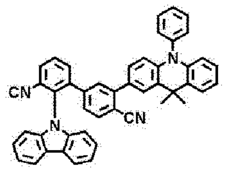
314
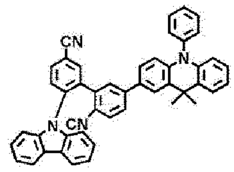
315

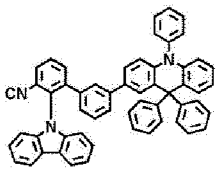
316
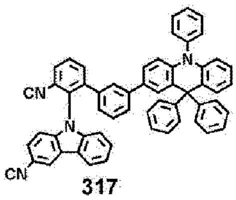
317
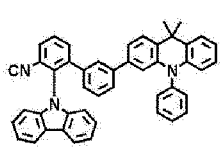
318
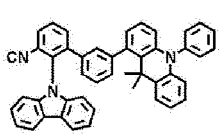
319
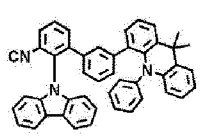
320

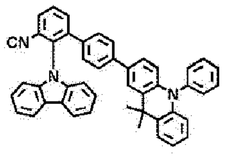

321

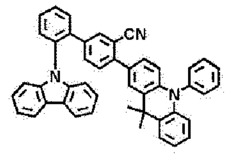

322

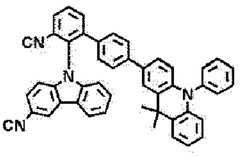

323

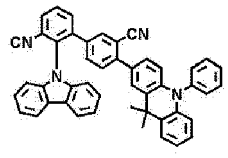

324

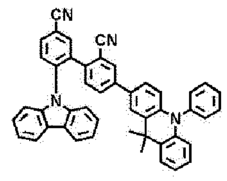

325

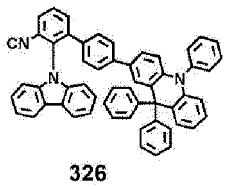

326

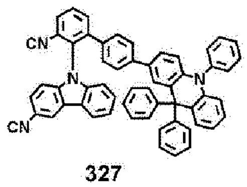

327

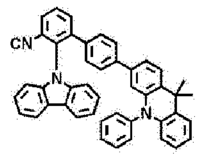

328

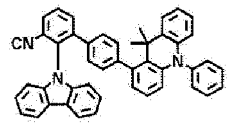

329

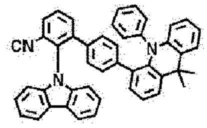

330

## Group HE7

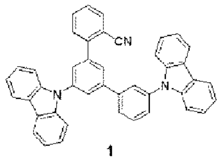
1

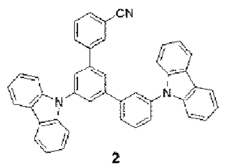
2

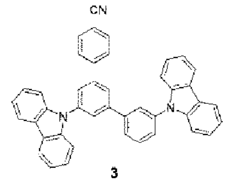
3

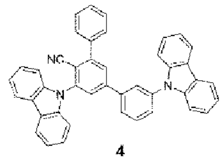
4

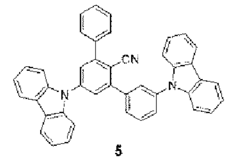
5

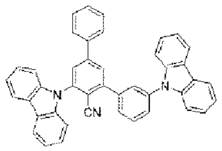
6

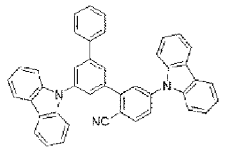
7

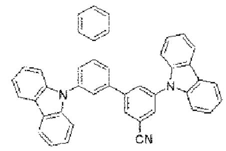
8

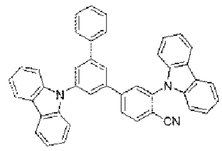
9

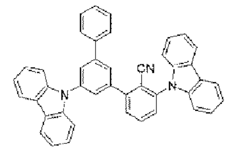
10

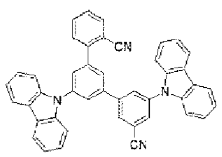
11

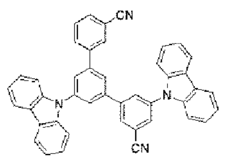
12

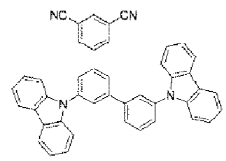
13

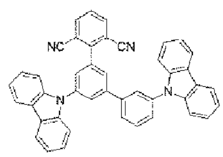
14

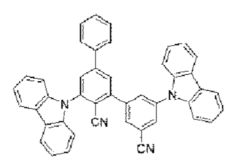
15

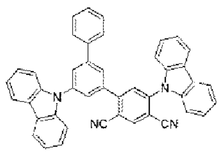
16

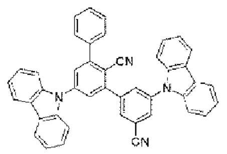
17

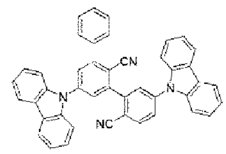
18

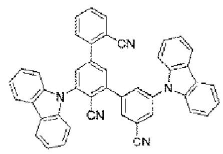
19

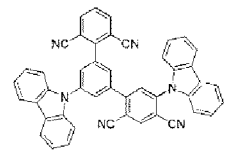
20

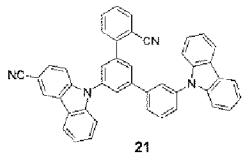
21

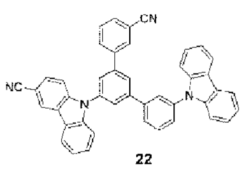
22

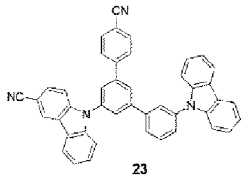
23

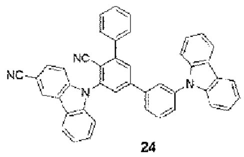
24

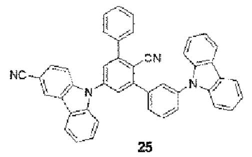
25

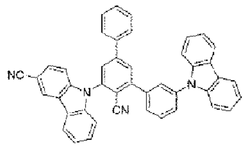
26

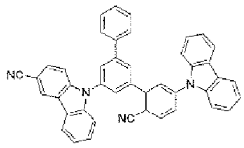
27

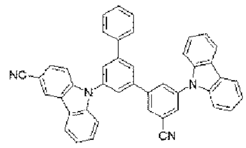
28

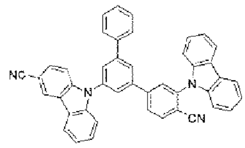
29

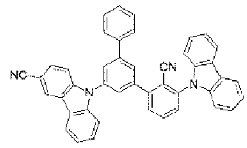
30

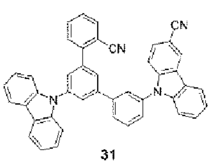
31

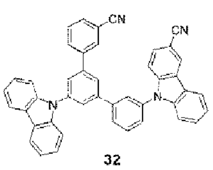
32

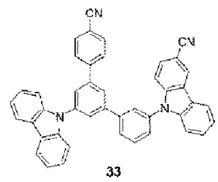
33

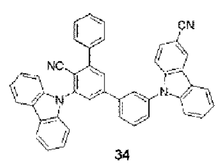
34

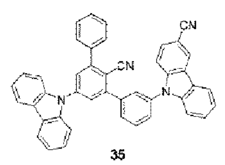
35

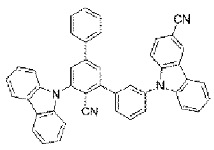
36

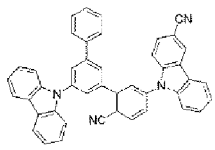
37

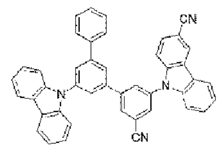
38

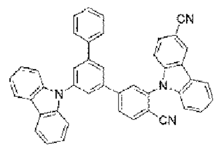
39

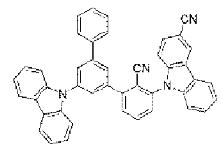
40

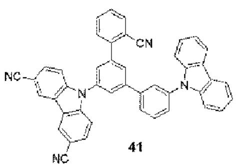
41
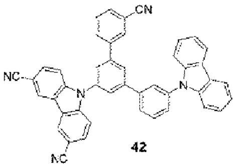
42
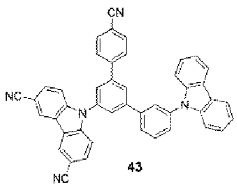
43
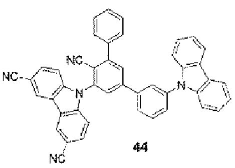
44
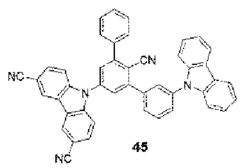
45
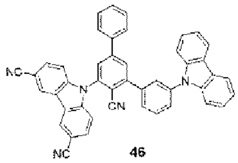
46
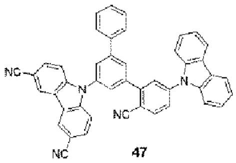
47
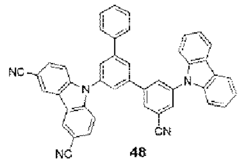
48
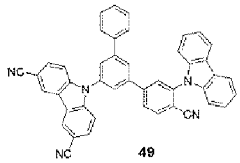
49
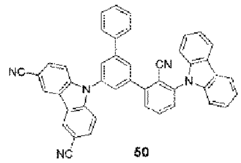
50
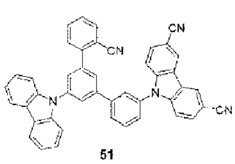
51
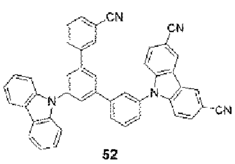
52
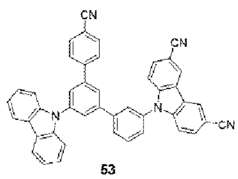
53
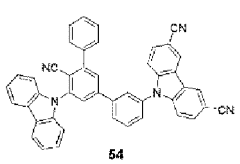
54
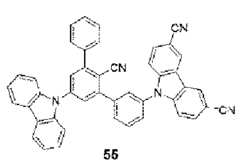
55
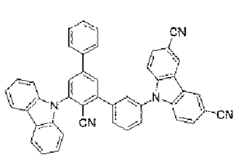
56
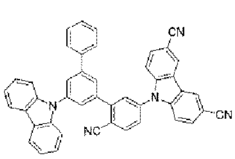
57
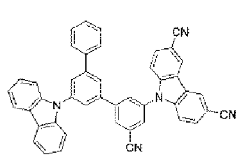
58
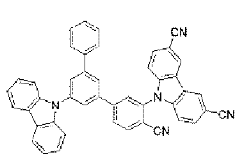
59
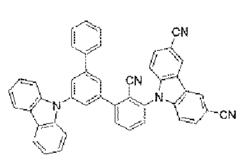
60
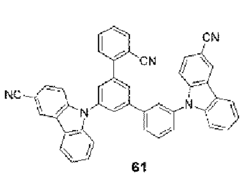
61
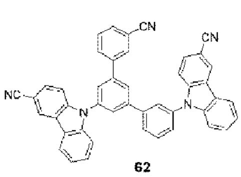
62
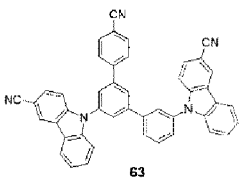
63
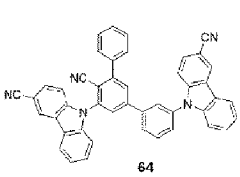
64
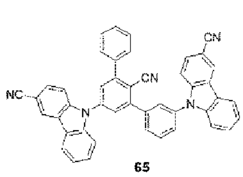
65
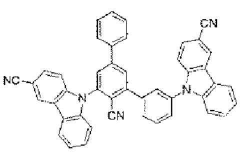
66
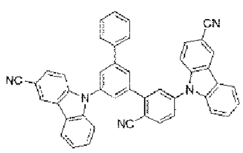
67
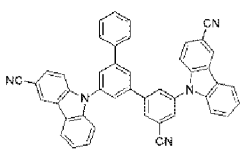
68
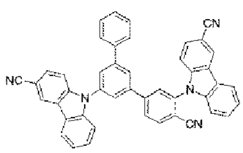
69
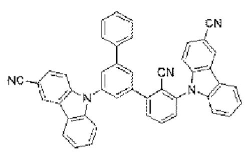
70
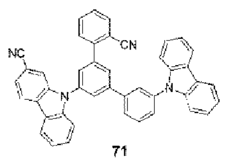
71
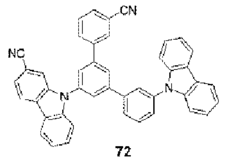
72
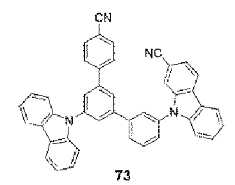
73
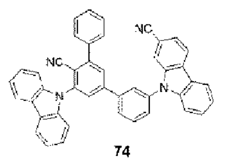
74
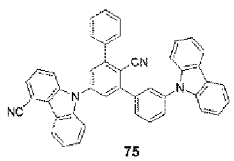
75
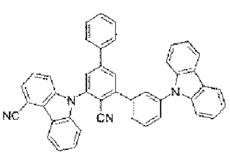
76
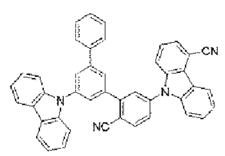
77
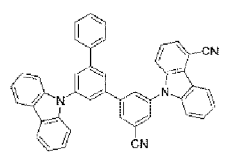
78
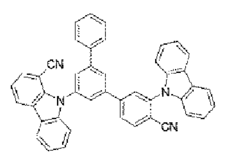
79
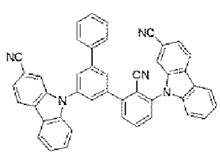
80

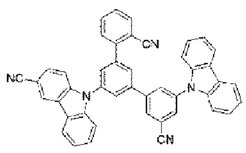
81
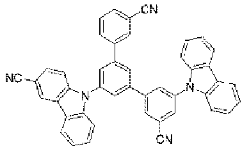
82
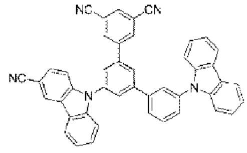
83
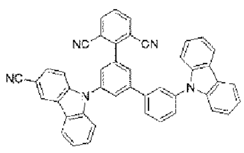
84
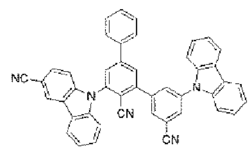
85

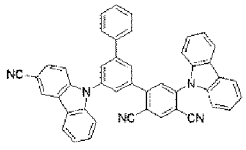
86
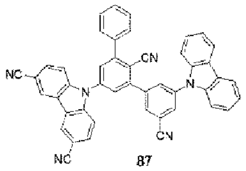
87
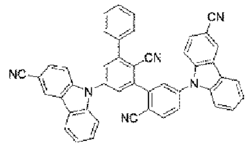
88
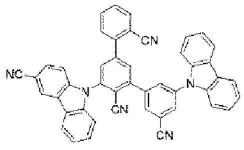
89
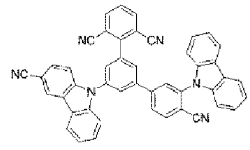
90

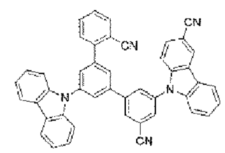
91
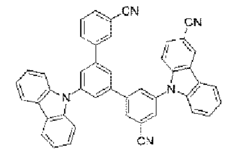
92
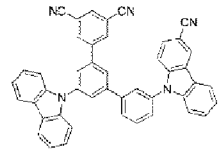
93
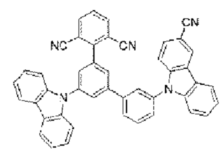
94
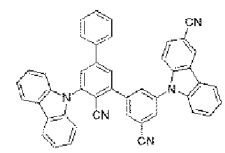
95

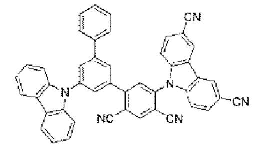
96
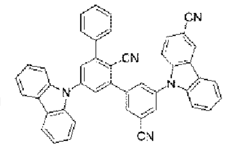
97
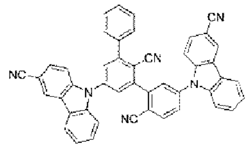
98
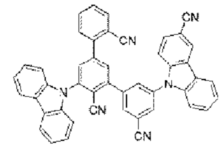
99
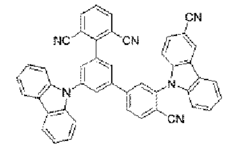
100

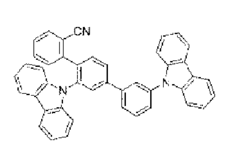
101
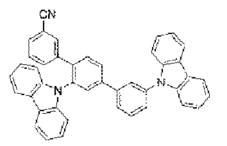
102
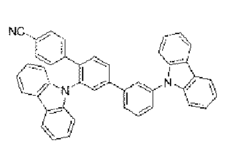
103
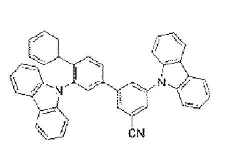
104
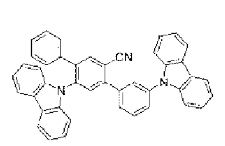
105

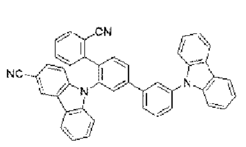
106
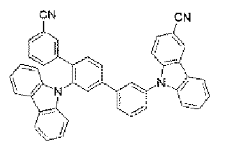
107
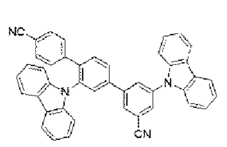
108
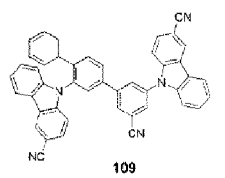
109
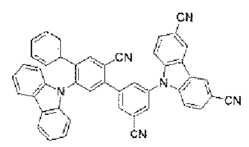
110

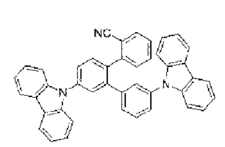
111
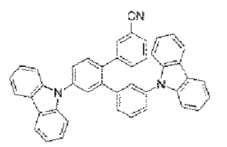
112
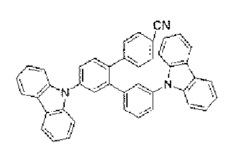
113
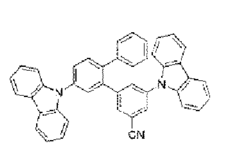
114
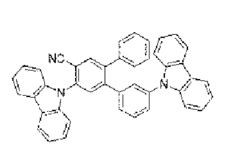
115

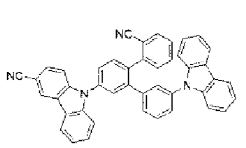
116
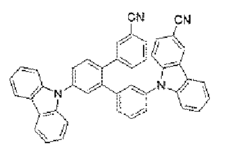
117
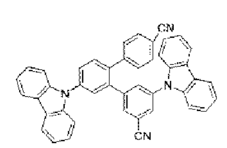
118
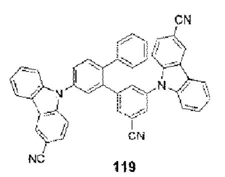
119
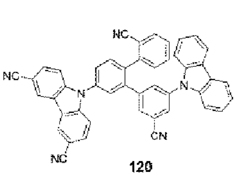
120

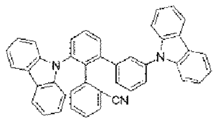
121
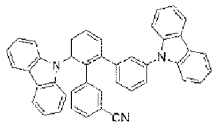
122
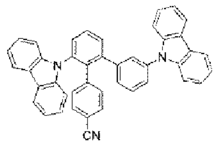
123
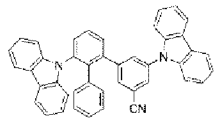
124
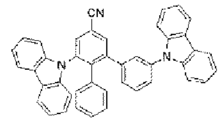
125

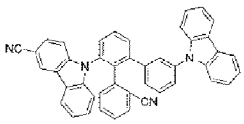
126
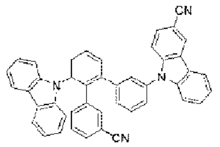
127
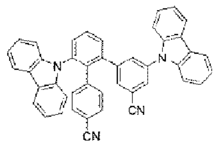
128
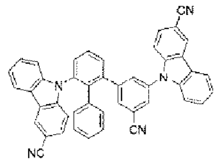
129
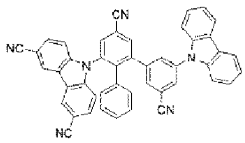
130

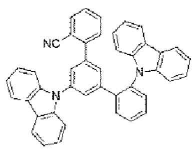
131
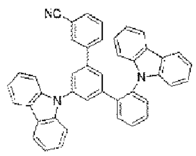
132
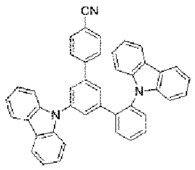
133
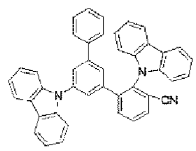
134
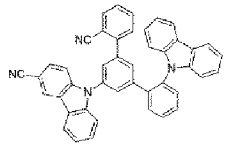
135

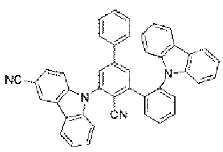
136
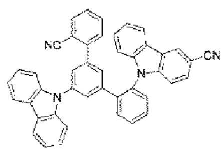
137
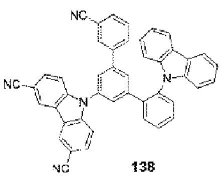
138
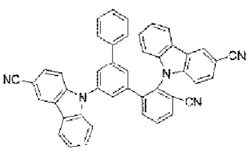
139
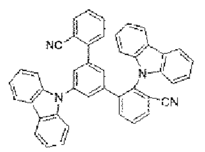
140

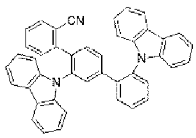
141
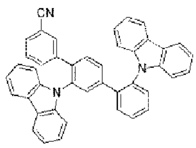
142
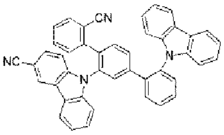
143
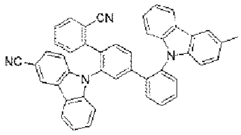
144
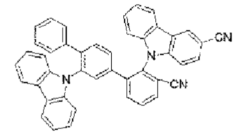
145

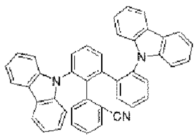
146
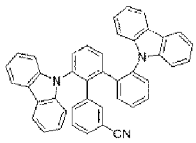
147
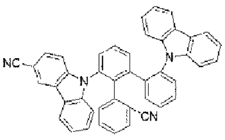
148
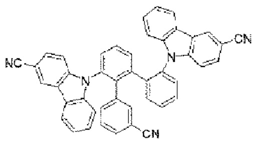
149
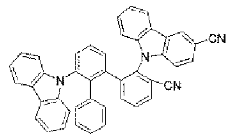
150

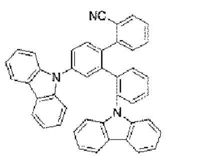
151
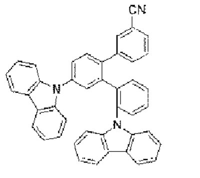
152
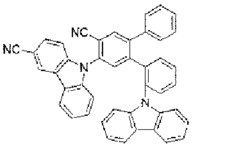
153
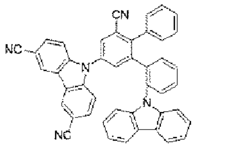
154
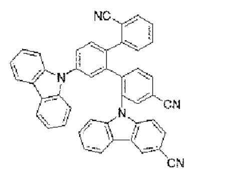
155

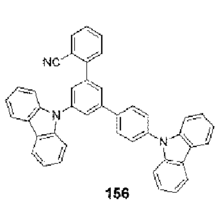
156
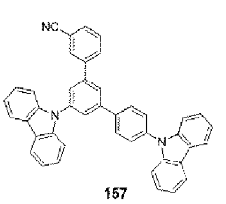
157
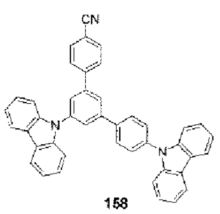
158
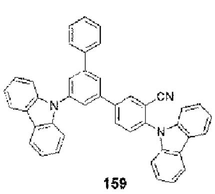
159
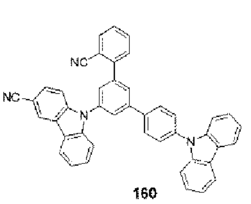
160

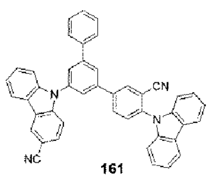
161
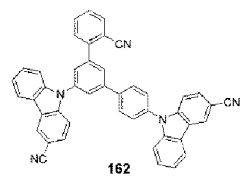
162
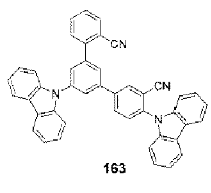
163
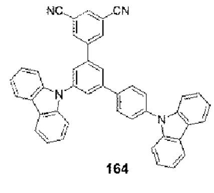
164
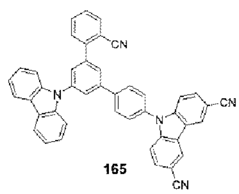
165

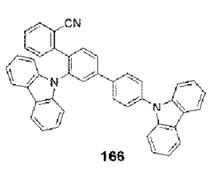
166
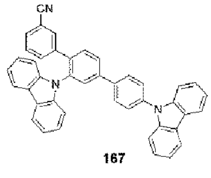
167
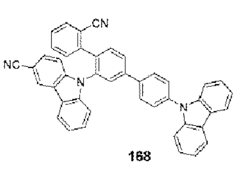
168
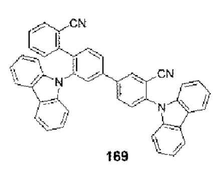
169
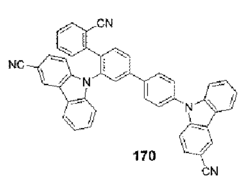
170

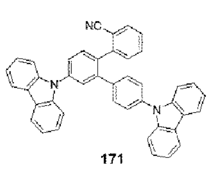
171
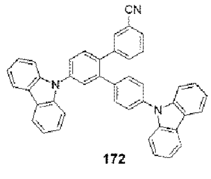
172
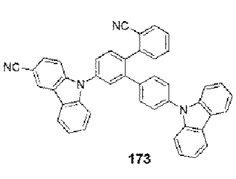
173
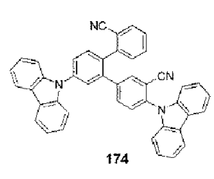
174
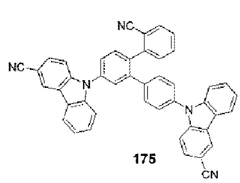
175

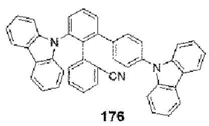
176
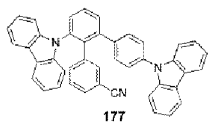
177
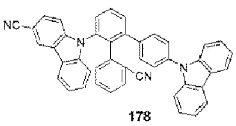
178
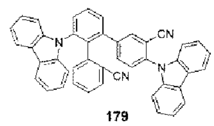
179
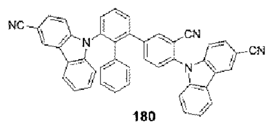
180

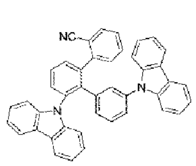
181
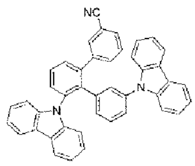
182
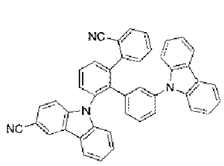
183
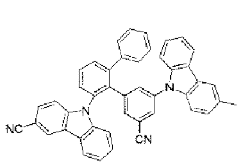
184
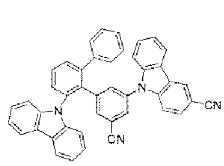
185

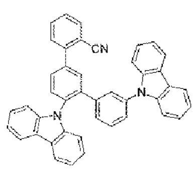
186
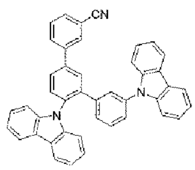
187
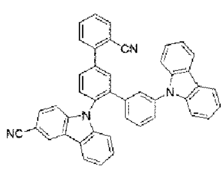
188
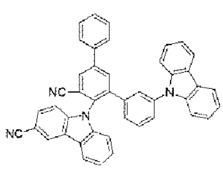
189
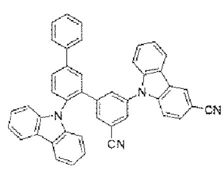
190

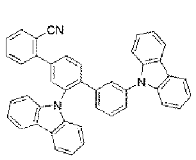
191
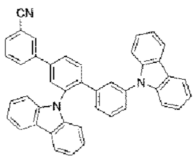
192
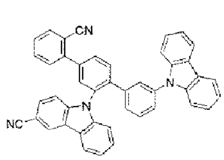
193
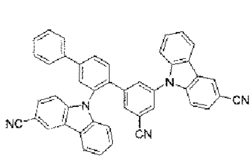
194
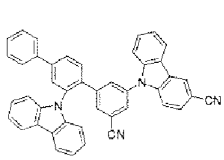
195

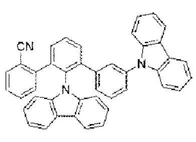
196
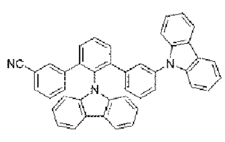
197
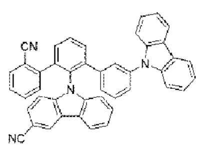
198
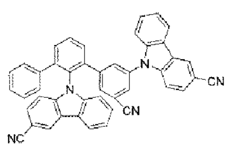
199
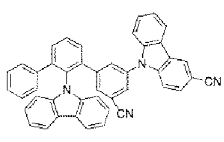
200

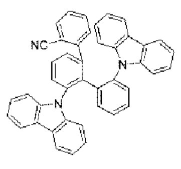
201
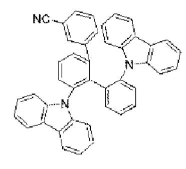
202
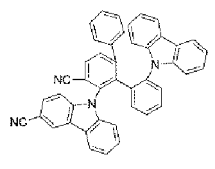
203
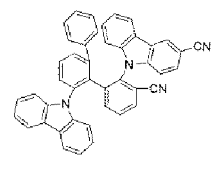
204
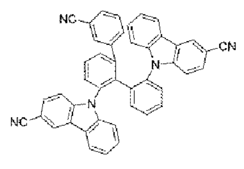
205

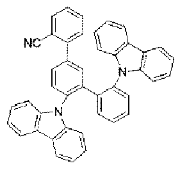
206
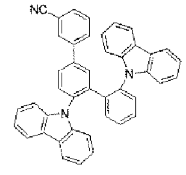
207
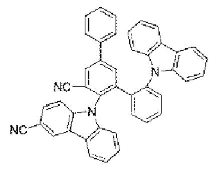
208
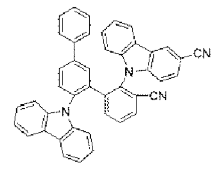
209
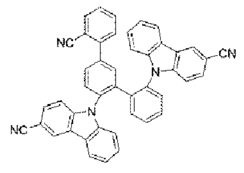
210

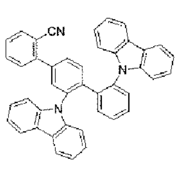
211
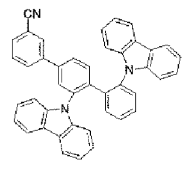
212
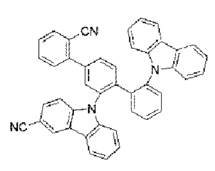
213
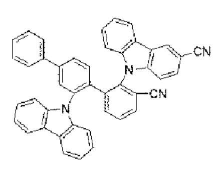
214
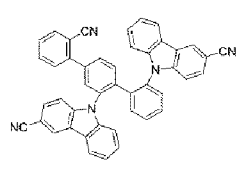
215

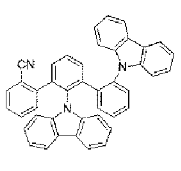
216
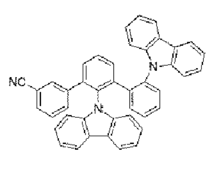
217
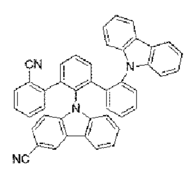
218
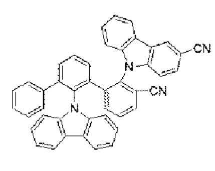
219
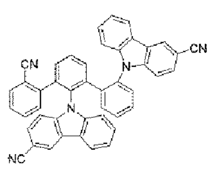
220

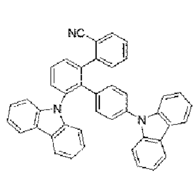
221
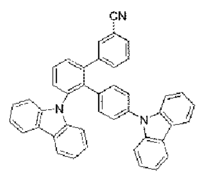
222
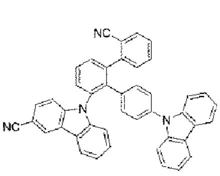
223
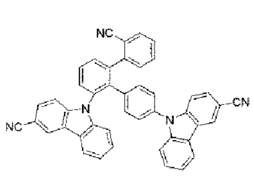
224
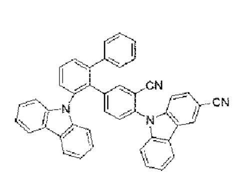
225

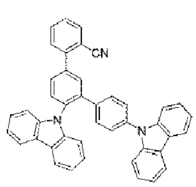
226
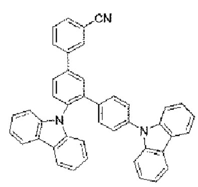
227
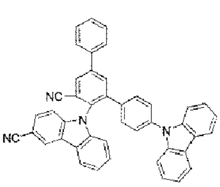
228
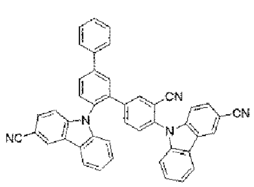
229
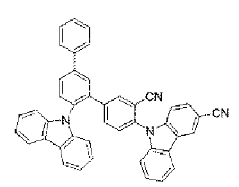
230

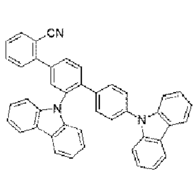
231
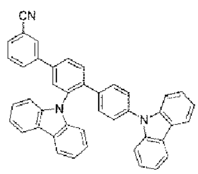
232
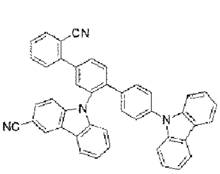
233
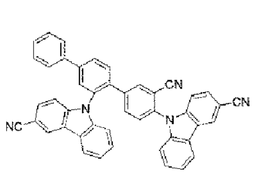
234
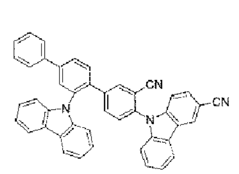
235

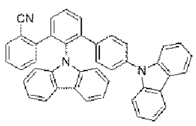
236
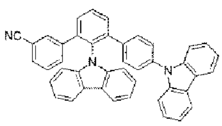
237
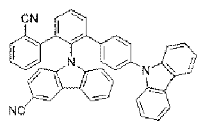
238
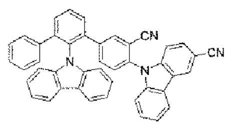
239
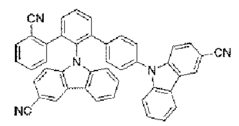
240

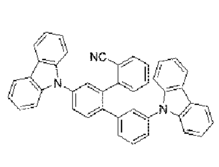
241
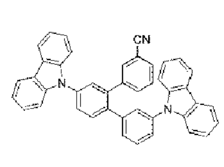
242
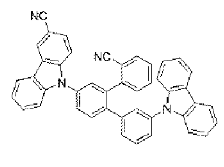
243
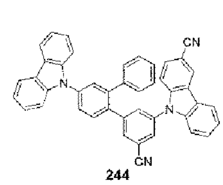
244
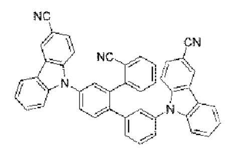
245

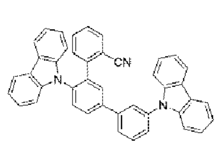
246
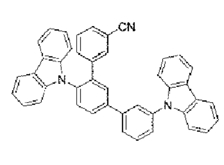
247
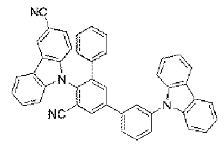
248
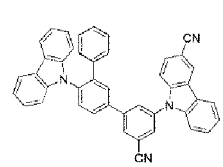
249
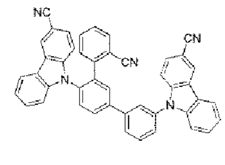
250

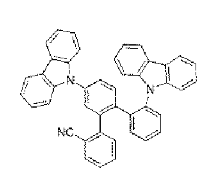
251
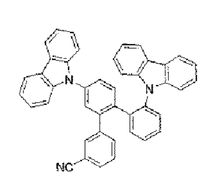
252
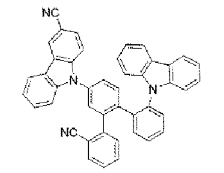
253
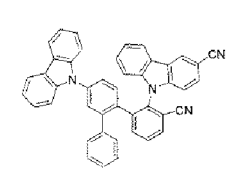
254
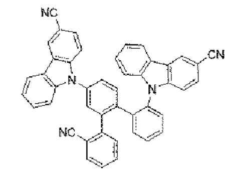
255

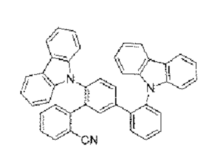
256
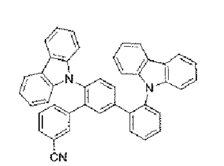
257
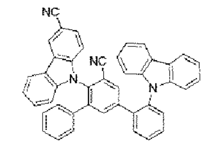
258
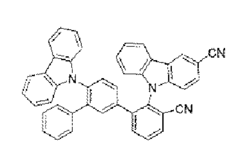
259
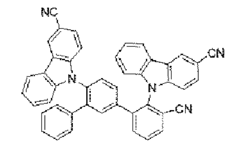
260

In one or more embodiments, the hole-transporting host may be one of Compounds H-H1 to H-H104, but embodiments of the present disclosure are not limited thereto:

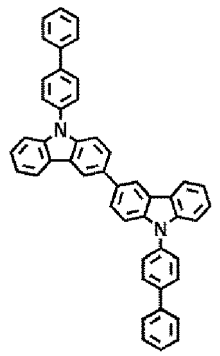
H-H1
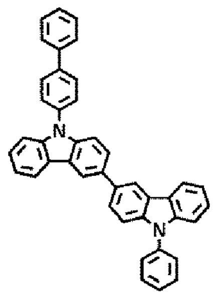
H-H2
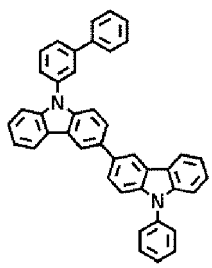
H-H3
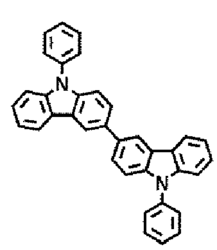
H-H4

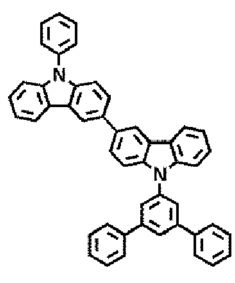
H-H5
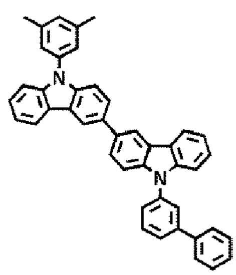
H-H6
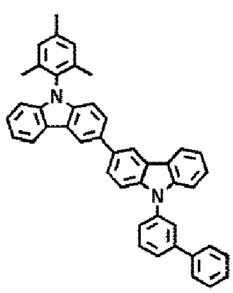
H-H7
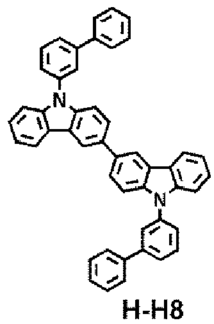
H-H8

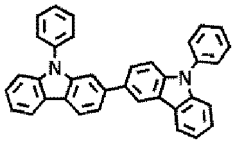
H-H9

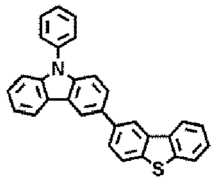
H-H10

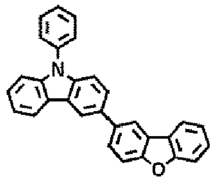
H-H11

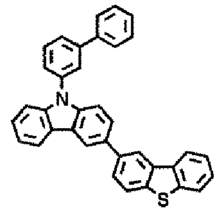
H-H12

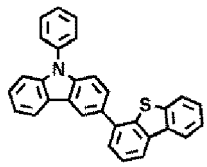
H-H13

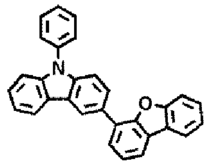
H-H14

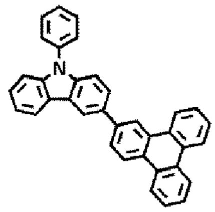
H-H15

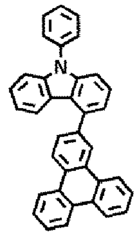
H-H16

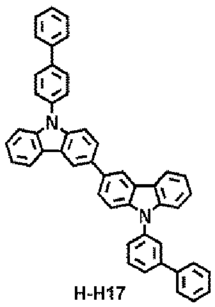
H-H17

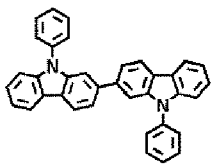
H-H18

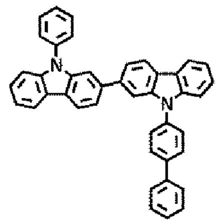
H-H19

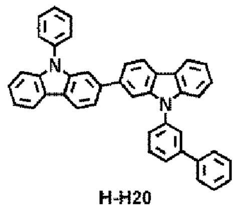
H-H20

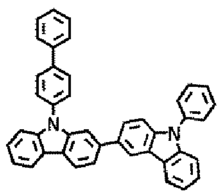
H-H21

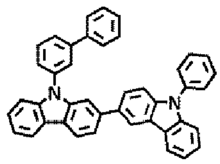
H-H22

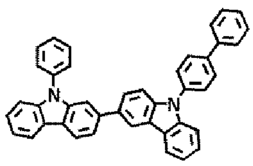
H-H23

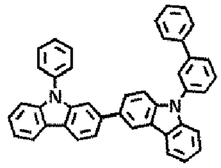
H-H24

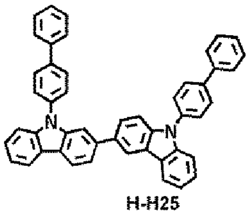
H-H25

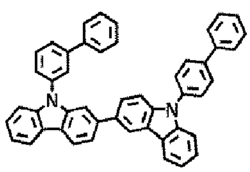
H-H26

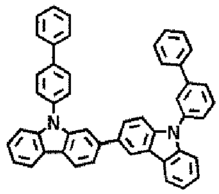
H-H27

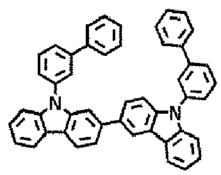
H-H28

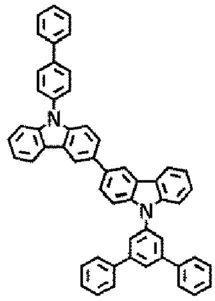

H-H29

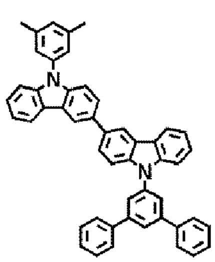

H-H30

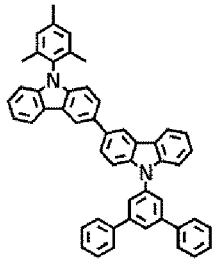

H-H31

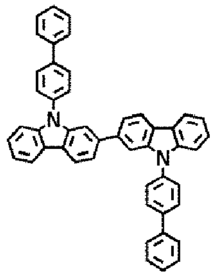

H-H32

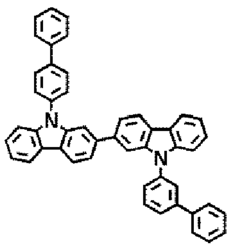

H-H33

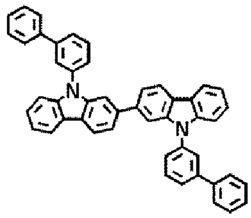

H-H34

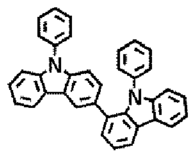

H-H35

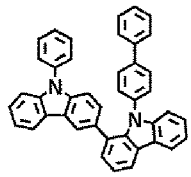

H-H36

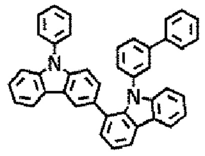

H-H37

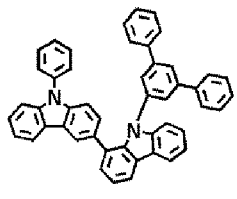

H-H38

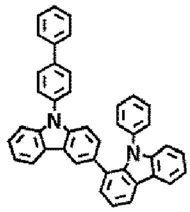

H-H39

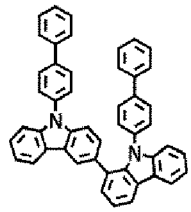

H-H40

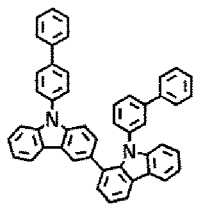

H-H41

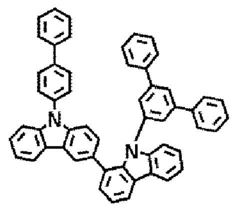

H-H42

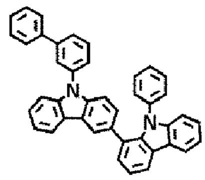

H-H43

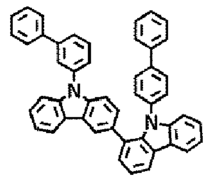

H-H44

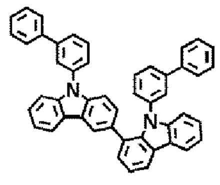

H-H45

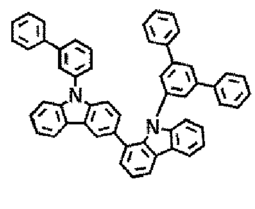

H-H46

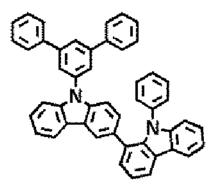

H-H47

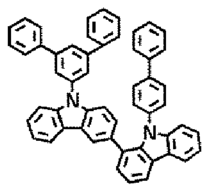

H-H48

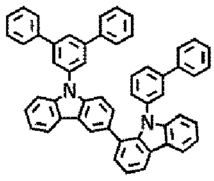

H-H49

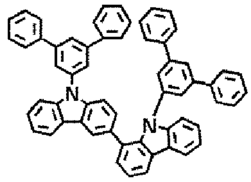

H-H50

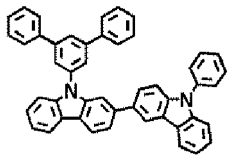

H-H51

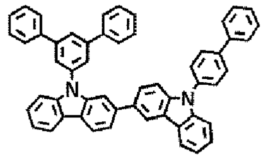

H-H52

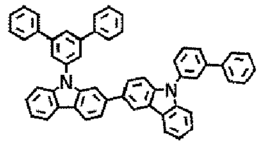

H-H53

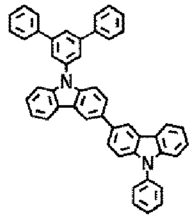

H-H54

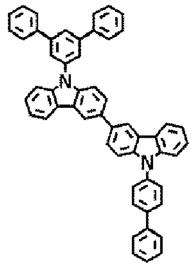

H-H55

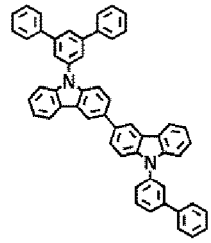

H-H56

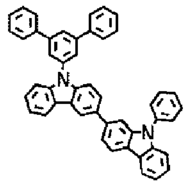

H-H57

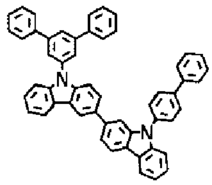

H-H58

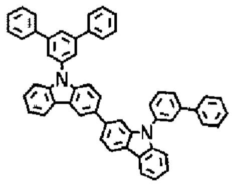

H-H59

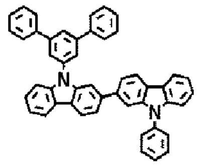

H-H60

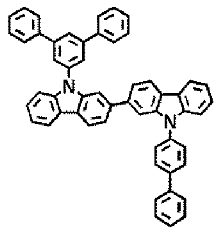

H-H61

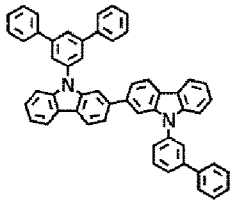

H-H62

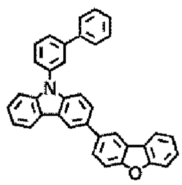

H-H63

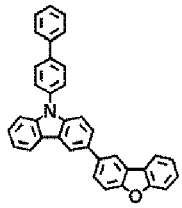

H-H64

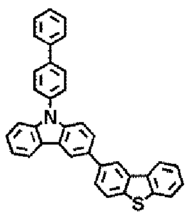

H-H65

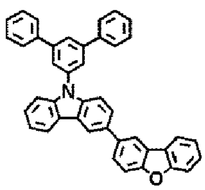

H-H66

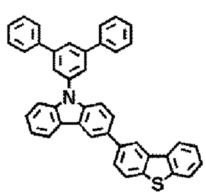

H-H67

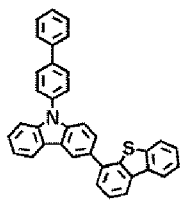

H-H68

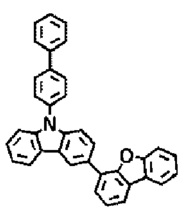

H-H69

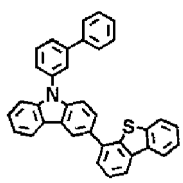

H-H70

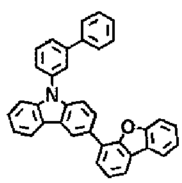

H-H71

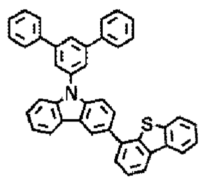

H-H72

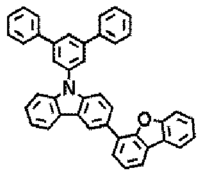
H-H73

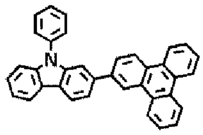
H-H74

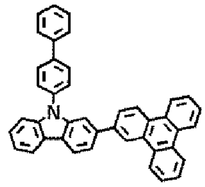
H-H75

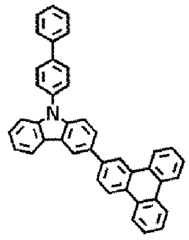
H-H76

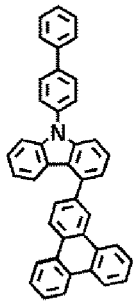
H-H77

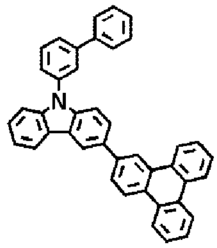
H-H78

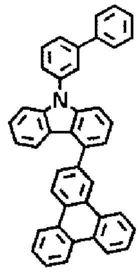
H-H79

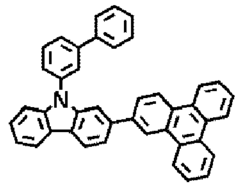
H-H80

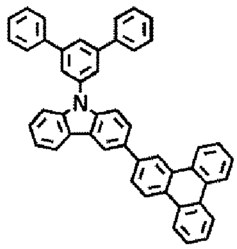
H-H81

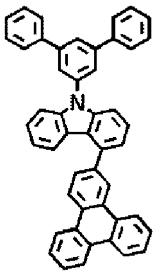
H-H82

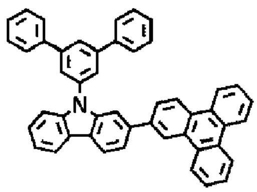
H-H83

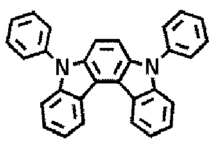
H-H84

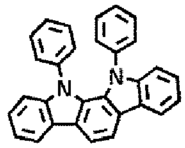
H-H85

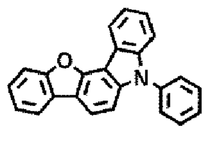
H-H86

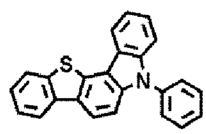
H-H87

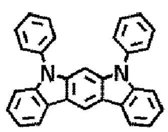
H-H88

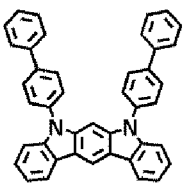
H-H89

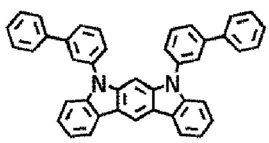
H-H90

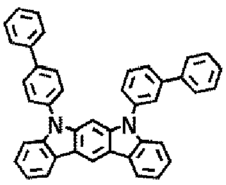
H-H91

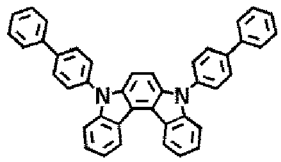

H-H92

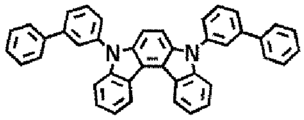

H-H93

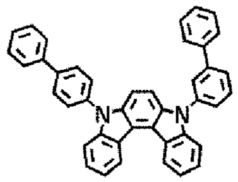

H-H94

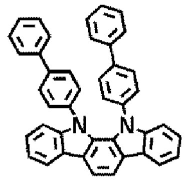

H-H95

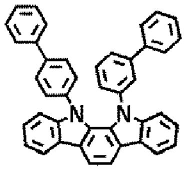

H-H96

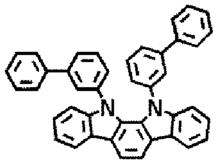

H-H97

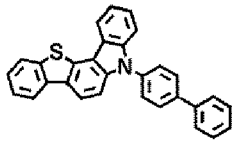

H-H98

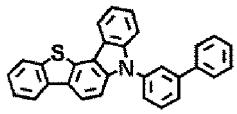

H-H99

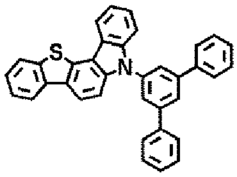

H-H100

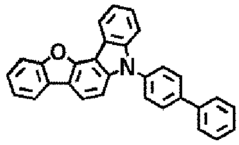

H-H101

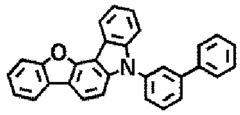

H-H102

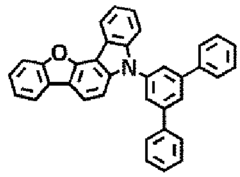

H-H103

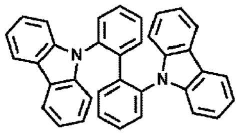

H-H104

In one or more embodiments, the bipolar host may be a compound from group HEH1, but embodiments of the present disclosure are not limited thereto:

Group HEH1

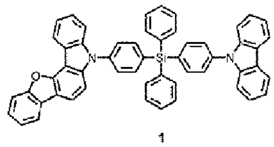
1

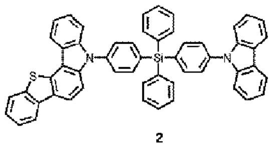
2

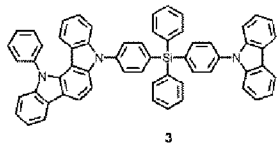
3

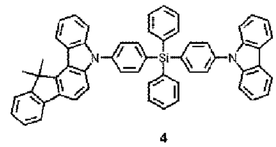
4

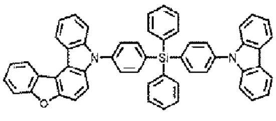
5

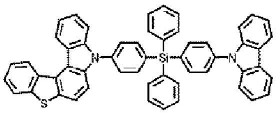
6

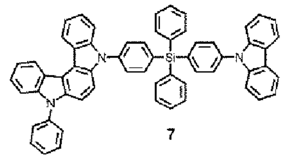
7

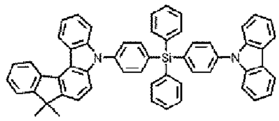
8

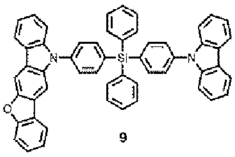
9

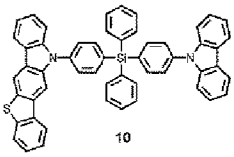
10

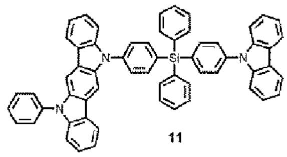
11

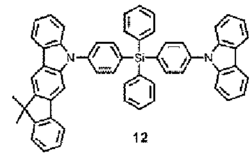
12

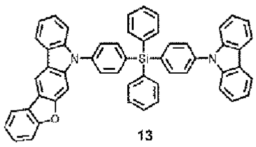
13

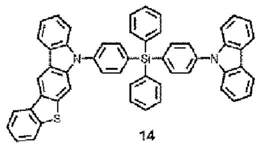
14

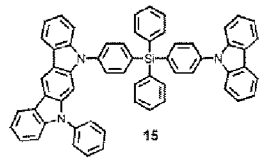
15

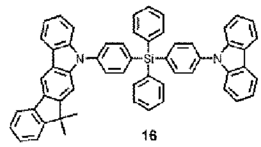
16

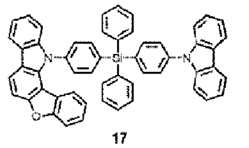
17

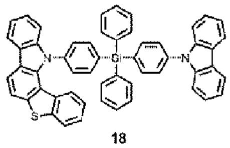
18

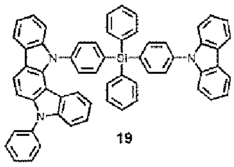
19

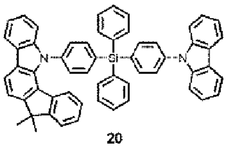
20

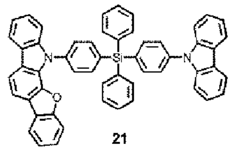
21

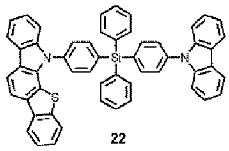
22

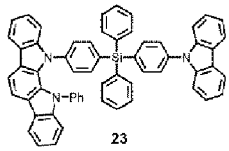
23

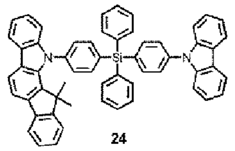
24

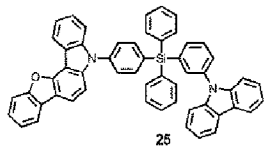
25

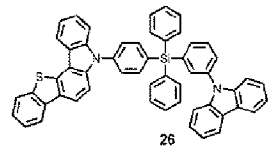
26

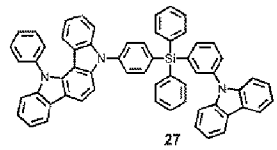
27

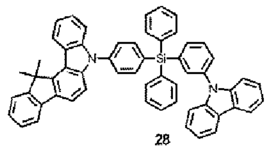
28

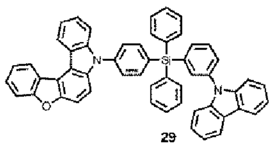
29

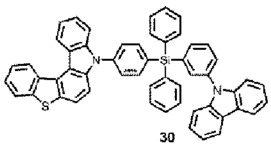
30

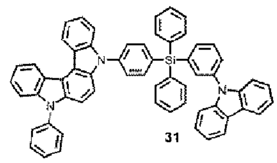
31

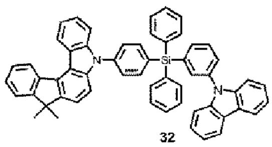
32

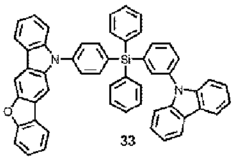
33

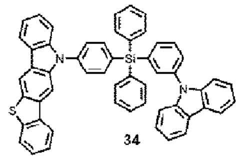
34

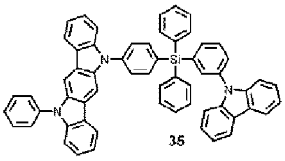
35

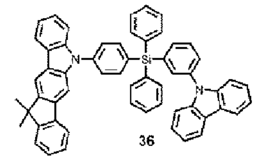
36

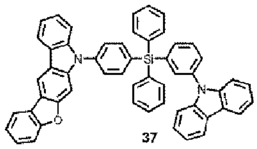
37

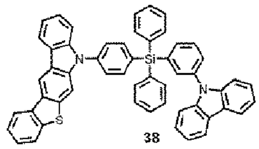
38

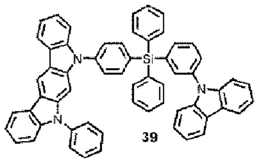
39

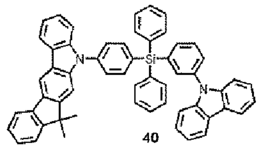
40

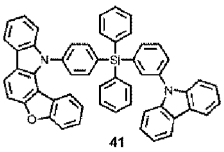
41

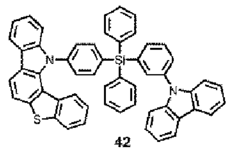
42

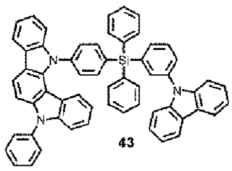
43

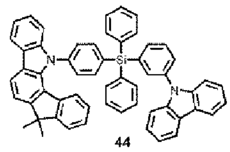
44

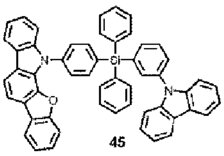
45

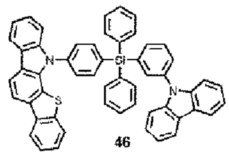
46

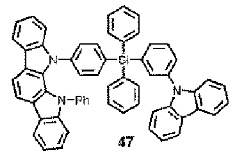
47

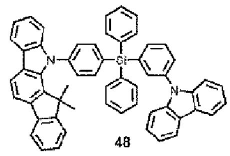
48

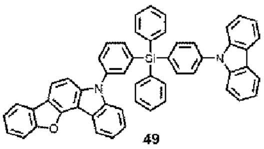
49

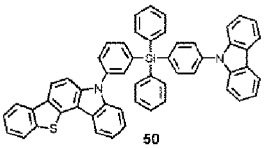
50

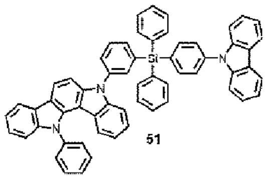
51

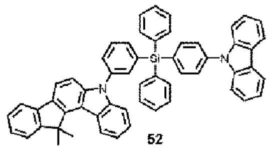
52

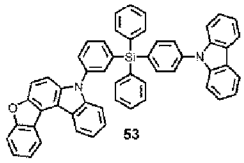
53

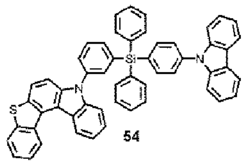
54

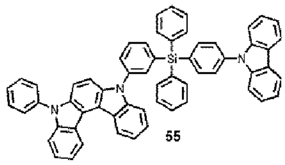
55

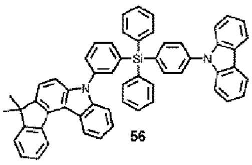
56

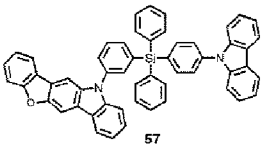
57

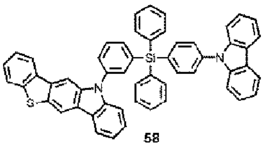
58

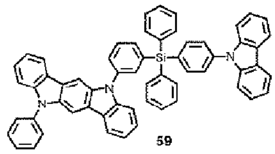
59

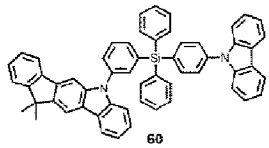
60

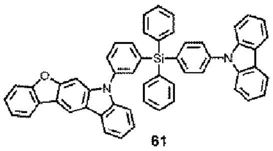
61

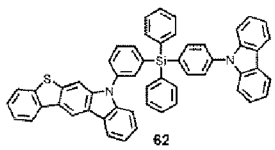
62

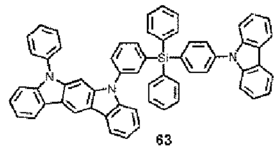
63

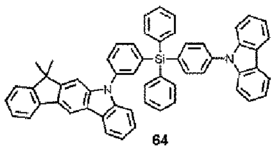
64

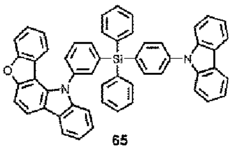
65

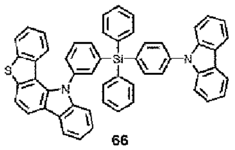
66

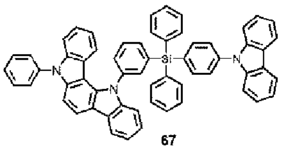
67

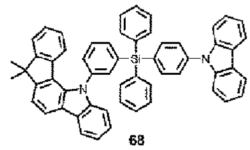
68

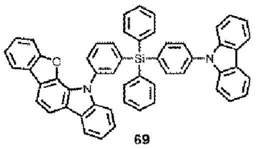
69

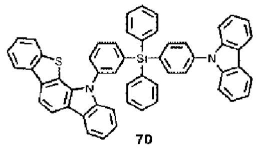
70

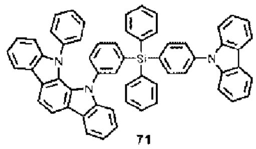
71

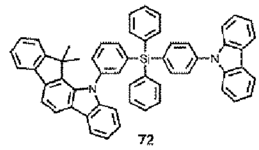
72

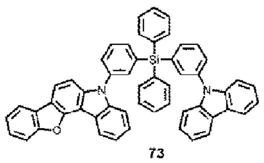
73

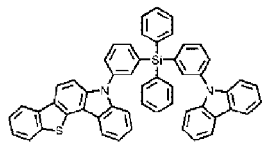
74

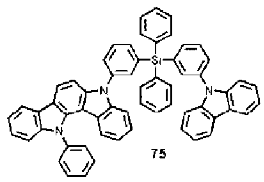
75

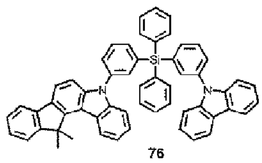
76

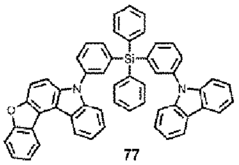
77

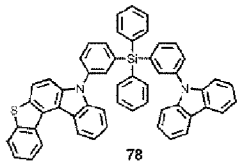
78

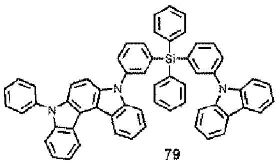
79

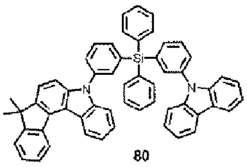
80

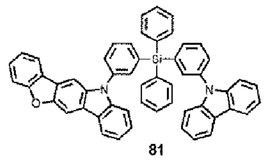
81
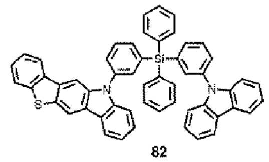
82
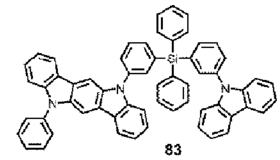
83
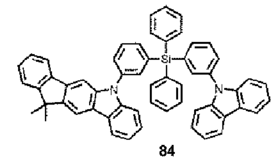
84

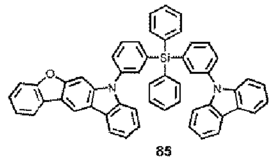
85
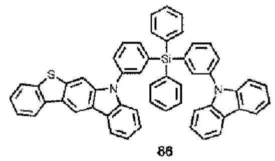
86
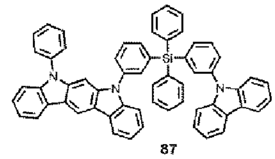
87
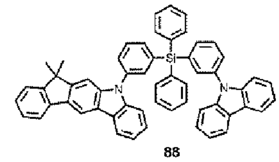
88

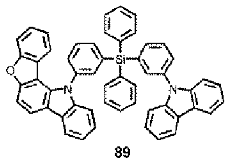
89
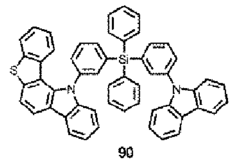
90
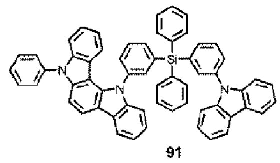
91
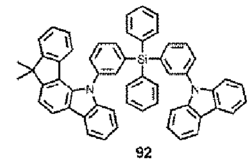
92

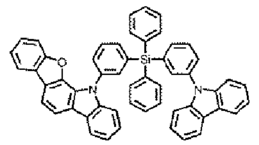
93
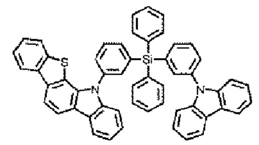
94
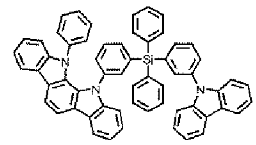
95
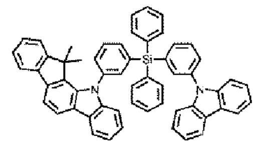
96

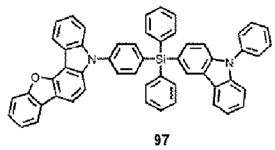
97
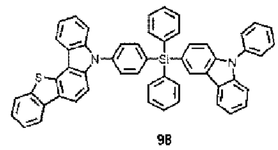
98
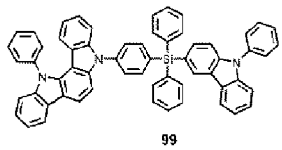
99
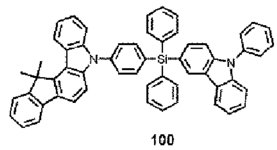
100

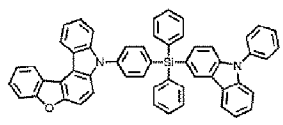
101
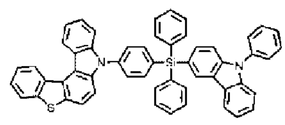
102
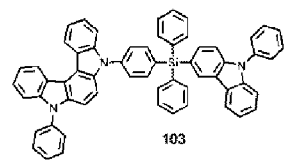
103
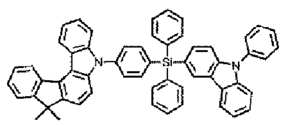
104

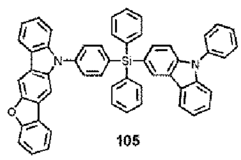
105
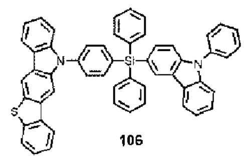
106
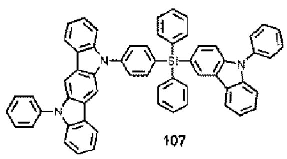
107
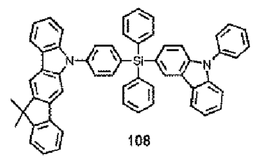
108

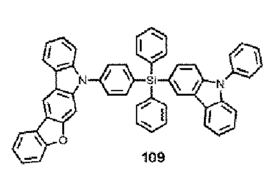
109
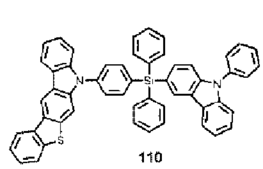
110
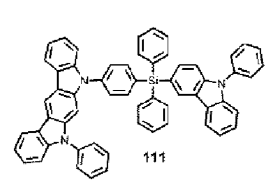
111
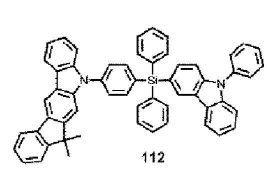
112

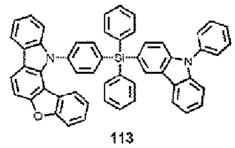
113

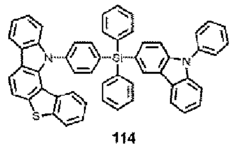
114

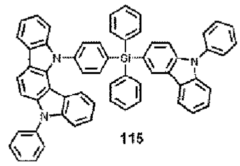
115

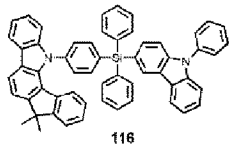
116

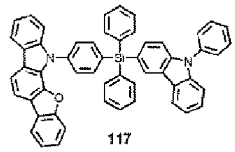
117

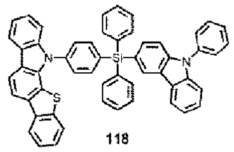
118

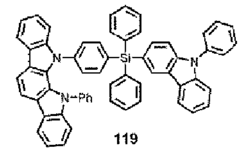
119

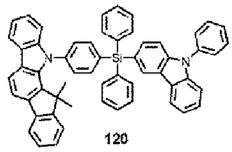
120

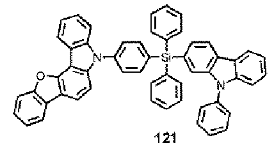
121

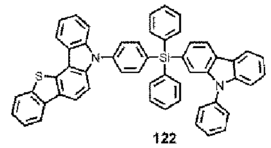
122

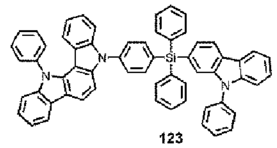
123

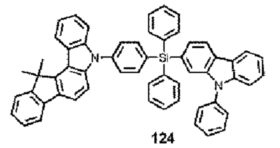
124

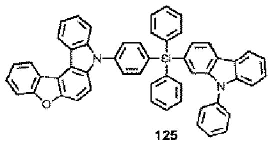
125

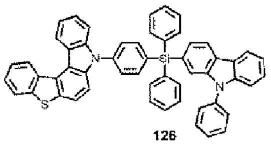
126

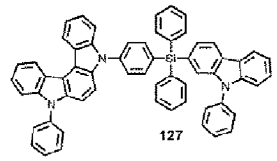
127

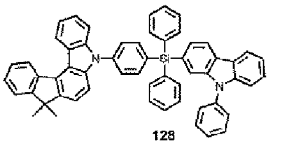
128

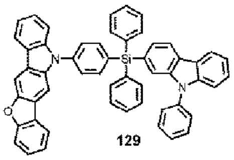
129

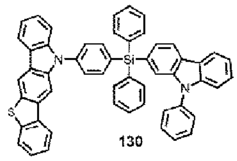
130

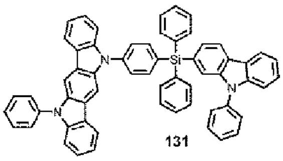
131

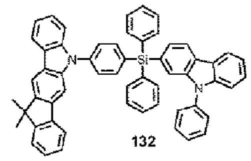
132

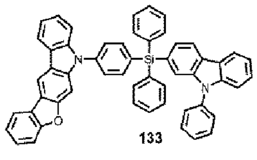
133

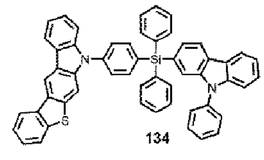
134

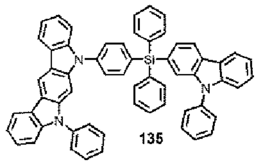
135

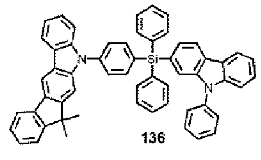
136

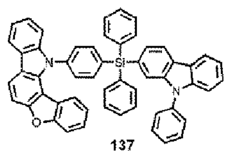
137

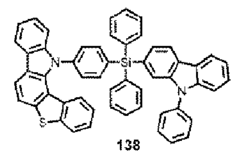
138

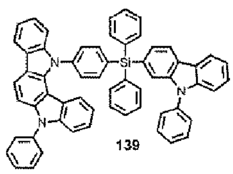
139

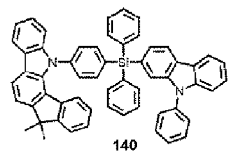
140

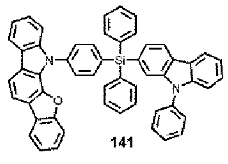
141

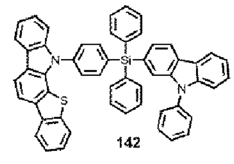
142

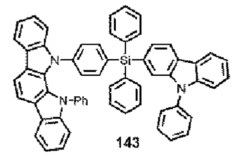
143

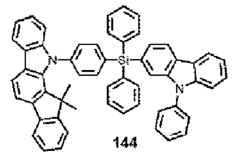
144

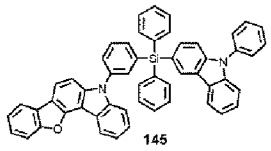
145

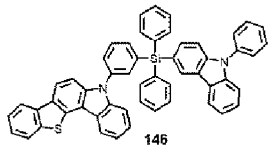
146

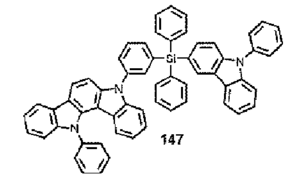
147

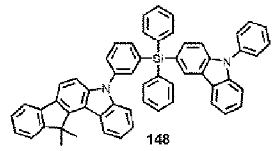
148

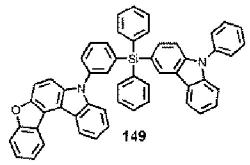
149

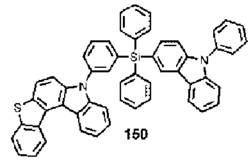
150

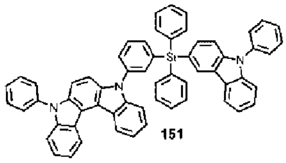
151

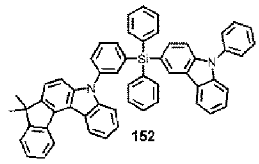
152

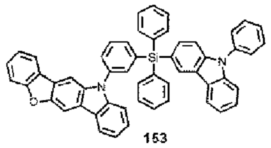
153

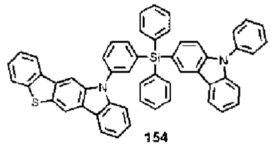
154

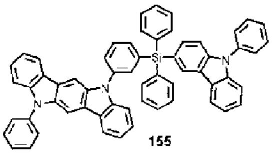
155

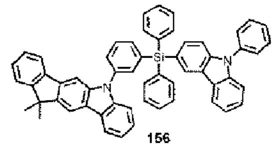
156

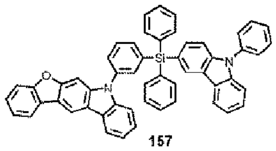
157

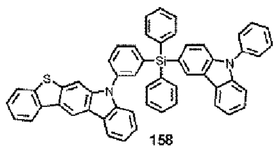
158

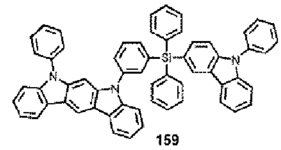
159

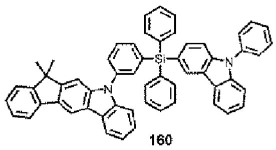
160

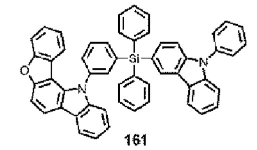
161

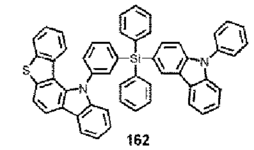
162

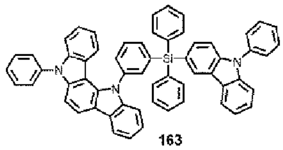
163

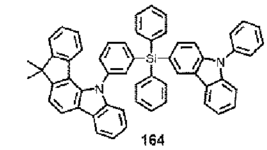
164

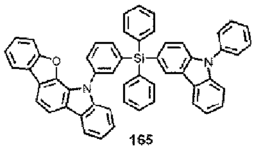
165

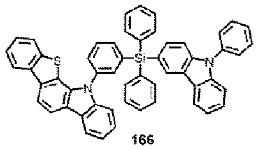
166

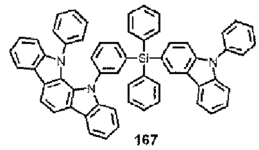
167

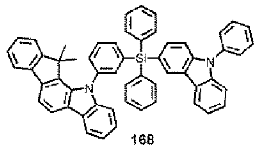
168

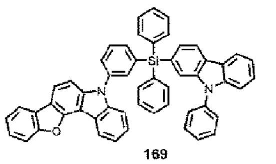
169

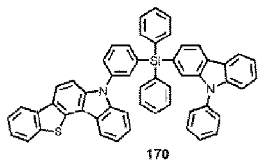
170

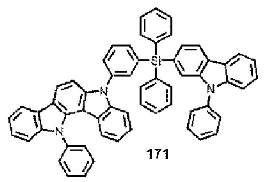
171

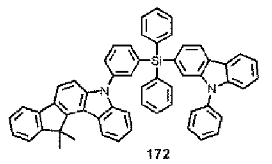
172

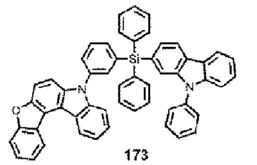
173

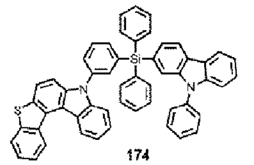
174

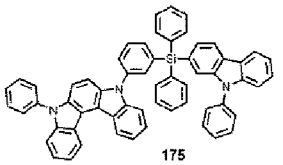
175

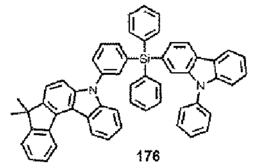
176

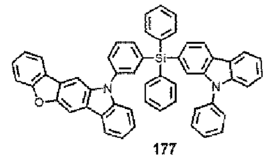
177
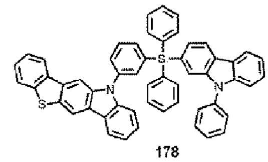
178
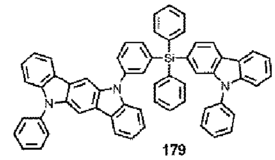
179
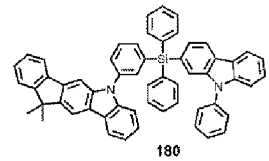
180

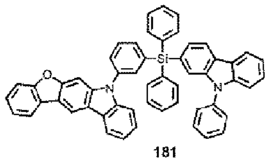
181
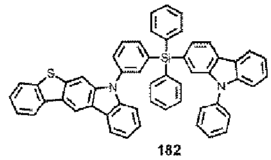
182
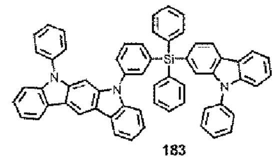
183
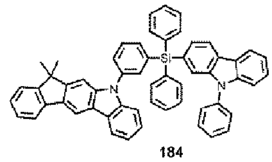
184

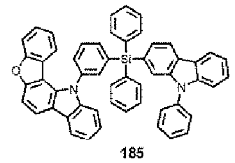
185
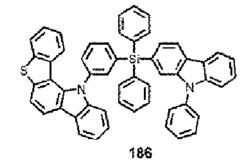
186
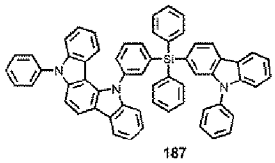
187
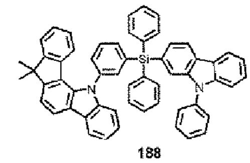
188

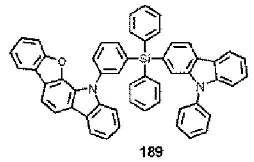
189
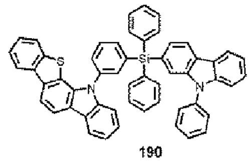
190
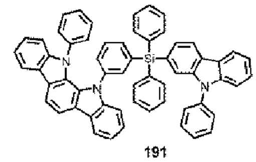
191
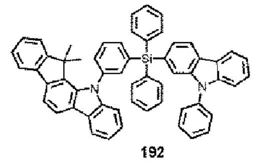
192

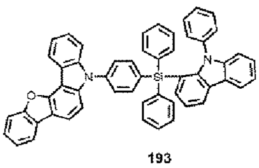
193
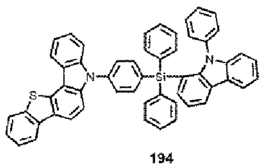
194
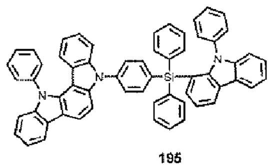
195
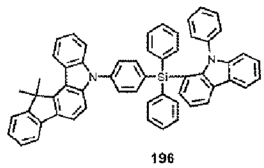
196

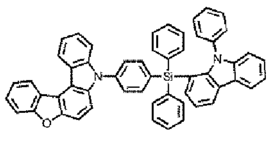
197
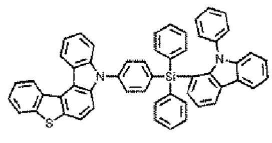
198
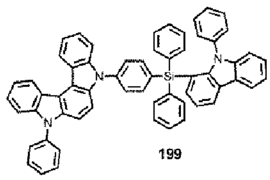
199
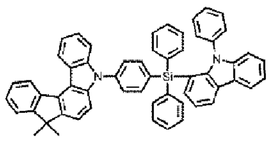
200

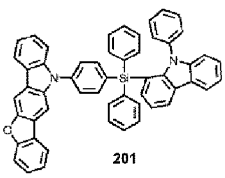
201
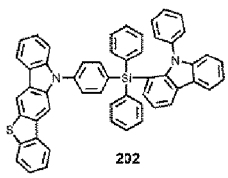
202
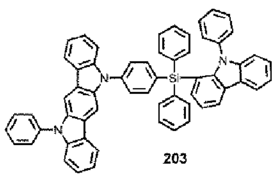
203
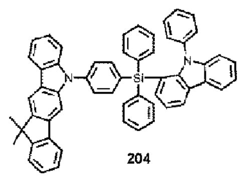
204

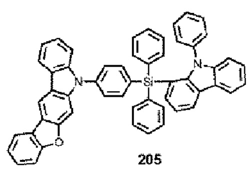
205
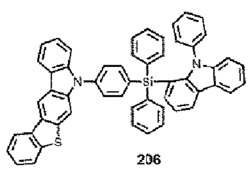
206
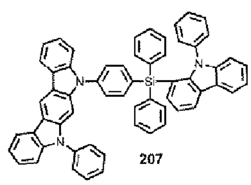
207
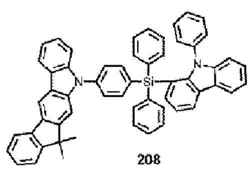
208

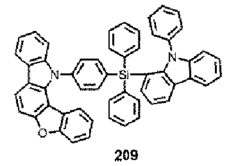

209

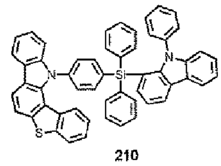

210

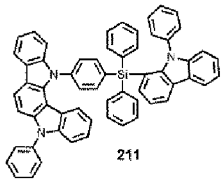

211

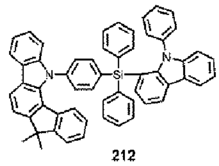

212

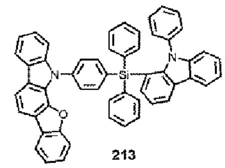

213

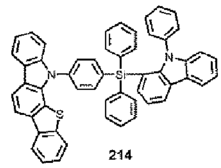

214

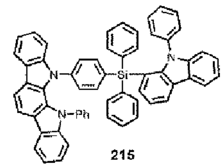

215

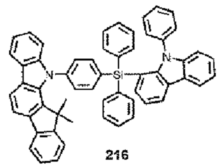

216

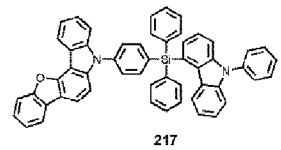

217

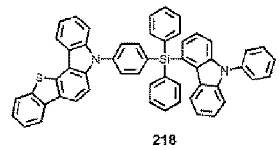

218

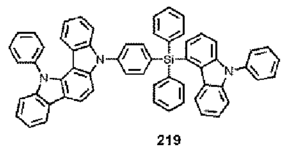

219

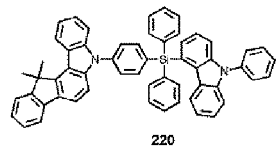

220

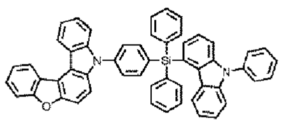

221

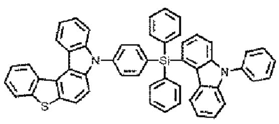

222

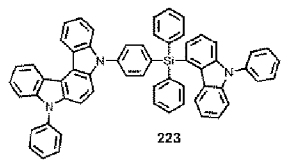

223

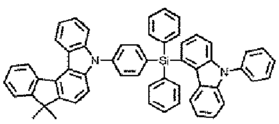

224

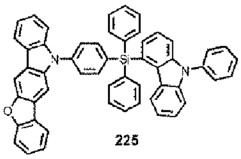

225

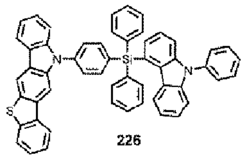

226

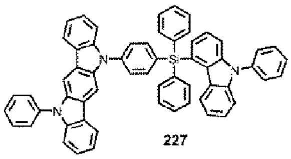

227

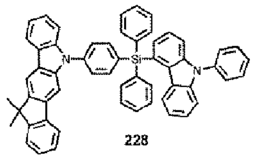

228

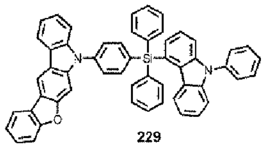

229

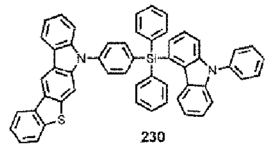

230

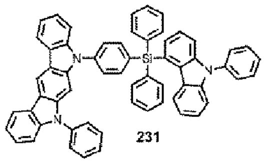

231

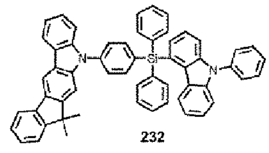

232

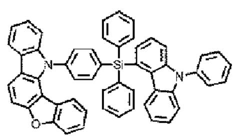

233

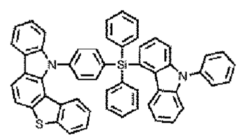

234

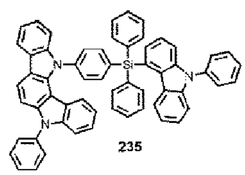

235

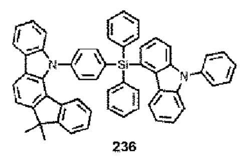

236

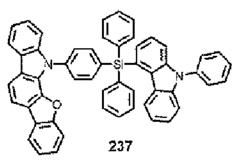

237

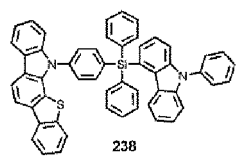

238

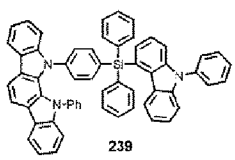

239

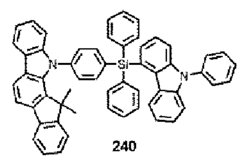

240

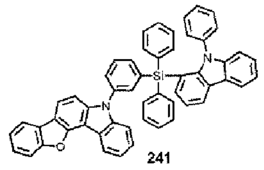
241

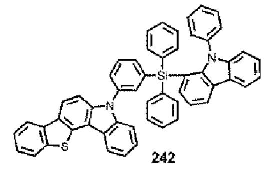
242

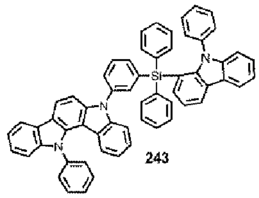
243

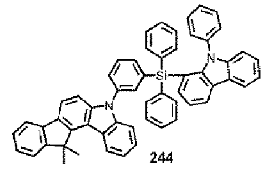
244

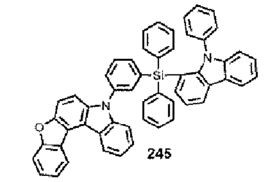
245

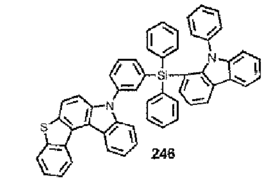
246

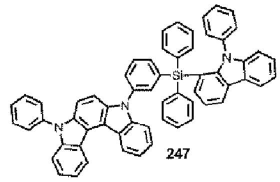
247

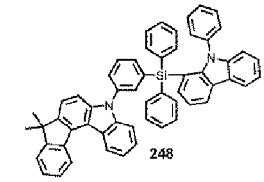
248

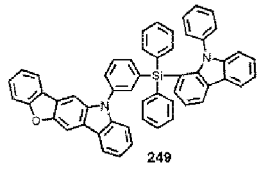
249

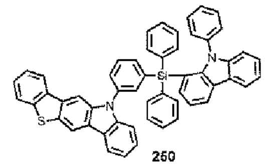
250

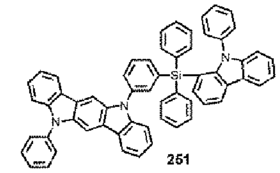
251

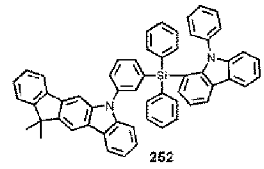
252

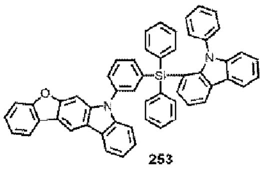
253

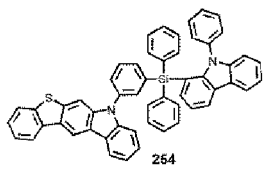
254

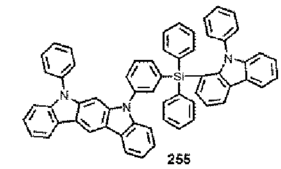
255

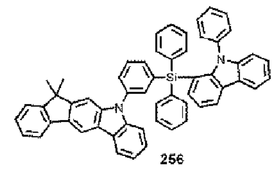
256

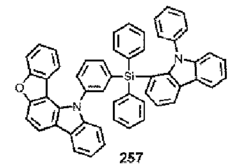
257

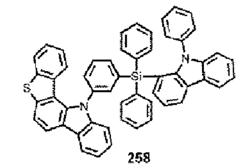
258

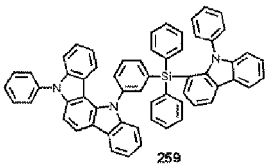
259

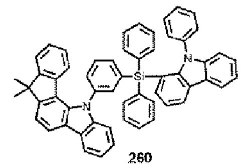
260

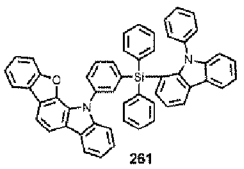
261

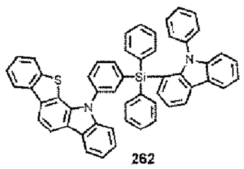
262

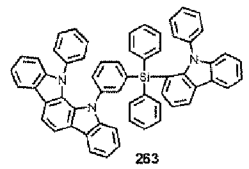
263

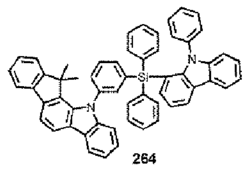
264

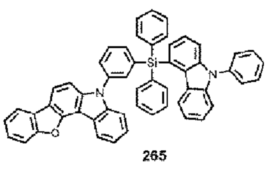
265

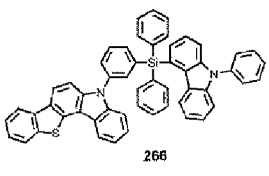
266

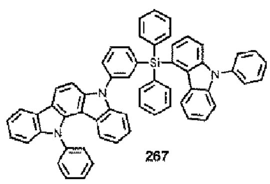
267

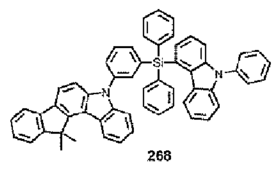
268

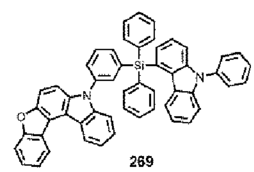
269

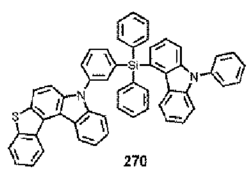
270

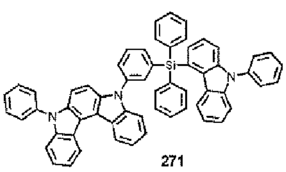
271

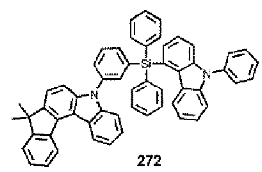
272

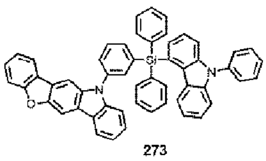
273

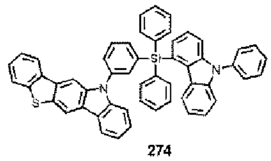
274

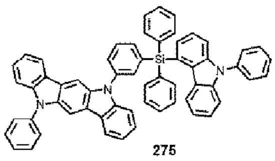
275

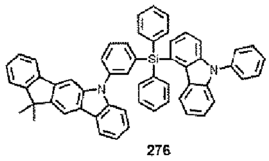
276

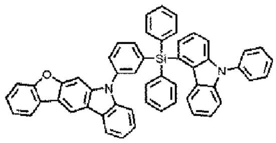
277

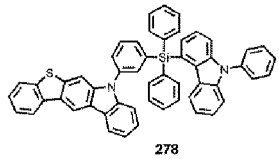
278

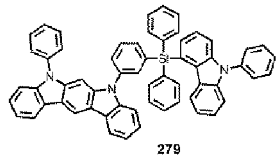
279

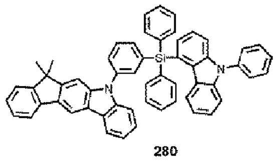
280

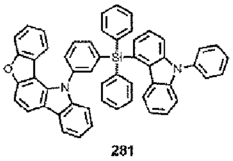
281

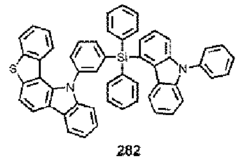
282

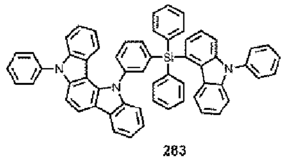
283

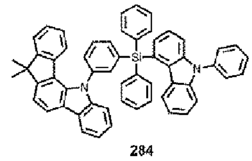
284

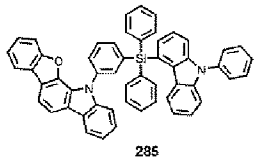
285

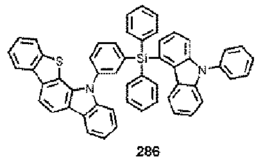
286

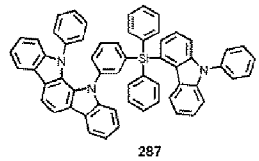
287

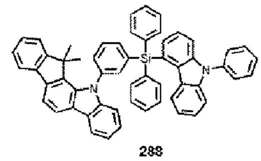
288

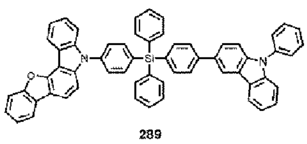
289

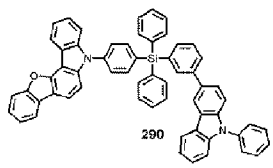
290

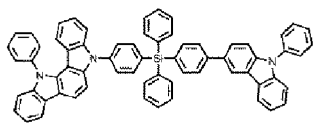
291

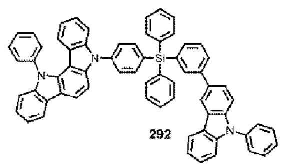
292

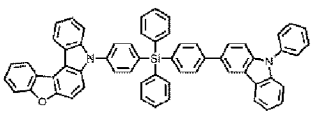
293

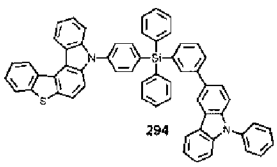
294

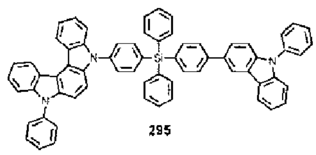
295

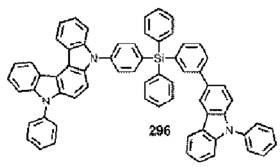
296

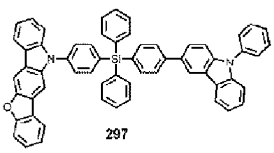
297

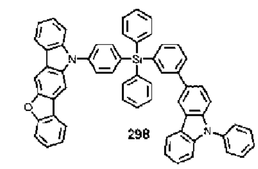
298

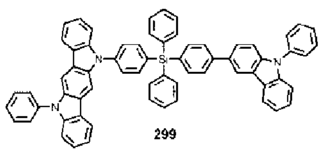
299

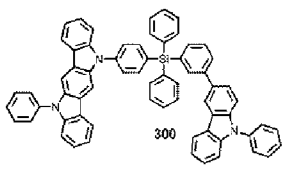
300

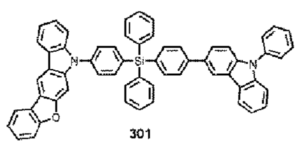
301

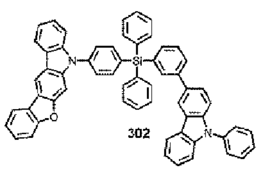
302

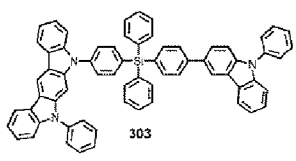
303

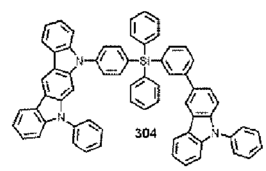
304

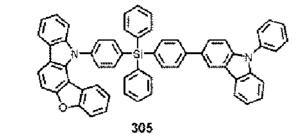
305

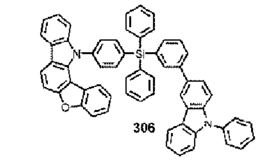
306

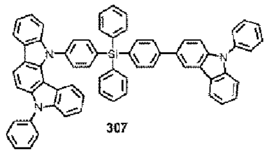
307

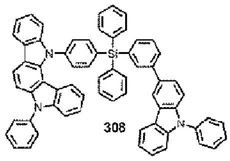
308

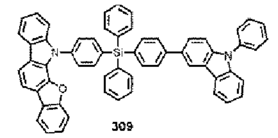
309

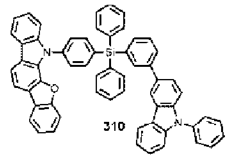
310

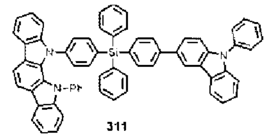
311

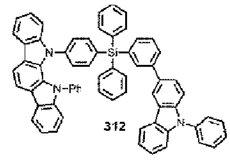
312

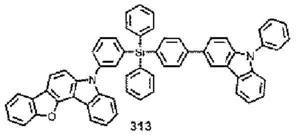
313

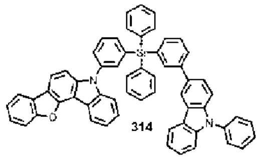
314

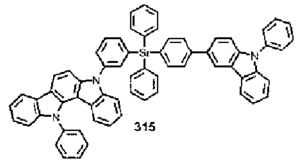
315

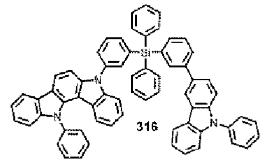
316

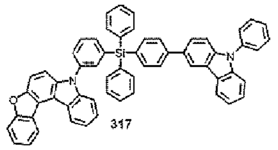
317

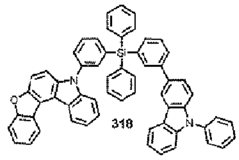
318

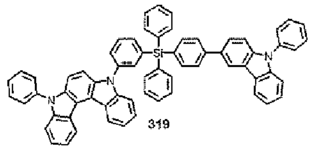
319

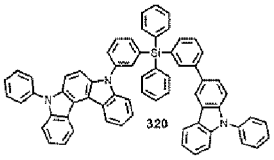
320

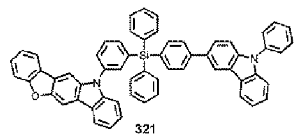
321

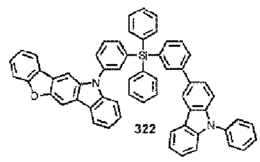
322

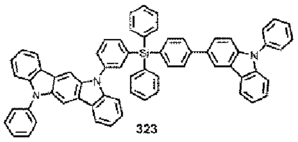
323

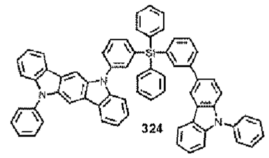
324

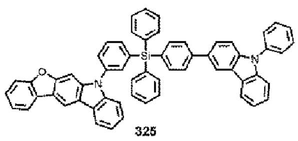
325

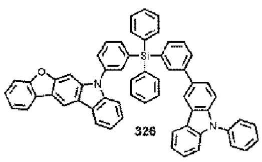
326

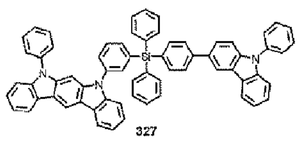
327

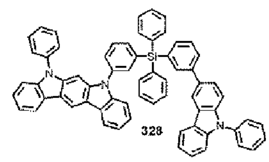
328

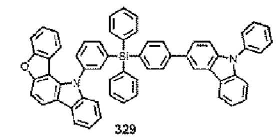
329

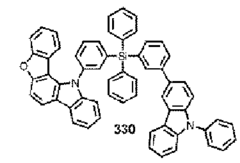
330

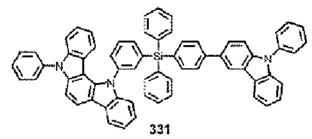
331

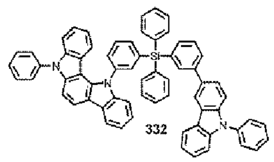
332

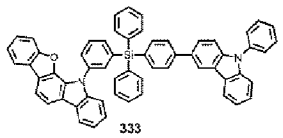
333

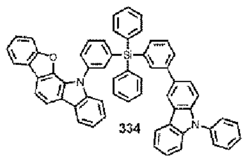
334

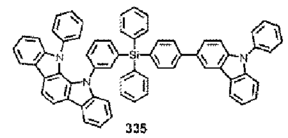
335

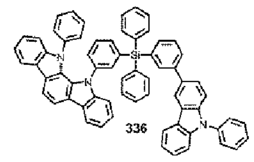
336

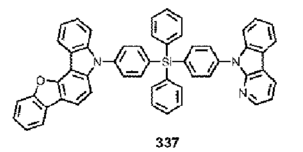
337
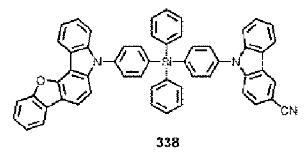
338
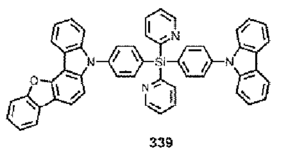
339
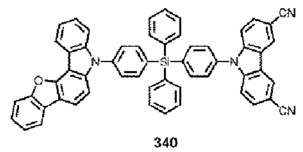
340

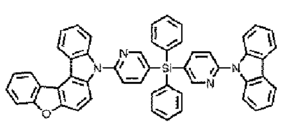
341
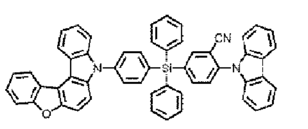
342
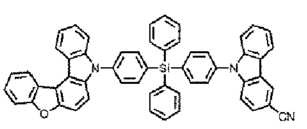
343
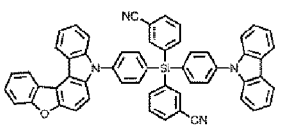
344

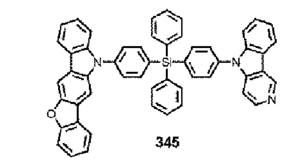
345
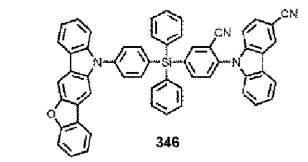
346
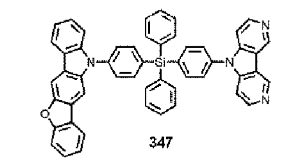
347
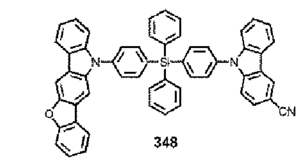
348

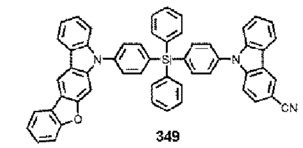
349
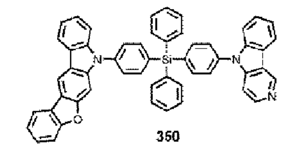
350
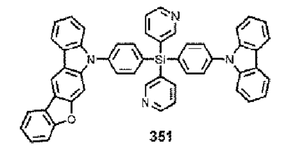
351
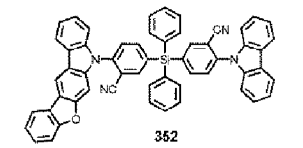
352

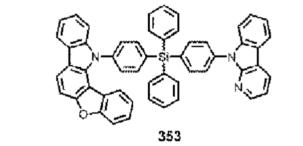
353
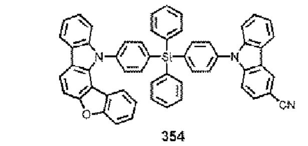
354
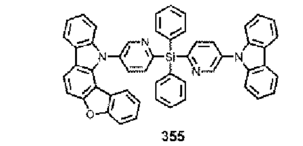
355
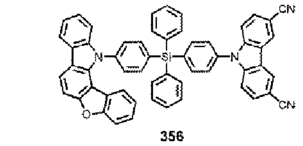
356

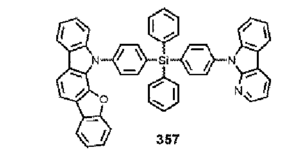
357
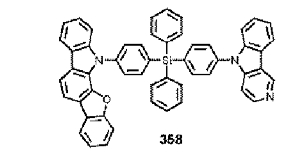
358
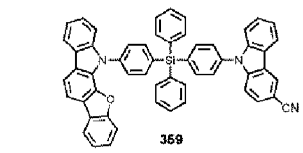
359
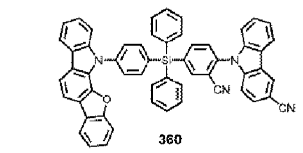
360

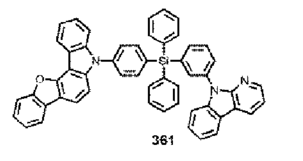
361
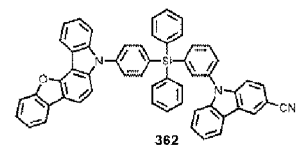
362
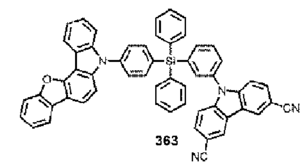
363
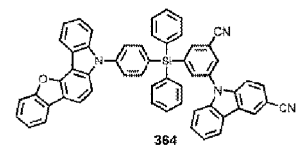
364

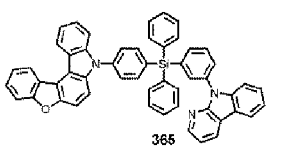
365
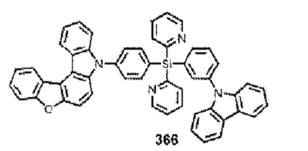
366
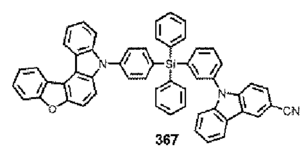
367
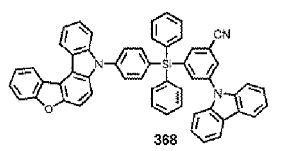
368

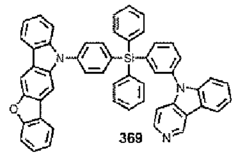
369

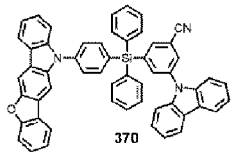
370

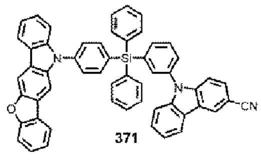
371

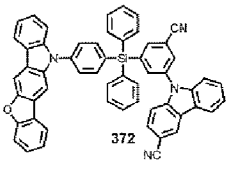
372

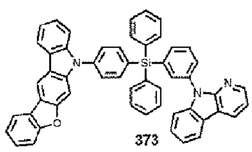
373

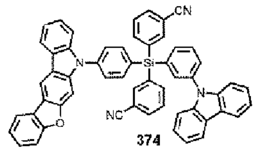
374

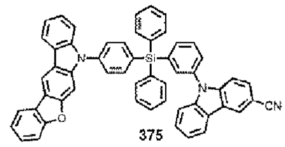
375

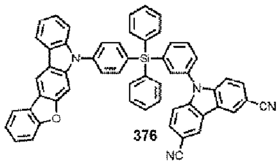
376

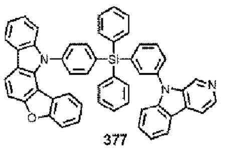
377

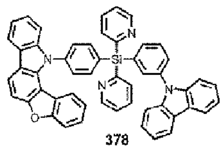
378

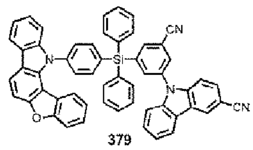
379

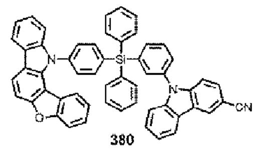
380

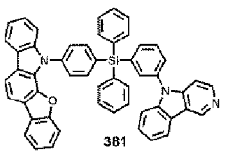
381

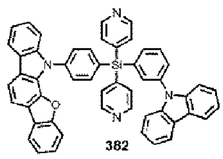
382

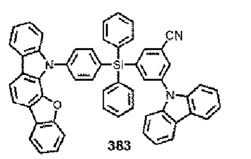
383

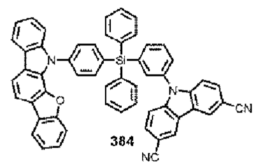
384

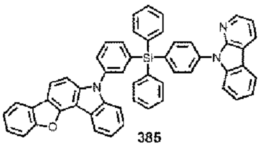
385

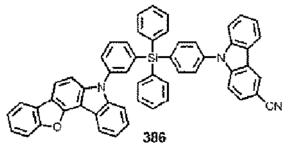
386

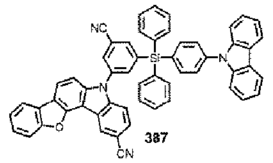
387

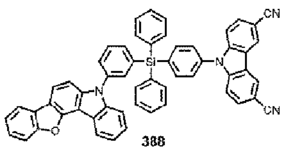
388

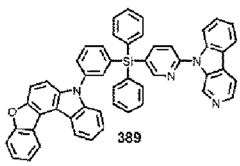
389

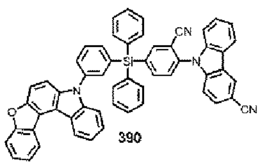
390

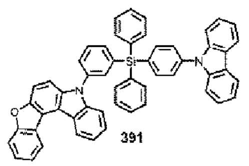
391

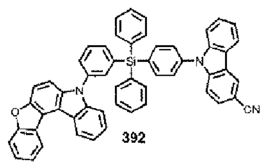
392

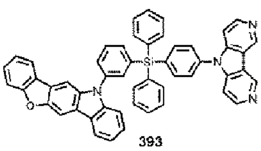
393

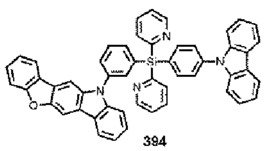
394

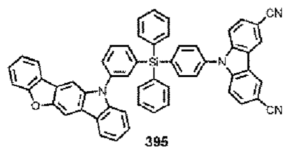
395

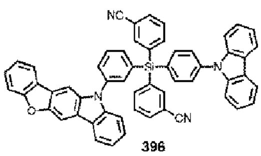
396

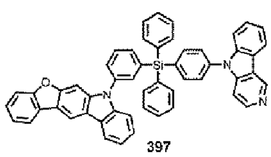
397

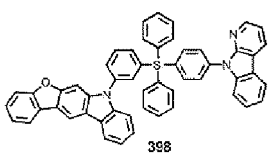
398

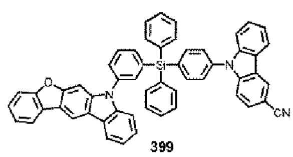
399

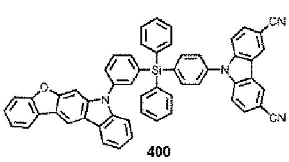
400

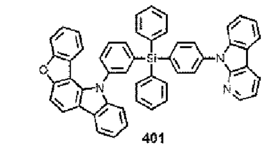
401
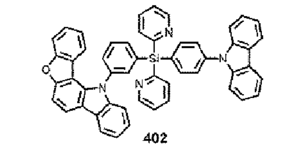
402
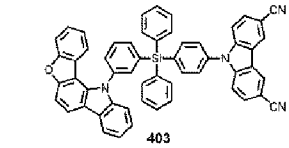
403
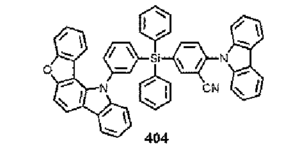
404
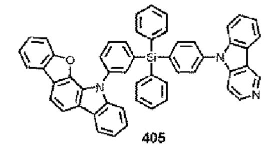
405
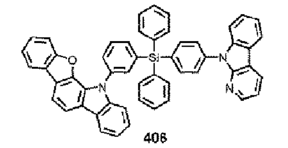
406
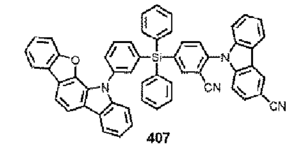
407
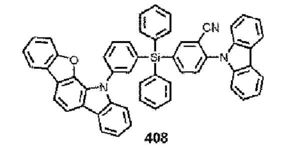
408
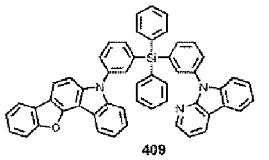
409
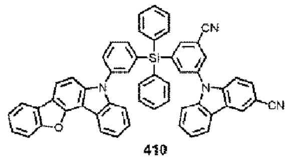
410
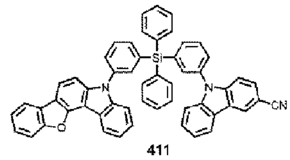
411
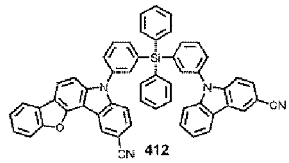
412
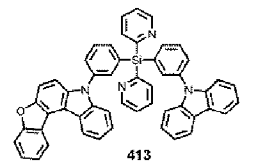
413
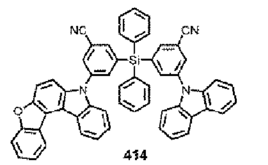
414
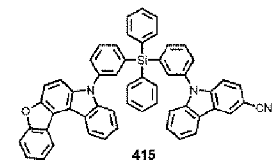
415
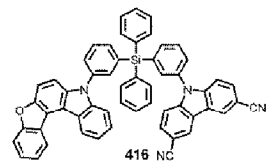
416
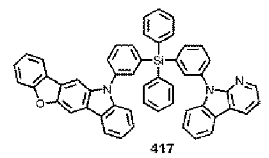
417
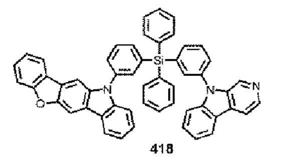
418
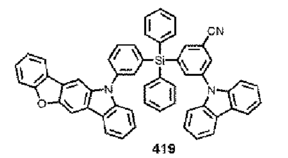
419
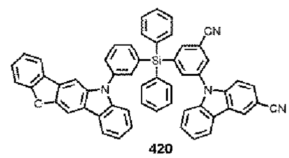
420
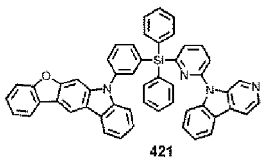
421
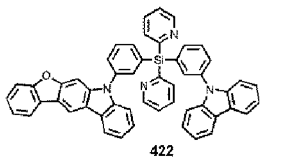
422
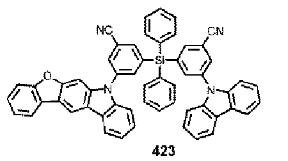
423
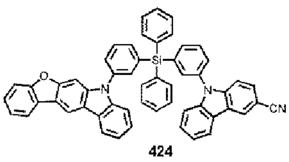
424
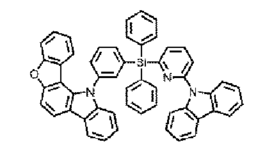
425
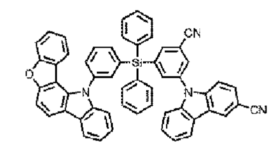
426
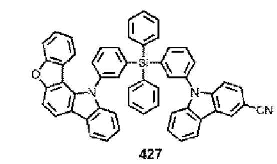
427
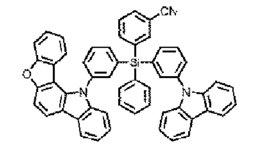
428
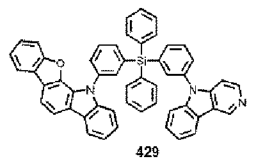
429
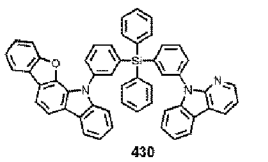
430
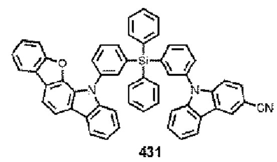
431
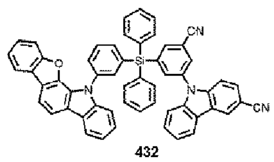
432

wherein, in Compounds 1 to 432,

Ph may be a phenyl group.

When the host is a mixture of an electron-transporting host and a hole-transporting host, the weight ratio of the electron-transporting host and the hole-transporting host may be about 1:9 to about 9:1, for example, about 2:8 to about 8:2, for example, about 4:6 to about 6:4, for example, about 5:5. When the weight ratio of the electron-transporting host and the hole-transporting host satisfies the above-described ranges, the hole-and-electron-transporting balance in the emission layer 15 may be made.

Dopant in emission layer 15

The dopant may include the second compound.

Since the dopant emits fluorescent light, organic light-emitting devices according to one or more embodiments of the present disclosure are clearly distinguished from organic light-emitting devices containing compounds that emit phosphorescent light.

In one or more embodiments, the dopant may be free of metal atoms.

In one or more embodiments, the dopant may further include, in addition to the second compound, a compound that is a condensed polycyclic compound or a styryl compound.

For example, the dopant may include one of a naphthalene-containing core, a fluorene-containing core, a spiro-bifluorene-containing core, a benzofluorene-containing core, a dibenzofluorene-containing core, a phenanthrene-containing core, an anthracene-containing core, a fluoranthene-containing core, a triphenylene-containing core, a pyrene-containing core, a chrysene-containing core, a naphthacene-containing core, a picene-containing core, a perylene-containing core, a pentaphene-containing core, an indenoanthracene-containing core, a tetracene-containing core, a bisanthracene-containing core, or core represented by one of Formulae 501-1 to 501-18, but embodiments of the present disclosure are not limited thereto:

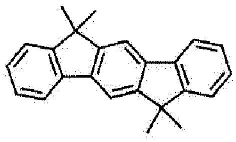

501-1

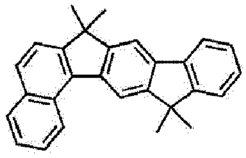

501-2

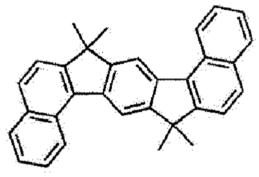

501-3

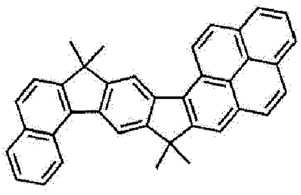

501-4

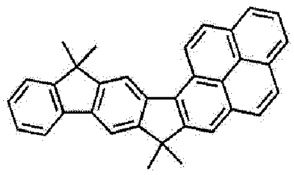

501-5

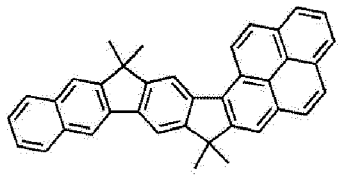

501-6

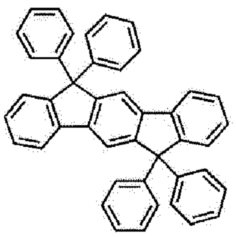

501-7

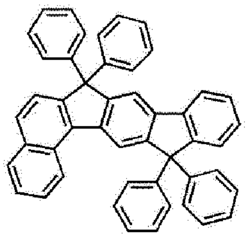

501-8

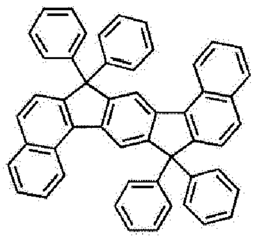

501-9

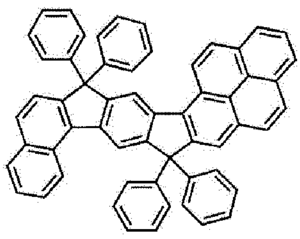

501-10

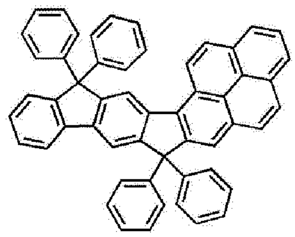

501-11

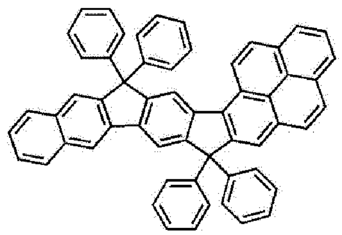

501-12

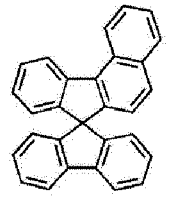

501-13

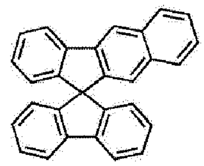

501-14

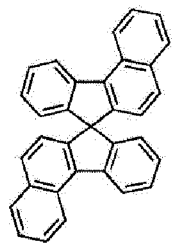

501-15

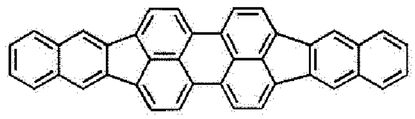

501-16

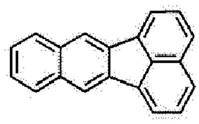

501-17

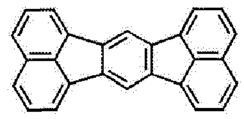

501-18

In one or more embodiments, the dopant may be a styryl-amine-containing compound or a styryl-carbazole-containing compound, but embodiments of the present disclosure are not limited thereto.

In one or more embodiments, the dopant may be a compound represented by Formula 501:

Formula 501

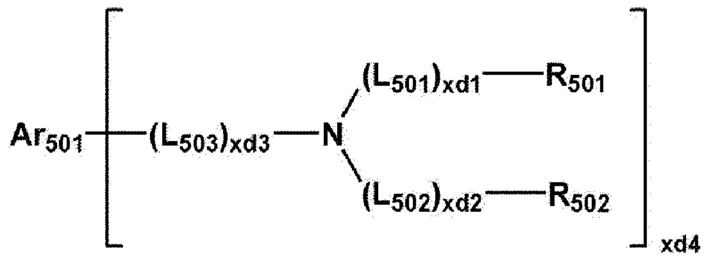

wherein, in Formula 501,

$Ar_{501}$ may be:

a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene group, an indenoanthracene group, a tetracene group, a bisanthracene group, or a groups represented by one of Formulae 501-1 to 501-18; or

a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene indenoanthracene group, a tetracene group, a bisanthracene group, or a group represented by one of Formulae 501-1 to 501-18, each substituted with at least one of deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_1$-$C_{60}$ alkylthio group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alky aryl group, a $C_7$-$C_{60}$ aryl alkyl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a $C_2$-$C_{60}$ heteroaryl alkyl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, or -Si($Q_{501}$)($Q_{502}$)($Q_{503}$) (wherein $Q_{501}$ to $Q_{503}$ may each independently be hydrogen, a $C_1$-$C_{60}$ alkyl group, a $C_1$-$C_{60}$ alkoxy group, a $C_6$-$C_{60}$ aryl group, a $C_7$-$C_{60}$ alky aryl group, a $C_7$-$C_{60}$ aryl alkyl group, a $C_6$-$C_{60}$ aryloxy group, a $C_1$-$C_{60}$ heteroaryl group, a $C_2$-$C_{60}$ alkyl heteroaryl group, a $C_2$-$C_{60}$ heteroaryl alkyl group, a $C_1$-$C_{60}$ heteroaryloxy group, a monovalent non-aromatic condensed polycyclic group, or a monovalent non-aromatic condensed heteropolycyclic group),

$L_{501}$ to $L_{503}$ may each independently be a substituted or unsubstituted $C_3$-$C_{10}$ cycloalkylene group, a substituted or unsubstituted $C_1$-$C_{10}$ heterocycloalkylene group, a substituted or unsubstituted $C_3$-$C_{10}$ cycloalkenylene group, a substituted or unsubstituted $C_1$-$C_{10}$ heterocycloalkenylene group, a substituted or unsubstituted $C_6$-$C_{60}$ arylene group, a substituted or unsubstituted $C_1$-$C_{60}$ heteroarylene group, a substituted or unsubstituted divalent non-aromatic condensed polycyclic group, or a substituted or unsubstituted divalent non-aromatic condensed heteropolycyclic group,
$R_{501}$ and $R_{502}$ may each independently be:

a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazole group, a triazinyl group, a dibenzofuranyl group, or a dibenzothiophenyl group; or
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, or a dibenzothiophenyl group, each substituted with at least one of deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, or a dibenzothiophenyl group,
xd1 to xd3 may each independently be 0, 1, 2, or 3, and
xd4 may be 0, 1, 2, 3, 4, 5, or 6.

For example, in Formula 501,
$Ar_{501}$ may be:

a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene group, an indenoanthracene group, a tetracene group, a bisanthracene group, or a group represented by one of Formulae 501-1 to 501 - 18; or
a naphthalene group, a fluorene group, a spiro-bifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene group, an indenoanthracene group, a tetracene group, a bisanthracene group, or a group represented by one of Formula 501-1 to 501-18, each substituted with at least one of deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a biphenyl group, a terphenyl group, a naphthyl group, a fluorenyl group, a dibenzofuranyl group, a dibenzothiophenyl group, a carbazolyl group, a pyridinyl group, a pyrimidinyl group, a triazinyl group, a quinolinyl group, an isoquinolinyl group, or - $Si(Q_{501})(Q_{502})(Q_{503})$ (wherein $Q_{501}$ to $Q_{503}$ may each independently be hydrogen, a $C_1$-$C_{20}$ alkyl group, a $C_1$-$C_{20}$ alkoxy group, a phenyl group, a biphenyl group, a terphenyl group, or a naphthyl group),
$L_{501}$ to $L_{503}$ are the same as described in connection with $L_{201}$ to $L_{209}$,
xd1 to xd3 may each independently be 0, 1, or 2, and
xd4 may be 0, 1, 2, or 3, but embodiments of the present disclosure are not limited thereto.

In one or more embodiments, the dopant may include a compound represented by one of Formulae 502-1 to 502-5:

Formula 502-1

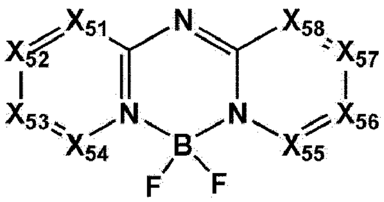

Formula 502-2

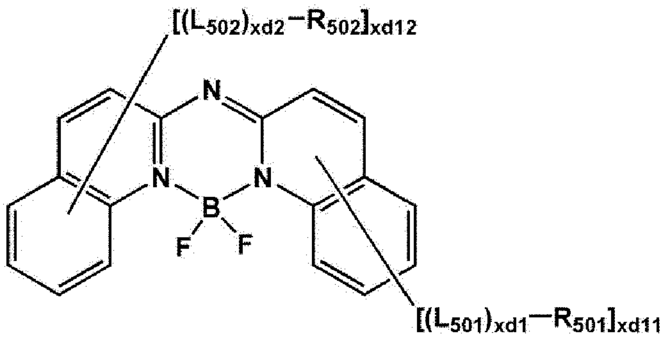

Formula 502-3

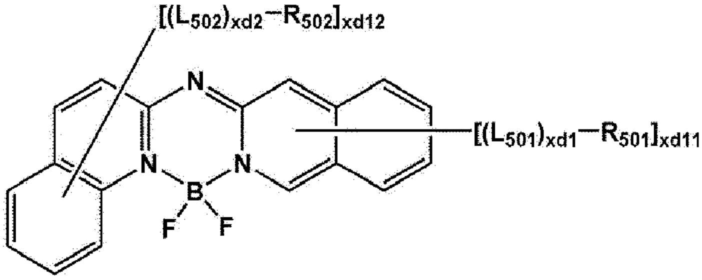

Formula 502-4

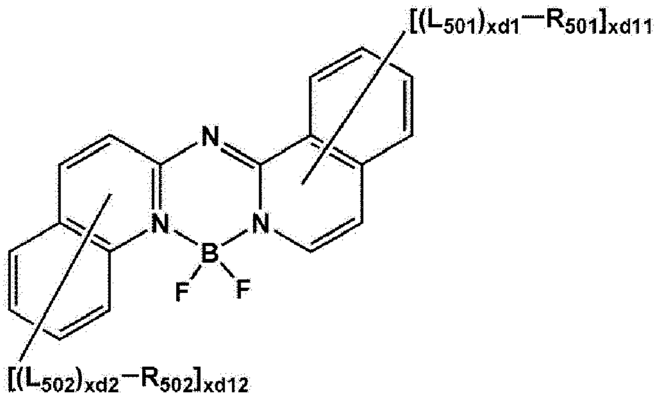

Formula 502-5

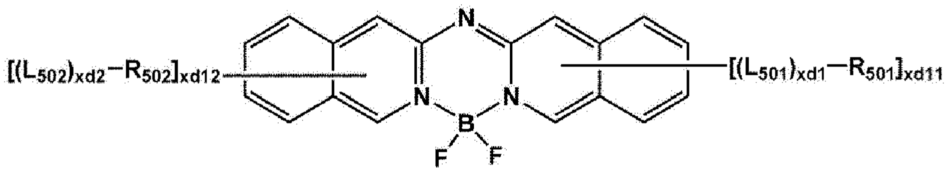

wherein, in Formulae 502-1 to 502-5,

X51 may be N or C-[$(L_{501})_{xd1}$-$R_{501}$], $X_{52}$ may be N or C-[$(L_{502})_{xd2}$-$R_{502}$], $X_{53}$ may be N or C-[$(L_{503})_{xd3}$-$R_{503}$], $X_{54}$ may be N or C-[$(L_{504})_{xd4}$-$R_{504}$], $X_{55}$ may be N or C-[$(L_{505})_{xd5}$-$R_{505}$], $X_{56}$ may be N or C-[$(L_{506})_{xd6}$-$R_{506}$], $X_{57}$ may be N or C-[$(L_{507})_{xd7}$-$R_{507}$], and $X_{58}$ may be N or C-[$(L_{508})_{xd8}$-$R_{508}$],
$L_{501}$ to $L_{508}$ are each the same as described in connection with $L_{501}$ in Formula 501,
xd1 to xd8 are each the same as described in connection with xd1 in Formula 501,
$R_{501}$ to $R_{508}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, or a $C_1$-$C_{20}$ alkoxy group,
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazole group, a triazinyl group, a dibenzofuranyl group, or a dibenzothiophenyl group; or
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, or a dibenzothiophenyl group, each substituted with at least one of deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazolyl group, a triazinyl group, a dibenzofuranyl group, or a dibenzothiophenyl group,
xd11 and xd12 may each independently be an integer from 0 to 5,
two of $R_{501}$ to $R_{504}$ may optionally be linked together to form a saturated or unsaturated ring, and
two of $R_{505}$ to $R_{508}$ may optionally be linked together to form a saturated or unsaturated ring.

The dopant may include, for example, at least one of Compounds FD(1) to FD(16) or FD1 to FD18:

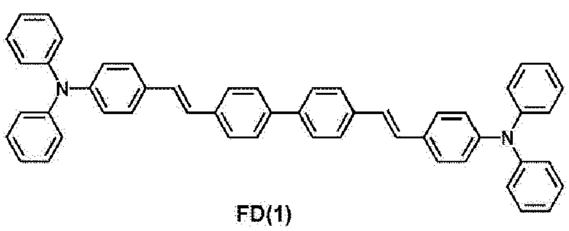

FD(1)

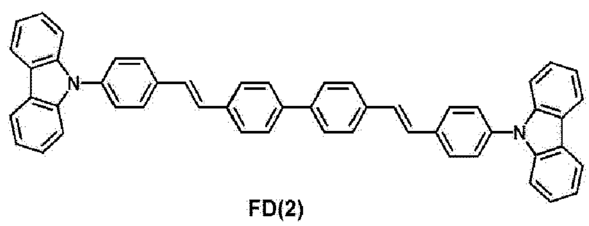

FD(2)

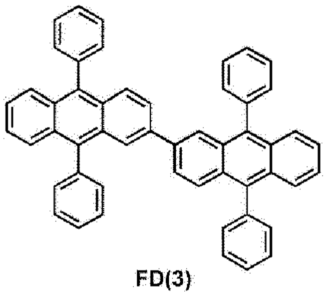

FD(3)

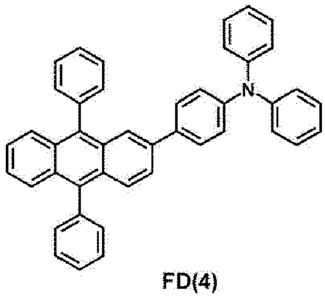

FD(4)

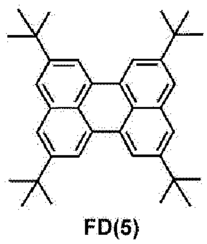

FD(5)

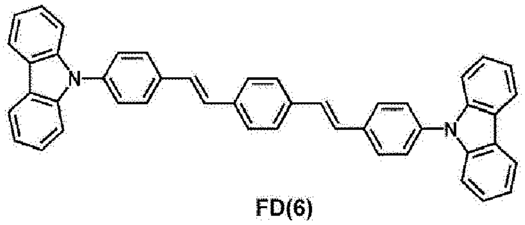

FD(6)

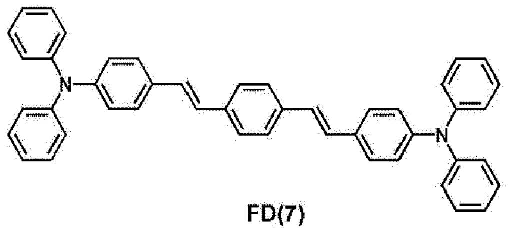

FD(7)

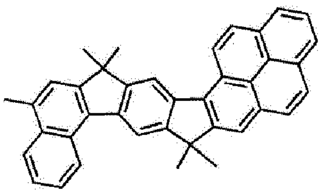

FD(8)

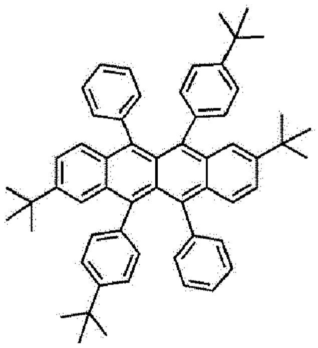

FD(9)

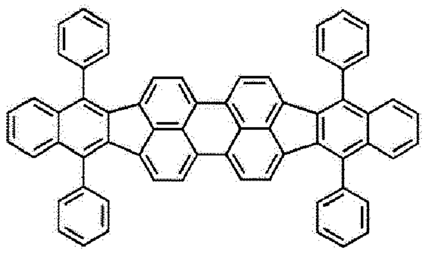

FD(10)

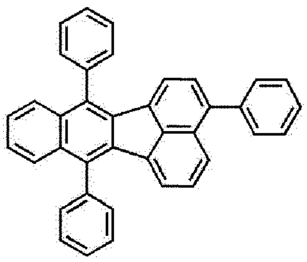

FD(11)

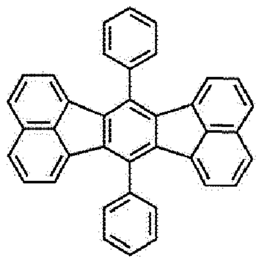

FD(12)

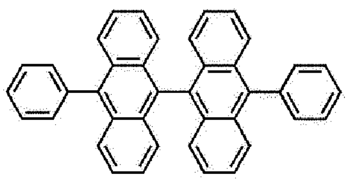

FD(13)

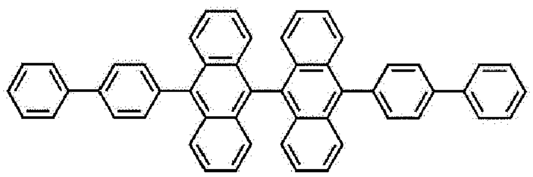

FD(14)

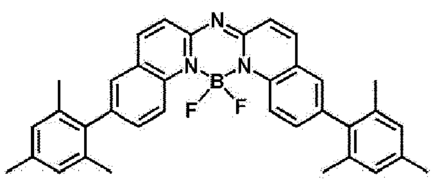

FD(15)

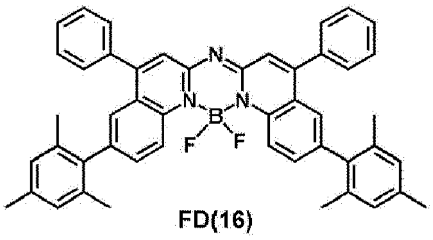

FD(16)

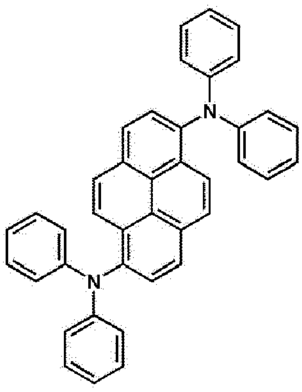

FD1

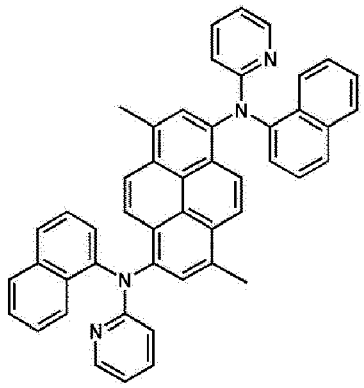

FD2

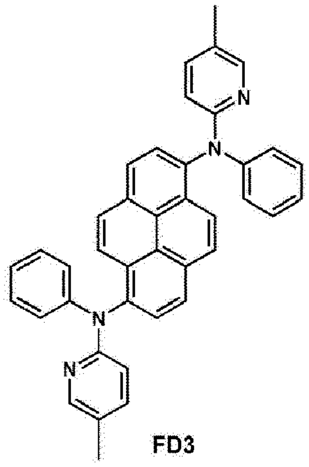

FD3

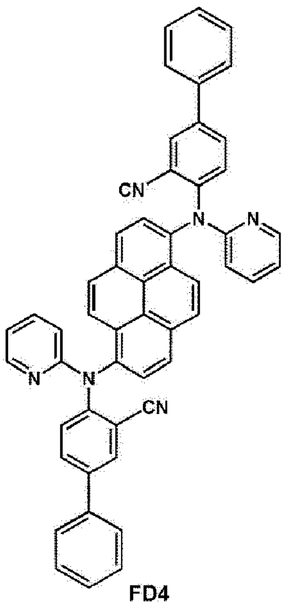

FD4

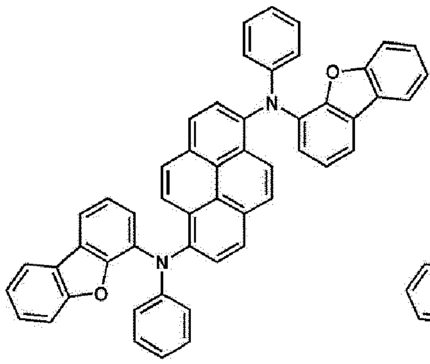

FD5

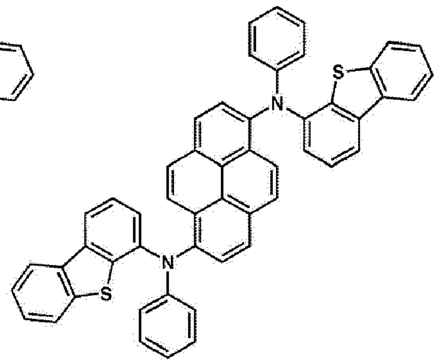

FD6

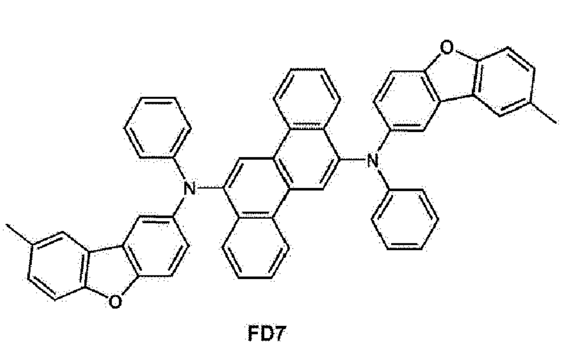

FD7

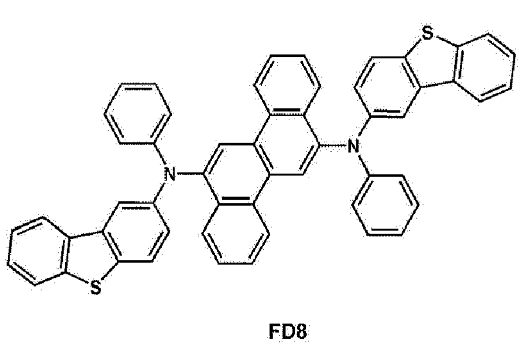

FD8

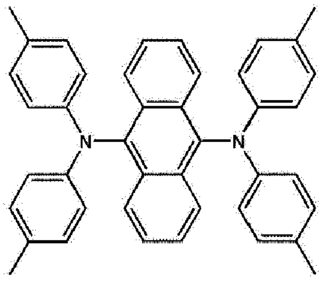

FD9

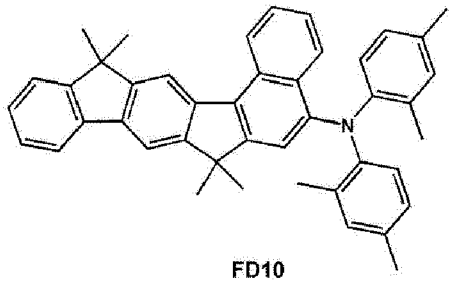

FD10

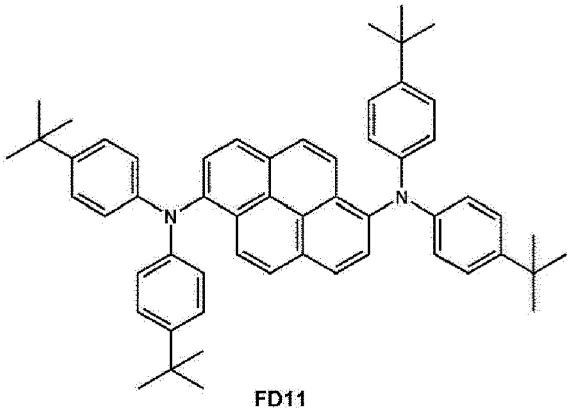

FD11

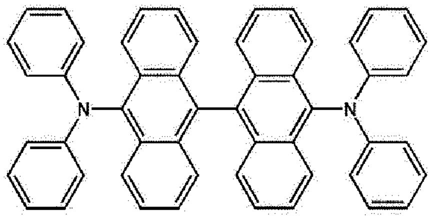

FD12

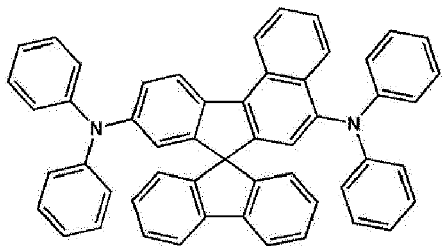

FD13

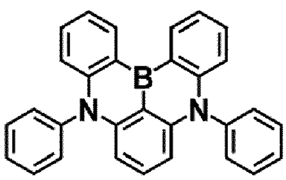

FD14

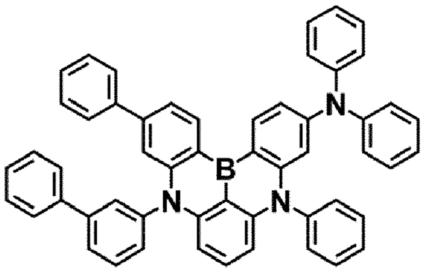

FD15

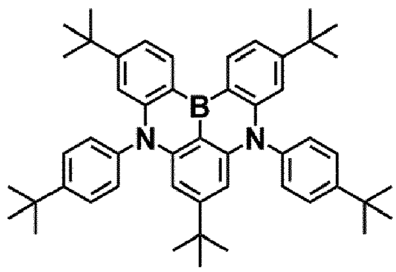

FD16

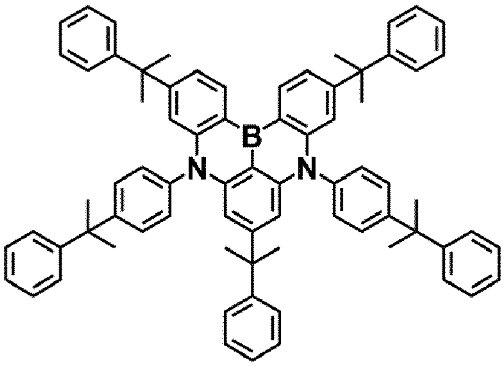

FD17

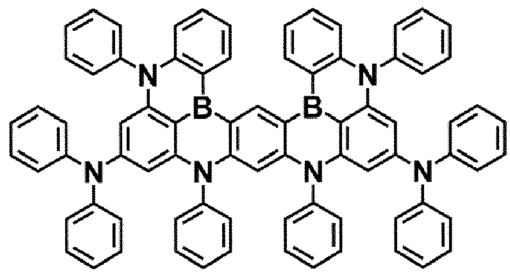

FD18

The amount of the dopant in the emission layer may be about 0.01 wt% to about 15 wt%, but embodiments of the present disclosure are not limited thereto.

Sensitizer in emission layer 15

The sensitizer may include the first compound.

In one or more embodiments, the sensitizer may be a compound from Groups I to VI below, but embodiments of the present disclosure are not limited thereto:

## Group I

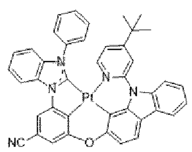
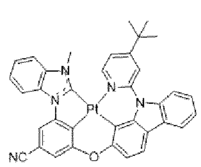
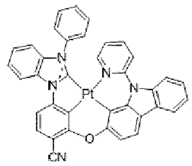
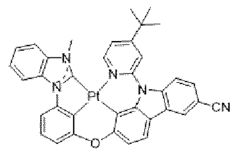
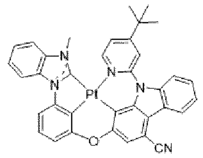

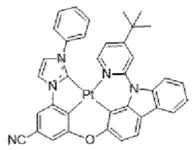
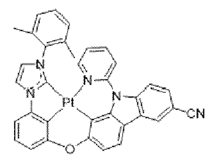

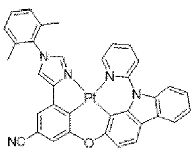
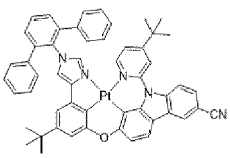
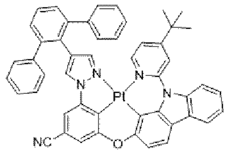
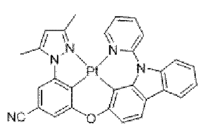

Group II

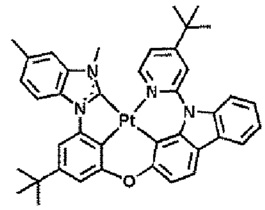

1

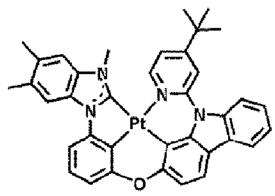

2

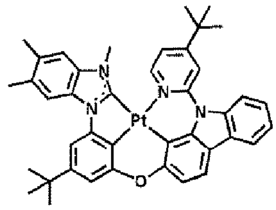

3

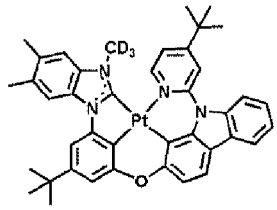

4

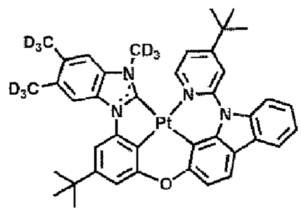

5

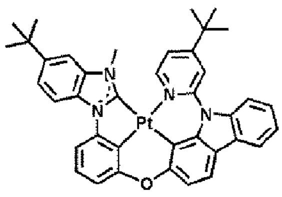

6

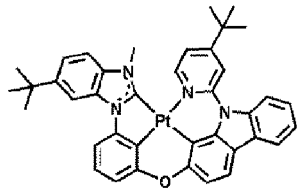

7

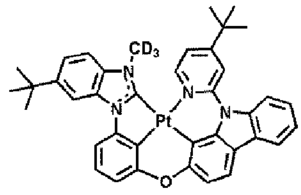

8

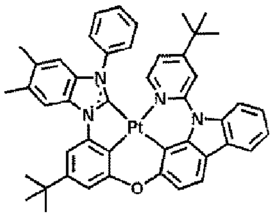

9

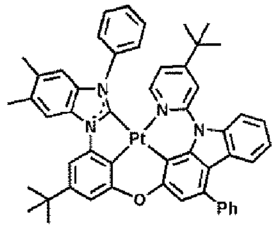

10

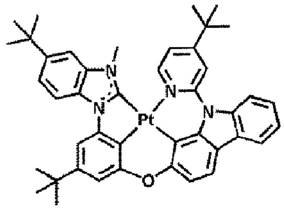

11

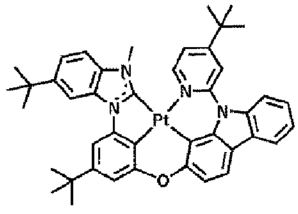

12

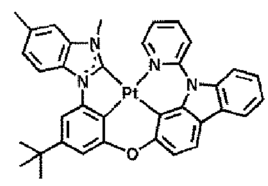

13

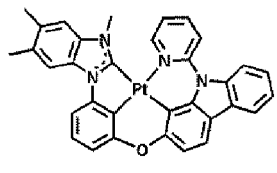

14

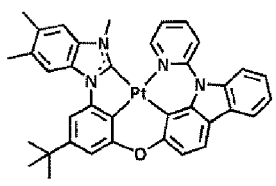

15

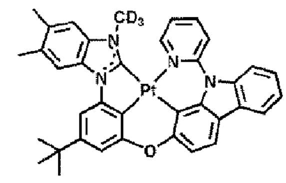

16

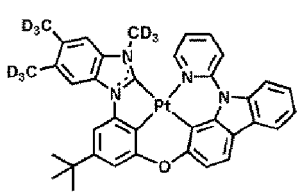

17

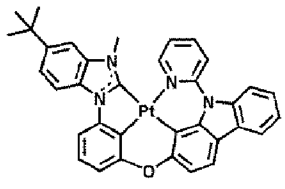

18

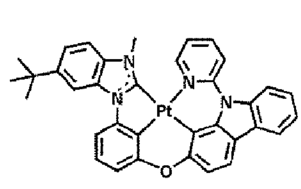

19

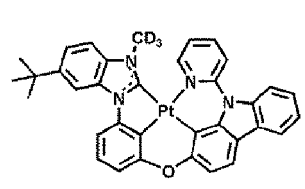

20

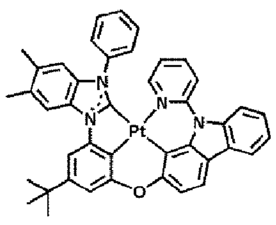

21

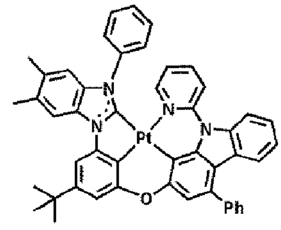

22

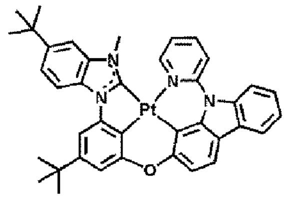

23

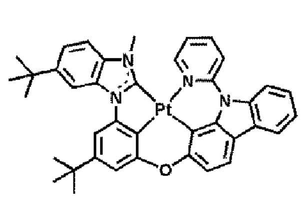

24

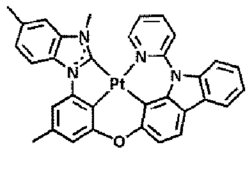

25

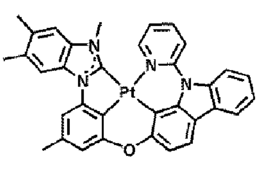

26

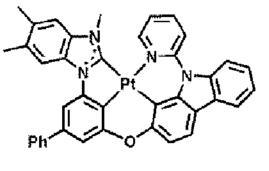

27

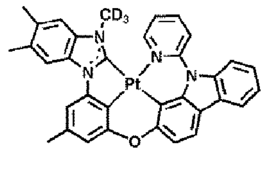

28

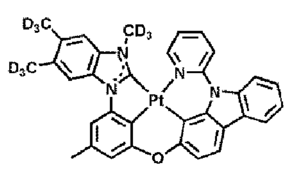

29

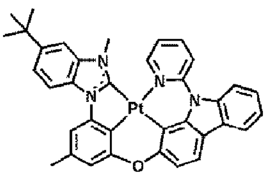

30

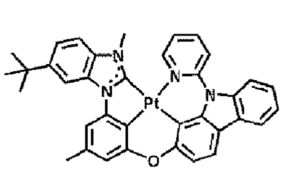

31

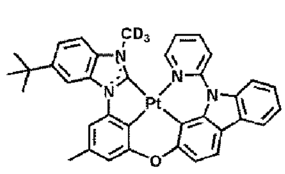

32

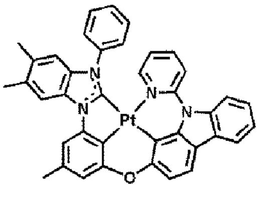

33

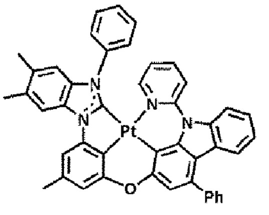

34

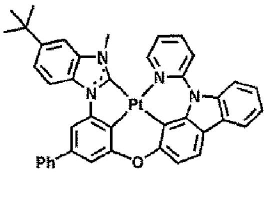

35

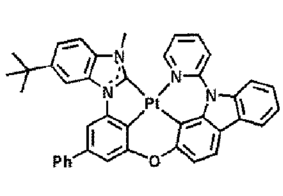

36

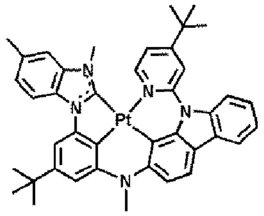

37

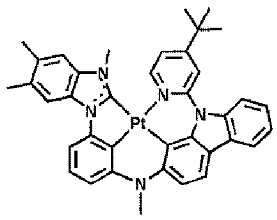

38

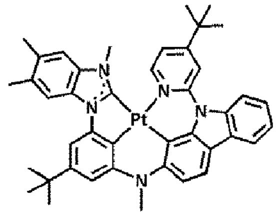

39

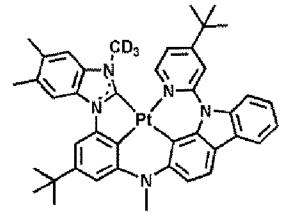

40

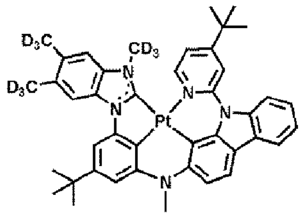

41

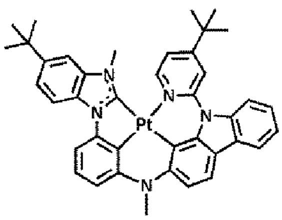

42

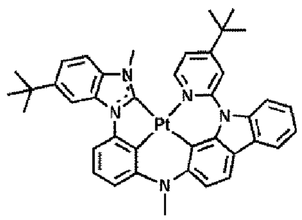

43

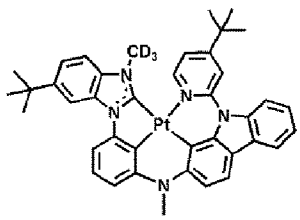

44

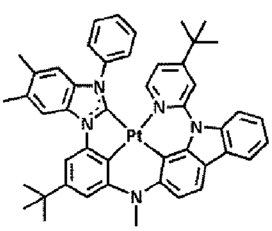

45

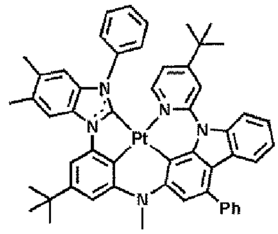

46

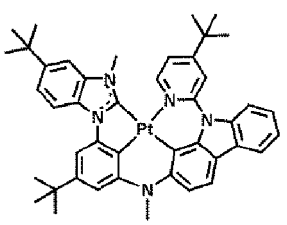

47

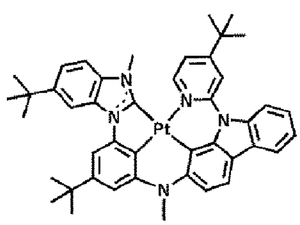

48

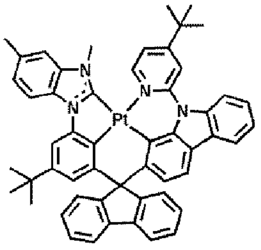
49

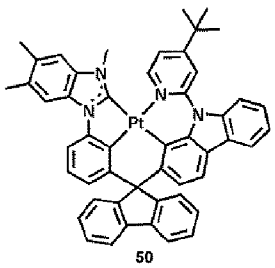
50

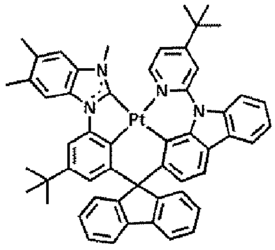
51

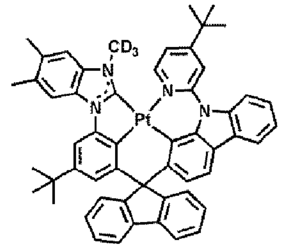
52

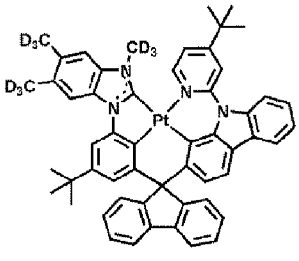
53

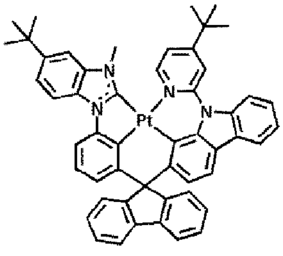
54

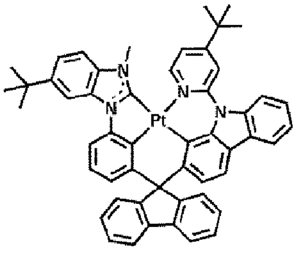
55

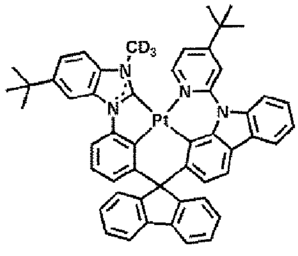
56

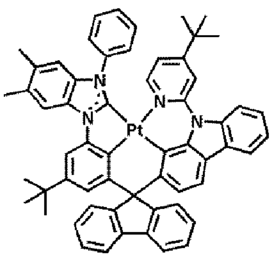
57

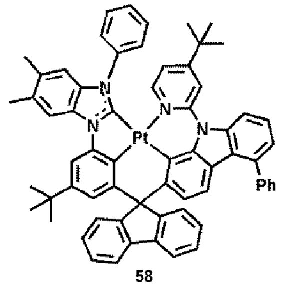
58

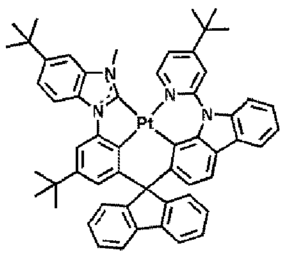
59

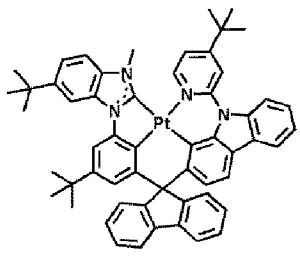
60

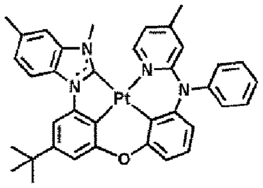
61

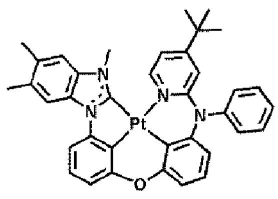
62

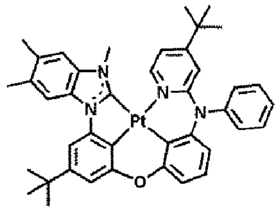
63

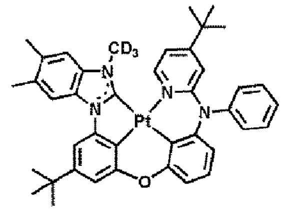
64

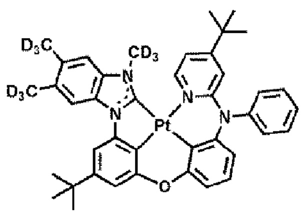
65

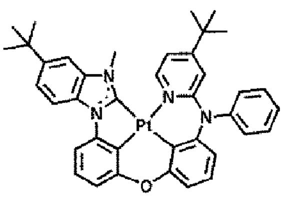
66

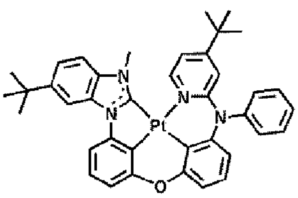
67

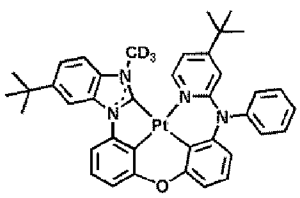
68

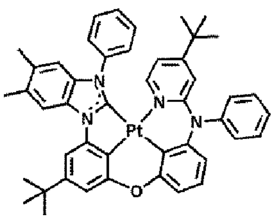
69

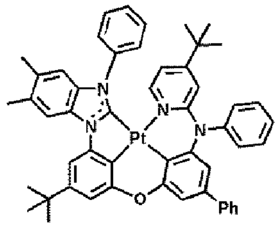
70

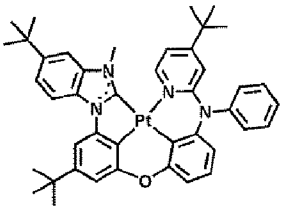
71

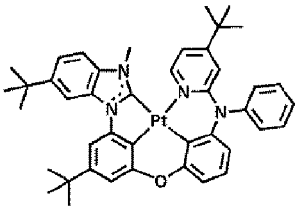
72

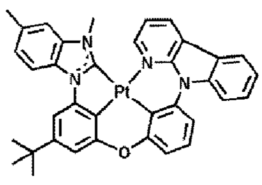
73

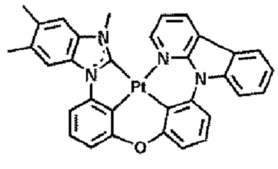
74

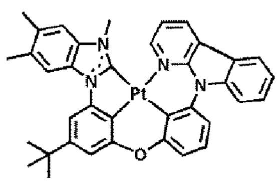
75

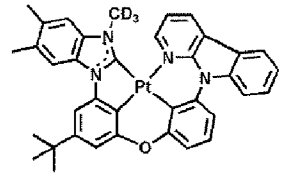
76

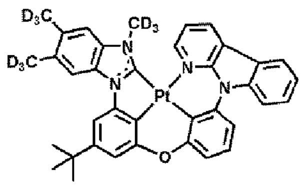
77

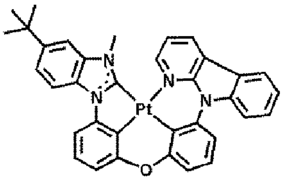
78

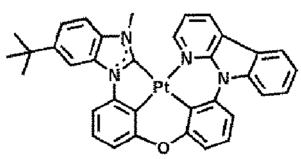
79

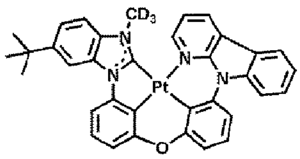
89

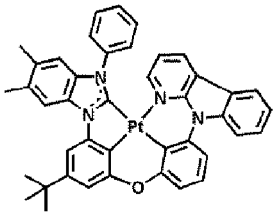
81

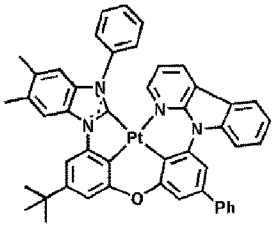
82

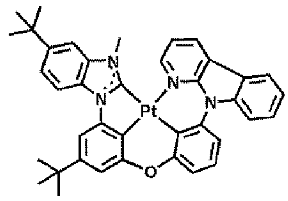
83

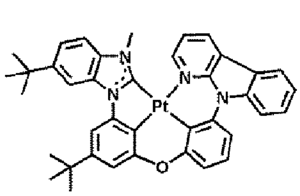
84

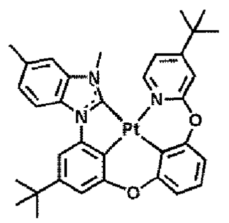
85

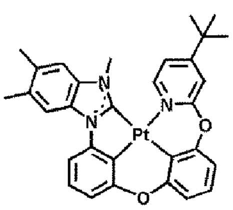
86

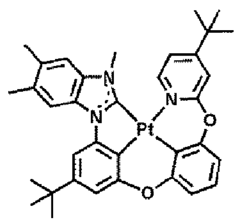
87

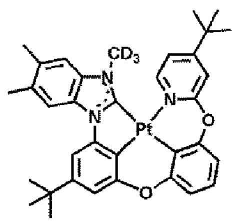
88

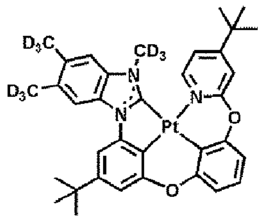
89

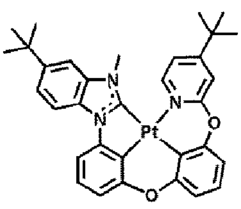
90

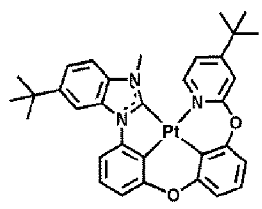
91

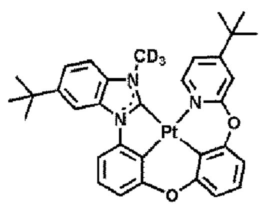
92

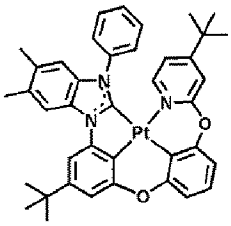
93

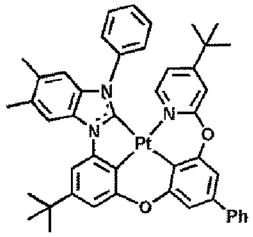
94

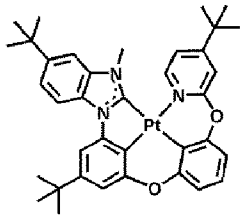
95

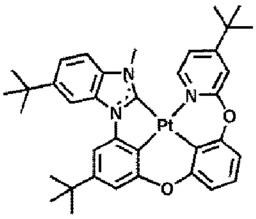
96

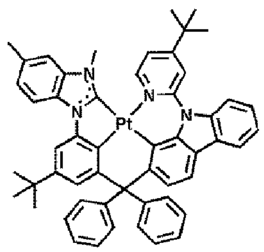
97

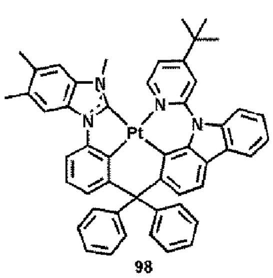
98

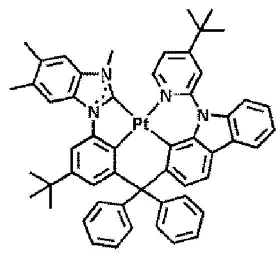
99

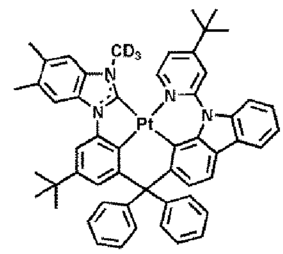
100

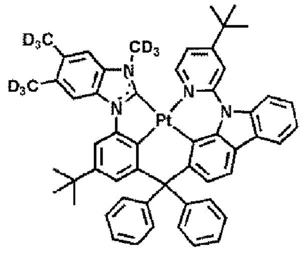
101

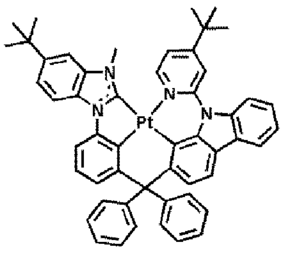
102

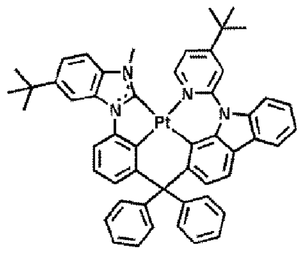
103

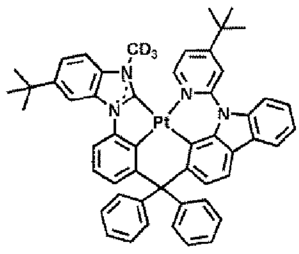
104

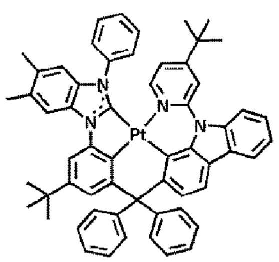
105

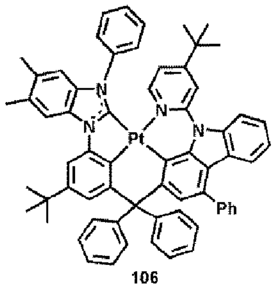
106

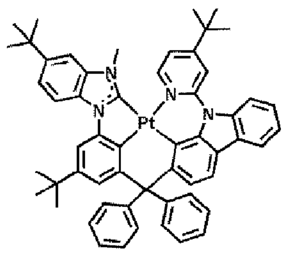
107

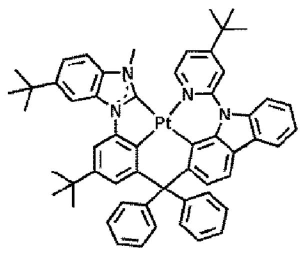
108

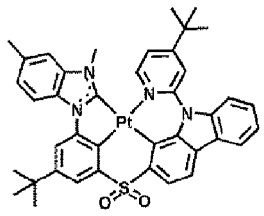
109

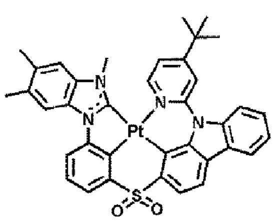
110

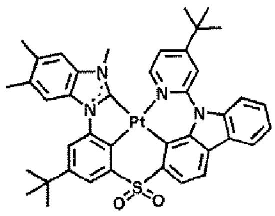
111

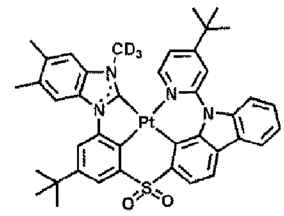
112

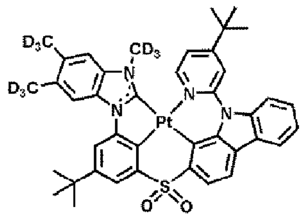
113

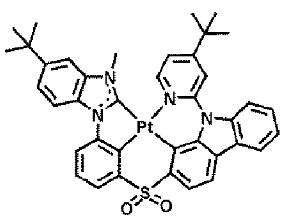
114

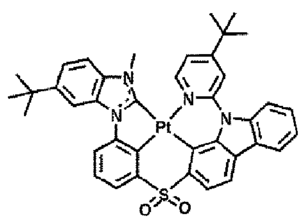
115

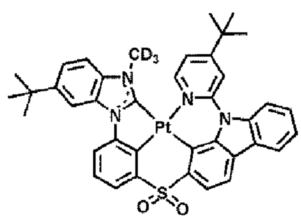
116

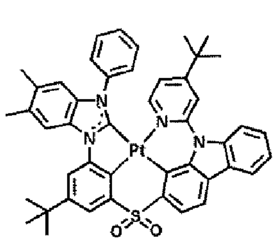
117

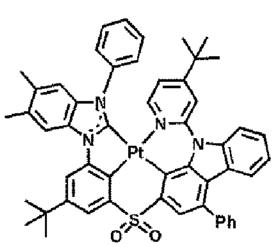
118

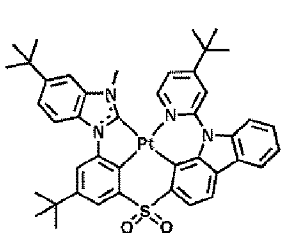
119

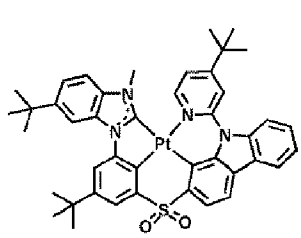
120

Group III

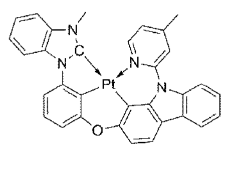
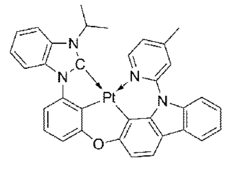
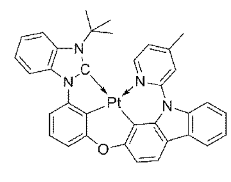
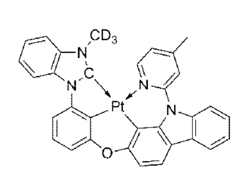
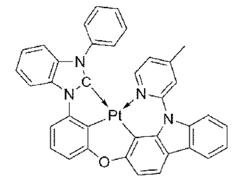
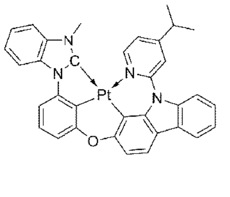
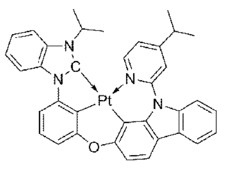
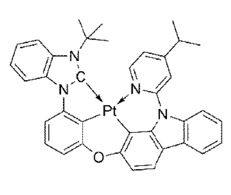
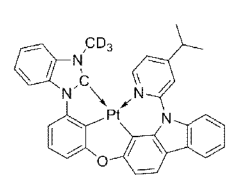
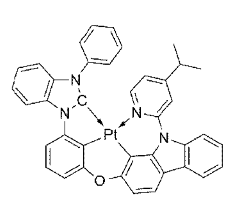
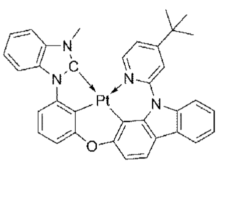
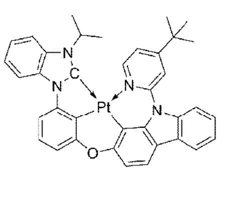
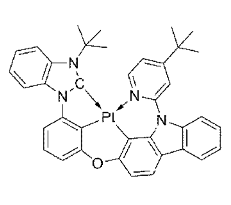
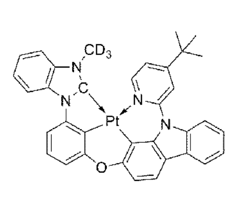
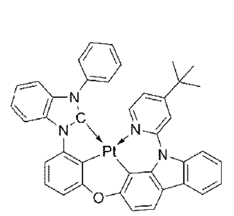
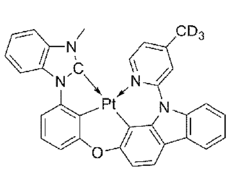
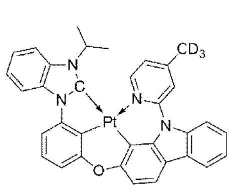
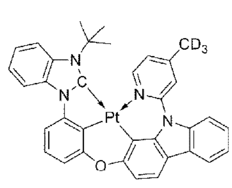
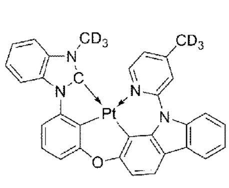
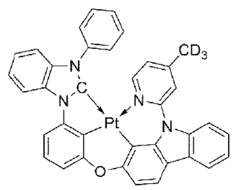
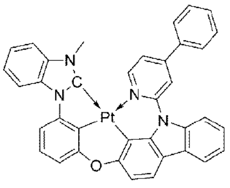
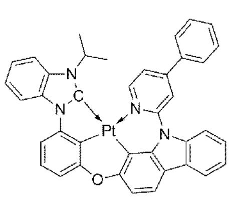
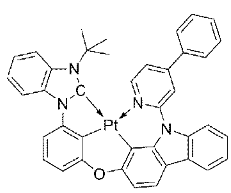
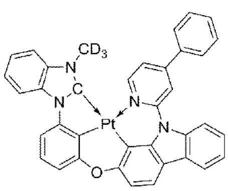
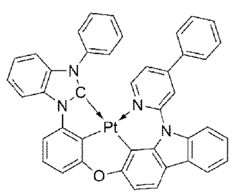
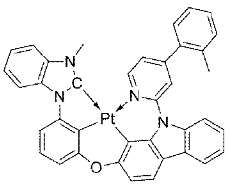
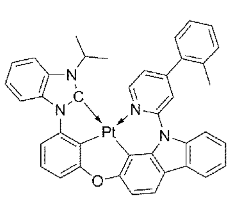
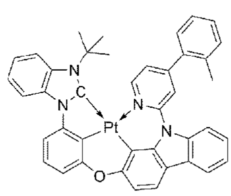
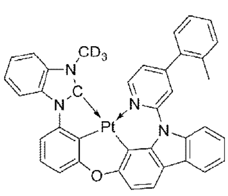
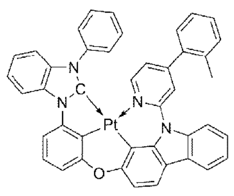

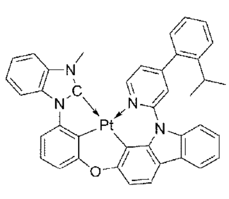 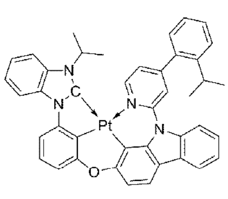 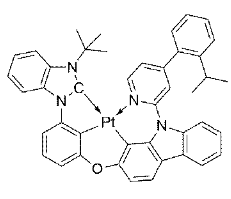 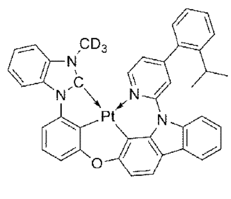 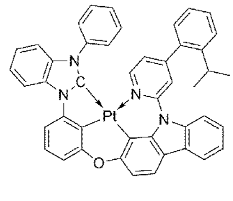

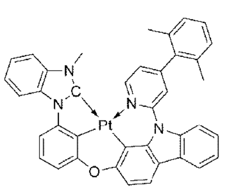 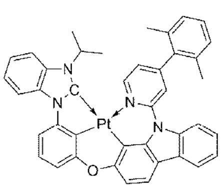 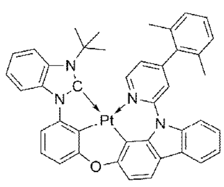 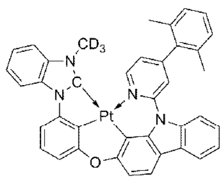 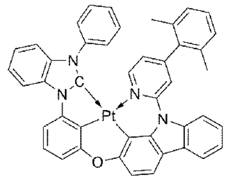

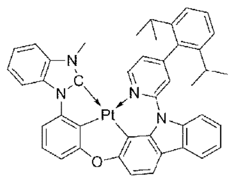 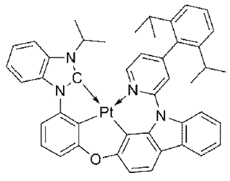 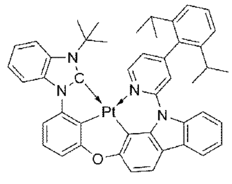 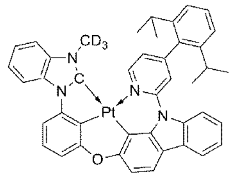 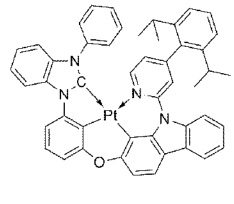

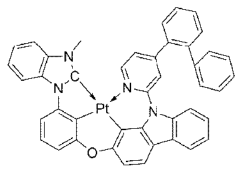 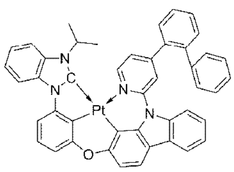 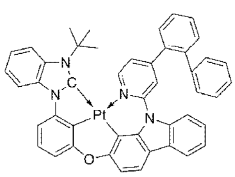 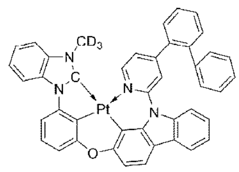 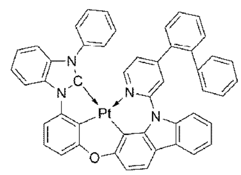

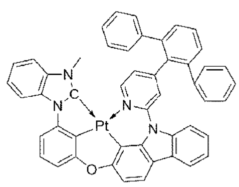 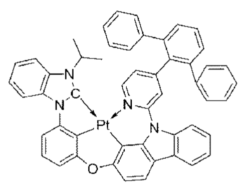 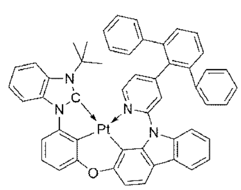 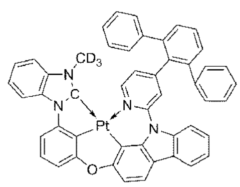 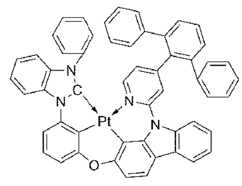

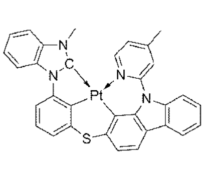 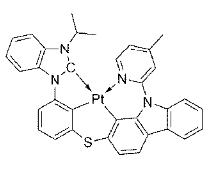 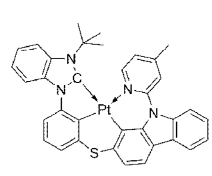 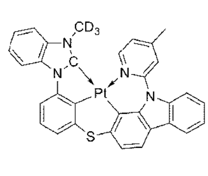 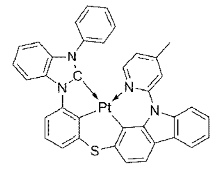

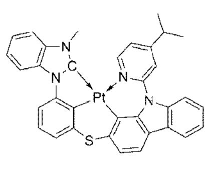 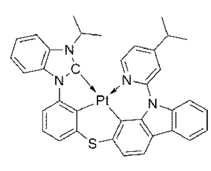 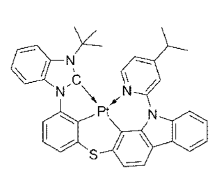 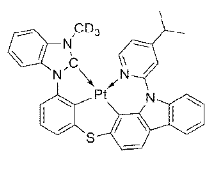 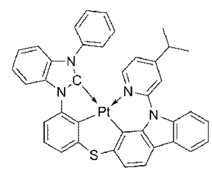

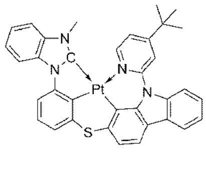 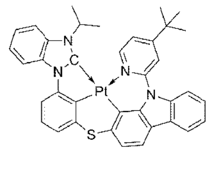 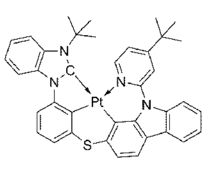 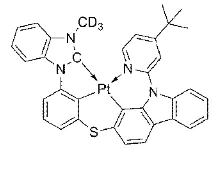 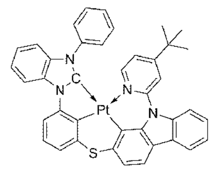

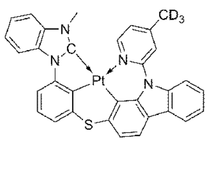
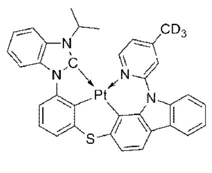
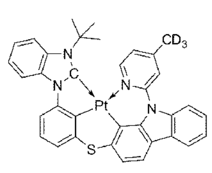
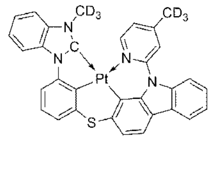
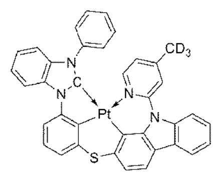

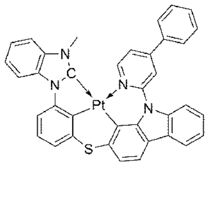
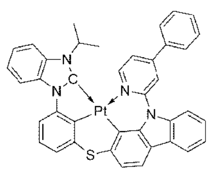
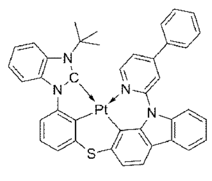
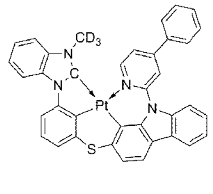
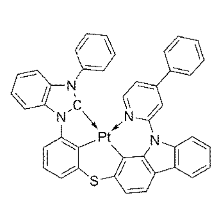

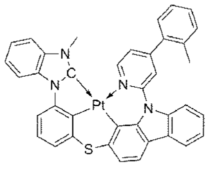
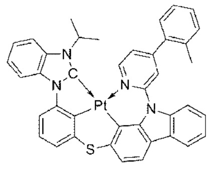
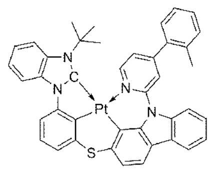
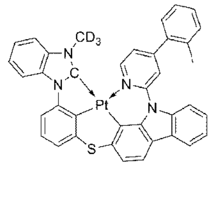
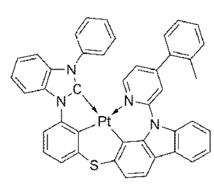

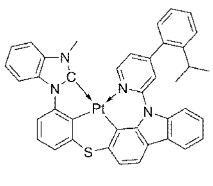
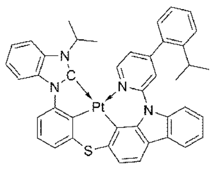
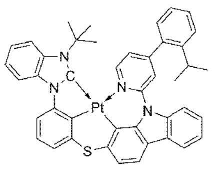
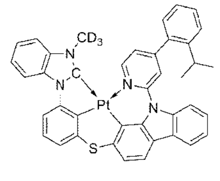
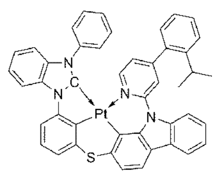

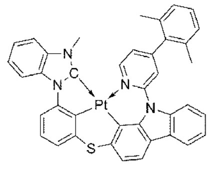
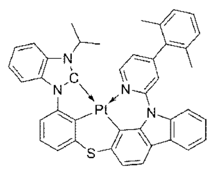
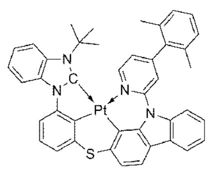
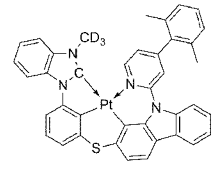
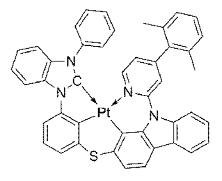

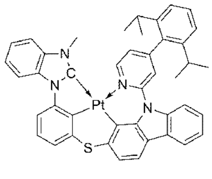
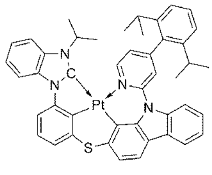
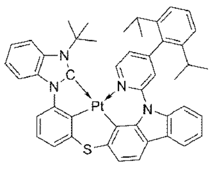
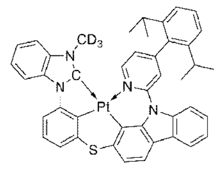
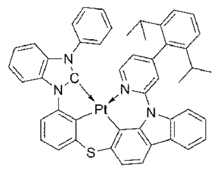

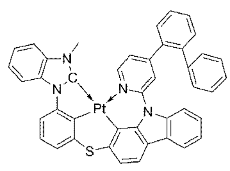
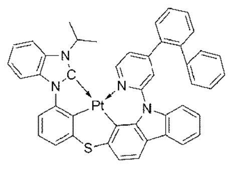
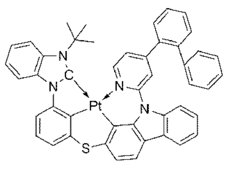
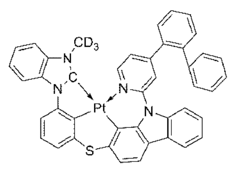
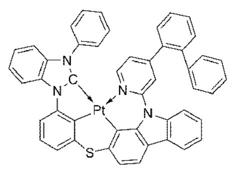

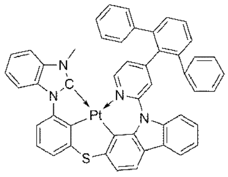
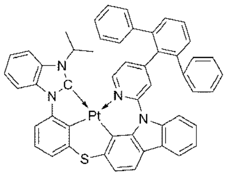
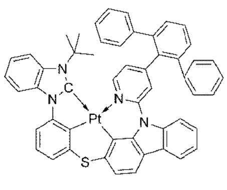
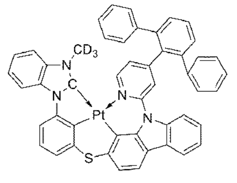
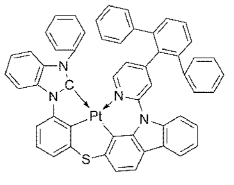

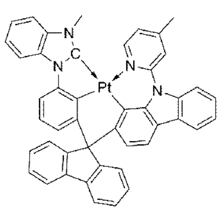
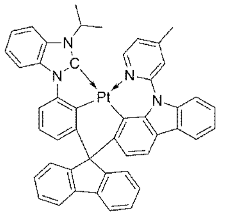
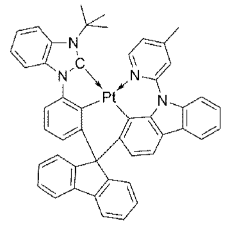
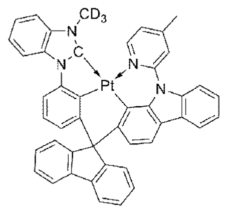
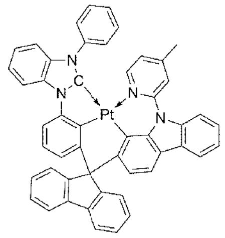
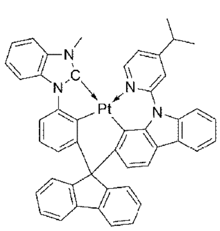
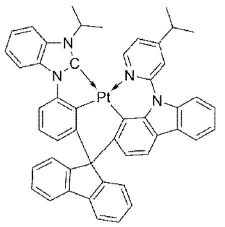
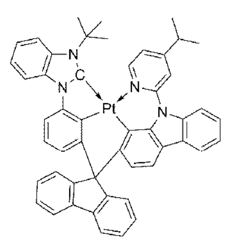
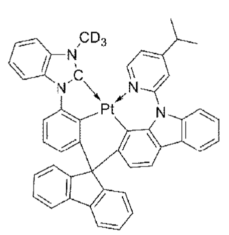
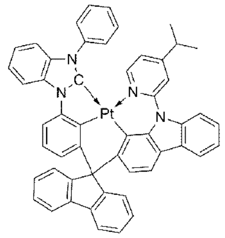
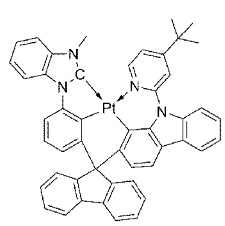
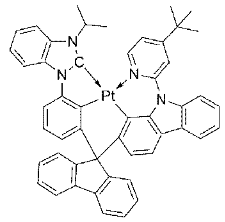
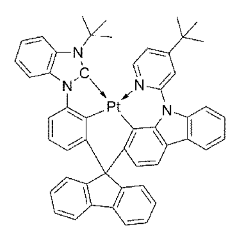
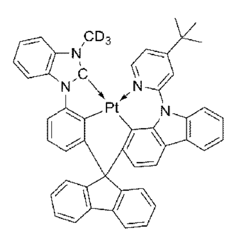
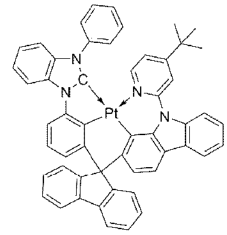
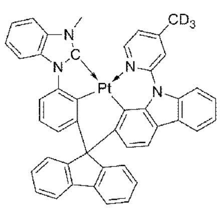
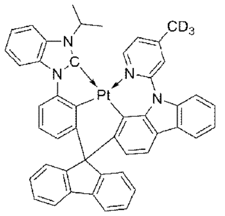
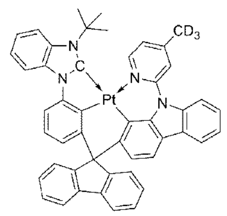
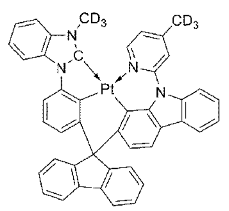
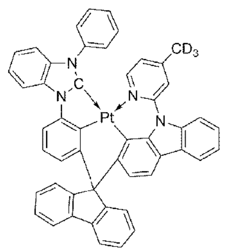
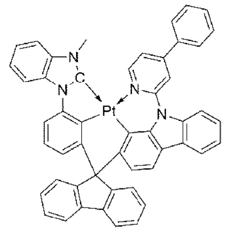
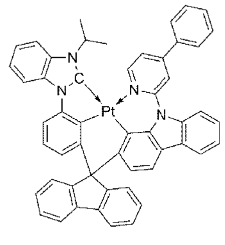
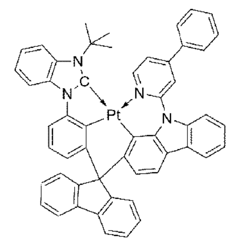
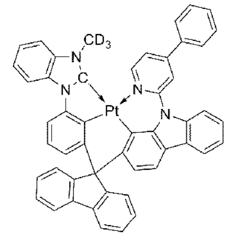
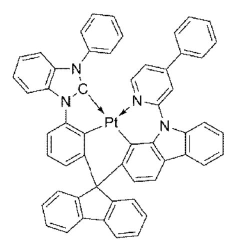
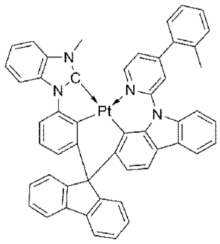
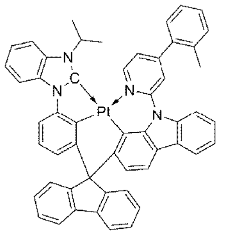
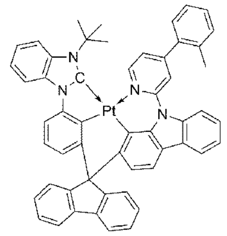
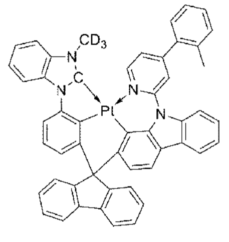
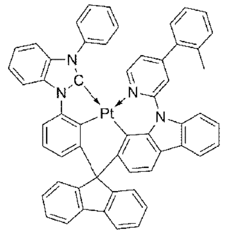

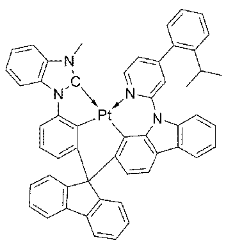
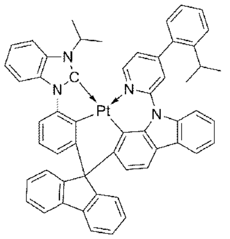
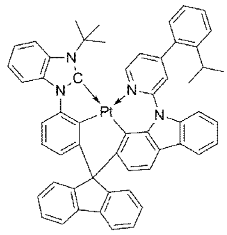
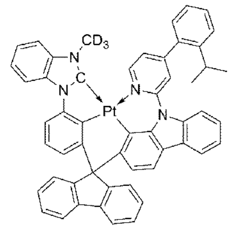
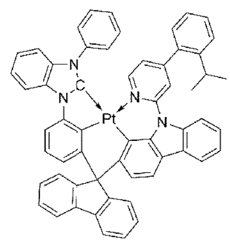
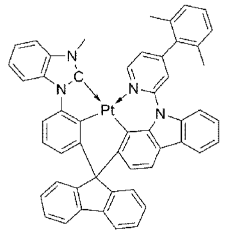
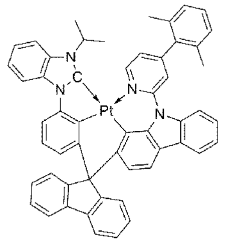
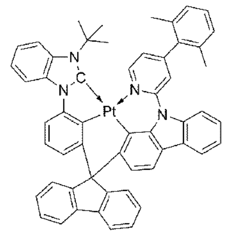
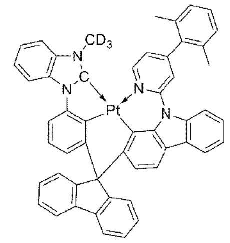
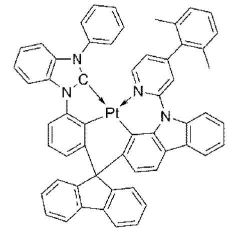
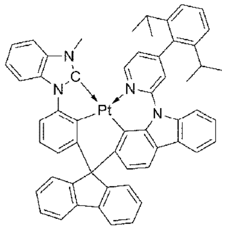
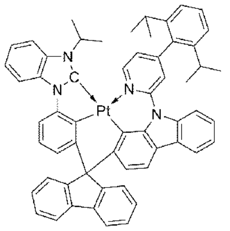
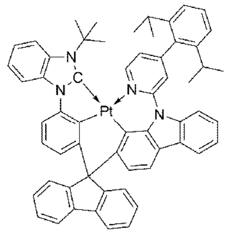
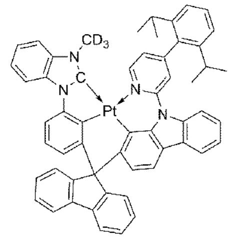
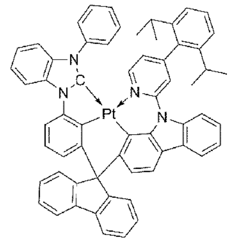
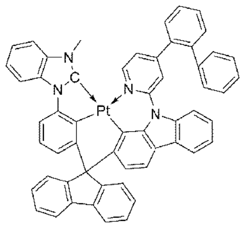
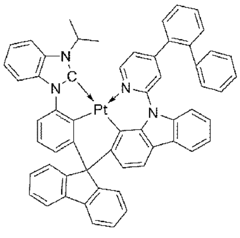
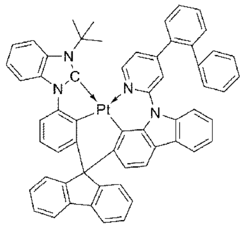
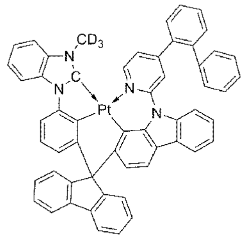
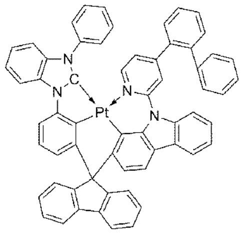
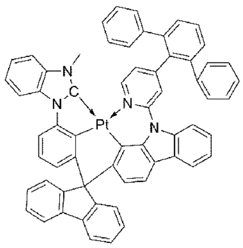
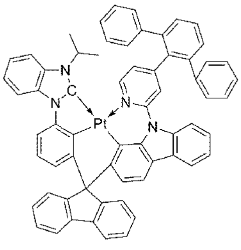
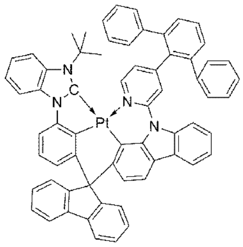
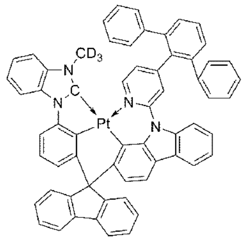
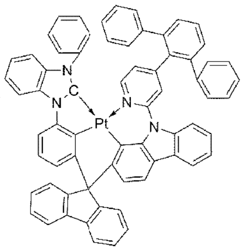

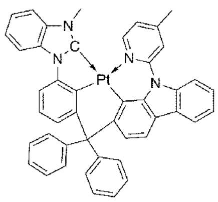 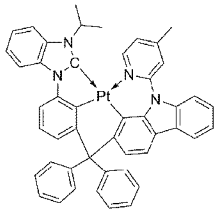 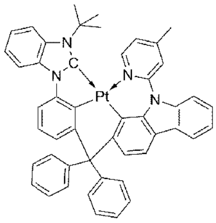 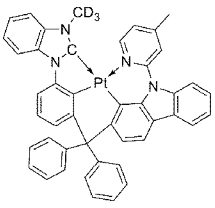 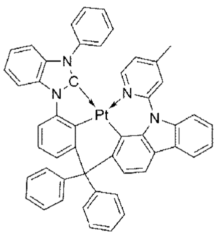

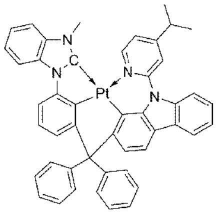 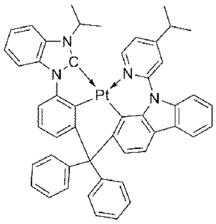 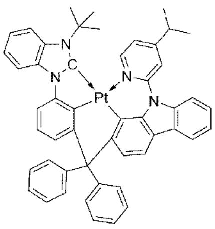 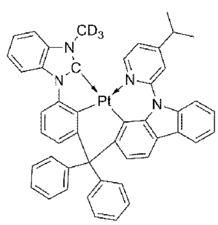 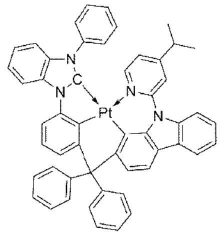

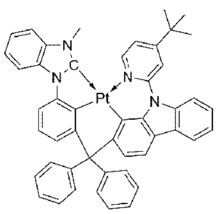 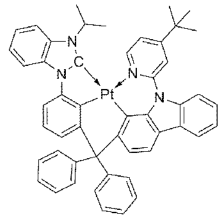 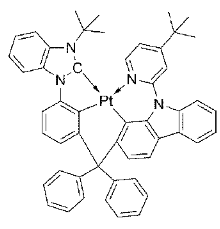 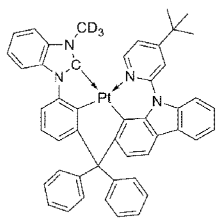 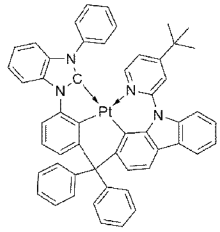

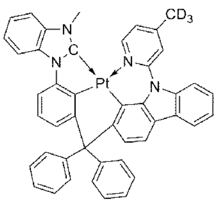 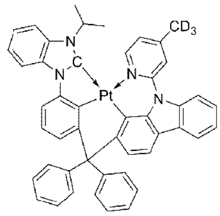 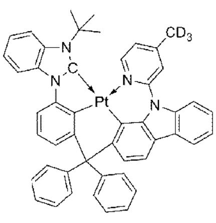 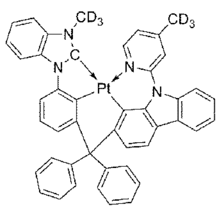 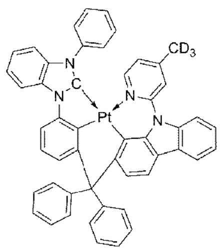

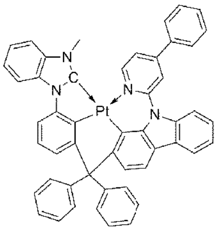 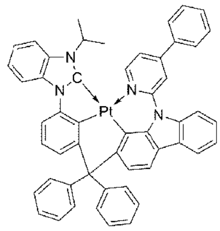 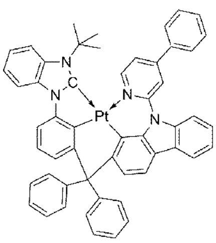 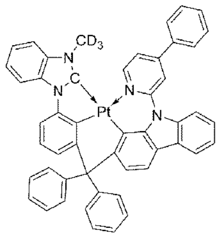 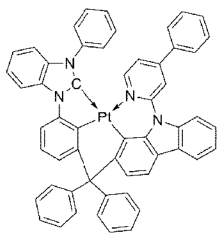

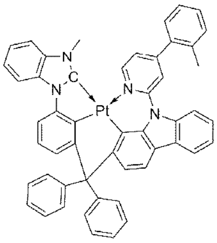 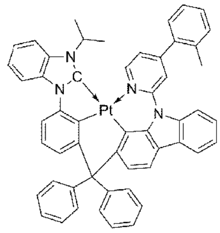 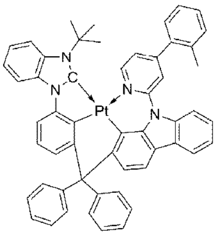 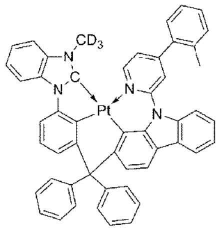 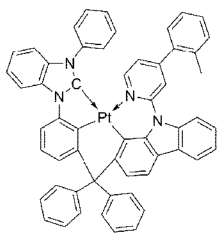

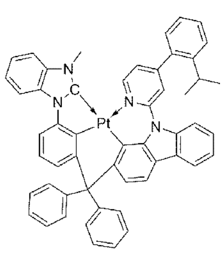
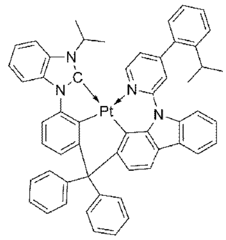
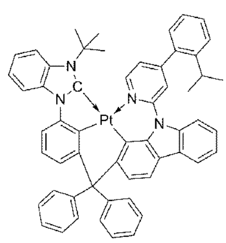
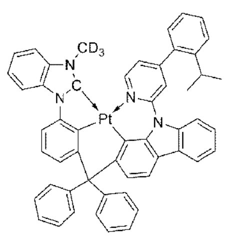
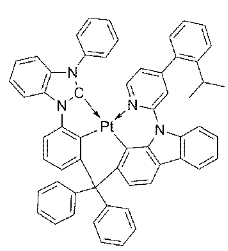
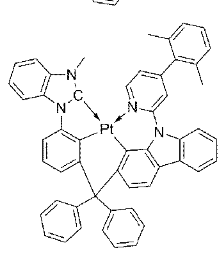
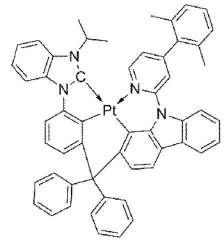
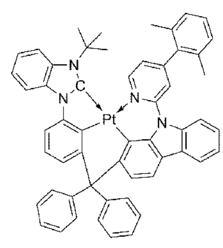
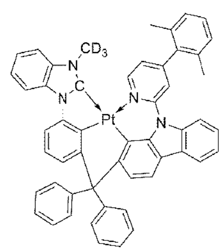
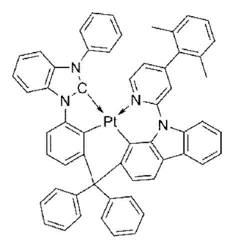
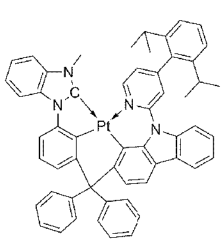
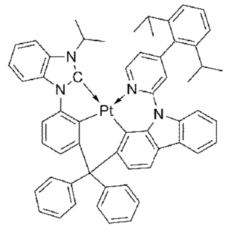
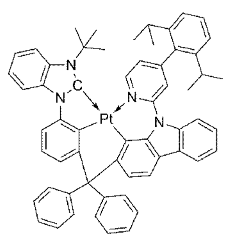
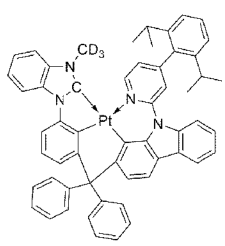
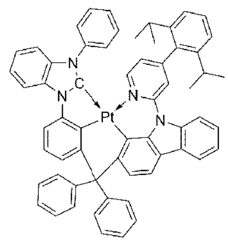
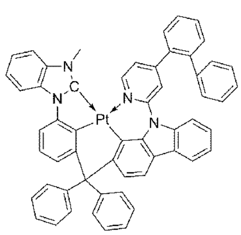
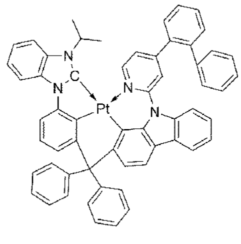
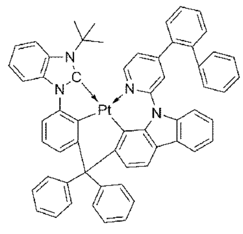
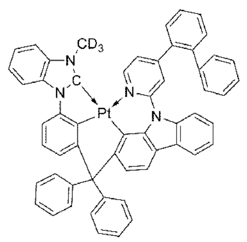
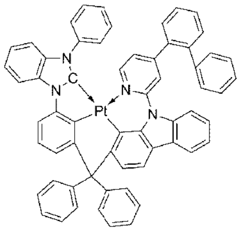
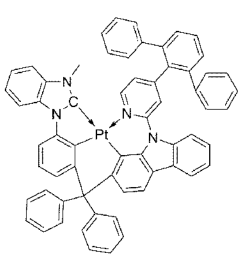
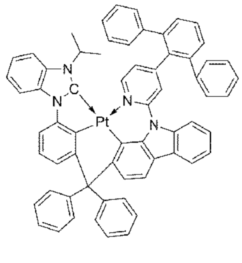
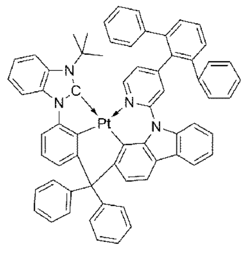
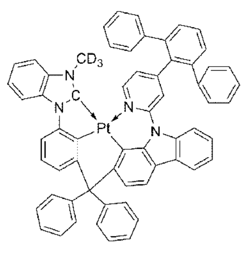
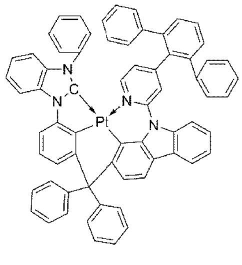

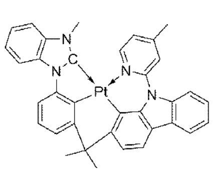
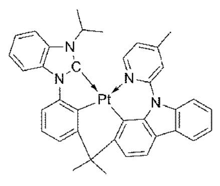
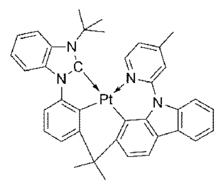
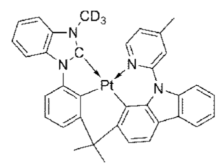
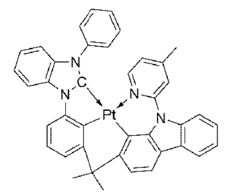

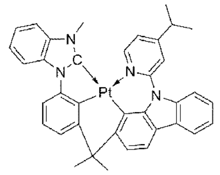
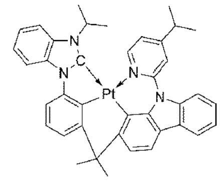
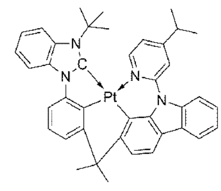
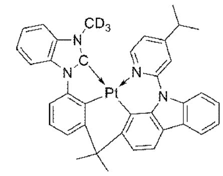
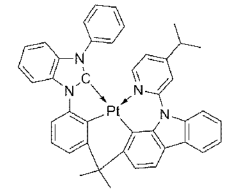

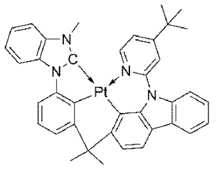
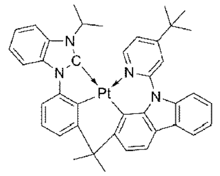
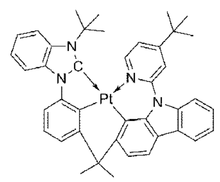
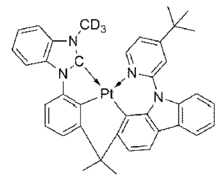
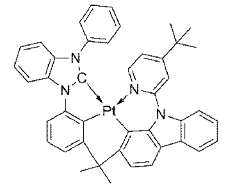

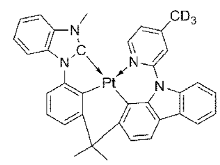
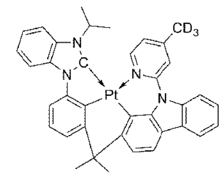
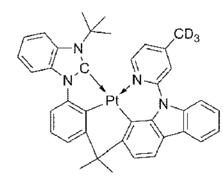
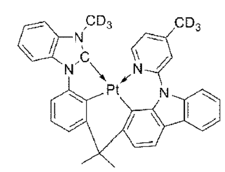
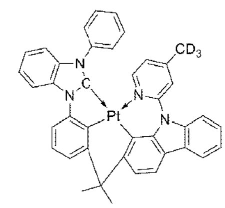

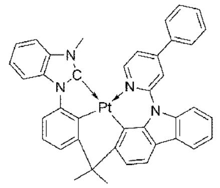
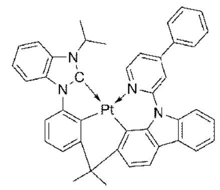
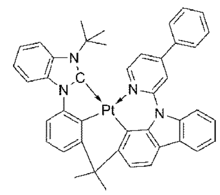
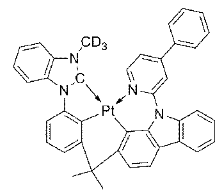
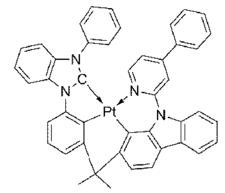

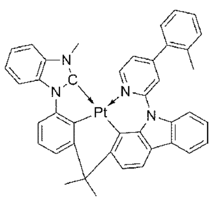
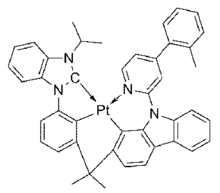
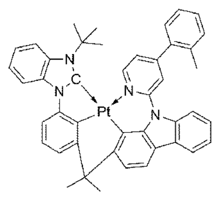
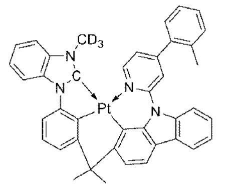
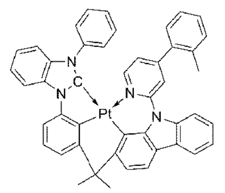

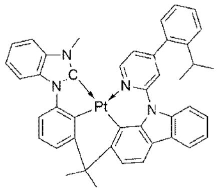
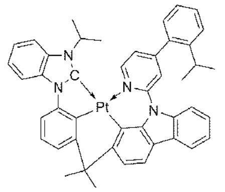
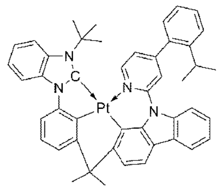
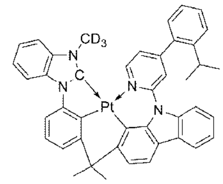
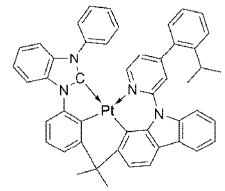
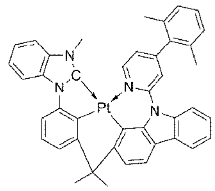
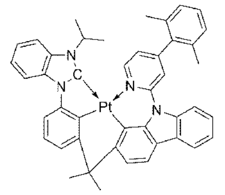
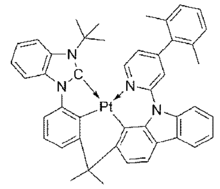
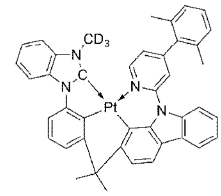
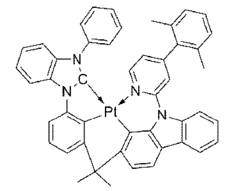
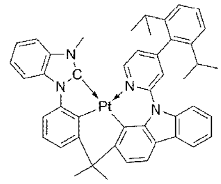
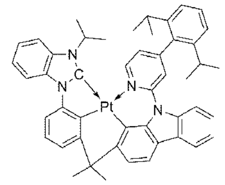
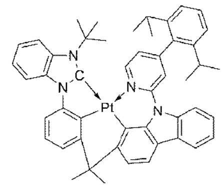
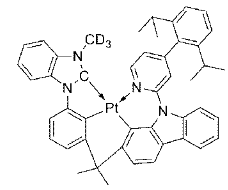
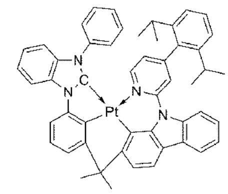
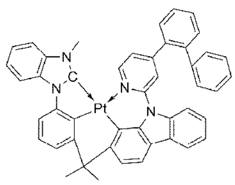
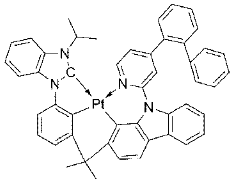
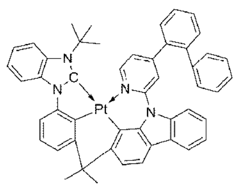
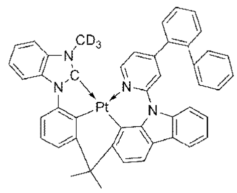
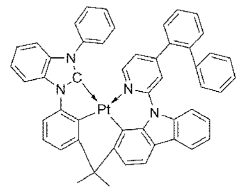
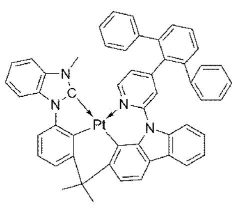
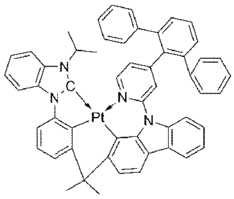
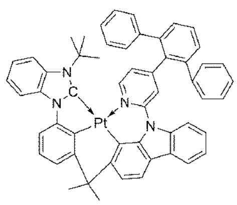
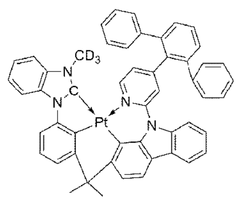
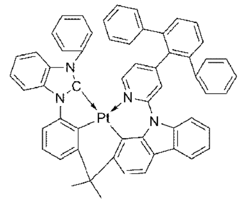
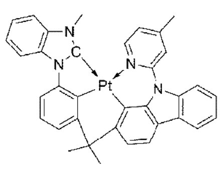
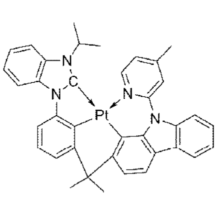
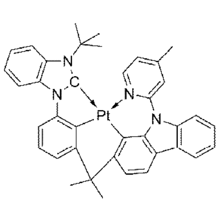
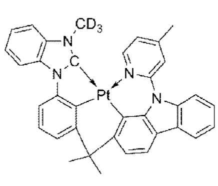
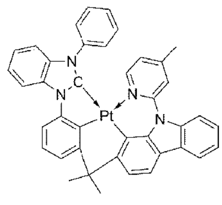
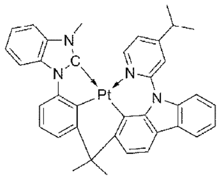
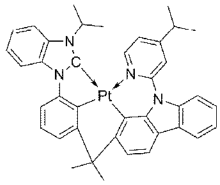
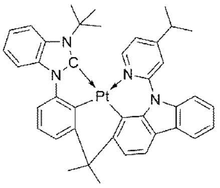
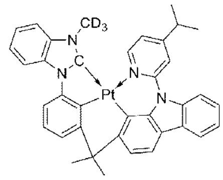
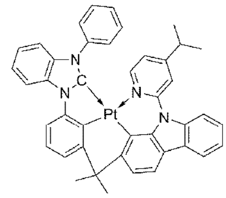
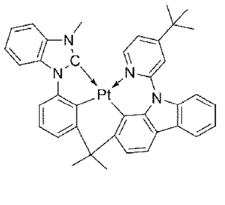
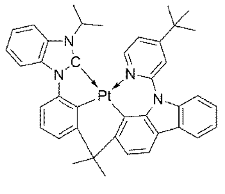
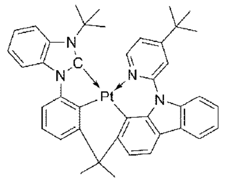
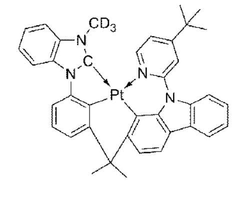
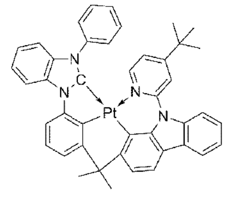

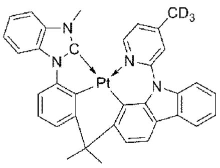 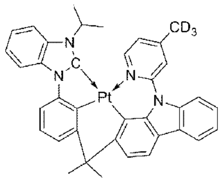 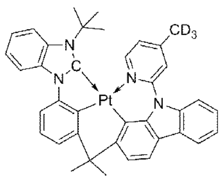 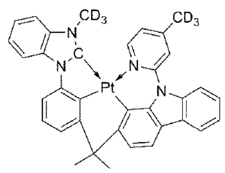 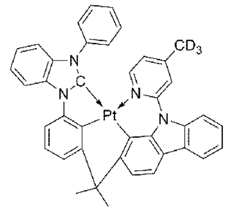

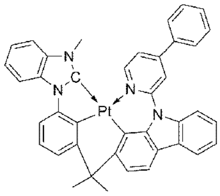 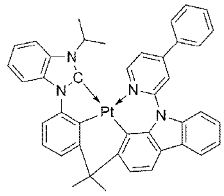 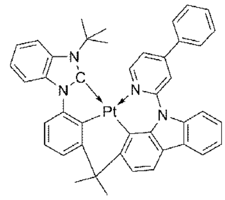 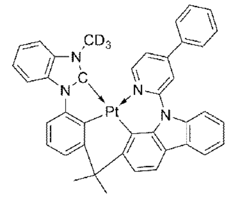 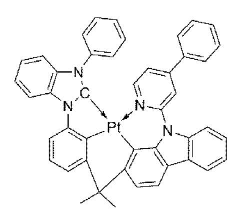

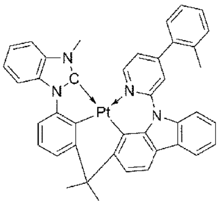 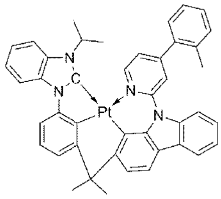 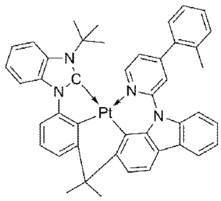 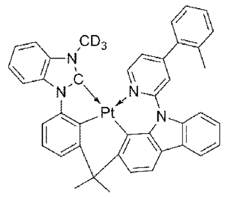 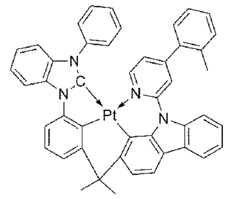

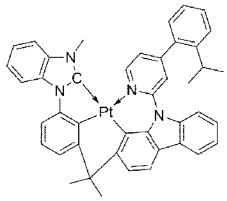 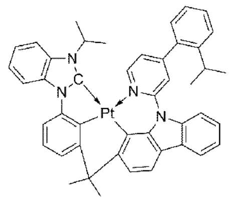 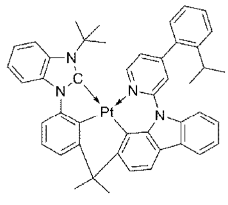 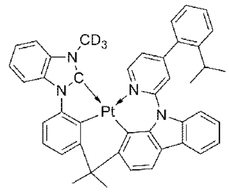 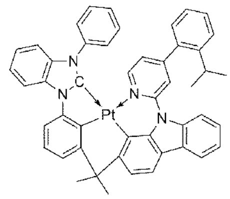

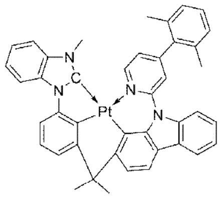 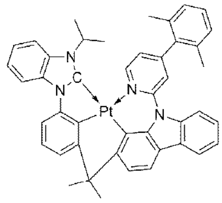 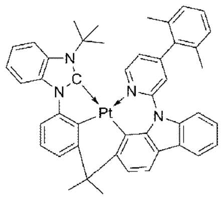 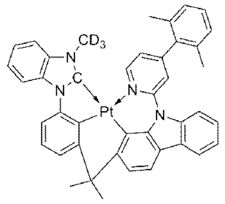 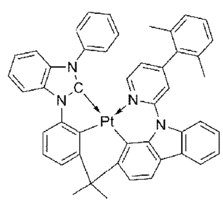

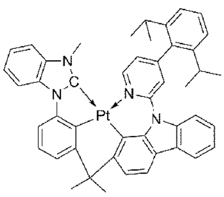 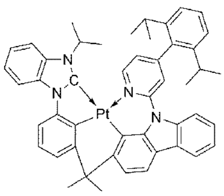 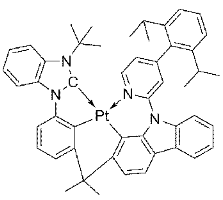 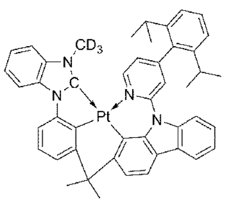 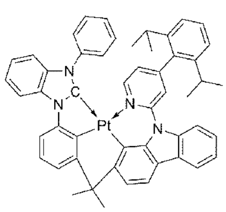

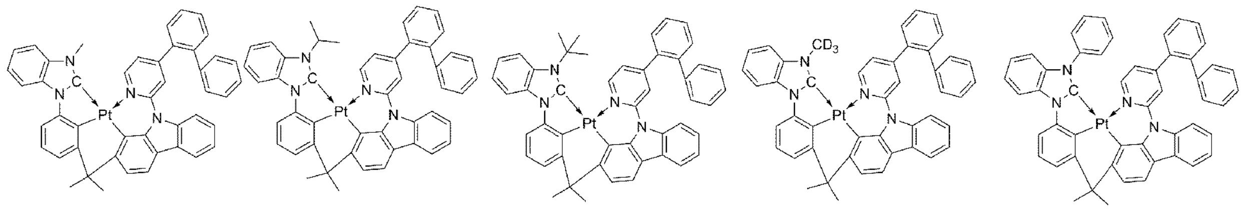

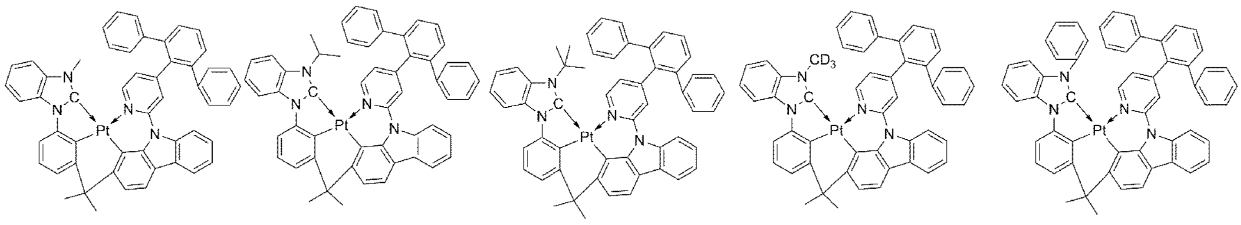

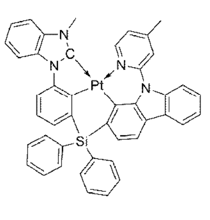
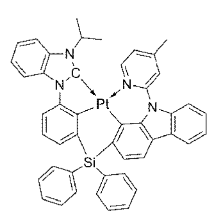
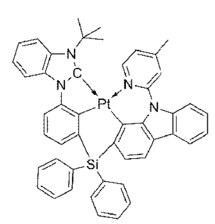
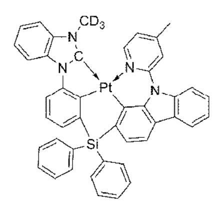
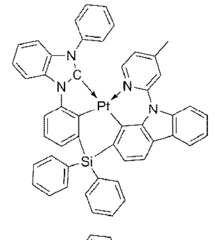

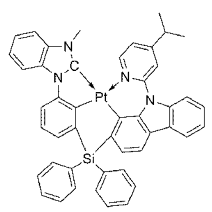
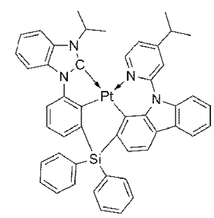
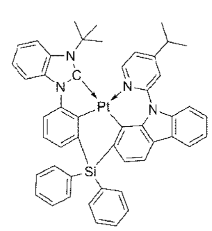
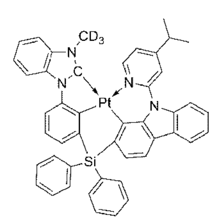
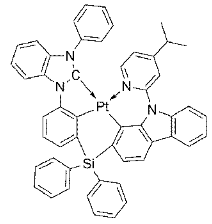

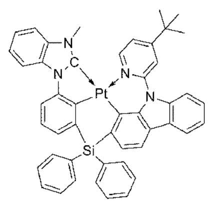
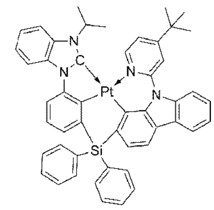
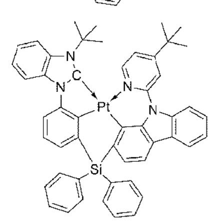
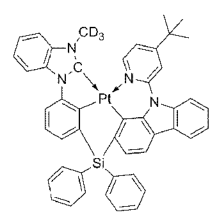
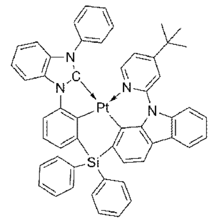

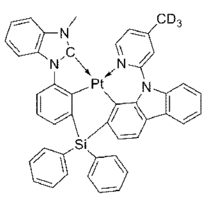
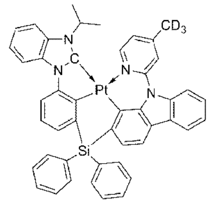
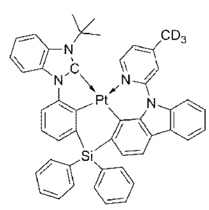
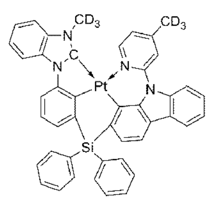
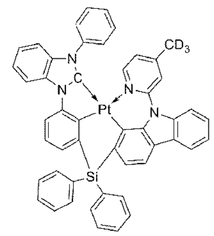
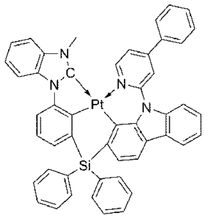
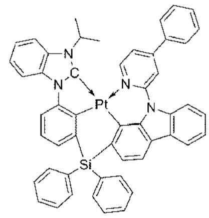
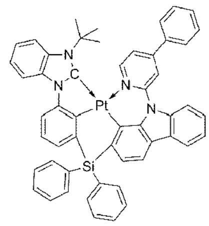
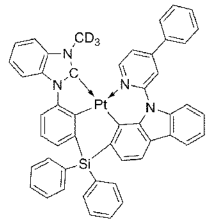
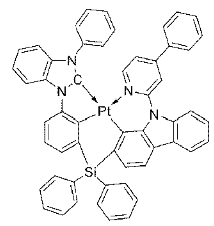
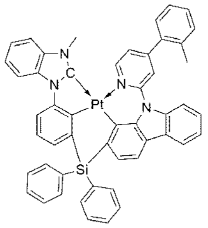
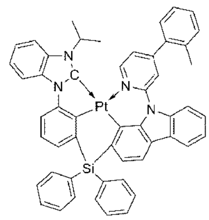
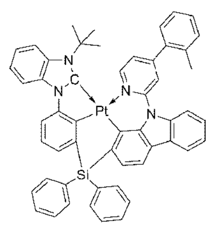
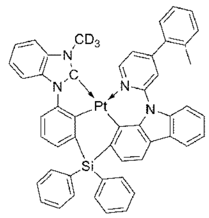
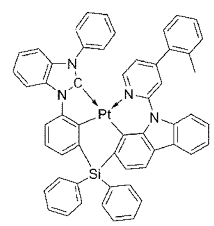
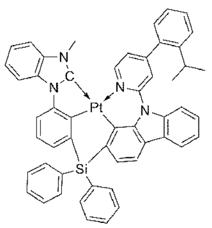
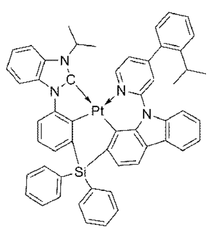
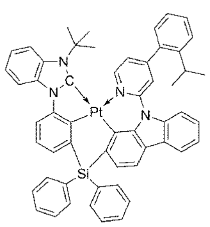
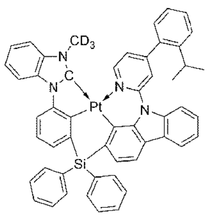
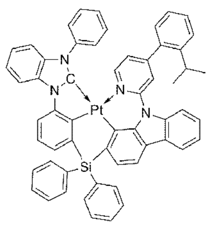
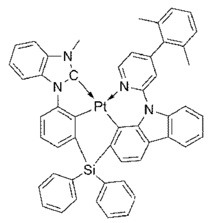
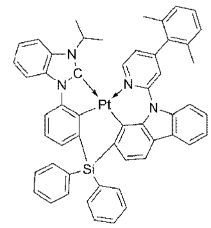
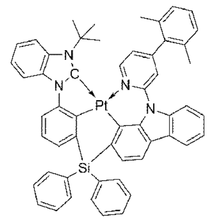
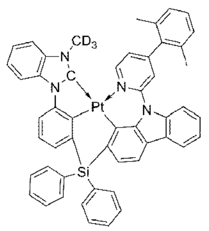
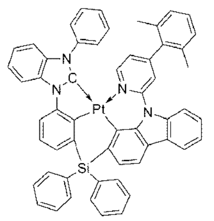
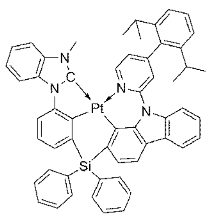
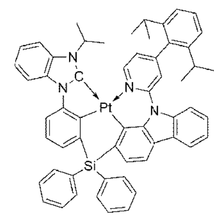
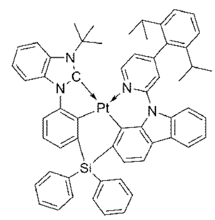
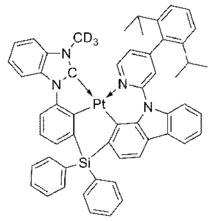
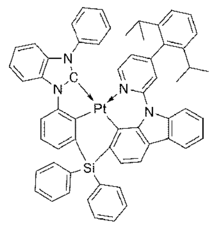

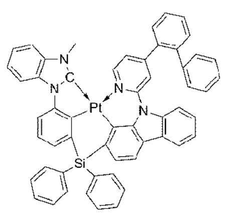
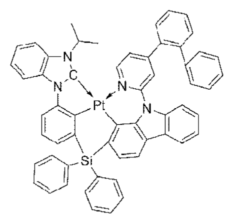
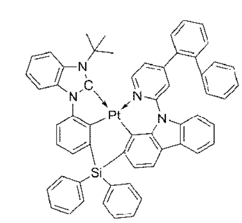
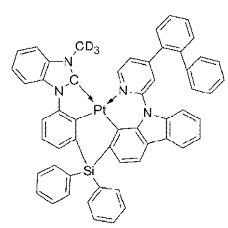
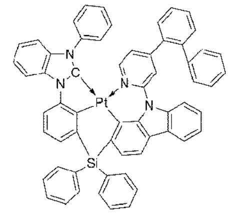
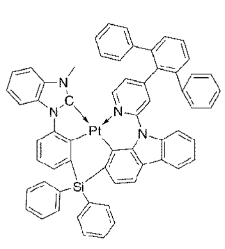
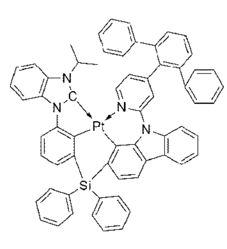
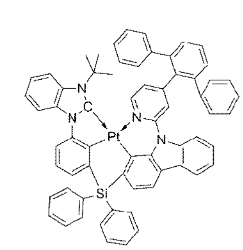
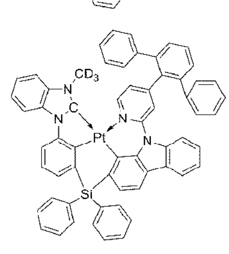
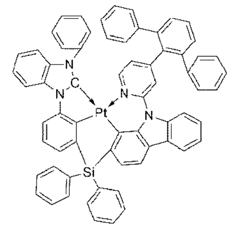
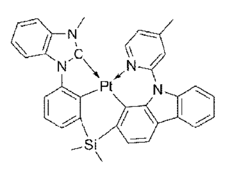
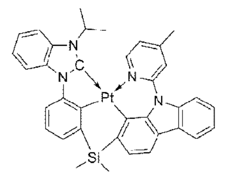
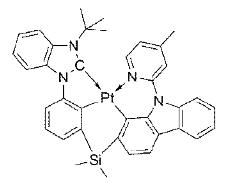
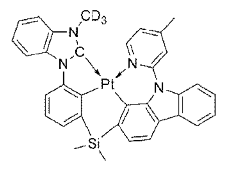
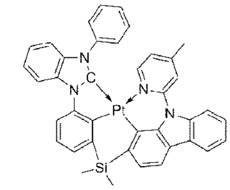
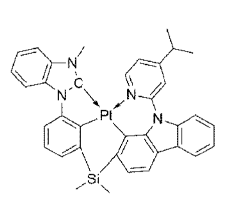
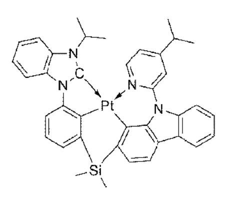
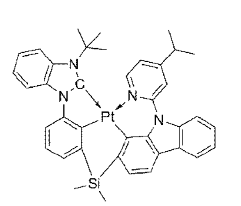
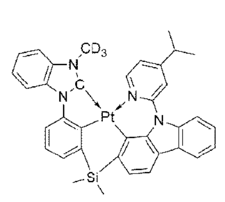
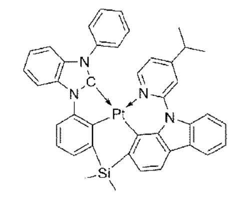
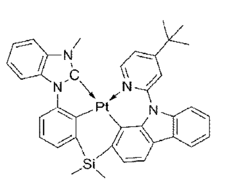
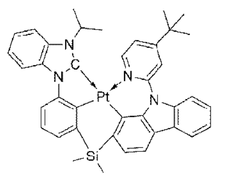
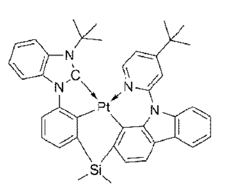
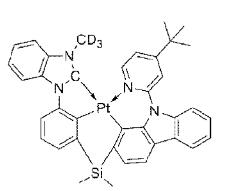
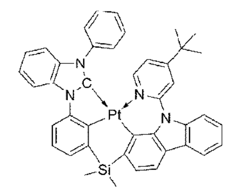
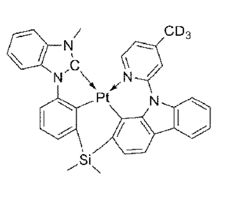
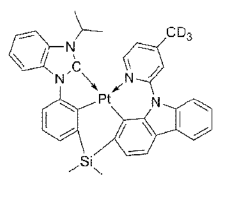
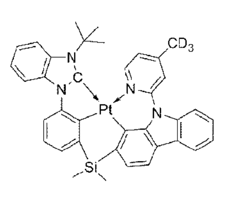
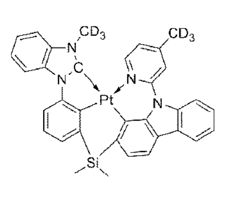
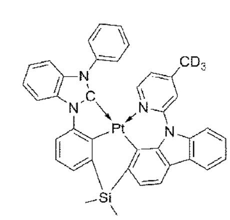
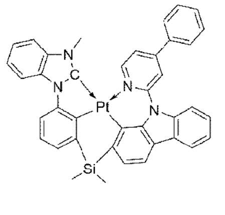
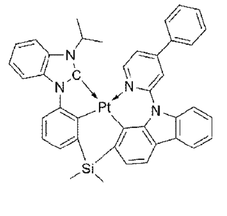
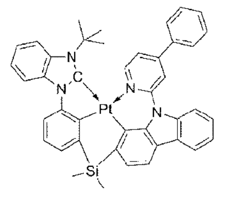
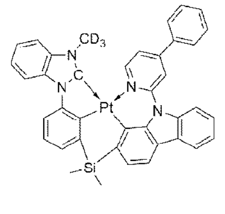
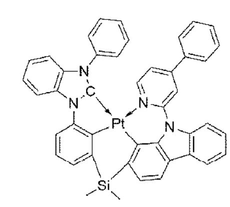
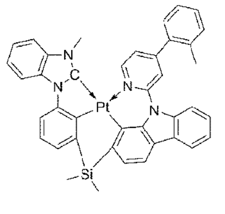
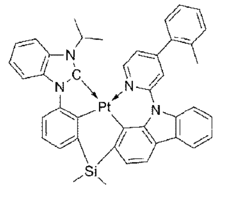
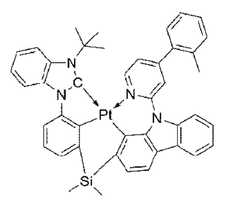
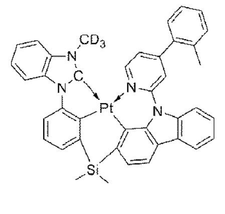
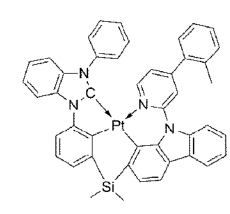

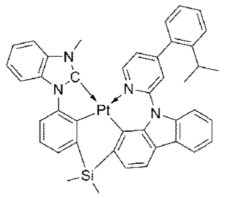 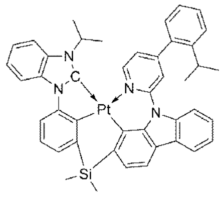 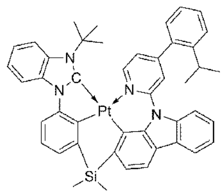 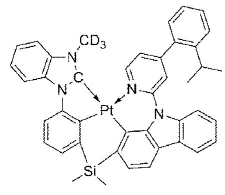 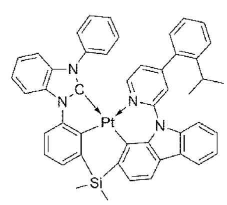

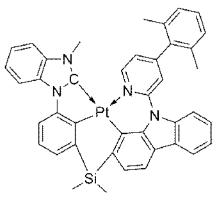 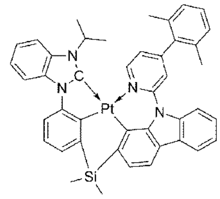 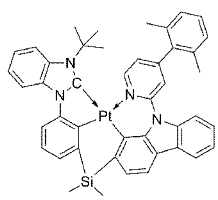 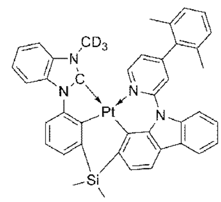 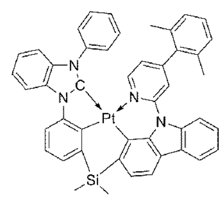

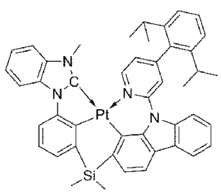 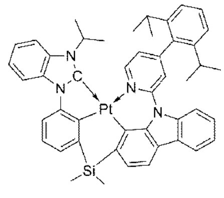 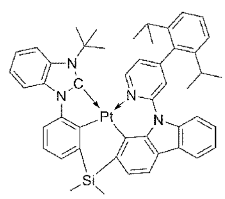 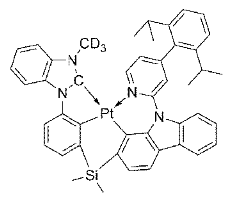 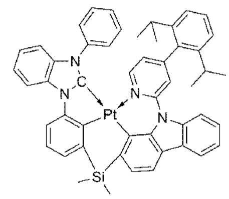

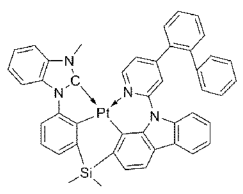 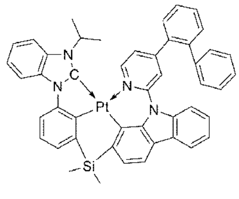 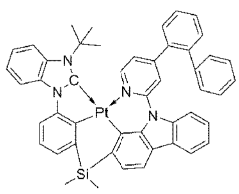 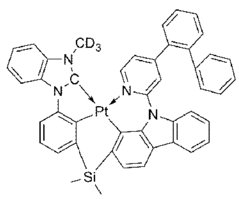 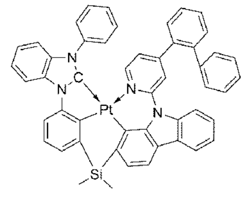

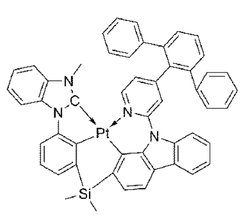 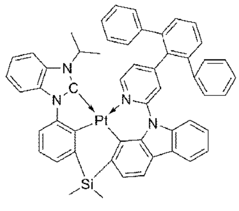 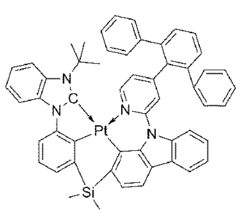 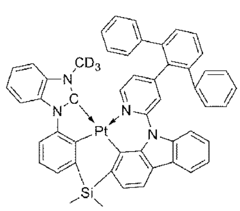 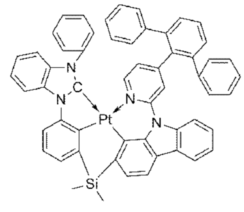

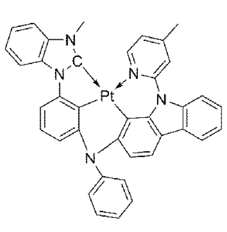
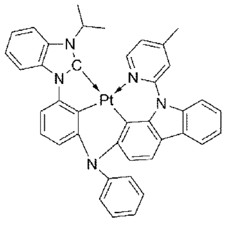
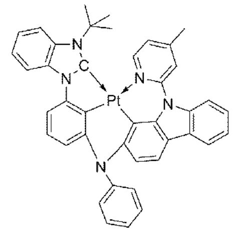
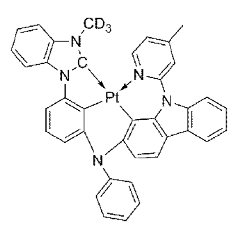
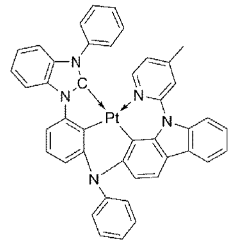
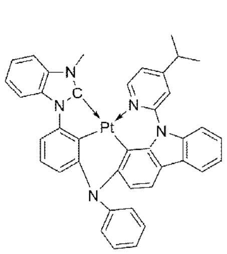
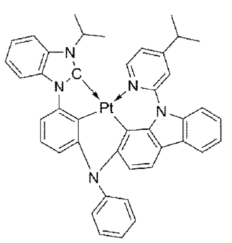
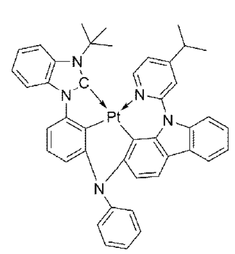
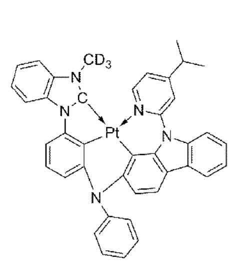
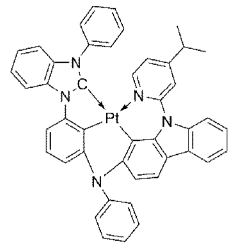
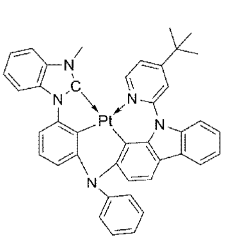
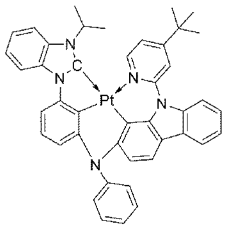
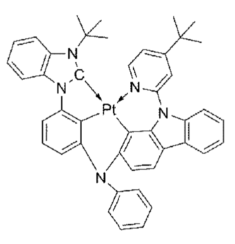
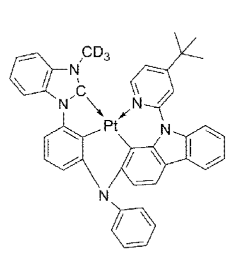
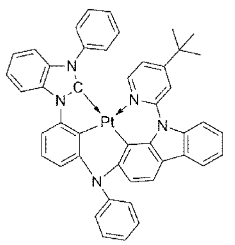
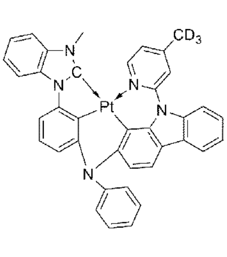
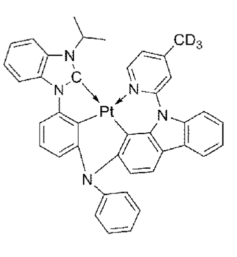
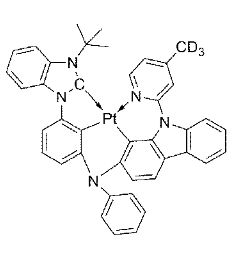
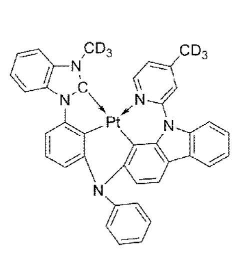
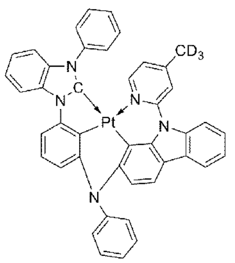
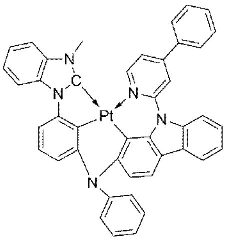
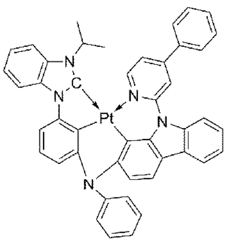
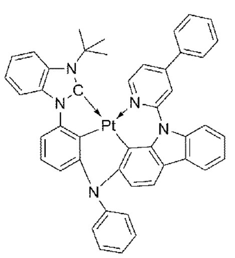
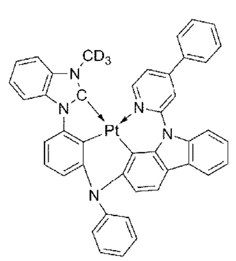
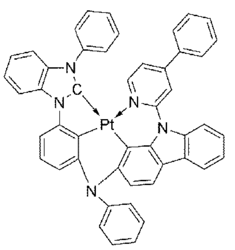
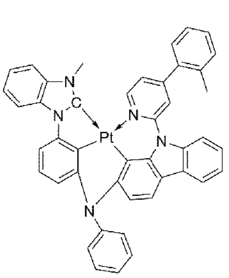
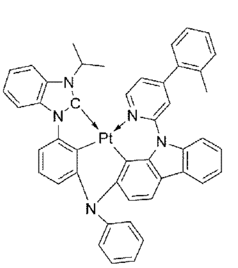
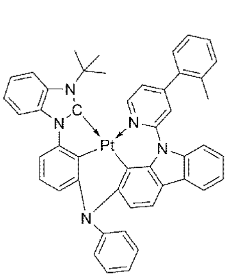
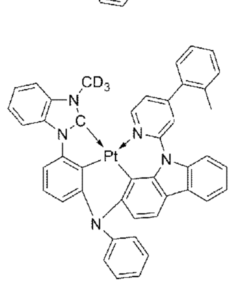
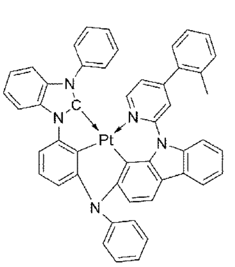

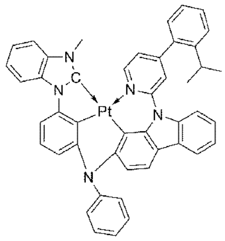
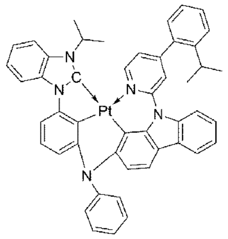
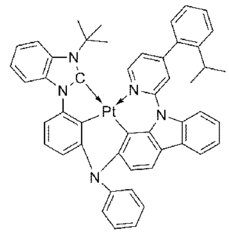
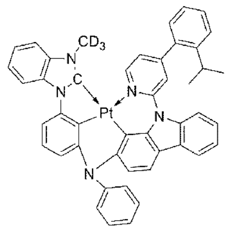
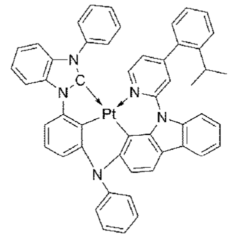

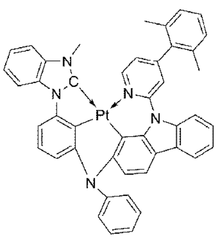
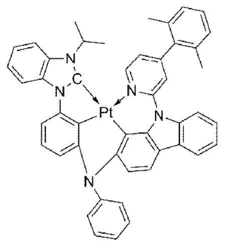
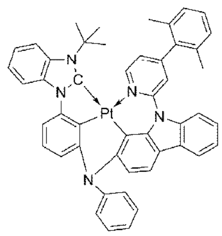
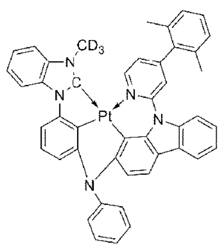
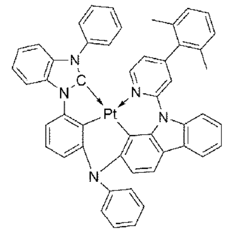

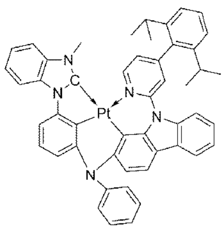
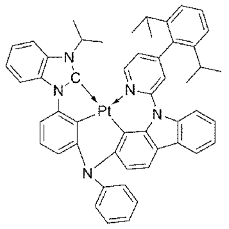
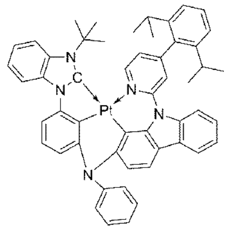
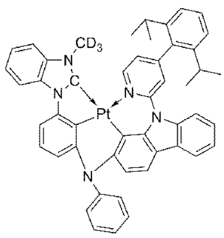
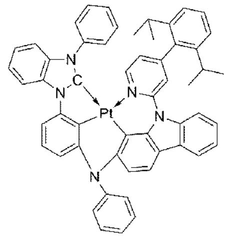

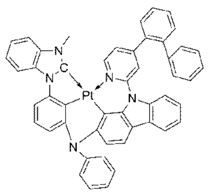
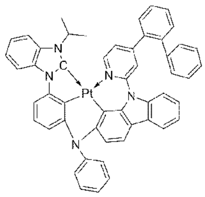
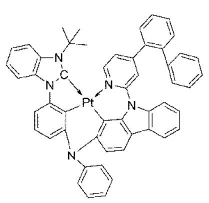
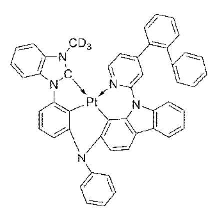
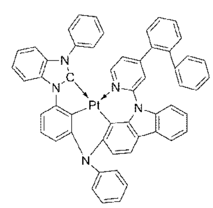

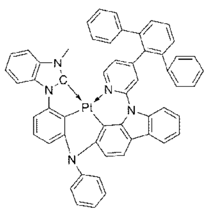
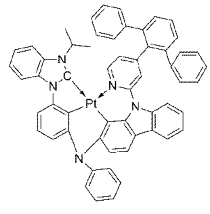
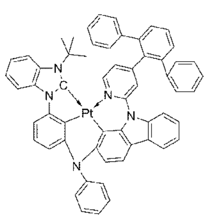
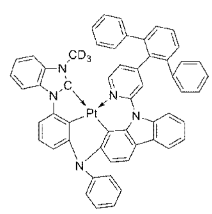
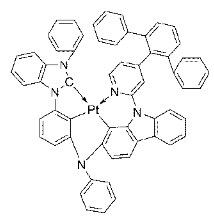

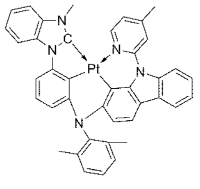
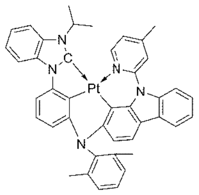
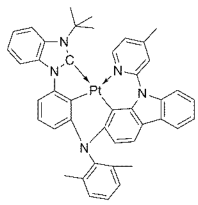
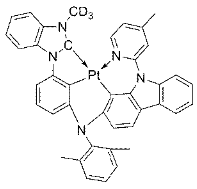
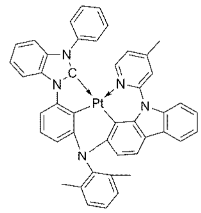

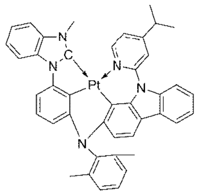
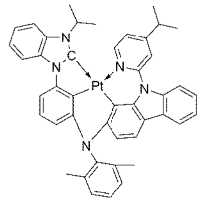
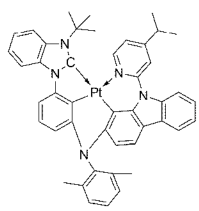
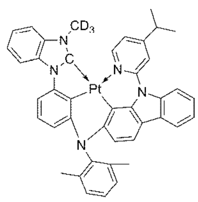
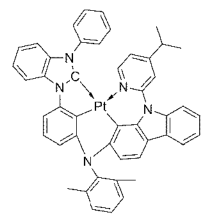

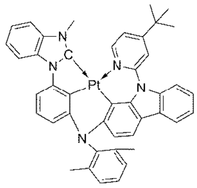
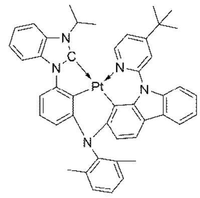
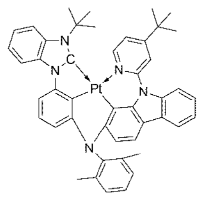
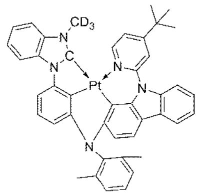
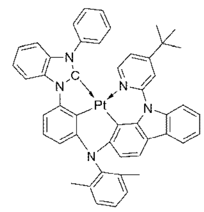

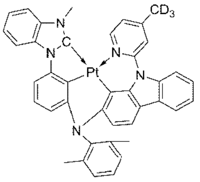
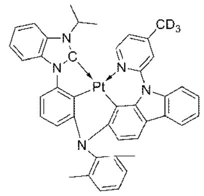
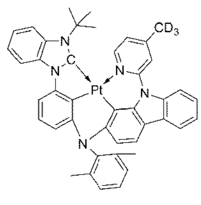
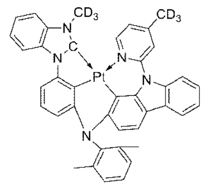
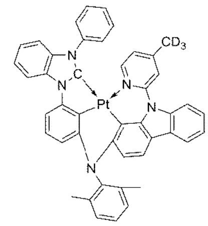

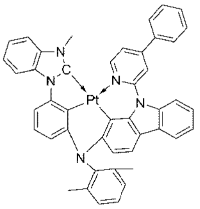
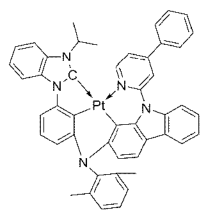
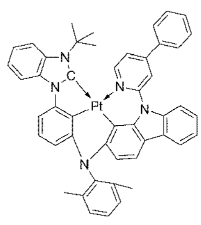
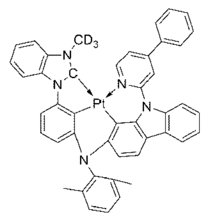
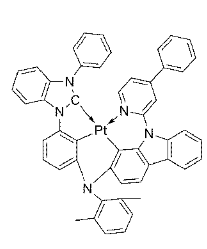

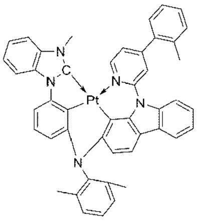
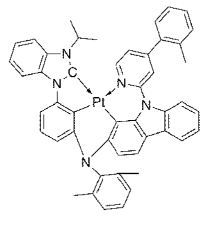
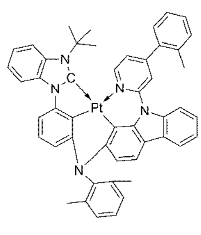
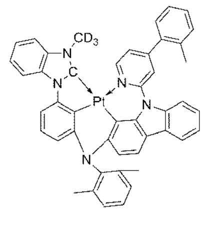
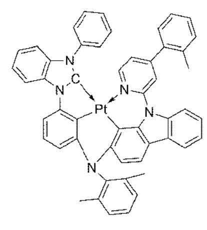

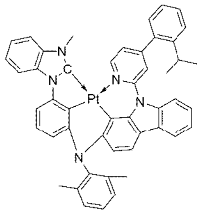
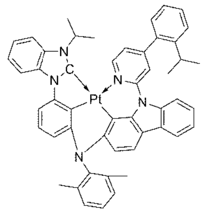
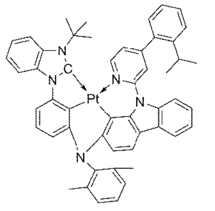
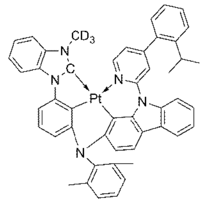
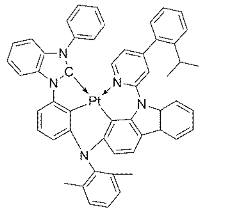
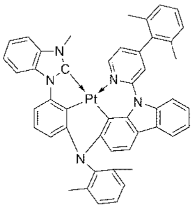
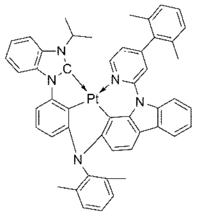
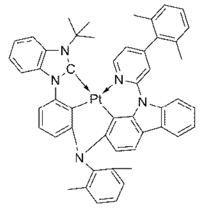
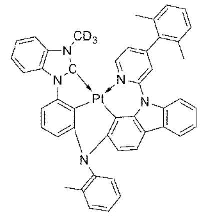
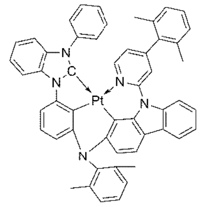
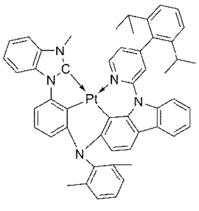
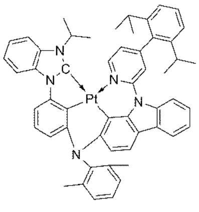
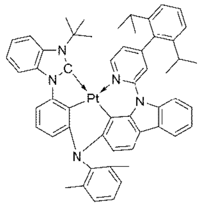
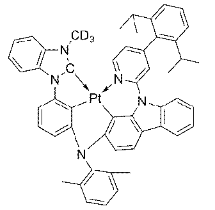
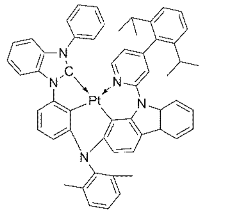
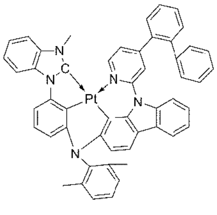
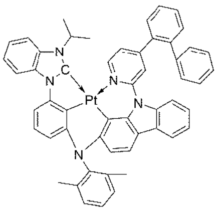
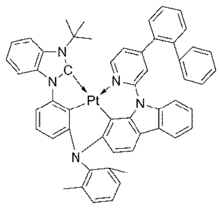
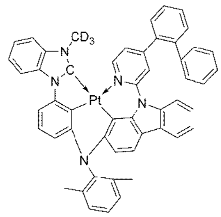
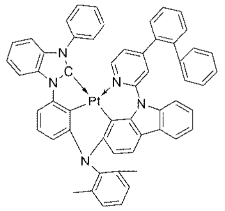
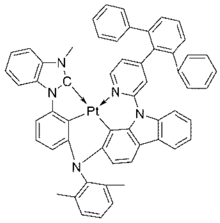
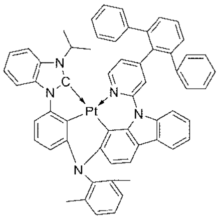
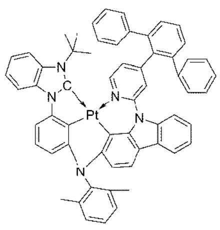
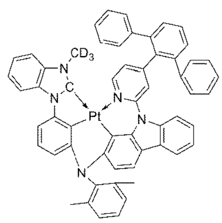
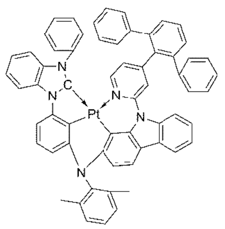

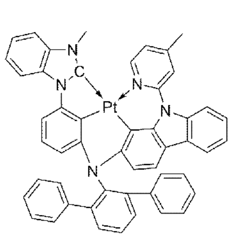
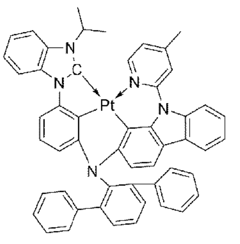
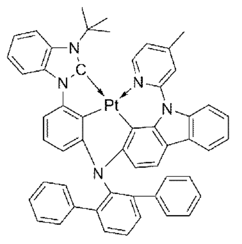
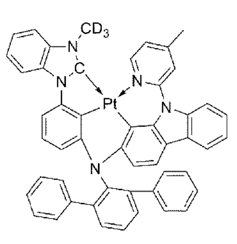
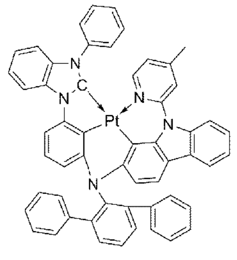
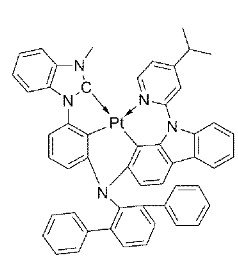
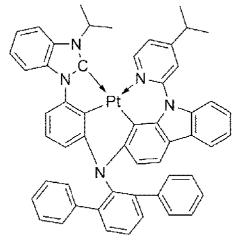
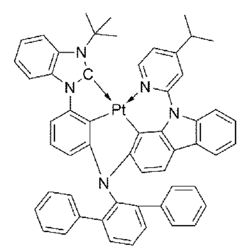
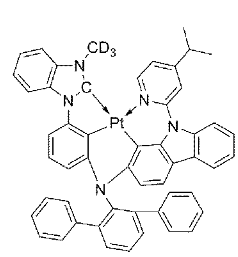
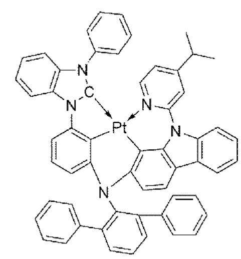
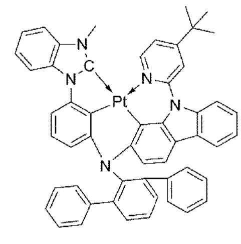
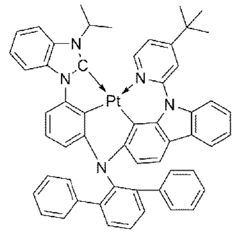
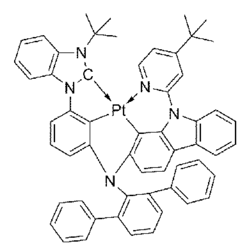
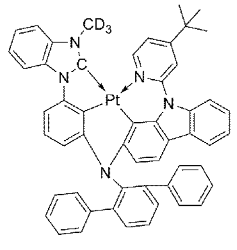
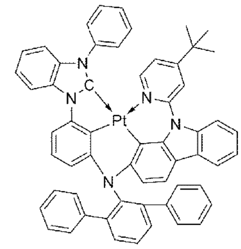
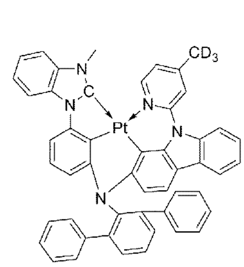
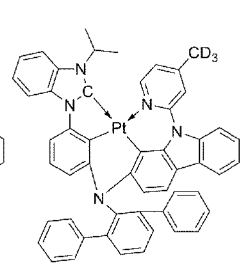
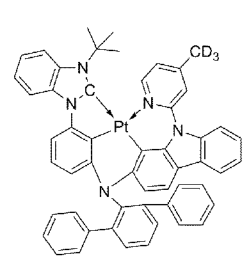
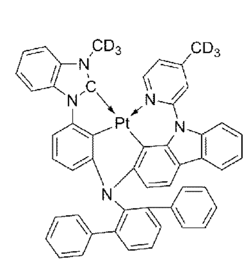
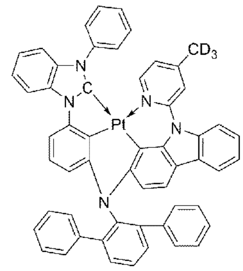
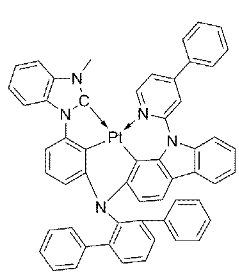
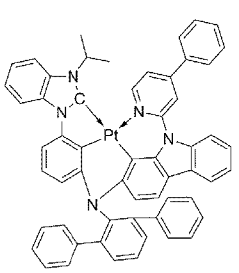
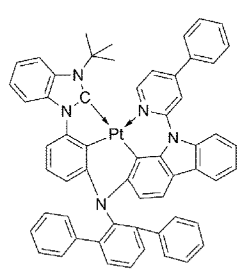
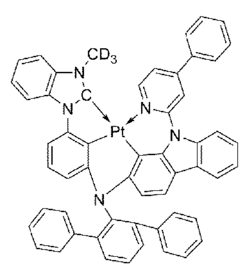
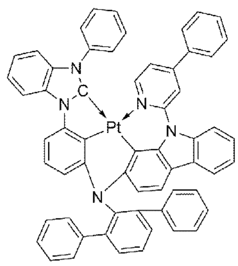
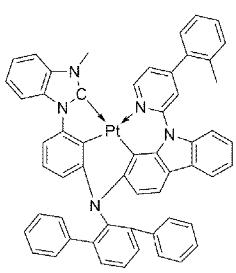
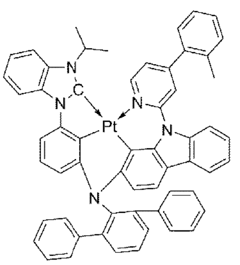
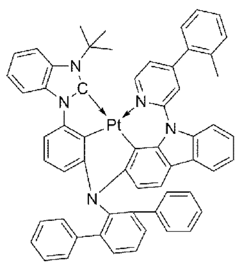
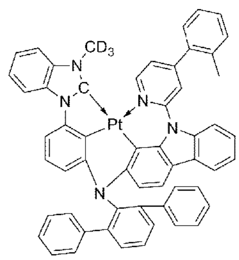
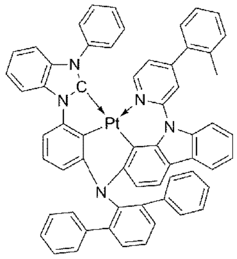

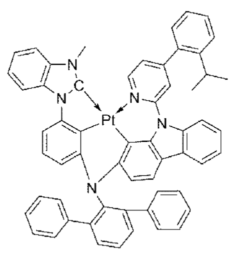 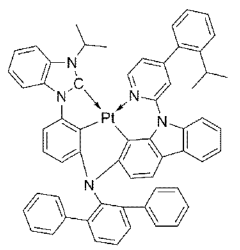 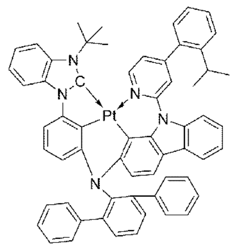 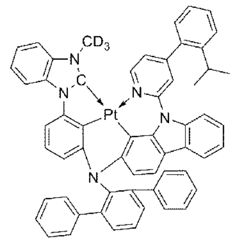 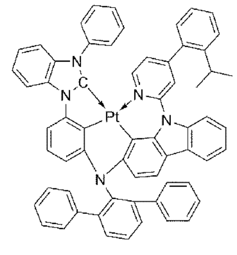

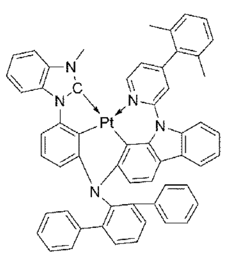 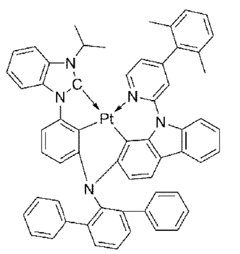 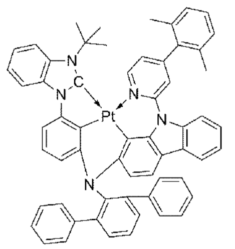 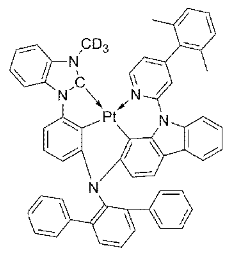 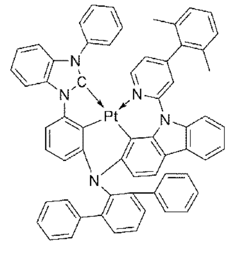

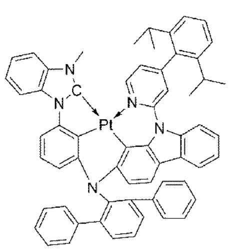 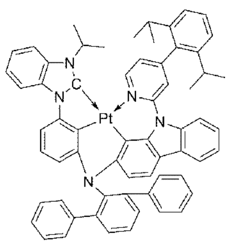 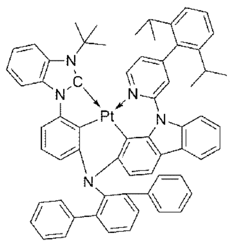 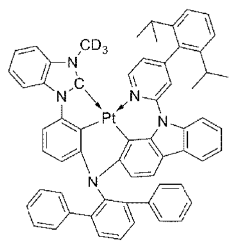 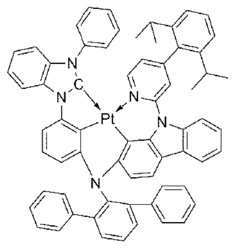

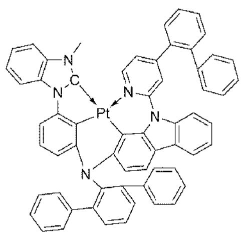 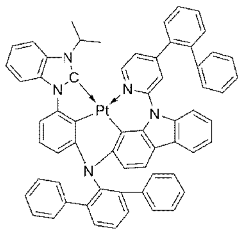 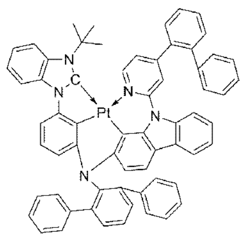 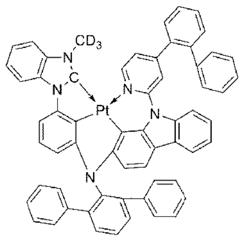 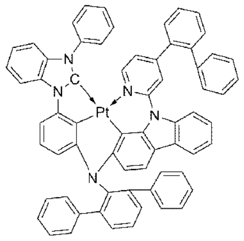

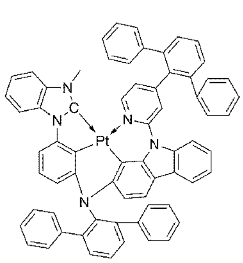 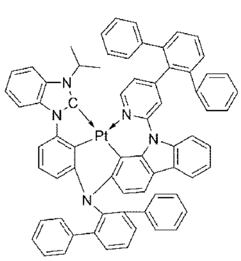 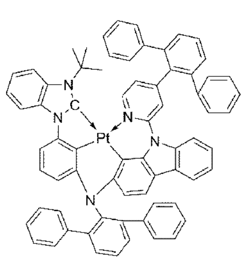 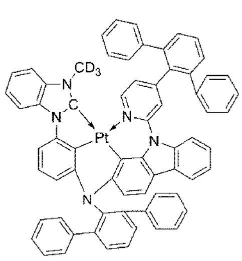 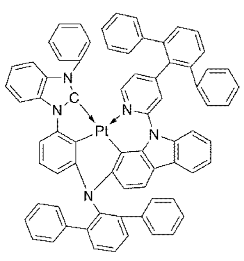

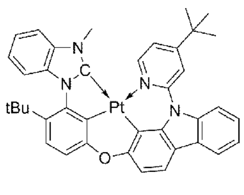
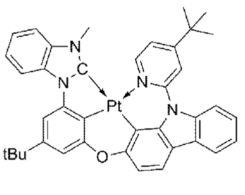
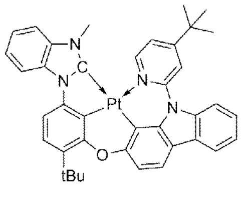
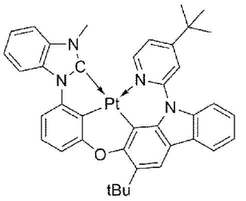
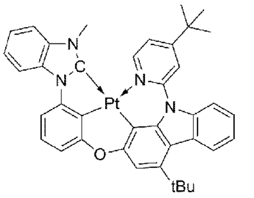

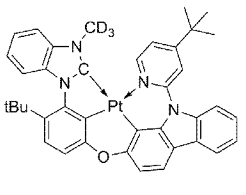
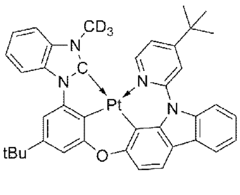
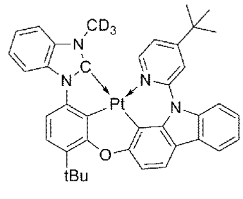
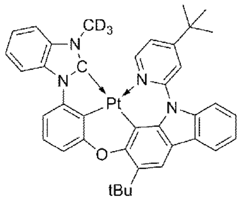
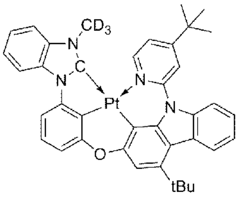

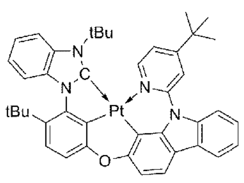
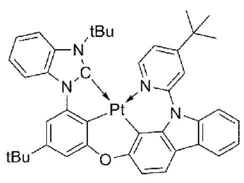
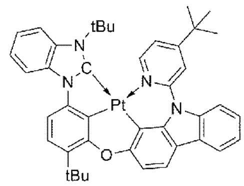
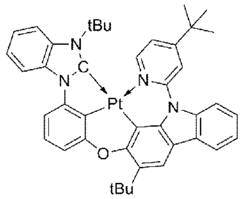
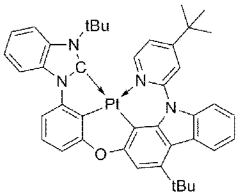

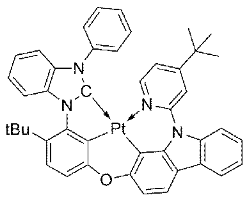
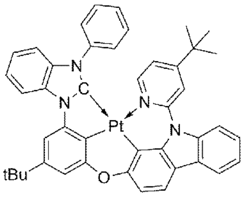
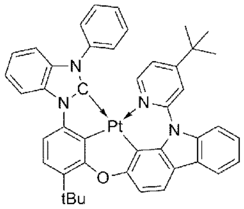
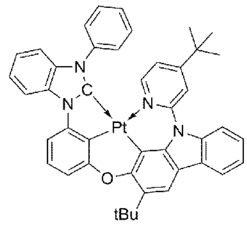
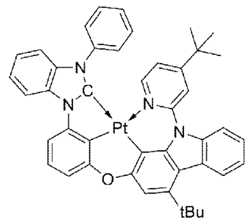

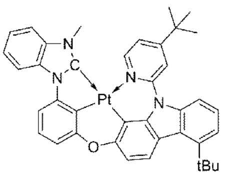
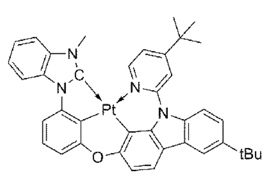
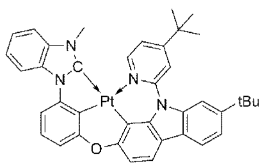
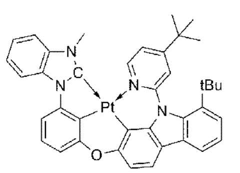
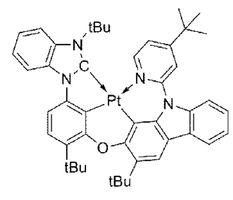

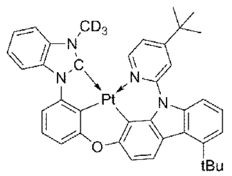
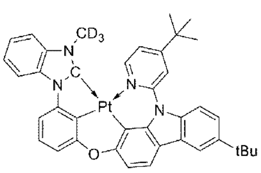
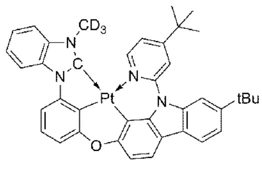
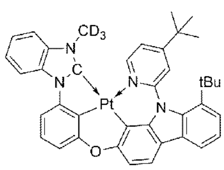
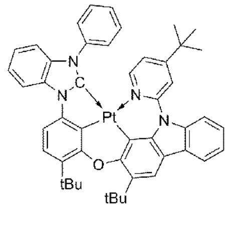

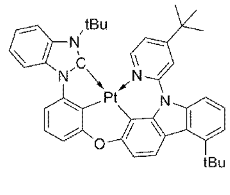
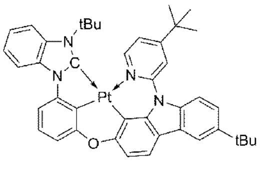
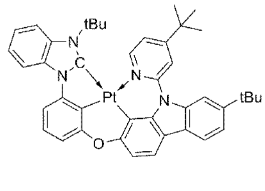
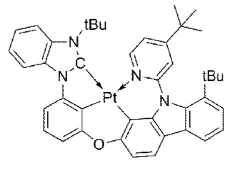
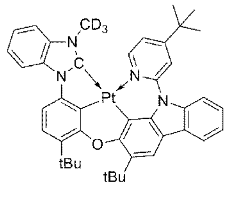

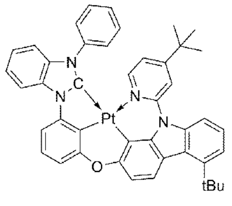
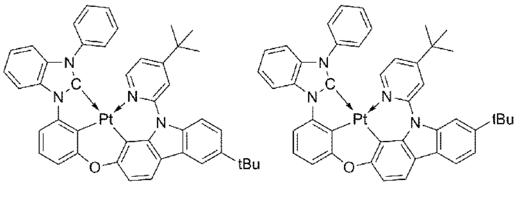
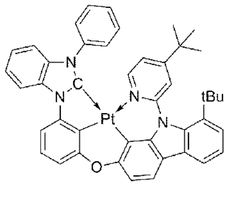
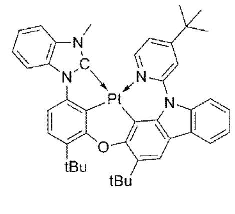

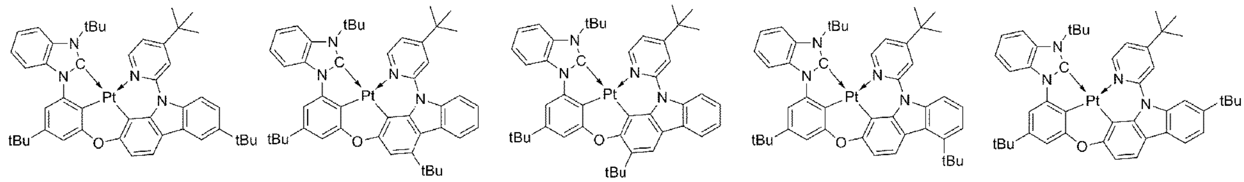

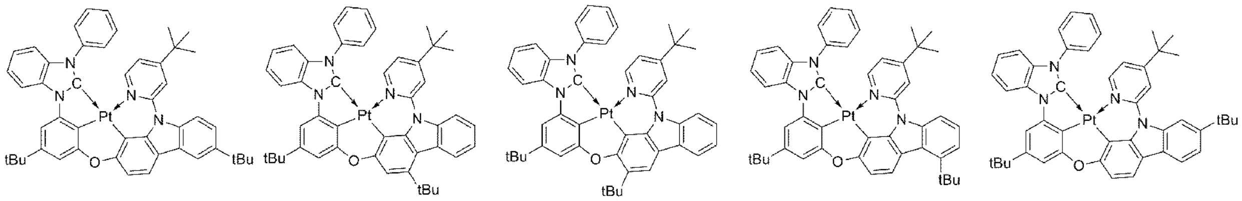

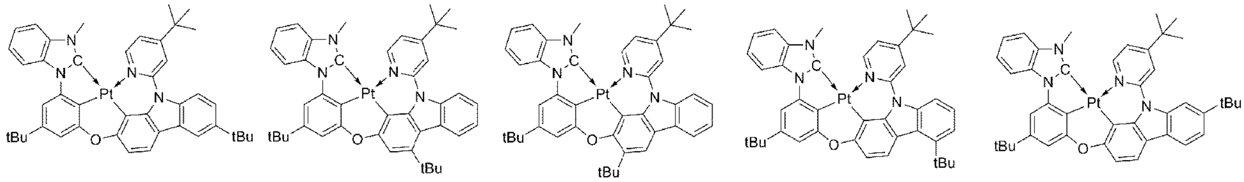

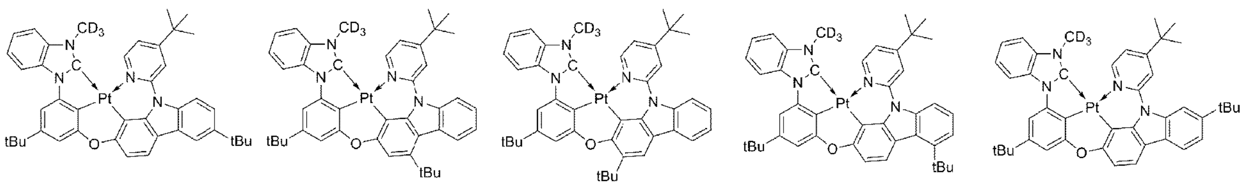

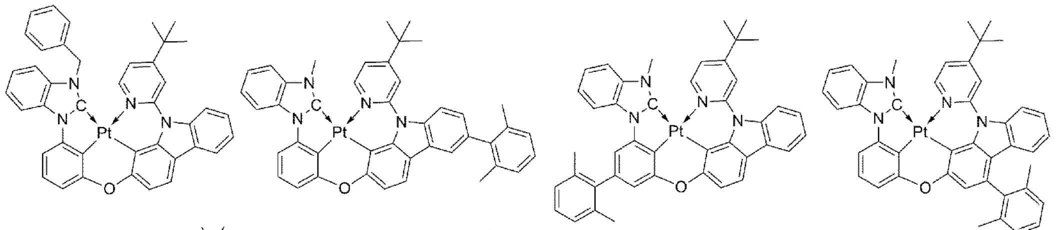

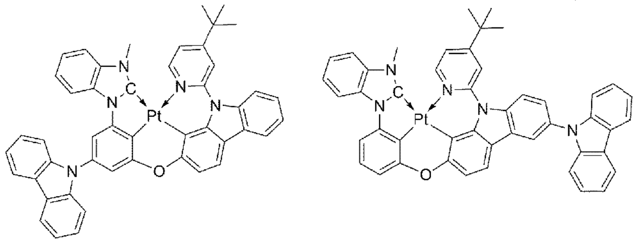

## Group IV

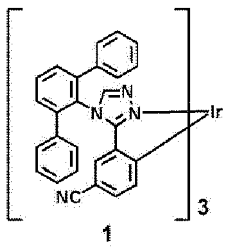
1

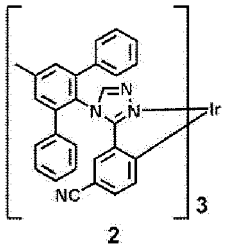
2

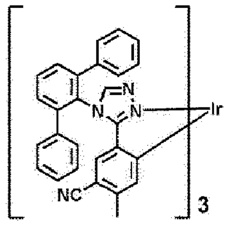
3

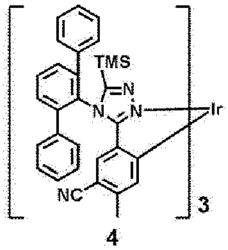
4

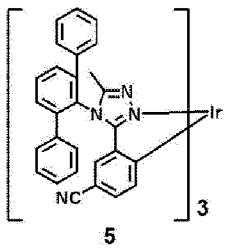
5

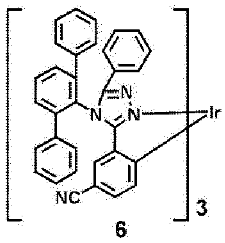
6

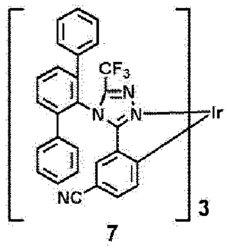
7

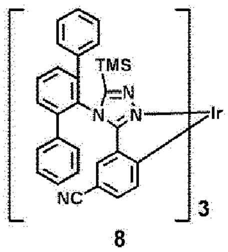
8

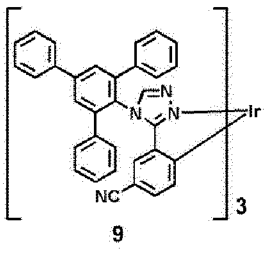
9

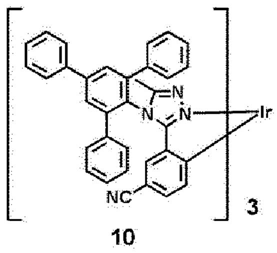
10

Group V
a compound represented by Formula A below:

Formula A $(L_{101})_{n101}\text{-}M_{101}\text{-}(L_{102})_{m101}$

wherein,
$L_{101}$, n101, $M_{101}$, $L_{102}$, and m101 in Formula A are the same as described in connection with Tables 1 to 3:

Table 1

| Compound Name | $L_{101}$ | n101 | $M_{101}$ | $L_{102}$ | m101 | Compound Name | $L_{101}$ | n101 | $M_{101}$ | $L_{102}$ | m101 |
|---|---|---|---|---|---|---|---|---|---|---|---|
| BD001 | LM1 | 3 | Ir | - | 0 | BD051 | LM51 | 3 | Ir | - | 0 |
| BD002 | LM2 | 3 | Ir | - | 0 | BD052 | LM52 | 3 | Ir | - | 0 |
| BD003 | LM3 | 3 | Ir | - | 0 | BD053 | LM53 | 3 | Ir | - | 0 |
| BD004 | LM4 | 3 | Ir | - | 0 | BD054 | LM54 | 3 | Ir | - | 0 |
| BD005 | LM5 | 3 | Ir | - | 0 | BD055 | LM55 | 3 | Ir | - | 0 |
| BD006 | LM6 | 3 | Ir | - | 0 | BD056 | LM56 | 3 | Ir | - | 0 |
| BD007 | LM7 | 3 | Ir | - | 0 | BD057 | LM57 | 3 | Ir | - | 0 |
| BD008 | LM8 | 3 | Ir | - | 0 | BD058 | LM58 | 3 | Ir | - | 0 |
| BD009 | LM9 | 3 | Ir | - | 0 | BD059 | LM59 | 3 | Ir | - | 0 |
| BD010 | LM10 | 3 | Ir | - | 0 | BD060 | LM60 | 3 | Ir | - | 0 |
| BD011 | LM11 | 3 | Ir | - | 0 | BD061 | LM61 | 3 | Ir | - | 0 |
| BD012 | LM12 | 3 | Ir | - | 0 | BD062 | LM62 | 3 | Ir | - | 0 |

(continued)

| Compound Name | $L_{101}$ | n101 | $M_{101}$ | $L_{102}$ | m101 | Compound Name | $L_{101}$ | n101 | $M_{101}$ | $L_{102}$ | m101 |
|---|---|---|---|---|---|---|---|---|---|---|---|
| BD013 | LM13 | 3 | Ir | - | 0 | BD063 | LM63 | 3 | Ir | - | 0 |
| BD014 | LM14 | 3 | Ir | - | 0 | BD064 | LM64 | 3 | Ir | - | 0 |
| BD015 | LM15 | 3 | Ir | - | 0 | BD065 | LM65 | 3 | Ir | - | 0 |
| BD016 | LM16 | 3 | Ir | - | 0 | BD066 | LM66 | 3 | Ir | - | 0 |
| BD017 | LM17 | 3 | Ir | - | 0 | BD067 | LM67 | 3 | Ir | - | 0 |
| BD018 | LM18 | 3 | Ir | - | 0 | BD068 | LM68 | 3 | Ir | - | 0 |
| BD019 | LM19 | 3 | Ir | - | 0 | BD069 | LM69 | 3 | Ir | - | 0 |
| BD020 | LM20 | 3 | Ir | - | 0 | BD070 | LM70 | 3 | Ir | - | 0 |
| BD021 | LM21 | 3 | Ir | - | 0 | BD071 | LM71 | 3 | Ir | - | 0 |
| BD022 | LM22 | 3 | Ir | - | 0 | BD072 | LM72 | 3 | Ir | - | 0 |
| BD023 | LM23 | 3 | Ir | - | 0 | BD073 | LM73 | 3 | Ir | - | 0 |
| BD024 | LM24 | 3 | Ir | - | 0 | BD074 | LM74 | 3 | Ir | - | 0 |
| BD025 | LM25 | 3 | Ir | - | 0 | BD075 | LM75 | 3 | Ir | - | 0 |
| BD026 | LM26 | 3 | Ir | - | 0 | BD076 | LM76 | 3 | Ir | - | 0 |
| BD027 | LM27 | 3 | Ir | - | 0 | BD077 | LM77 | 3 | Ir | - | 0 |
| BD028 | LM28 | 3 | Ir | - | 0 | BD078 | LM78 | 3 | Ir | - | 0 |
| BD029 | LM29 | 3 | Ir | - | 0 | BD079 | LM79 | 3 | Ir | - | 0 |
| BD030 | LM30 | 3 | Ir | - | 0 | BD080 | LM80 | 3 | Ir | - | 0 |
| BD031 | LM31 | 3 | Ir | - | 0 | BD081 | LM81 | 3 | Ir | - | 0 |
| BD032 | LM32 | 3 | Ir | - | 0 | BD082 | LM82 | 3 | Ir | - | 0 |
| BD033 | LM33 | 3 | Ir | - | 0 | BD083 | LM83 | 3 | Ir | - | 0 |
| BD034 | LM34 | 3 | Ir | - | 0 | BD084 | LM84 | 3 | Ir | - | 0 |
| BD035 | LM35 | 3 | Ir | - | 0 | BD085 | LM85 | 3 | Ir | - | 0 |
| BD036 | LM36 | 3 | Ir | - | 0 | BD086 | LM86 | 3 | Ir | - | 0 |
| BD037 | LM37 | 3 | Ir | - | 0 | BD087 | LM87 | 3 | Ir | - | 0 |
| BD038 | LM38 | 3 | Ir | - | 0 | BD088 | LM88 | 3 | Ir | - | 0 |
| BD039 | LM39 | 3 | Ir | - | 0 | BD089 | LM89 | 3 | Ir | - | 0 |
| BD040 | LM40 | 3 | Ir | - | 0 | BD090 | LM90 | 3 | Ir | - | 0 |
| BD041 | LM41 | 3 | Ir | - | 0 | BD091 | LM91 | 3 | Ir | - | 0 |
| BD042 | LM42 | 3 | Ir | - | 0 | BD092 | LM92 | 3 | Ir | - | 0 |
| BD043 | LM43 | 3 | Ir | - | 0 | BD093 | LM93 | 3 | Ir | - | 0 |
| BD044 | LM44 | 3 | Ir | - | 0 | BD094 | LM94 | 3 | Ir | - | 0 |
| BD045 | LM45 | 3 | Ir | - | 0 | BD095 | LM95 | 3 | Ir | - | 0 |
| BD046 | LM46 | 3 | Ir | - | 0 | BD096 | LM96 | 3 | Ir | - | 0 |
| BD047 | LM47 | 3 | Ir | - | 0 | BD097 | LM97 | 3 | Ir | - | 0 |
| BD048 | LM48 | 3 | Ir | - | 0 | BD098 | LM98 | 3 | Ir | - | 0 |
| BD049 | LM49 | 3 | Ir | - | 0 | BD099 | LM99 | 3 | Ir | - | 0 |
| BD050 | LM50 | 3 | Ir | - | 0 | BD100 | LM100 | 3 | Ir | - | 0 |

Table 2

| Compound Name | $L_{101}$ | n101 | $M_{101}$ | $L_{102}$ | m101 | Compound Name | $L_{101}$ | n101 | $M_{101}$ | $L_{102}$ | m101 |
|---|---|---|---|---|---|---|---|---|---|---|---|
| BD101 | LM101 | 3 | Ir | - | 0 | BD151 | LM151 | 3 | Ir | - | 0 |
| BD102 | LM102 | 3 | Ir | - | 0 | BD152 | LM152 | 3 | Ir | - | 0 |
| BD103 | LM103 | 3 | Ir | - | 0 | BD153 | LM153 | 3 | Ir | - | 0 |
| BD104 | LM104 | 3 | Ir | - | 0 | BD154 | LM154 | 3 | Ir | - | 0 |
| BD105 | LM105 | 3 | Ir | - | 0 | BD155 | LM155 | 3 | Ir | - | 0 |
| BD106 | LM106 | 3 | Ir | - | 0 | BD156 | LM156 | 3 | Ir | - | 0 |
| BD107 | LM107 | 3 | Ir | - | 0 | BD157 | LM157 | 3 | Ir | - | 0 |
| BD108 | LM108 | 3 | Ir | - | 0 | BD158 | LM158 | 3 | Ir | - | 0 |
| BD109 | LM109 | 3 | Ir | - | 0 | BD159 | LM159 | 3 | Ir | - | 0 |
| BD110 | LM110 | 3 | Ir | - | 0 | BD160 | LM160 | 3 | Ir | - | 0 |
| BD111 | LM111 | 3 | Ir | - | 0 | BD161 | LM161 | 3 | Ir | - | 0 |
| BD112 | LM112 | 3 | Ir | - | 0 | BD162 | LM162 | 3 | Ir | - | 0 |
| BD113 | LM113 | 3 | Ir | - | 0 | BD163 | LM163 | 3 | Ir | - | 0 |
| BD114 | LM114 | 3 | Ir | - | 0 | BD164 | LM164 | 3 | Ir | - | 0 |
| BD115 | LM115 | 3 | Ir | - | 0 | BD165 | LM165 | 3 | Ir | - | 0 |
| BD116 | LM116 | 3 | Ir | - | 0 | BD166 | LM166 | 3 | Ir | - | 0 |
| BD117 | LM117 | 3 | Ir | - | 0 | BD167 | LM167 | 3 | Ir | - | 0 |
| BD118 | LM118 | 3 | Ir | - | 0 | BD168 | LM168 | 3 | Ir | - | 0 |
| BD119 | LM119 | 3 | Ir | - | 0 | BD169 | LM169 | 3 | Ir | - | 0 |
| BD120 | LM120 | 3 | Ir | - | 0 | BD170 | LM170 | 3 | Ir | - | 0 |
| BD121 | LM121 | 3 | Ir | - | 0 | BD171 | LM171 | 3 | Ir | - | 0 |
| BD122 | LM122 | 3 | Ir | - | 0 | BD172 | LM172 | 3 | Ir | - | 0 |
| BD123 | LM123 | 3 | Ir | - | 0 | BD173 | LM173 | 3 | Ir | - | 0 |
| BD124 | LM124 | 3 | Ir | - | 0 | BD174 | LM174 | 3 | Ir | - | 0 |
| BD125 | LM125 | 3 | Ir | - | 0 | BD175 | LM175 | 3 | Ir | - | 0 |
| BD126 | LM126 | 3 | Ir | - | 0 | BD176 | LM176 | 3 | Ir | - | 0 |
| BD127 | LM127 | 3 | Ir | - | 0 | BD177 | LM177 | 3 | Ir | - | 0 |
| BD128 | LM128 | 3 | Ir | - | 0 | BD178 | LM178 | 3 | Ir | - | 0 |
| BD129 | LM129 | 3 | Ir | - | 0 | BD179 | LM179 | 3 | Ir | - | 0 |
| BD130 | LM130 | 3 | Ir | - | 0 | BD180 | LM180 | 3 | Ir | - | 0 |
| BD131 | LM131 | 3 | Ir | - | 0 | BD181 | LM181 | 3 | Ir | - | 0 |
| BD132 | LM132 | 3 | Ir | - | 0 | BD182 | LM182 | 3 | Ir | - | 0 |
| BD133 | LM133 | 3 | Ir | - | 0 | BD183 | LM183 | 3 | Ir | - | 0 |
| BD134 | LM134 | 3 | Ir | - | 0 | BD184 | LM184 | 3 | Ir | - | 0 |
| BD135 | LM135 | 3 | Ir | - | 0 | BD185 | LM185 | 3 | Ir | - | 0 |
| BD136 | LM136 | 3 | Ir | - | 0 | BD186 | LM186 | 3 | Ir | - | 0 |
| BD137 | LM137 | 3 | Ir | - | 0 | BD187 | LM187 | 3 | Ir | - | 0 |

(continued)

| Compound Name | $L_{101}$ | n101 | $M_{101}$ | $L_{102}$ | m101 | Compound Name | $L_{101}$ | n101 | $M_{101}$ | $L_{102}$ | m101 |
|---|---|---|---|---|---|---|---|---|---|---|---|
| BD138 | LM138 | 3 | Ir | - | 0 | BD188 | LM188 | 3 | Ir | - | 0 |
| BD139 | LM139 | 3 | Ir | - | 0 | BD189 | LM189 | 3 | Ir | - | 0 |
| BD140 | LM140 | 3 | Ir | - | 0 | BD190 | LM190 | 3 | Ir | - | 0 |
| BD141 | LM141 | 3 | Ir | - | 0 | BD191 | LM191 | 3 | Ir | - | 0 |
| BD142 | LM142 | 3 | Ir | - | 0 | BD192 | LM192 | 3 | Ir | - | 0 |
| BD143 | LM143 | 3 | Ir | - | 0 | BD193 | LM193 | 3 | Ir | - | 0 |
| BD144 | LM144 | 3 | Ir | - | 0 | BD194 | LM194 | 3 | Ir | - | 0 |
| BD145 | LM145 | 3 | Ir | - | 0 | BD195 | LM195 | 3 | Ir | - | 0 |
| BD146 | LM146 | 3 | Ir | - | 0 | BD196 | LM196 | 3 | Ir | - | 0 |
| BD147 | LM147 | 3 | Ir | - | 0 | BD197 | LM197 | 3 | Ir | - | 0 |
| BD148 | LM148 | 3 | Ir | - | 0 | BD198 | LM198 | 3 | Ir | - | 0 |
| BD149 | LM149 | 3 | Ir | - | 0 | BD199 | LM199 | 3 | Ir | - | 0 |
| BD150 | LM150 | 3 | Ir | - | 0 | BD200 | LM200 | 3 | Ir | - | 0 |

Table 3

| Compound Name | $L_{101}$ | n101 | $M_{101}$ | $L_{102}$ | m101 | Compound Name | $L_{101}$ | n101 | $M_{101}$ | $L_{102}$ | m101 |
|---|---|---|---|---|---|---|---|---|---|---|---|
| BD201 | LM201 | 3 | Ir | - | 0 | BD251 | LFP1 | 3 | Ir | - | 0 |
| BD202 | LM202 | 3 | Ir | - | 0 | BD252 | LFP2 | 3 | Ir | - | 0 |
| BD203 | LM203 | 3 | Ir | - | 0 | BD253 | LFP3 | 3 | Ir | - | 0 |
| BD204 | LM204 | 3 | Ir | - | 0 | BD254 | LFP4 | 3 | Ir | - | 0 |
| BD205 | LM205 | 3 | Ir | - | 0 | BD255 | LFP5 | 3 | Ir | - | 0 |
| BD206 | LM206 | 3 | Ir | - | 0 | BD256 | LFP6 | 3 | Ir | - | 0 |
| BD207 | LM207 | 3 | Ir | - | 0 | BD257 | LFP7 | 3 | Ir | - | 0 |
| BD208 | LM208 | 3 | Ir | - | 0 | BD258 | LM47 | 2 | Ir | AN1 | 1 |
| BD209 | LM209 | 3 | Ir | - | 0 | BD259 | LM47 | 2 | Ir | AN2 | 1 |
| BD210 | LM210 | 3 | Ir | - | 0 | BD260 | LM47 | 2 | Ir | AN3 | 1 |
| BD211 | LM211 | 3 | Ir | - | 0 | BD261 | LM47 | 2 | Ir | AN4 | 1 |
| BD212 | LM212 | 3 | Ir | - | 0 | BD262 | LM47 | 2 | Ir | AN5 | 1 |
| BD213 | LM213 | 3 | Ir | - | 0 | BD263 | LM11 | 2 | Pt | - | 0 |
| BD214 | LM214 | 3 | Ir | - | 0 | BD264 | LM13 | 2 | Pt | - | 0 |
| BD215 | LM215 | 3 | Ir | - | 0 | BD265 | LM15 | 2 | Pt | - | 0 |
| BD216 | LM216 | 3 | Ir | - | 0 | BD266 | LM45 | 2 | Pt | - | 0 |
| BD217 | LM217 | 3 | Ir | - | 0 | BD267 | LM47 | 2 | Pt | - | 0 |
| BD218 | LM218 | 3 | Ir | - | 0 | BD268 | LM49 | 2 | Pt | - | 0 |
| BD219 | LM219 | 3 | Ir | - | 0 | BD269 | LM98 | 2 | Pt | - | 0 |
| BD220 | LM220 | 3 | Ir | - | 0 | BD270 | LM100 | 2 | Pt | - | 0 |
| BD221 | LM221 | 3 | Ir | - | 0 | BD271 | LM102 | 2 | Pt | - | 0 |

(continued)

| Compound Name | $L_{101}$ | n101 | $M_{101}$ | $L_{102}$ | m101 | Compound Name | $L_{101}$ | n101 | $M_{101}$ | $L_{102}$ | m101 |
|---|---|---|---|---|---|---|---|---|---|---|---|
| BD222 | LM222 | 3 | Ir | - | 0 | BD272 | LM132 | 2 | Pt | - | 0 |
| BD223 | LM223 | 3 | Ir | - | 0 | BD273 | LM134 | 2 | Pt | - | 0 |
| BD224 | LM224 | 3 | Ir | - | 0 | BD274 | LM136 | 2 | Pt | - | 0 |
| BD225 | LM225 | 3 | Ir | - | 0 | BD275 | LM151 | 2 | Pt | - | 0 |
| BD226 | LM226 | 3 | Ir | - | 0 | BD276 | LM153 | 2 | Pt | - | 0 |
| BD227 | LM227 | 3 | Ir | - | 0 | BD277 | LM158 | 2 | Pt | - | 0 |
| BD228 | LM228 | 3 | Ir | - | 0 | BD278 | LM180 | 2 | Pt | - | 0 |
| BD229 | LM229 | 3 | Ir | - | 0 | BD279 | LM182 | 2 | Pt | - | 0 |
| BD230 | LM230 | 3 | Ir | - | 0 | BD280 | LM187 | 2 | Pt | - | 0 |
| BD231 | LM231 | 3 | Ir | - | 0 | BD281 | LM201 | 2 | Pt | - | 0 |
| BD232 | LM232 | 3 | Ir | - | 0 | BD282 | LM206 | 2 | Pt | - | 0 |
| BD233 | LM233 | 3 | Ir | - | 0 | BD283 | LM211 | 2 | Pt | - | 0 |
| BD234 | LM234 | 3 | Ir | - | 0 | BD284 | LM233 | 2 | Pt | - | 0 |
| BD235 | LM235 | 3 | Ir | - | 0 | BD285 | LM235 | 2 | Pt | - | 0 |
| BD236 | LM236 | 3 | Ir | - | 0 | BD286 | LM240 | 2 | Pt | - | 0 |
| BD237 | LM237 | 3 | Ir | - | 0 | BD287 | LFM5 | 2 | Pt | - | 0 |
| BD238 | LM238 | 3 | Ir | - | 0 | BD288 | LFM6 | 2 | Pt | - | 0 |
| BD239 | LM239 | 3 | Ir | - | 0 | BD289 | LFM7 | 2 | Pt | - | 0 |
| BD240 | LM240 | 3 | Ir | - | 0 | BD290 | LFP5 | 2 | Pt | - | 0 |
| BD241 | LM241 | 3 | Ir | - | 0 | BD291 | LFP6 | 2 | Pt | - | 0 |
| BD242 | LM242 | 3 | Ir | - | 0 | BD292 | LFP7 | 2 | Pt | - | 0 |
| BD243 | LM243 | 3 | Ir | - | 0 | BD293 | LM47 | 1 | Pt | AN1 | 1 |
| BD244 | LFM1 | 3 | Ir | - | 0 | BD294 | LM47 | 1 | Pt | AN2 | 1 |
| BD245 | LFM2 | 3 | Ir | - | 0 | BD295 | LM47 | 1 | Pt | AN3 | 1 |
| BD246 | LFM3 | 3 | Ir | - | 0 | BD296 | LM47 | 1 | Pt | AN4 | 1 |
| BD247 | LFM4 | 3 | Ir | - | 0 | BD297 | LM47 | 1 | Pt | AN5 | 1 |
| BD248 | LFM5 | 3 | Ir | - | 0 | | | | | | |
| BD249 | LFM6 | 3 | Ir | - | 0 | | | | | | |
| BD250 | LFM7 | 3 | Ir | - | 0 | | | | | | |

LM1 to LM243, LFM1 to LFM7, and LFP1 to LFP7 in Tables 1 to 3 may be understood by referring to Formulae 1-1 to 1-3 and Tables 4 to 6:

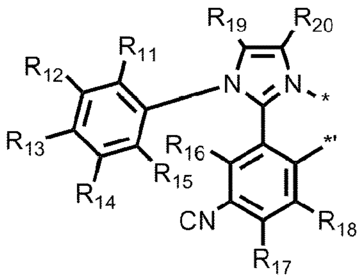

1-1

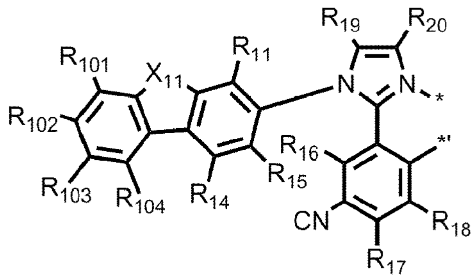

1-2

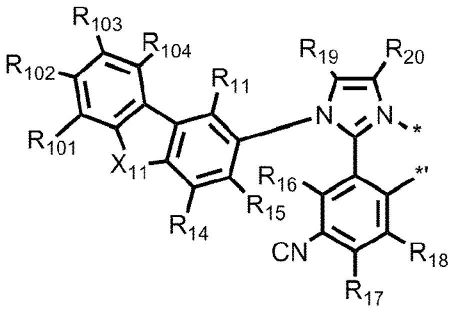

1-3

Table 4

| Formula 1-1 | | | | | | | | | | |
|---|---|---|---|---|---|---|---|---|---|---|
| Ligand Name | $R_{11}$ | $R_{12}$ | $R_{13}$ | $R_{14}$ | $R_{15}$ | $R_{16}$ | $R_{17}$ | $R_{18}$ | $R_{19}$ | $R_{20}$ |
| LM1 | X1 | H | X3 | H | X1 | H | H | H | H | D |
| LM2 | X1 | H | X3 | H | X1 | H | H | H | D | H |
| LM3 | X1 | H | X3 | H | X1 | H | H | H | D | D |
| LM4 | Y1 | H | X3 | H | Y1 | H | H | H | D | D |
| LM5 | Y2 | H | X3 | H | Y2 | H | H | H | D | D |
| LM6 | Y3 | H | X3 | H | Y3 | H | H | H | D | D |
| LM7 | Y3 | D | X3 | D | Y3 | H | H | H | D | D |
| LM8 | Y3 | D | X3 | D | Y3 | D | H | H | D | D |
| LM9 | Y3 | D | X3 | D | Y3 | D | D | H | D | D |
| LM10 | Y3 | D | X3 | D | Y3 | D | D | D | D | D |
| LM11 | Y3 | D | Y11 | D | Y3 | D | D | D | D | D |
| LM12 | Y3 | D | Y11 | D | Y3 | H | X1 | H | D | D |
| LM13 | Y3 | D | Y11 | D | Y3 | D | Y3 | D | D | D |
| LM14 | Y3 | D | Y11 | D | Y3 | H | X4 | H | D | D |
| LM15 | Y3 | D | Y11 | D | Y3 | D | Y12 | D | D | D |
| LM16 | X2 | H | X3 | H | X2 | H | H | H | H | D |
| LM17 | X2 | H | X3 | H | X2 | H | H | H | D | H |
| LM18 | X2 | H | X3 | H | X2 | H | H | H | D | D |

(continued)

| Formula 1-1 | | | | | | | | | | |
|---|---|---|---|---|---|---|---|---|---|---|
| Ligand Name | $R_{11}$ | $R_{12}$ | $R_{13}$ | $R_{14}$ | $R_{15}$ | $R_{16}$ | $R_{17}$ | $R_{18}$ | $R_{19}$ | $R_{20}$ |
| LM19 | Y4 | H | X3 | H | Y4 | H | H | H | D | D |
| LM20 | Y5 | H | X3 | H | Y5 | H | H | H | D | D |
| LM21 | Y6 | H | X3 | H | Y6 | H | H | H | D | D |
| LM22 | Y7 | H | X3 | H | Y7 | H | H | H | D | D |
| LM23 | Y8 | H | X3 | H | Y8 | H | H | H | D | D |
| LM24 | Y9 | H | X3 | H | Y9 | H | H | H | D | D |
| LM25 | Y10 | H | X3 | H | Y10 | H | H | H | D | D |
| LM26 | Y10 | D | X3 | D | Y10 | H | H | H | D | D |
| LM27 | Y10 | D | X3 | D | Y10 | D | H | H | D | D |
| LM28 | Y10 | D | X3 | D | Y10 | D | D | H | D | D |
| LM29 | Y10 | D | X3 | D | Y10 | D | D | D | D | D |
| LM30 | Y10 | D | Y11 | D | Y10 | D | D | D | D | D |
| LM31 | Y10 | D | Y11 | D | Y10 | H | X1 | H | D | D |
| LM32 | Y10 | D | Y11 | D | Y10 | D | Y3 | D | D | D |
| LM33 | Y10 | D | Y11 | D | Y10 | H | X4 | H | D | D |
| LM34 | Y10 | D | Y11 | D | Y10 | D | Y12 | D | D | D |
| LM35 | X1 | H | X4 | H | X1 | H | H | H | H | D |
| LM36 | X1 | H | X4 | H | X1 | H | H | H | D | H |
| LM37 | X1 | H | X4 | H | X1 | H | H | H | D | D |
| LM38 | Y1 | H | X4 | H | Y1 | H | H | H | D | D |
| LM39 | Y2 | H | X4 | H | Y2 | H | H | H | D | D |
| LM40 | Y3 | H | X4 | H | Y3 | H | H | H | D | D |
| LM41 | Y3 | D | X4 | D | Y3 | H | H | H | D | D |
| LM42 | Y3 | D | X4 | D | Y3 | D | H | H | D | D |
| LM43 | Y3 | D | X4 | D | Y3 | D | D | H | D | D |
| LM44 | Y3 | D | X4 | D | Y3 | D | D | D | D | D |
| LM45 | Y3 | D | Y12 | D | Y3 | D | D | D | D | D |
| LM46 | Y3 | D | Y12 | D | Y3 | H | X1 | H | D | D |
| LM47 | Y3 | D | Y12 | D | Y3 | D | Y3 | D | D | D |
| LM48 | Y3 | D | Y12 | D | Y3 | H | X4 | H | D | D |
| LM49 | Y3 | D | Y12 | D | Y3 | D | Y12 | D | D | D |
| LM50 | X2 | H | X4 | H | X2 | H | H | H | H | D |
| LM51 | X2 | H | X4 | H | X2 | H | H | H | D | H |
| LM52 | X2 | H | X4 | H | X2 | H | H | H | D | D |
| LM53 | Y4 | H | X4 | H | Y4 | H | H | H | D | D |
| LM54 | Y5 | H | X4 | H | Y5 | H | H | H | D | D |
| LM55 | Y6 | H | X4 | H | Y6 | H | H | H | D | D |

(continued)

| Formula 1-1 | | | | | | | | | | |
|---|---|---|---|---|---|---|---|---|---|---|
| Ligand Name | $R_{11}$ | $R_{12}$ | $R_{13}$ | $R_{14}$ | $R_{15}$ | $R_{16}$ | $R_{17}$ | $R_{18}$ | $R_{19}$ | $R_{20}$ |
| LM56 | Y7 | H | X4 | H | Y7 | H | H | H | D | D |
| LM57 | Y8 | H | X4 | H | Y8 | H | H | H | D | D |
| LM58 | Y9 | H | X4 | H | Y9 | H | H | H | D | D |
| LM59 | Y10 | H | X4 | H | Y10 | H | H | H | D | D |
| LM60 | Y10 | D | X4 | D | Y10 | H | H | H | D | D |
| LM61 | Y10 | D | X4 | D | Y10 | D | H | H | D | D |
| LM62 | Y10 | D | X4 | D | Y10 | D | D | H | D | D |
| LM63 | Y10 | D | X4 | D | Y10 | D | D | D | D | D |
| LM64 | Y10 | D | Y12 | D | Y10 | D | D | D | D | D |
| LM65 | Y10 | D | Y12 | D | Y10 | H | X1 | H | D | D |
| LM66 | Y10 | D | Y12 | D | Y10 | D | Y3 | D | D | D |
| LM67 | Y10 | D | Y12 | D | Y10 | H | X4 | H | D | D |
| LM68 | Y10 | D | Y12 | D | Y10 | D | Y12 | D | D | D |
| LM69 | X1 | H | X5 | H | X1 | H | H | H | H | D |
| LM70 | X1 | H | X5 | H | X1 | H | H | H | D | H |
| LM71 | X1 | H | X5 | H | X1 | H | H | H | D | D |
| LM72 | Y1 | H | X5 | H | Y1 | H | H | H | D | D |
| LM73 | Y2 | H | X5 | H | Y2 | H | H | H | D | D |
| LM74 | Y3 | H | X5 | H | Y3 | H | H | H | D | D |
| LM75 | Y3 | D | X5 | D | Y3 | H | H | H | D | D |
| LM76 | Y3 | D | X5 | D | Y3 | D | H | H | D | D |
| LM77 | Y3 | D | X5 | D | Y3 | D | D | H | D | D |
| LM78 | Y3 | D | X5 | D | Y3 | D | D | D | D | D |
| LM79 | Y3 | D | Y13 | D | Y3 | D | D | D | D | D |
| LM80 | Y3 | D | Y13 | D | Y3 | H | X1 | H | D | D |
| LM81 | Y3 | D | Y13 | D | Y3 | D | Y3 | D | D | D |
| LM82 | Y3 | D | Y13 | D | Y3 | H | X4 | H | D | D |
| LM83 | Y3 | D | Y13 | D | Y3 | D | Y12 | D | D | D |
| LM84 | X2 | H | X5 | H | X2 | H | H | H | H | D |
| LM85 | X2 | H | X5 | H | X2 | H | H | H | D | H |
| LM86 | X2 | H | X5 | H | X2 | H | H | H | D | D |
| LM87 | Y4 | H | X5 | H | Y4 | H | H | H | D | D |
| LM88 | Y5 | H | X5 | H | Y5 | H | H | H | D | D |
| LM89 | Y6 | H | X5 | H | Y6 | H | H | H | D | D |
| LM90 | Y7 | H | X5 | H | Y7 | H | H | H | D | D |
| LM91 | Y8 | H | X5 | H | Y8 | H | H | H | D | D |
| LM92 | Y9 | H | X5 | H | Y9 | H | H | H | D | D |

(continued)

| Formula 1-1 | | | | | | | | | | |
|---|---|---|---|---|---|---|---|---|---|---|
| Ligand Name | $R_{11}$ | $R_{12}$ | $R_{13}$ | $R_{14}$ | $R_{15}$ | $R_{16}$ | $R_{17}$ | $R_{18}$ | $R_{19}$ | $R_{20}$ |
| LM93 | Y10 | H | X5 | H | Y10 | H | H | H | D | D |
| LM94 | Y10 | D | X5 | D | Y10 | H | H | H | D | D |
| LM95 | Y10 | D | X5 | D | Y10 | D | H | H | D | D |
| LM96 | Y10 | D | X5 | D | Y10 | D | D | H | D | D |
| LM97 | Y10 | D | X5 | D | Y10 | D | D | D | D | D |
| LM98 | Y10 | D | Y13 | D | Y10 | D | D | D | D | D |
| LM99 | Y10 | D | Y13 | D | Y10 | H | X1 | H | D | D |
| LM100 | Y10 | D | Y13 | D | Y10 | D | Y3 | D | D | D |
| LM101 | Y10 | D | Y13 | D | Y10 | H | X4 | H | D | D |
| LM102 | Y10 | D | Y13 | D | Y10 | D | Y12 | D | D | D |
| LM103 | X1 | H | X6 | H | X1 | H | H | H | H | D |
| LM104 | X1 | H | X6 | H | X1 | H | H | H | D | H |
| LM105 | X1 | H | X6 | H | X1 | H | H | H | D | D |
| LM106 | Y1 | H | X6 | H | Y1 | H | H | H | D | D |
| LM107 | Y2 | H | X6 | H | Y2 | H | H | H | D | D |
| LM108 | Y3 | H | X6 | H | Y3 | H | H | H | D | D |
| LM109 | Y3 | D | X6 | D | Y3 | H | H | H | D | D |
| LM110 | Y3 | D | X6 | D | Y3 | D | H | H | D | D |
| LM111 | Y3 | D | X6 | D | Y3 | D | D | H | D | D |
| LM112 | Y3 | D | X6 | D | Y3 | D | D | D | D | D |
| LM113 | Y3 | D | Y14 | D | Y3 | D | D | D | D | D |
| LM114 | Y3 | D | Y14 | D | Y3 | H | X1 | H | D | D |
| LM115 | Y3 | D | Y14 | D | Y3 | D | Y3 | D | D | D |
| LM116 | Y3 | D | Y14 | D | Y3 | H | X4 | H | D | D |
| LM117 | Y3 | D | Y14 | D | Y3 | D | Y12 | D | D | D |
| LM118 | X2 | H | X6 | H | X2 | H | H | H | H | D |
| LM119 | X2 | H | X6 | H | X2 | H | H | H | D | H |
| LM120 | X2 | H | X6 | H | X2 | H | H | H | D | D |
| LM121 | Y4 | H | X6 | H | Y4 | H | H | H | D | D |
| LM122 | Y5 | H | X6 | H | Y5 | H | H | H | D | D |
| LM123 | Y6 | H | X6 | H | Y6 | H | H | H | D | D |
| LM124 | Y7 | H | X6 | H | Y7 | H | H | H | D | D |
| LM125 | Y8 | H | X6 | H | Y8 | H | H | H | D | D |
| LM126 | Y9 | H | X6 | H | Y9 | H | H | H | D | D |
| LM127 | Y10 | H | X6 | H | Y10 | H | H | H | D | D |
| LM128 | Y10 | D | X6 | D | Y10 | H | H | H | D | D |
| LM129 | Y10 | D | X6 | D | Y10 | D | H | H | D | D |

(continued)

| Formula 1-1 | | | | | | | | | | |
|---|---|---|---|---|---|---|---|---|---|---|
| Ligand Name | $R_{11}$ | $R_{12}$ | $R_{13}$ | $R_{14}$ | $R_{15}$ | $R_{16}$ | $R_{17}$ | $R_{18}$ | $R_{19}$ | $R_{20}$ |
| LM130 | Y10 | D | X6 | D | Y10 | D | D | H | D | D |
| LM131 | Y10 | D | X6 | D | Y10 | D | D | D | D | D |
| LM132 | Y10 | D | Y14 | D | Y10 | D | D | D | D | D |
| LM133 | Y10 | D | Y14 | D | Y10 | H | X1 | H | D | D |
| LM134 | Y10 | D | Y14 | D | Y10 | D | Y3 | D | D | D |
| LM135 | Y10 | D | Y14 | D | Y10 | H | X4 | H | D | D |
| LM136 | Y10 | D | Y14 | D | Y10 | D | Y12 | D | D | D |
| LM137 | X1 | H | X7 | H | X1 | H | H | H | H | D |
| LM138 | X1 | H | X7 | H | X1 | H | H | H | D | H |
| LM139 | X1 | H | X7 | H | X1 | H | H | H | D | D |
| LM140 | Y1 | H | X7 | H | Y1 | H | H | H | D | D |
| LM141 | Y2 | H | X7 | H | Y2 | H | H | H | D | D |
| LM142 | Y3 | H | X7 | H | Y3 | H | H | H | D | D |
| LM143 | Y3 | D | X7 | D | Y3 | H | H | H | D | D |
| LM144 | Y3 | D | X7 | D | Y3 | D | H | H | D | D |
| LM145 | Y3 | D | X7 | D | Y3 | D | D | H | D | D |
| LM146 | Y3 | D | X7 | D | Y3 | D | D | D | D | D |
| LM147 | Y3 | D | X8 | D | Y3 | D | D | D | D | D |
| LM148 | Y3 | D | Y16 | D | Y3 | D | D | D | D | D |
| LM149 | Y3 | D | Y17 | D | Y3 | D | D | D | D | D |
| LM150 | Y3 | D | Y18 | D | Y3 | D | D | D | D | D |
| LM151 | Y3 | D | Y15 | D | Y3 | D | D | D | D | D |
| LM152 | Y3 | D | Y15 | D | Y3 | H | X1 | H | D | D |
| LM153 | Y3 | D | Y15 | D | Y3 | D | Y3 | D | D | D |
| LM154 | Y3 | D | Y16 | D | Y3 | D | Y3 | D | D | D |
| LM155 | Y3 | D | Y17 | D | Y3 | D | Y3 | D | D | D |
| LM156 | Y3 | D | Y18 | D | Y3 | D | Y3 | D | D | D |
| LM157 | Y3 | D | Y15 | D | Y3 | H | X4 | H | D | D |
| LM158 | Y3 | D | Y15 | D | Y3 | D | Y12 | D | D | D |
| LM159 | Y3 | D | Y16 | D | Y3 | D | Y12 | D | D | D |
| LM160 | Y3 | D | Y17 | D | Y3 | D | Y12 | D | D | D |
| LM161 | Y3 | D | Y18 | D | Y3 | D | Y12 | D | D | D |
| LM162 | X2 | H | X7 | H | X2 | H | H | H | H | D |
| LM163 | X2 | H | X7 | H | X2 | H | H | H | D | H |
| LM164 | X2 | H | X7 | H | X2 | H | H | H | D | D |
| LM165 | Y4 | H | X7 | H | Y4 | H | H | H | D | D |
| LM166 | Y5 | H | X7 | H | Y5 | H | H | H | D | D |

(continued)

| Formula 1-1 | | | | | | | | | | |
|---|---|---|---|---|---|---|---|---|---|---|
| Ligand Name | $R_{11}$ | $R_{12}$ | $R_{13}$ | $R_{14}$ | $R_{15}$ | $R_{16}$ | $R_{17}$ | $R_{18}$ | $R_{19}$ | $R_{20}$ |
| LM167 | Y6 | H | X7 | H | Y6 | H | H | H | D | D |
| LM168 | Y7 | H | X7 | H | Y7 | H | H | H | D | D |
| LM169 | Y8 | H | X7 | H | Y8 | H | H | H | D | D |
| LM170 | Y9 | H | X7 | H | Y9 | H | H | H | D | D |
| LM171 | Y10 | H | X7 | H | Y10 | H | H | H | D | D |
| LM172 | Y10 | D | X7 | D | Y10 | H | H | H | D | D |
| LM173 | Y10 | D | X7 | D | Y10 | D | H | H | D | D |
| LM174 | Y10 | D | X7 | D | Y10 | D | D | H | D | D |
| LM175 | Y10 | D | X7 | D | Y10 | D | D | D | D | D |
| LM176 | Y10 | D | X8 | D | Y10 | D | D | D | D | D |
| LM177 | Y10 | D | Y16 | D | Y10 | D | D | D | D | D |
| LM178 | Y10 | D | Y17 | D | Y10 | D | D | D | D | D |
| LM179 | Y10 | D | Y18 | D | Y10 | D | D | D | D | D |
| LM180 | Y10 | D | Y15 | D | Y10 | D | D | D | D | D |
| LM181 | Y10 | D | Y15 | D | Y10 | H | X1 | H | D | D |
| LM182 | Y10 | D | Y15 | D | Y10 | D | Y3 | D | D | D |
| LM183 | Y10 | D | Y16 | D | Y10 | D | Y3 | D | D | D |
| LM184 | Y10 | D | Y17 | D | Y10 | D | Y3 | D | D | D |
| LM185 | Y10 | D | Y18 | D | Y10 | D | Y3 | D | D | D |
| LM186 | Y10 | D | Y15 | D | Y10 | H | X4 | H | D | D |
| LM187 | Y10 | D | Y15 | D | Y10 | D | Y12 | D | D | D |
| LM188 | Y10 | D | Y16 | D | Y10 | D | Y12 | D | D | D |
| LM189 | Y10 | D | Y17 | D | Y10 | D | Y12 | D | D | D |
| LM190 | Y10 | D | Y18 | D | Y10 | D | Y12 | D | D | D |
| LM191 | X1 | X7 | H | H | X1 | H | H | H | H | D |
| LM192 | X1 | X7 | H | H | X1 | H | H | H | D | H |
| LM193 | X1 | X7 | H | H | X1 | H | H | H | D | D |
| LM194 | Y1 | X7 | H | H | Y1 | H | H | H | D | D |
| LM195 | Y2 | X7 | H | H | Y2 | H | H | H | D | D |
| LM196 | Y3 | X7 | H | H | Y3 | H | H | H | D | D |
| LM197 | Y3 | X7 | D | D | Y3 | H | H | H | D | D |
| LM198 | Y3 | X7 | D | D | Y3 | D | H | H | D | D |
| LM199 | Y3 | X7 | D | D | Y3 | D | D | H | D | D |
| LM200 | Y3 | X7 | D | D | Y3 | D | D | D | D | D |
| LM201 | Y3 | Y15 | D | D | Y3 | D | D | D | D | D |
| LM202 | Y3 | Y16 | D | D | Y3 | D | D | D | D | D |
| LM203 | Y3 | Y17 | D | D | Y3 | D | D | D | D | D |

(continued)

| Formula 1-1 | | | | | | | | | | |
|---|---|---|---|---|---|---|---|---|---|---|
| Ligand Name | $R_{11}$ | $R_{12}$ | $R_{13}$ | $R_{14}$ | $R_{15}$ | $R_{16}$ | $R_{17}$ | $R_{18}$ | $R_{19}$ | $R_{20}$ |
| LM204 | Y3 | Y18 | D | D | Y3 | D | D | D | D | D |
| LM205 | Y3 | Y15 | D | D | Y3 | H | X1 | H | D | D |
| LM206 | Y3 | Y15 | D | D | Y3 | D | Y3 | D | D | D |
| LM207 | Y3 | Y16 | D | D | Y3 | D | Y3 | D | D | D |
| LM208 | Y3 | Y17 | D | D | Y3 | D | Y3 | D | D | D |
| LM209 | Y3 | Y18 | D | D | Y3 | D | Y3 | D | D | D |
| LM210 | Y3 | Y15 | D | D | Y3 | H | X4 | H | D | D |
| LM211 | Y3 | Y15 | D | D | Y3 | D | Y12 | D | D | D |
| LM212 | Y3 | Y16 | D | D | Y3 | D | Y12 | D | D | D |
| LM213 | Y3 | Y17 | D | D | Y3 | D | Y12 | D | D | D |
| LM214 | Y3 | Y18 | D | D | Y3 | D | Y12 | D | D | D |
| LM215 | X2 | X7 | H | H | X2 | H | H | H | H | D |
| LM216 | X2 | X7 | H | H | X2 | H | H | H | D | H |
| LM217 | X2 | X7 | H | H | X2 | H | H | H | D | D |
| LM218 | Y4 | X7 | H | H | Y4 | H | H | H | D | D |
| LM219 | Y5 | X7 | H | H | Y5 | H | H | H | D | D |
| LM220 | Y6 | X7 | H | H | Y6 | H | H | H | D | D |
| LM221 | Y7 | X7 | H | H | Y7 | H | H | H | D | D |
| LM222 | Y8 | X7 | H | H | Y8 | H | H | H | D | D |
| LM223 | Y9 | X7 | H | H | Y9 | H | H | H | D | D |
| LM224 | Y10 | X7 | H | H | Y10 | H | H | H | D | D |
| LM225 | Y10 | X7 | D | D | Y10 | H | H | H | D | D |
| LM226 | Y10 | X7 | D | D | Y10 | D | H | H | D | D |
| LM227 | Y10 | X7 | D | D | Y10 | D | D | H | D | D |
| LM228 | Y10 | X7 | D | D | Y10 | D | D | D | D | D |
| LM229 | Y10 | X8 | D | D | Y10 | D | D | D | D | D |
| LM230 | Y10 | Y16 | D | D | Y10 | D | D | D | D | D |
| LM231 | Y10 | Y17 | D | D | Y10 | D | D | D | D | D |
| LM232 | Y10 | Y18 | D | D | Y10 | D | D | D | D | D |
| LM233 | Y10 | Y15 | D | D | Y10 | D | D | D | D | D |
| LM234 | Y10 | Y15 | D | D | Y10 | H | X1 | H | D | D |
| LM235 | Y10 | Y15 | D | D | Y10 | D | Y3 | D | D | D |
| LM236 | Y10 | Y16 | D | D | Y10 | D | Y3 | D | D | D |
| LM237 | Y10 | Y17 | D | D | Y10 | D | Y3 | D | D | D |
| LM238 | Y10 | Y18 | D | D | Y10 | D | Y3 | D | D | D |
| LM239 | Y10 | Y15 | D | D | Y10 | H | X4 | H | D | D |
| LM240 | Y10 | Y15 | D | D | Y10 | D | Y12 | D | D | D |

(continued)

| Formula 1-1 | | | | | | | | | | |
|---|---|---|---|---|---|---|---|---|---|---|
| Ligand Name | $R_{11}$ | $R_{12}$ | $R_{13}$ | $R_{14}$ | $R_{15}$ | $R_{16}$ | $R_{17}$ | $R_{18}$ | $R_{19}$ | $R_{20}$ |
| LM241 | Y10 | Y16 | D | D | Y10 | D | Y12 | D | D | D |
| LM242 | Y10 | Y17 | D | D | Y10 | D | Y12 | D | D | D |
| LM243 | Y10 | Y18 | D | D | Y10 | D | Y12 | D | D | D |

Table 5

| Formula 1-2 | | | | | | | | | | | | |
|---|---|---|---|---|---|---|---|---|---|---|---|---|
| Ligand Name | $R_{11}$ | $X_{11}$ | $R_{101}$ | $R_{102}$ | $R_{103}$ | $R_{104}$ | $R_{14}$ | $R_{15}$ | $R_{16}$ | $R_{17}$ | $R_{18}$ | $R_{19}$ | $R_{20}$ |
| LFM1 | Y10 | N-Ph | D | D | D | D | D | Y10 | D | D | D | D | D |
| LFM2 | Y10 | S | D | D | D | D | D | Y10 | D | D | D | D | D |
| LFM3 | Y10 | O | D | D | D | D | D | Y10 | D | D | D | D | D |
| LFM4 | Y3 | O | D | D | D | D | D | Y3 | D | D | D | D | D |
| LFM5 | Y10 | O | D | D | D | D | D | Y10 | D | D | D | D | D |
| LFM6 | Y10 | O | D | D | D | D | D | Y10 | D | Y3 | D | D | D |
| LFM7 | Y10 | O | D | D | D | D | D | Y10 | D | Y12 | D | D | D |

Table 6

| Formula 1-3 | | | | | | | | | | | | |
|---|---|---|---|---|---|---|---|---|---|---|---|---|
| Ligand Name | $R_{11}$ | $X_{11}$ | $R_{101}$ | $R_{102}$ | $R_{103}$ | $R_{104}$ | $R_{14}$ | $R_{15}$ | $R_{16}$ | $R_{17}$ | $R_{18}$ | $R_{19}$ | $R_{20}$ |
| LFP1 | Y10 | N-Ph | D | D | D | D | D | Y10 | D | D | D | D | D |
| LFP2 | Y10 | S | D | D | D | D | D | Y10 | D | D | D | D | D |
| LFP3 | Y10 | O | D | D | D | D | D | Y10 | D | D | D | D | D |
| LFP4 | Y3 | O | D | D | D | D | D | Y3 | D | D | D | D | D |
| LFP5 | Y10 | O | D | D | D | D | D | Y10 | D | D | D | D | D |
| LFP6 | Y10 | O | D | D | D | D | D | Y10 | D | Y3 | D | D | D |
| LFP7 | Y10 | O | D | D | D | D | D | Y10 | D | Y12 | D | D | D |

X1 to X10 and Y1 to Y18 in Tables 4 to 6 are the same as described below, and Ph in the tables refers to a phenyl group:

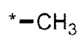
X1
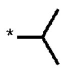
X2
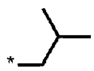
X3
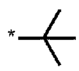
X4
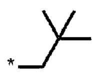
X5
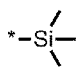
X6
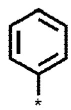
X7
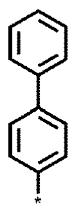
X8
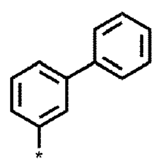
X9
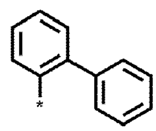
X10

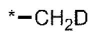
Y1
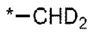
Y2
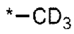
Y3
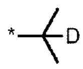
Y4
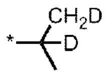
Y5
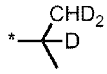
Y6
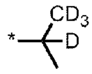
Y7
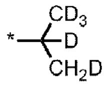
Y8
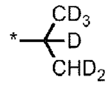
Y9

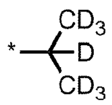
Y10
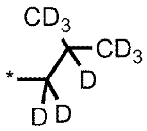
Y11
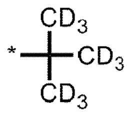
Y12
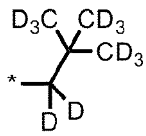
Y13
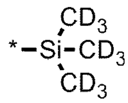
Y14
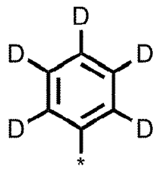
Y15
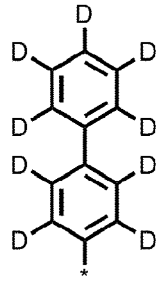
Y16

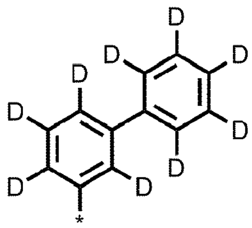
Y17
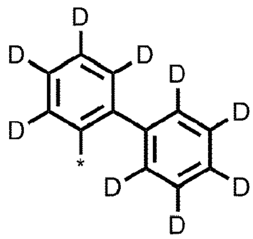
Y18

Group VI

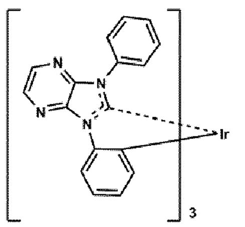
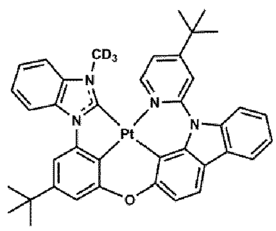
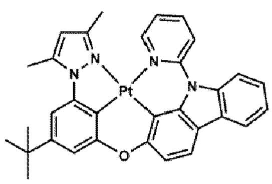
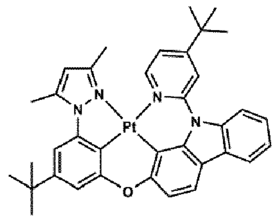
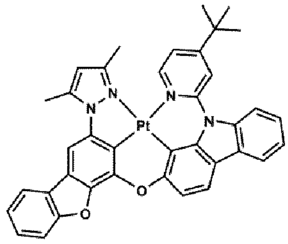
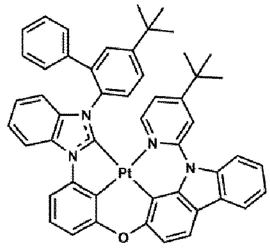
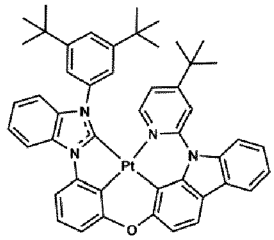

In one or more embodiments, the sensitizer may include the thermally activated delayed fluorescence emitter represented by Formula 201 or 202.

For example, $A_{211}$ in Formula 202 may be a substituted or unsubstituted $\pi$ electron-deficient nitrogen-free cyclic group.

In one or more embodiments, the $\pi$ electron-deficient nitrogen-free cyclic group may be a phenyl group, a heptalene group, an indene group, a naphthalene group, an azulene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, an isoindole group, an indole group, a

furan group, a thiophene group, a benzofuran group, a benzothiophene group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, a triindolobenzene group; or a condensed cyclic group of two or more π electron-deficient nitrogen-free cyclic groups, but embodiments of the present disclosure are not limited thereto.

For example, $D_{211}$ in Formula 202 may be: -F, a cyano group, or an π-electron deficient nitrogen-containing cyclic group;

a $C_1$-$C_{60}$ alkyl group, an π-electron deficient nitrogen-containing cyclic group, or an π electron-deficient nitrogen-free cyclic group, each substituted with at least one of -F or a cyano group; or

an π-electron deficient nitrogen-containing cyclic group, substituted with at least one deuterium, a $C_1$-$C_{60}$ alkyl group, an π -electron deficient nitrogen-containing cyclic group, or an π electron-deficient nitrogen-free cyclic group.

In one or more embodiments, the π electron-deficient nitrogen-free cyclic group is the same as described herein.

The term "π electron-deficient nitrogen-containing cyclic group" used herein refers to a cyclic group having at least one -N= moiety, and, for example, may be an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, an azacarbazole group, or a benzimidazolobenzimidazole group; or a condensed cyclic group in which two or more π electron-efficient nitrogen-containing cyclic groups are condensed with each other.

In one or more embodiments, the sensitizer may be one from Groups VII to XIII, but embodiments of the present disclosure are not limited thereto:

## Group VII

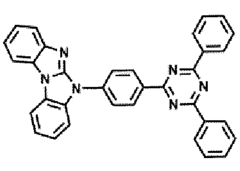
1

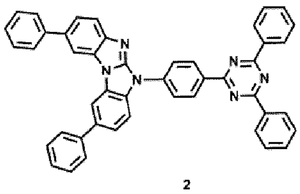
2

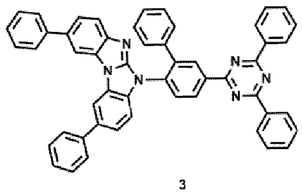
3

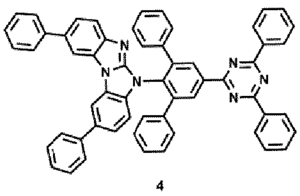
4

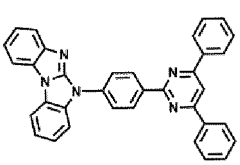
5

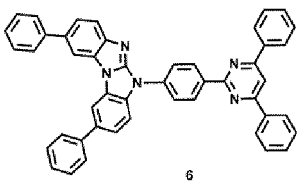
6

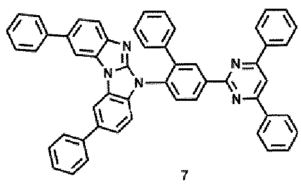
7

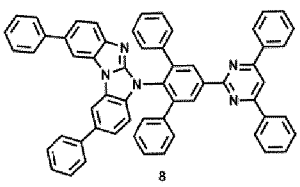
8

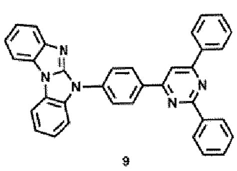
9

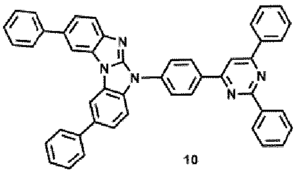
10

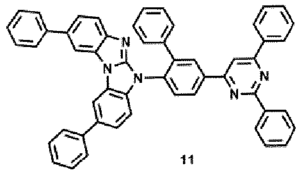
11

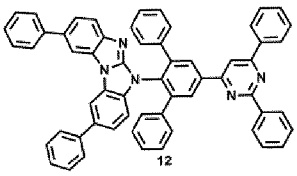
12

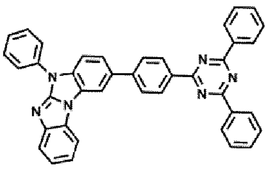
13

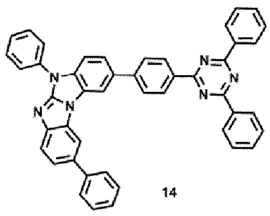
14

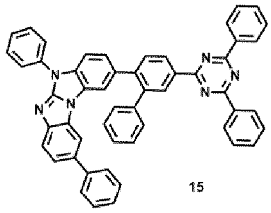
15

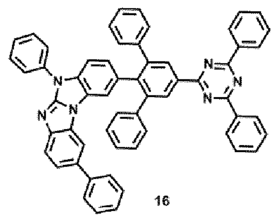
16

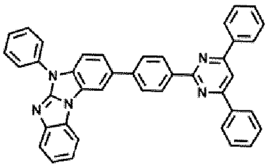
17

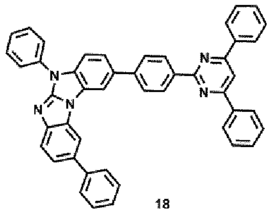
18

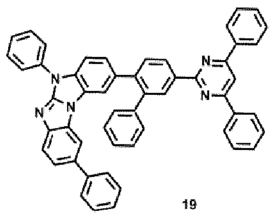
19

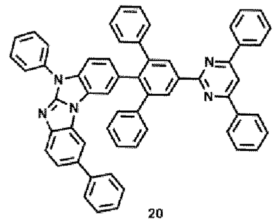
20

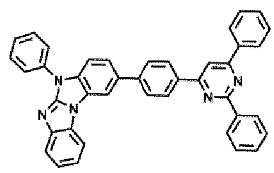
21

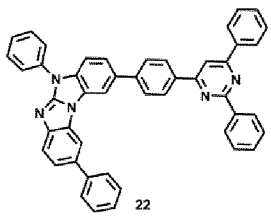
22

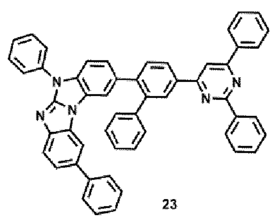
23

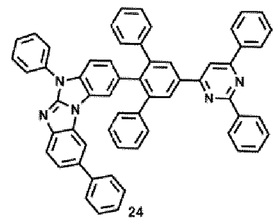
24

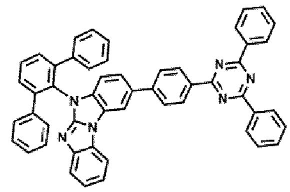
25
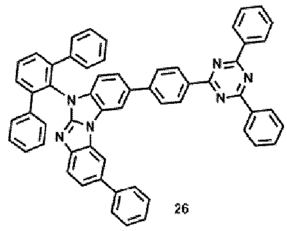
26
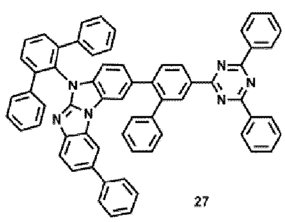
27
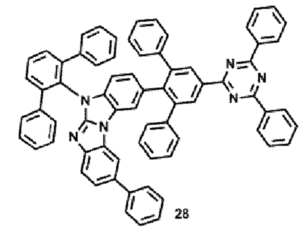
28

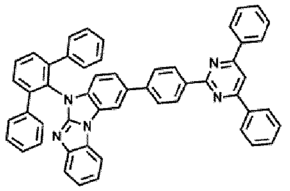
29
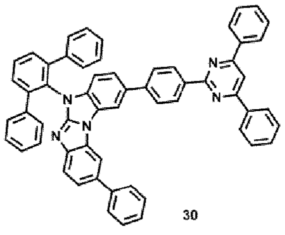
30
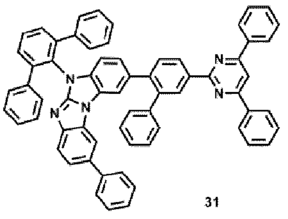
31
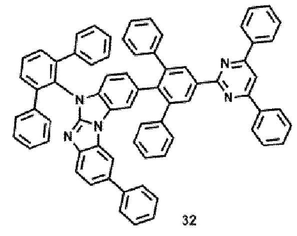
32

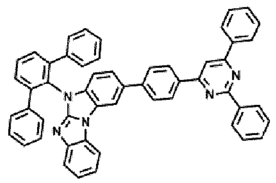
33
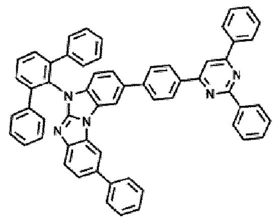
34
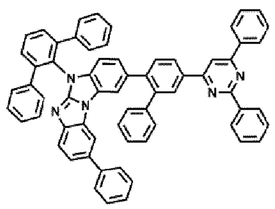
35
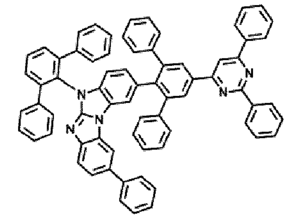
36

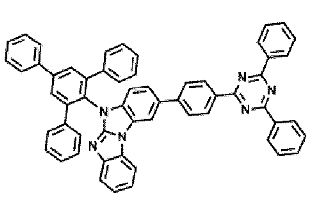
37
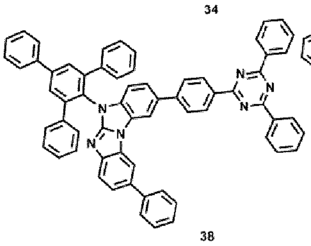
38
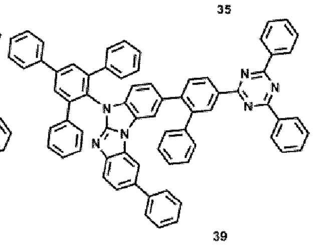
39
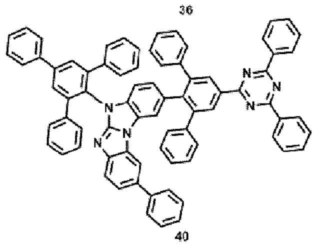
40

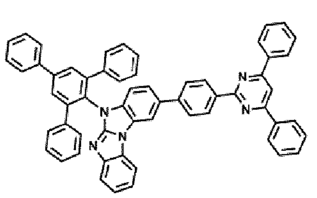
41
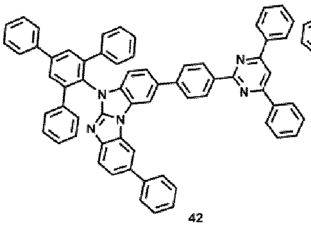
42
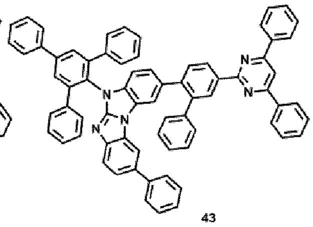
43
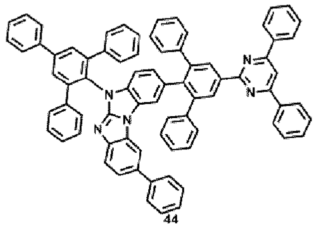
44

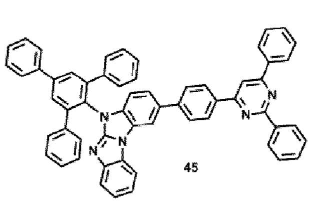
45
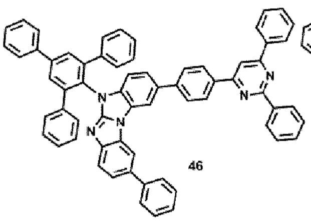
46
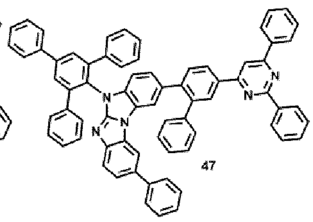
47
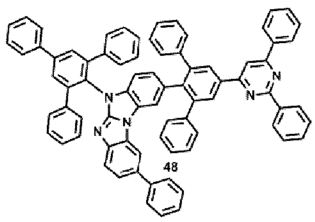
48

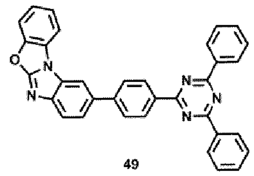
49
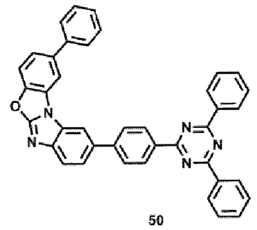
50
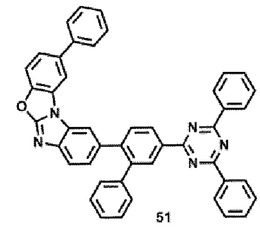
51
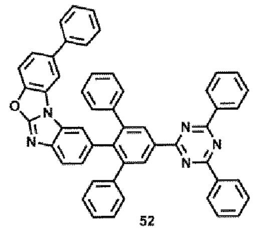
52

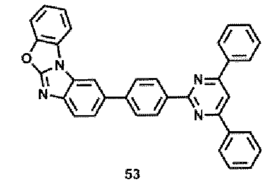
53
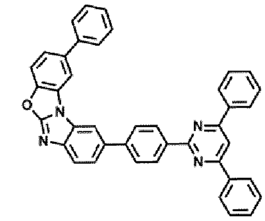
54
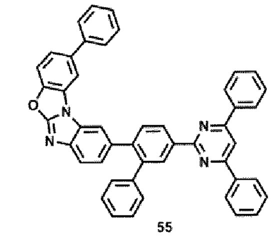
55
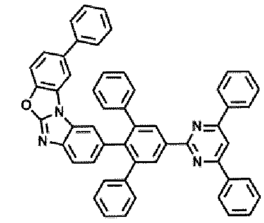
56

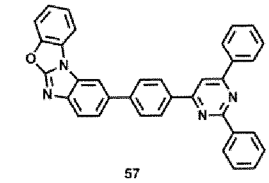
57
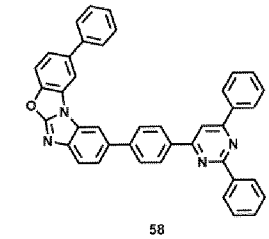
58
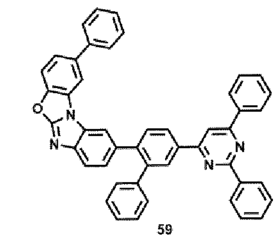
59
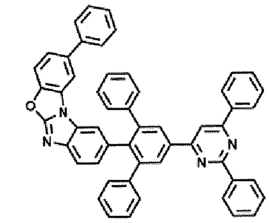
60

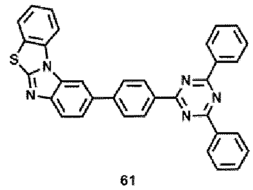
61
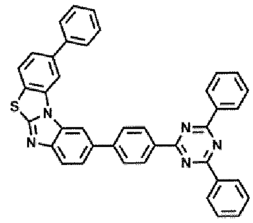
62
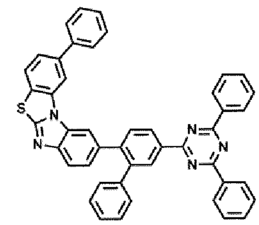
63
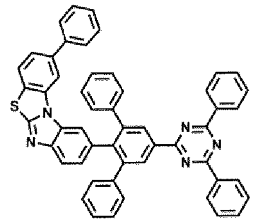
64

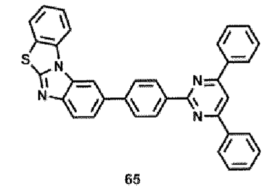
65
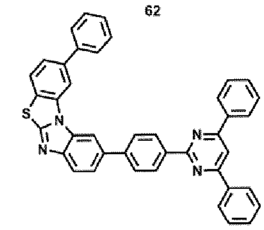
66
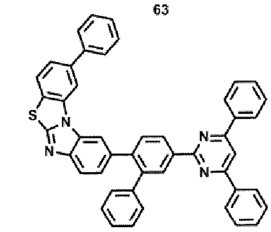
67
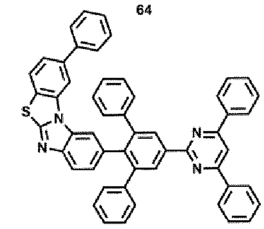
68

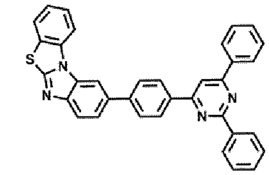
69
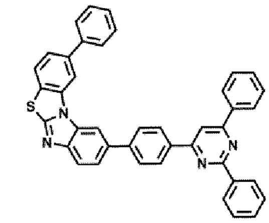
70
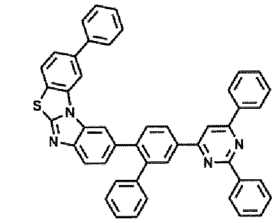
71
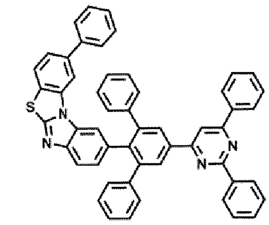
72

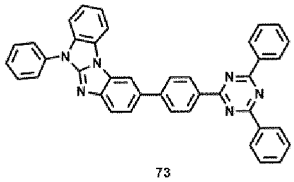
73
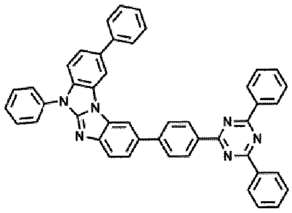
74
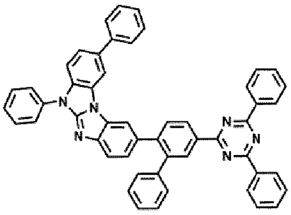
75
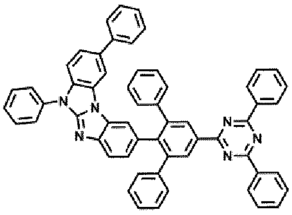
76
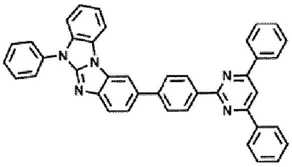
77
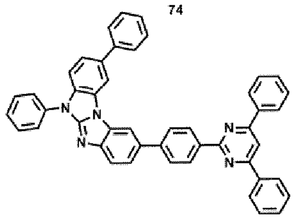
78
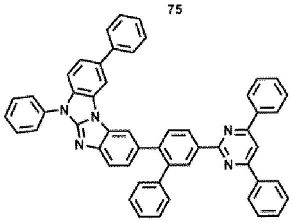
79
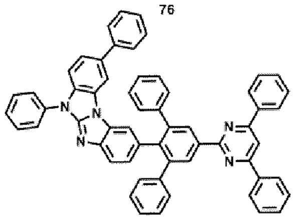
80
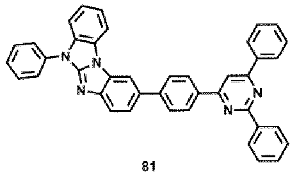
81
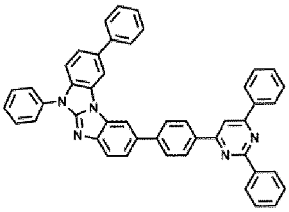
82
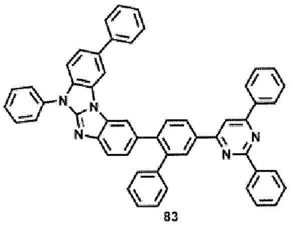
83
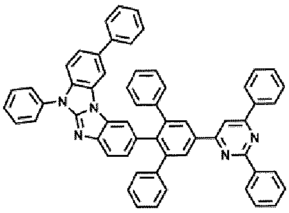
84
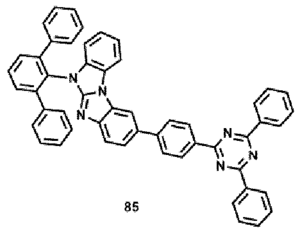
85
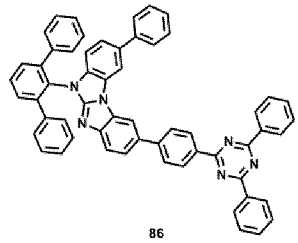
86
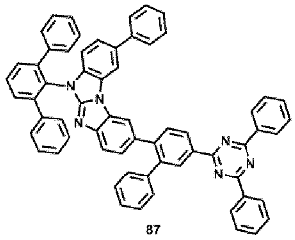
87
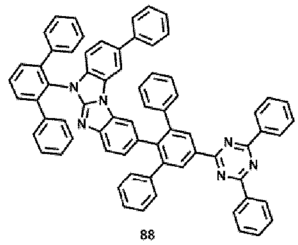
88
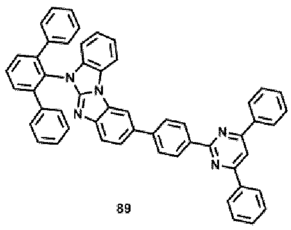
89
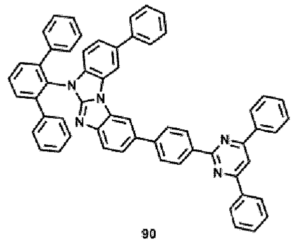
90
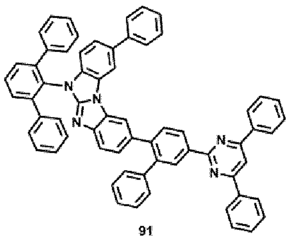
91
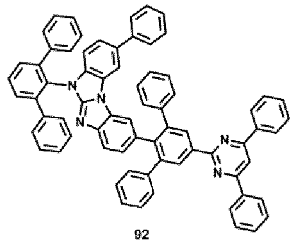
92
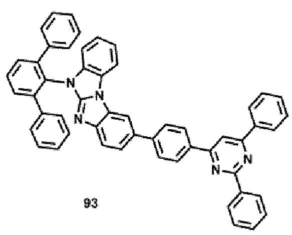
93
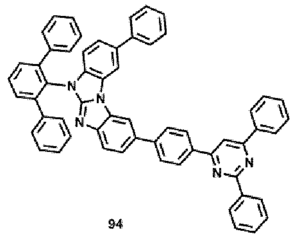
94
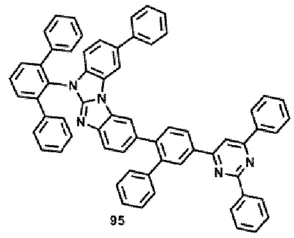
95
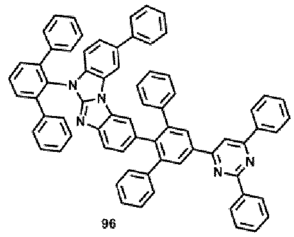
96

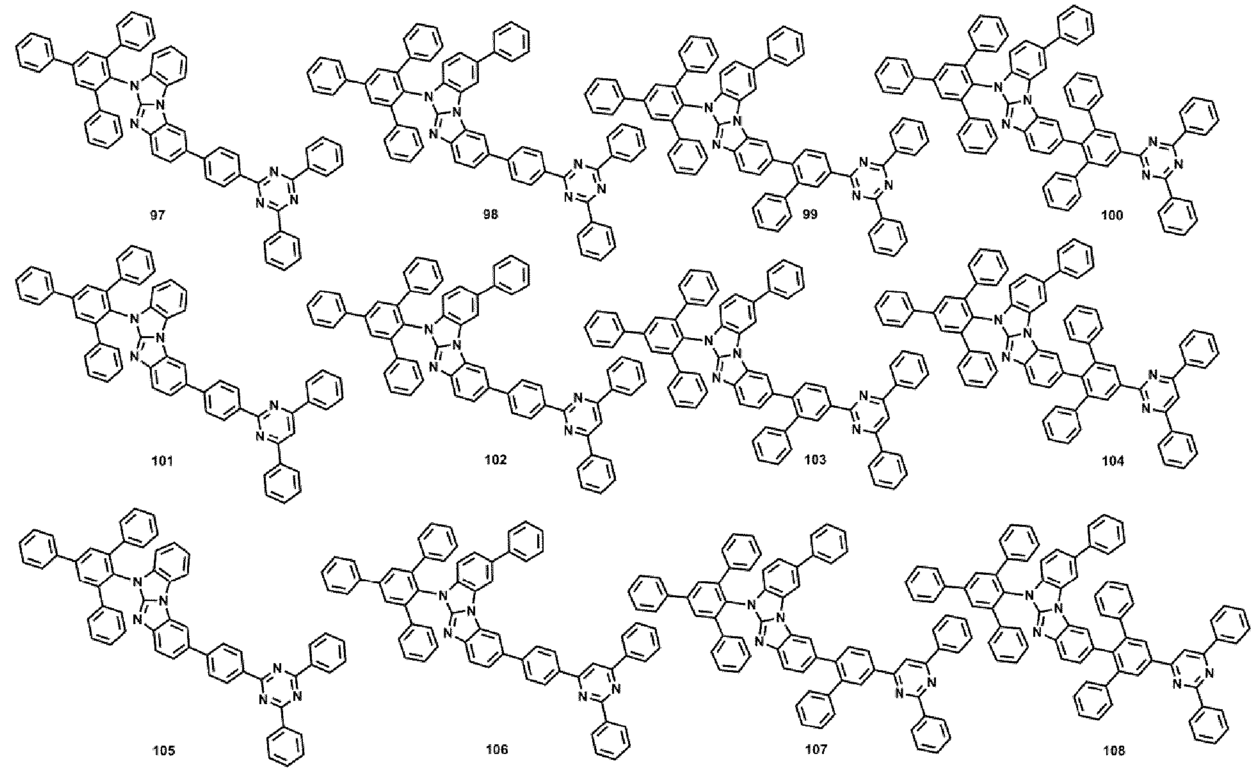

97 98 99 100

101 102 103 104

105 106 107 108

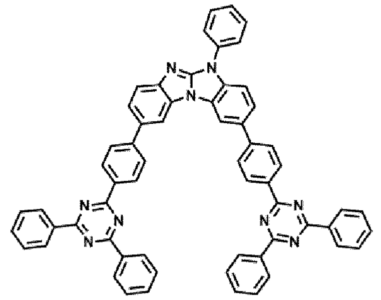

109

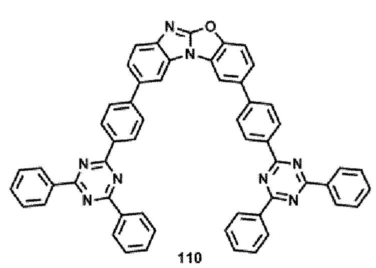

110

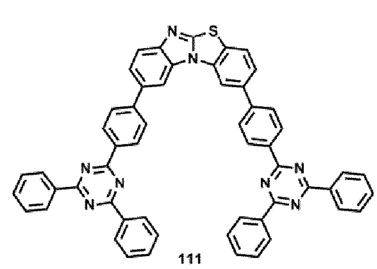

111

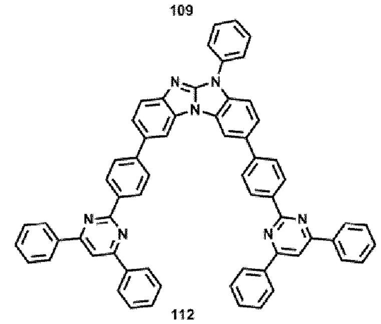

112

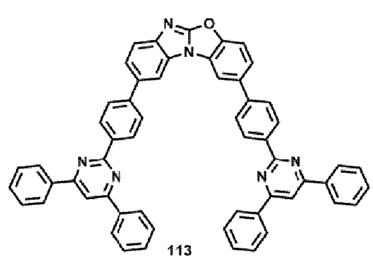

113

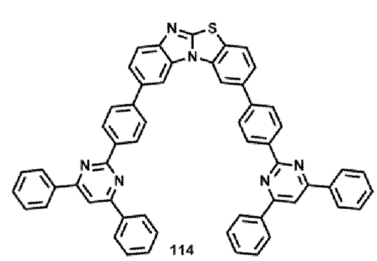

114

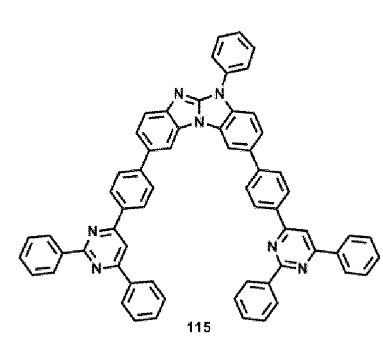

115

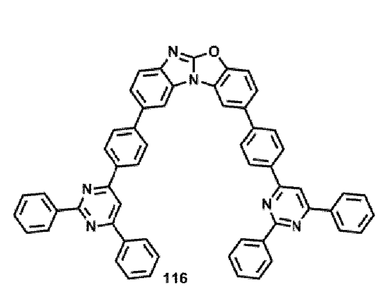

116

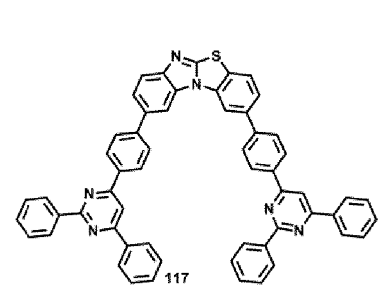

117

Group VIII

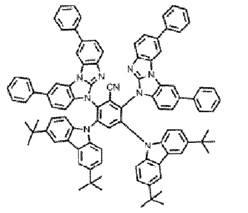 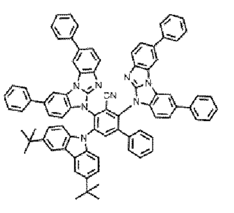 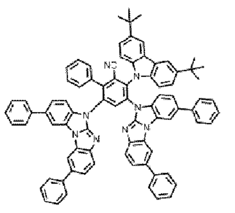 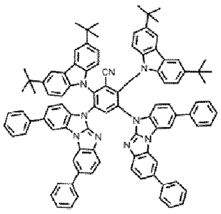 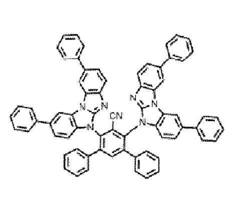

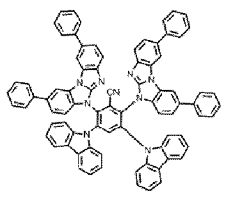 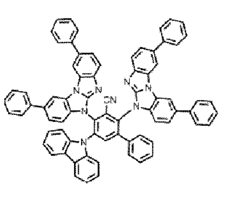 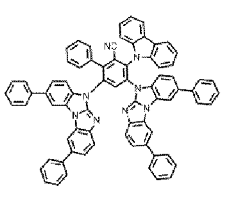 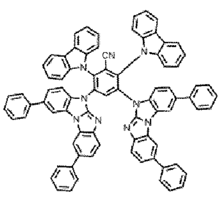 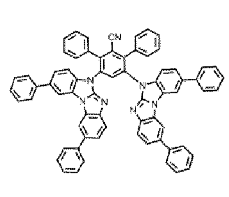

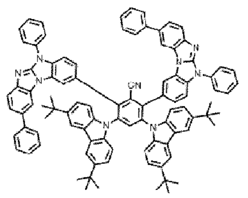 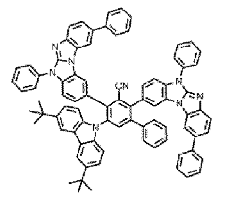 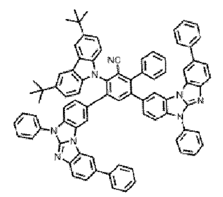 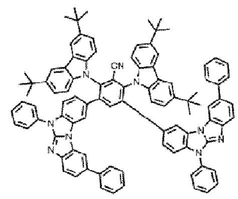 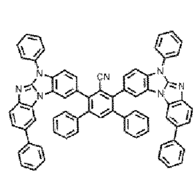

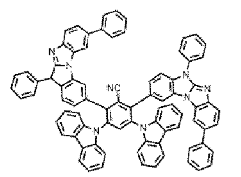 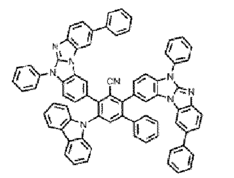 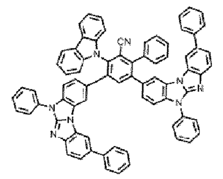 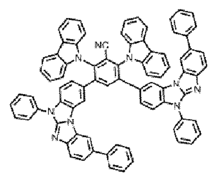 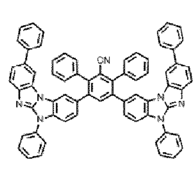

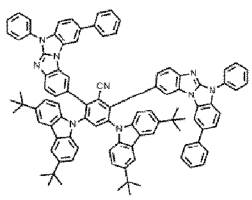 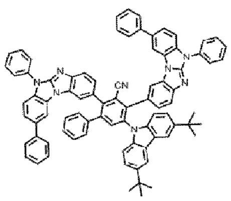 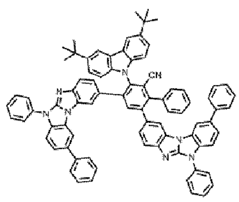 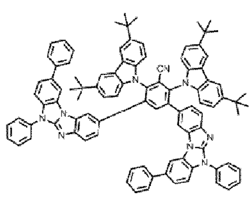 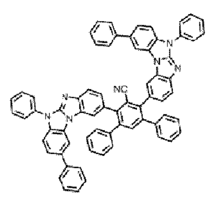

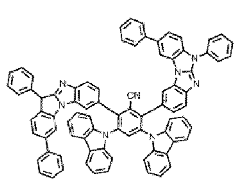 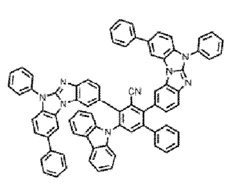 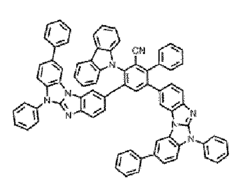 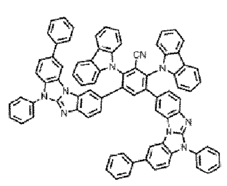 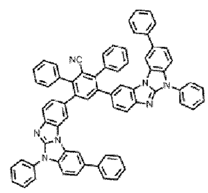

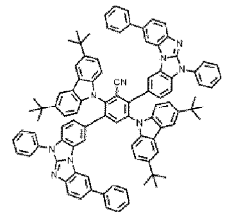
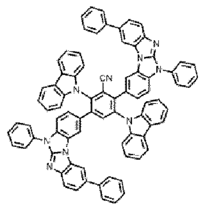
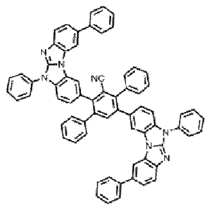
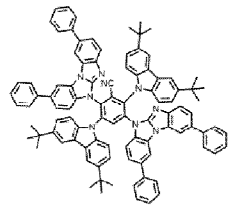
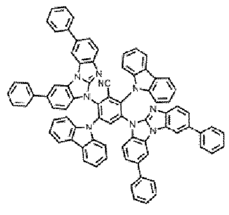

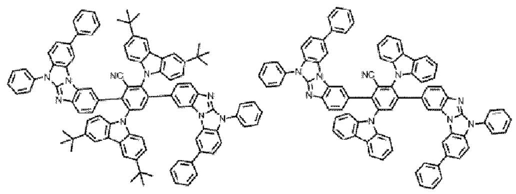
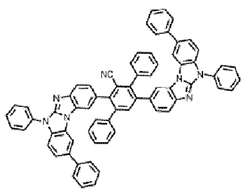
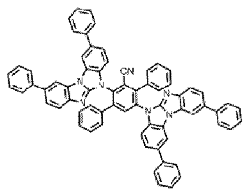

## Group IX

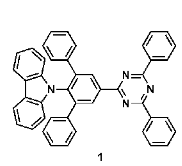
1
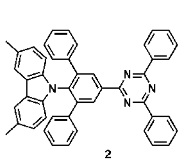
2
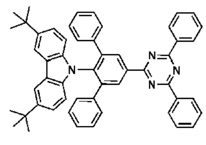
3
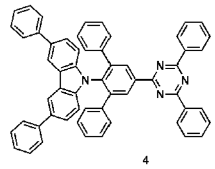
4

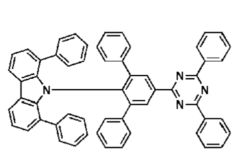
5
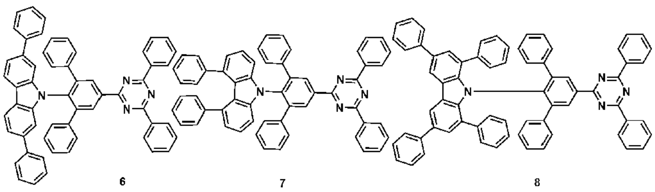
6

7

8

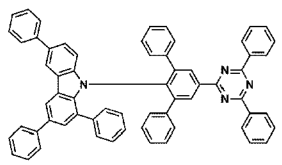
9
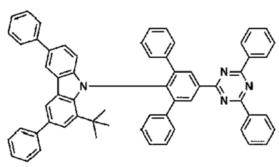
10
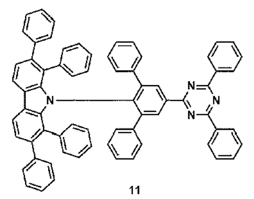
11
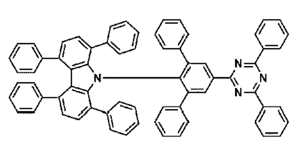
12

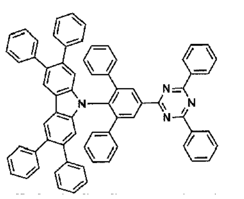
13
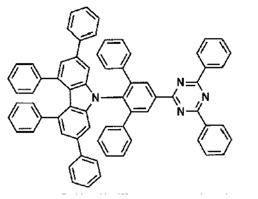
14
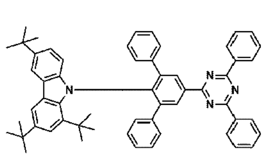
15
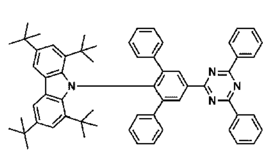
16

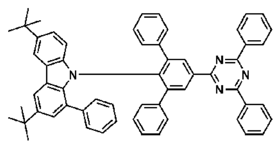
17
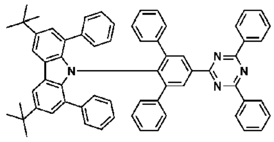
18
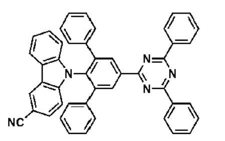
19
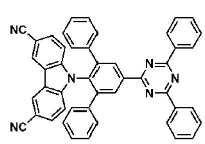
20

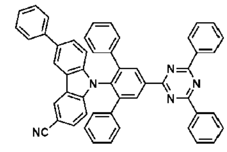
21
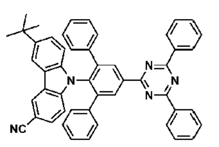
22
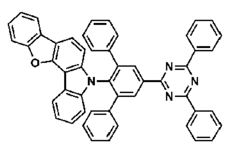
23
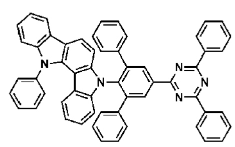
24

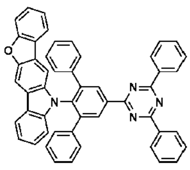
25

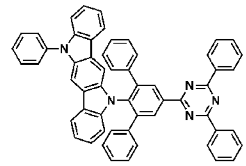
26

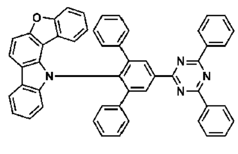
27

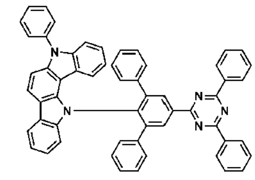
28

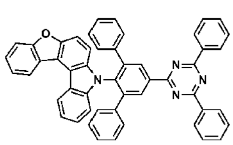
29

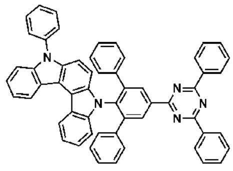
30

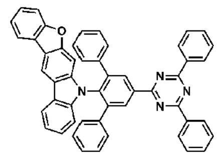
31

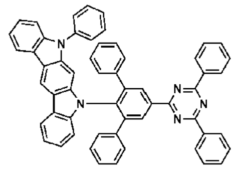
32

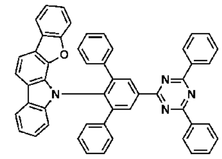
33

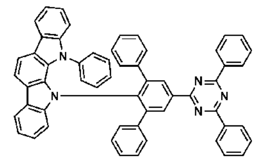
34

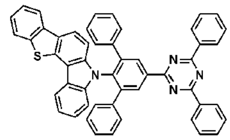
35

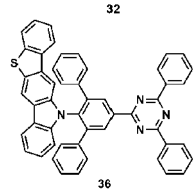
36

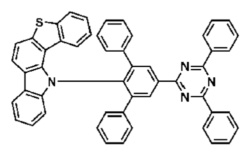
37

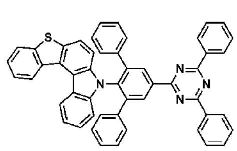
38

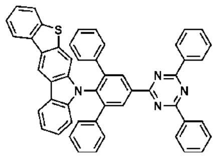
39

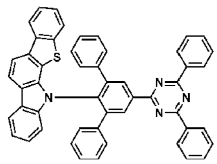
40

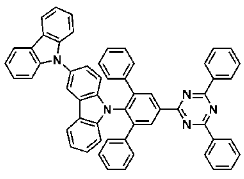
41

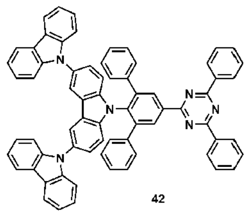
42

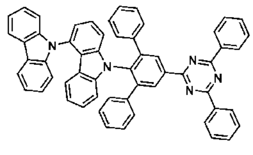
43

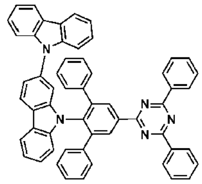
44

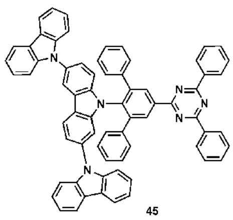
45

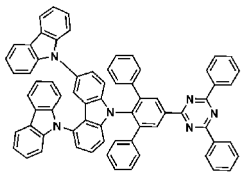
46

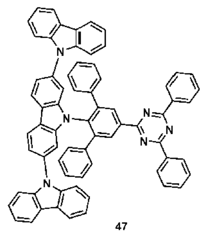
47

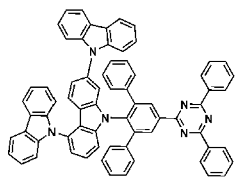
48

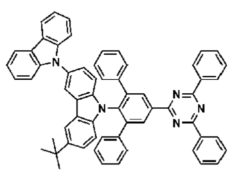
49

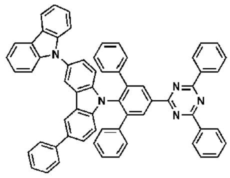
50

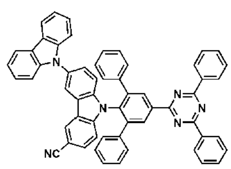
51

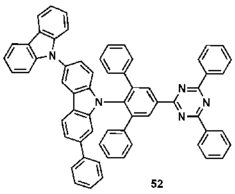
52

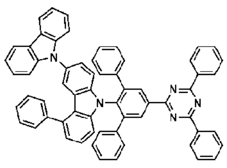
53

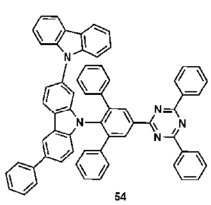
54

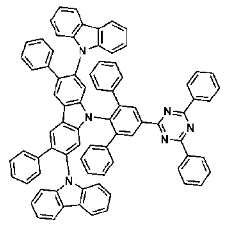
55

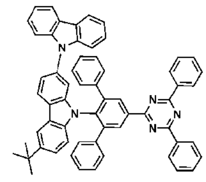
56

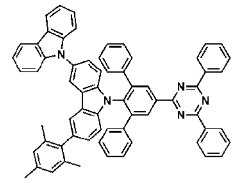
57

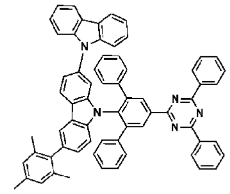
58

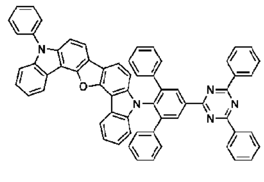
59

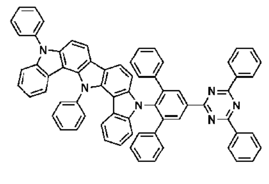
60

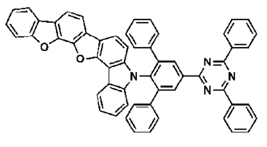
61

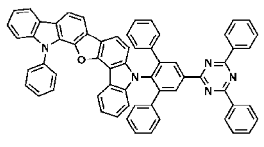
62

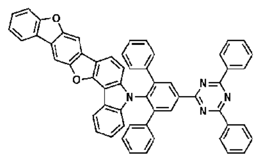
63

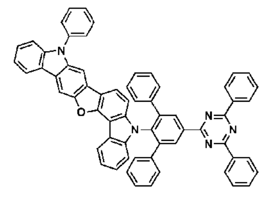
64

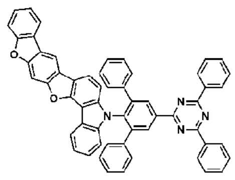
65

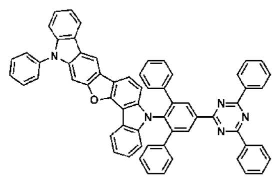
66

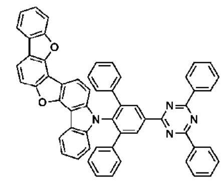
67

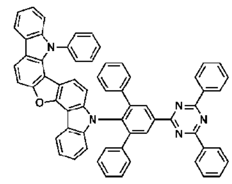
68

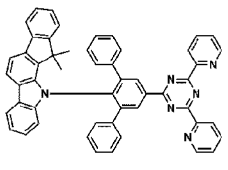
69

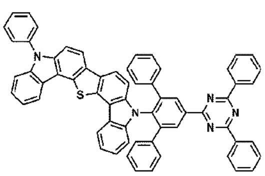
70

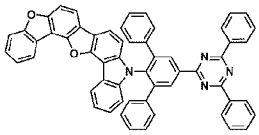
71

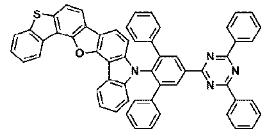
72

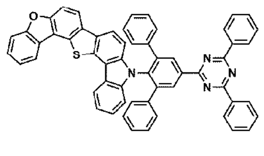
73

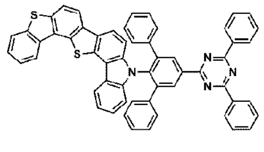
74

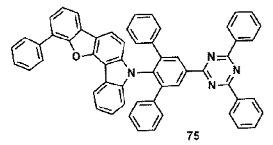
75

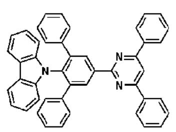
76

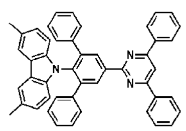
77

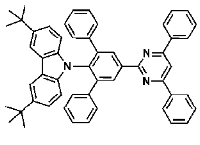
78

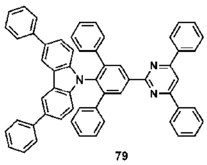
79

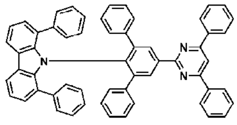
80

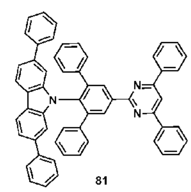
81

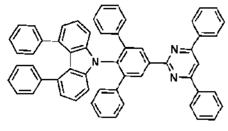
82

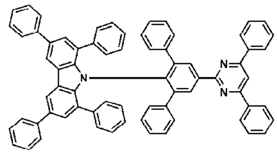
83

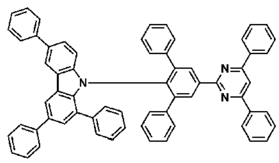
84

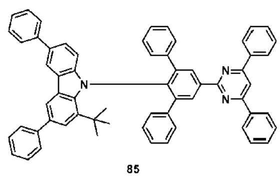
85

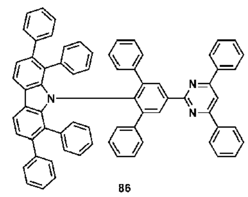
86

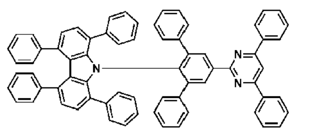
87

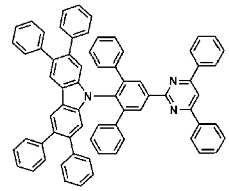
88

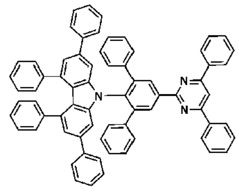
89

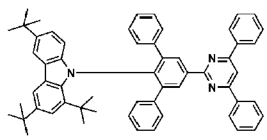
90

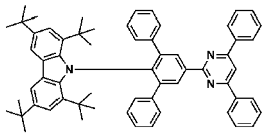
91

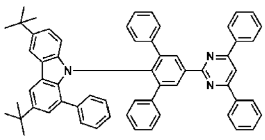
92

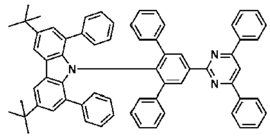
93

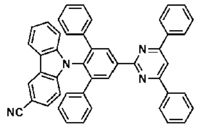
94

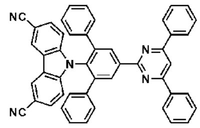
95

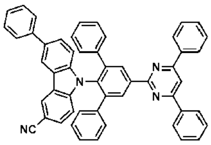
96

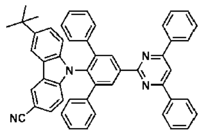
97

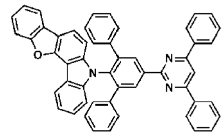
98

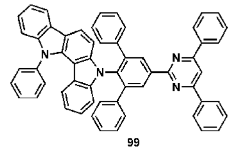
99

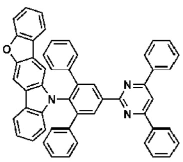
100

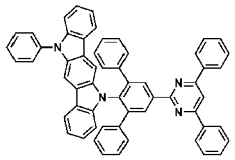
101

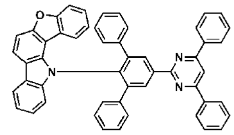
102

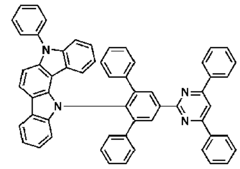
103

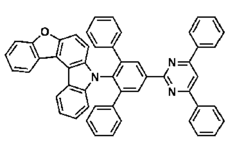
104

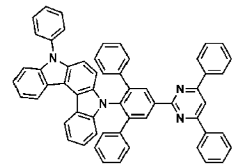
105

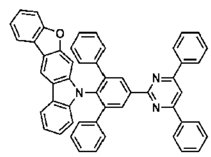
106

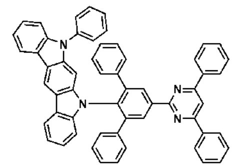
107

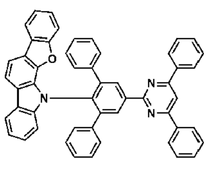
108

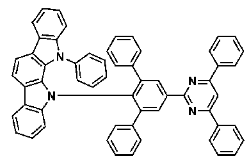
109

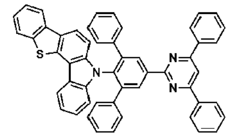
110

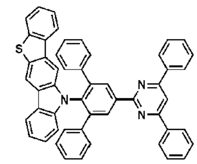
111

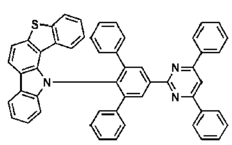
112

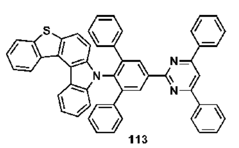
113

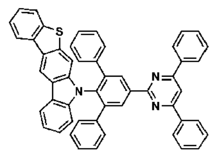
114

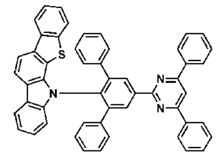
115

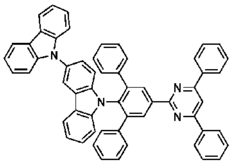
116
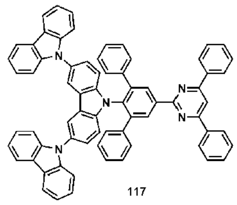
117
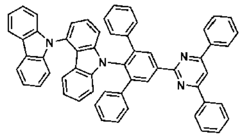
118
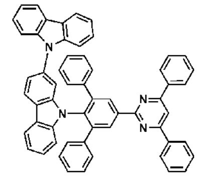
119
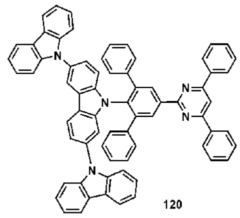
120
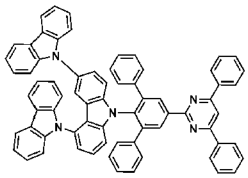
121
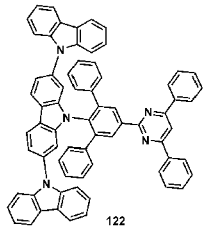
122
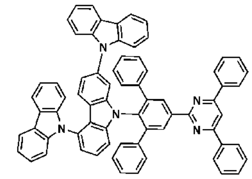
123
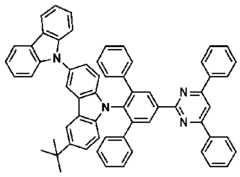
124
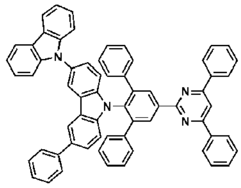
125
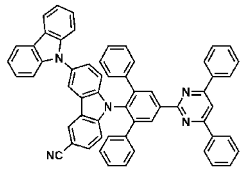
126
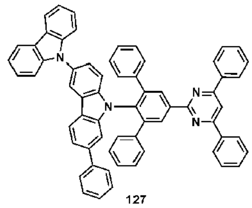
127
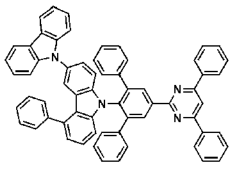
128
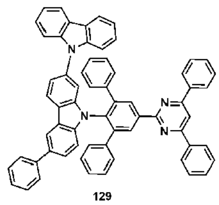
129
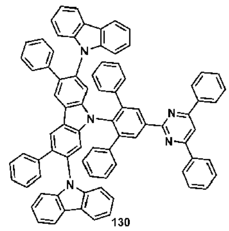
130
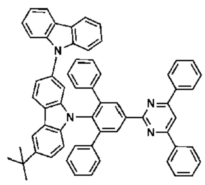
131
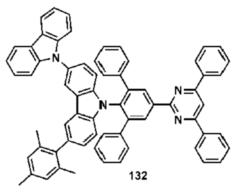
132
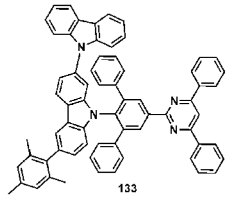
133
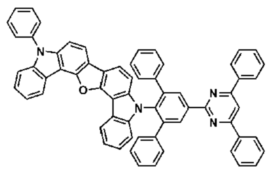
134
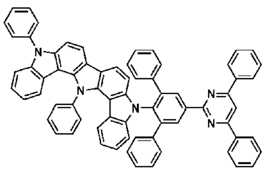
135
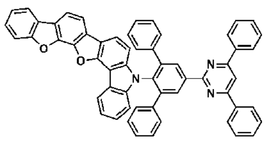
136
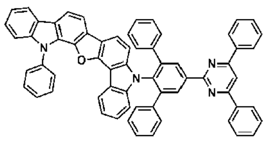
137
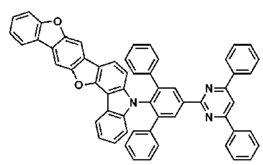
138
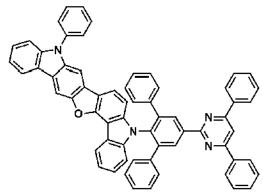
139
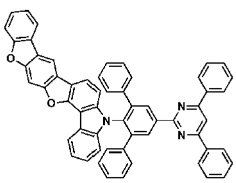
140
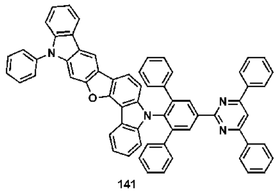
141
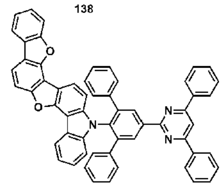
142
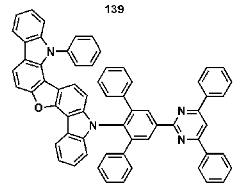
143

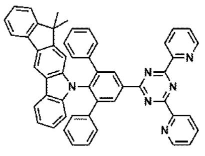
144

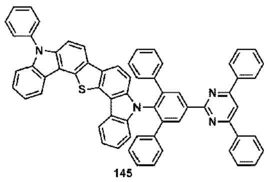
145

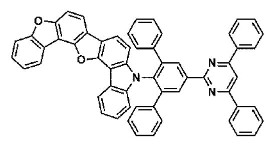
146

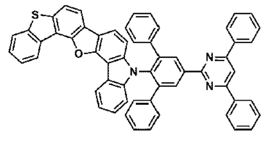
147

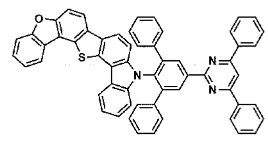
148

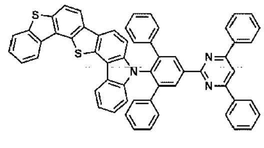
149

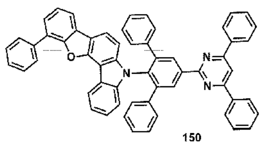
150

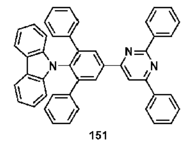
151

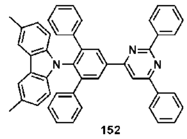
152

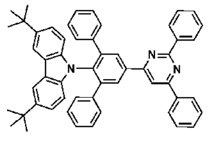
153

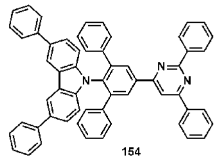
154

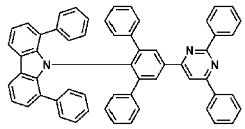
155

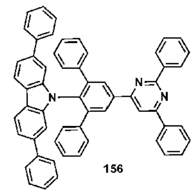
156

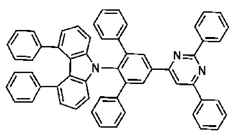
157

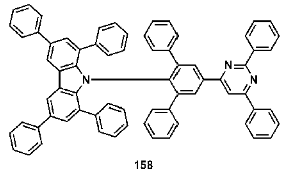
158

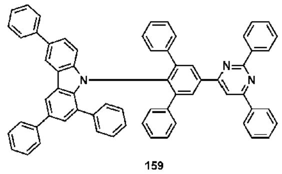
159

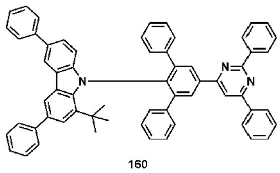
160

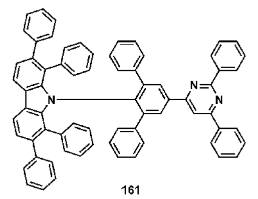
161

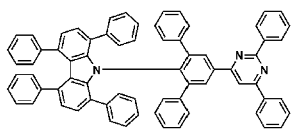
162

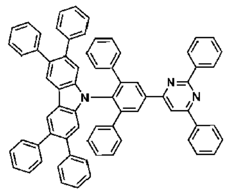
163

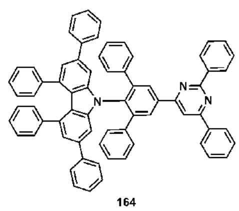
164

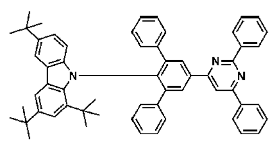
165

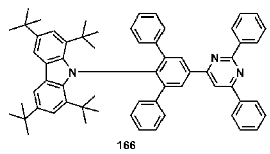
166

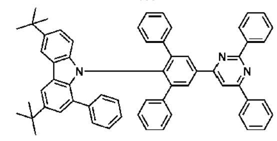
167

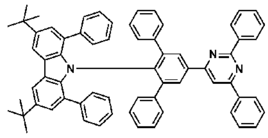
168

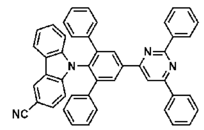
169

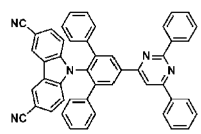
170

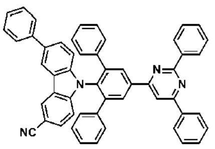
171

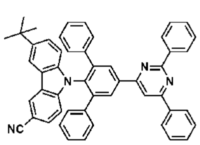
172

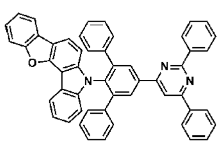
173

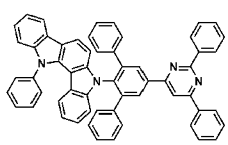
174

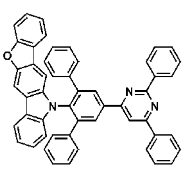
175

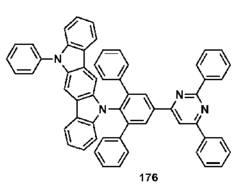
176

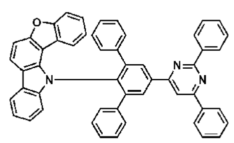
177

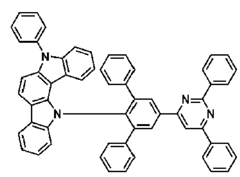
178

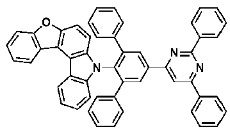
179

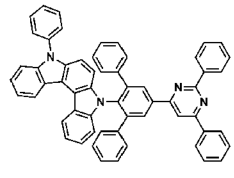
180

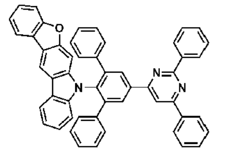
181

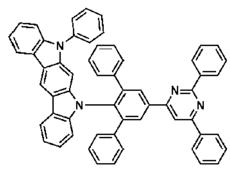
182

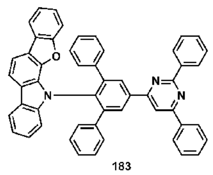
183

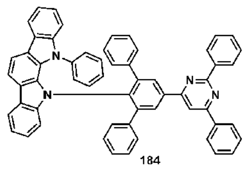
184

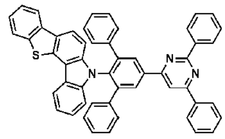
185

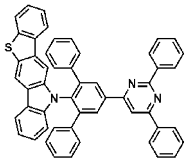
186

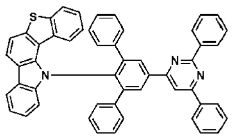
187

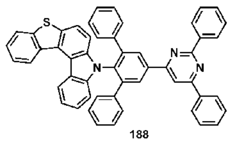
188

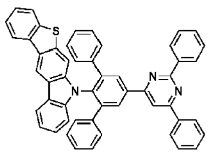
189

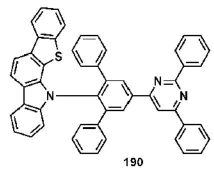
190

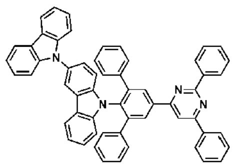
191

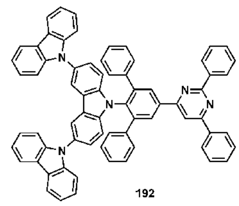
192

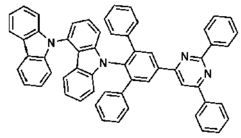
193

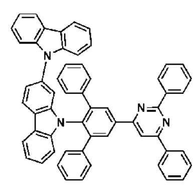
194

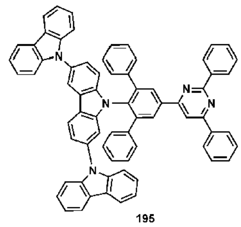
195

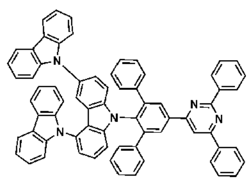
196

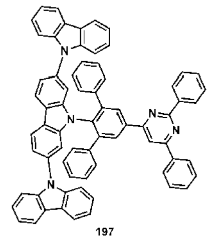
197

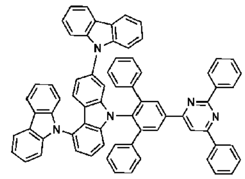
198

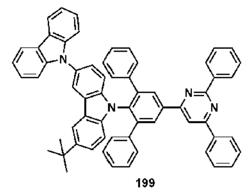
199

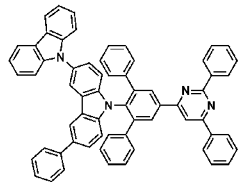
200

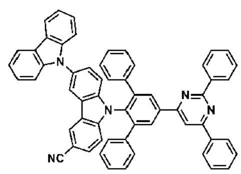
201

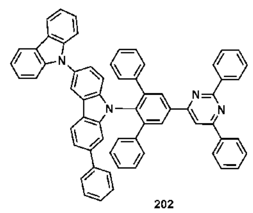
202

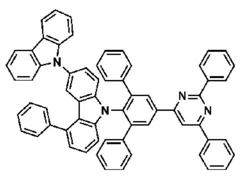
203

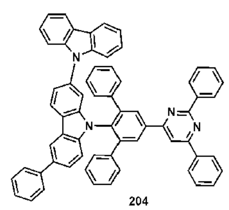
204

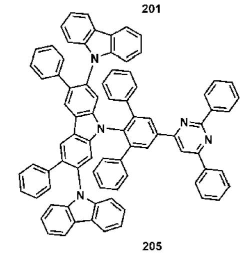
205

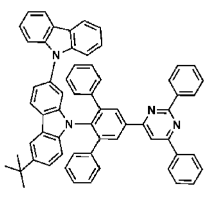
206

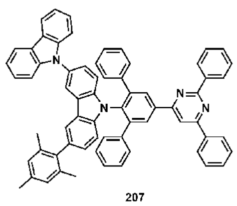
207

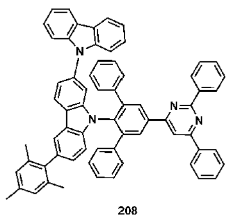
208

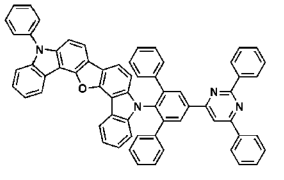
209

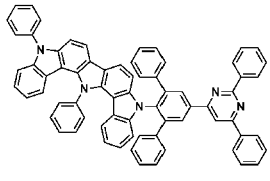
210

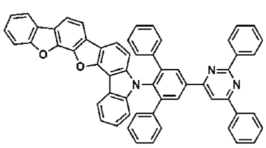
211
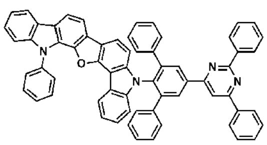
212
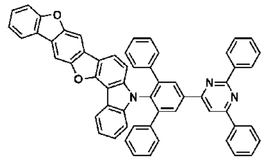
213
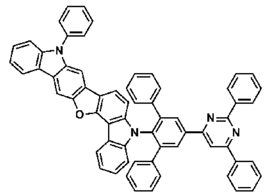
214
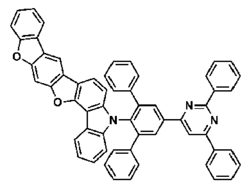
215
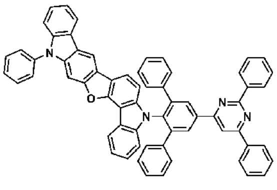
216
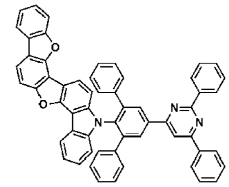
217
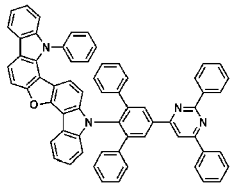
218
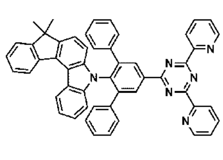
219
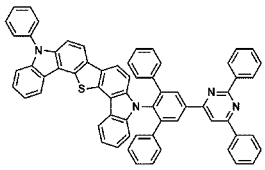
220
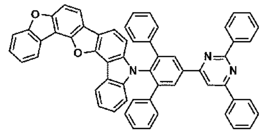
221
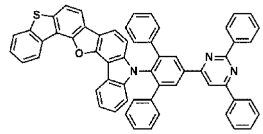
222
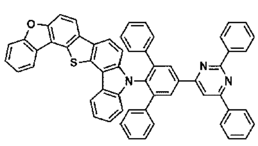
223
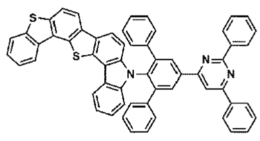
224
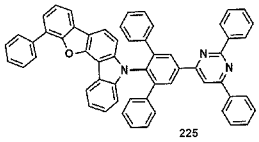
225
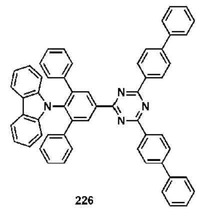
226
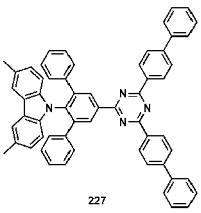
227
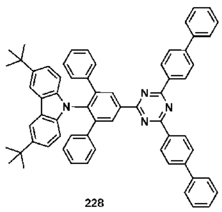
228
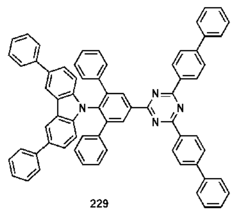
229
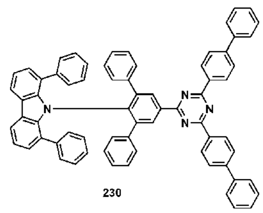
230
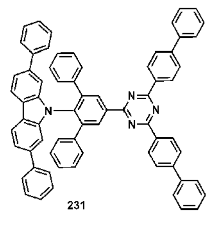
231
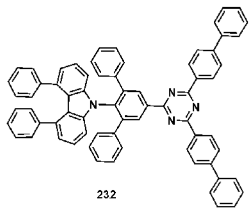
232
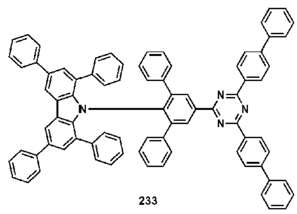
233
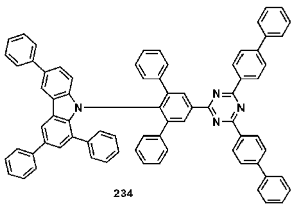
234
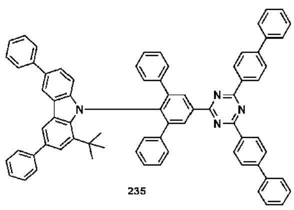
235
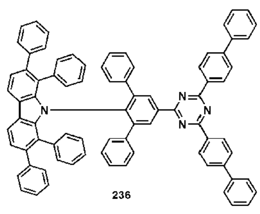
236
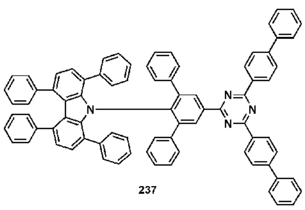
237

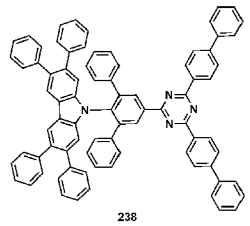
238

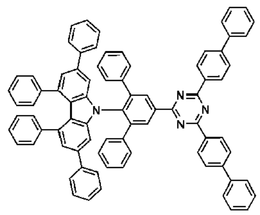
239

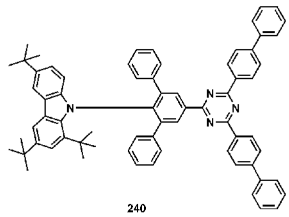
240

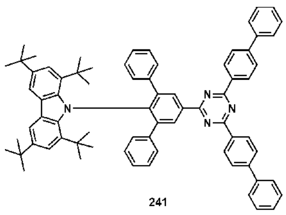
241

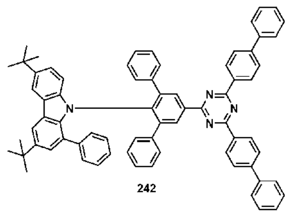
242

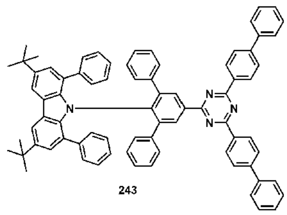
243

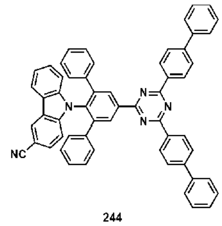
244

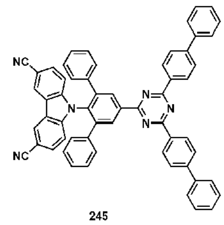
245

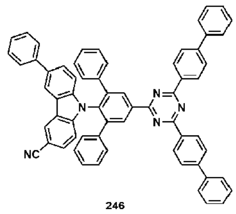
246

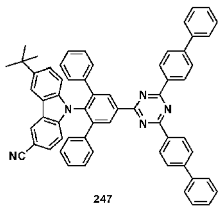
247

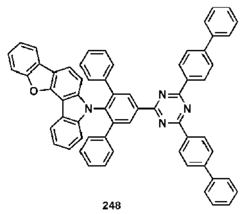
248

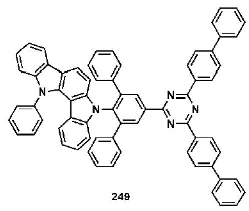
249

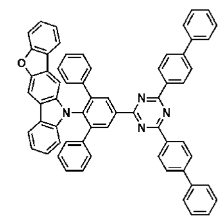
250

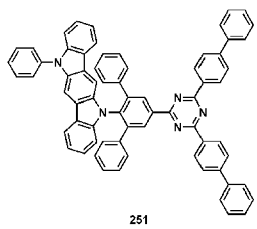
251

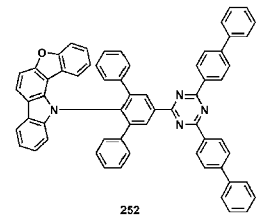
252

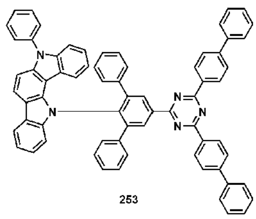
253

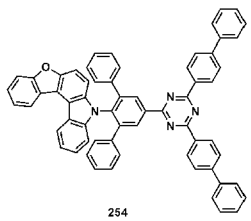
254

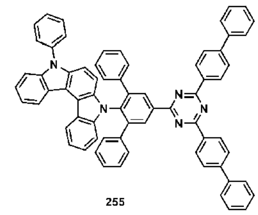
255

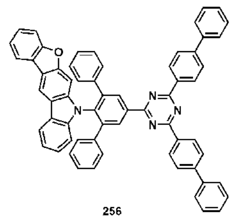
256

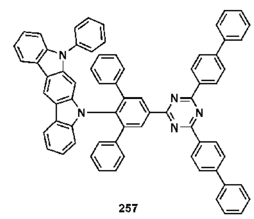
257

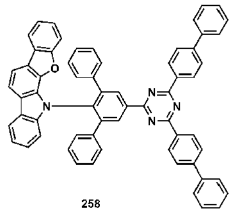
258

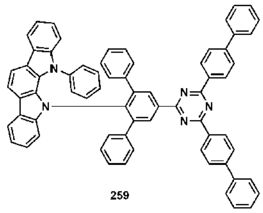
259

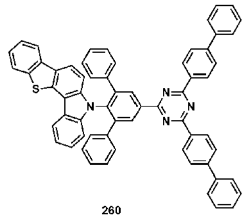
260

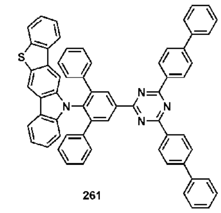
261

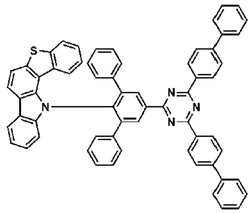
262

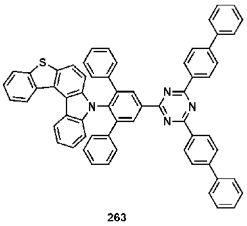
263

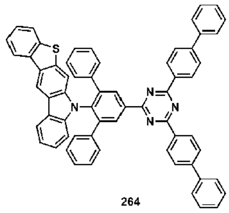
264

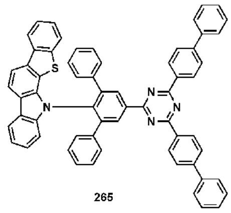
265

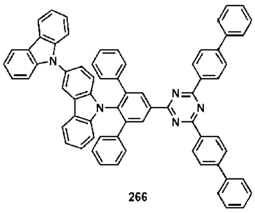
266

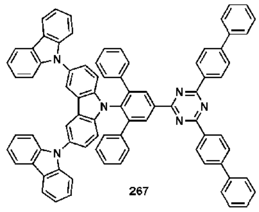
267

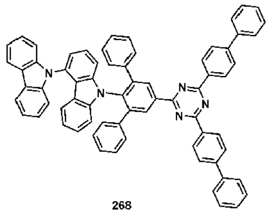
268

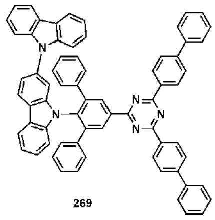
269

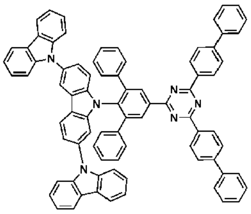
270

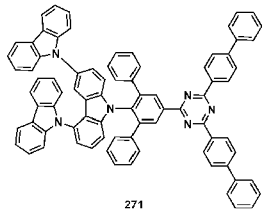
271

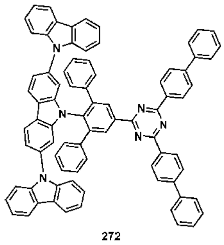
272

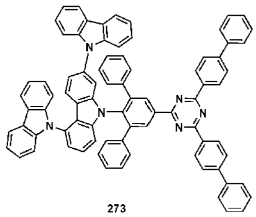
273

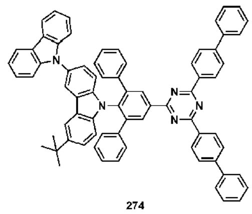
274

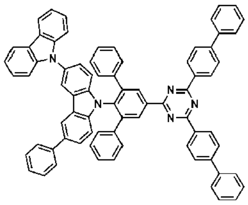
275

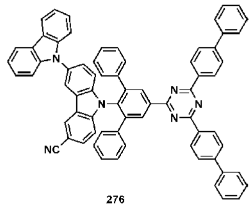
276

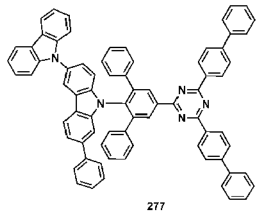
277

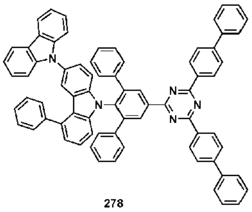
278

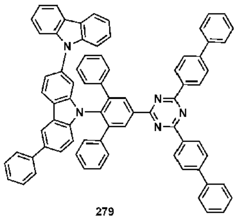
279

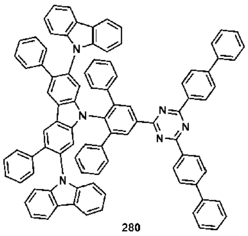
280

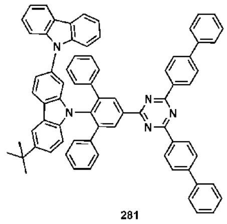
281

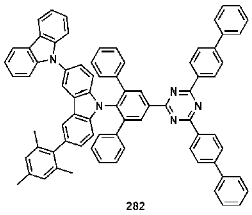
282

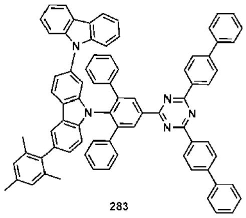
283

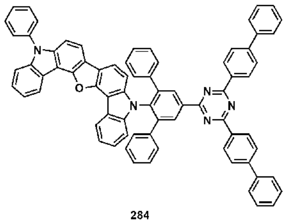
284

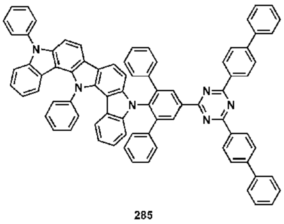
285

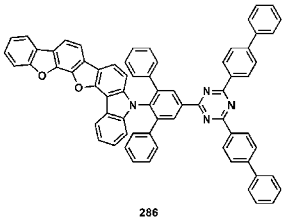
286

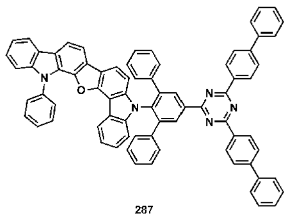
287

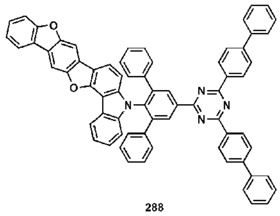
288

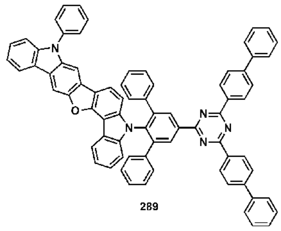
289

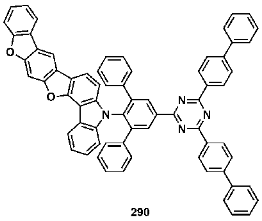
290

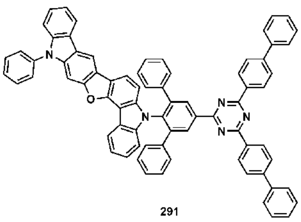
291

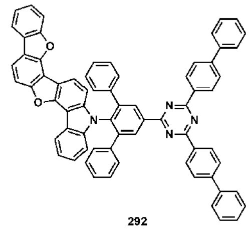
292

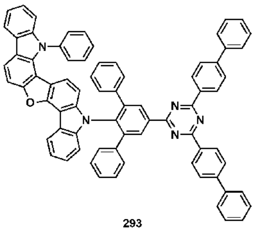
293

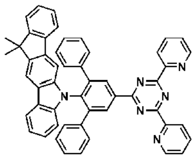
294

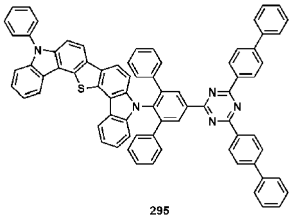
295

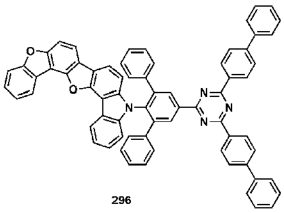
296

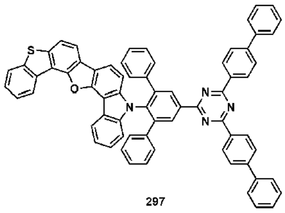
297

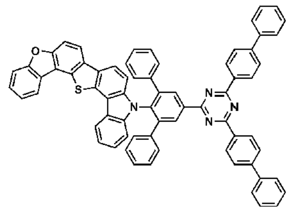
298

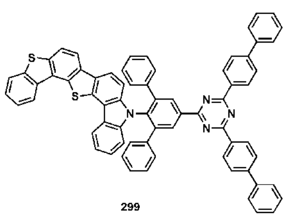
299

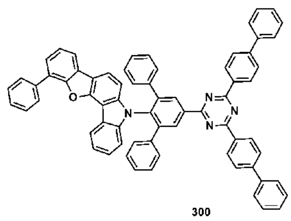
300

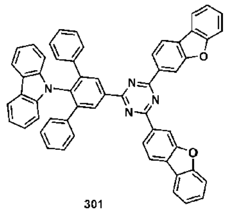
301
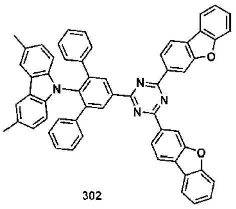
302
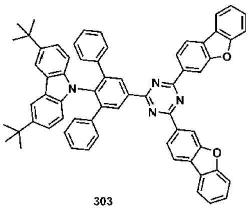
303
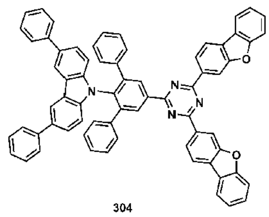
304

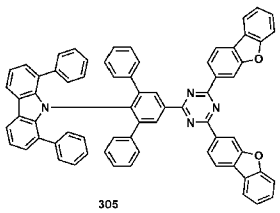
305
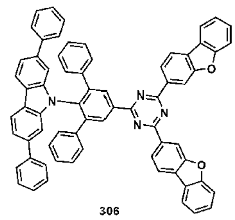
306
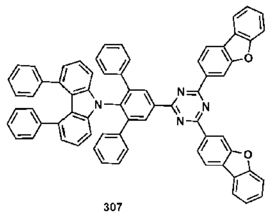
307
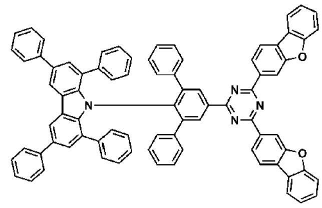
308

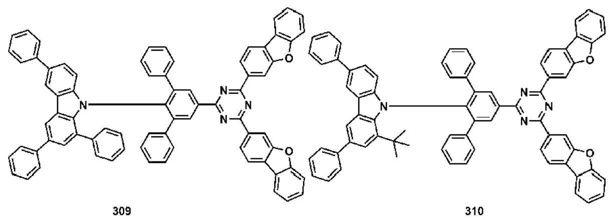
309
310
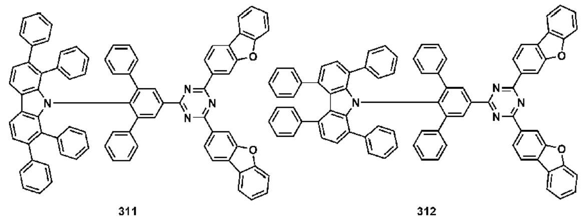
311
312

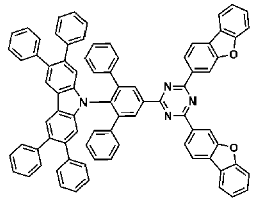
313
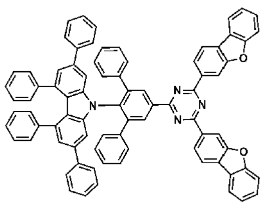
314
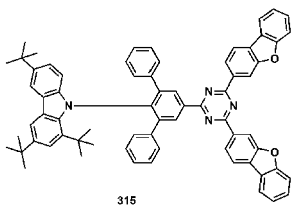
315
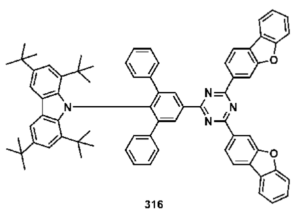
316

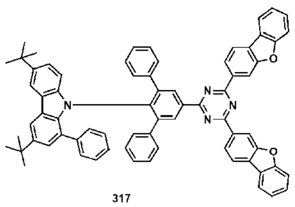
317
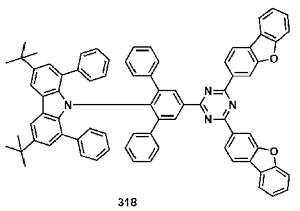
318
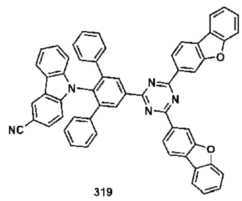
319
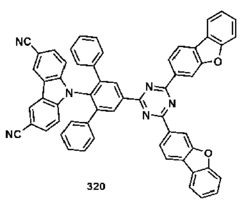
320

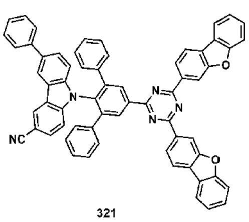
321
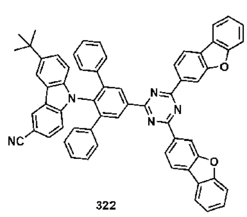
322
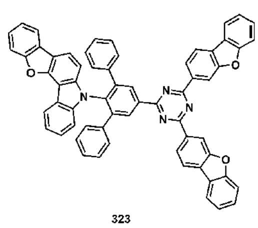
323
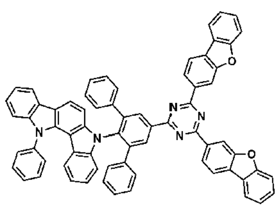
324

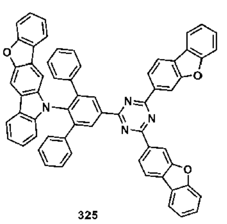
325
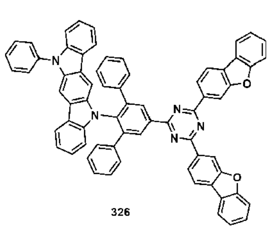
326
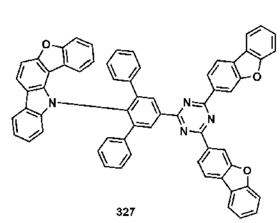
327
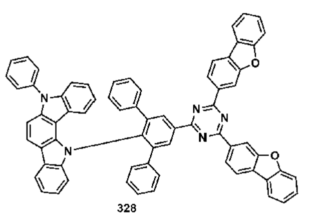
328

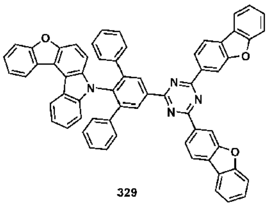
329

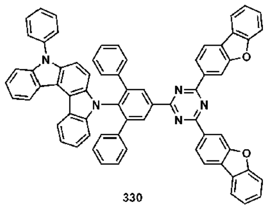
330

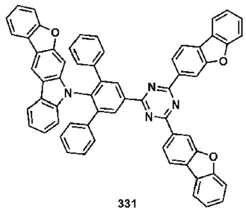
331

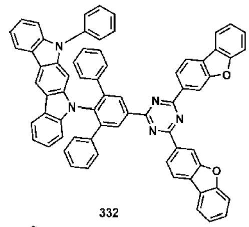
332

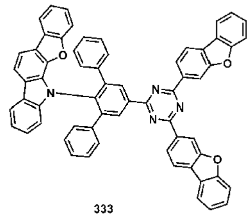
333

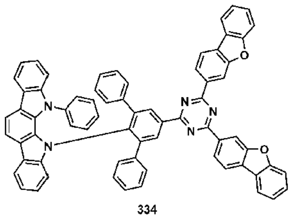
334

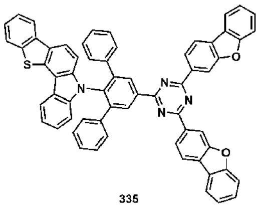
335

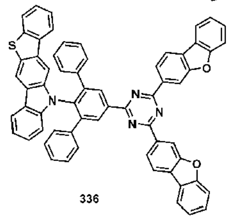
336

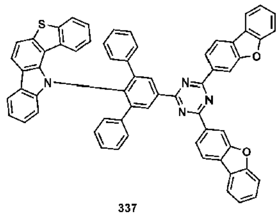
337

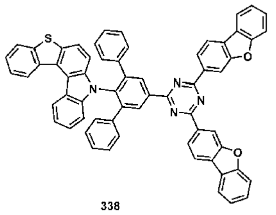
338

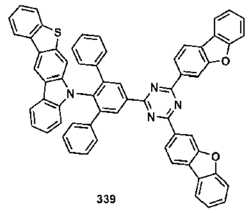
339

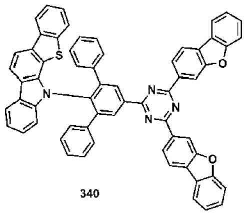
340

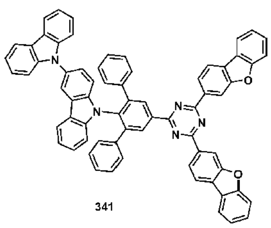
341

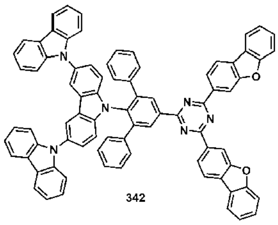
342

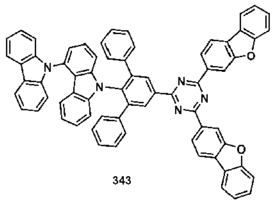
343

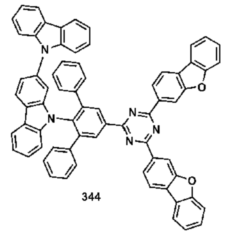
344

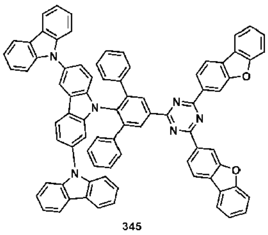
345

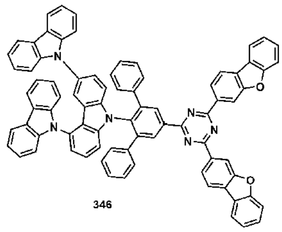
346

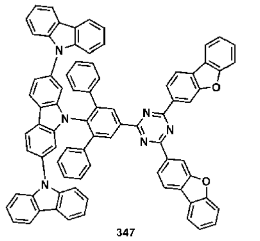
347

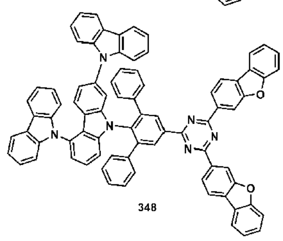
348

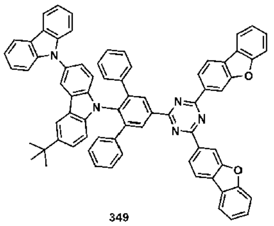
349
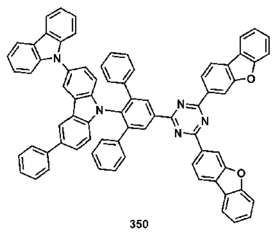
350
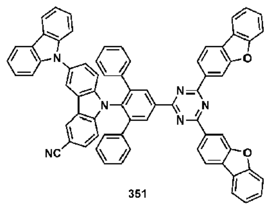
351
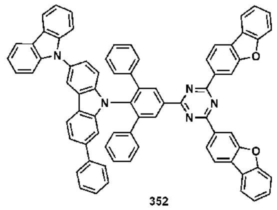
352
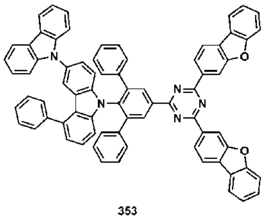
353
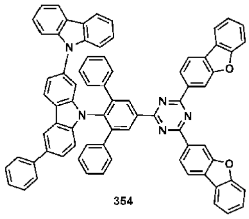
354
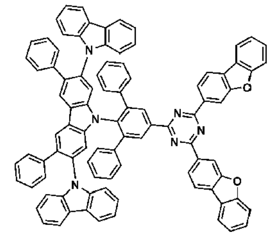
355
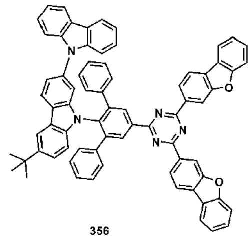
356
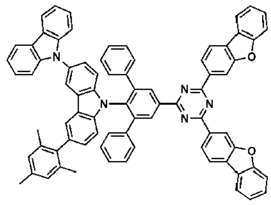
357
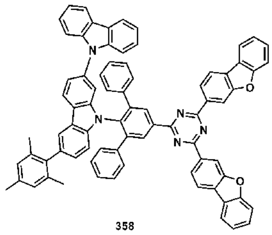
358
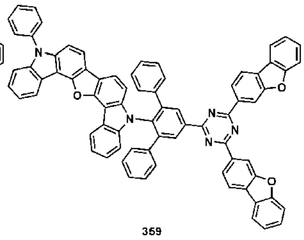
359
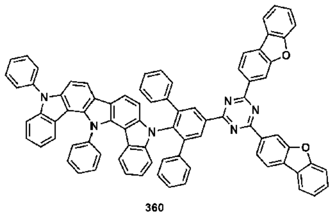
360
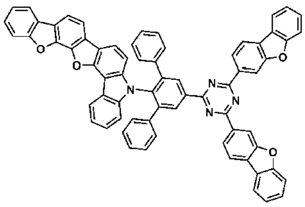
361
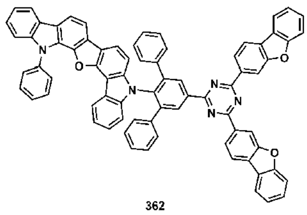
362
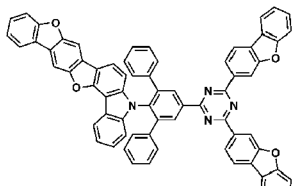
363
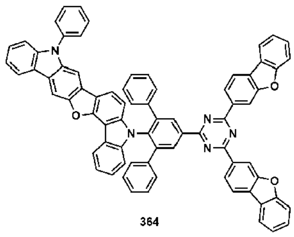
364
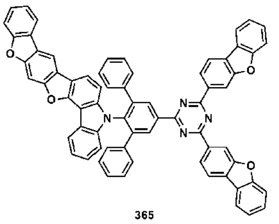
365
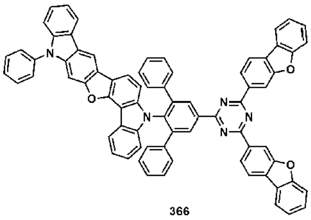
366
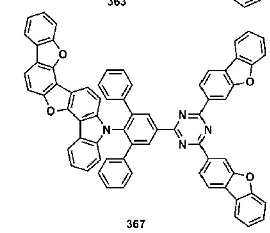
367
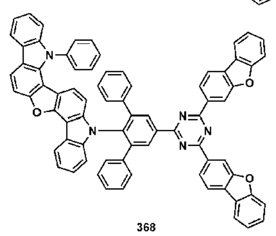
368
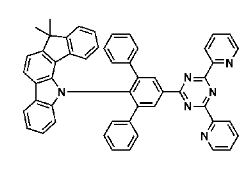
369
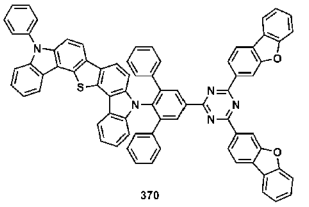
370
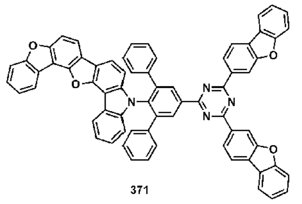
371
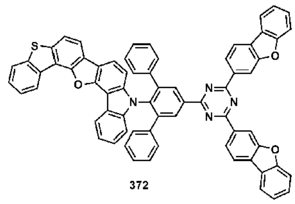
372
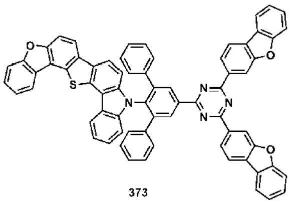
373
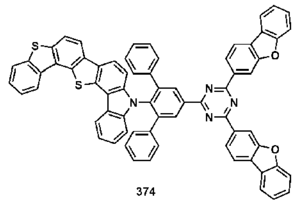
374
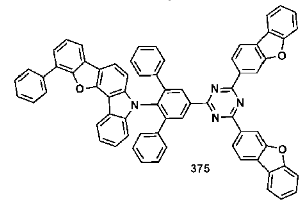
375

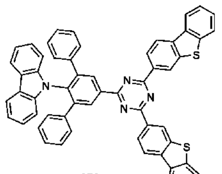
376

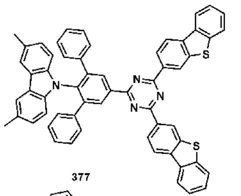
377

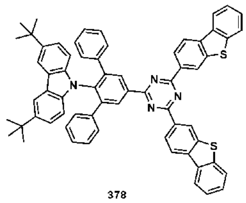
378

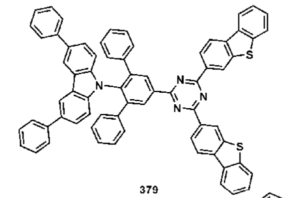
379

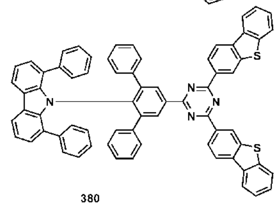
380

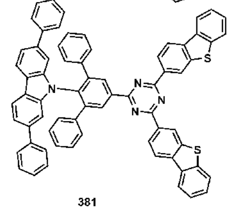
381

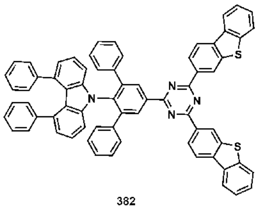
382

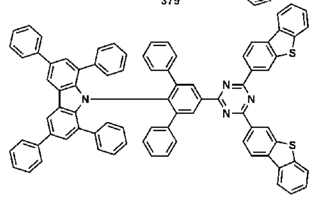
383

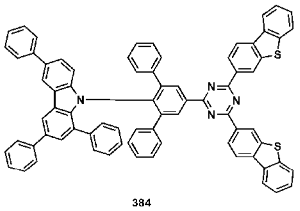
384

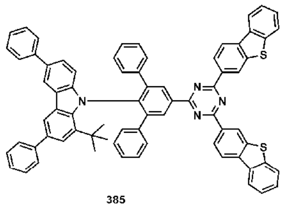
385

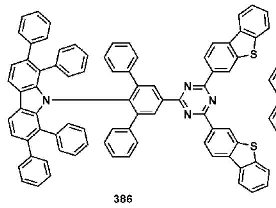
386

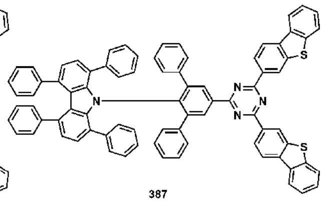
387

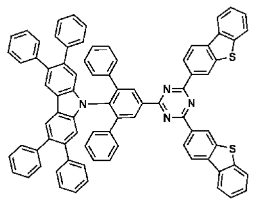
388

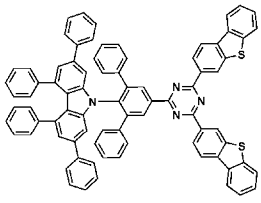
389

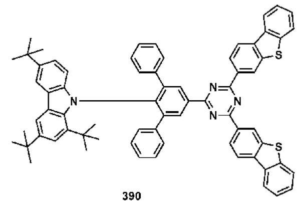
390

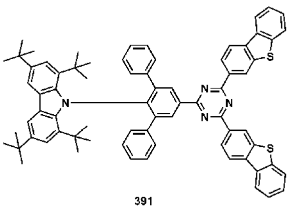
391

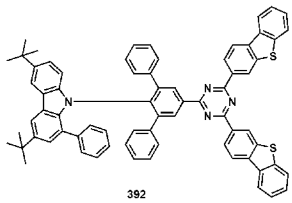
392

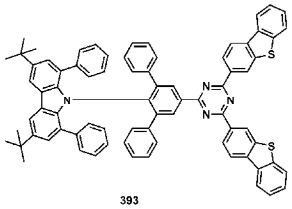
393

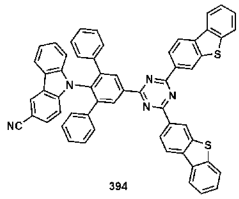
394

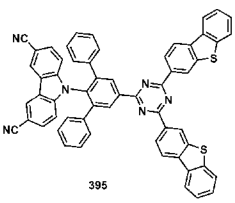
395

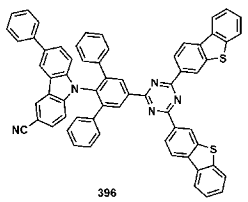
396

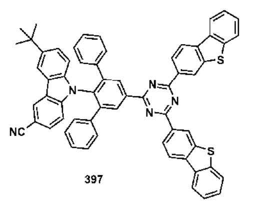
397

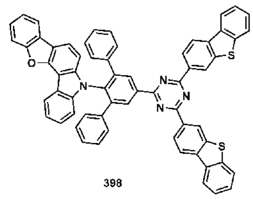
398

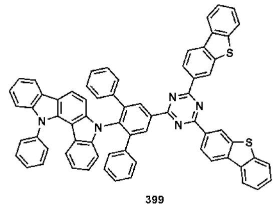
399

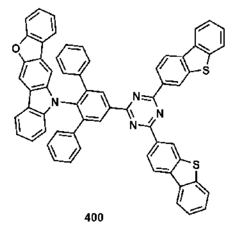
400

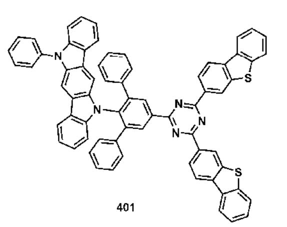
401

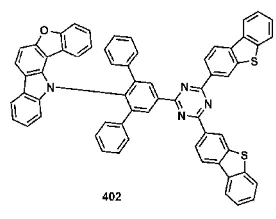
402

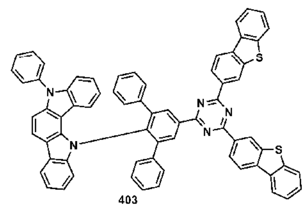
403

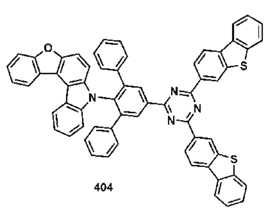
404

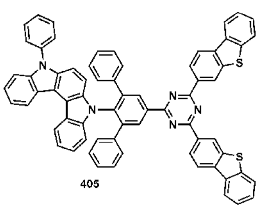
405

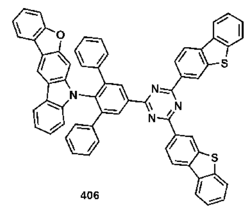
406

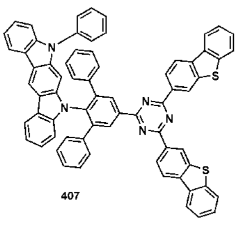
407

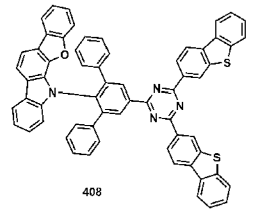
408

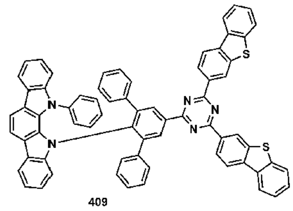
409

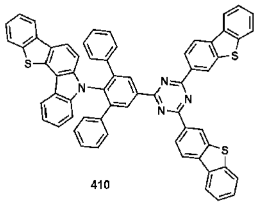
410

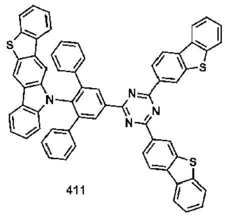
411

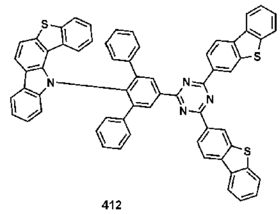
412

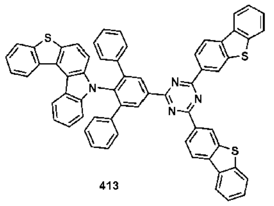
413

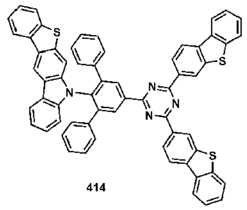
414

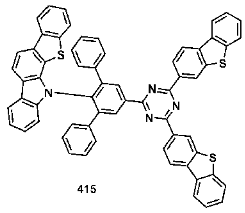
415

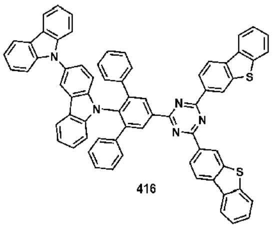
416

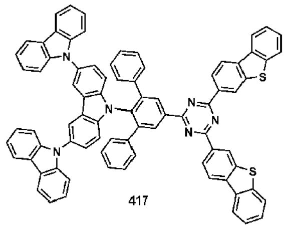
417

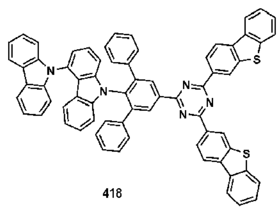
418

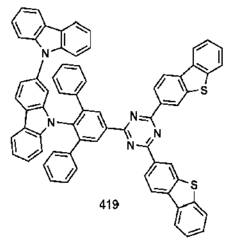
419

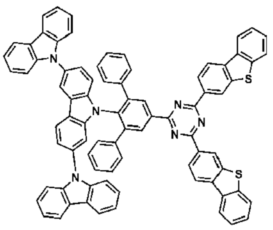
420

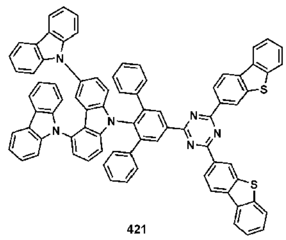
421

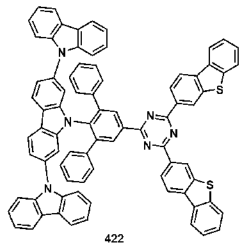
422

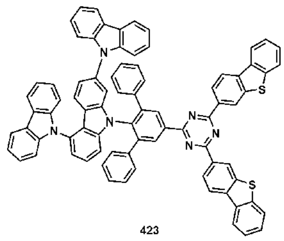
423

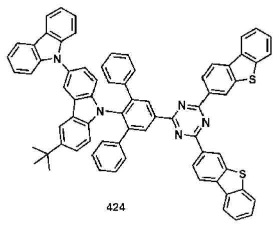
424

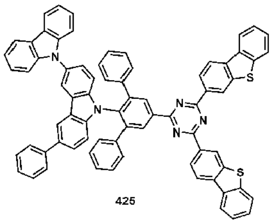
425

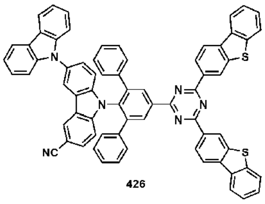
426

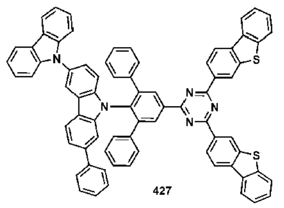
427

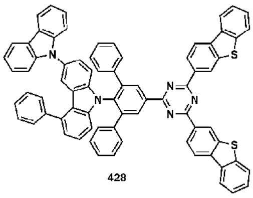

428

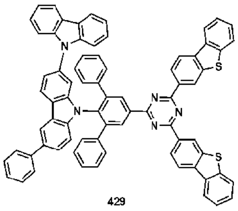

429

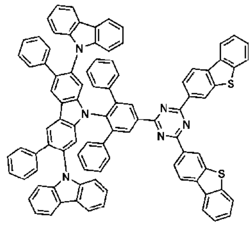

430

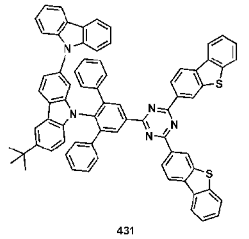

431

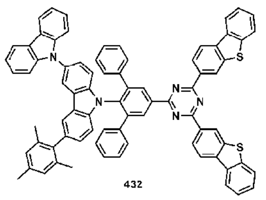

432

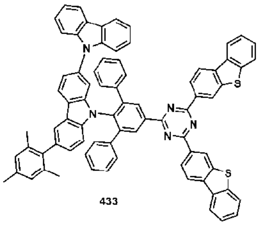

433

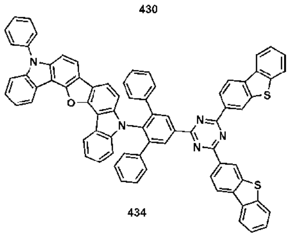

434

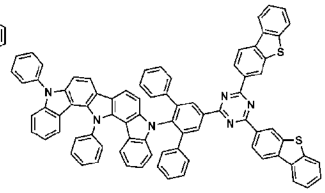

435

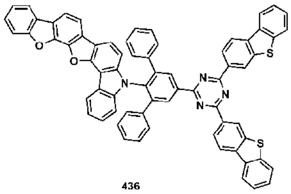

436

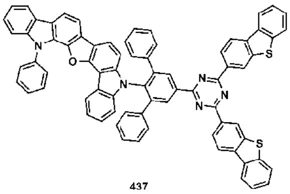

437

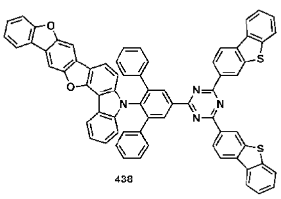

438

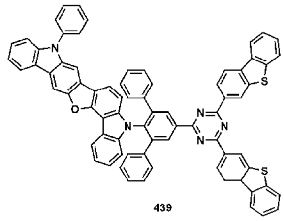

439

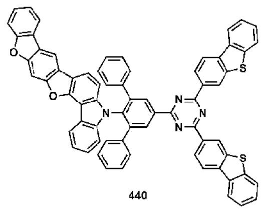

440

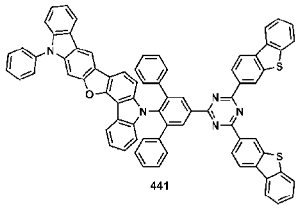

441

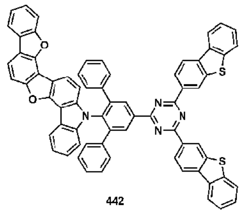

442

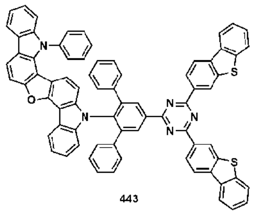

443

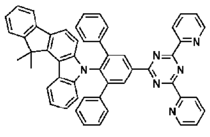

444

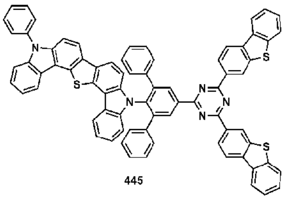

445

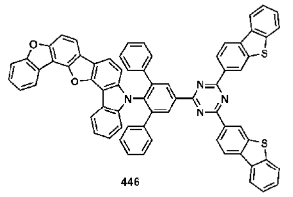

446

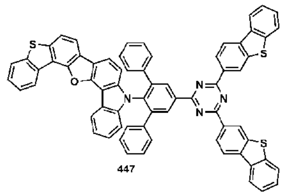

447

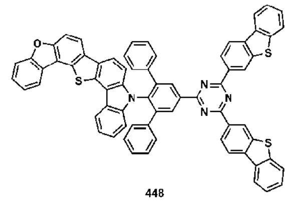

448

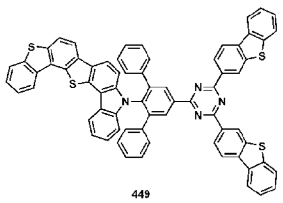

449

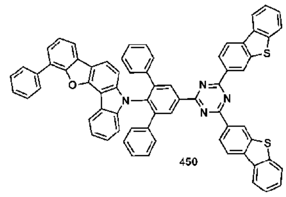

450

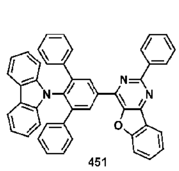

451

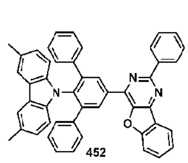

452

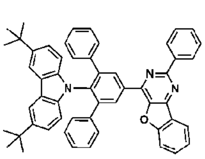

453

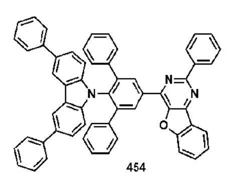

454

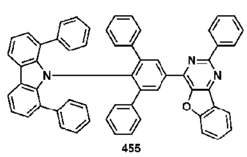

455

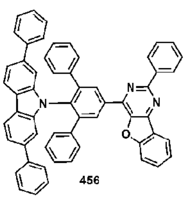

456

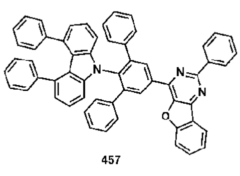

457

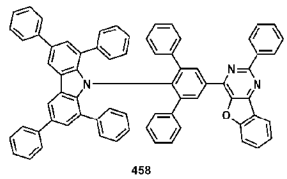

458

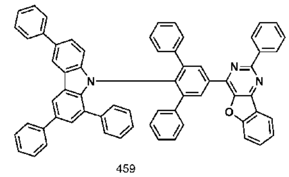
459
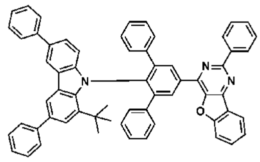
460
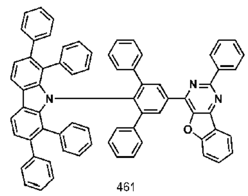
461
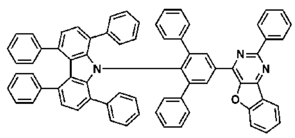
462

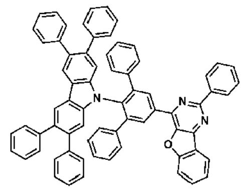
463
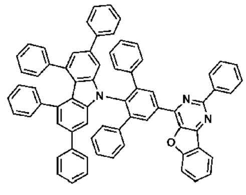
464
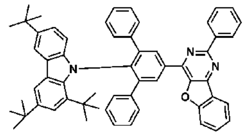
465
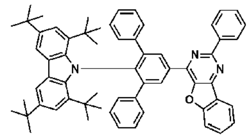
466

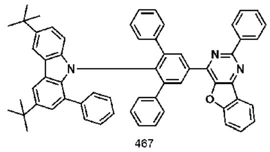
467
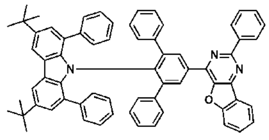
468
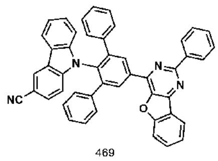
469
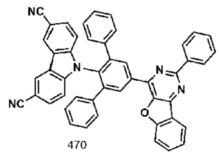
470

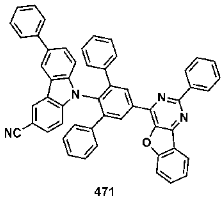
471
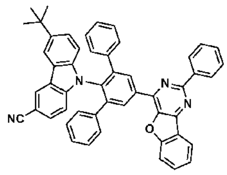
472
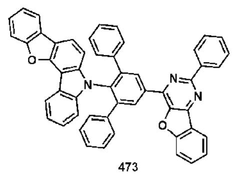
473
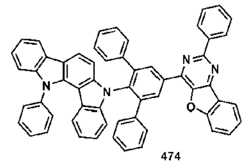
474

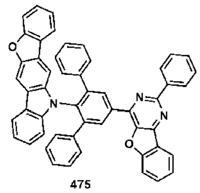
475
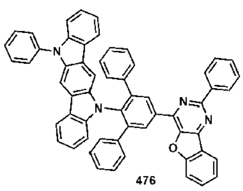
476
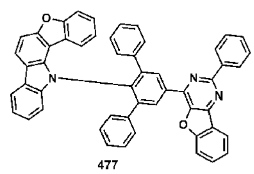
477
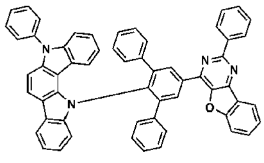
478

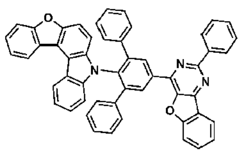
479
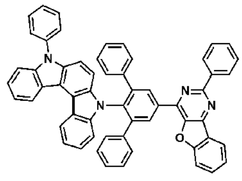
480
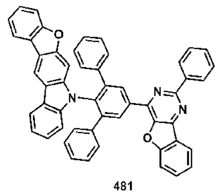
481
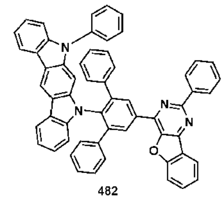
482

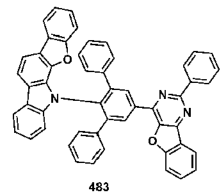
483
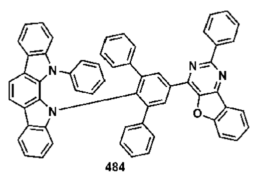
484
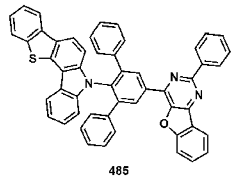
485
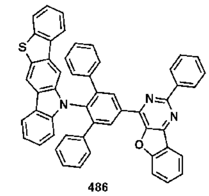
486

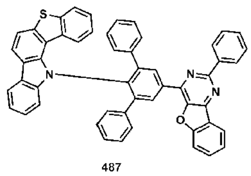
487
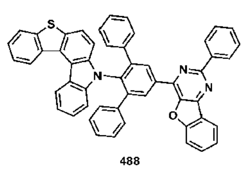
488
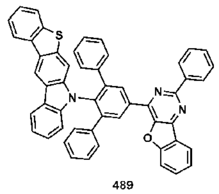
489
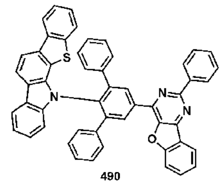
490

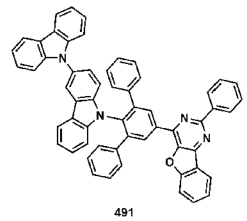
491
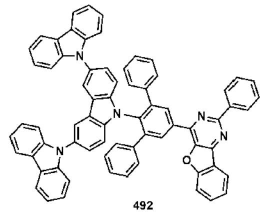
492
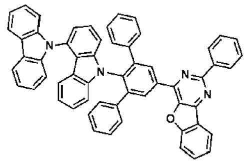
493
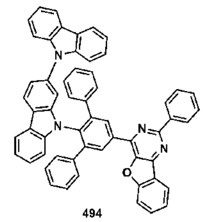
494
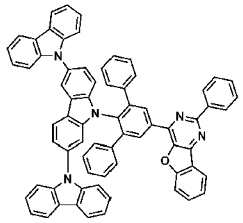
495
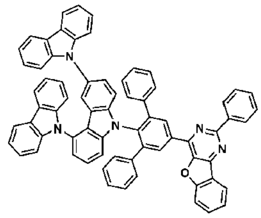
496
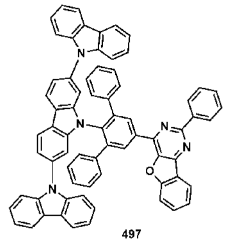
497
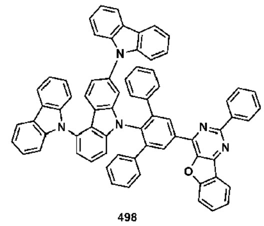
498
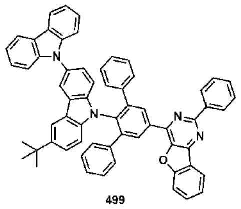
499
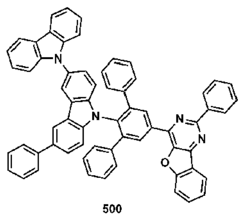
500
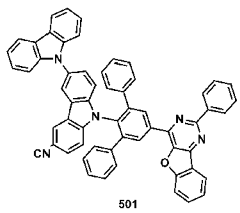
501
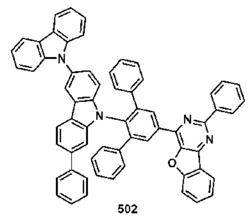
502
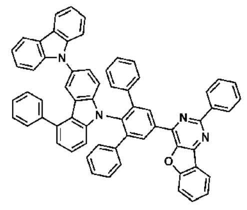
503
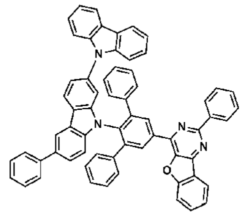
504
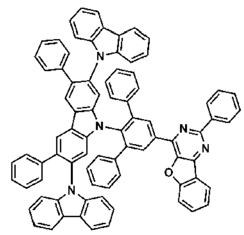
505
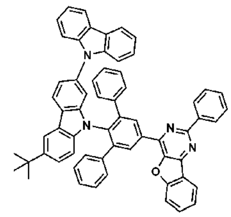
506
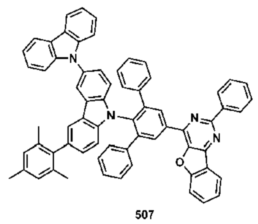
507
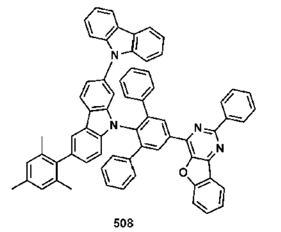
508
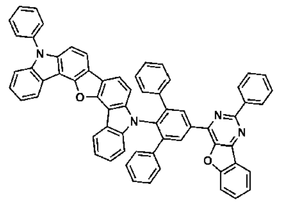
509
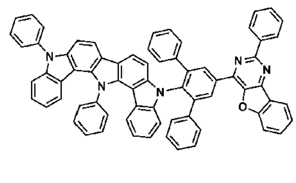
510
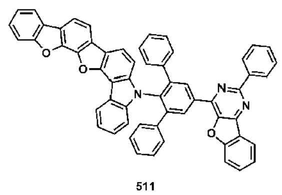
511
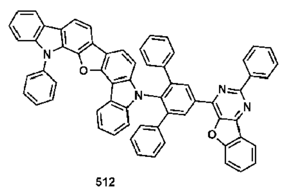
512
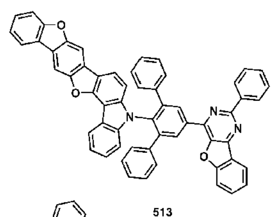
513
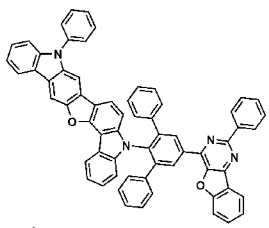
514
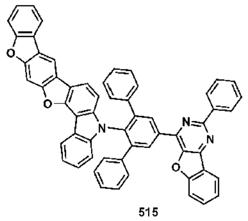
515
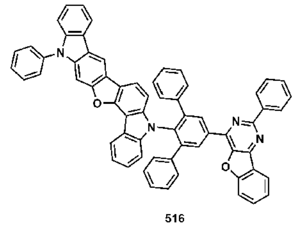
516
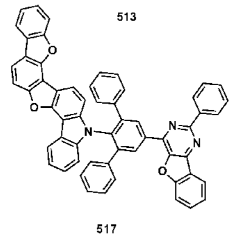
517
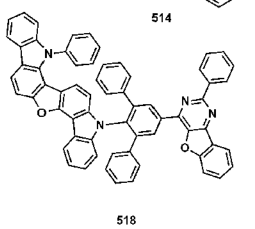
518

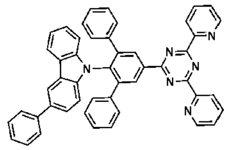
519

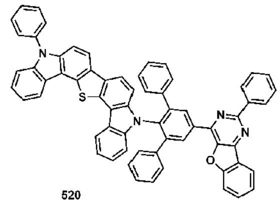
520

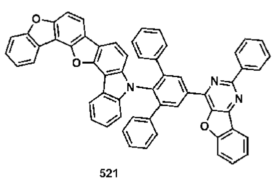
521

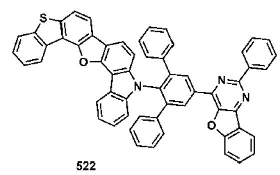
522

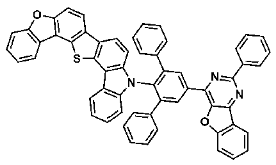
523

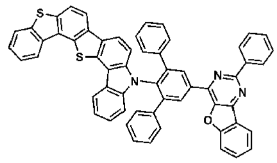
524

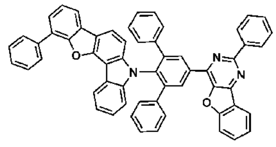
525

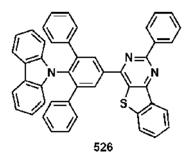
526

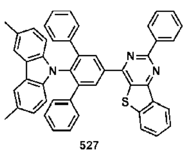
527

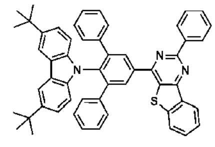
528

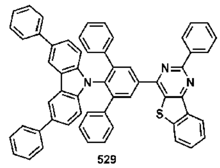
529

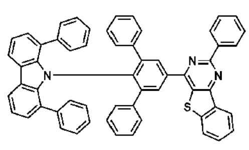
530

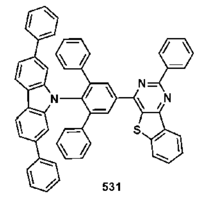
531

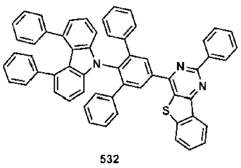
532

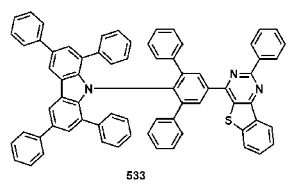
533

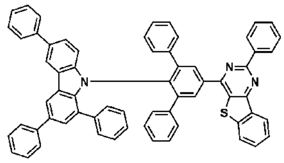
534

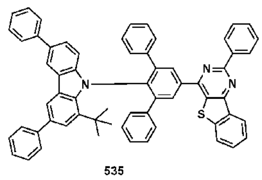
535

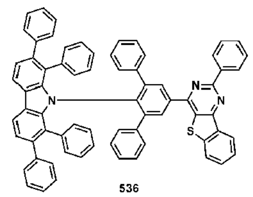
536

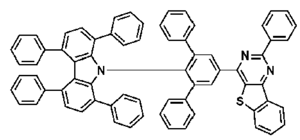
537

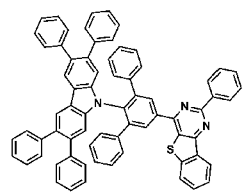
538

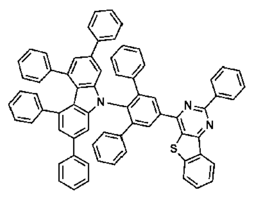
539

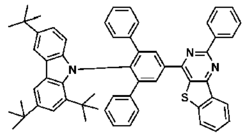
540

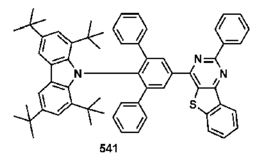
541

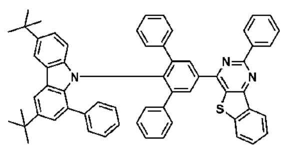
542

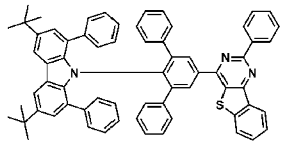
543

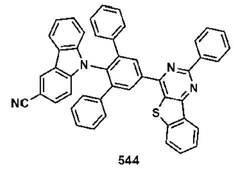
544

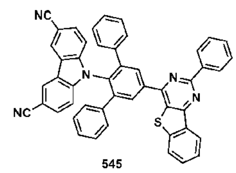
545

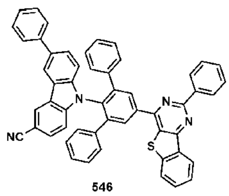
546

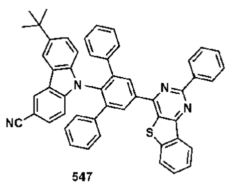
547

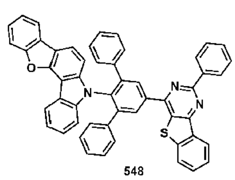
548

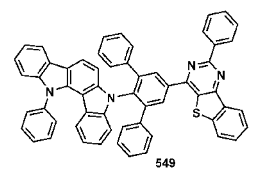
549

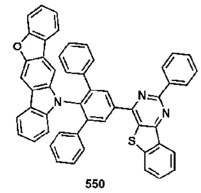
550
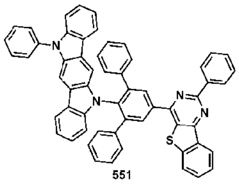
551
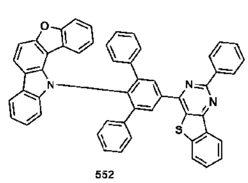
552
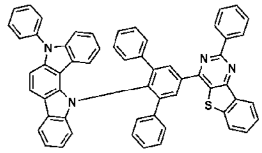
553
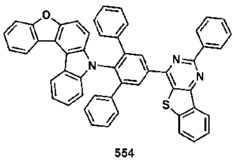
554
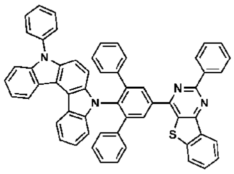
555
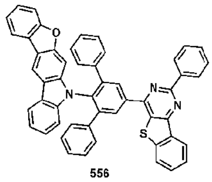
556
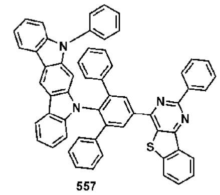
557
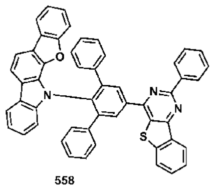
558
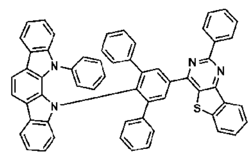
559
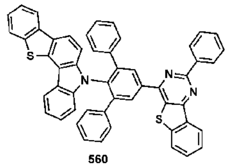
560
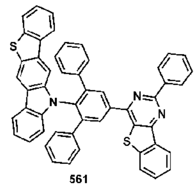
561
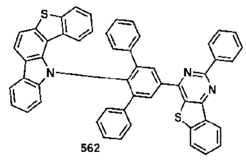
562
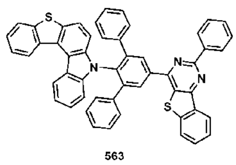
563
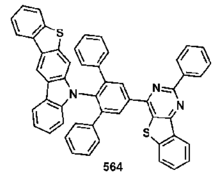
564
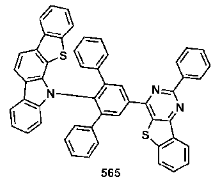
565
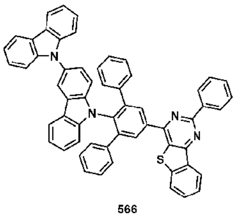
566
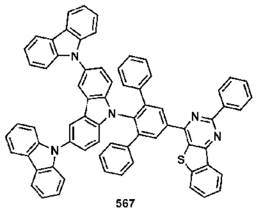
567
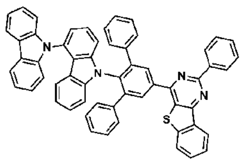
568
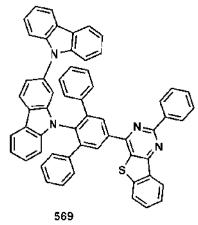
569
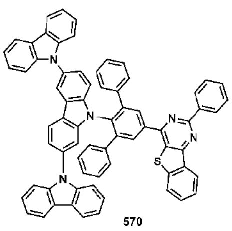
570
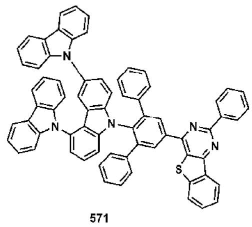
571
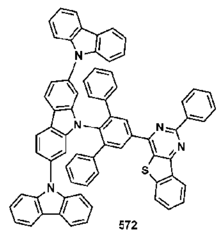
572
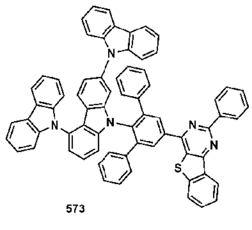
573
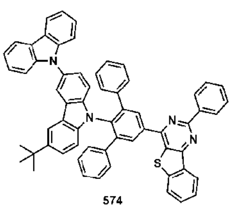
574
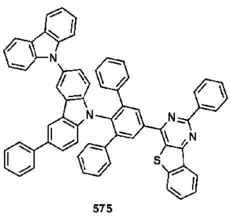
575
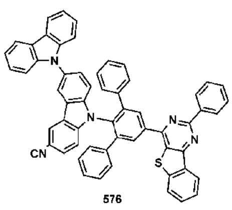
576
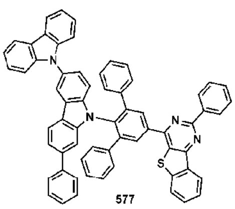
577
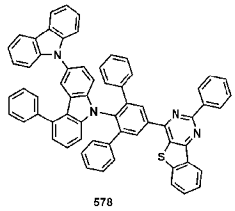
578
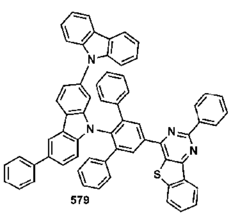
579
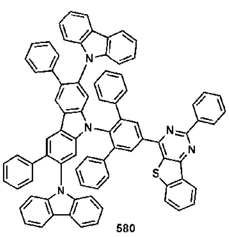
580
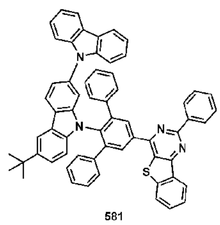
581

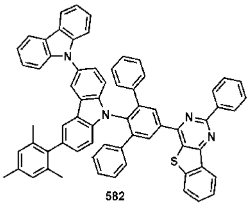
582

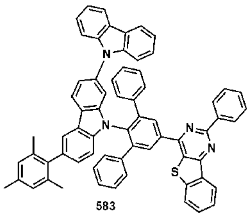
583

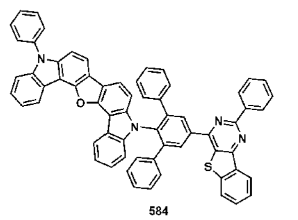
584

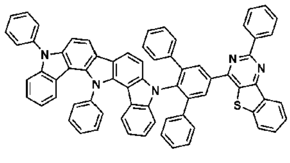
585

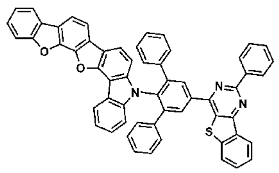
586

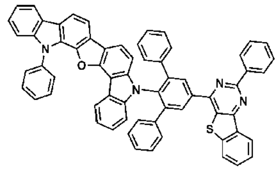
587

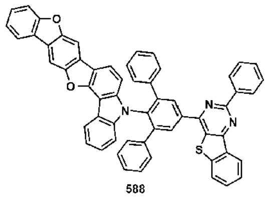
588

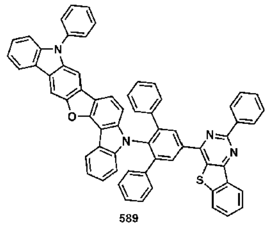
589

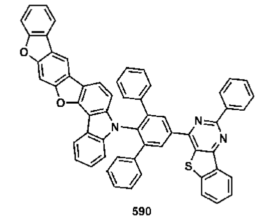
590

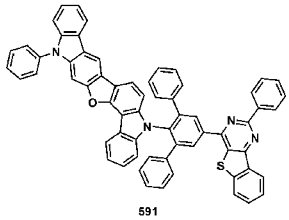
591

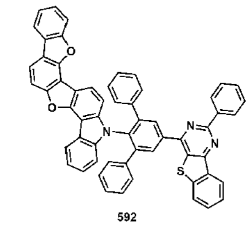
592

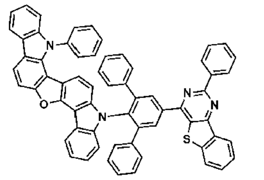
593

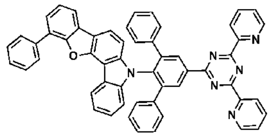
594

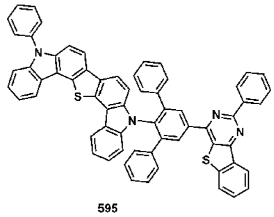
595

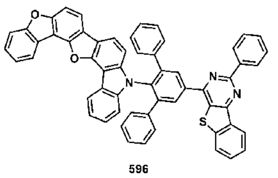
596

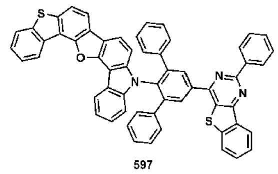
597

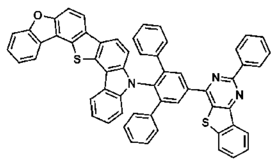
598

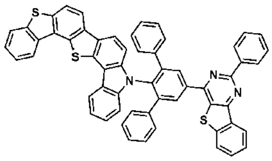
599

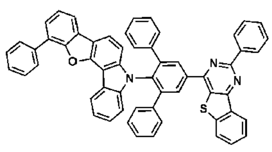
600

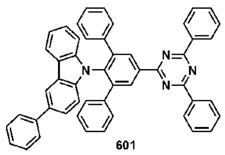
601

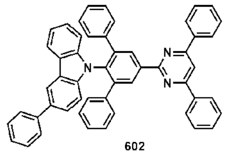
602

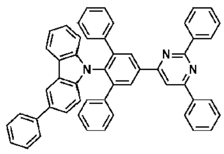
603

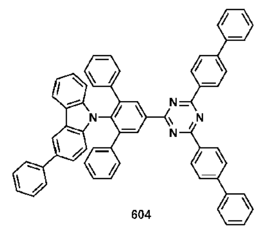
604

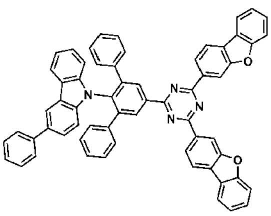
605

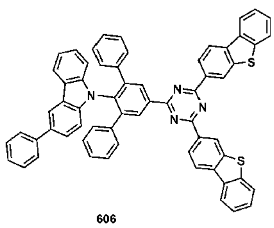
606

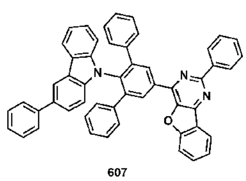
607

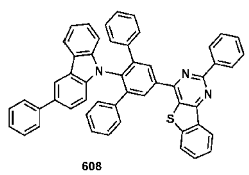
608

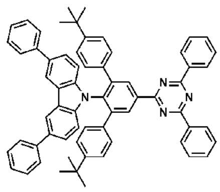
609

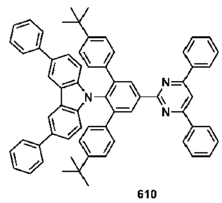
610

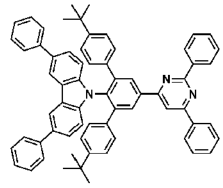
611

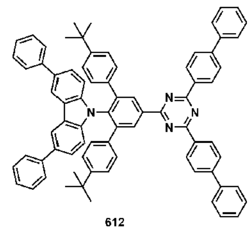
612

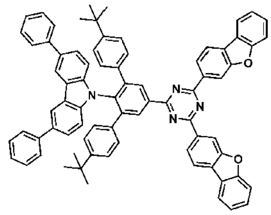
613

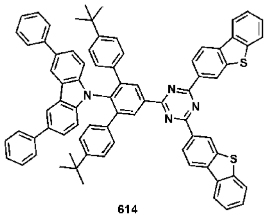
614

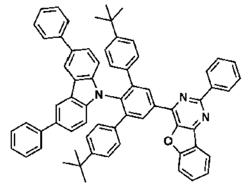
615

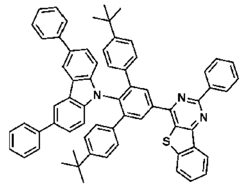
616

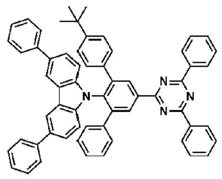
617

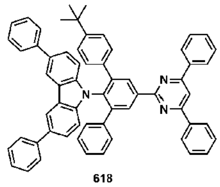
618

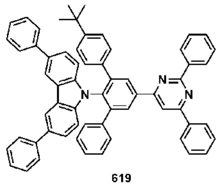
619

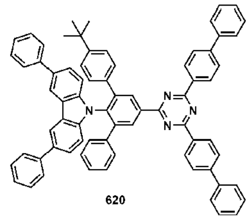
620

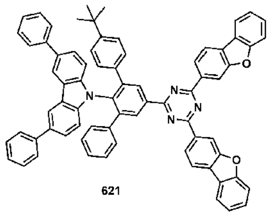
621

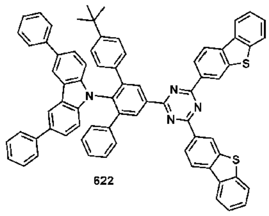
622

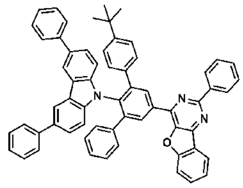
623

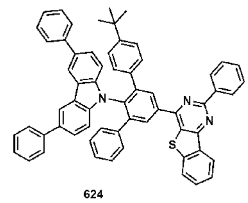
624

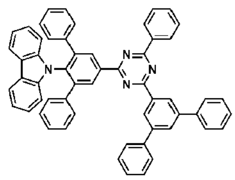
625

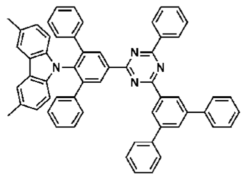
626

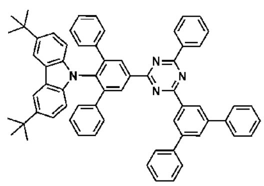
627

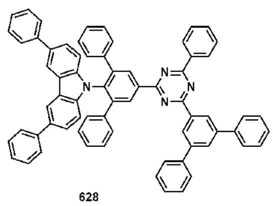
628

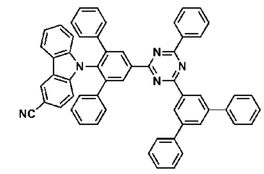
629

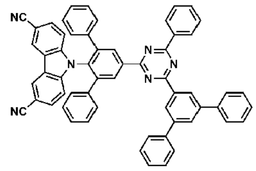
630

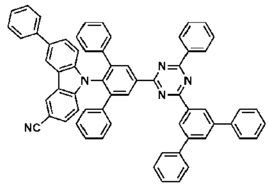
631

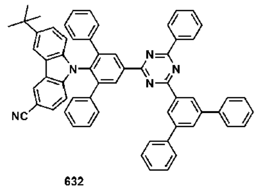
632

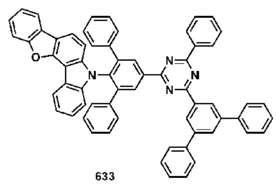
633

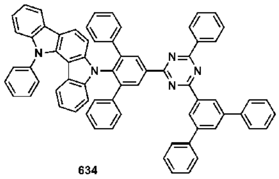
634

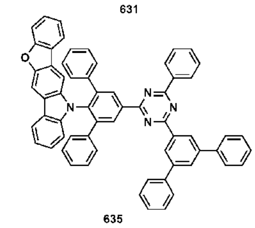
635

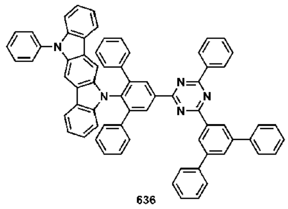
636

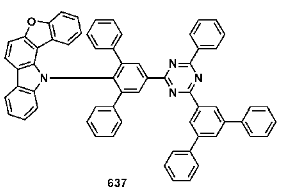
637

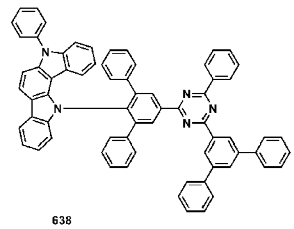
638

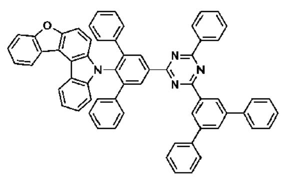
639

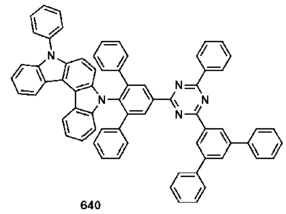
640

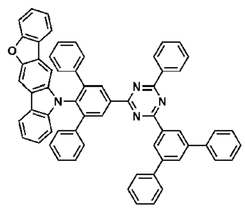
641

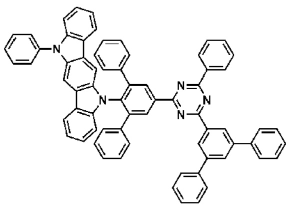
642

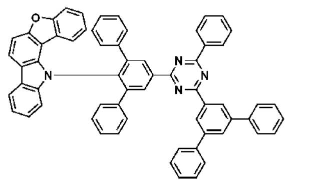
643

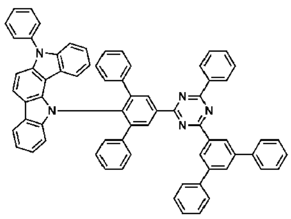
644

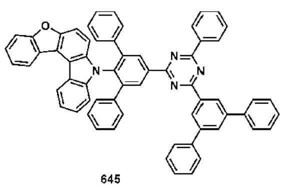
645

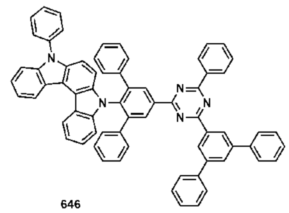
646

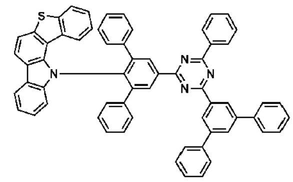
647

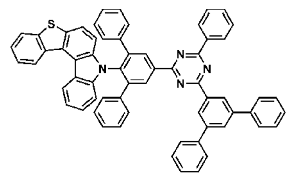
648

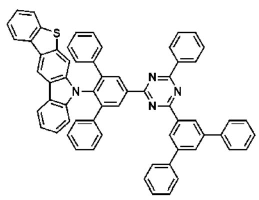

649

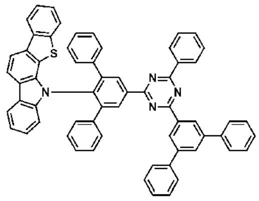

650

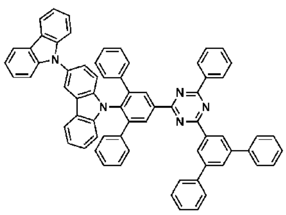

651

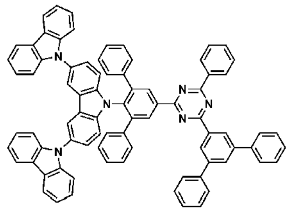

652

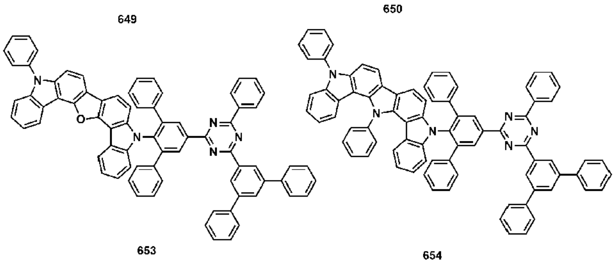

653

654

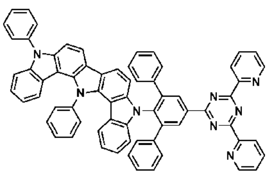

655

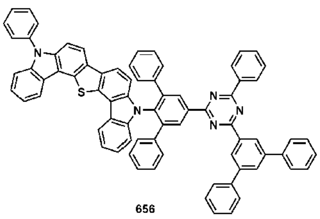

656

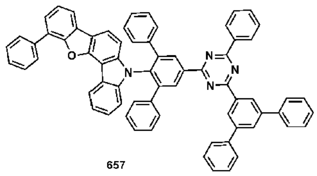

657

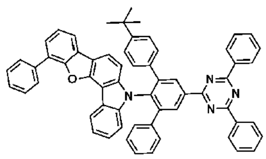

658

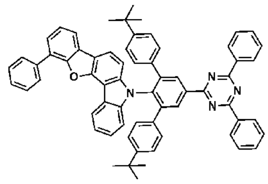

659

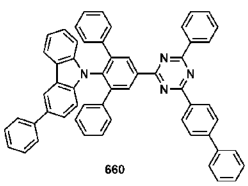

660

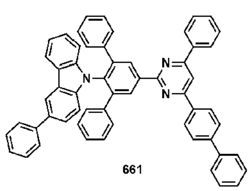

661

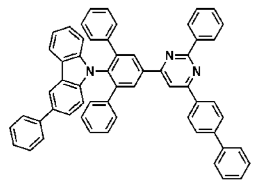

662

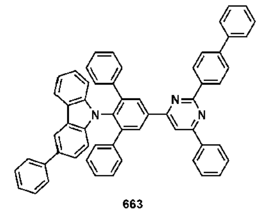

663

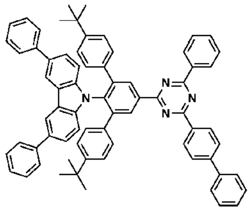

664

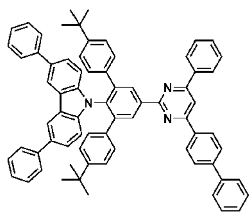

665

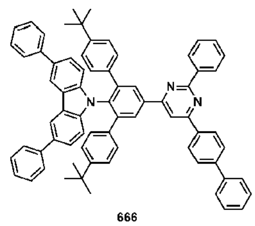

666

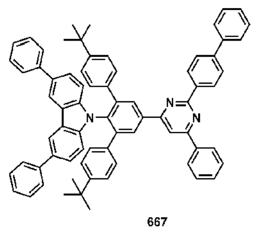

667

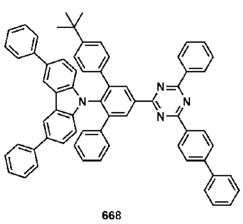
668

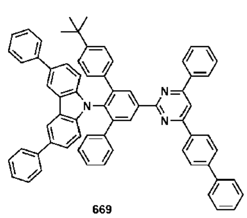
669

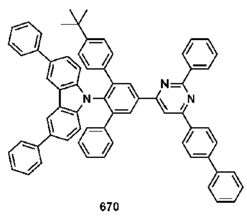
670

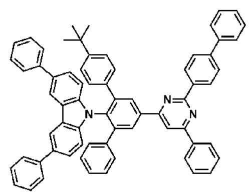
671

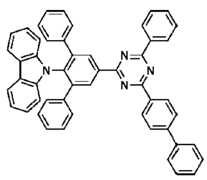
672

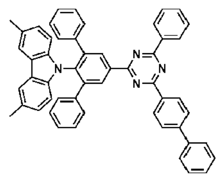
673

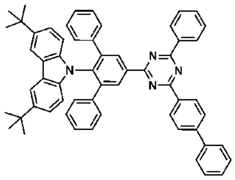
674

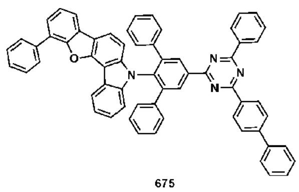
675

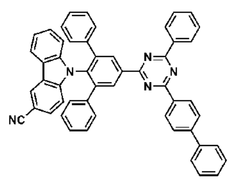
676

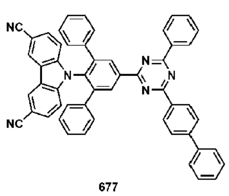
677

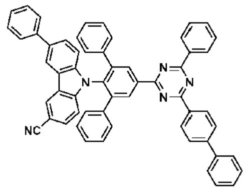
678

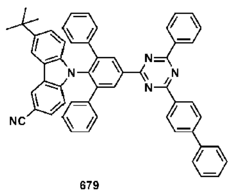
679

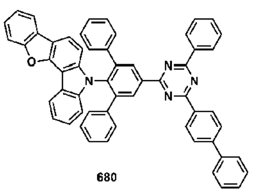
680

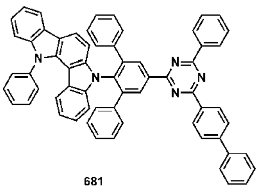
681

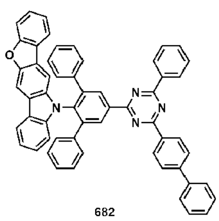
682

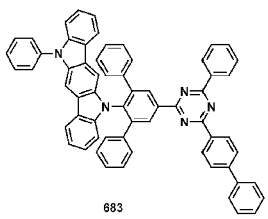
683

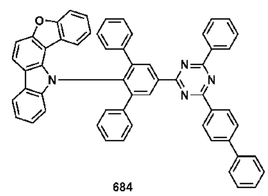
684

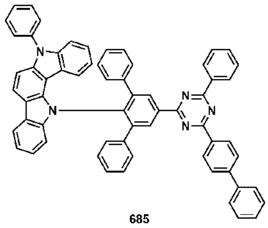
685

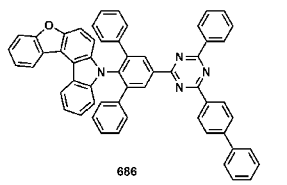
686

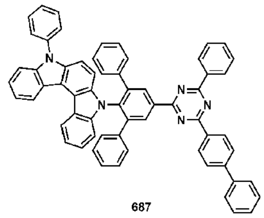
687

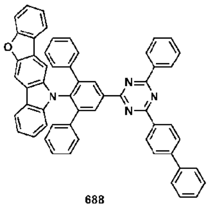
688

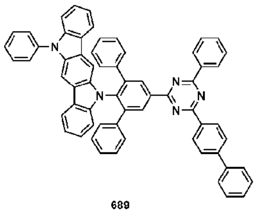
689

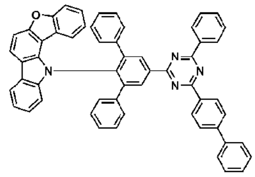
690

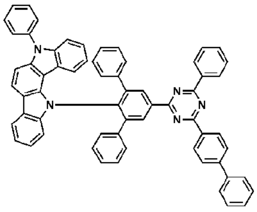
691

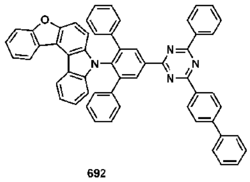
692

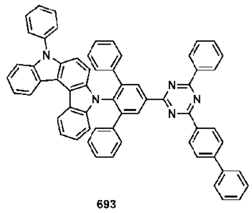
693

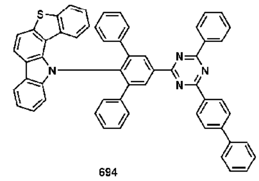
694

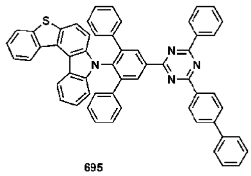
695

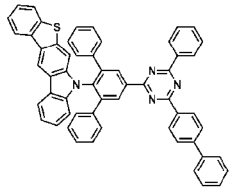
696

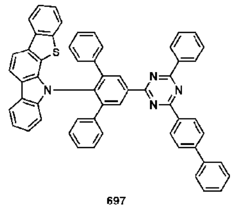
697

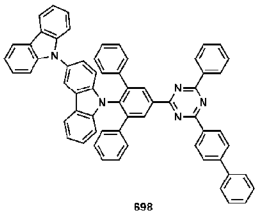
698

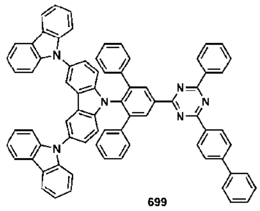
699

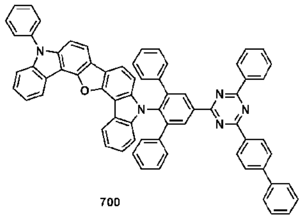
700

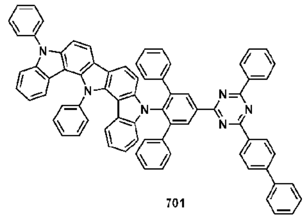
701

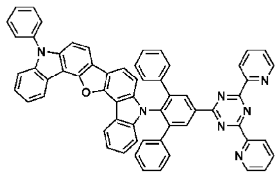
702

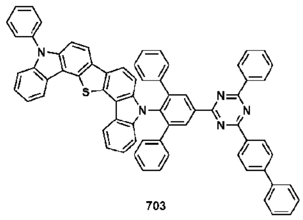
703

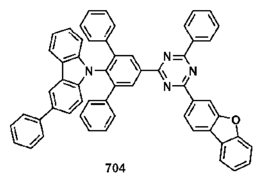
704

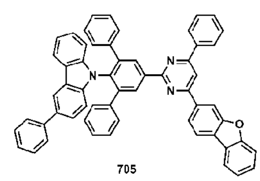
705

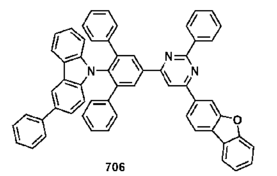
706

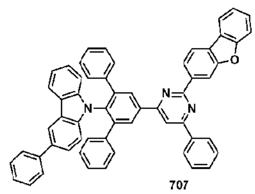
707

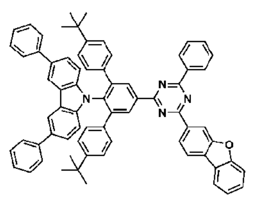
708

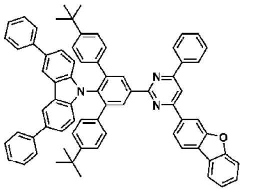
709

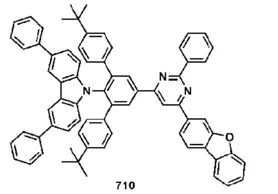
710

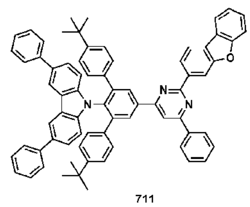
711

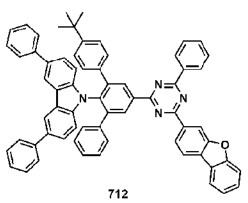
712

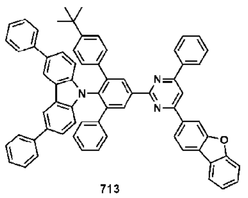
713

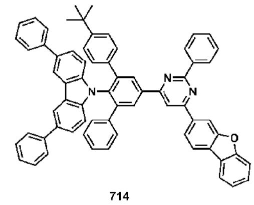
714

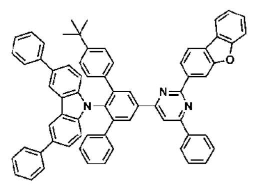
715

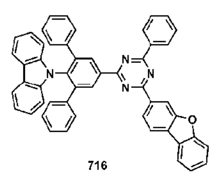
716

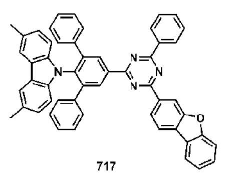
717

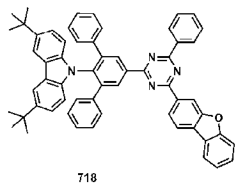
718

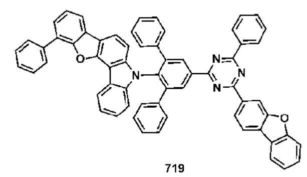
719

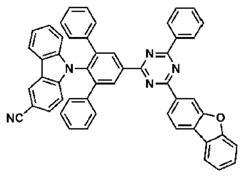

720

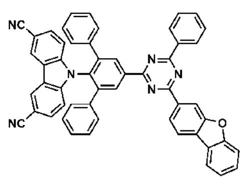

721

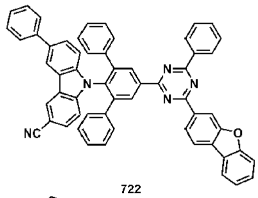

722

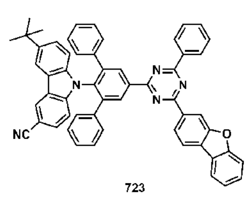

723

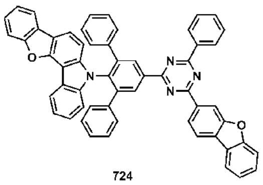

724

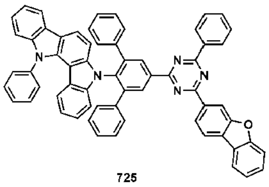

725

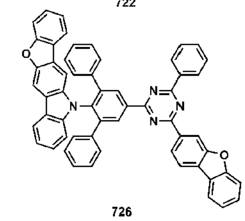

726

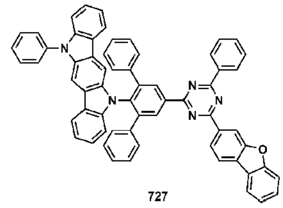

727

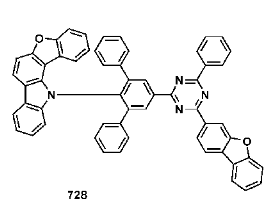

728

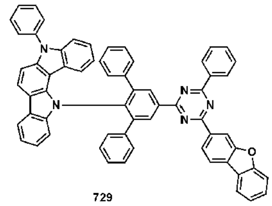

729

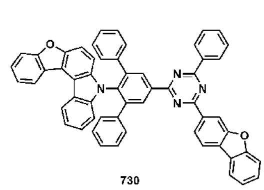

730

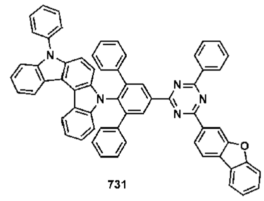

731

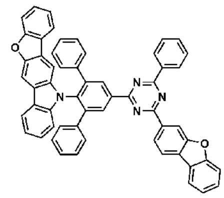

732

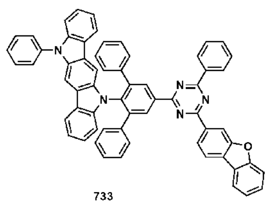

733

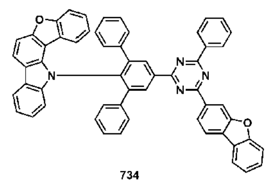

734

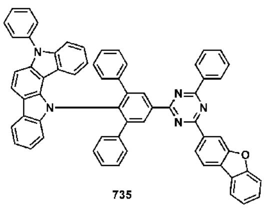

735

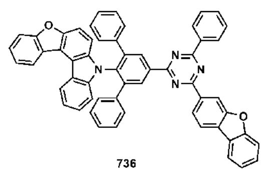

736

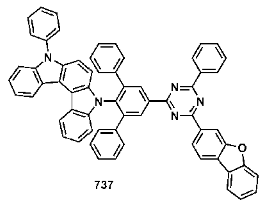

737

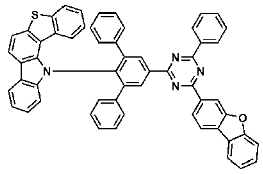

738

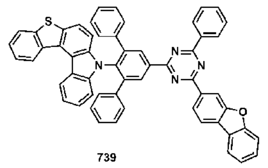

739

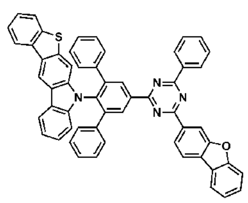

740

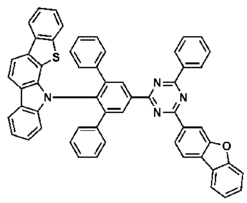

741

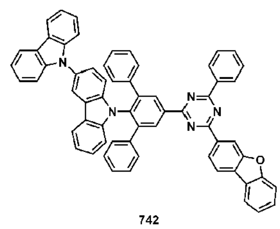

742

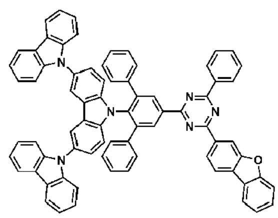

743

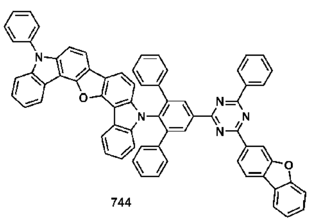

744

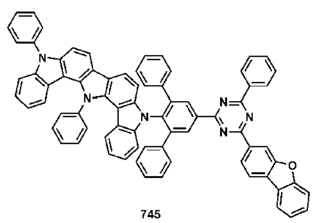

745

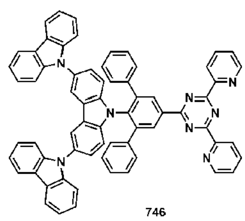

746

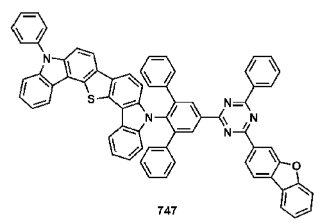

747

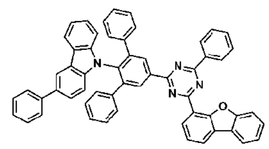
748
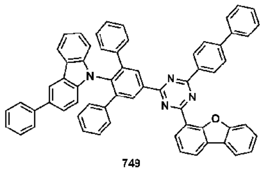
749
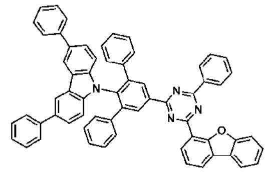
750
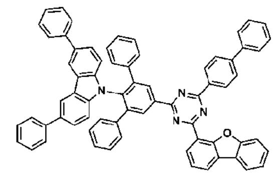
751

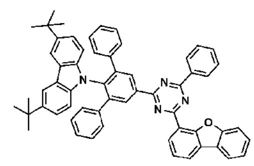
752
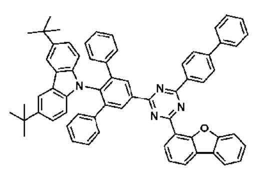
753
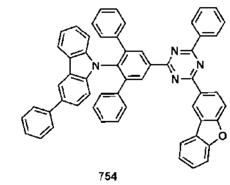
754
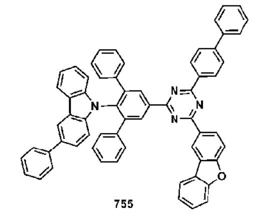
755

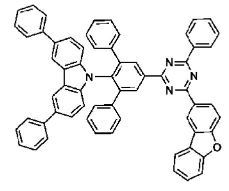
756
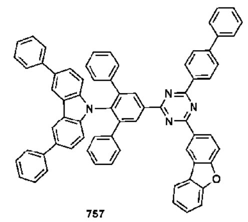
757
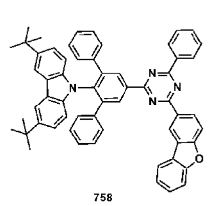
758
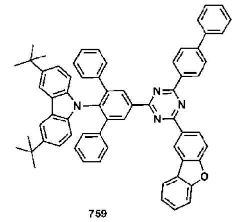
759

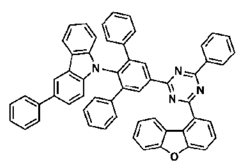
760
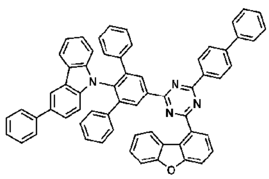
761
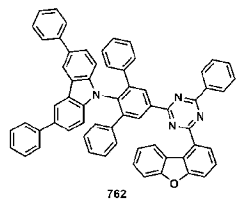
762
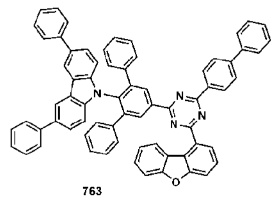
763

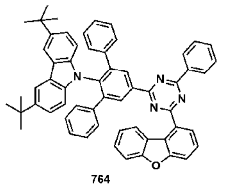
764
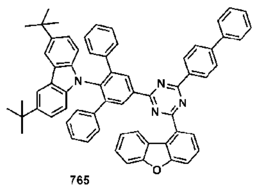
765
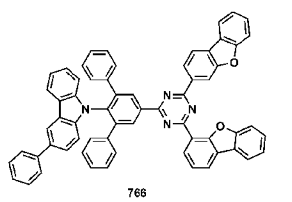
766
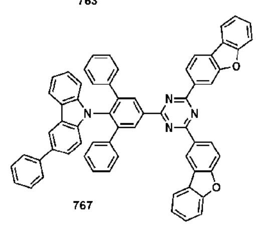
767

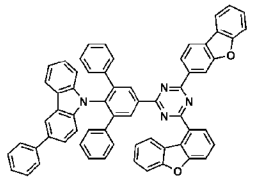
768
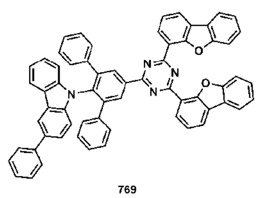
769
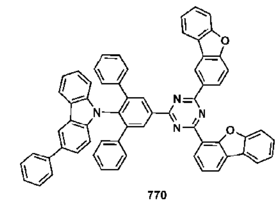
770
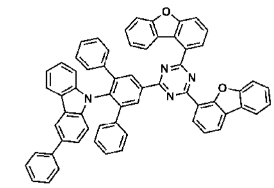
771

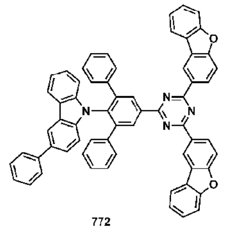
772
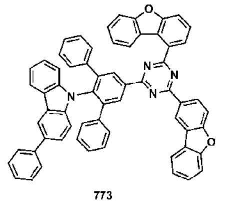
773
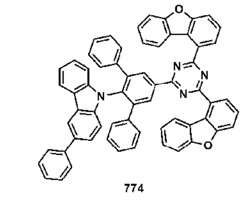
774
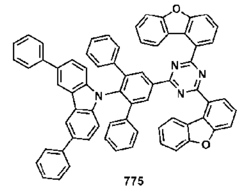
775

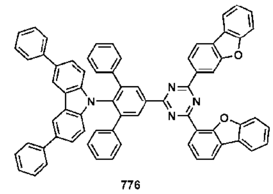
776
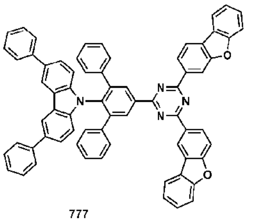
777
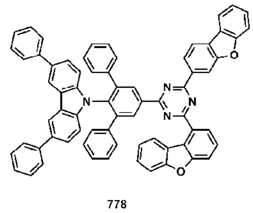
778
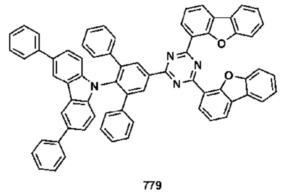
779

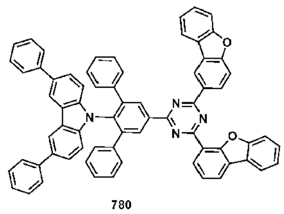
780
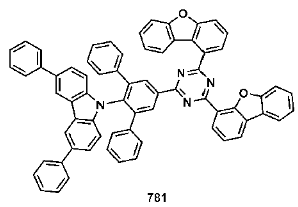
781
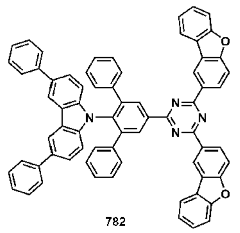
782
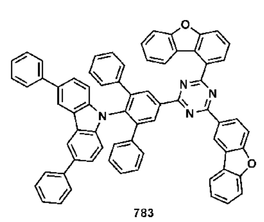
783
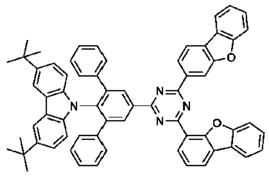
784
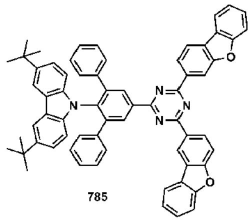
785
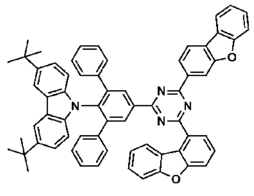
786
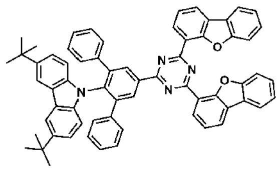
787
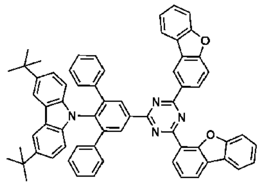
788
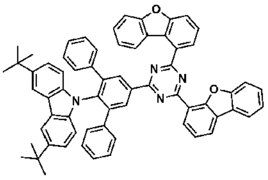
789
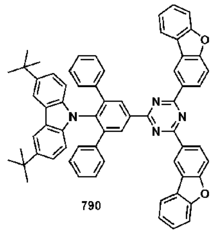
790
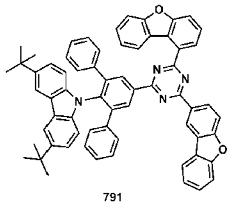
791
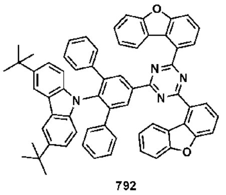
792
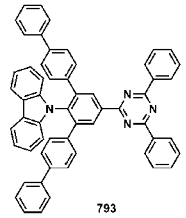
793
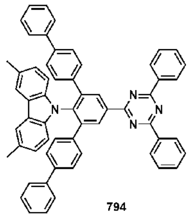
794
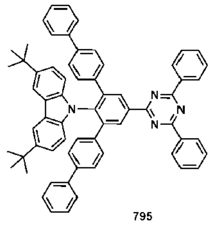
795
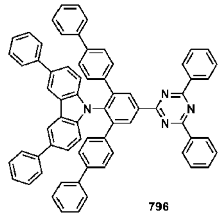
796
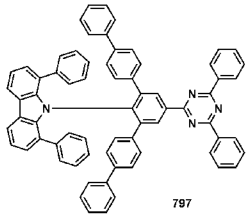
797
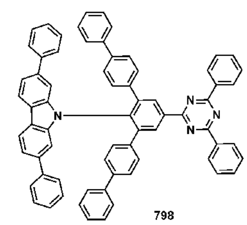
798
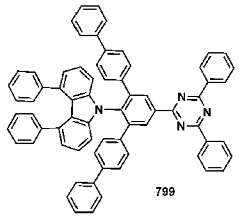
799
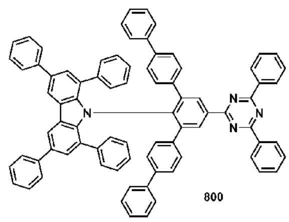
800
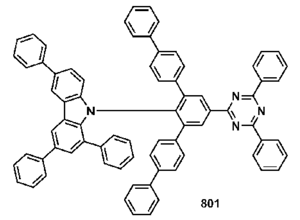
801
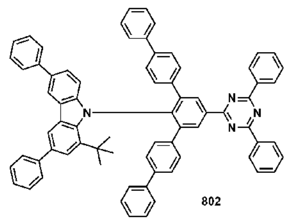
802
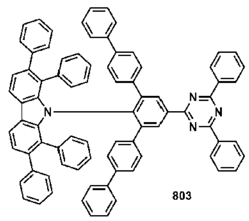
803
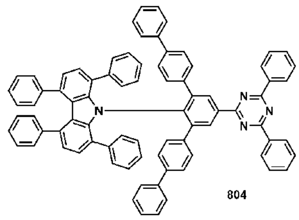
804
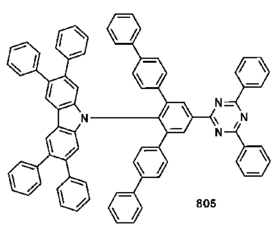
805
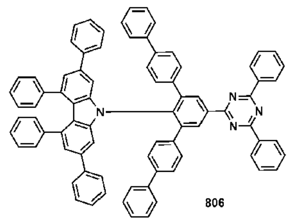
806
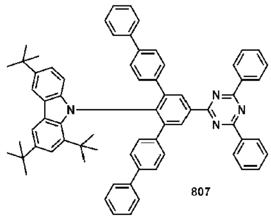
807
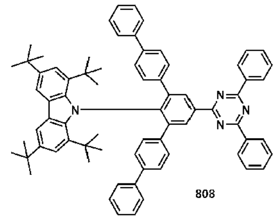
808

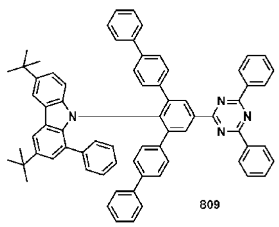
809

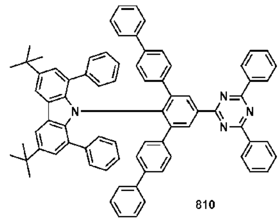
810

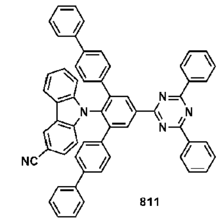
811

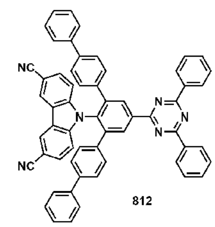
812

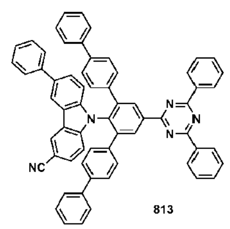
813

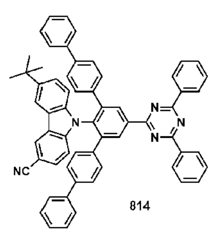
814

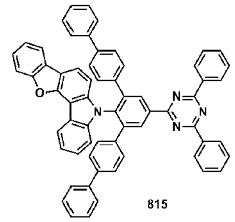
815

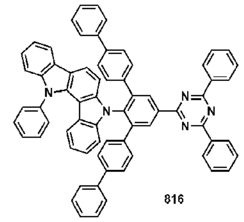
816

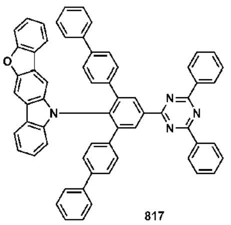
817

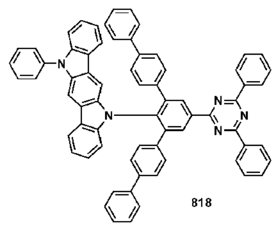
818

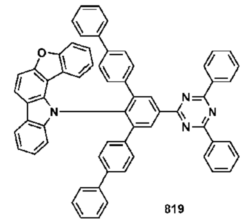
819

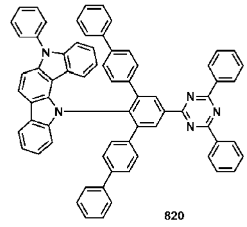
820

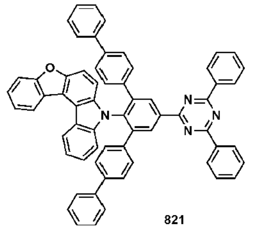
821

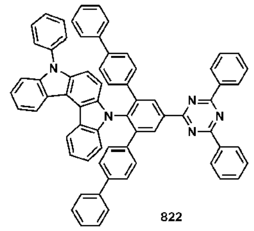
822

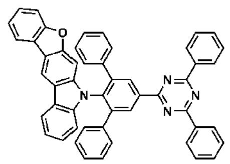
823

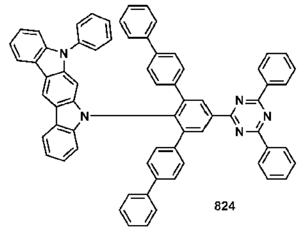
824

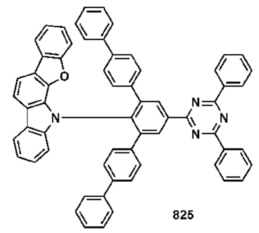
825

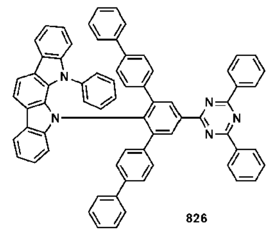
826

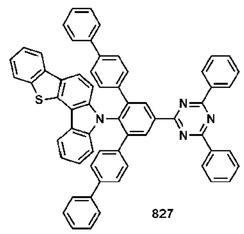
827

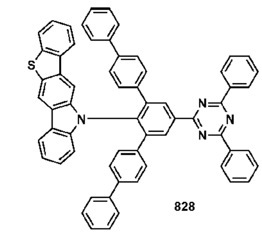
828

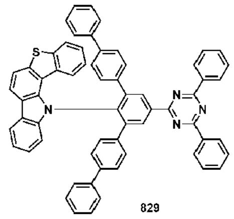
829

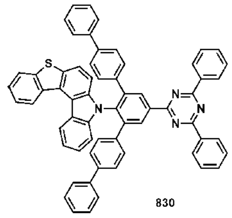
830

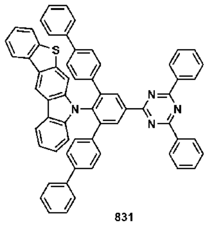
831

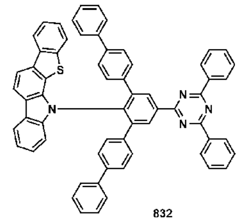
832

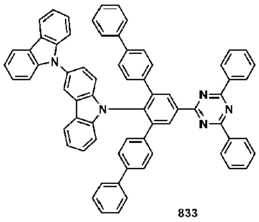
833

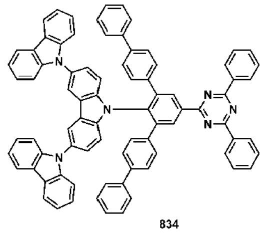
834

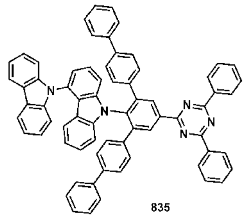
835

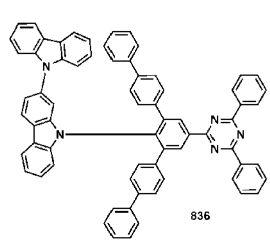
836

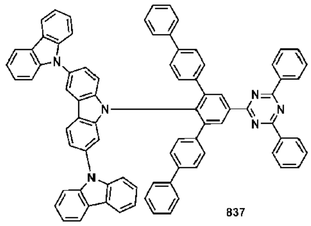
837

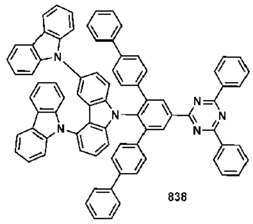
838

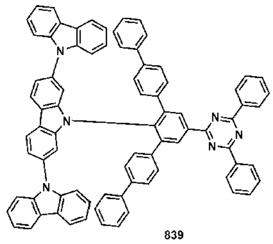
839

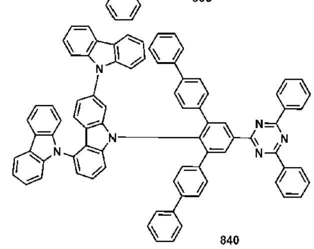
840

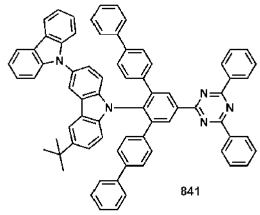
841

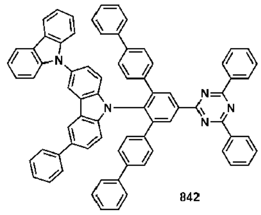
842

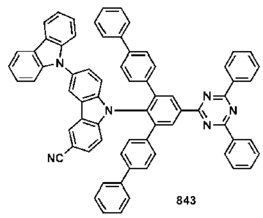
843

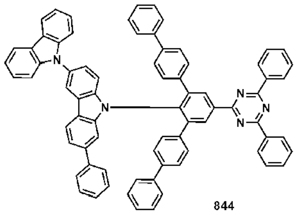
844

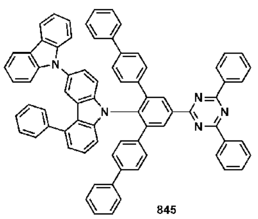
845

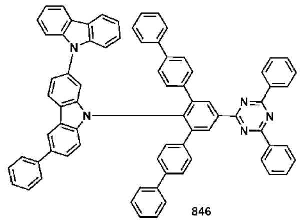
846

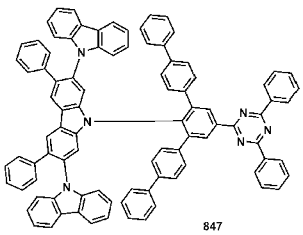
847

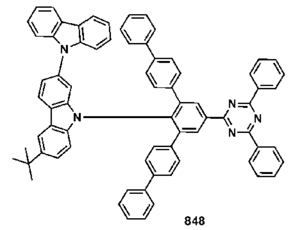
848

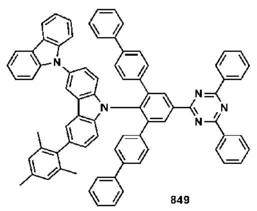
849

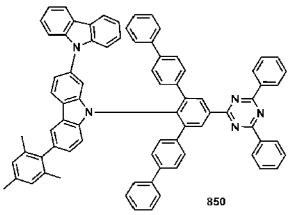
850

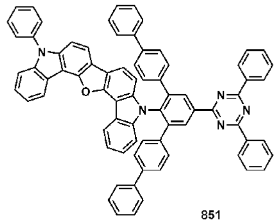
851

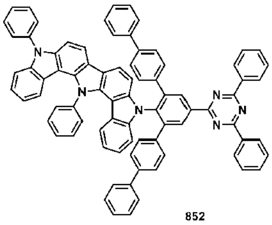
852

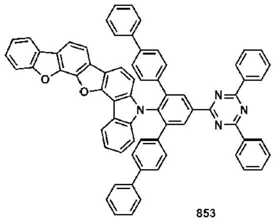
853

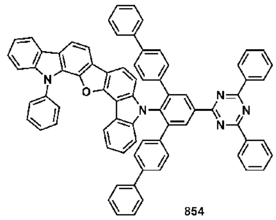
854

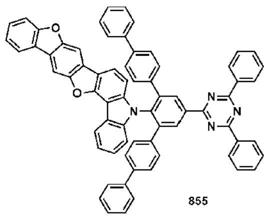
855

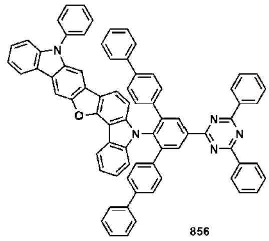
856

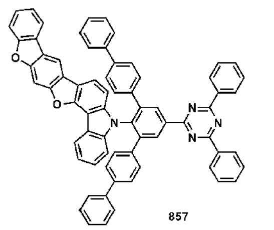
857

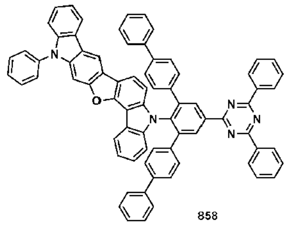
858

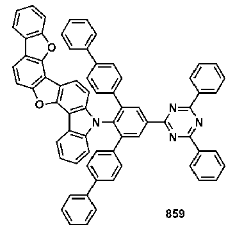
859

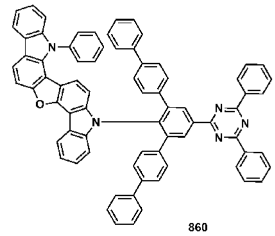
860

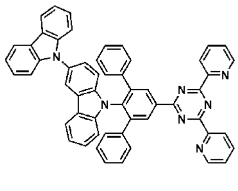
861

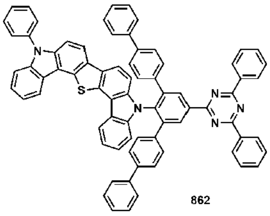
862

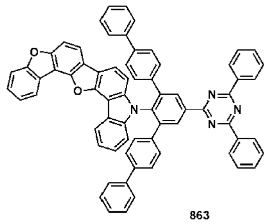
863

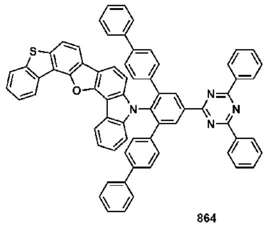
864

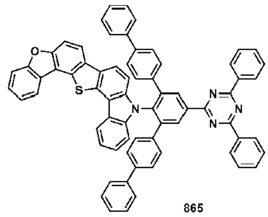
865

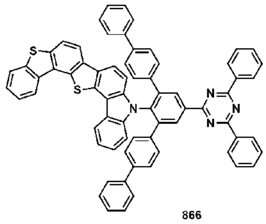
866

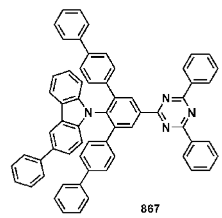
867

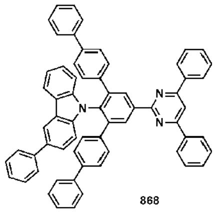
868

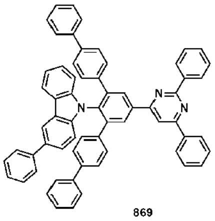
869

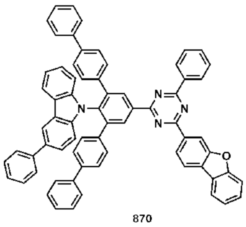
870

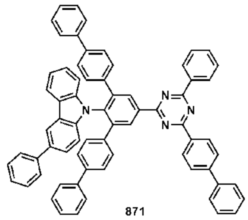
871

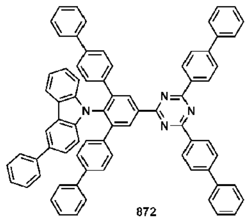
872

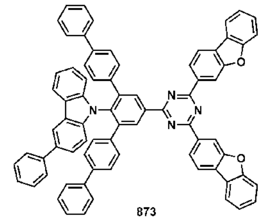
873

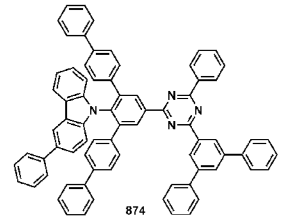
874

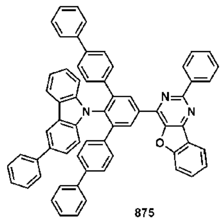
875

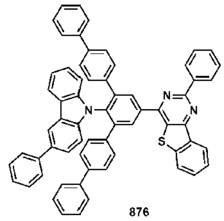
876

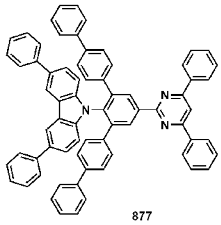
877

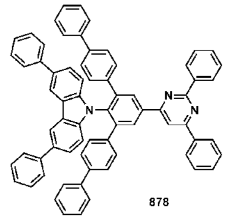
878

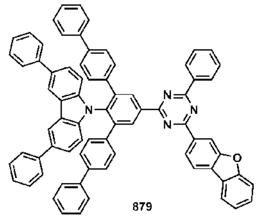
879

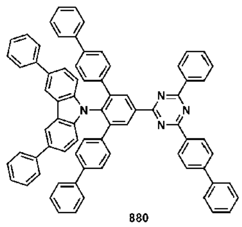
880

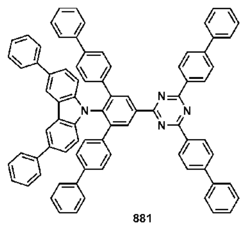
881

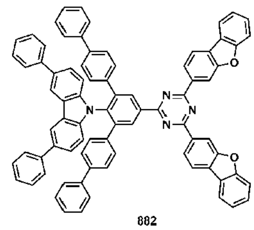
882

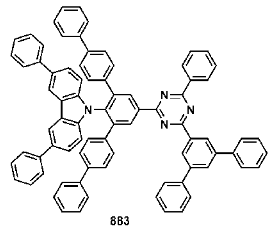
883

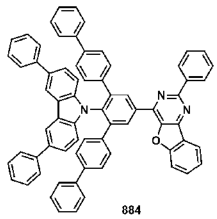
884

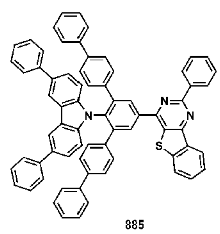
885

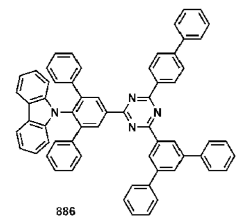
886

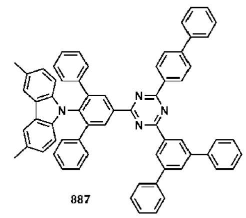
887

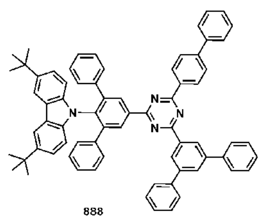
888

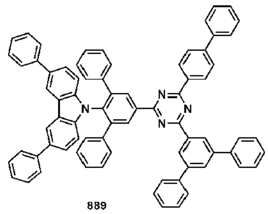
889

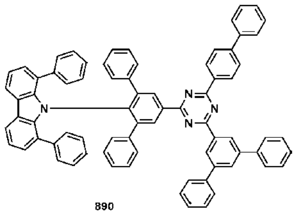
890

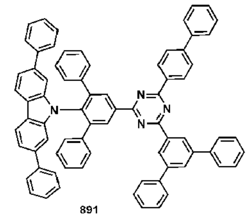
891

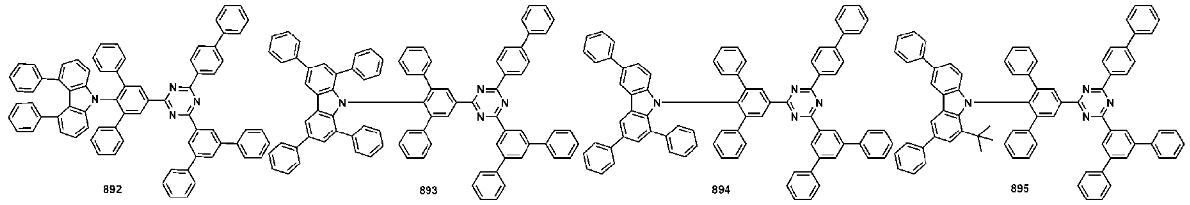

892 893 894 895

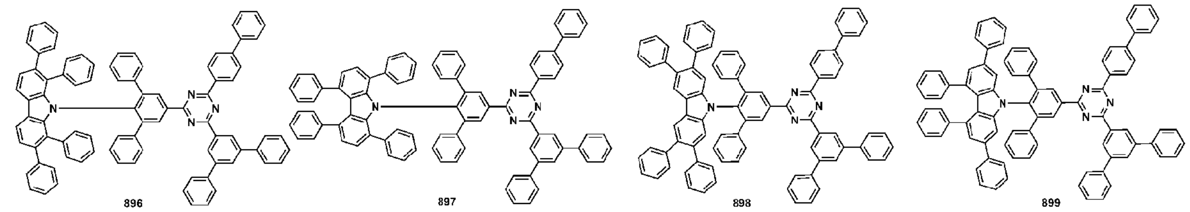

896 897 898 899

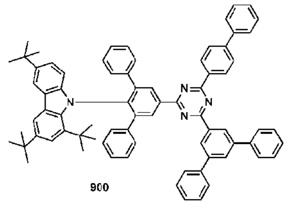

900

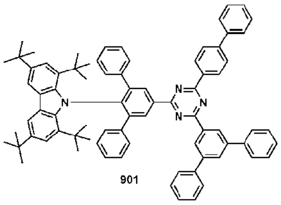

901

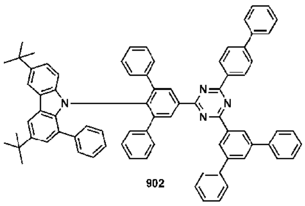

902

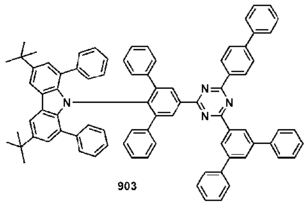

903

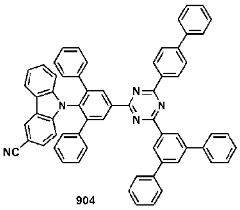

904

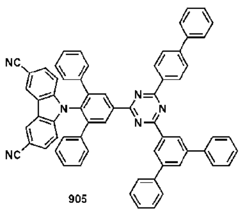

905

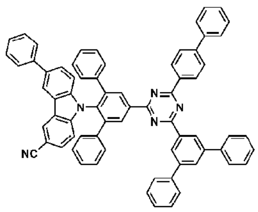

906

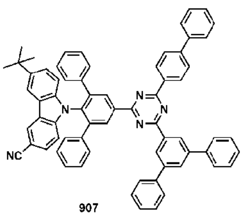

907

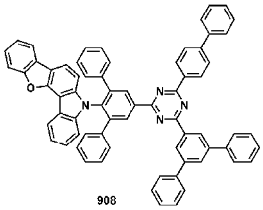

908

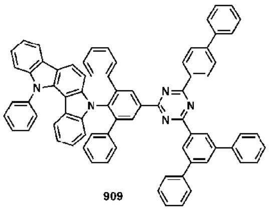

909

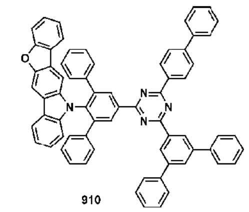

910

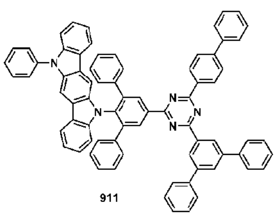

911

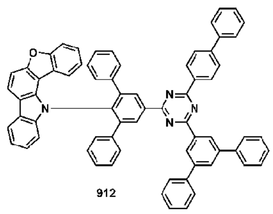

912

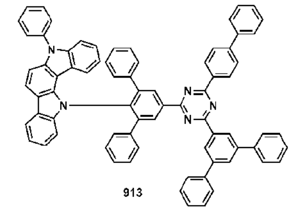

913

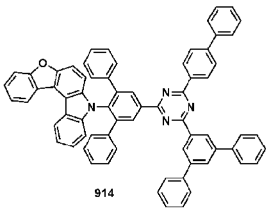

914

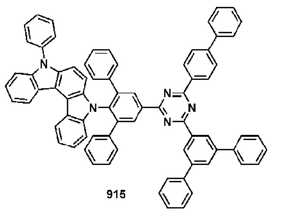

915

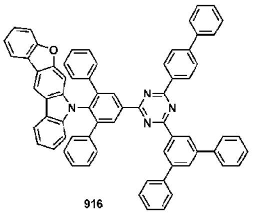

916

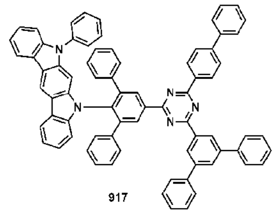

917

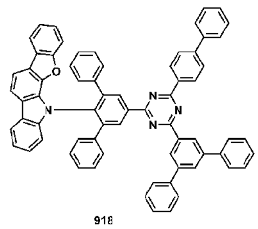

918

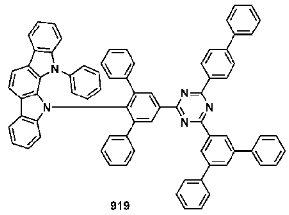

919

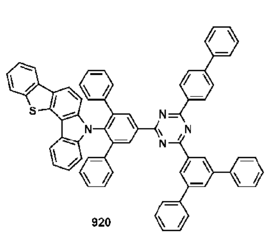

920

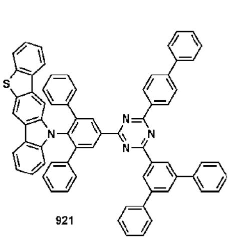

921

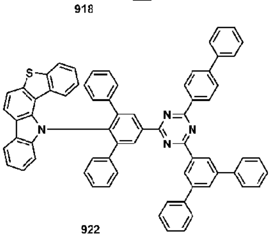

922

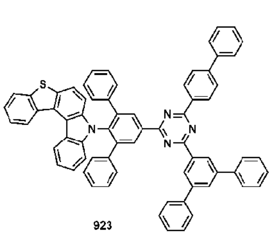

923

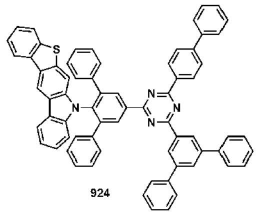
924

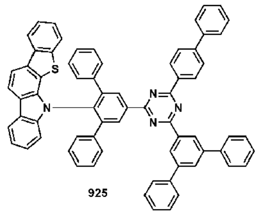
925

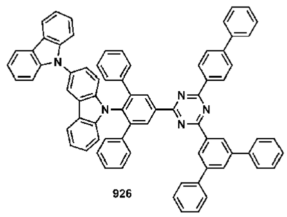
926

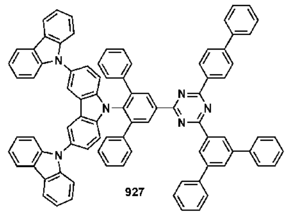
927

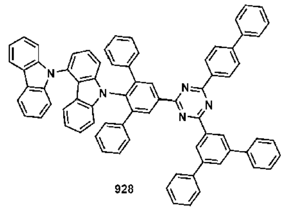
928

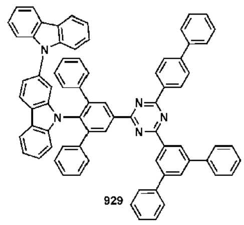
929

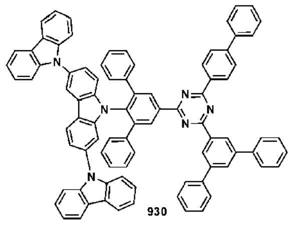
930

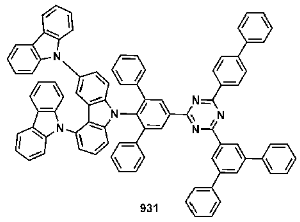
931

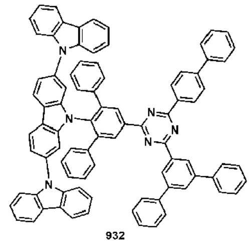
932

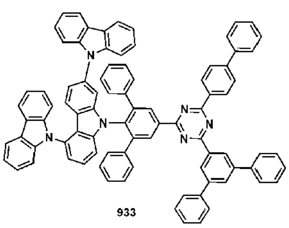
933

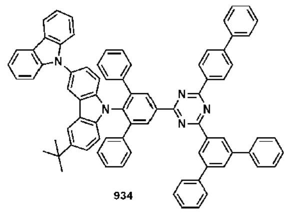
934

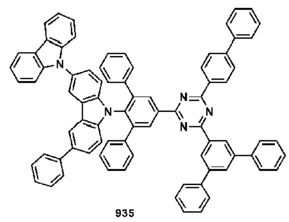
935

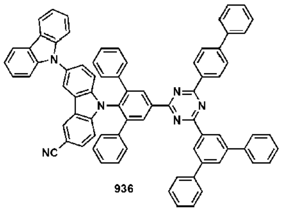
936

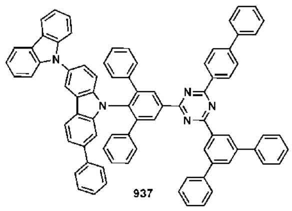
937

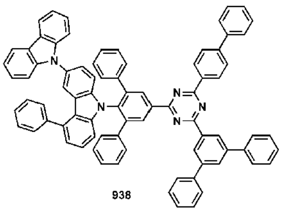
938

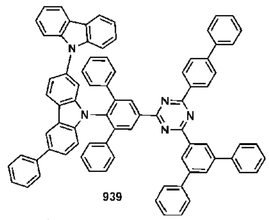
939

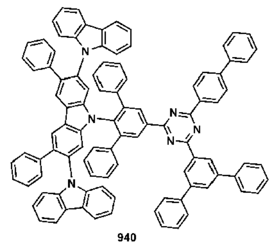
940

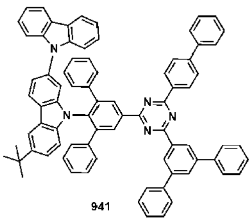
941

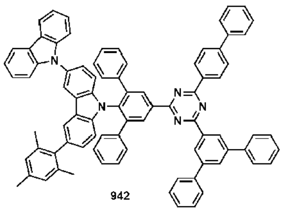
942

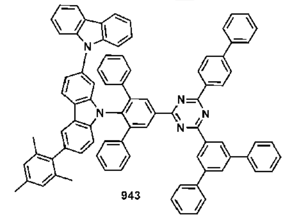
943

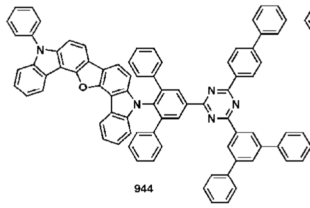
944

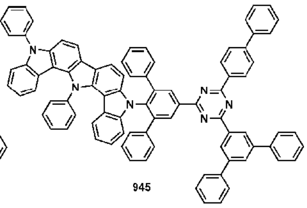
945

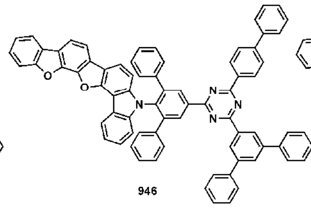
946

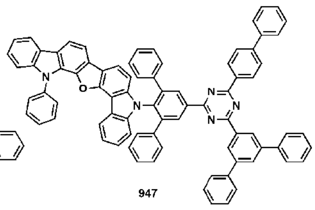
947

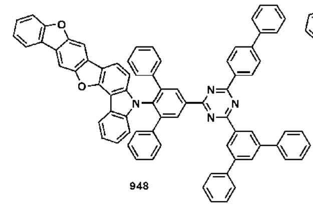
948

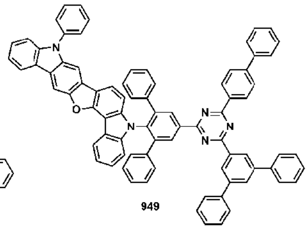
949

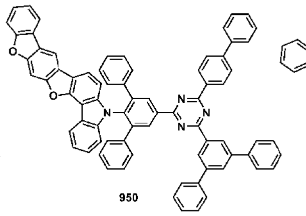
950

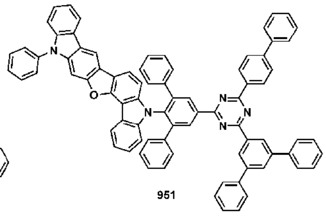
951

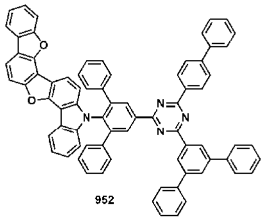
952

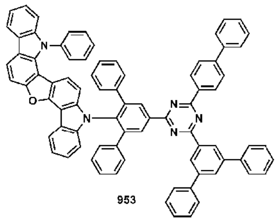
953

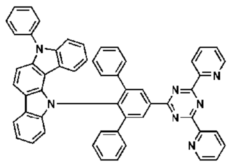
954

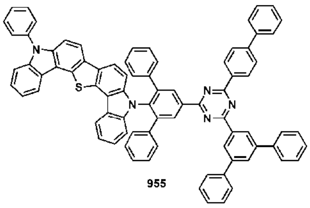
955

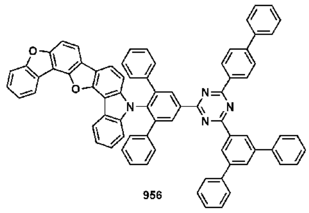
956

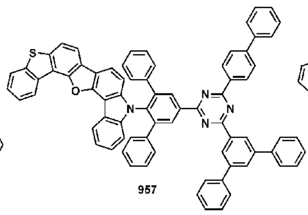
957

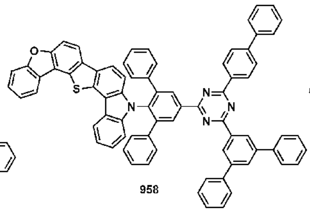
958

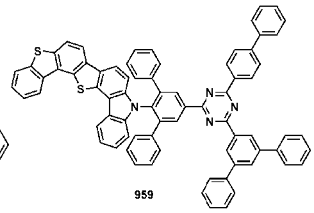
959

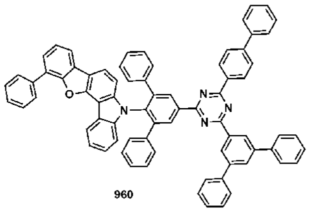
960

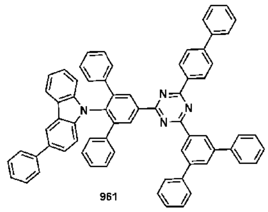
961

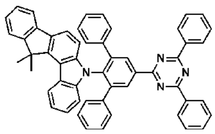
962

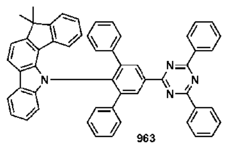
963

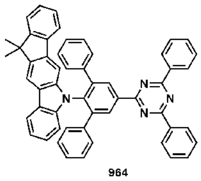
964

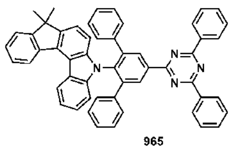
965

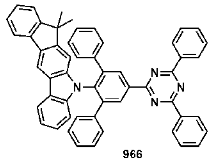
966

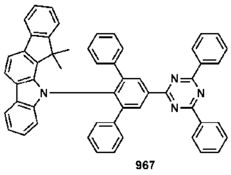
967

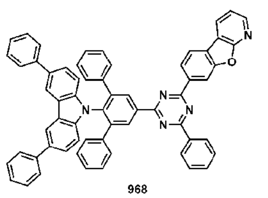
968

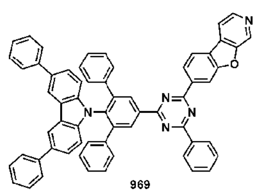
969

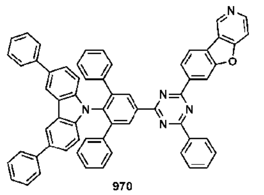
970

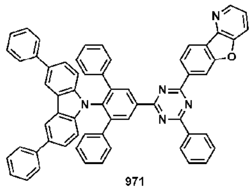
971

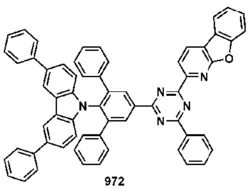
972

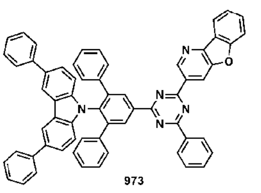
973

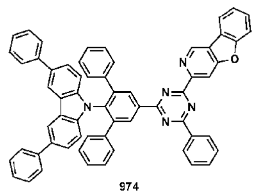
974

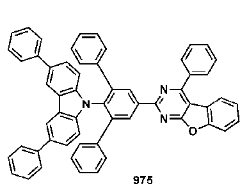
975

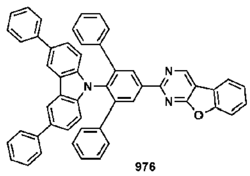
976

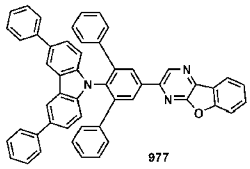
977

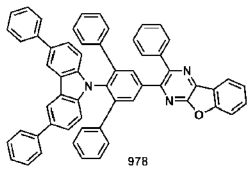
978

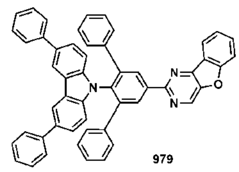
979

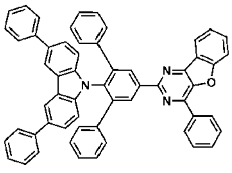
980

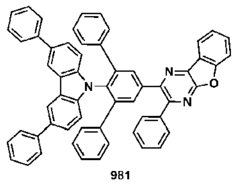
981

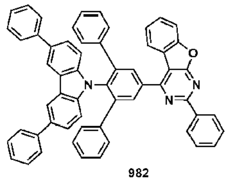
982

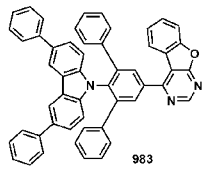
983

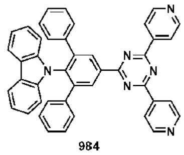
984

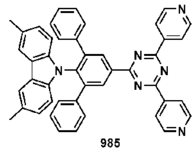
985

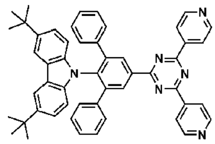
986

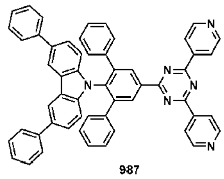
987

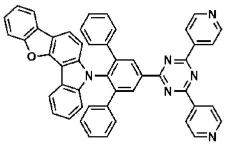
988

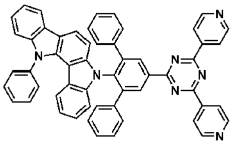
989

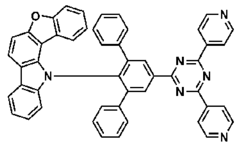
990

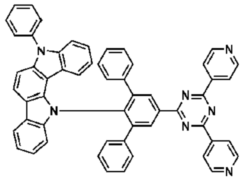
991

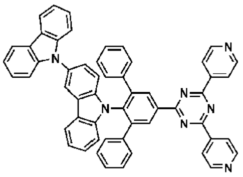
992

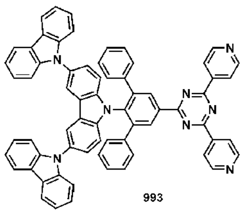
993

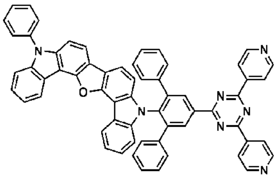
994

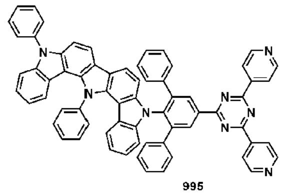
995

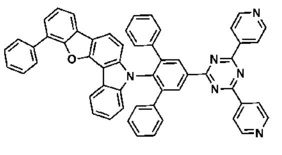

996

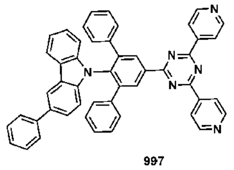

997

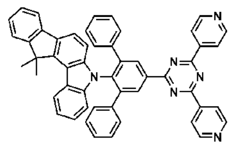

998

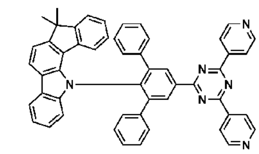

999

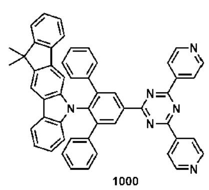

1000

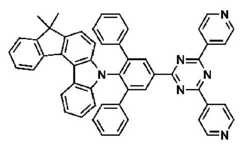

1001

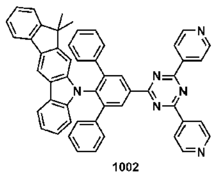

1002

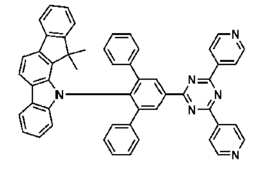

1003

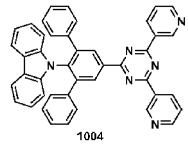

1004

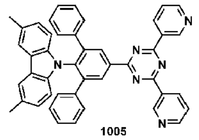

1005

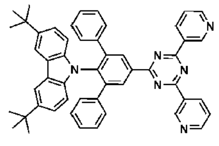

1006

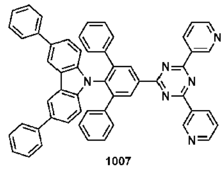

1007

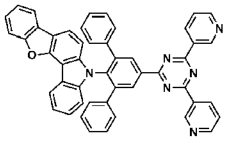

1008

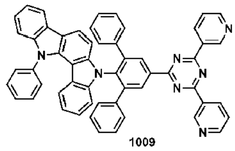

1009

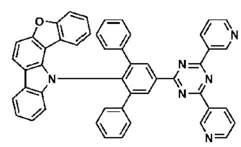

1010

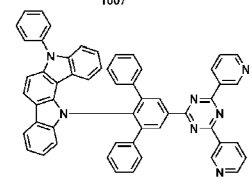

1011

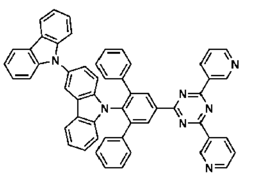

1012

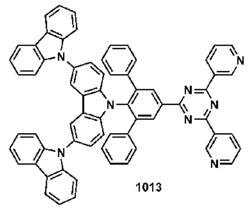

1013

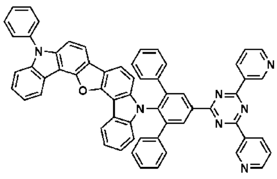

1014

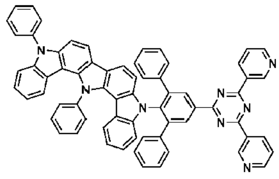

1015

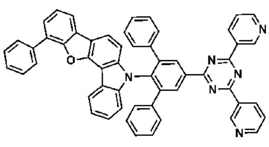

1016

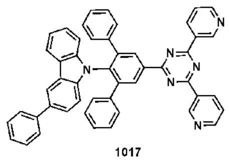

1017

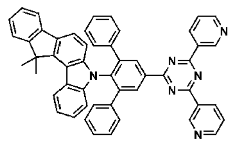

1018

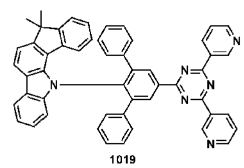

1019

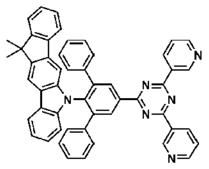

1020

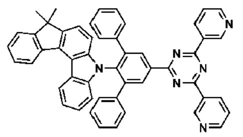

1021

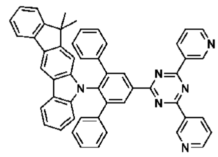

1022

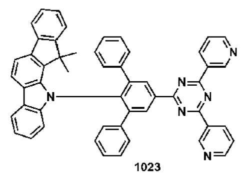

1023

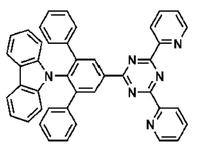

1024

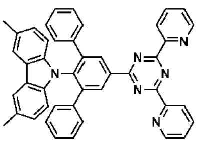

1025

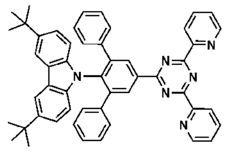

1026

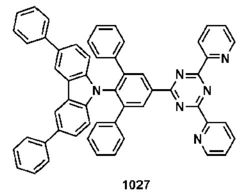

1027

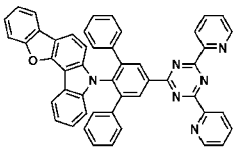

1028

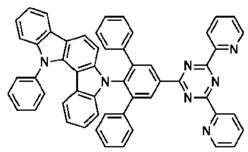

1029

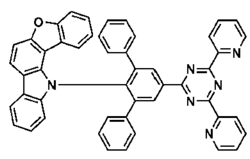

1030

Group X

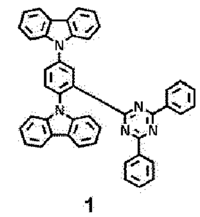

1

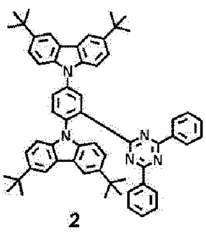

2

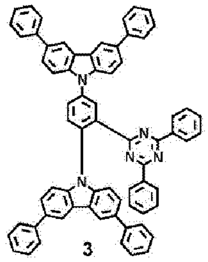

3

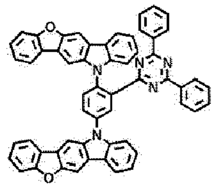

4

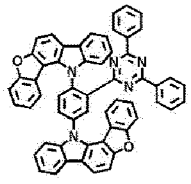

5

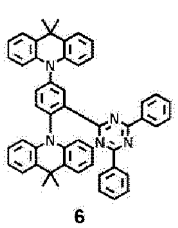

6

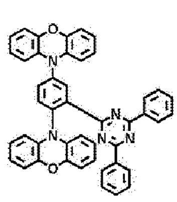

7

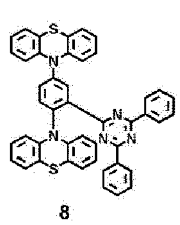

8

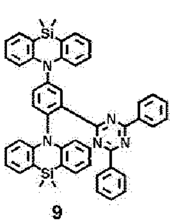

9

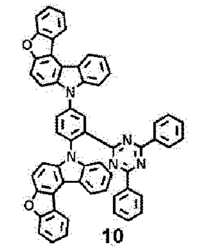

10

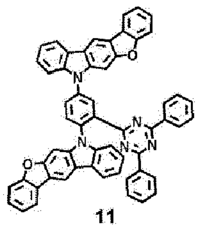

11

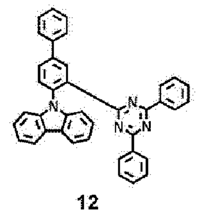

12

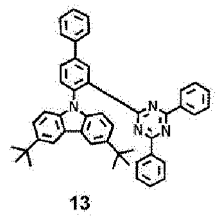

13

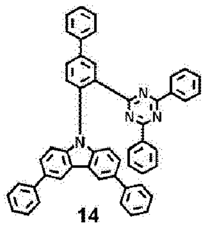

14

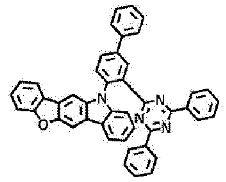

15

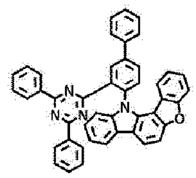

16

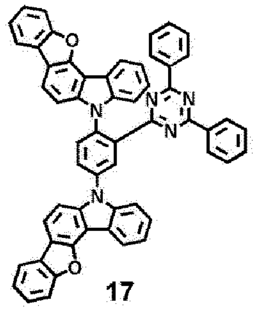

17

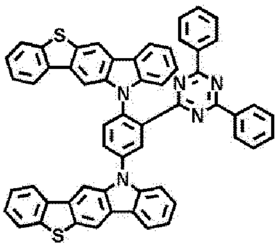

18

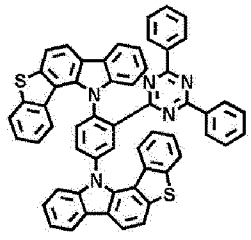

19

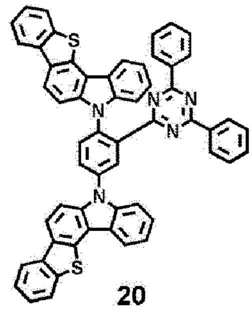

20

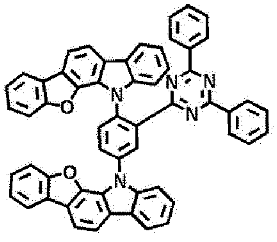

21

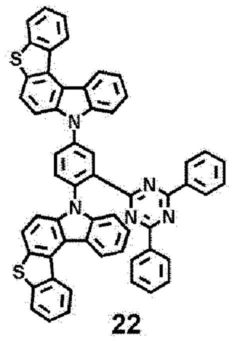

22

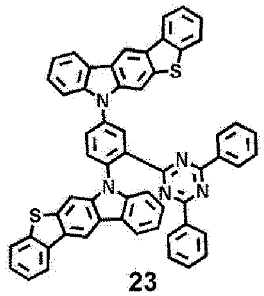

23

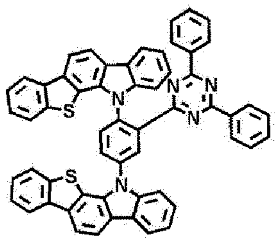

24

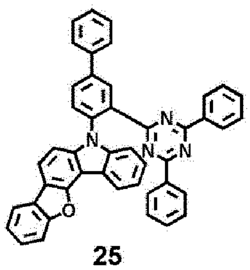

25

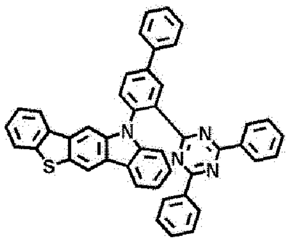

26

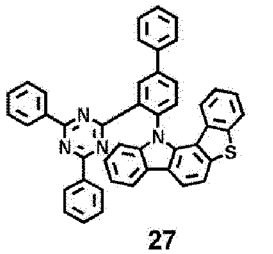

27

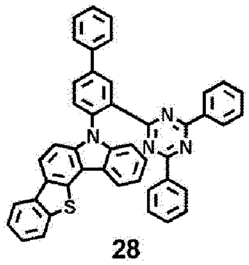

28

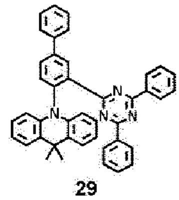
29
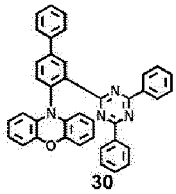
30
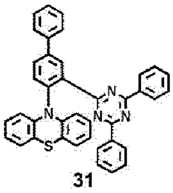
31
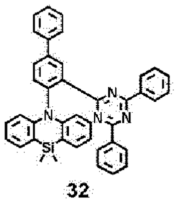
32
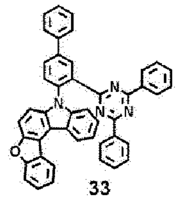
33
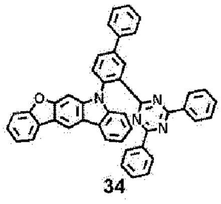
34

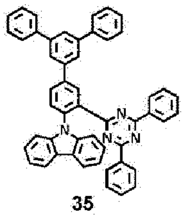
35
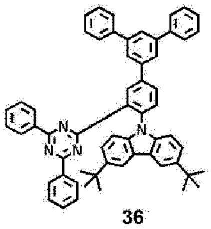
36
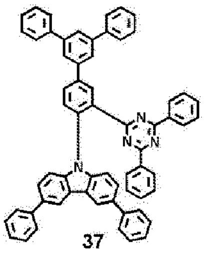
37
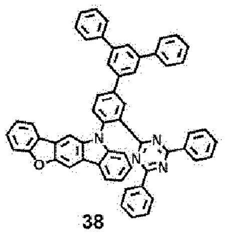
38
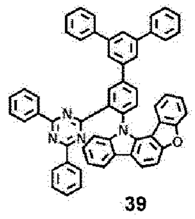
39

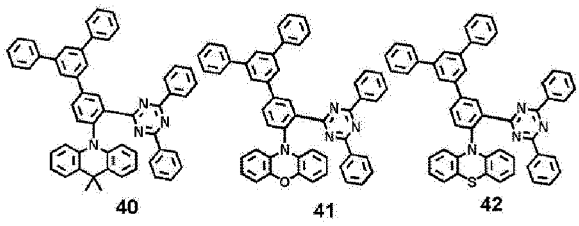
40
41
42
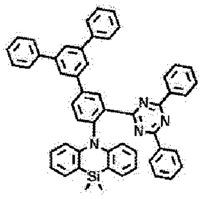
43
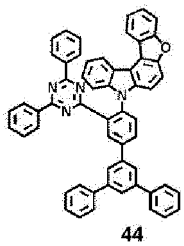
44
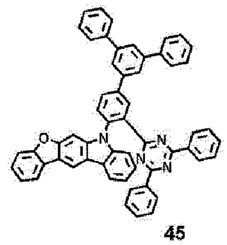
45

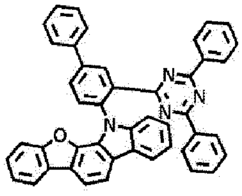
46
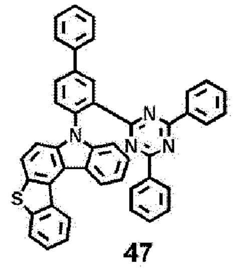
47
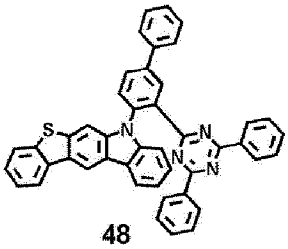
48
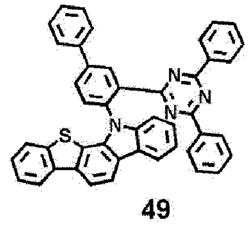
49

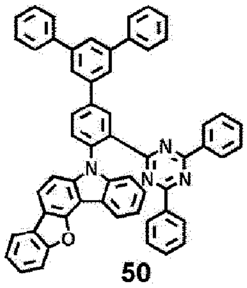
50
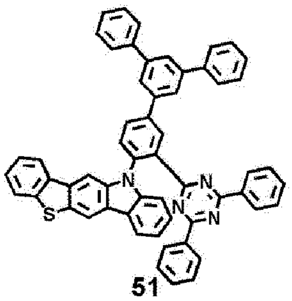
51
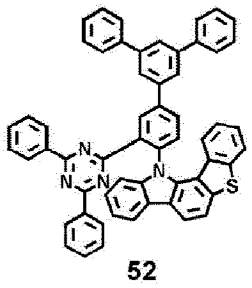
52
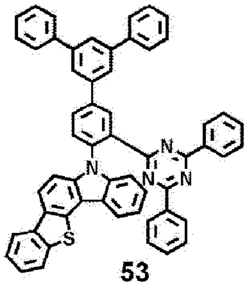
53

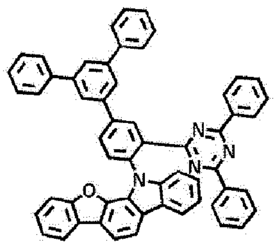
54
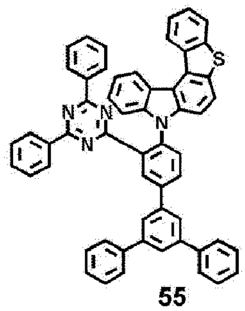
55
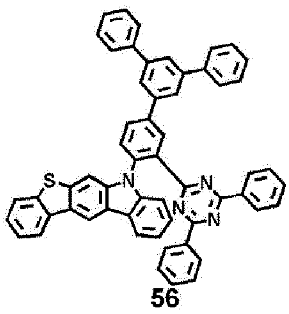
56
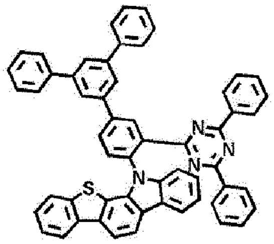
57

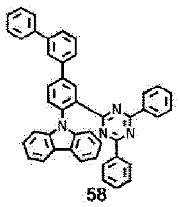
58
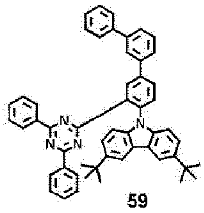
59
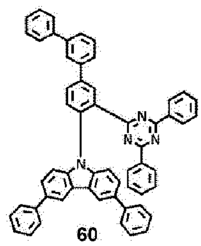
60
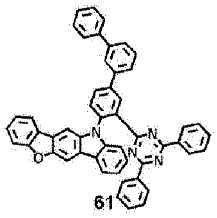
61
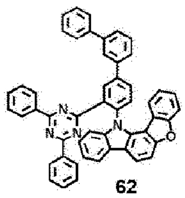
62

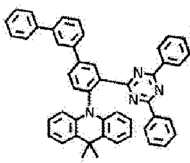
63
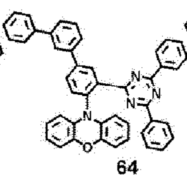
64
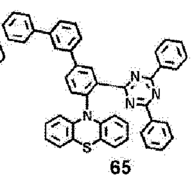
65
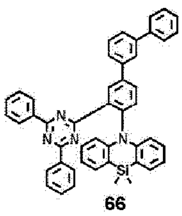
66
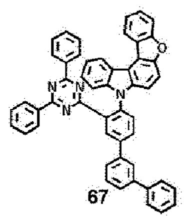
67
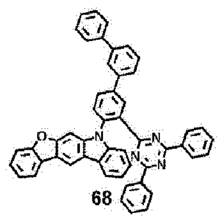
68

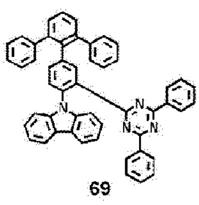
69
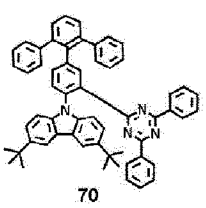
70
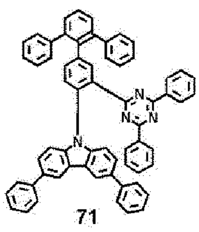
71
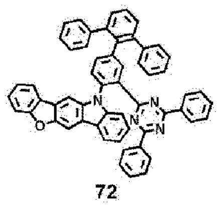
72
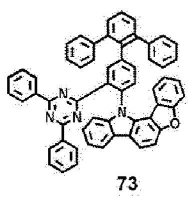
73

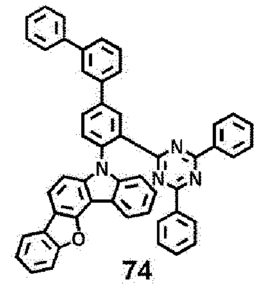
74

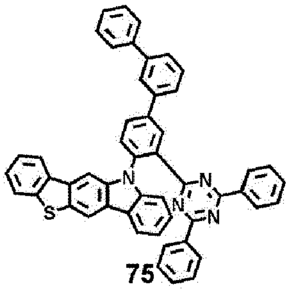
75

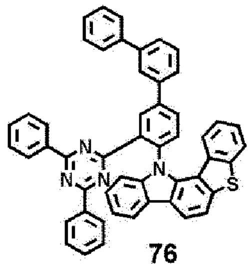
76

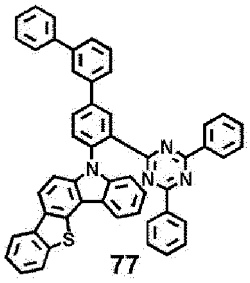
77

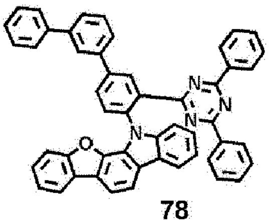
78

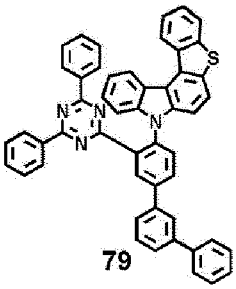
79

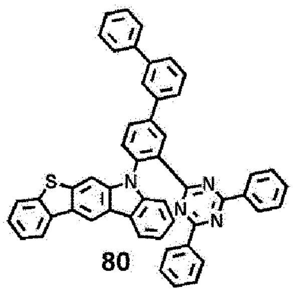
80

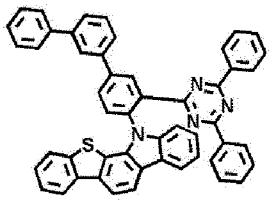
81

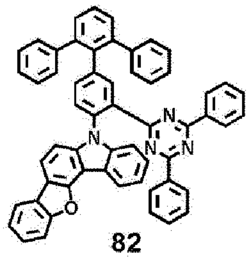
82

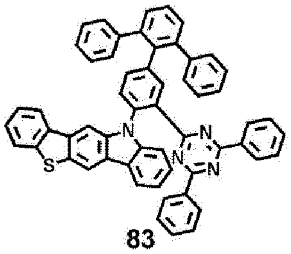
83

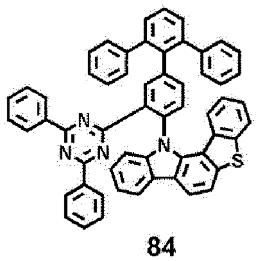
84

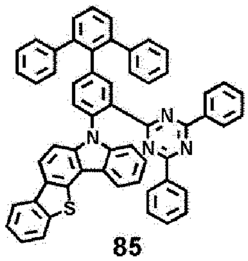
85

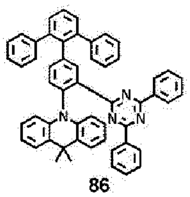
86

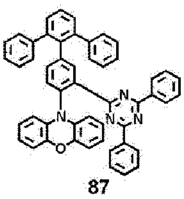
87

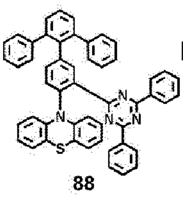
88

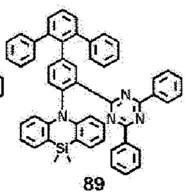
89

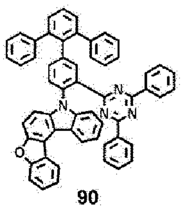
90

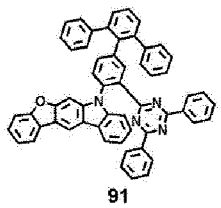
91

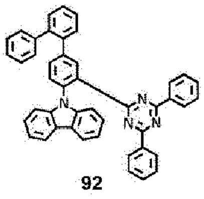
92

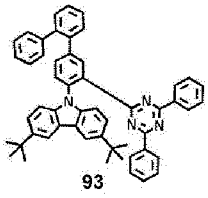
93

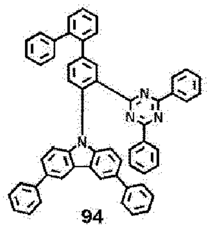
94

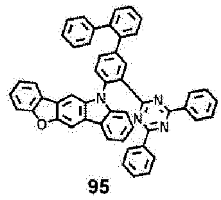
95

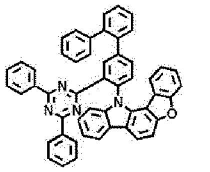
96

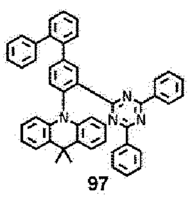
97

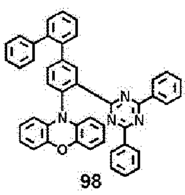
98

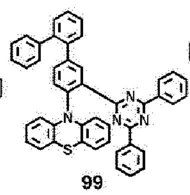
99

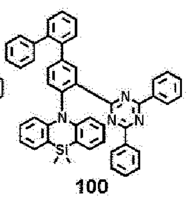
100

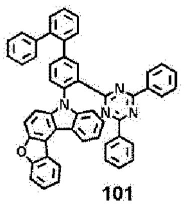
101

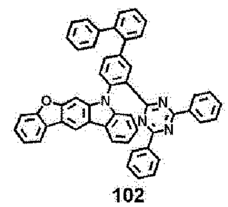
102

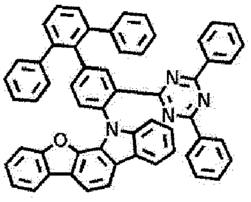
**103**

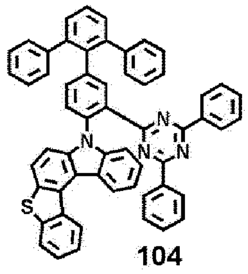
**104**

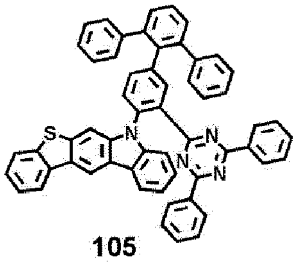
**105**

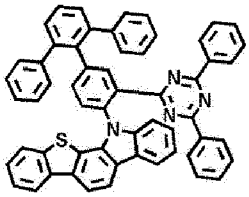
**106**

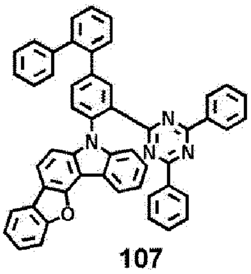
**107**

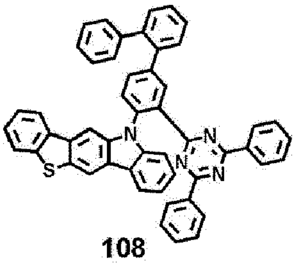
**108**

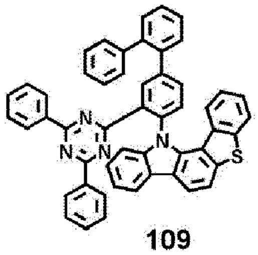
**109**

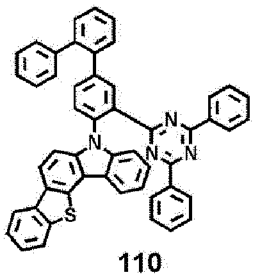
**110**

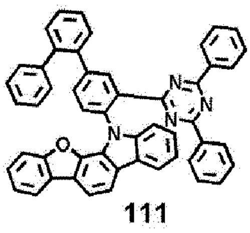
**111**

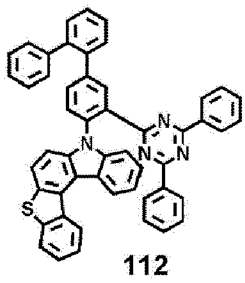
**112**

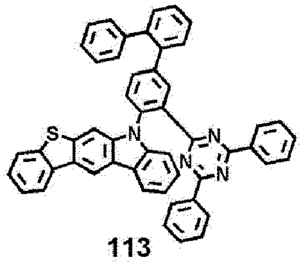
**113**

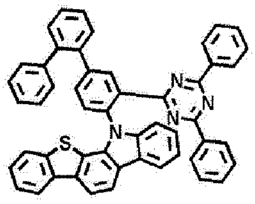
**114**

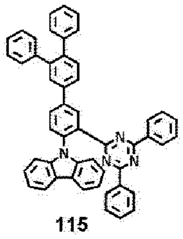
115

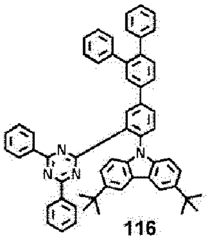
116

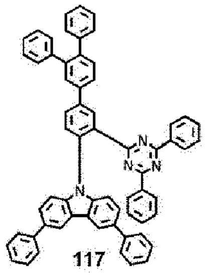
117

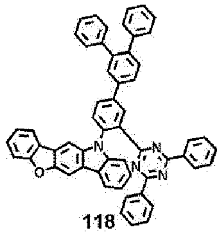
118

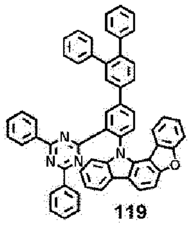
119

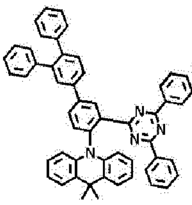
120

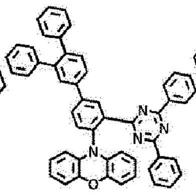
121

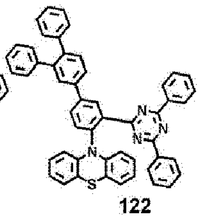
122

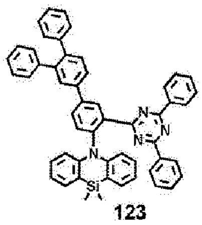
123

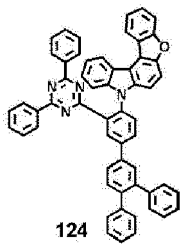
124

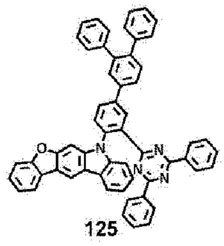
125

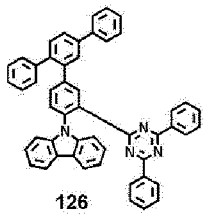
126

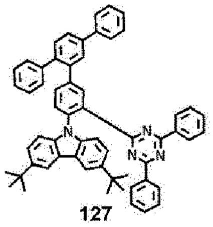
127

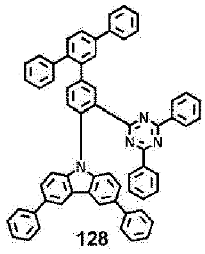
128

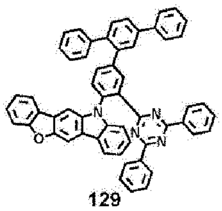
129

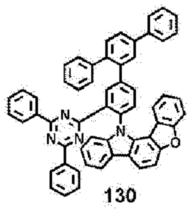
130

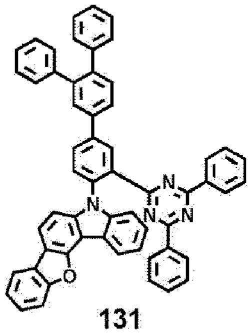
131

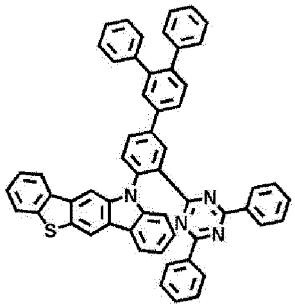
132

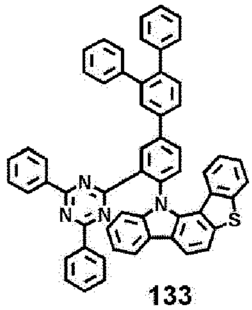
133

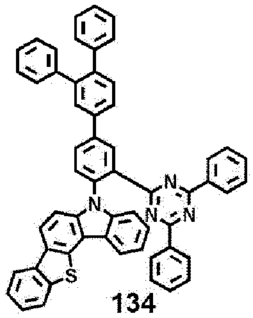
134

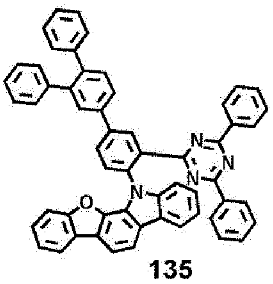
135

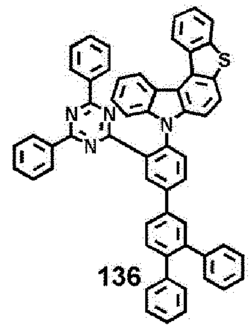
136

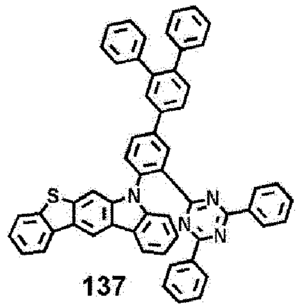
137

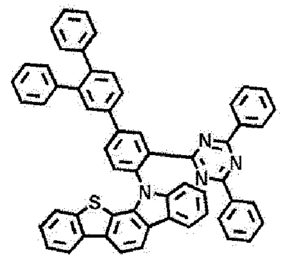
138

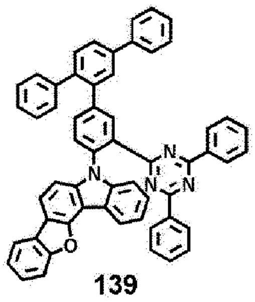
139

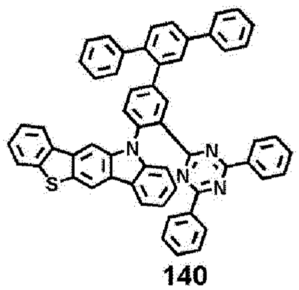
140

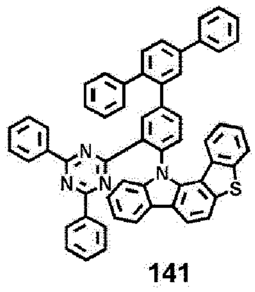
141

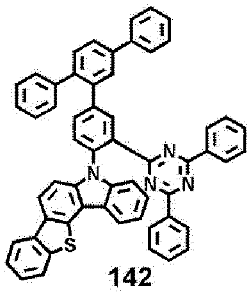
142

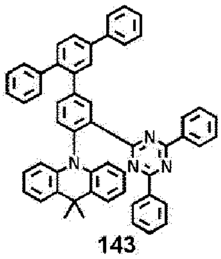
143

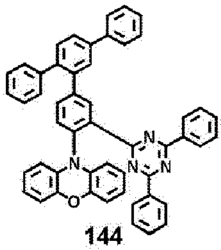
144

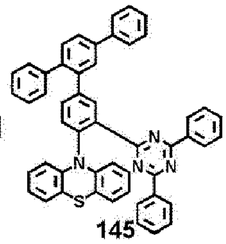
145

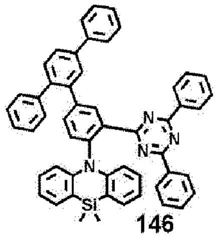
146

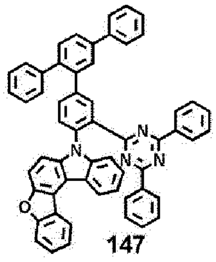
147

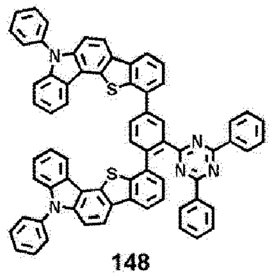
148

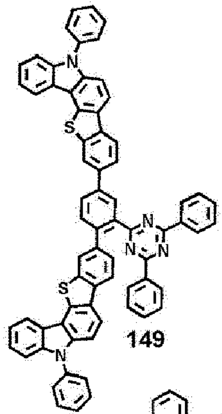
149

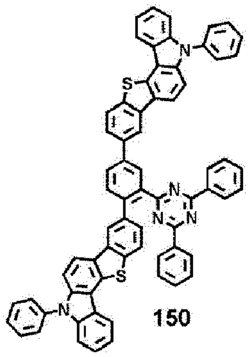
150

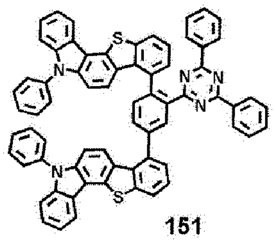
151

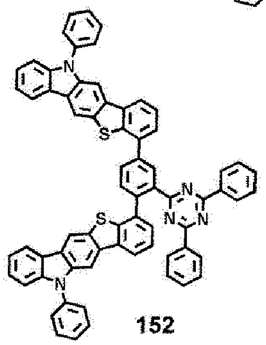
152

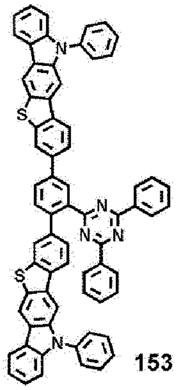
153

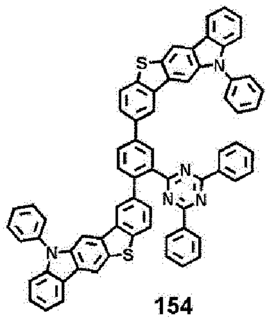
154

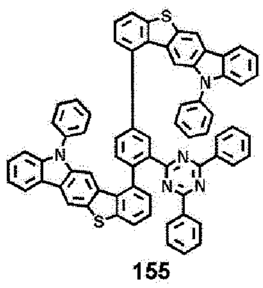
155

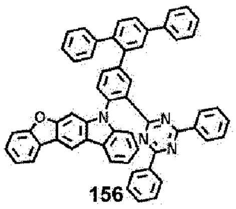
156

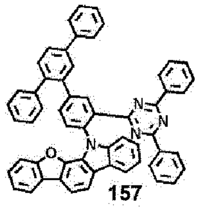
157

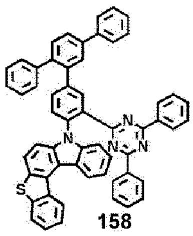
158

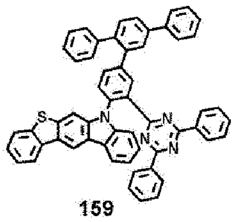
159

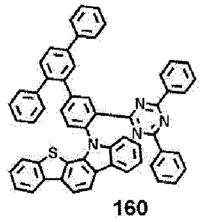
160

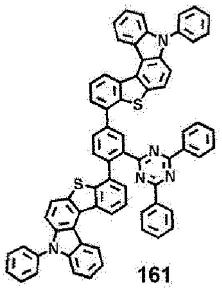
161

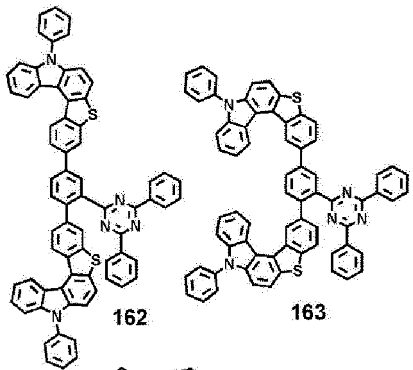
162

163

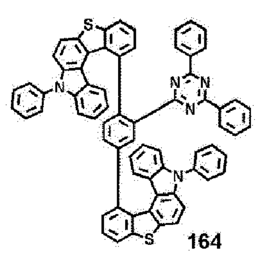
164

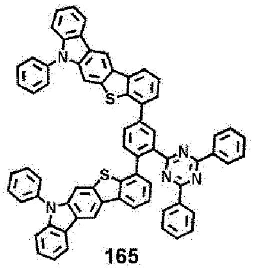
165

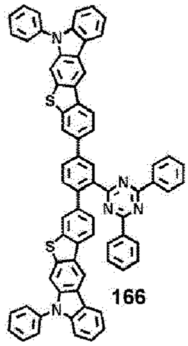
166

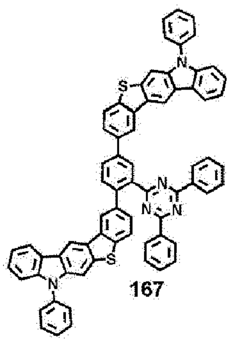
167

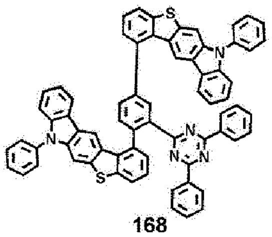
168

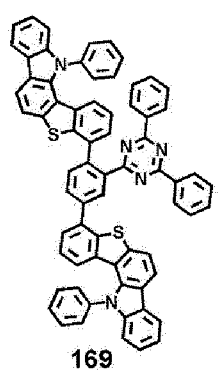
169

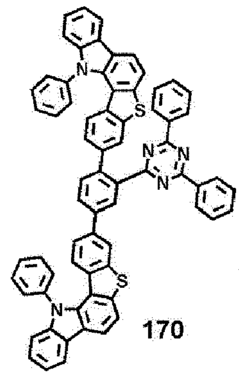
170

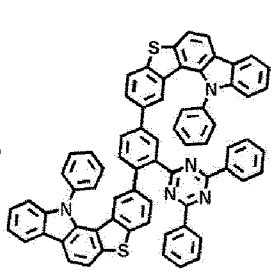
171

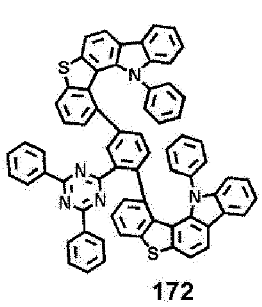
172

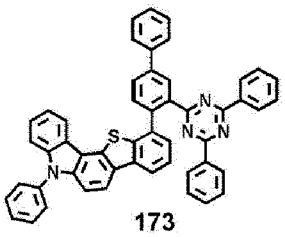
173

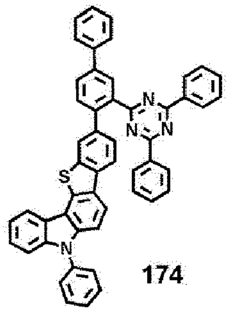
174

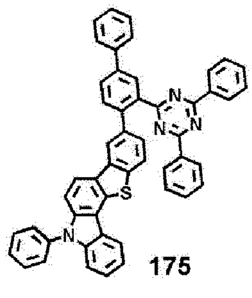
175

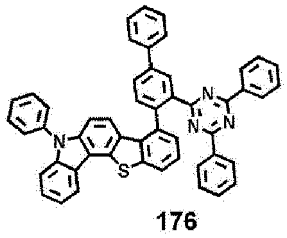
176

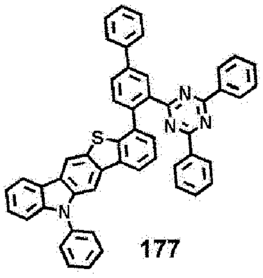
177

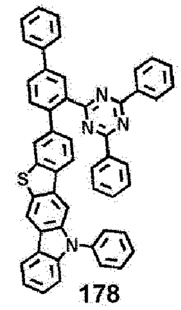
178

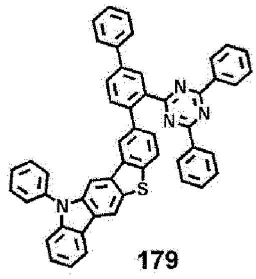
179

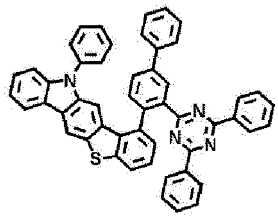
180

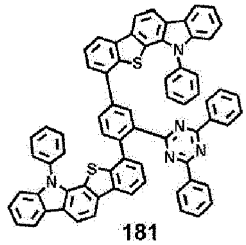
181

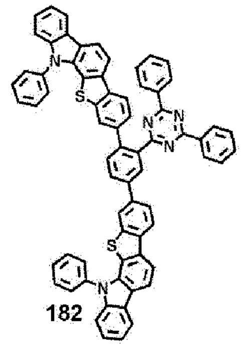
182

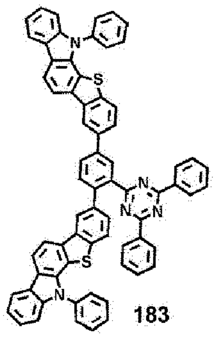
183

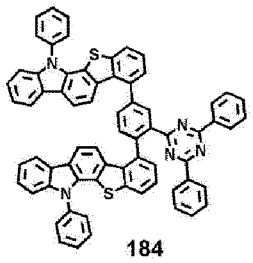
184

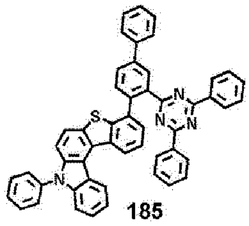
185

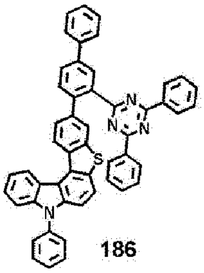
186

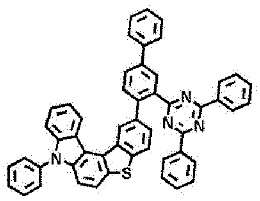
187

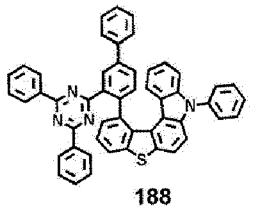
188

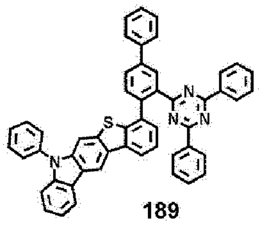
189

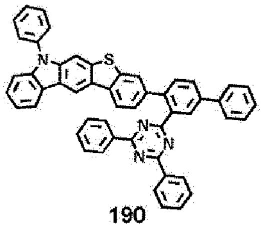
190

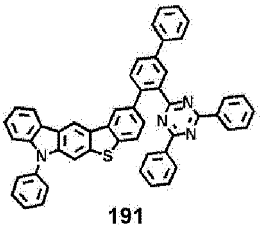
191

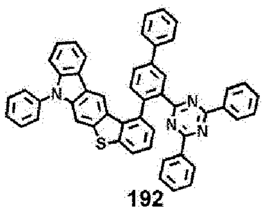
192

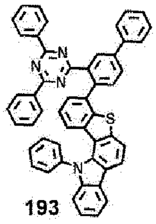
193

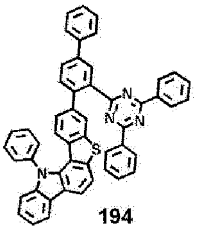
194

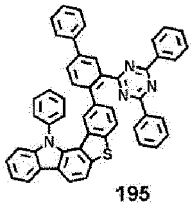
195

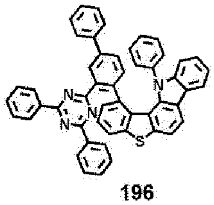
196

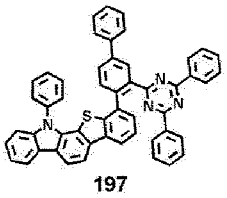
197

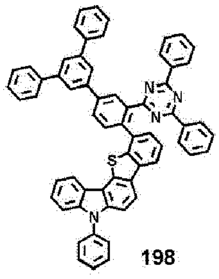
198

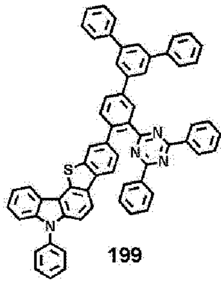
199

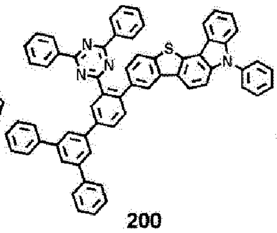
200

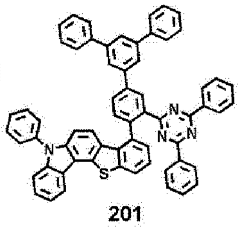
201

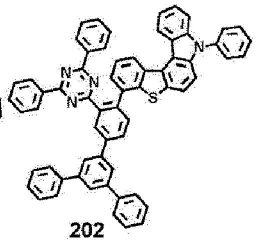
202

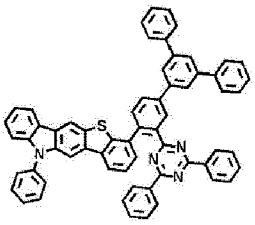
203

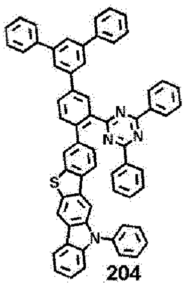
204

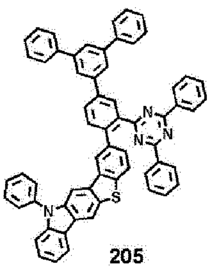
205

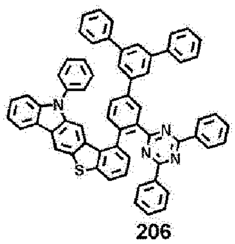
206

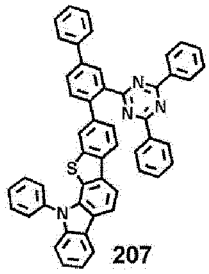
207

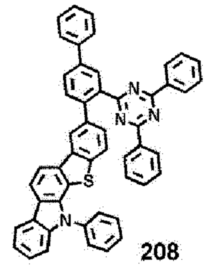
208

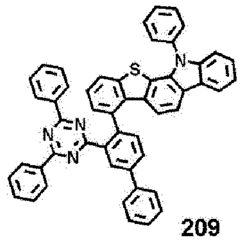
209

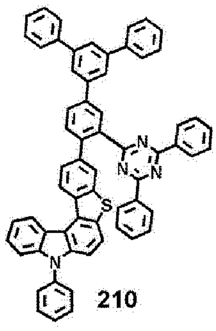
210

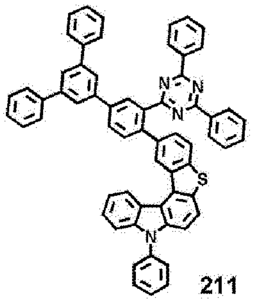
211

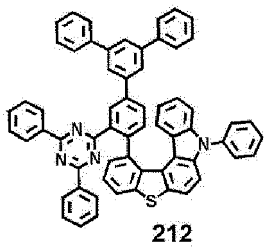
212

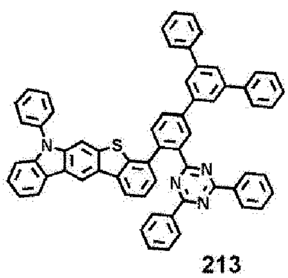
213

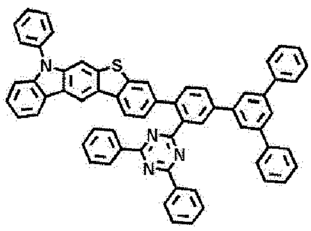
214

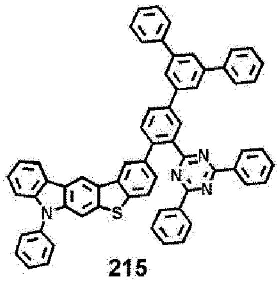
215

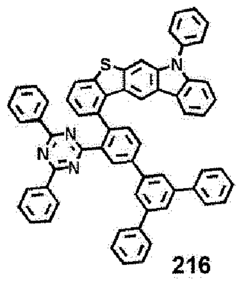
216

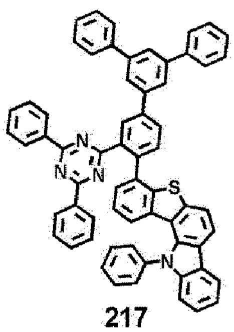

217

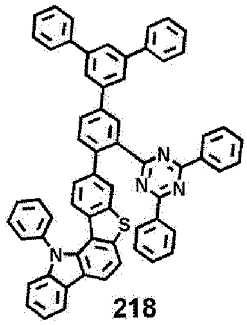

218

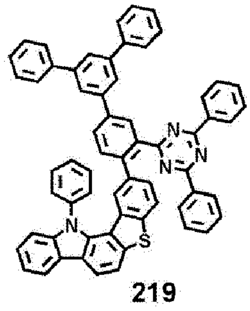

219

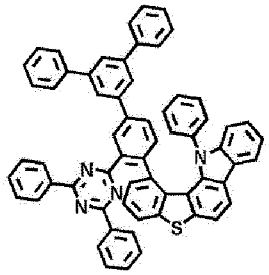

220

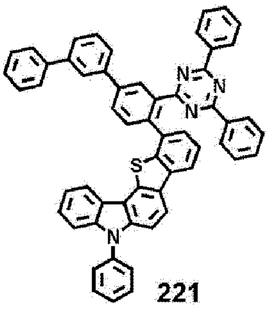

221

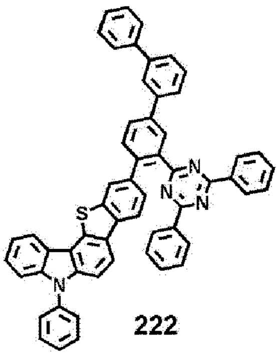

222

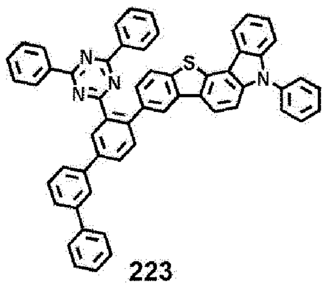

223

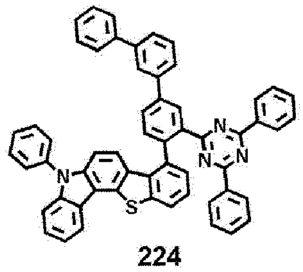

224

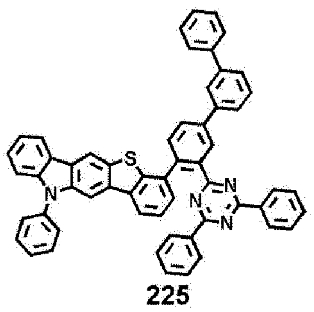

225

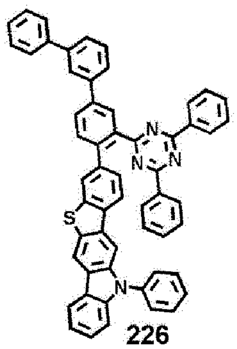

226

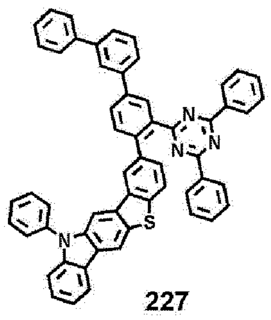

227

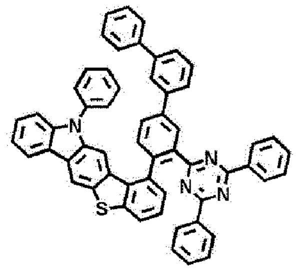

228

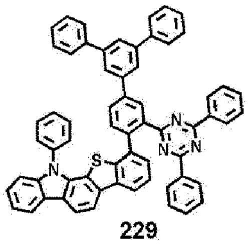

229

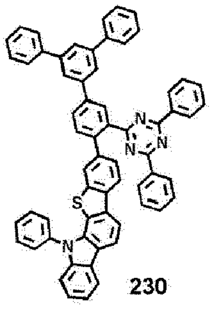

230

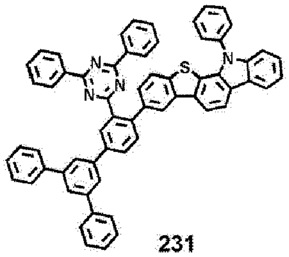

231

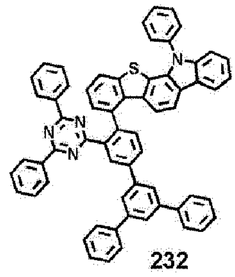

232

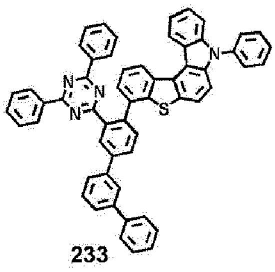

233

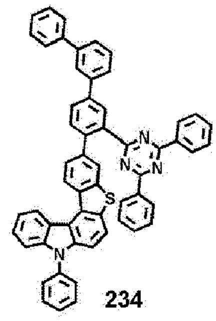

234

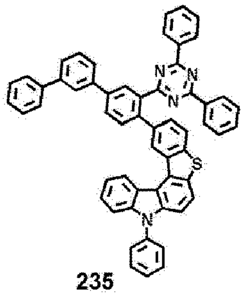

235

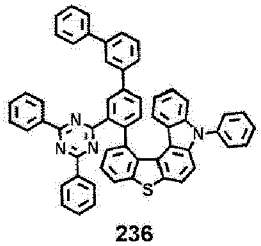

236

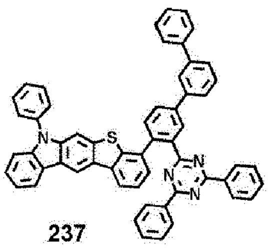

237

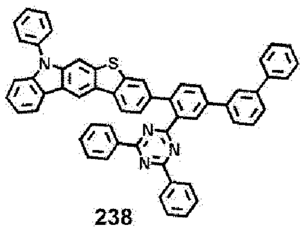

238

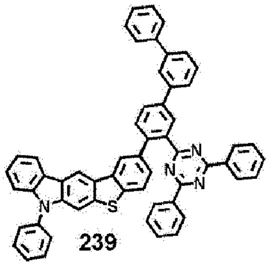

239

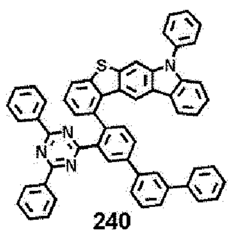

240

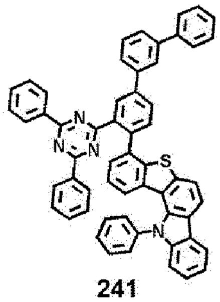
241

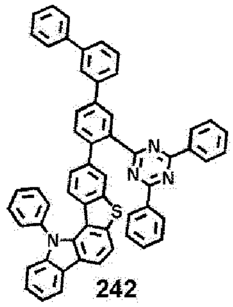
242

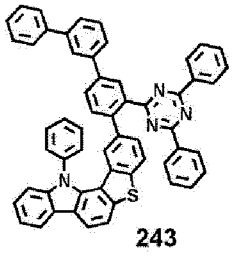
243

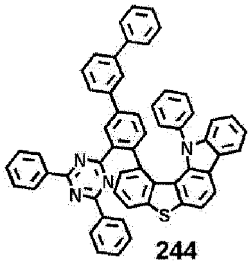
244

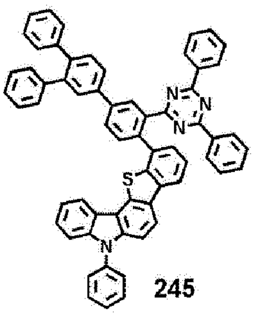
245

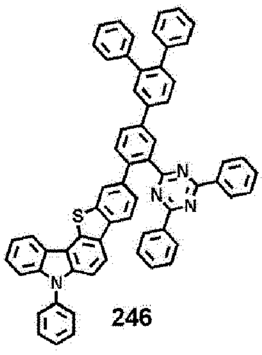
246

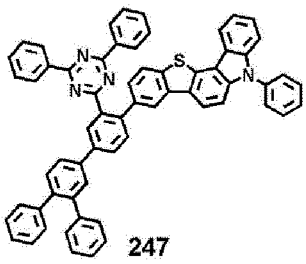
247

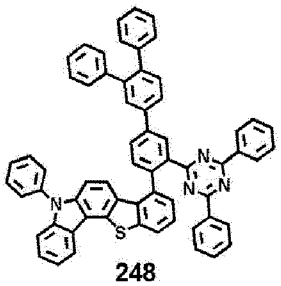
248

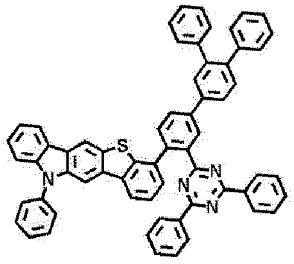
249

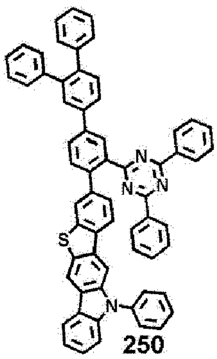
250

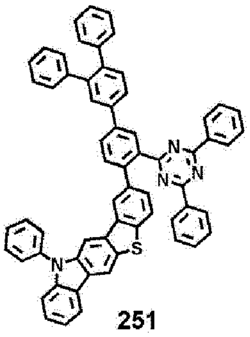
251

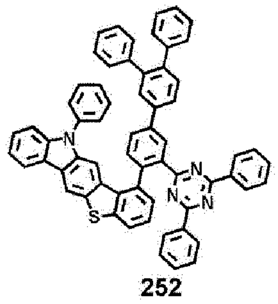
252

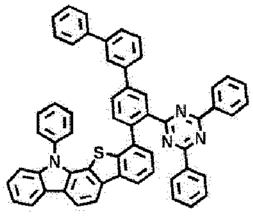

253

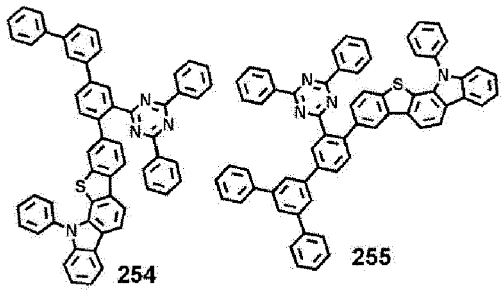

254 255

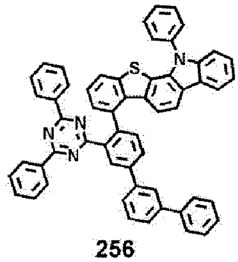

256

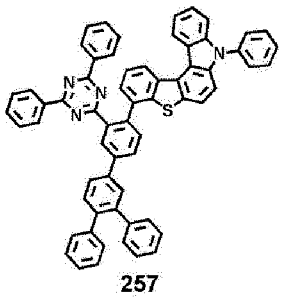

257

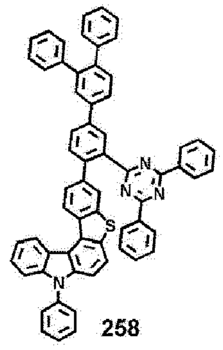

258

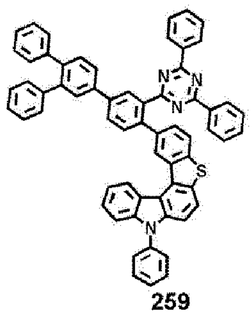

259

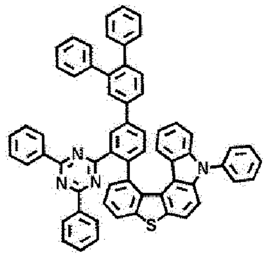

260

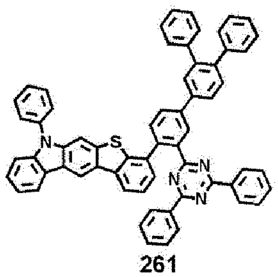

261

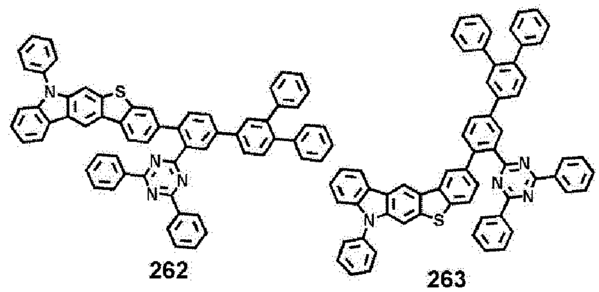

262 263

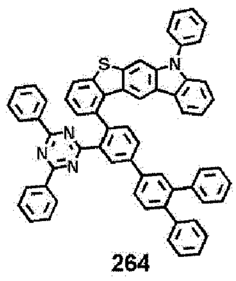

264

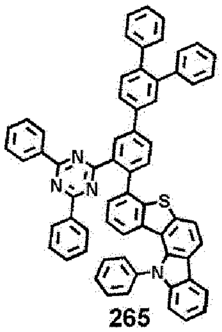

265

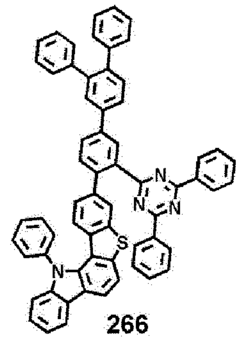

266

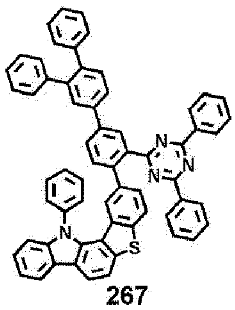

267

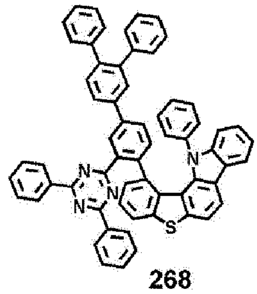

268

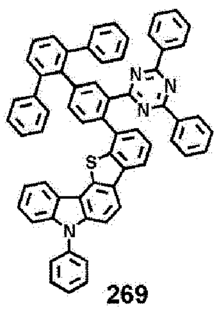

269

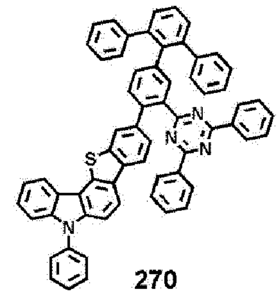

270

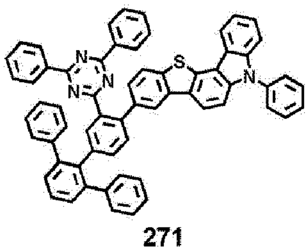

271

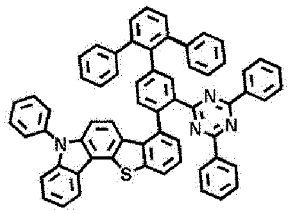

272

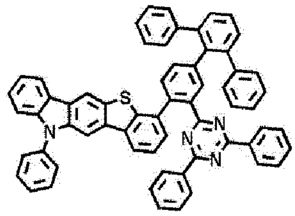

273

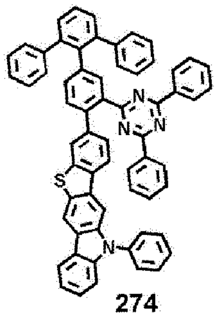

274

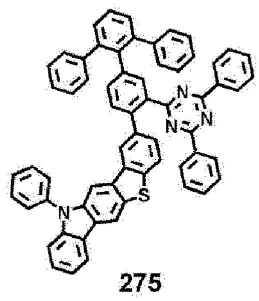

275

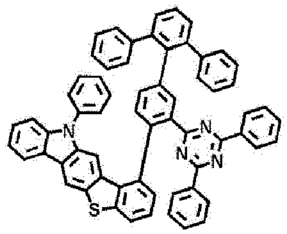

276

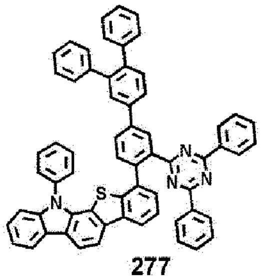

277

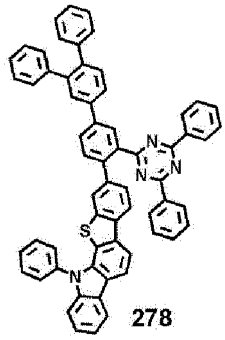

278

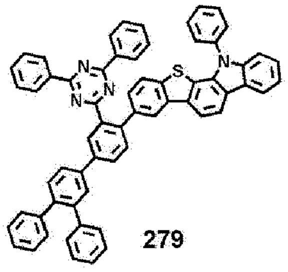

279

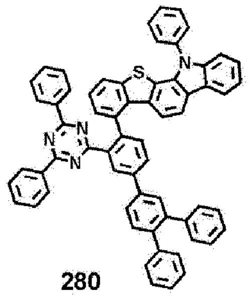

280

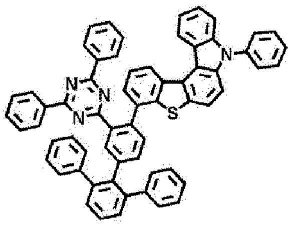

281

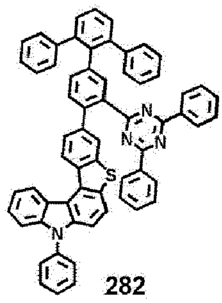

282

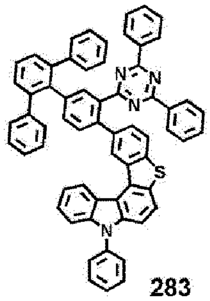

283

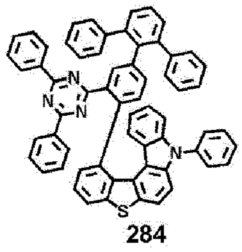

284

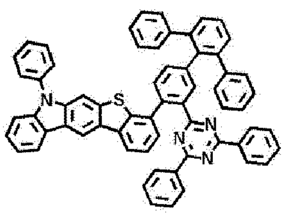

285

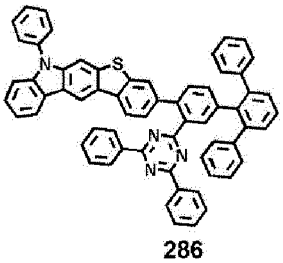

286

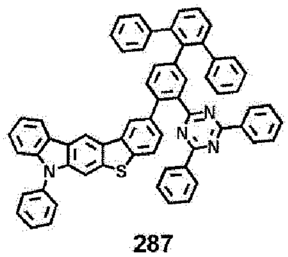

287

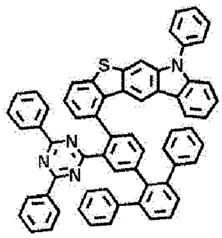

288

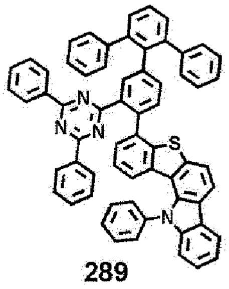

289

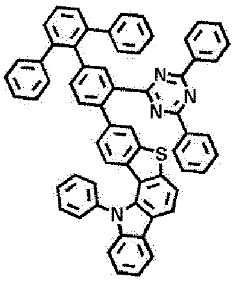

290

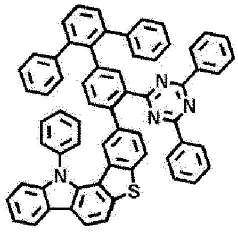

291

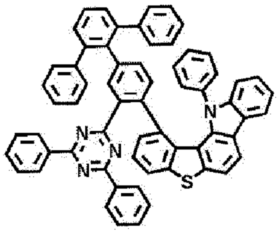

292

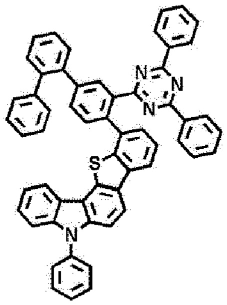

293

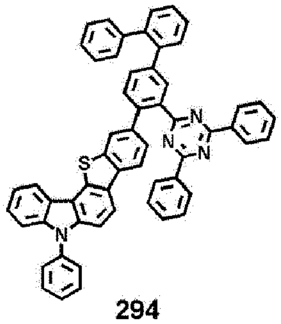

294

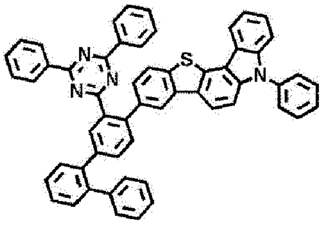

295

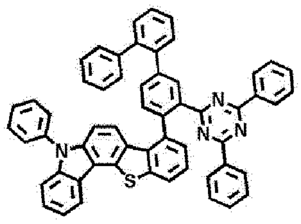

296

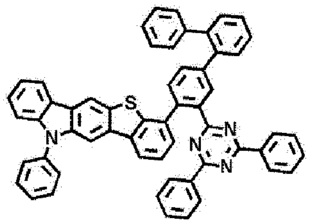

297

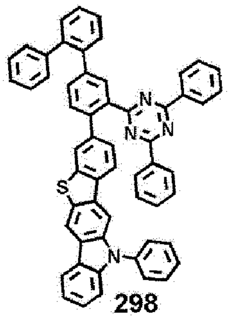

298

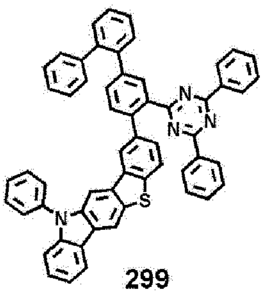

299

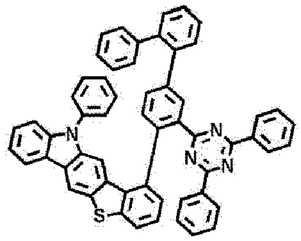

300

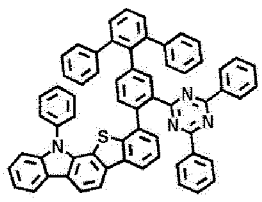
301

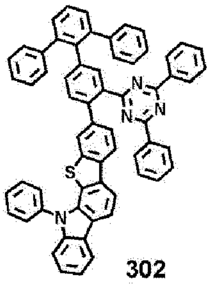
302

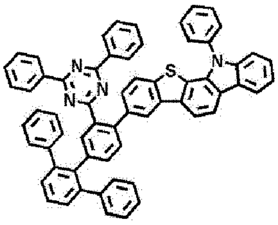
303

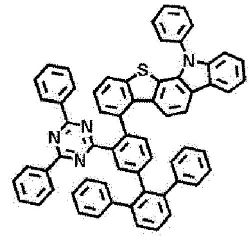
304

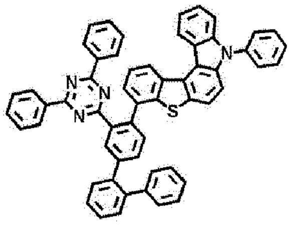
305

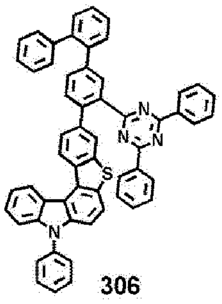
306

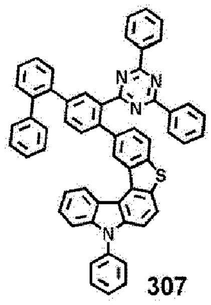
307

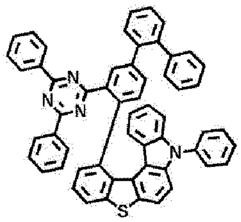
308

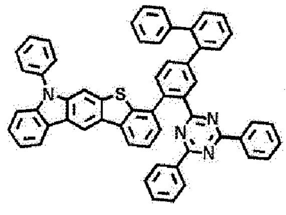
309

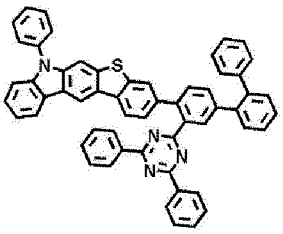
310

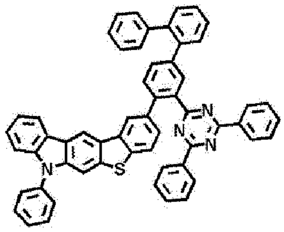
311

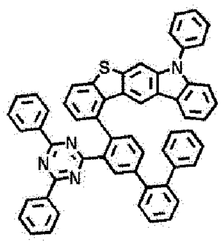
312

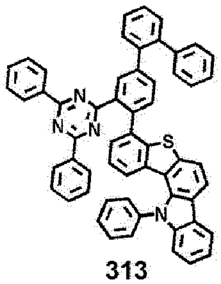

313

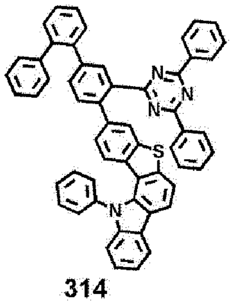

314

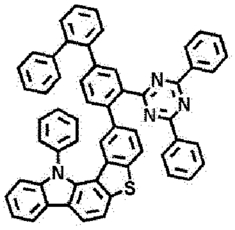

315

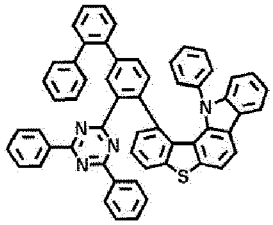

316

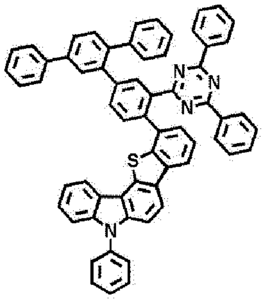

317

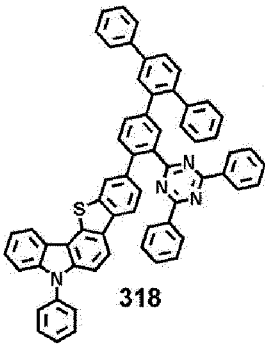

318

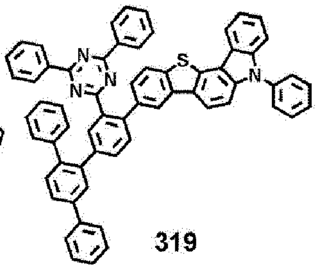

319

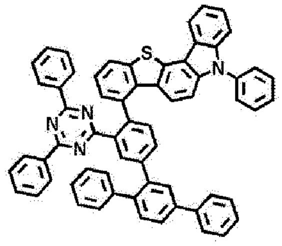

320

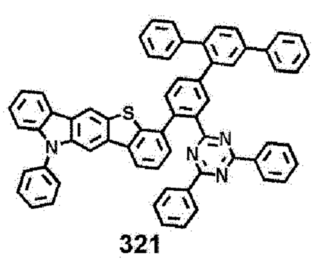

321

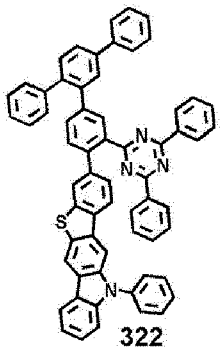

322

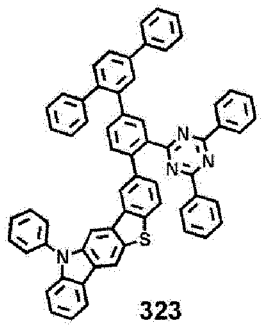

323

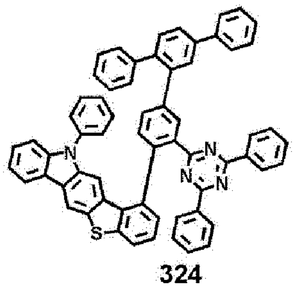

324

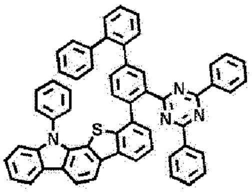
325

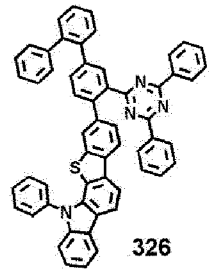
326

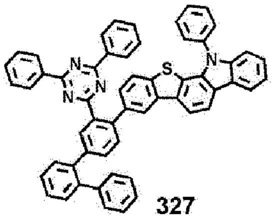
327

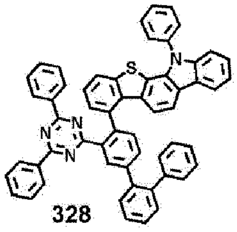
328

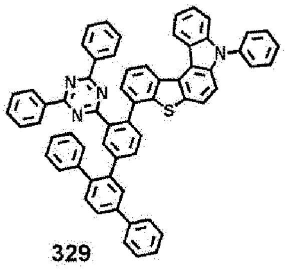
329

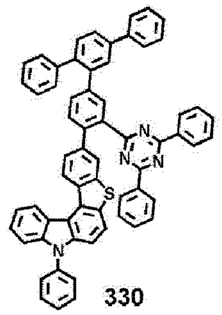
330

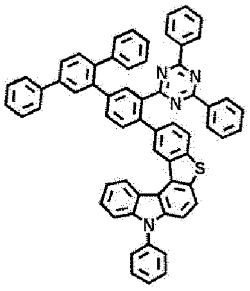
331

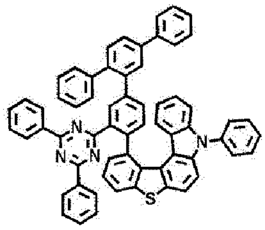
332

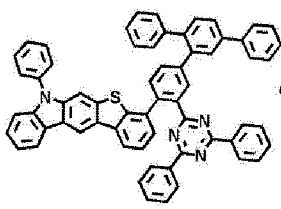
333

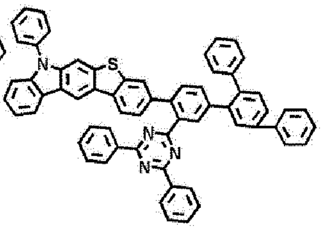
334

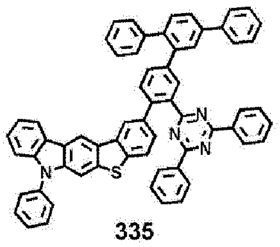
335

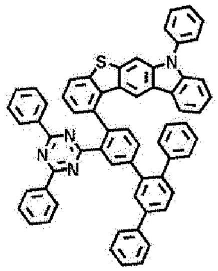
336

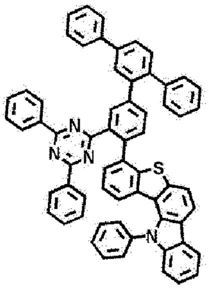
337

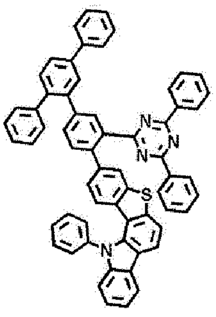
338

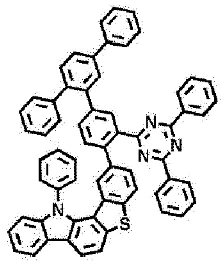
339

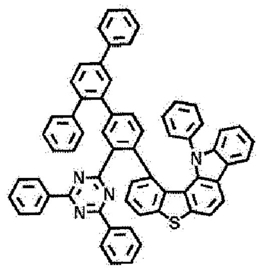
340

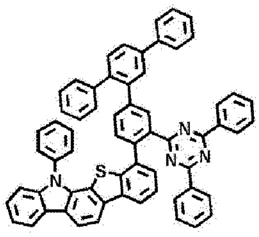
341

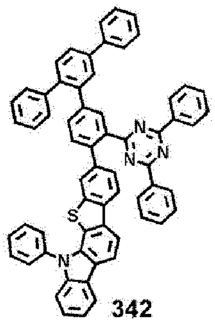
342

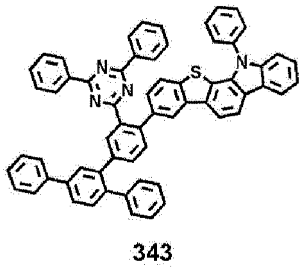
343

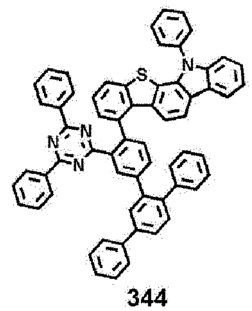
344

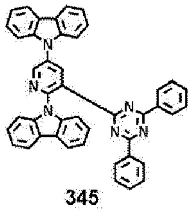
345

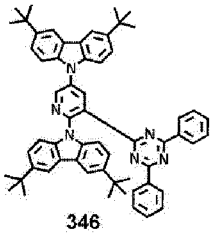
346

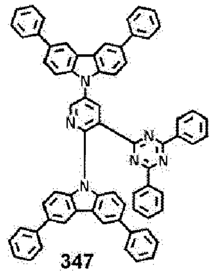
347

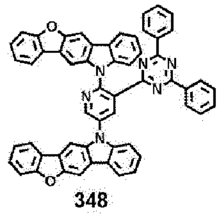
348

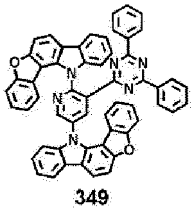
349

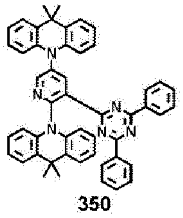
350

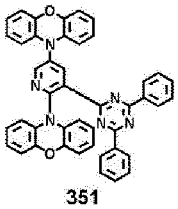
351

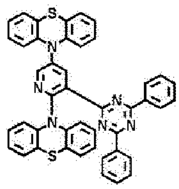
352

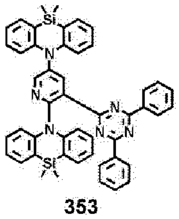
353

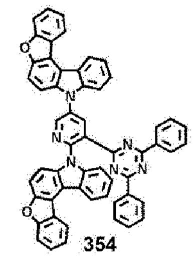
354

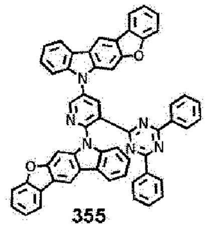
355

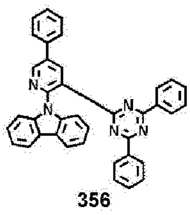
356

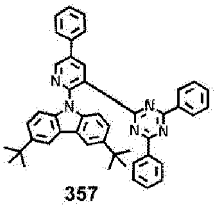
357

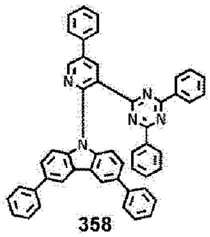
358

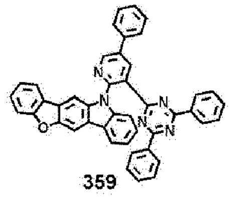
359

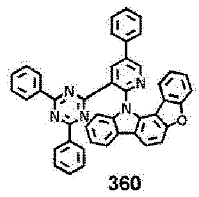
360

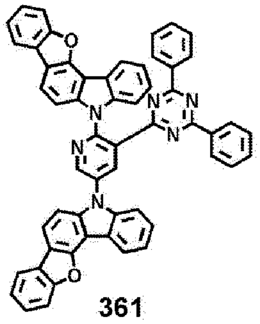
361
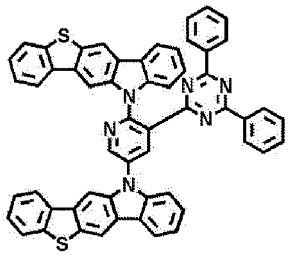
362
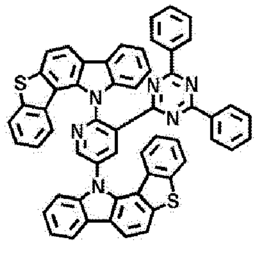
363
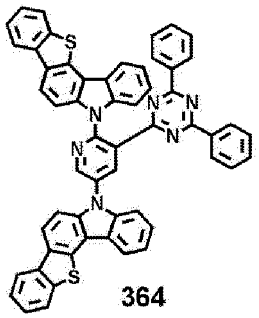
364

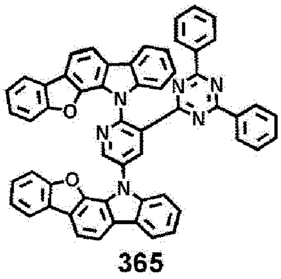
365
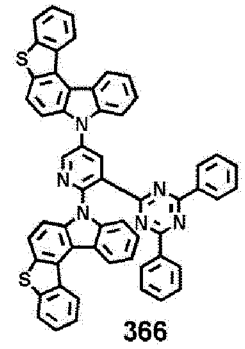
366
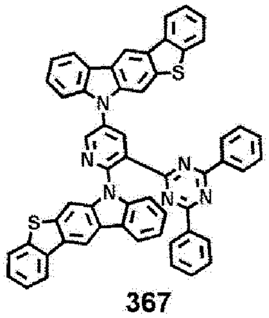
367
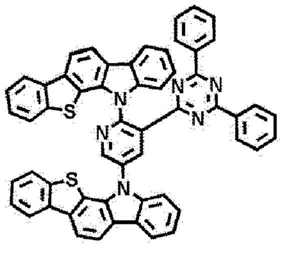
368

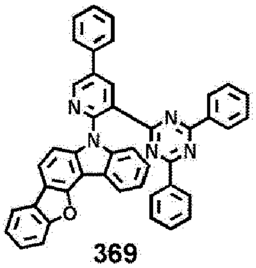
369
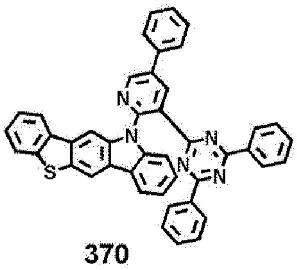
370
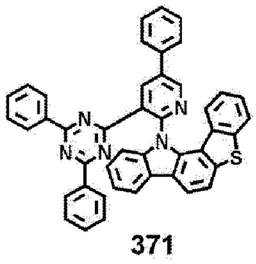
371
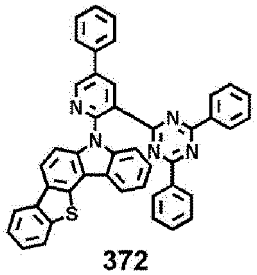
372

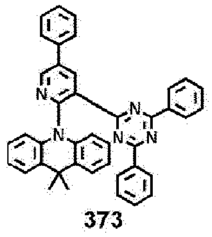
373
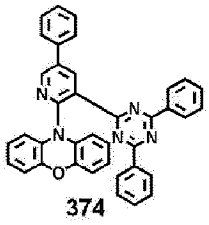
374
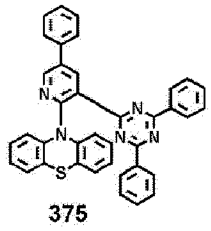
375
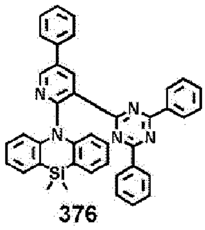
376
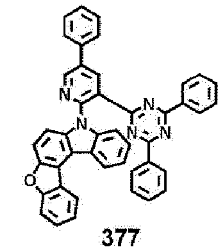
377

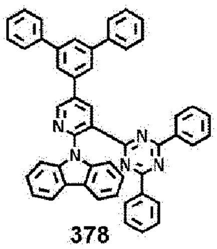
378
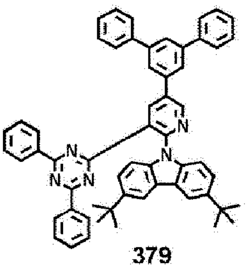
379
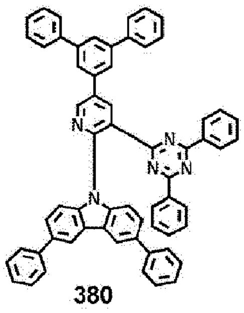
380
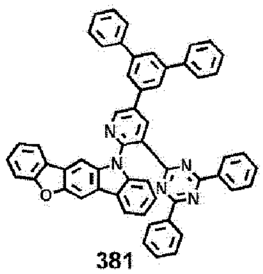
381

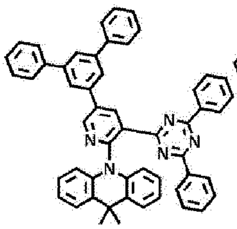
382
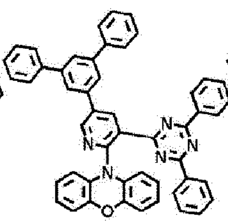
383
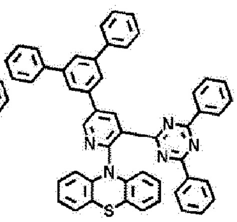
384
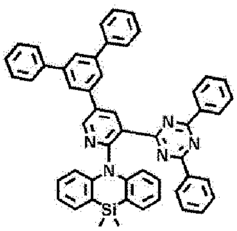
385
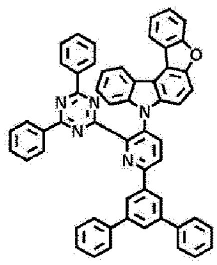
386

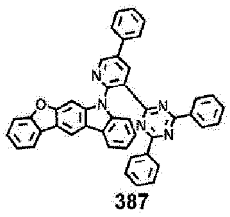
387
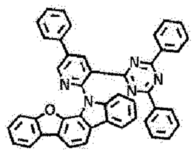
388
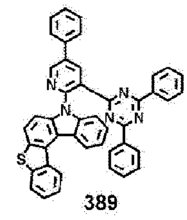
389
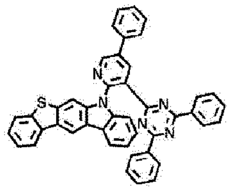
390
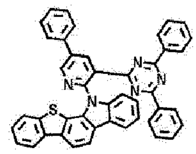
391

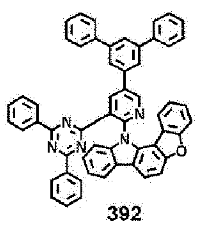
392
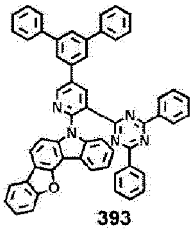
393
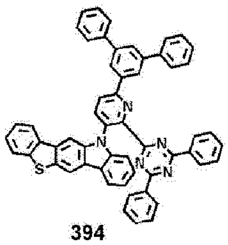
394
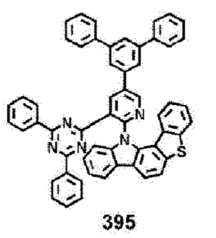
395
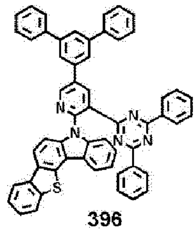
396

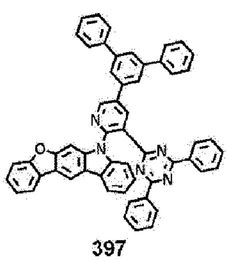
397
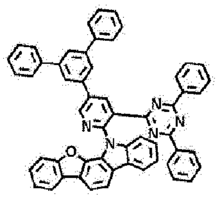
398
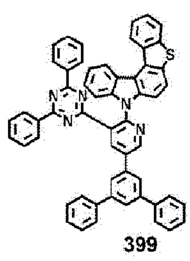
399
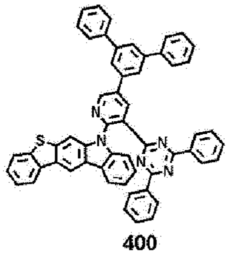
400
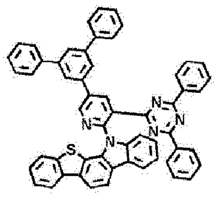
401

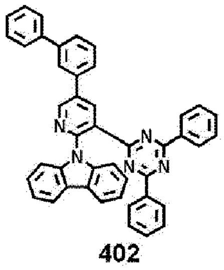
**402**

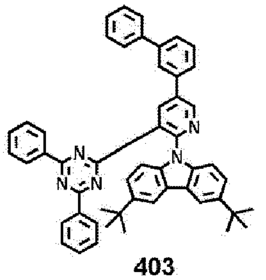
**403**

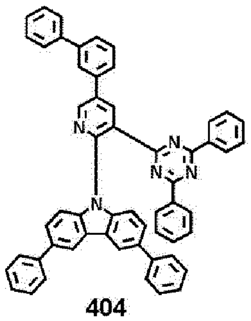
**404**

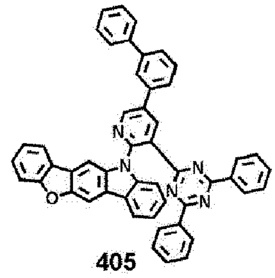
**405**

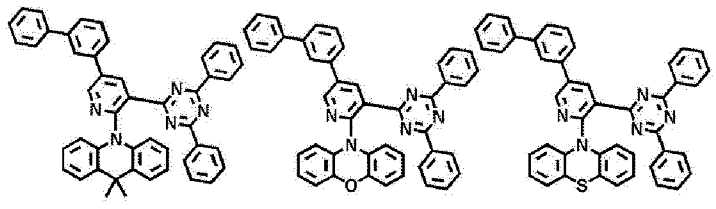
**406** **407** **408**

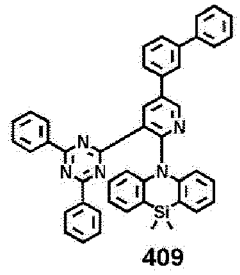
**409**

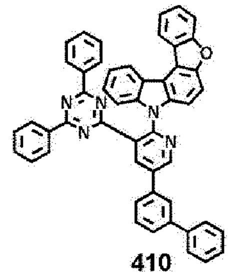
**410**

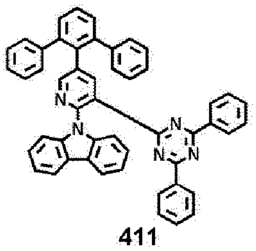
**411**

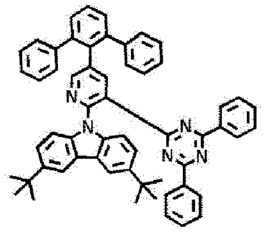
**412**

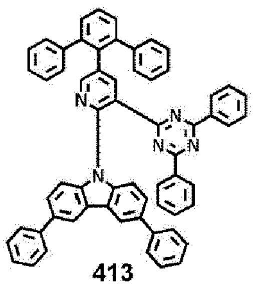
**413**

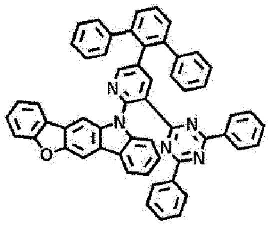
**414**

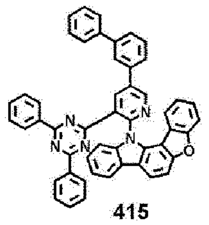
**415**

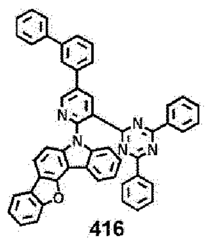
**416**

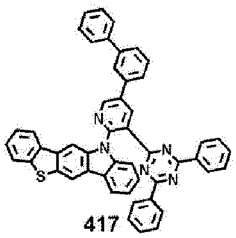
**417**

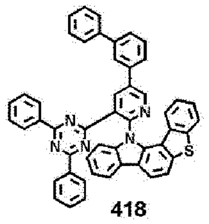
**418**

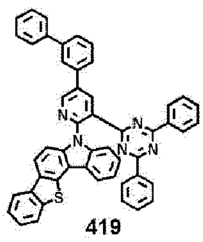
**419**

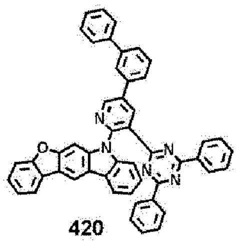
**420**

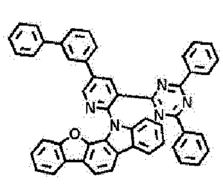
**421**

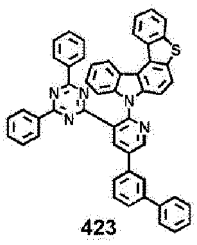
**423**

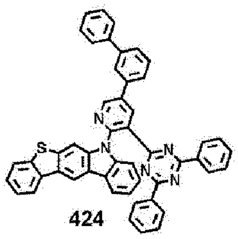
**424**

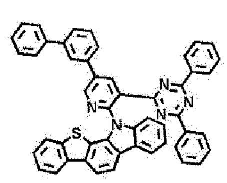
**425**

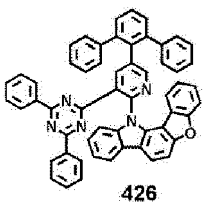
**426**

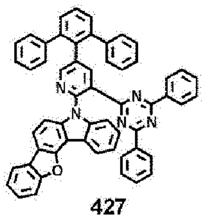
**427**

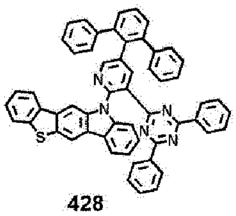
**428**

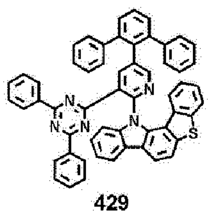
**429**

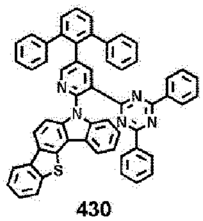
**430**

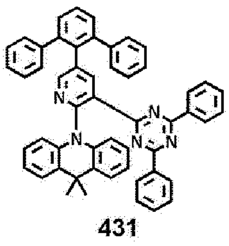
431
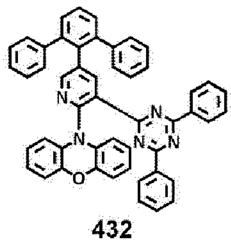
432
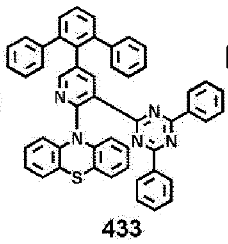
433
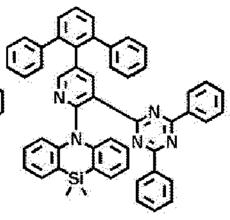
434
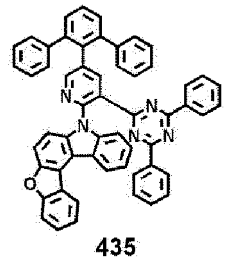
435

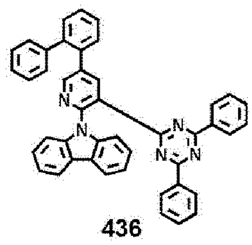
436
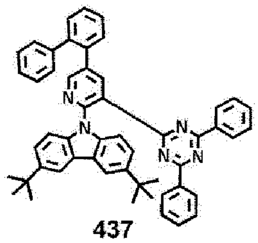
437
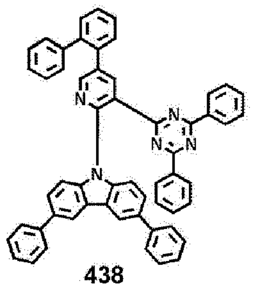
438
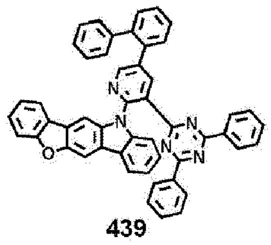
439

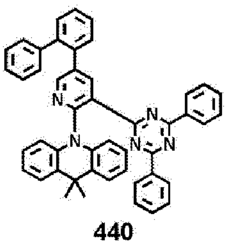
440
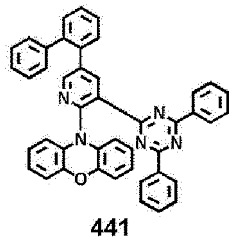
441
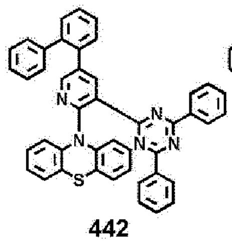
442
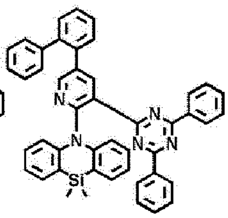
443
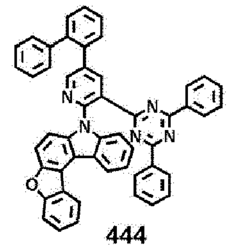
444

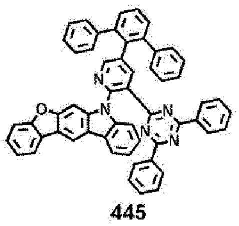
445
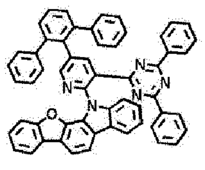
446
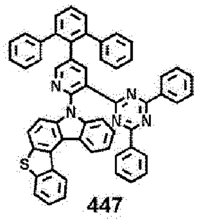
447
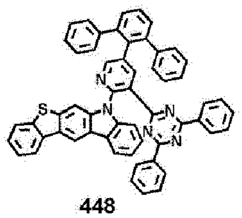
448
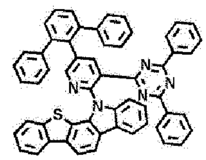
449

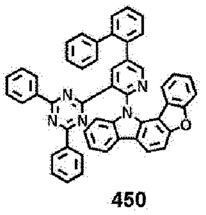
450
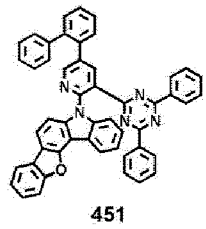
451
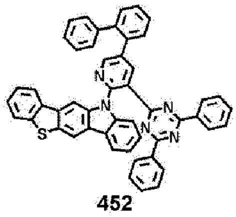
452
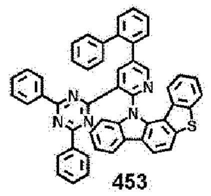
453
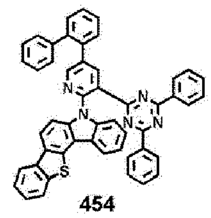
454

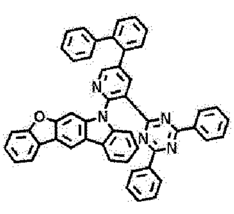
455
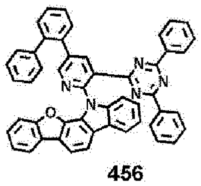
456
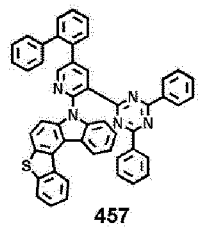
457
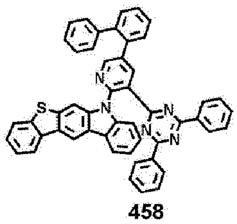
458
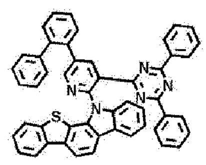
459

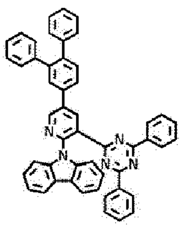

460

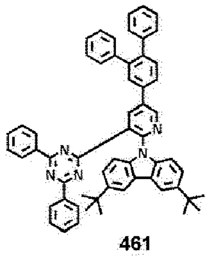

461

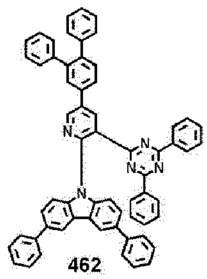

462

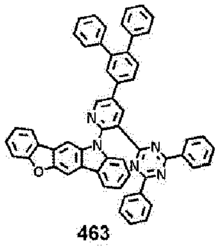

463

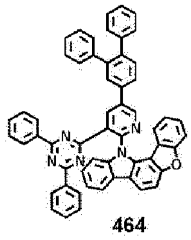

464

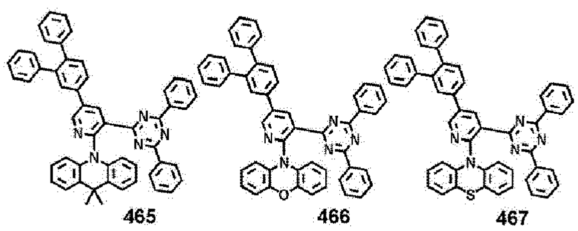

465

466

467

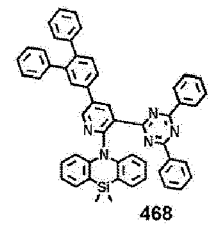

468

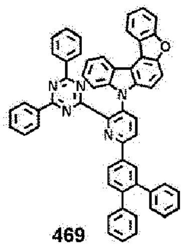

469

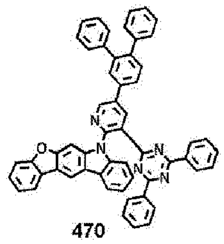

470

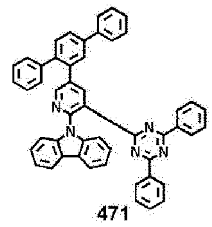

471

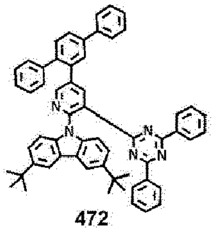

472

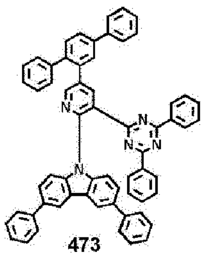

473

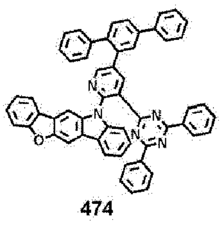

474

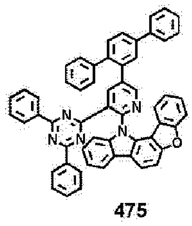

475

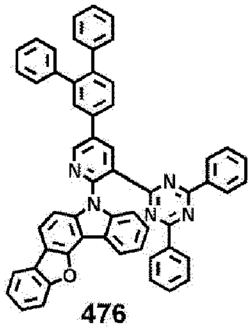

476

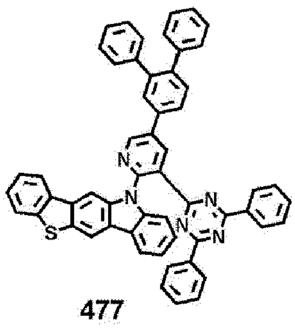

477

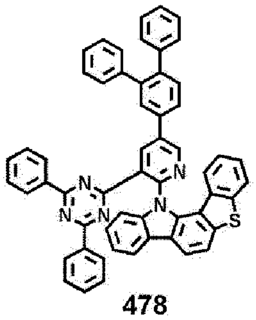

478

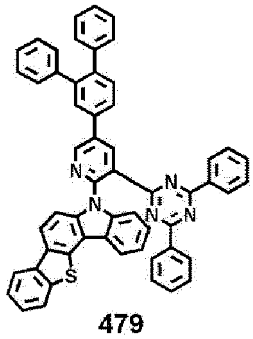

479

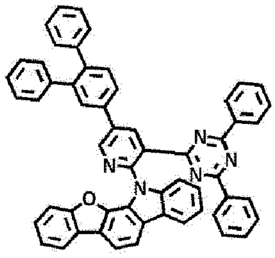

480

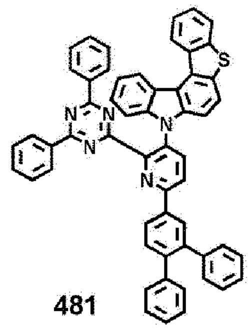

481

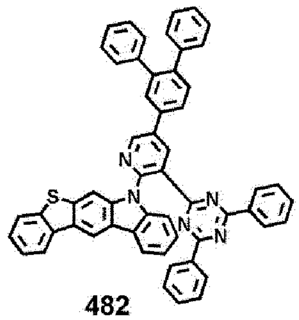

482

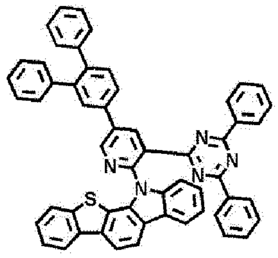

483

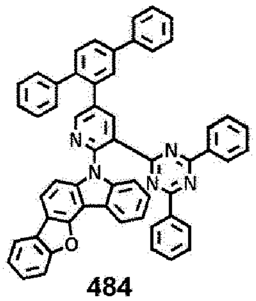

484

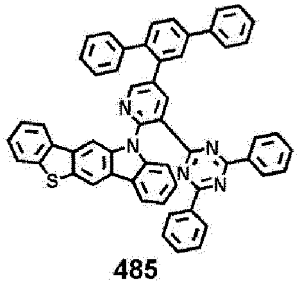

485

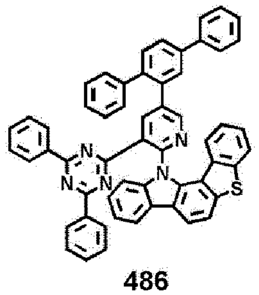

486

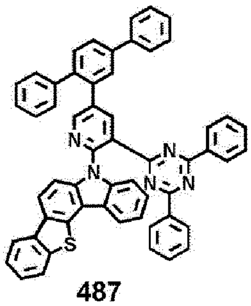

487

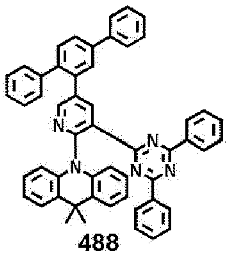
488
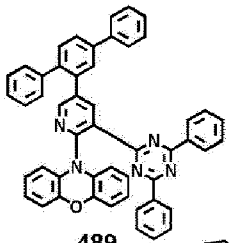
489
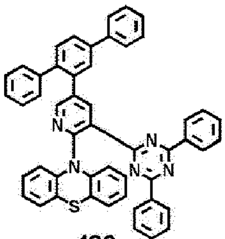
490
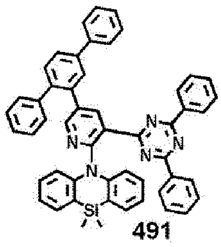
491
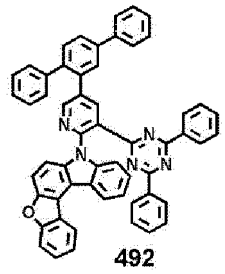
492

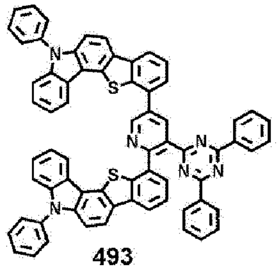
493
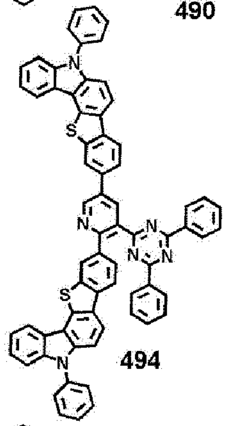
494
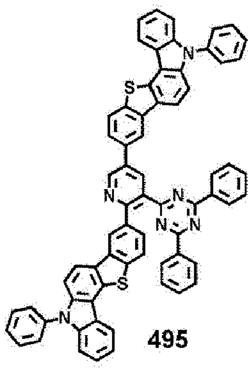
495
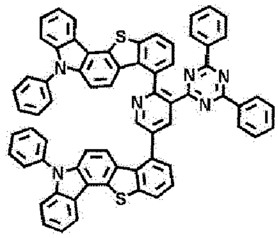
496

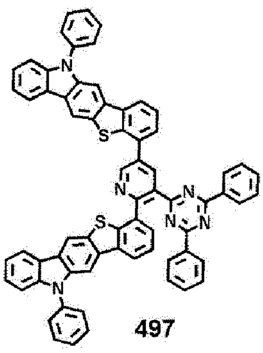
497
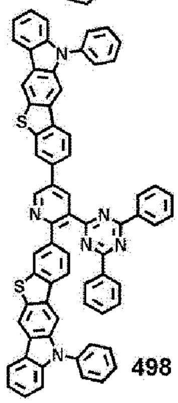
498
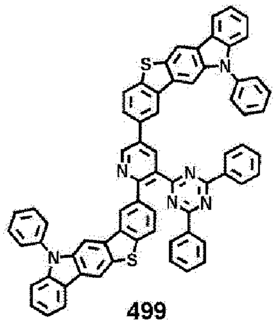
499
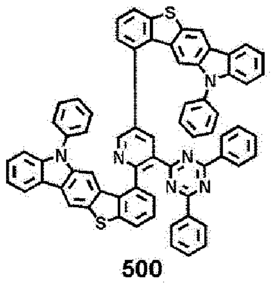
500

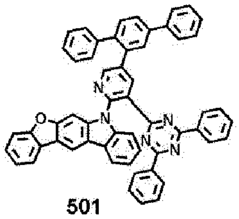
501
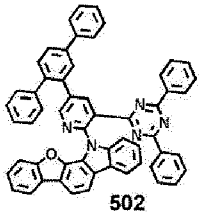
502
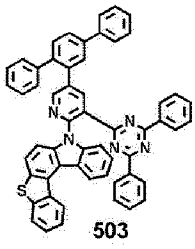
503
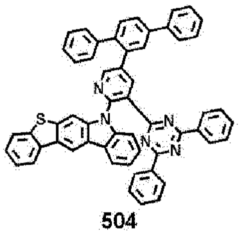
504
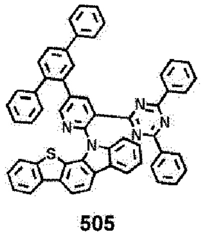
505
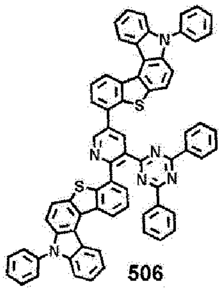
506
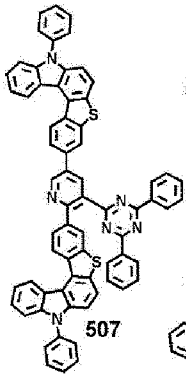
507
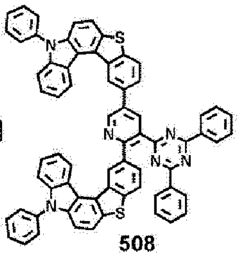
508
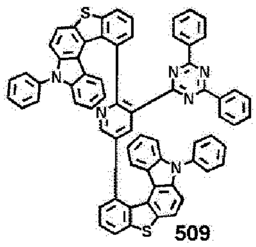
509
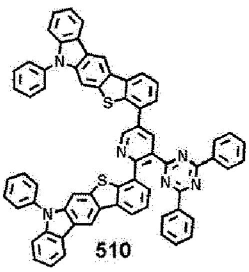
510
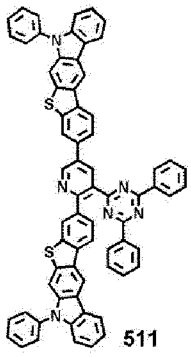
511
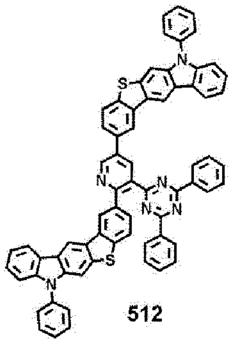
512
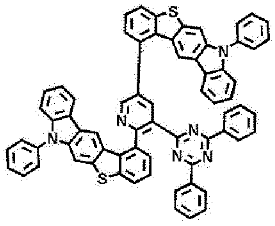
513

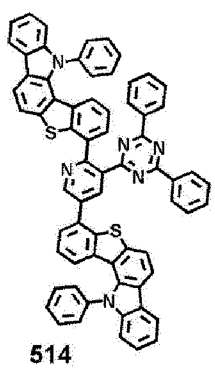
514

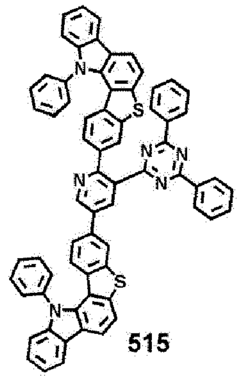
515

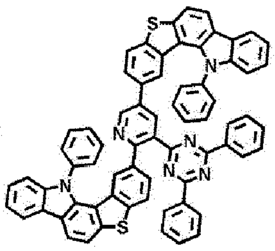
516

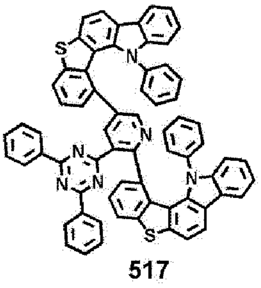
517

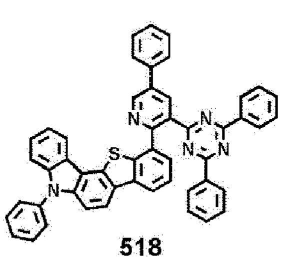
518

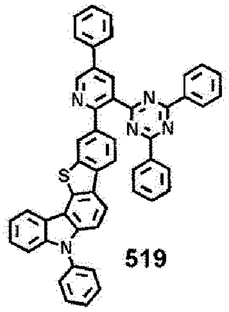
519

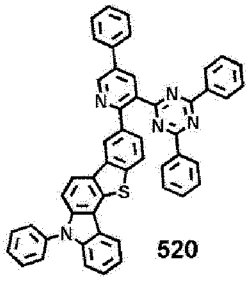
520

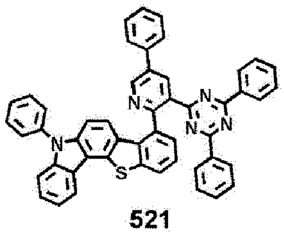
521

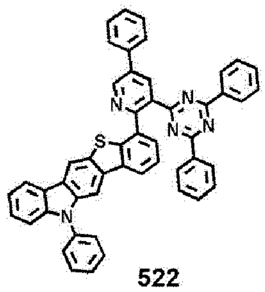
522

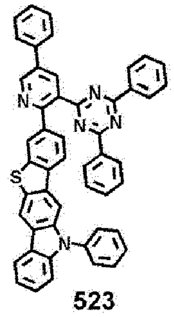
523

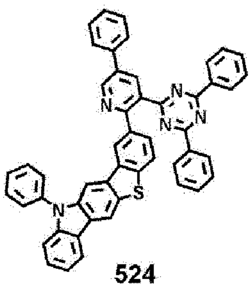
524

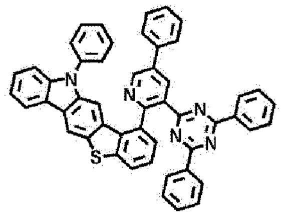
525

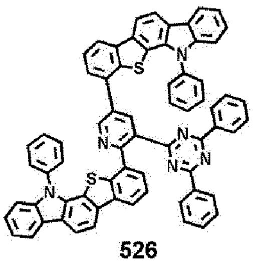

526

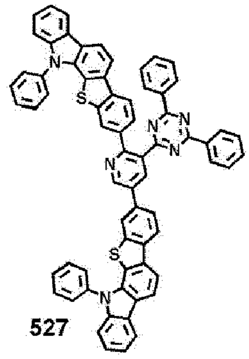

527

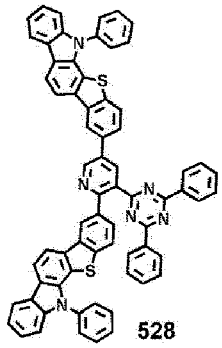

528

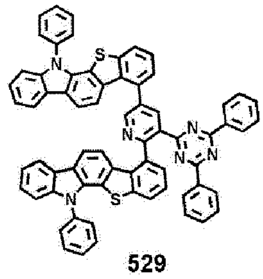

529

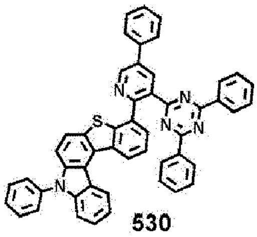

530

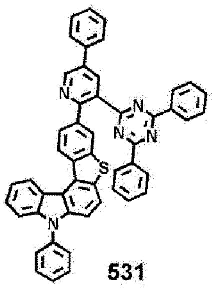

531

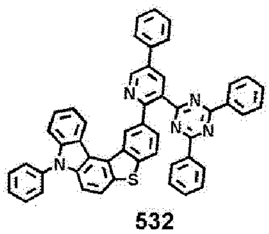

532

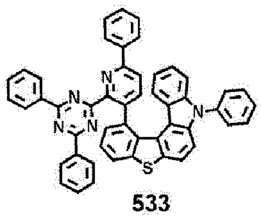

533

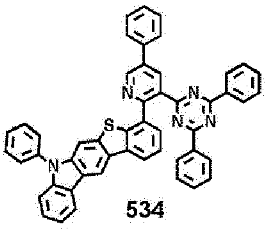

534

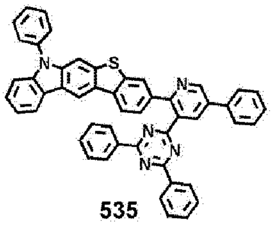

535

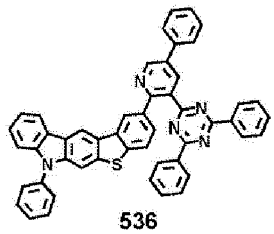

536

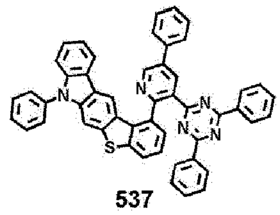

537

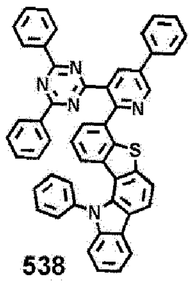
538

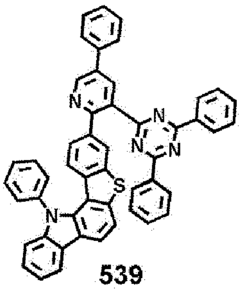
539

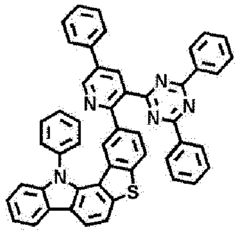
540

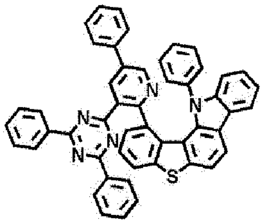
541

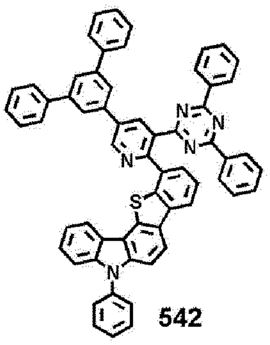
542

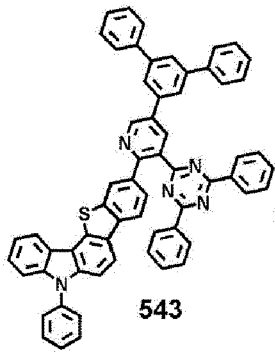
543

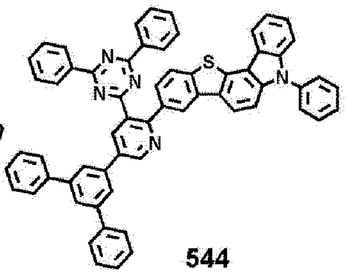
544

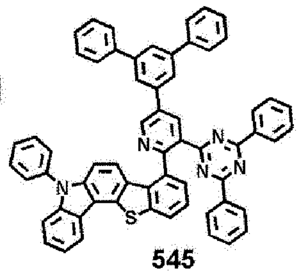
545

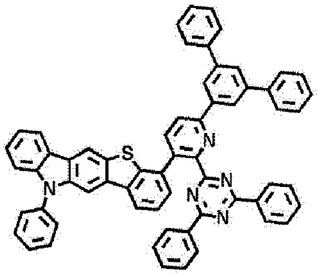
546

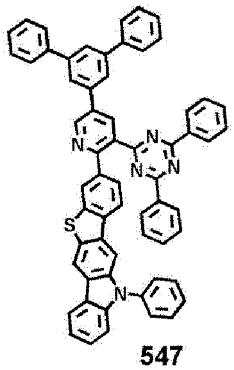
547

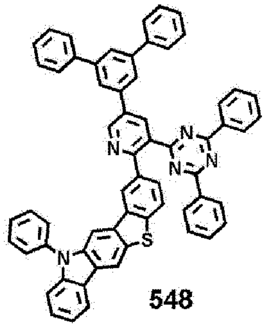
548

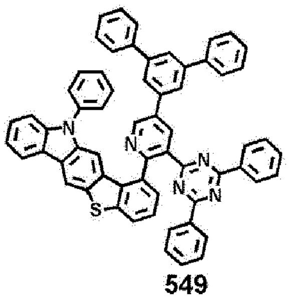
549

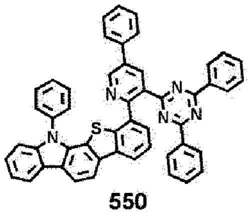

550

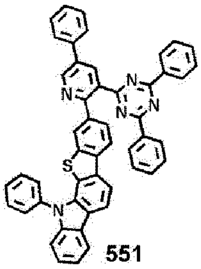

551

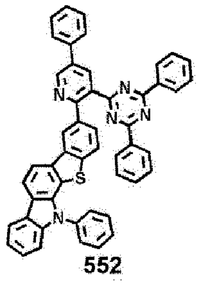

552

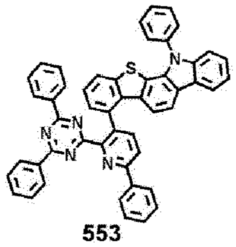

553

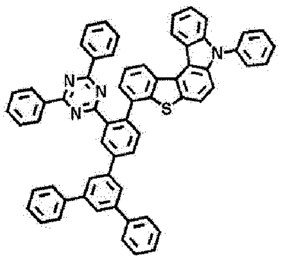

554

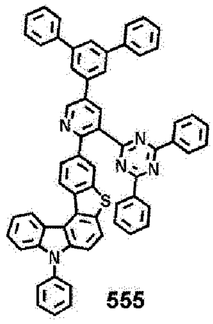

555

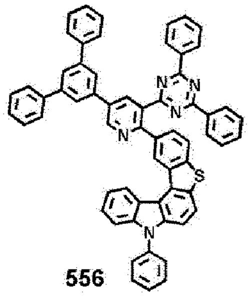

556

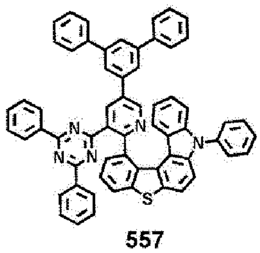

557

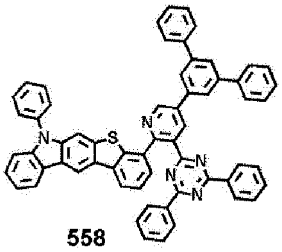

558

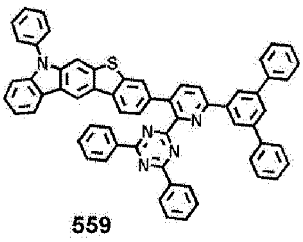

559

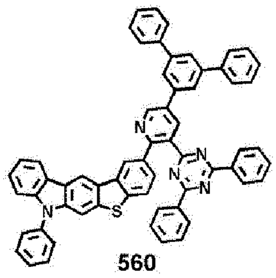

560

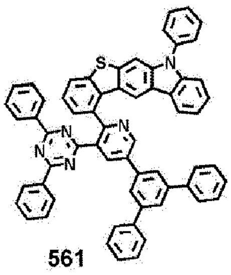

561

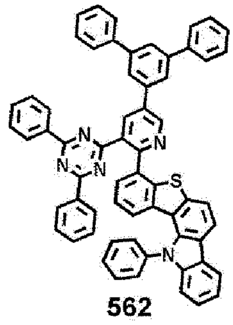

562

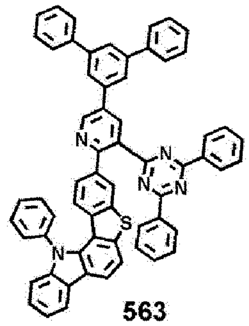

563

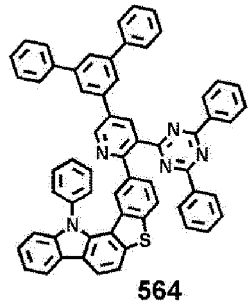

564

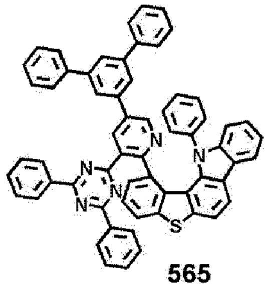

565

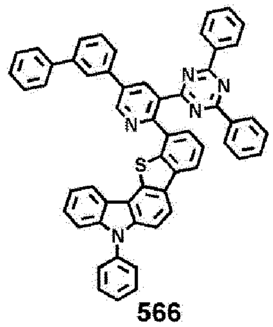

566

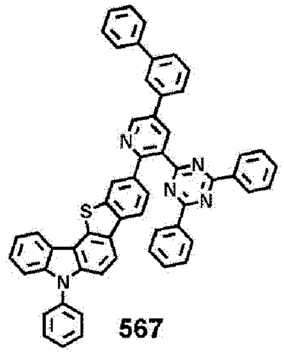

567

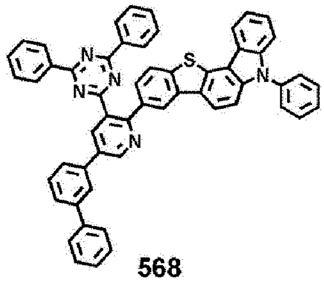

568

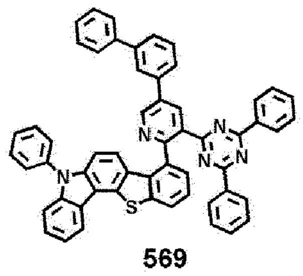

569

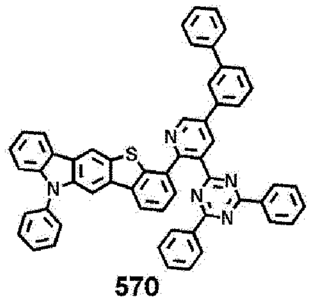

570

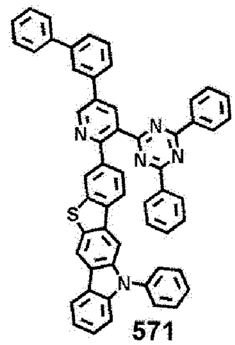

571

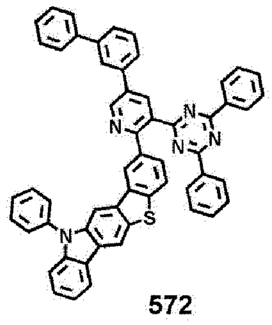

572

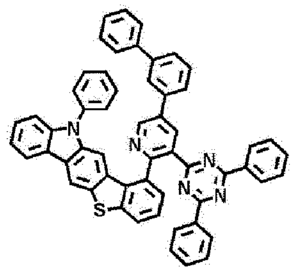

573

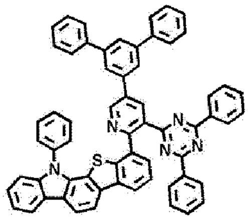
574

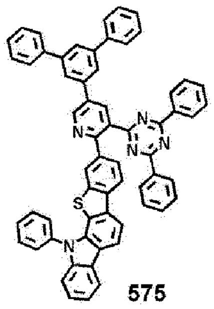
575

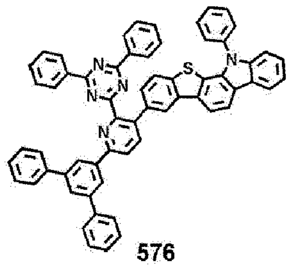
576

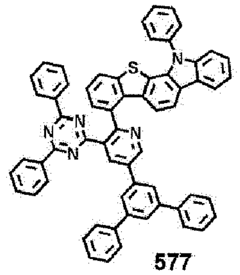
577

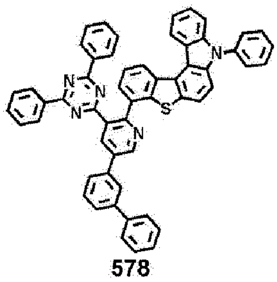
578

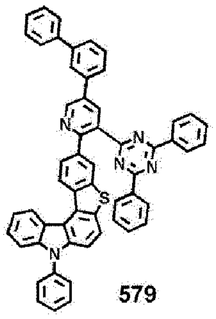
579

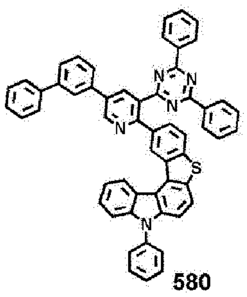
580

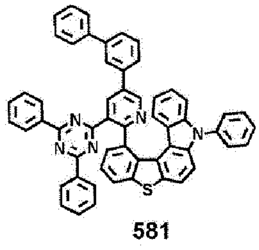
581

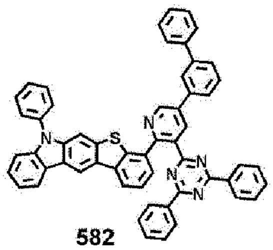
582

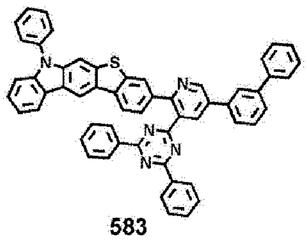
583

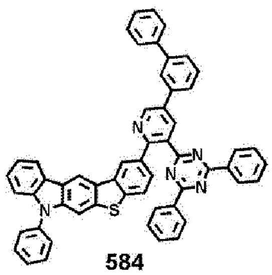
584

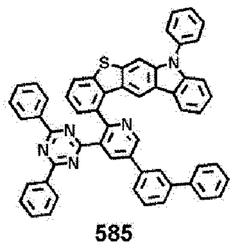
585

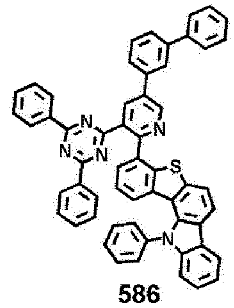
586

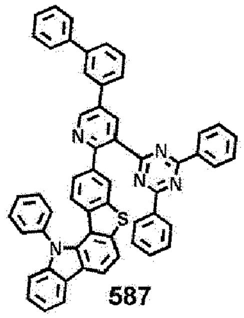
587

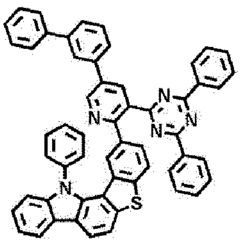
588

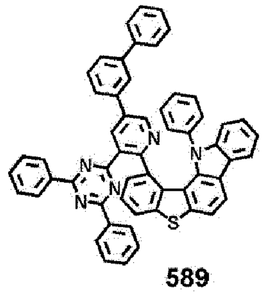
589

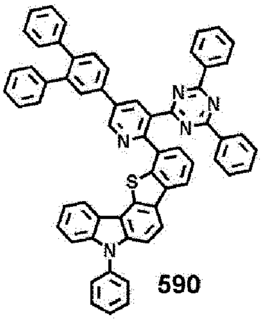
590

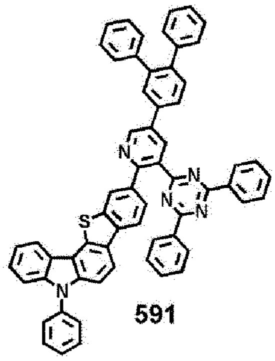
591

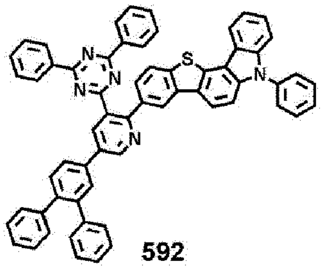
592

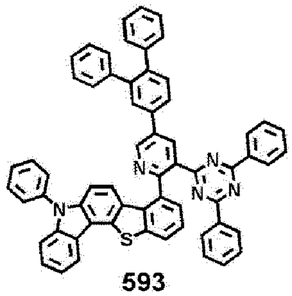
593

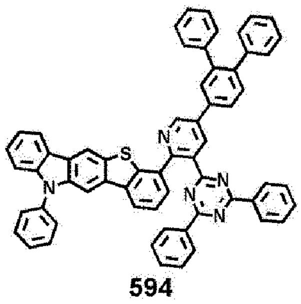
594

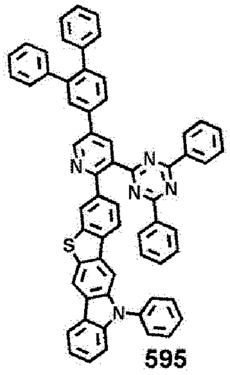
595

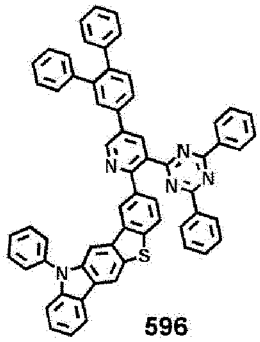
596

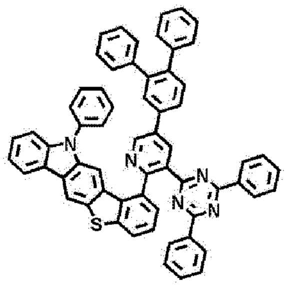
597

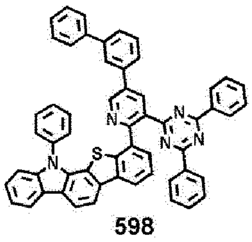
598

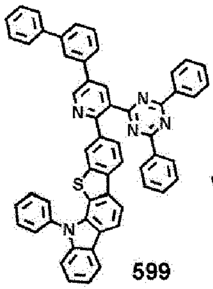
599

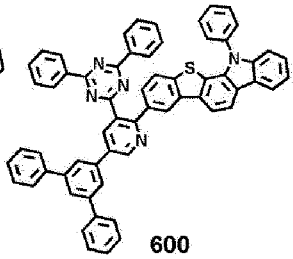
600

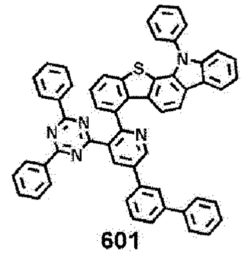
601

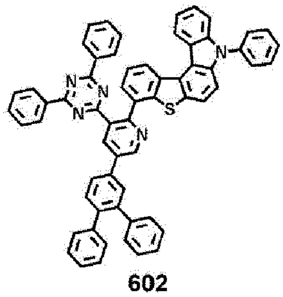
602

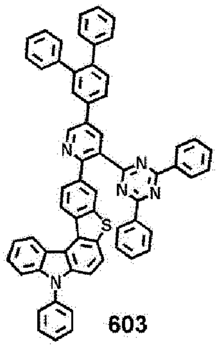
603

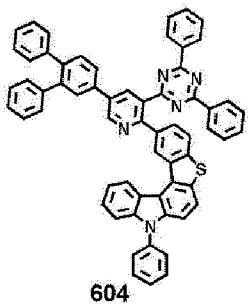
604

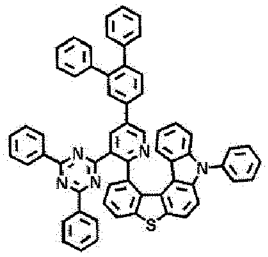
605

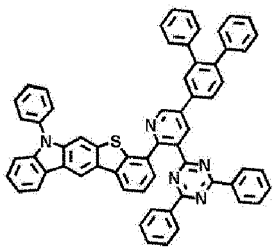
606

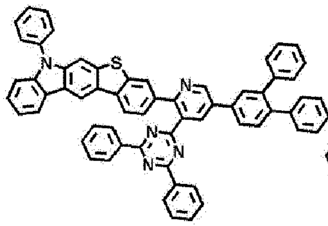
607

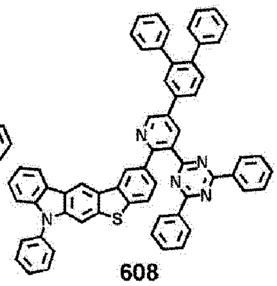
608

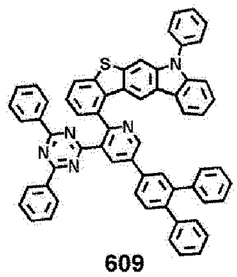
609

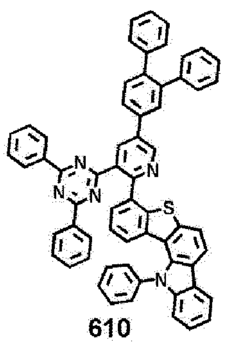
610

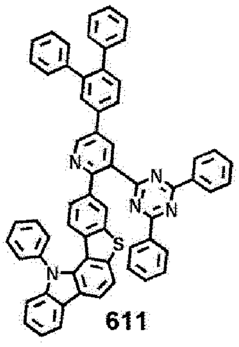
611

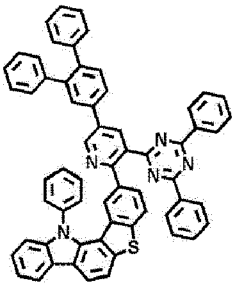
612

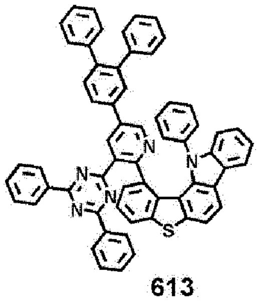
613

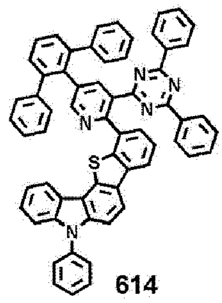
614

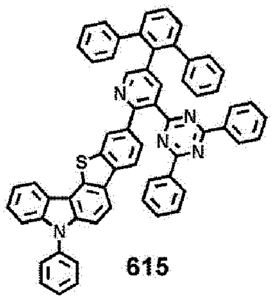
615

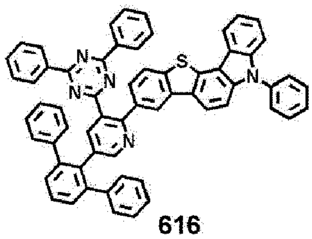
616

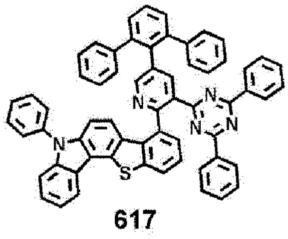
617

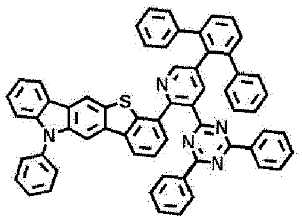
618

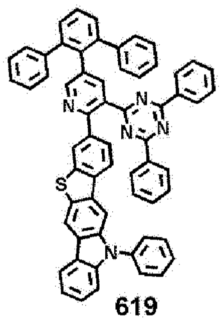
619

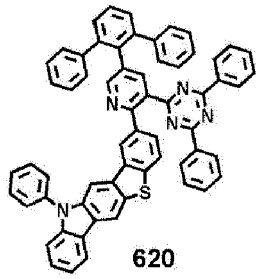
620

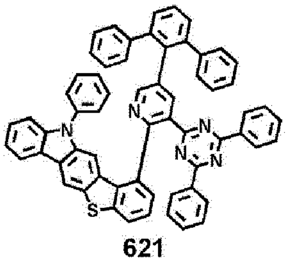
621

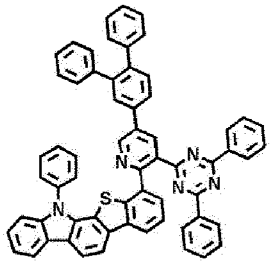
622

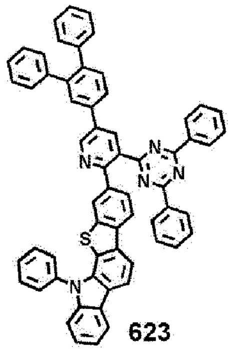
623

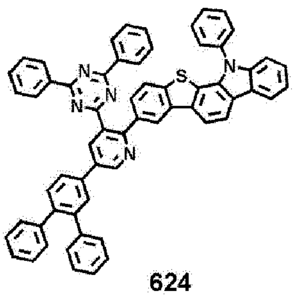
624

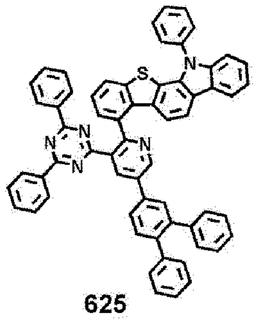
625

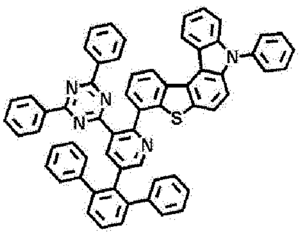
626

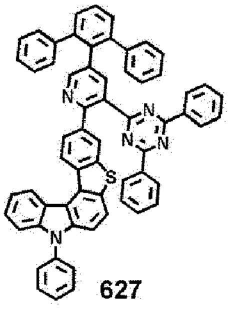
627

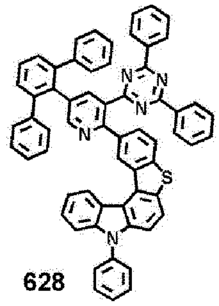
628

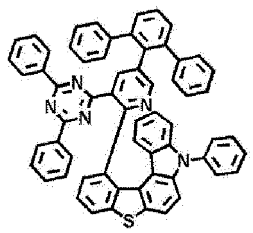
629

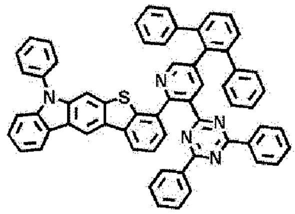
630

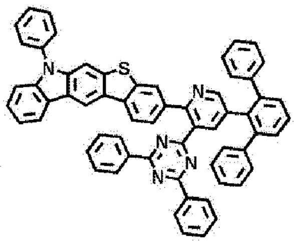
631

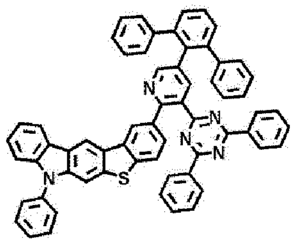
632

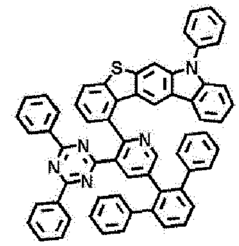
633

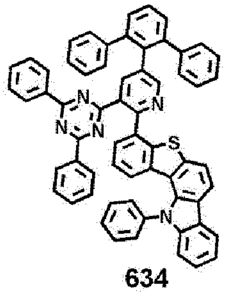
634

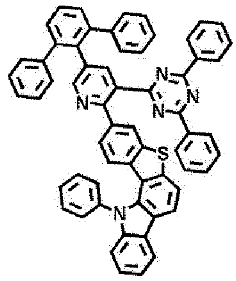
635

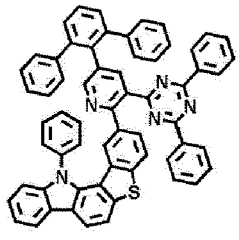
636

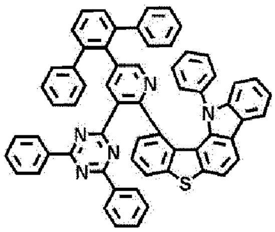
637

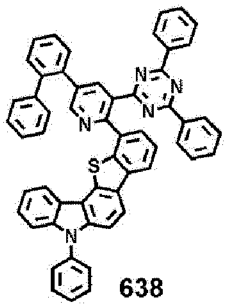
638

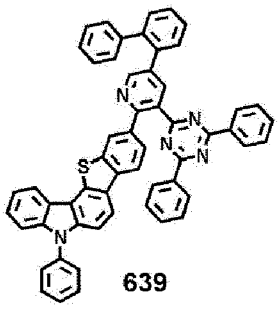
639

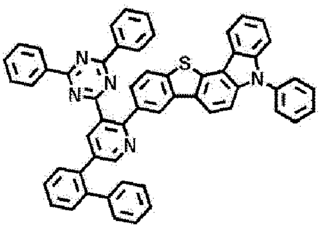
640

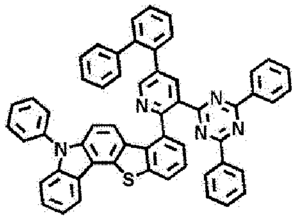
641

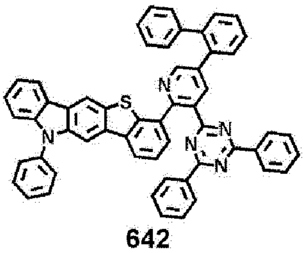
642

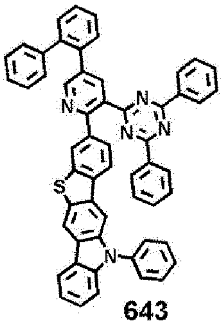
643

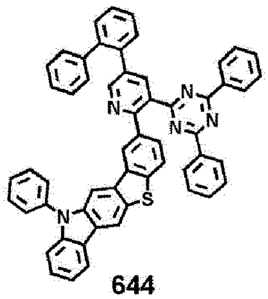
644

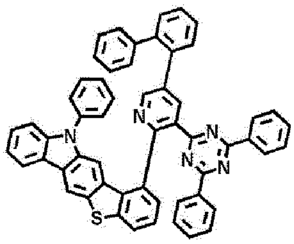
645

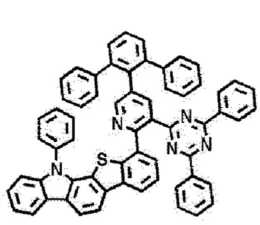

646

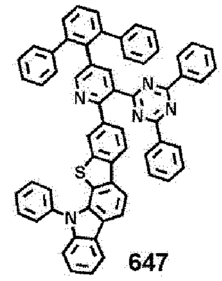

647

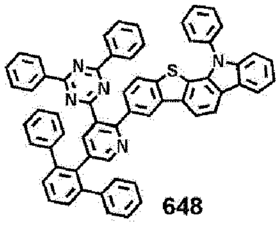

648

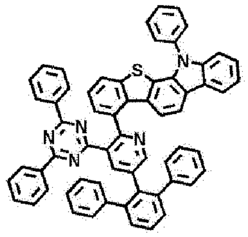

649

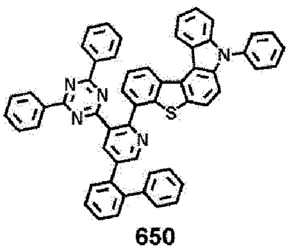

650

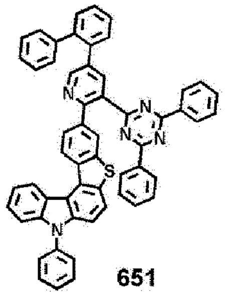

651

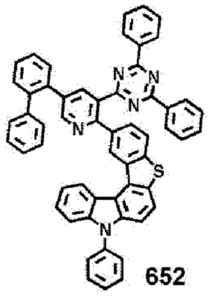

652

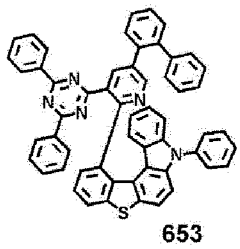

653

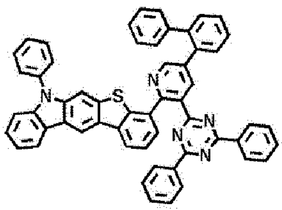

654

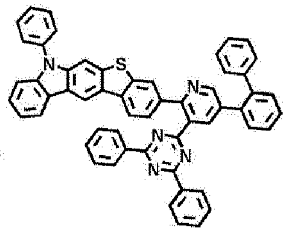

655

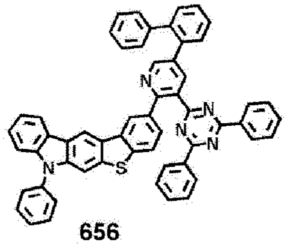

656

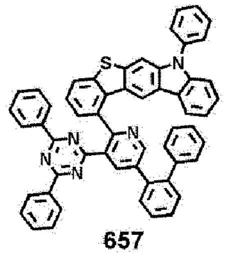

657

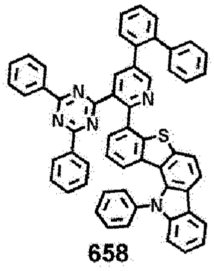
658

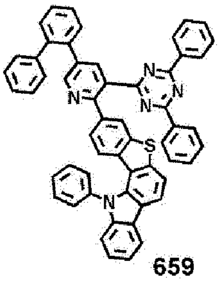
659

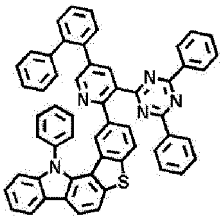
660

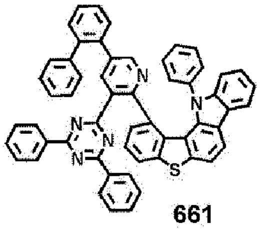
661

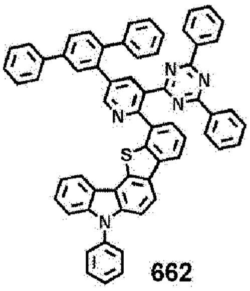
662

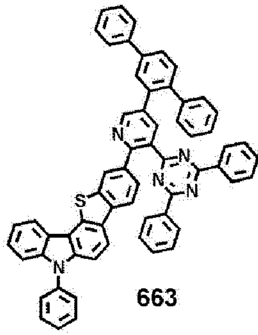
663

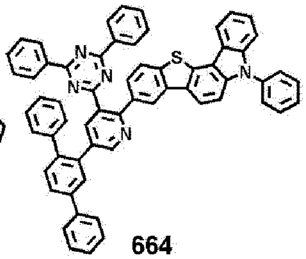
664

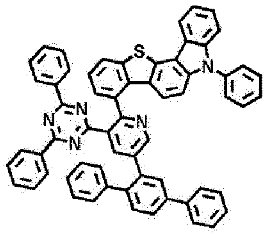
665

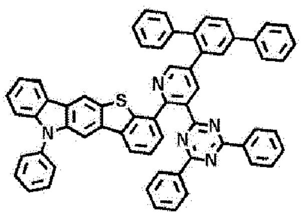
666

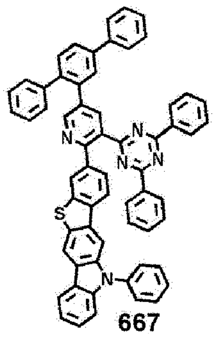
667

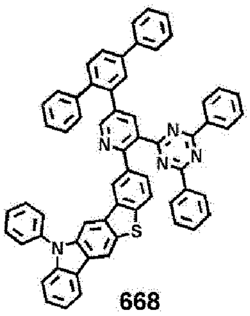
668

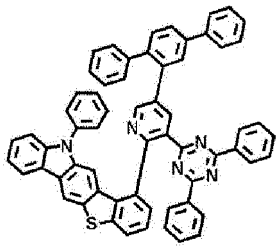
669

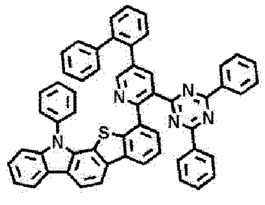
670

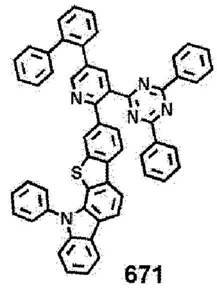
671

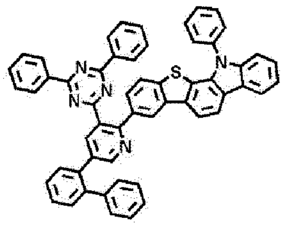
672

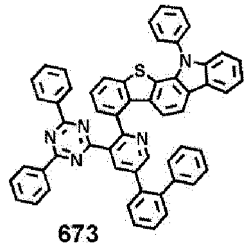
673

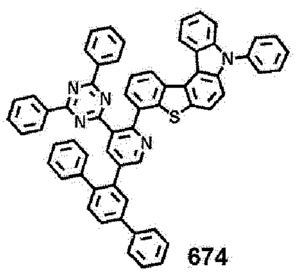
674

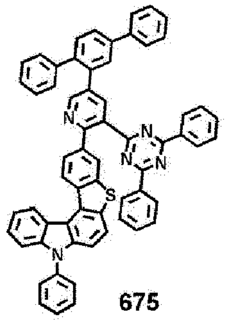
675

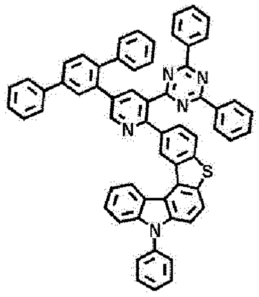
676

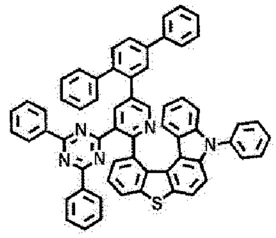
677

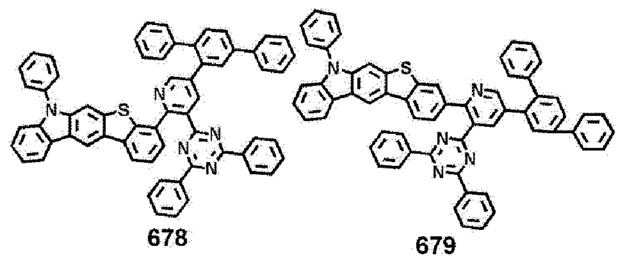
678

679

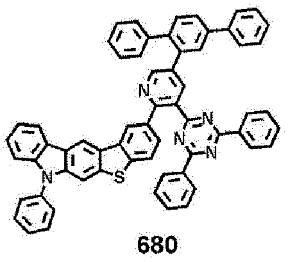
680

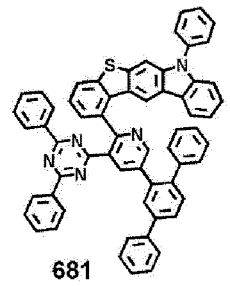
681

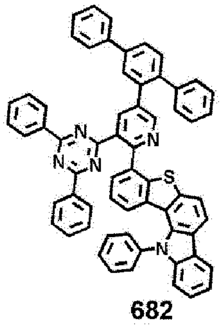
682

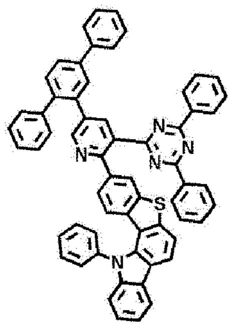
683

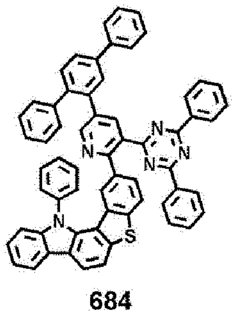
684

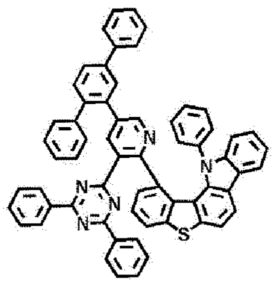
685

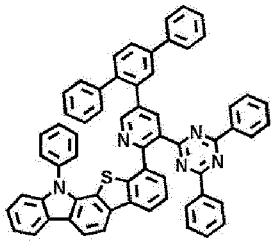
686

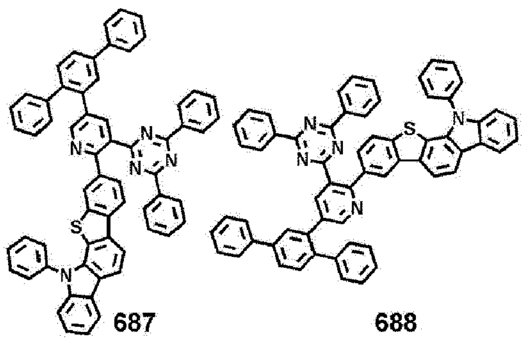
687

688

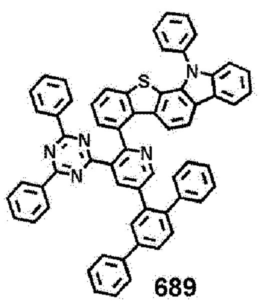
689

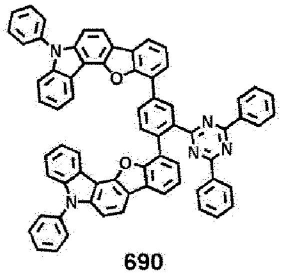
690

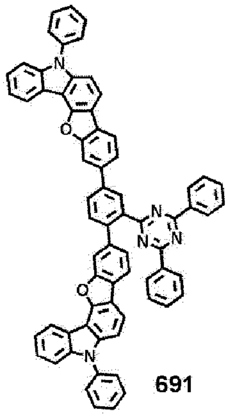
691

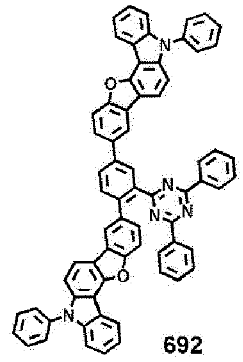
692

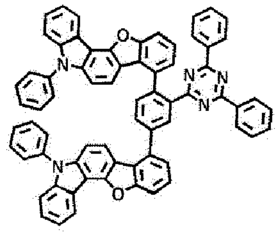
693

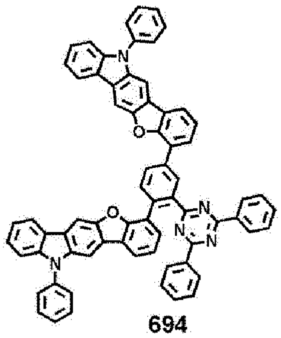
694

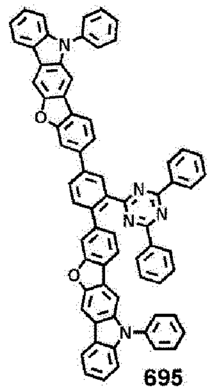
695

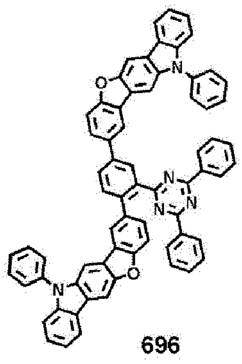
696

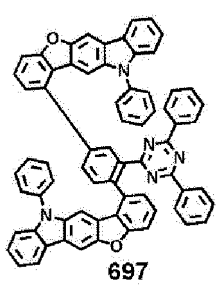
697

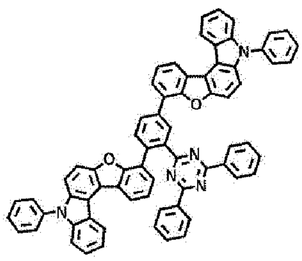
698

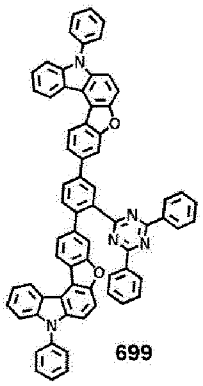
699

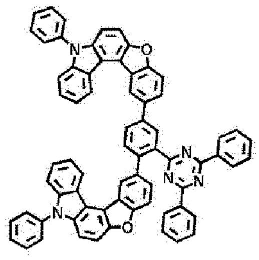
700

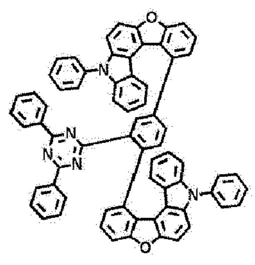
701

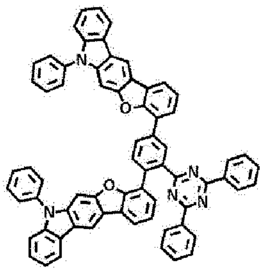
702

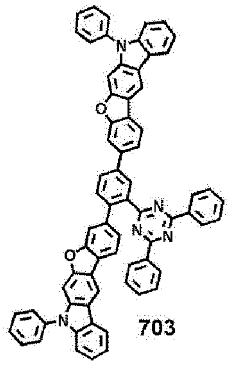
703

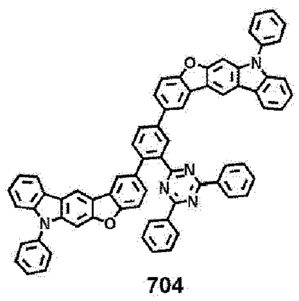
704

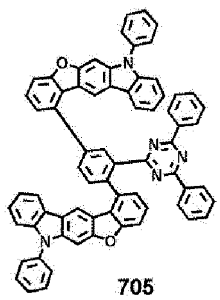
705

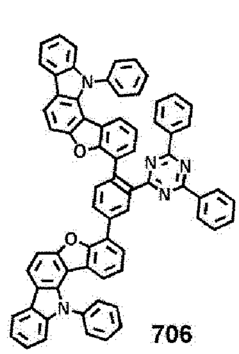
706

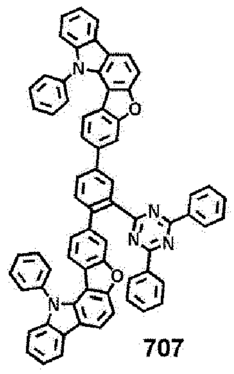
707

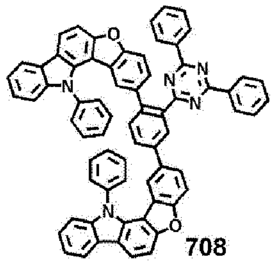
708

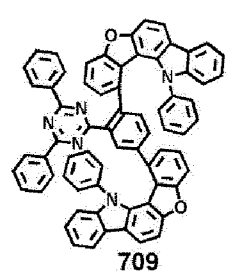
709

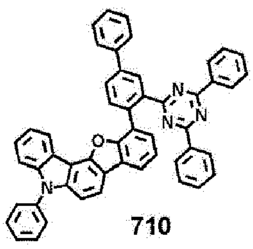
710

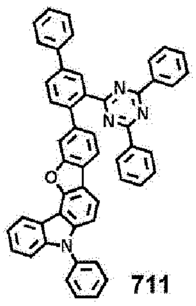
711

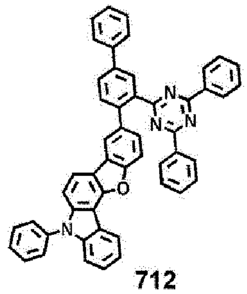
712

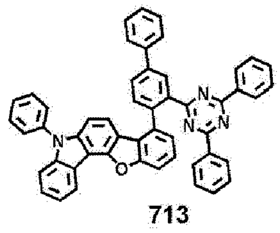
713

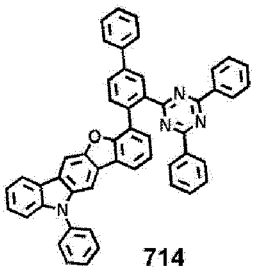
714

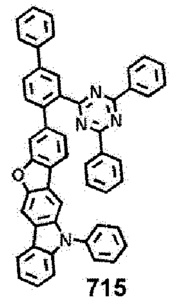
715

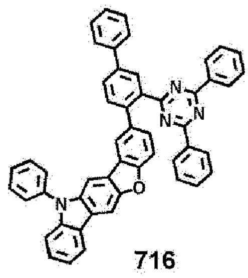
716

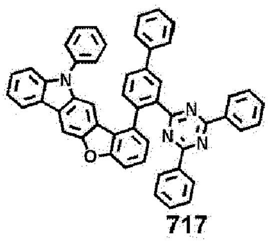
717

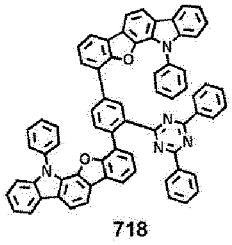
718

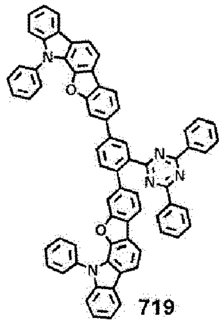
719

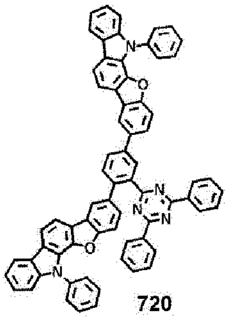
720

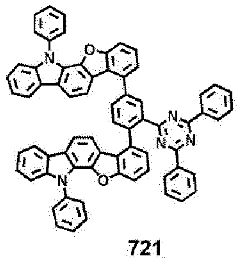
721

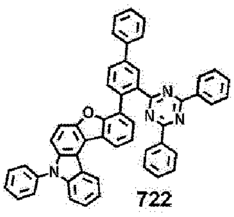
722

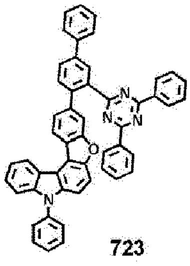
723

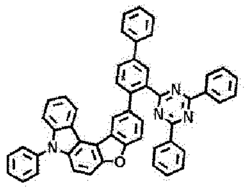
724

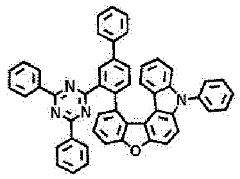
725

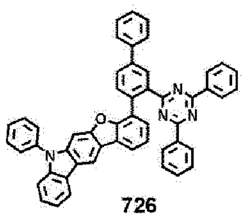
726

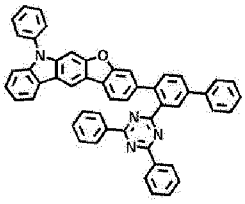
727

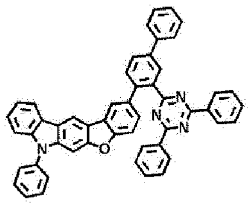
728

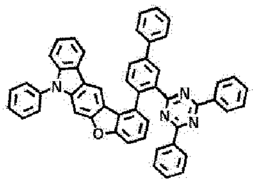
729

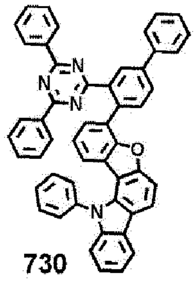
730

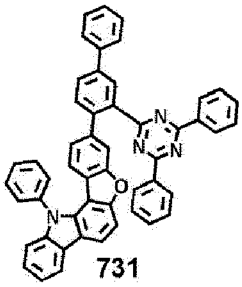
731

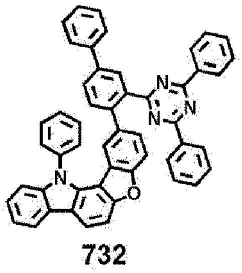
732

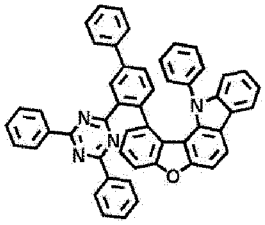
733

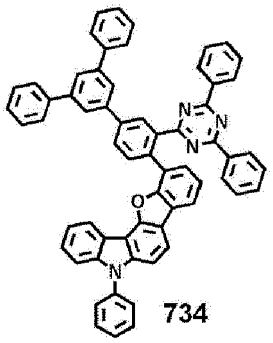
734

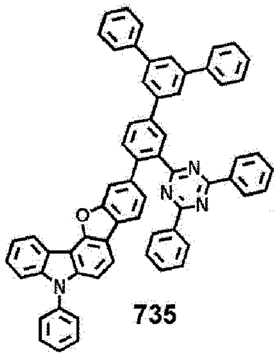
735

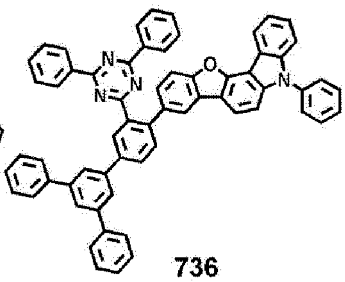
736

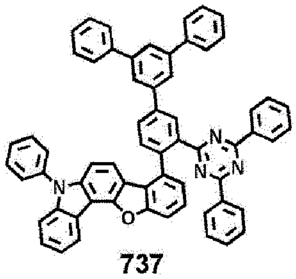
737

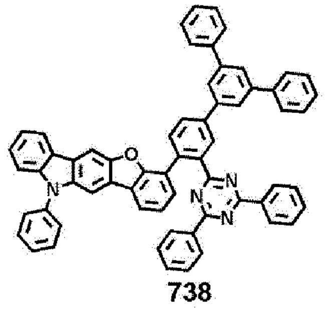
738

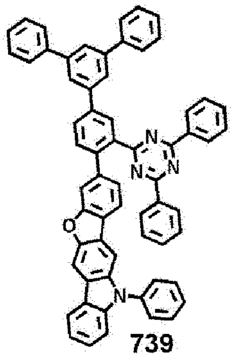
739

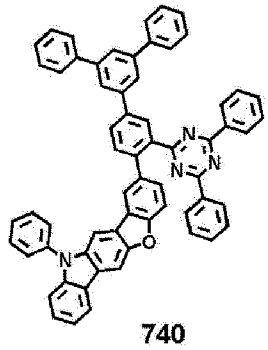
740

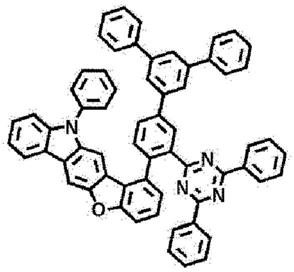
741

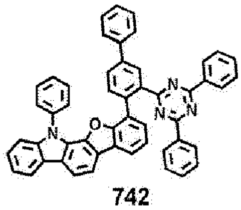
742

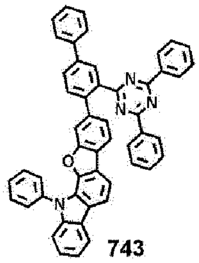
743

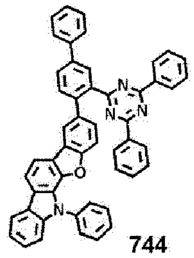
744

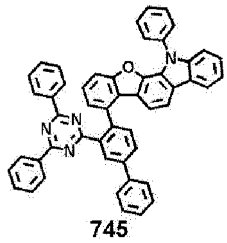
745

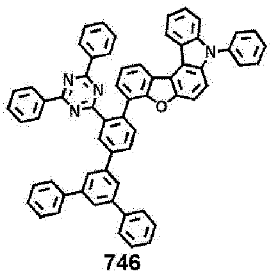
746

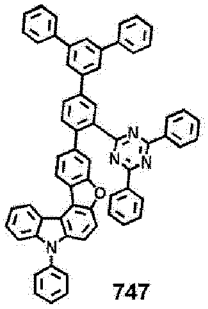
747

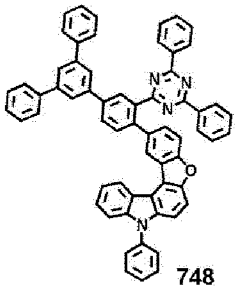
748

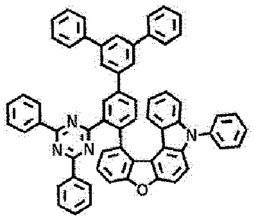
749

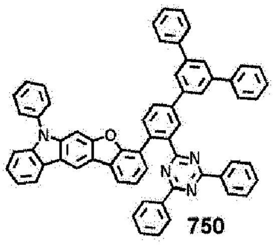
750

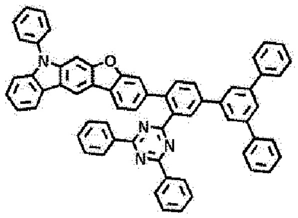
751

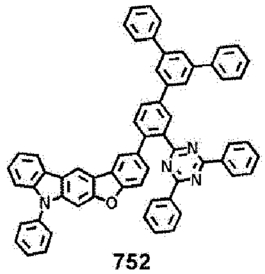
752

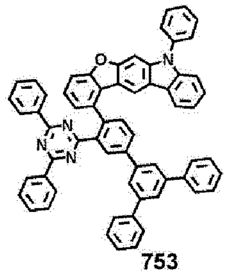
753

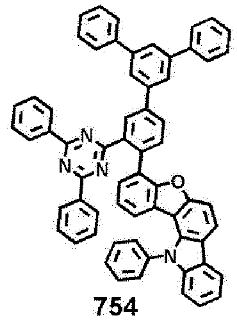
754

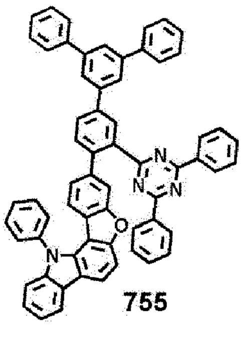
755

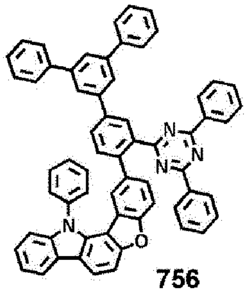
756

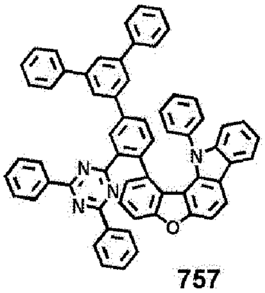
757

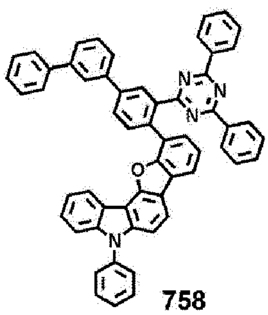
758

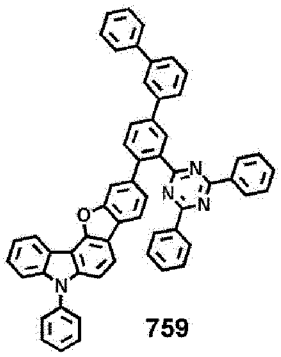
759

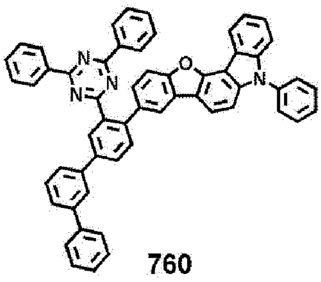
760

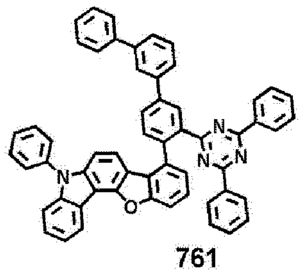
761

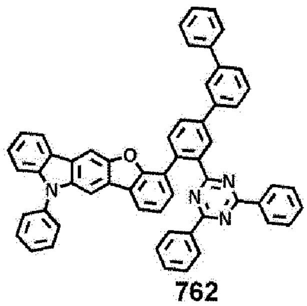
762

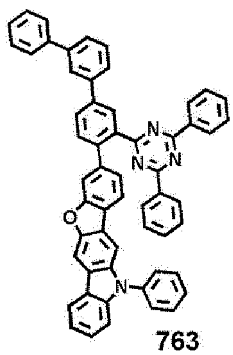
763

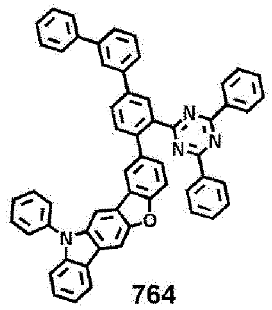
764

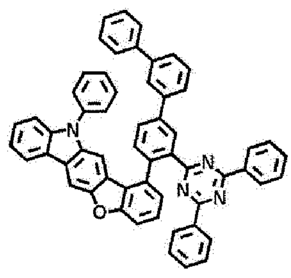
765

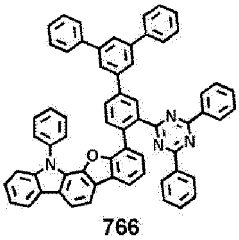
766

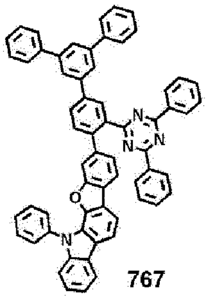
767

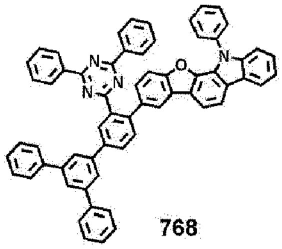
768

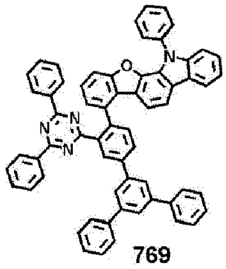
769

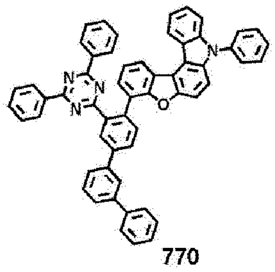
770

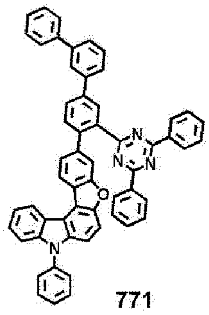
771

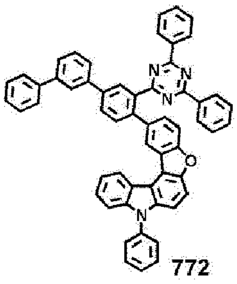
772

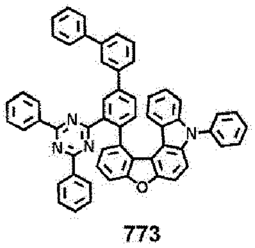
773

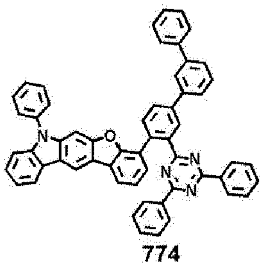
774

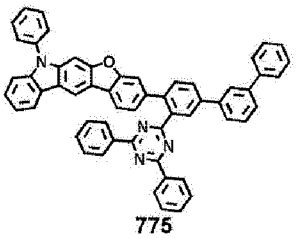
775

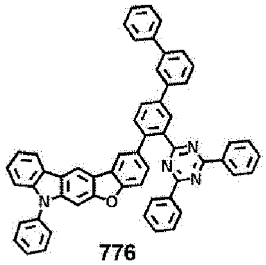
776

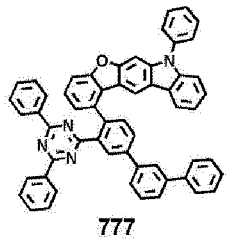
777

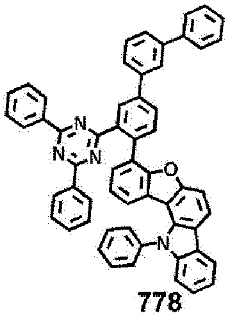
778

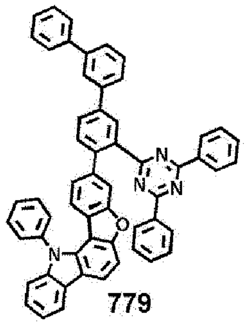
779

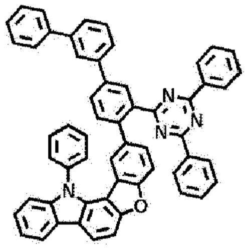
780

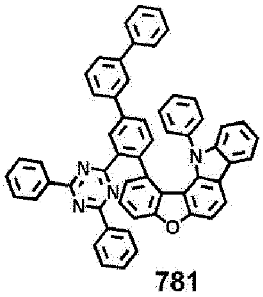
781

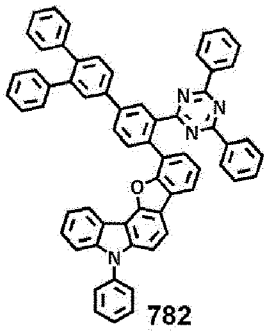
782

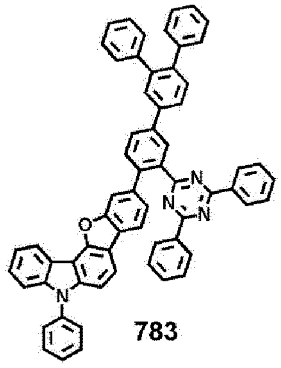
783

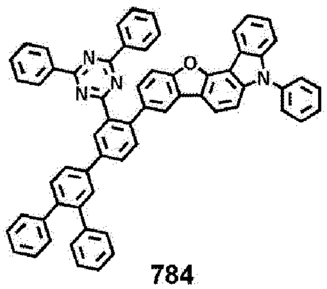
784

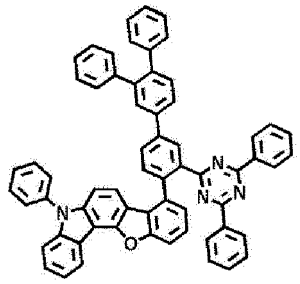
785

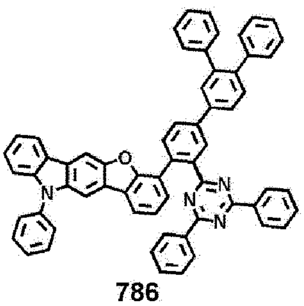
786

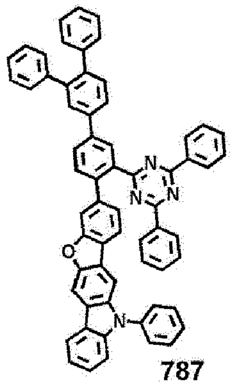
787

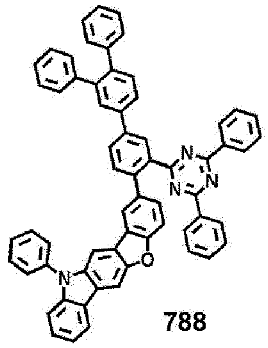
788

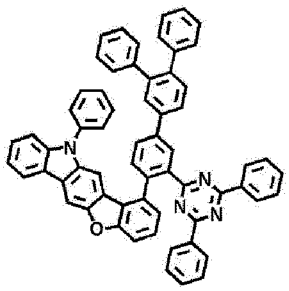
789

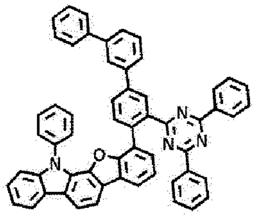
790

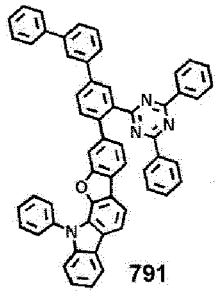
791

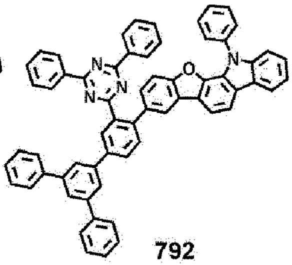
792

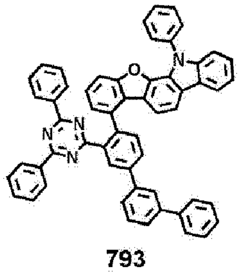
793

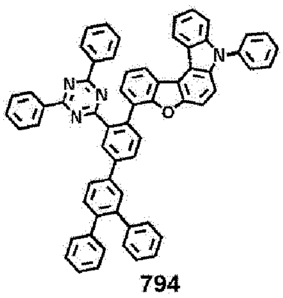
794

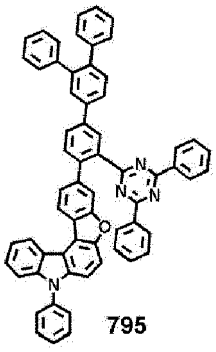
795

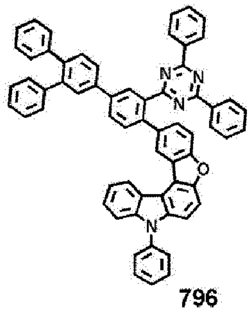
796

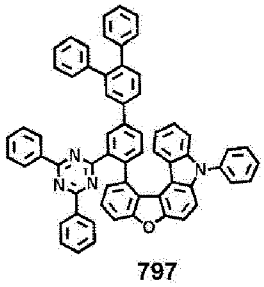
797

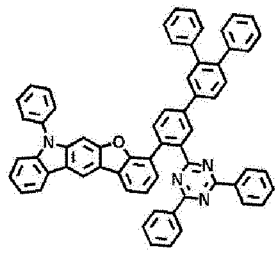
798

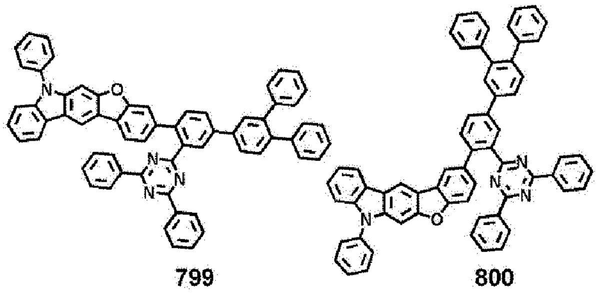
799

800

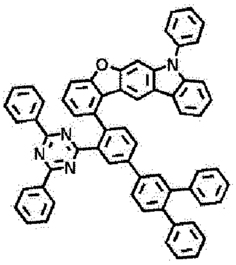
801

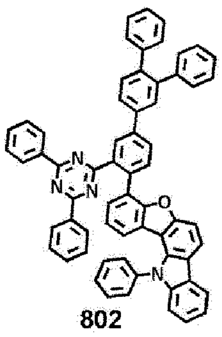

802

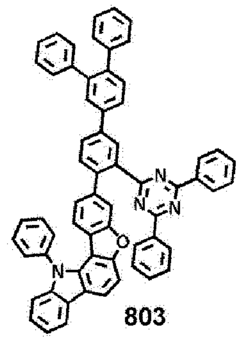

803

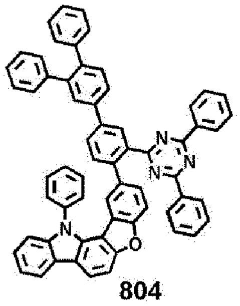

804

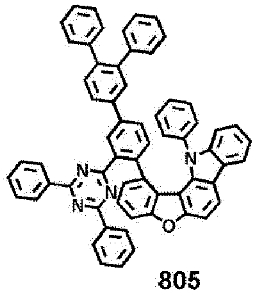

805

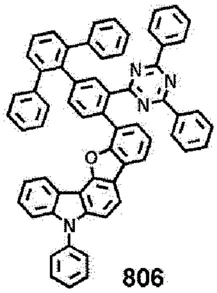

806

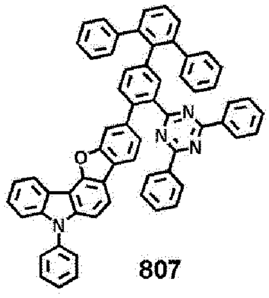

807

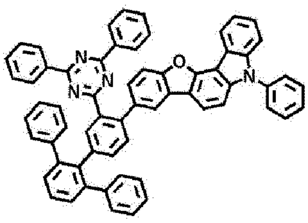

808

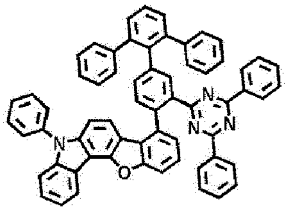

809

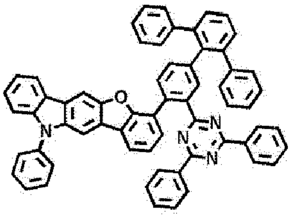

810

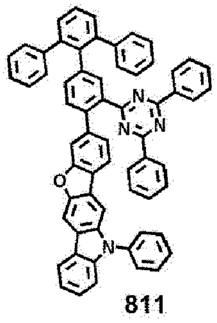

811

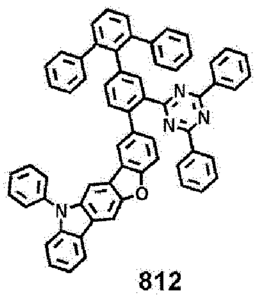

812

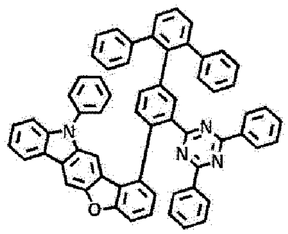

813

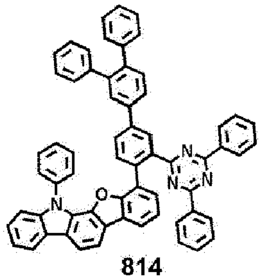
814

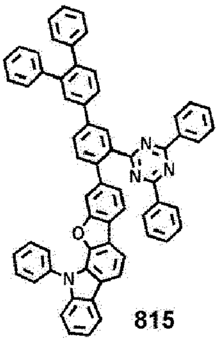
815

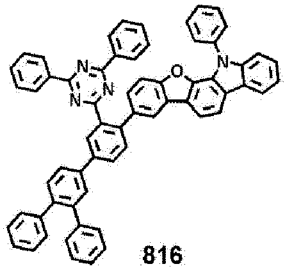
816

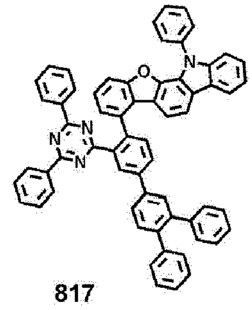
817

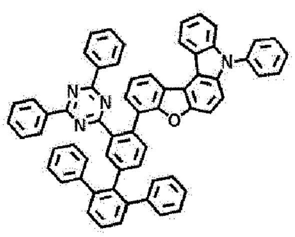
818

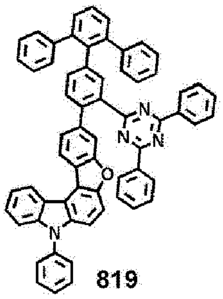
819

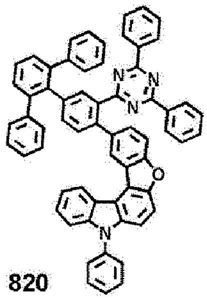
820

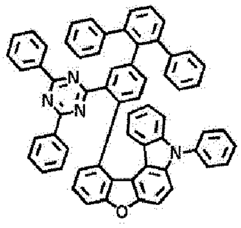
821

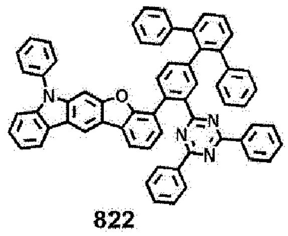
822

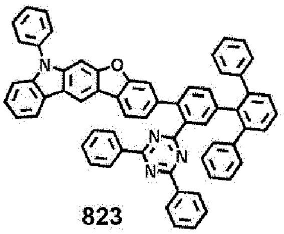
823

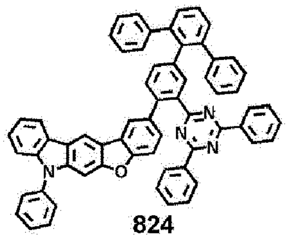
824

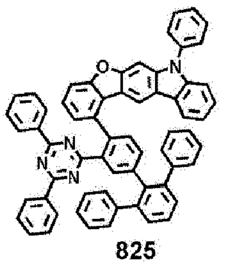
825

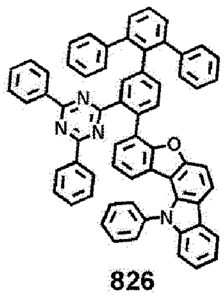
826

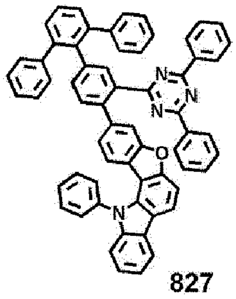
827

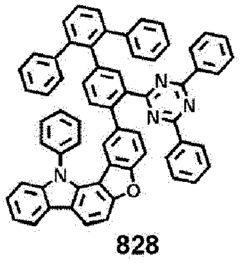
828

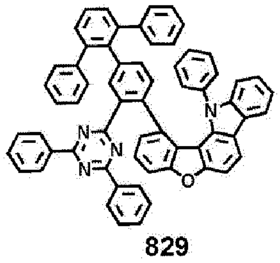
829

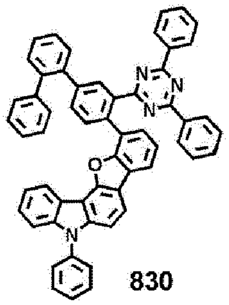
830

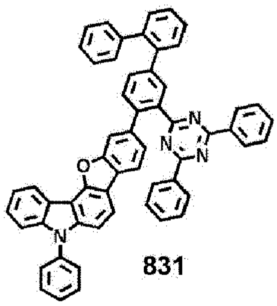
831

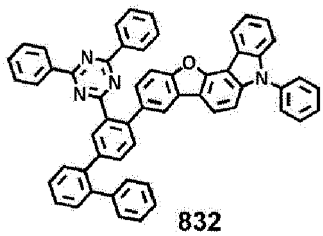
832

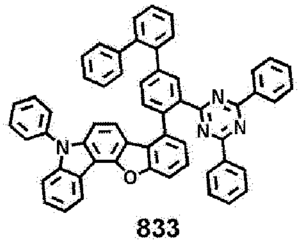
833

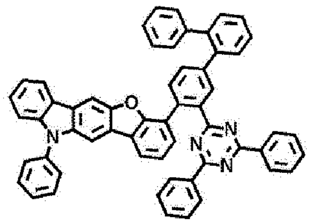
834

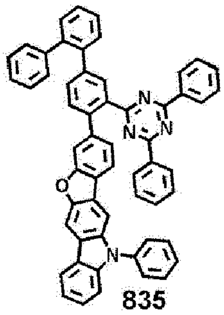
835

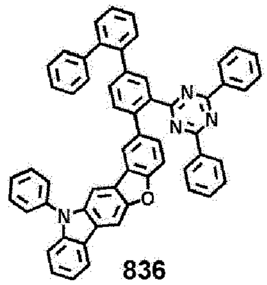
836

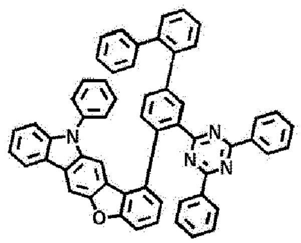
837

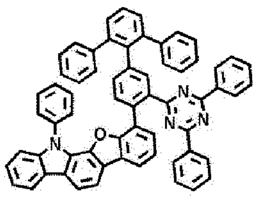

838

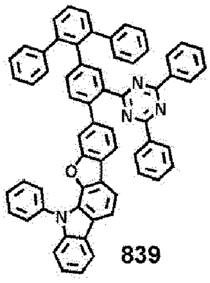

839

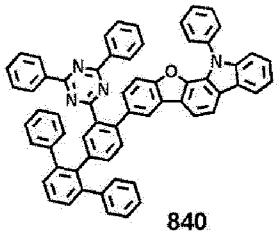

840

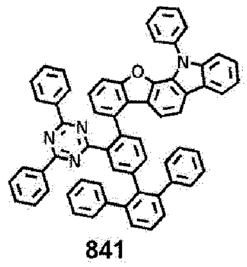

841

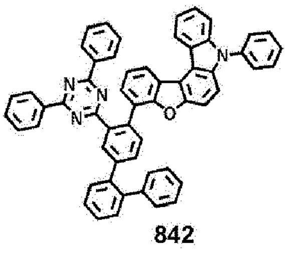

842

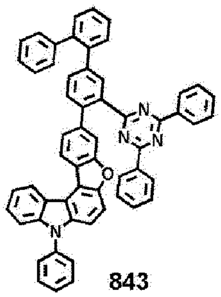

843

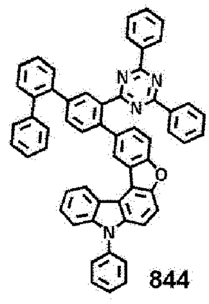

844

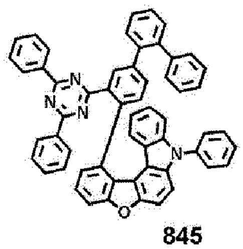

845

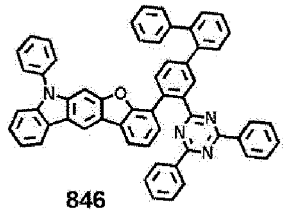

846

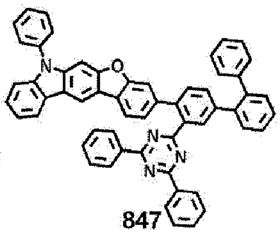

847

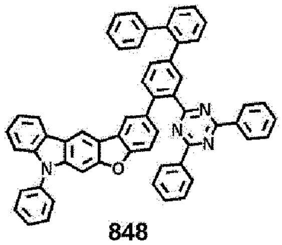

848

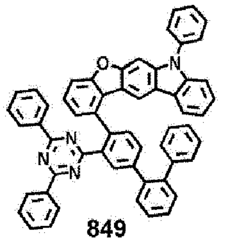

849

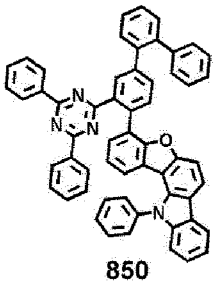

850

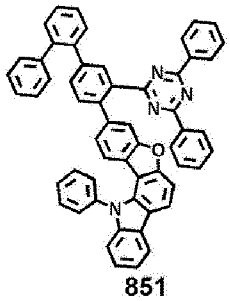

851

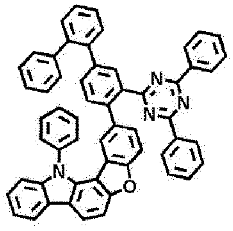

852

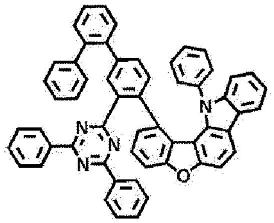

853

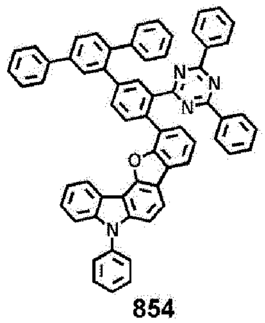

854

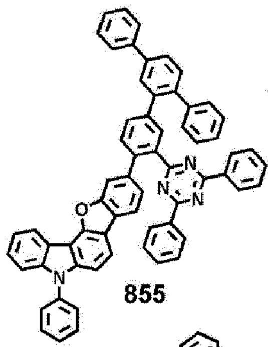

855

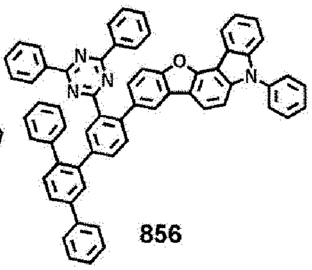

856

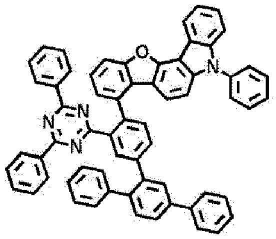

857

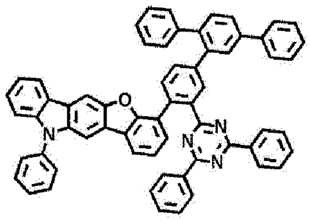

858

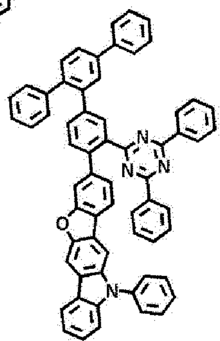

859

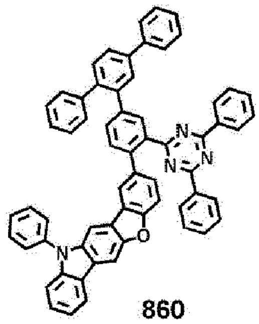

860

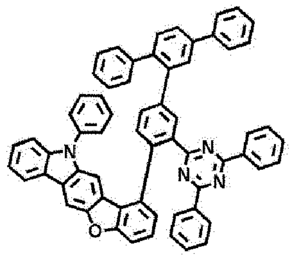

861

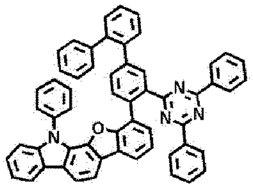

862

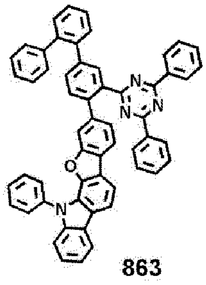

863

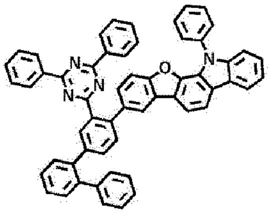

864

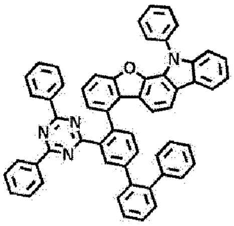

865

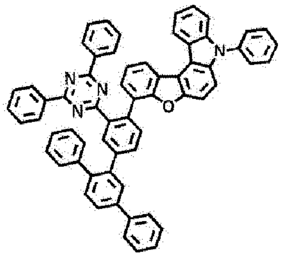

866

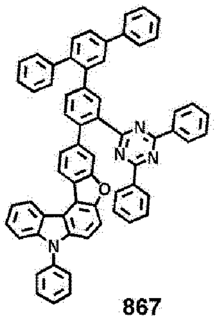

867

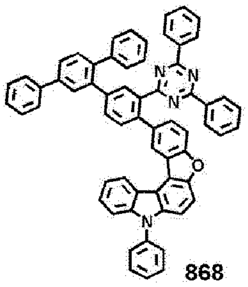

868

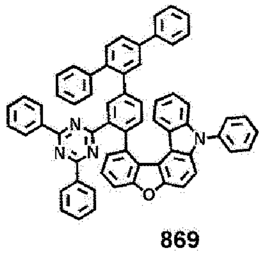

869

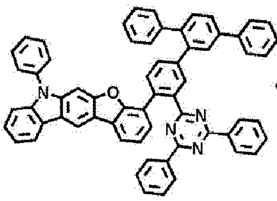

870

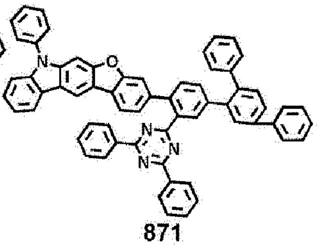

871

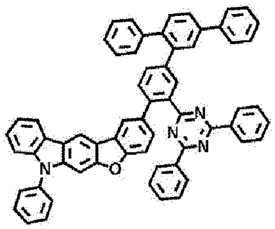

872

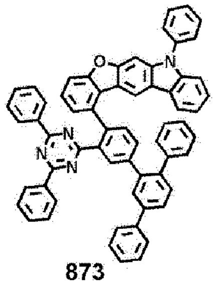

873

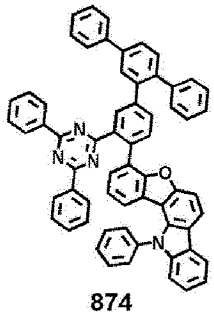

874

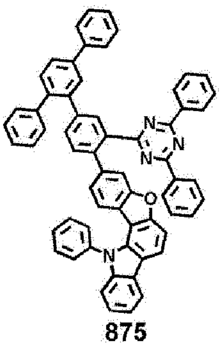

875

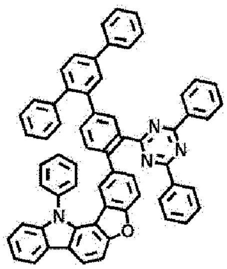

876

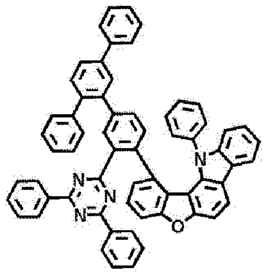

877

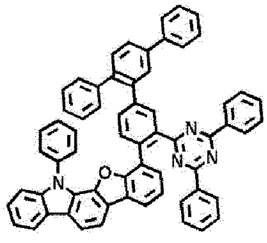

878

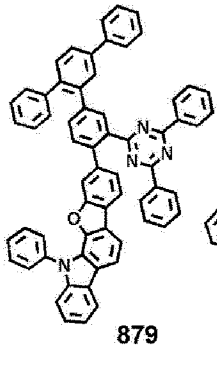

879

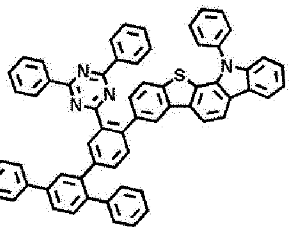

880

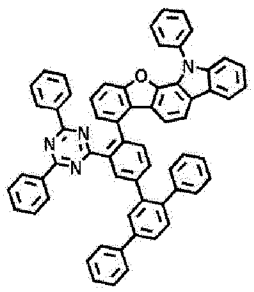

881

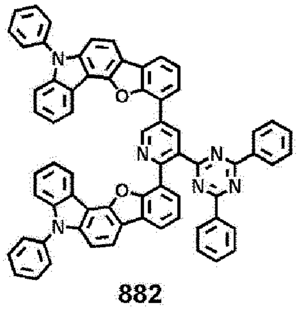
882

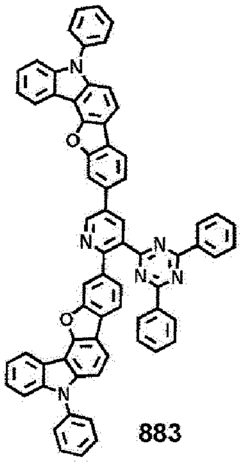
883

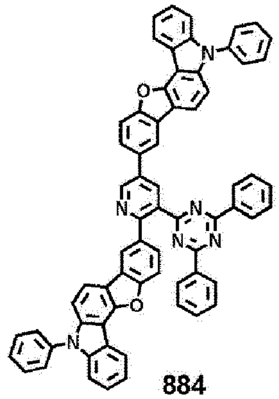
884

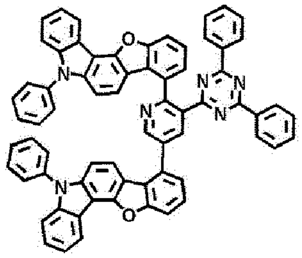
885

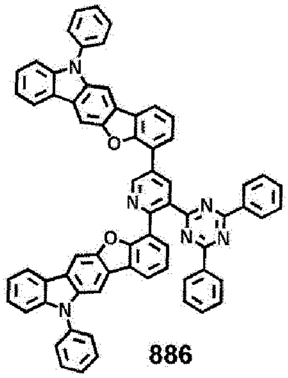
886

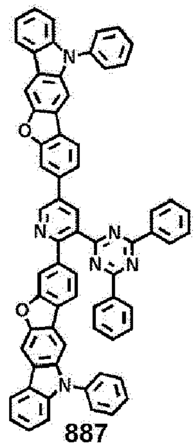
887

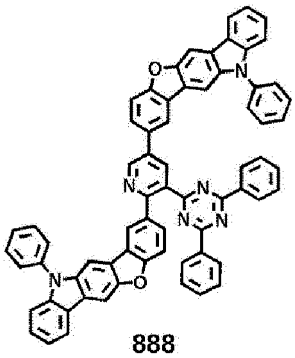
888

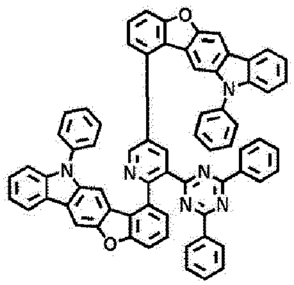
889

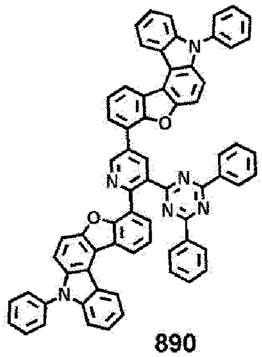
890

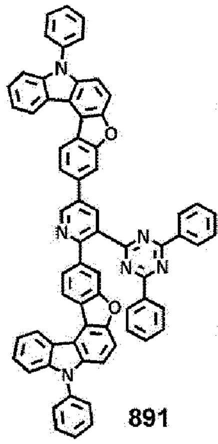
891

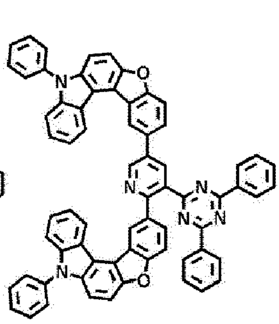
892

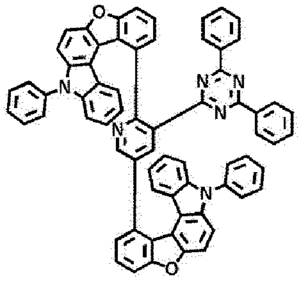
893

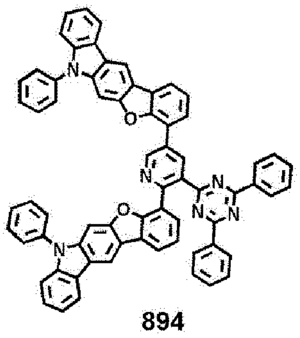
894

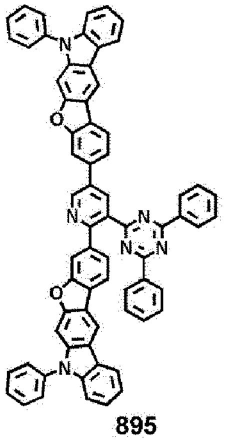
895

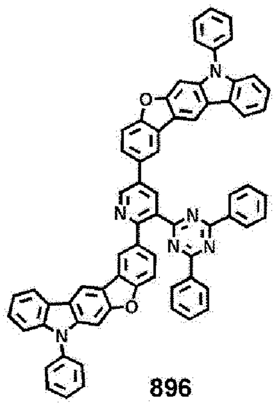
896

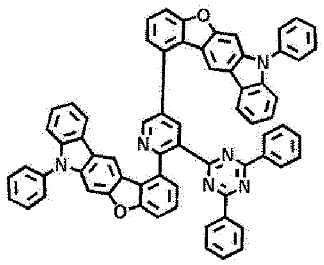
897

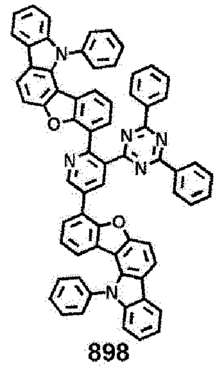

898

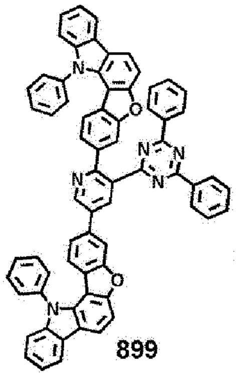

899

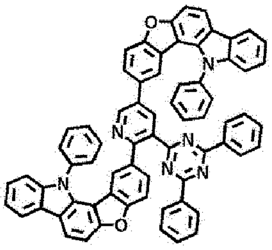

900

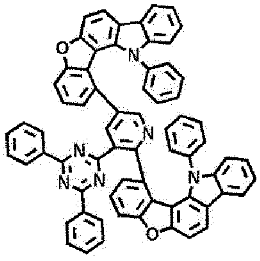

901

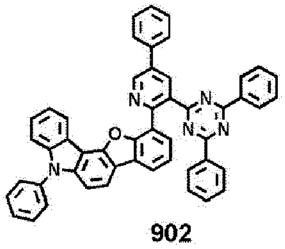

902

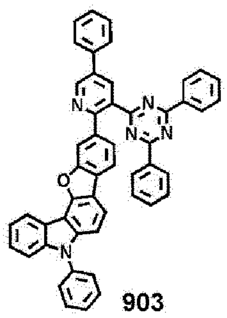

903

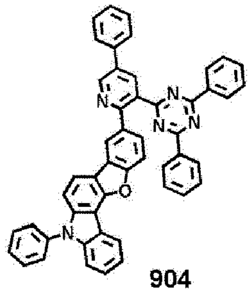

904

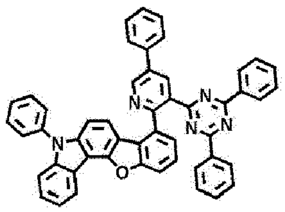

905

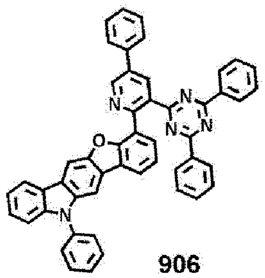

906

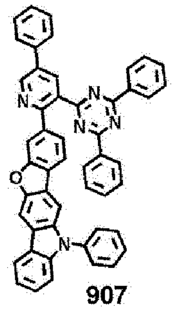

907

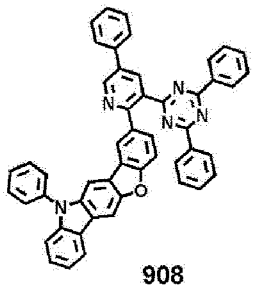

908

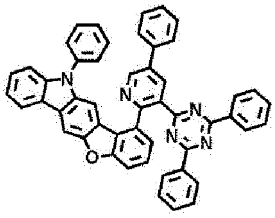

909

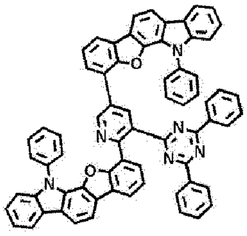

910

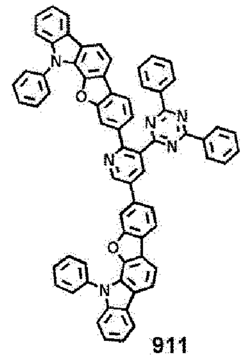

911

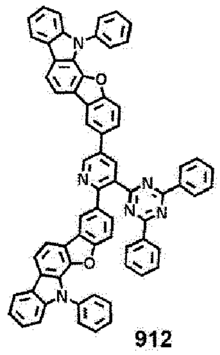

912

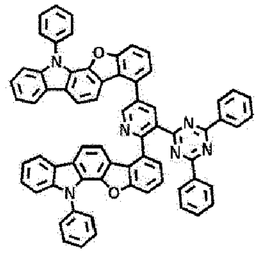

913

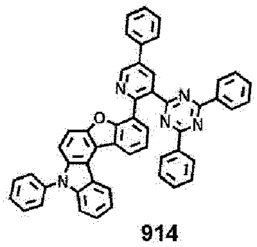

914

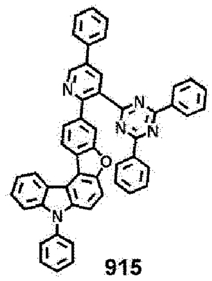

915

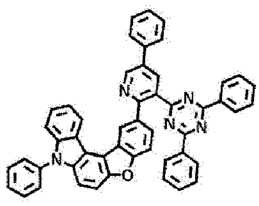

916

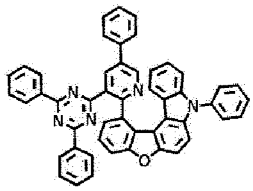

917

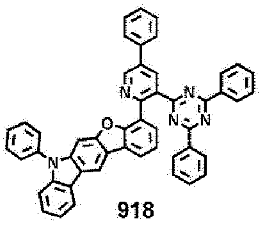

918

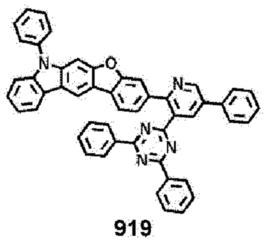

919

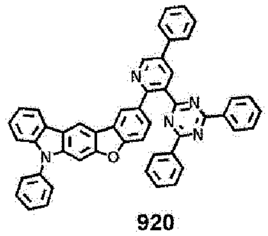

920

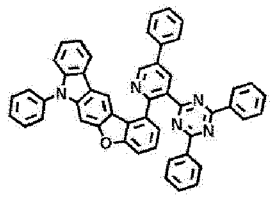

921

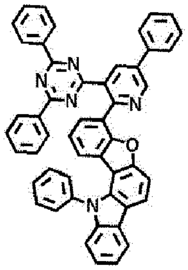

922

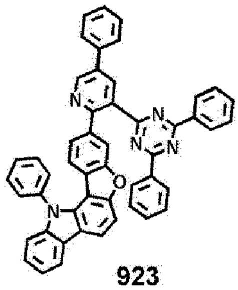

923

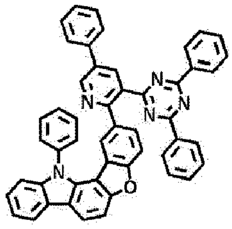

924

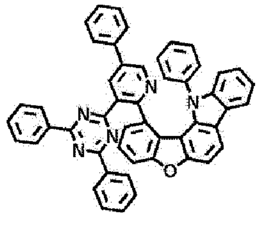

925

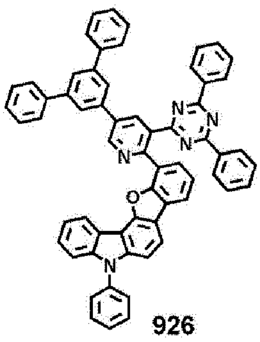

926

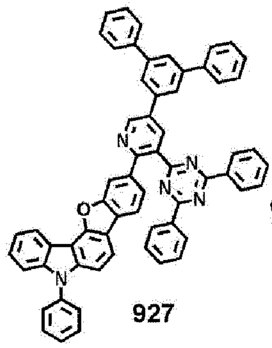

927

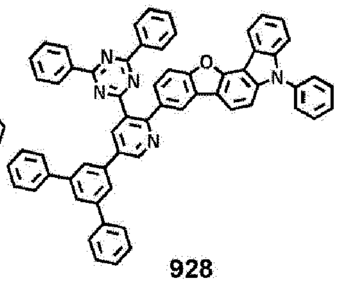

928

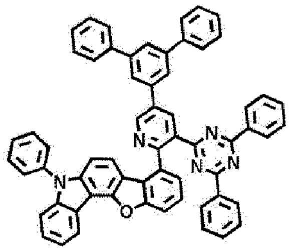

929

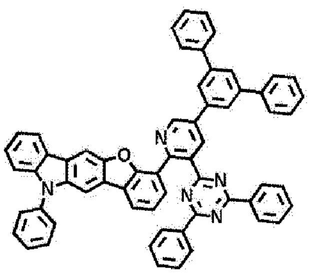

930

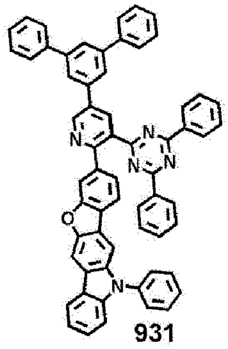

931

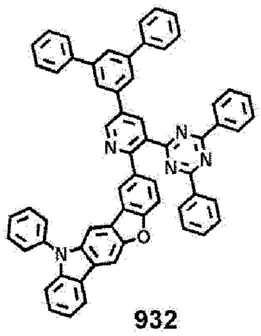

932

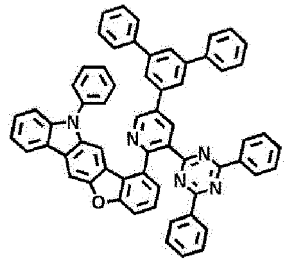

933

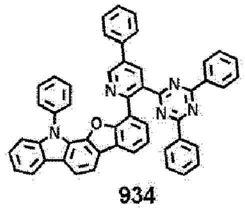
934

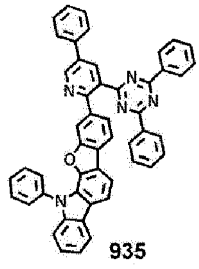
935

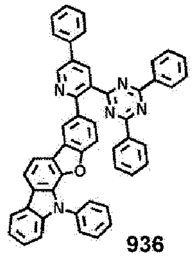
936

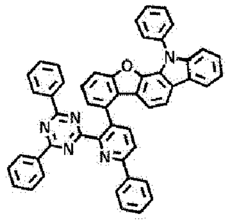
937

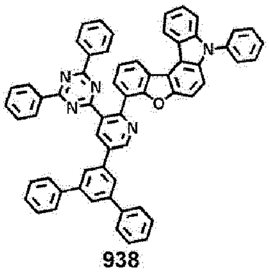
938

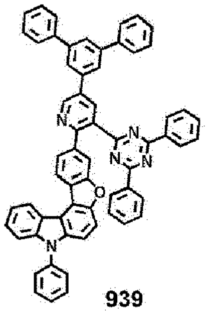
939

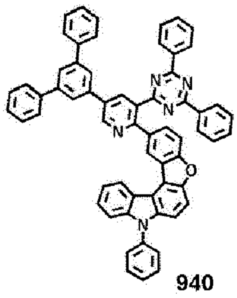
940

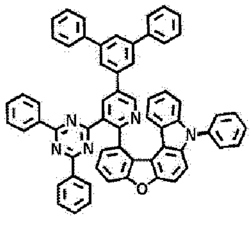
941

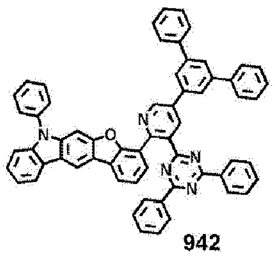
942

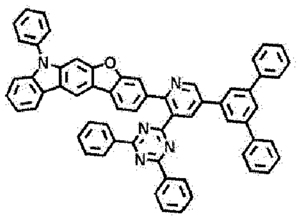
943

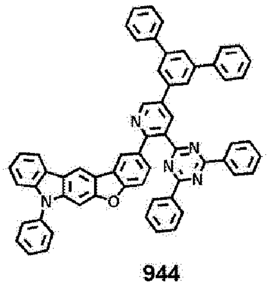
944

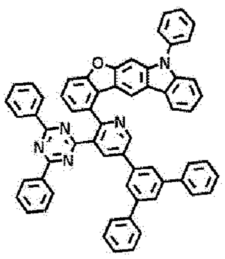
945

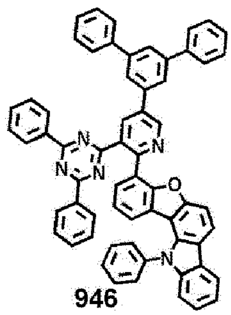
946

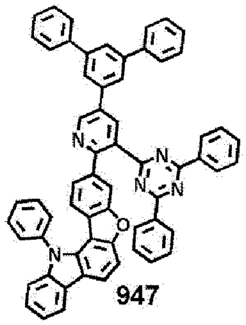
947

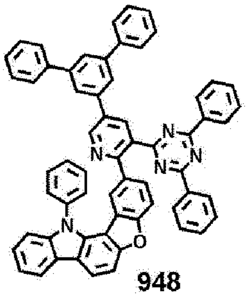
948

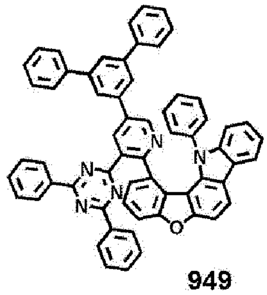
949

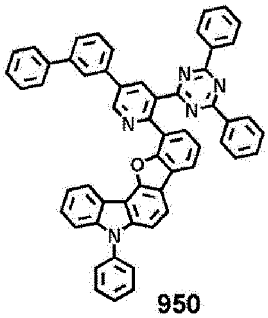
950

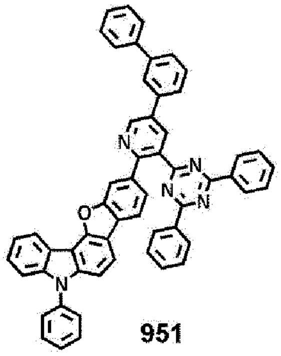
951

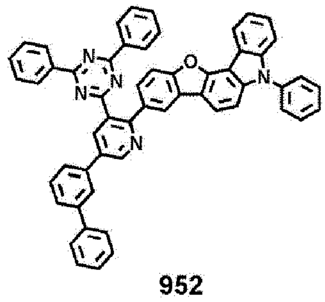
952

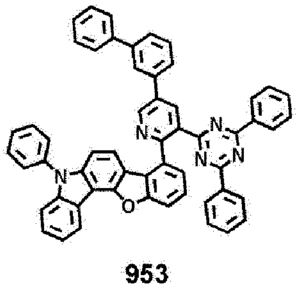
953

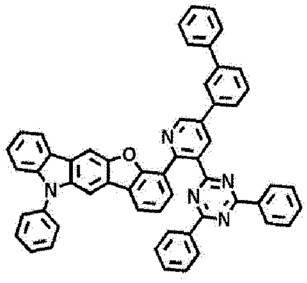
954

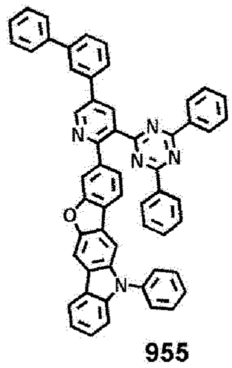
955

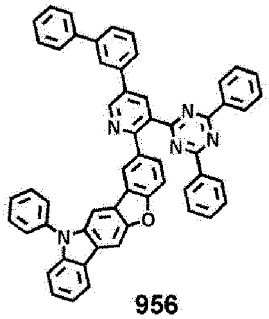
956

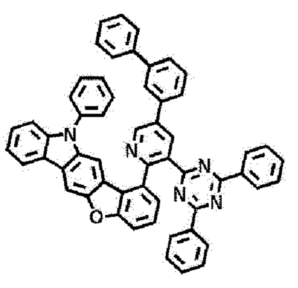
957

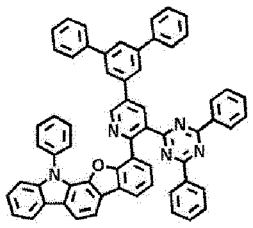
958

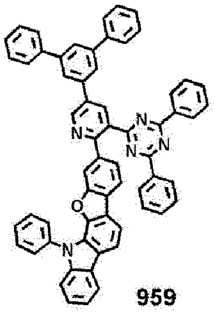
959

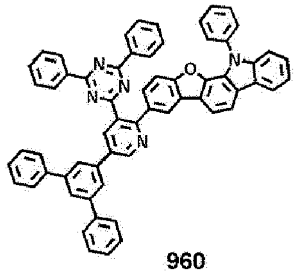
960

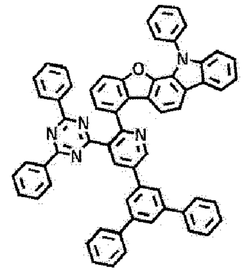
961

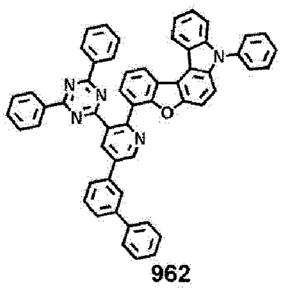
962

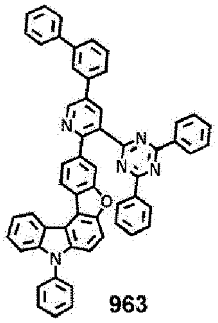
963

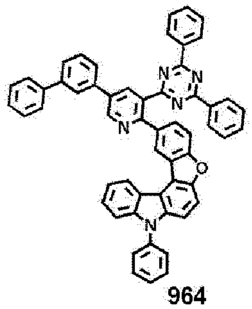
964

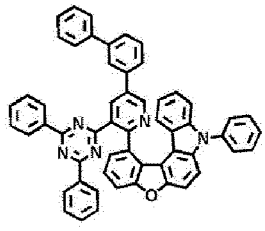
965

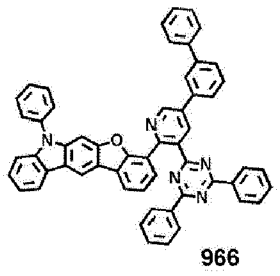
966

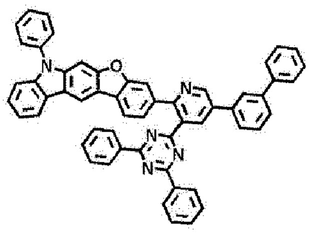
967

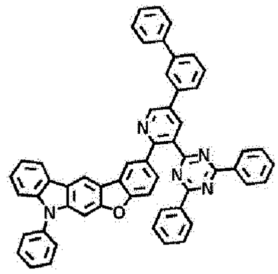
968

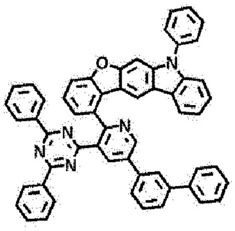
969

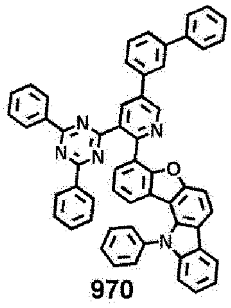
970

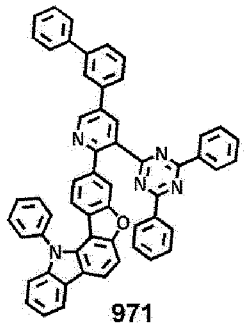
971

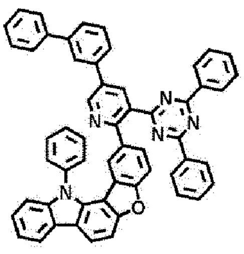
972

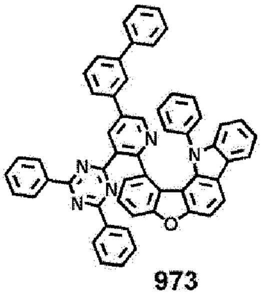
973

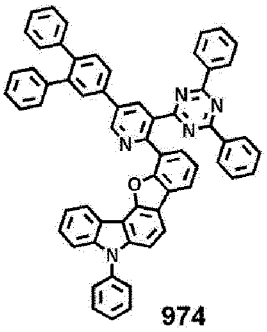
974

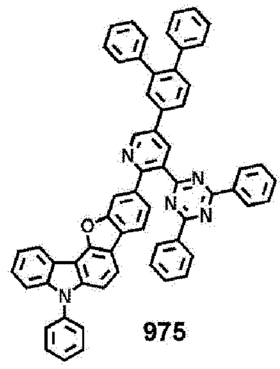
975

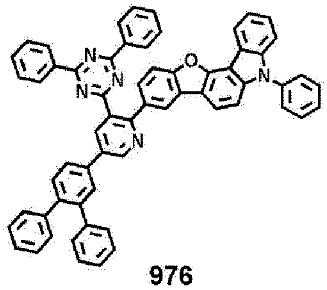
976

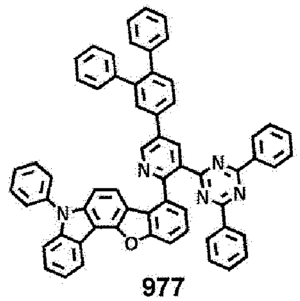
977

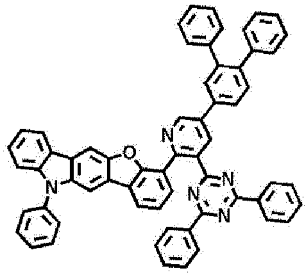
978

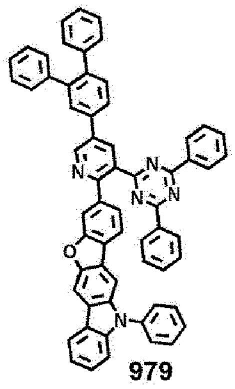
979

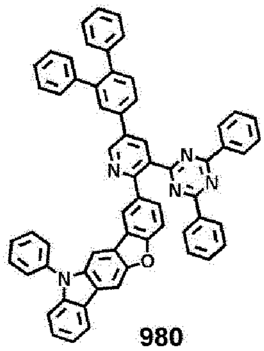
980

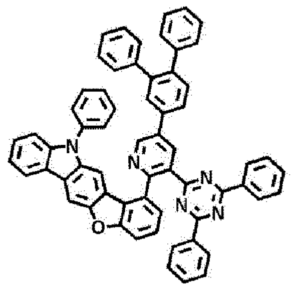
981

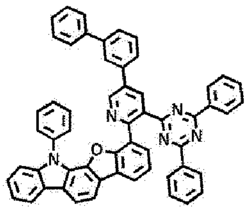
982

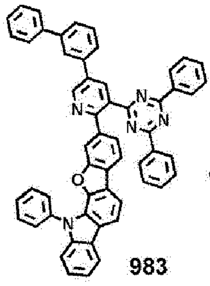
983

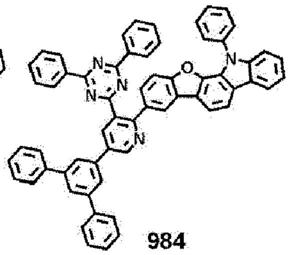
984

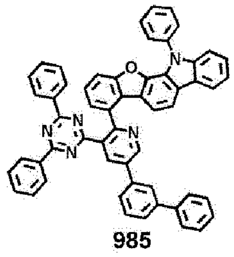
985

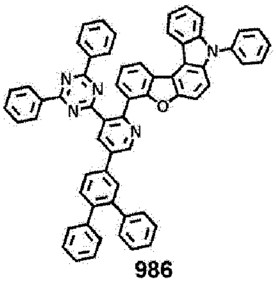
986

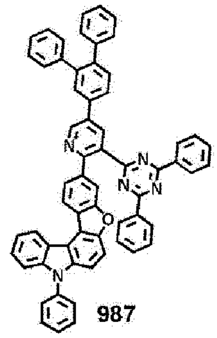
987

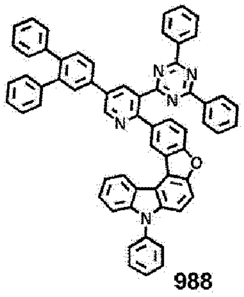
988

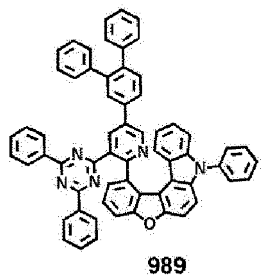
989

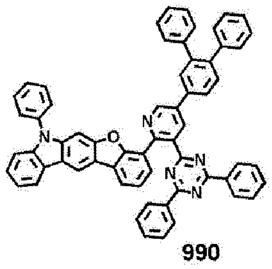
990

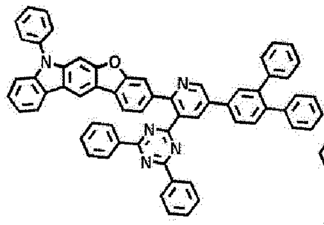
991

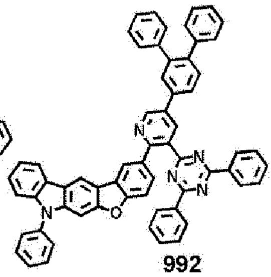
992

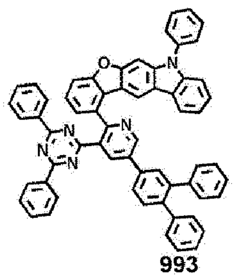
993

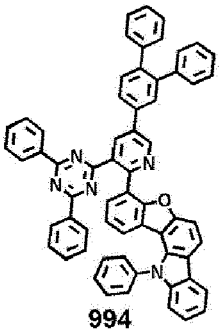
994

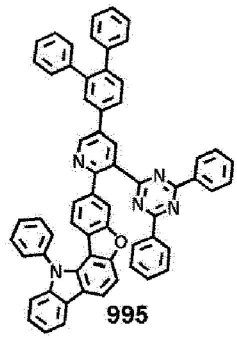
995

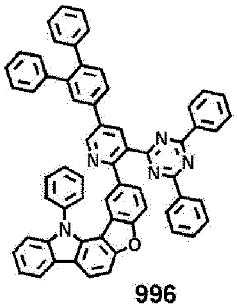
996

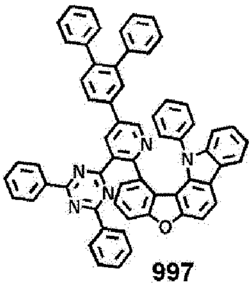
997

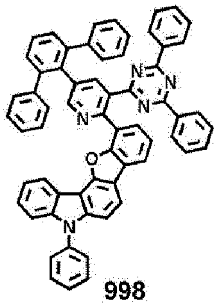
998

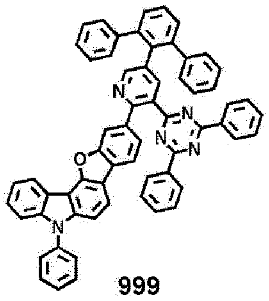
999

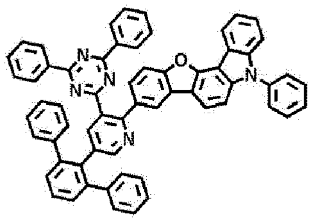
1000

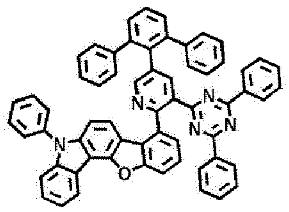
1001

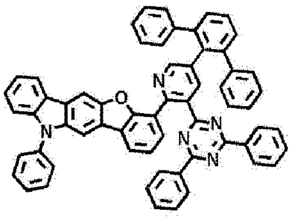
1002

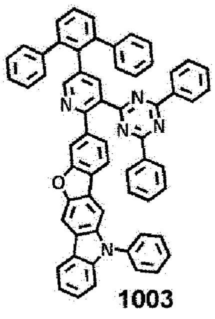
1003

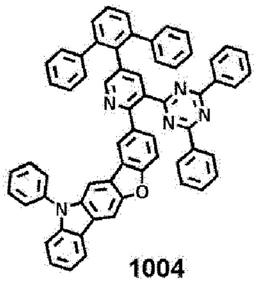
1004

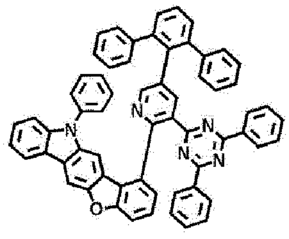
1005

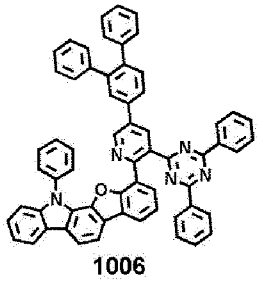
1006

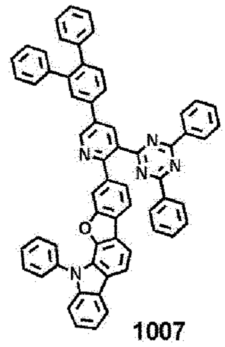
1007

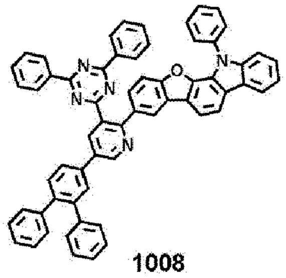
1008

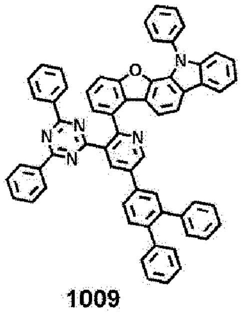
1009

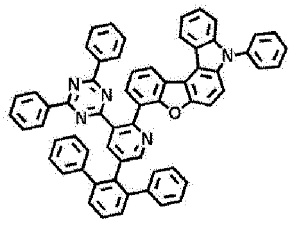
1010

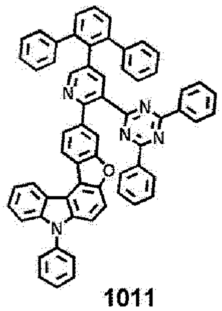
1011

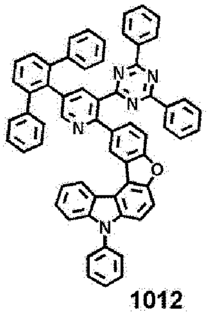
1012

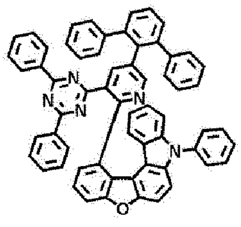
1013

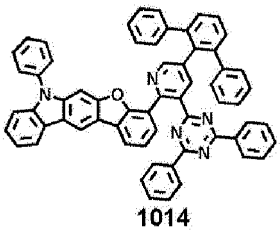
1014

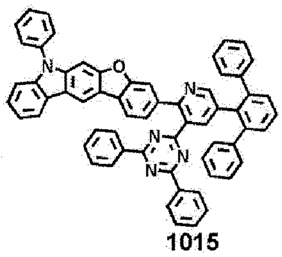
1015

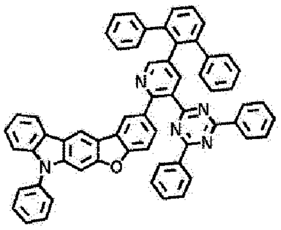
1016

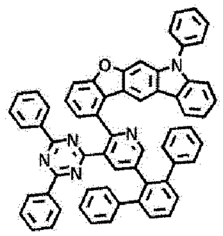
1017

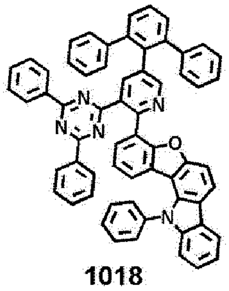

1018

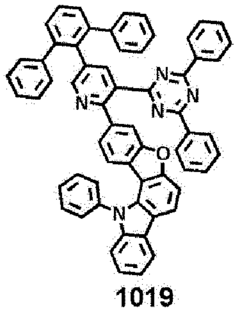

1019

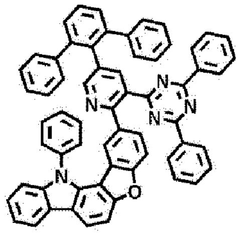

1020

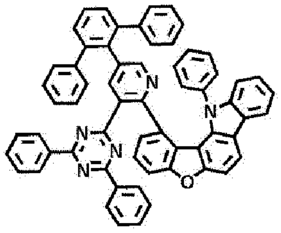

1021

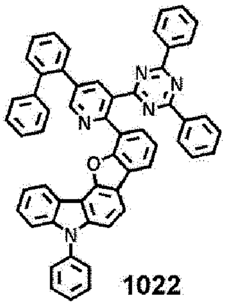

1022

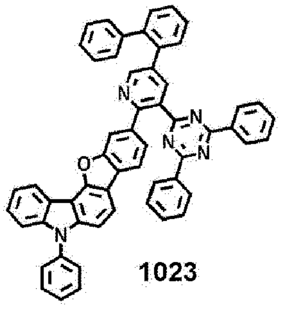

1023

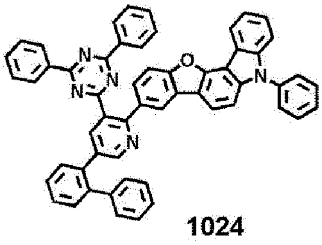

1024

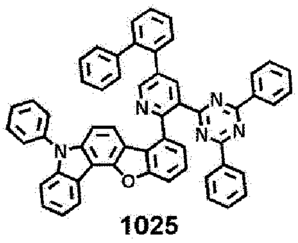

1025

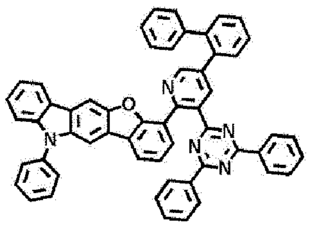

1026

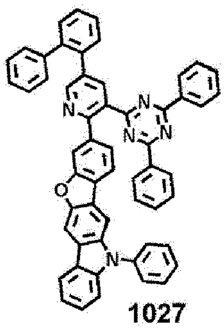

1027

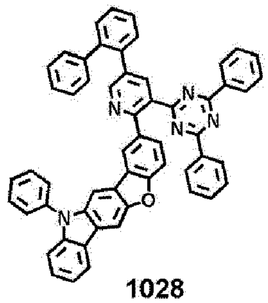

1028

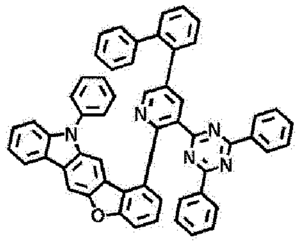

1029

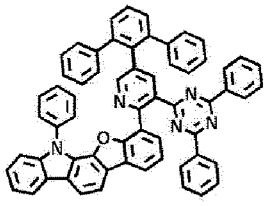
1030

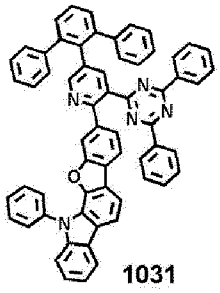
1031

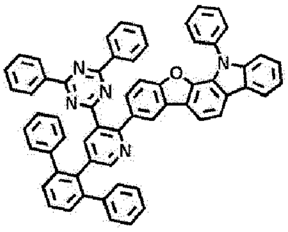
1032

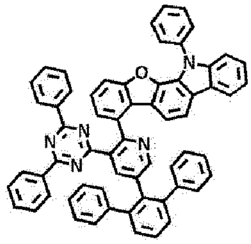
1033

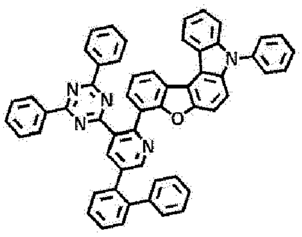
1034

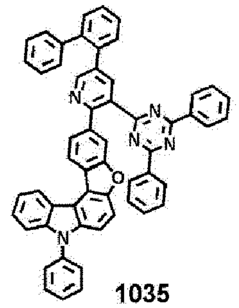
1035

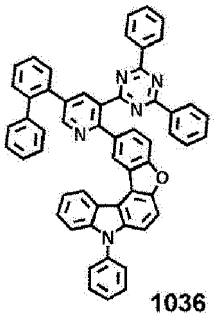
1036

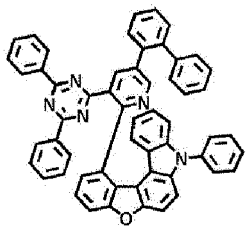
1037

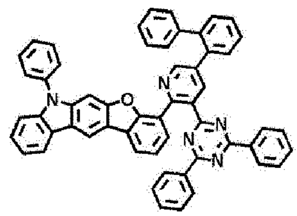
1038

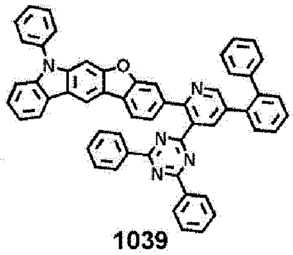
1039

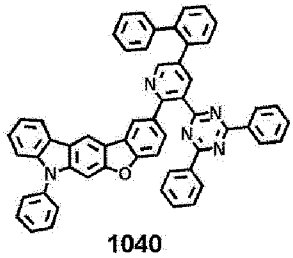
1040

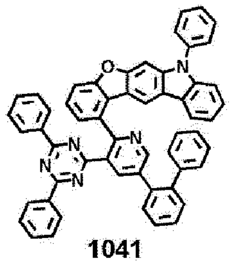
1041

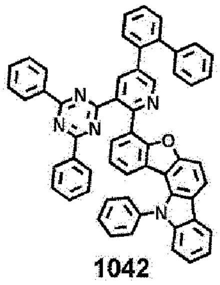

1042

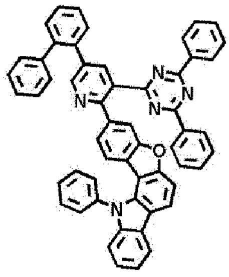

1043

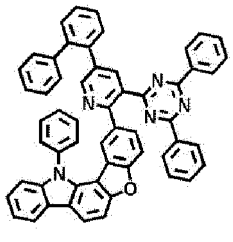

1044

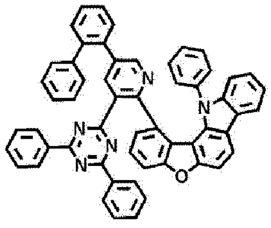

1045

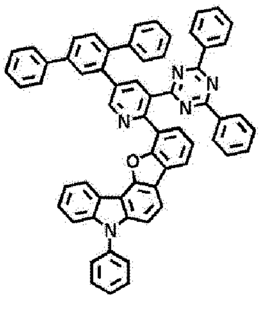

1046

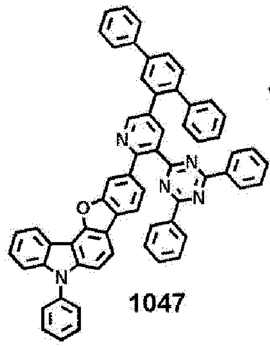

1047

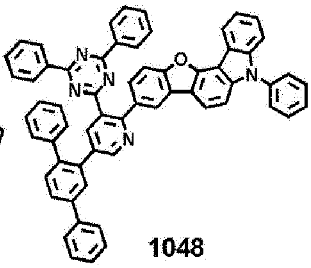

1048

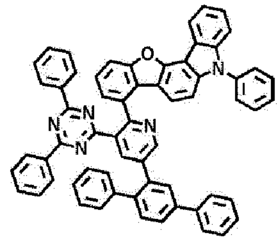

1049

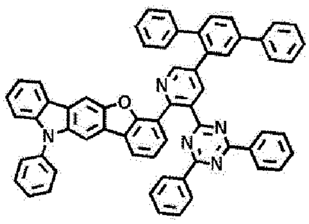

1050

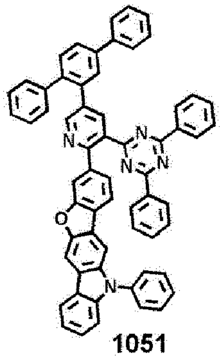

1051

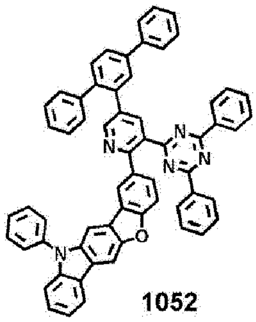

1052

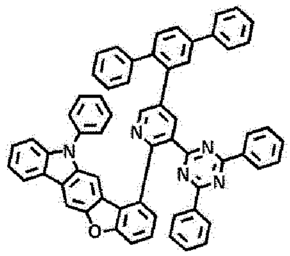

1053

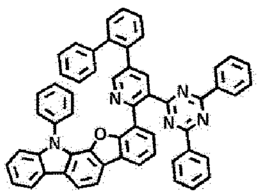

1054

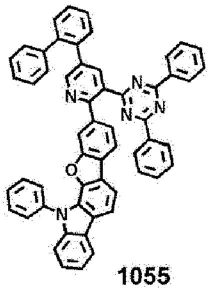

1055

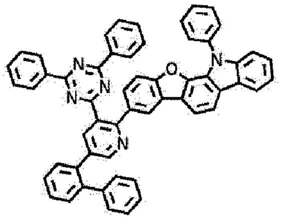

1056

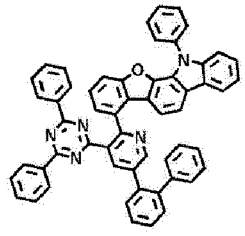

1057

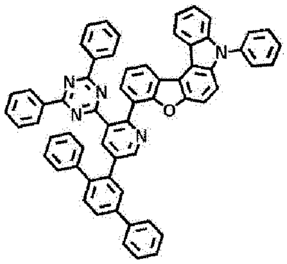

1058

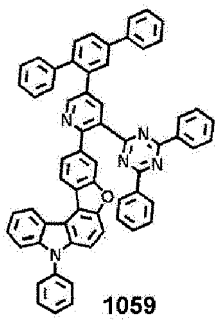

1059

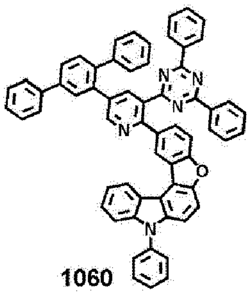

1060

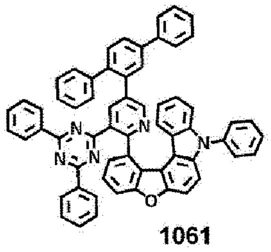

1061

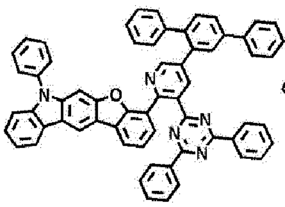

1062

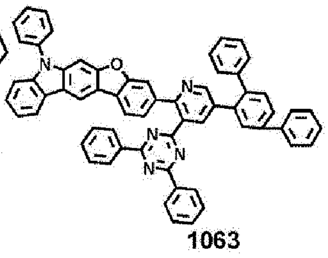

1063

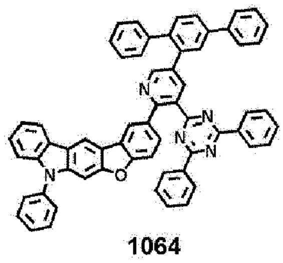

1064

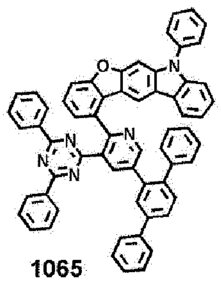

1065

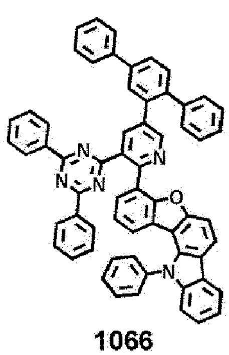

**1066**

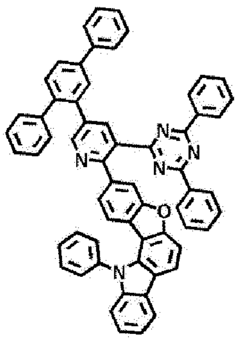

**1067**

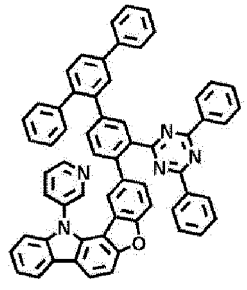

**1068**

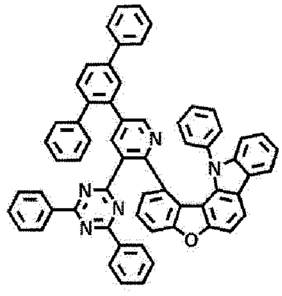

**1069**

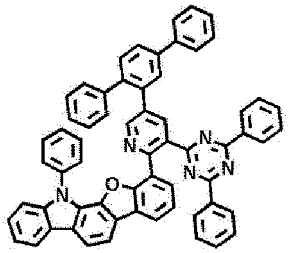

**1070**

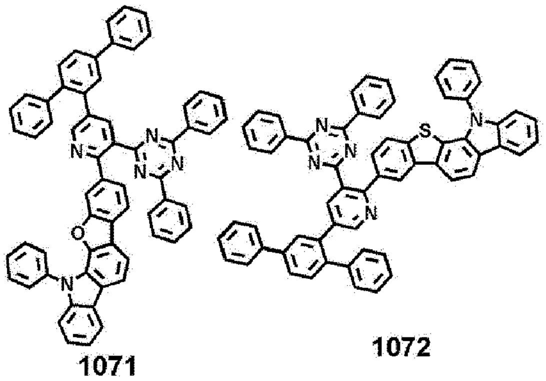

**1071**

**1072**

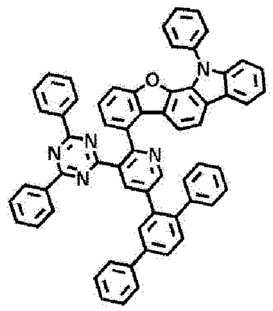

**1073**

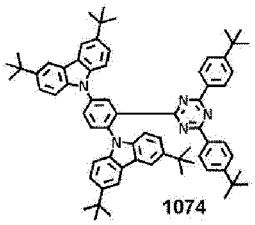

1074

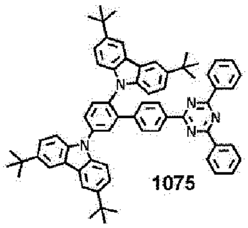

1075

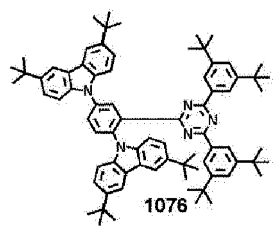

1076

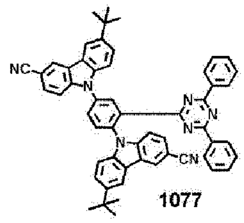

1077

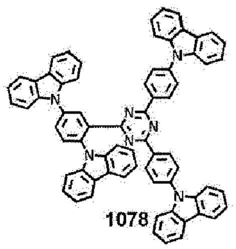

1078

Group XI

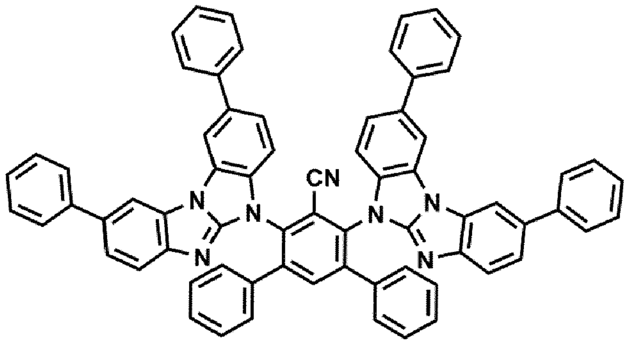

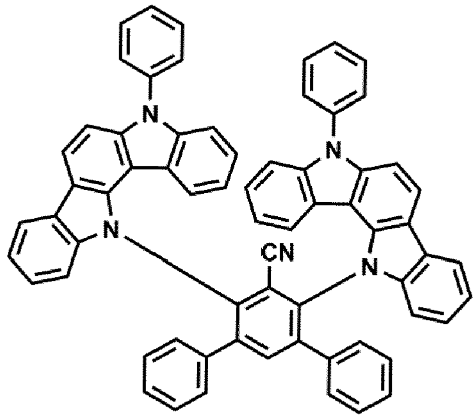

Group XII

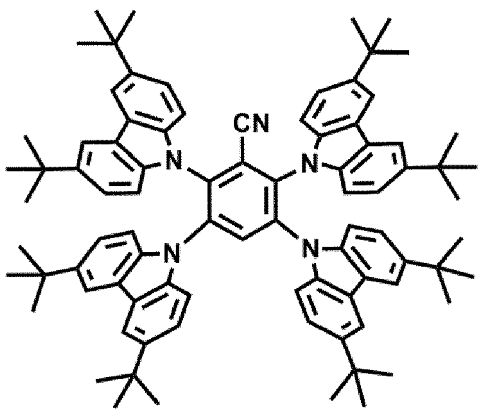

Group XIII

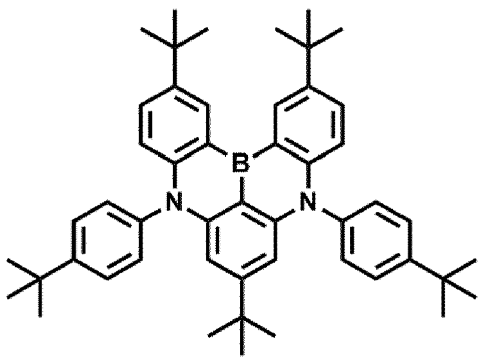

Hole transport region 12

The hole transport region 12 may be located between the first electrode 11 and the emission layer 15 of the organic light-emitting device 10.

The hole transport region 12 may have a single-layered structure or a multi-layered structure.

For example, the hole transport region 12 may have a hole injection layer, a hole transport layer, a hole injection layer/hole transport layer structure, a hole injection layer/first hole transport layer/second hole transport layer structure, a hole transport layer/middle layer structure, a hole injection layer/hole transport layer/middle layer structure, a hole transport layer/electron-blocking layer structure, or a hole injection layer/hole transport layer/electron-blocking layer structure, but embodiments of the present disclosure are not limited thereto.

The hole transport region 12 may include any compound having hole-transporting properties.

For example, the hole transport region 12 may include an amine-containing compound.

In one or more embodiments, the hole transport region 1 may include at least one of a compound represented by Formula 201 to a compound represented by Formula 205, but embodiments of the present disclosure are not limited thereto:

Formula 201

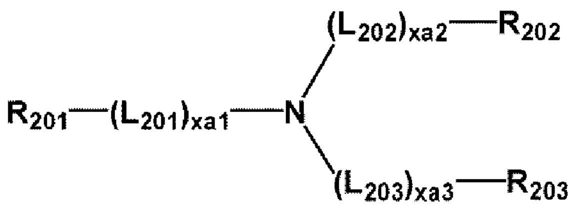

Formula 202

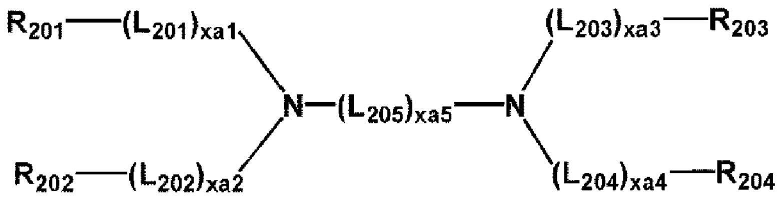

Formula 203

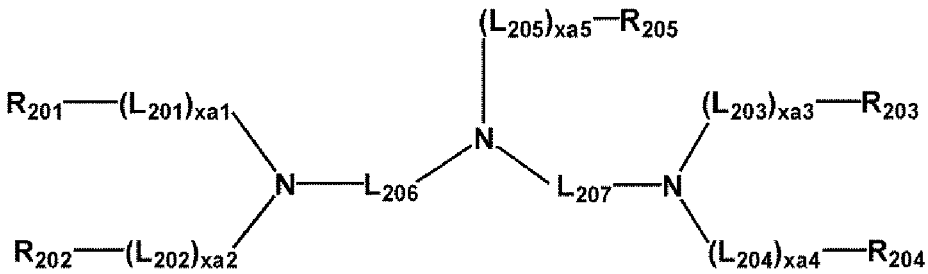

Formula 204

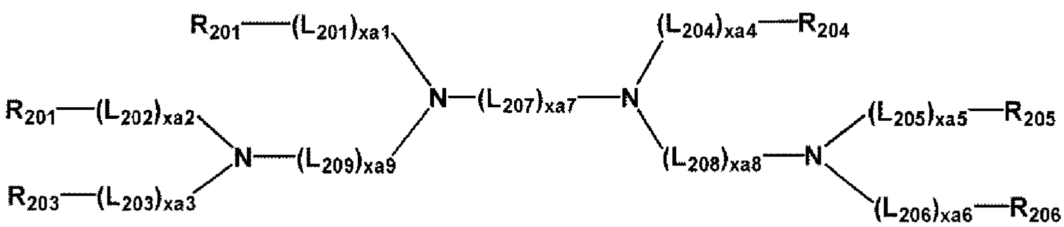

Formula 205

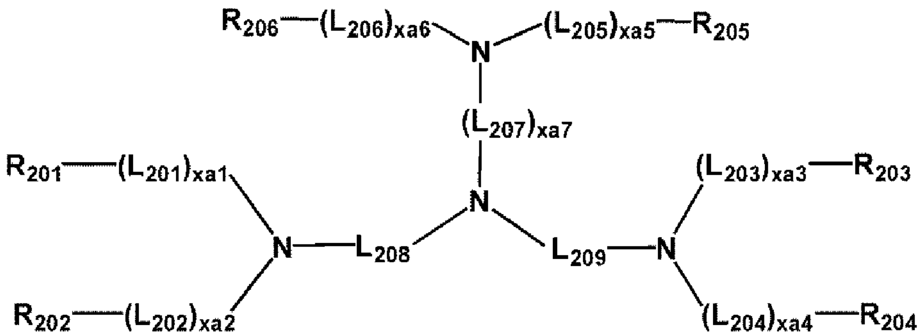

wherein, in Formulae 201 to 205,

$L_{201}$ to $L_{209}$ may each independently be O, S, a substituted or unsubstituted $C_5$-$C_{60}$ carbocyclic group, or a substituted or unsubstituted $C_1$-$C_{60}$ heterocyclic group,
xa1 to xa9 may each independently be an integer from 0 to 5, and
$R_{201}$ to $R_{206}$ may each independently be a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, or a substituted or unsubstituted monovalent non-aromatic condensed heteropolycyclic group, wherein neighboring two groups of $R_{201}$ to $R_{206}$ may optionally be linked to each other via a single bond, a dimethyl-methylene group, or a diphenyl-methylene group.

For example, in one or more embodiments,

$L_{201}$ to $L_{209}$ may be a phenyl group, a heptalene group, an indene group, a naphthalene group, an azulene group, a heptalene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, an isoindole group, an indole group, a furan group, a thiophene group, a benzofuran group, a benzothiophene group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, and a triindolobenzene group, each unsubstituted or substituted with deuterium, a $C_1$-$C_{10}$ alkyl group, a $C_1$-$C_{10}$ alkoxy group, a phenyl group, a naphthyl group, a fluorenyl group, a carbazolyl group, a dibenzofuranyl group, a dibenzothiophenyl group, a triphenylenyl group, a biphenyl group, a terphenyl group, a quaterphenyl group, or -$Si(Q_{11})(Q_{12})(Q_{13})$,
xa1 to xa9 may each independently be 0, 1, or 2, and
$R_{201}$ to $R_{206}$ may each independently be a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an indeno carbazolyl group, an indolocarbazolyl group, a benzofurocarbazolyl group, or a benzothienocarbazolyl group, each unsubstituted or substituted with at least one of deuterium, -F, -Cl, -Br, -I, a hydroxyl group, a cyano group, a nitro group, an amidino group, a hydrazine group, a hydrazone group, a $C_1$-$C_{20}$ alkyl group, a $C_1$-$C_{20}$ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted

with a $C_1$-$C_{10}$ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, - $Si(Q_{31})(Q_{32})(Q_{33})$, or $-N(Q_{31})(Q_{32})$,

wherein $Q_{11}$ to $Q_{13}$ and $Q_{31}$ to $Q_{33}$ may each independently be a $C_1$-$C_{10}$ alkyl group, a $C_1$-$C_{10}$ alkoxy group, a phenyl group, a biphenyl group, a terphenyl group, or a naphthyl group.

In one or more embodiments, the hole transport region 12 may include a carbazole-containing amine compound.

In one or more embodiments, the hole transport region 12 may include a carbazole-containing amine compound and a carbazole-free amine compound.

The carbazole-containing amine compound may be s, for example, a compound represented by Formula 201 including a carbazole group and further including at least one of a dibenzofuran group, a dibenzothiophene group, a fluorene group, a spiro-bifluorene group, an indenocarbazole group, an indolocarbazole group, a benzofurocarbazole group, or a benzothienocarbazole group.

The carbazole-free amine compound may be , for example, a compound represented by Formula 201 which does not include a carbazole group and which includes at least one of a dibenzofuran group, a dibenzothiophene group, a fluorene group, a spiro-bifluorene group, an indenocarbazole group, an indolocarbazole group, a benzofurocarbazole group, or a benzothienocarbazole group.

In one or more embodiments, the hole transport region 12 may include at least one compound represented by Formulae 201 or 202.

In one or more embodiments, the hole transport region 12 may include at least one compound represented by Formulae 201-1, 202-1, or 201-2, but embodiments of the present disclosure are not limited thereto:

Formula 201-1

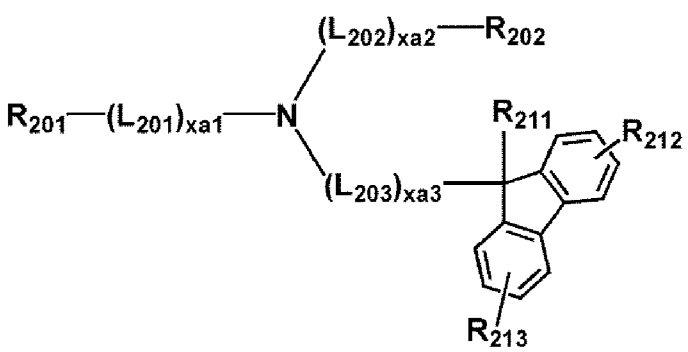

Formula 202-1

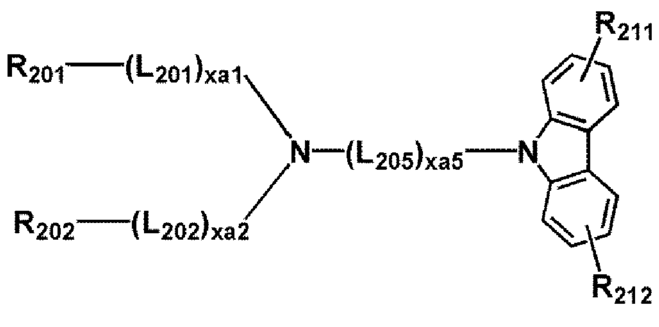

Formula 201-2

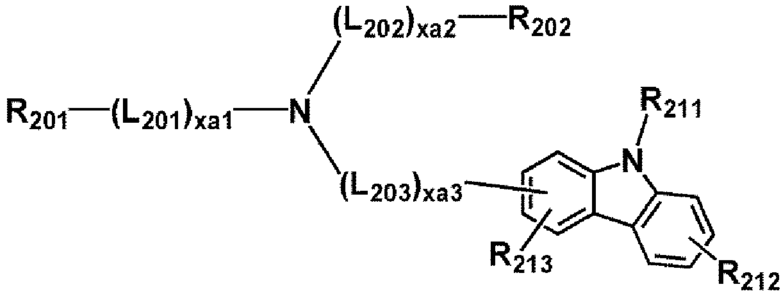

In Formulae 201-1, 202-1, and 201-2, $L_{201}$ to $L_{203}$, $L_{205}$, xa1 to xa3, xa5, $R_{201}$ and $R_{202}$ are the same as described herein, and $R_{211}$ to $R_{213}$ are each independently hydrogen, deuterium, -F, -Cl, -Br, -I, a hydroxyl group, a cyano group, a nitro group, an amidino group, a hydrazine group, a hydrazone group, a $C_1$-$C_{20}$ alkyl group, a $C_1$-$C_{20}$ alkoxy group, a phenyl group, a biphenyl group, a terphenyl group, a phenyl group substituted with a $C_1$-$C_{10}$ alkyl group, a phenyl group substituted with -F, a naphthyl group, a fluorenyl group, a spiro-bifluorenyl group, a dimethylfluorenyl group, a diphenyla fluorenyl group, a triphenylenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, or a pyridinyl group.

For example, the hole transport region 12 may include at least one of Compounds HT1 to HT39, but embodiments of the present disclosure are not limited thereto.

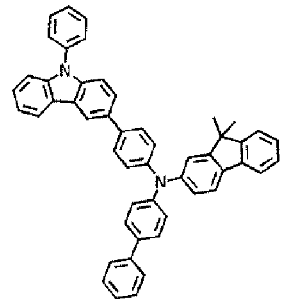

HT1

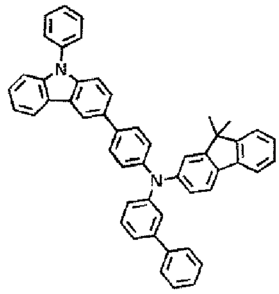

HT2

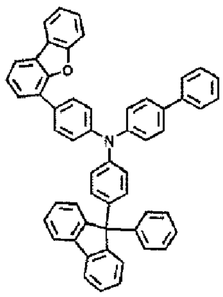

HT3

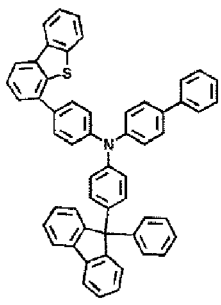

HT4

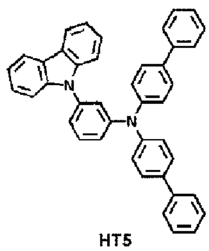

HT5

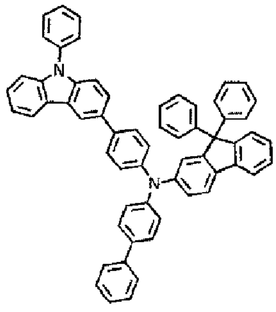

HT6

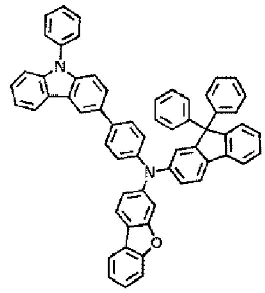

HT7

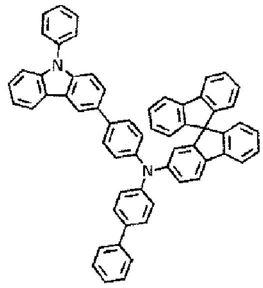

HT8

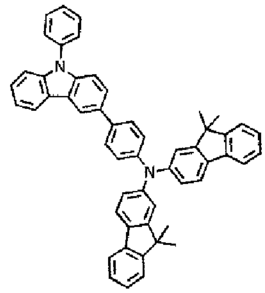

HT9

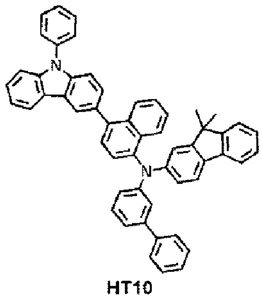

HT10

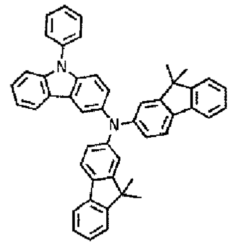

HT11

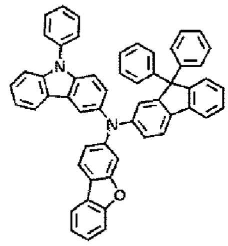

HT12

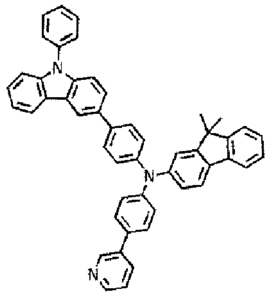

HT13

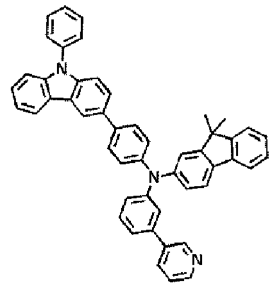

HT14

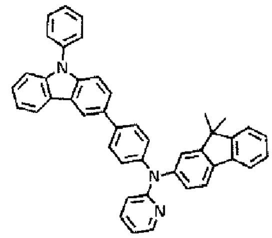

HT15

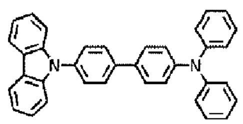

HT16

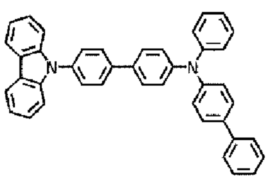

HT17

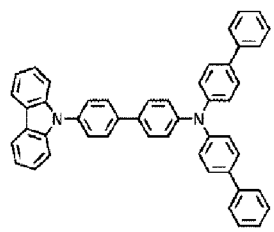

HT18

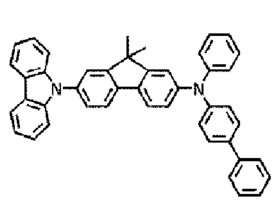

HT19

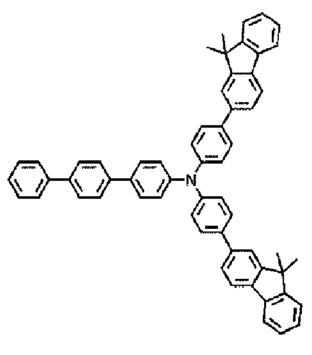

HT20

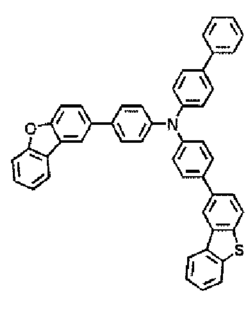

HT21

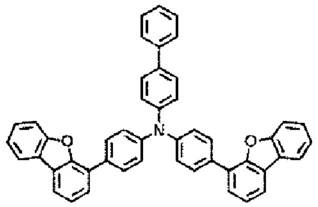

HT22

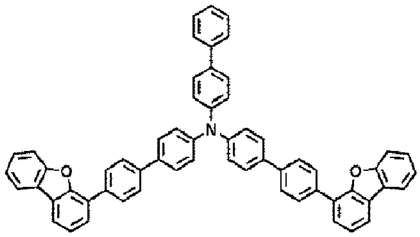

HT23

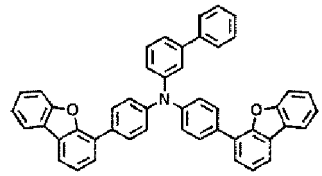

HT24

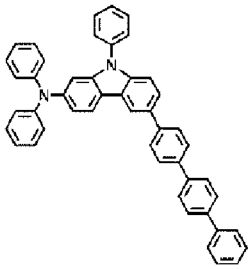

HT25

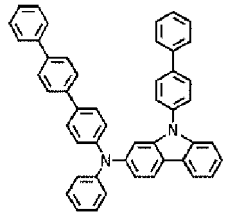

HT26

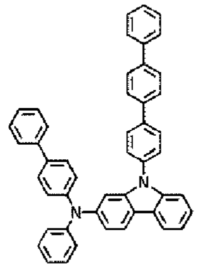

HT27

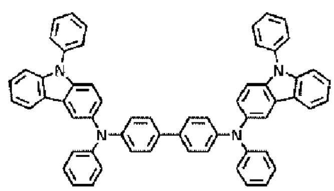

HT28

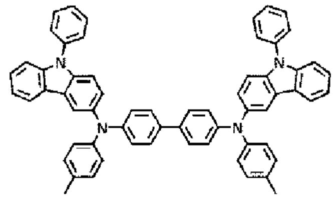

HT29

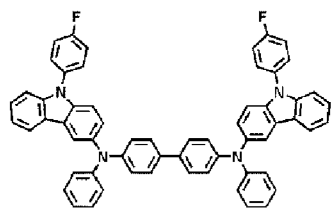

HT30

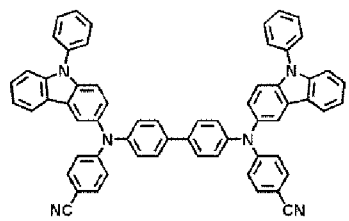

HT31

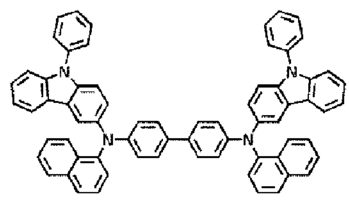

HT32

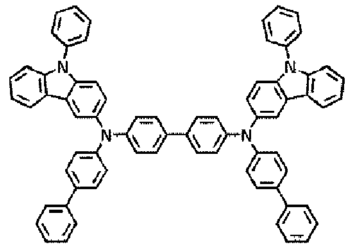

HT33

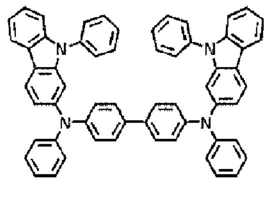

HT34

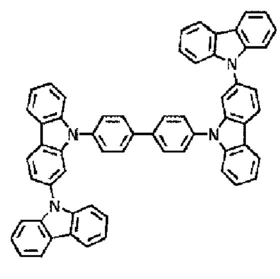

HT35

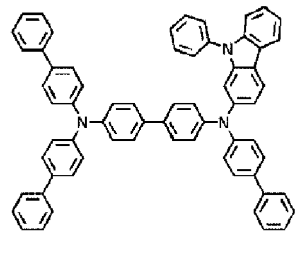

HT36

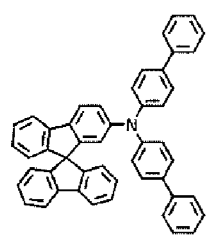

HT37

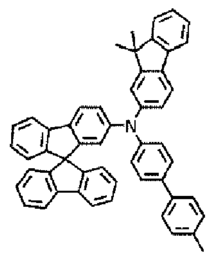

HT38

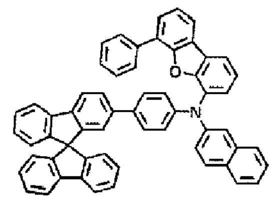

HT39

In one or more embodiments, hole transport region 12 of the organic light-emitting device 10 may further include a p-dopant. When the hole transport region 12 further includes a p-dopant, the hole transport region 12 may have a matrix (for example, at least one of compounds represented by Formulae 201 to 205) and a p-dopant included in the matrix. The p-dopant may be uniformly or non-uniformly doped in the hole transport region 12.

In one or more embodiments, the LUMO energy level of the p-dopant may be -3.5 electron volts (eV) or less.

The p-dopant may include at least one of a quinone derivative, a metal oxide, or a cyano group-containing compound, but embodiments of the present disclosure are not limited thereto.

In one or more embodiments, the p-dopant may include at least one of:

a quinone derivative, such as tetracyanoquinodimethane (TCNQ), 2,3,5,6-tetrafluoro-7,7,8,8-tetracyanoquinodimethane (F4-TCNQ), or F6-TCNNQ;
a metal oxide, such as tungsten oxide or molybdenum oxide;
1,4,5,8,9,12-hexaazatriphenylene-hexacarbonitrile (HAT-CN); or
a compound represented by Formula 221 below, but embodiments of the present disclosure are not limited thereto:

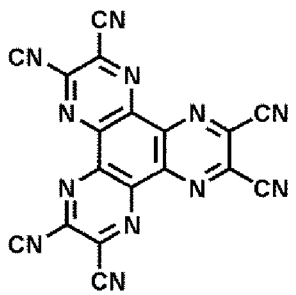

HAT-CN

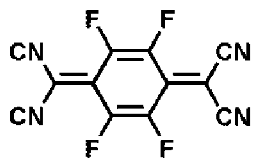

F4-TCNQ

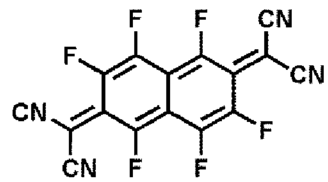

**F6-TCNNQ**

Formula 221

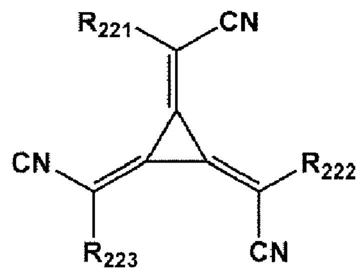

In Formula 221,

$R_{221}$ to $R_{223}$ may each independently be a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_1$-$C_{60}$ heteroaryl group, a substituted or unsubstituted monovalent non-aromatic condensed polycyclic group, or a substituted or unsubstituted monovalent non-aromatic condensed heteropolycyclic group, and at least one of $R_{221}$ to $R_{223}$ may have at least one substituent that is a cyano group, -F, -Cl, -Br, -I, a $C_1$-$C_{20}$ alkyl group substituted with -F, a $C_1$-$C_{20}$ alkyl group substituted with -Cl, a $C_1$-$C_{20}$ alkyl group substituted with -Br, or a $C_1$-$C_{20}$ alkyl group substituted

with -I.

The hole transport region 12 may have a thickness of about 100 Å to about 10000 Å, for example, about 400 Å to about 2000 Å, and the emission layer 15 may have a thickness of about 100 Å to about 3000 Å, for example, about 300 Å to about 1000 Å. When the thickness of each of the hole transport region 12 and the emission layer 15 is within these ranges described above, satisfactory hole transportation characteristics and/or luminescent characteristics may be obtained without a substantial increase in driving voltage.

Electron transport region 17

The electron transport region 17 is placed between the emission layer 15 and the second electrode 19 of the organic light-emitting device 10.

The electron transport region 17 may have a single-layered structure or a multi-layered structure.

For example, the electron transport region 17 may have an electron transport layer, an electron transport layer/electron injection layer structure, a buffer layer/electron transport layer structure, hole-blocking layer/electron transport layer structure, a buffer layer/electron transport layer/electron injection layer structure, or a hole-blocking layer/electron transport layer/electron injection layer structure, but embodiments of the present disclosure are not limited thereto. The electron transport region 17 may further include an electron control layer.

The electron transport region 17 may include known electron-transporting materials.

The electron transport region (for example, a buffer layer, a hole-blocking layer, an electron control layer, or an electron transport layer in the electron transport region) may include a metal-free compound containing at least one $\pi$ electron-deficient nitrogen-containing cyclic group. The $\pi$ electron-deficient nitrogen-containing cyclic group is the same as described above.

In one or more embodiments, the electron transport region may include a compound represented by Formula 601 below:

Formula 601 $$[Ar_{601}]_{xe11}\text{-}[(L_{601})_{xe1}\text{-}R_{601}]_{xe21}$$

wherein, in Formula 601,

$Ar_{601}$ and $L_{601}$ may each independently be a substituted or unsubstituted $C_5$-$C_{60}$ carbocyclic group or a substituted or unsubstituted $C_1$-$C_{60}$ heterocyclic group,
xe11 may be 1, 2, or 3,
xe1 is an integer from 0 to 5,
$R_{601}$ may be a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -Si($Q_{601}$)($Q_{602}$)($Q_{603}$), -C(=O)($Q_{601}$), -S(=O)$_2$($Q_{601}$), - P(Q601)(Q602), or -P(=O)($Q_{601}$)($Q_{602}$),
Q601 to $Q_{603}$ may each independently be a $C_1$-$C_{10}$ alkyl group, a $C_1$-$C_{10}$ alkoxy group, a phenyl group, a biphenyl group, a terphenyl group, or a naphthyl group, and
xe21 is an integer from 1 to 5.

In one or more embodiments, at least one of $Ar_{601}$(s) in the number of xe11 and $R_{601}$(s) in the number of xe21 may include the $\pi$ electron-deficient nitrogen-containing cyclic group.

In one or more embodiments, ring $Ar_{601}$ and $L_{601}$ in Formula 601 may each independently be a phenyl group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, or an

azacarbazole group, each unsubstituted or substituted with at least one of deuterium, -F, -Cl, -Br, -I, a hydroxyl group, a cyano group, a nitro group, an amidino group, a hydrazine group, a hydrazone group, a $C_1$-$C_{20}$ alkyl group, a $C_1$-$C_{20}$ alkoxy group, a phenyl group, a biphenyl group, a terphenyl group, a naphthyl group, -Si($Q_{31}$)($Q_{32}$)($Q_{33}$), -S(=O)$_2$($Q_{31}$), or -P(=O)($Q_{31}$)($Q_{32}$),

wherein $Q_{31}$ to $Q_{33}$ may each independently be a $C_1$-$C_{10}$ alkyl group, a $C_1$-$C_{10}$ alkoxy group, a phenyl group, a biphenyl group, a terphenyl group, or a naphthyl group.

When xe11 in Formula 601 is 2 or more, two or more of $Ar_{601}$(S) may be linked to each other via a single bond.

In one or more embodiments, $Ar_{601}$ in Formula 601 may be an anthracene group.

In one or more embodiments, the compound represented by Formula 601 may be represented by Formula 601-1:

Formula 601-1

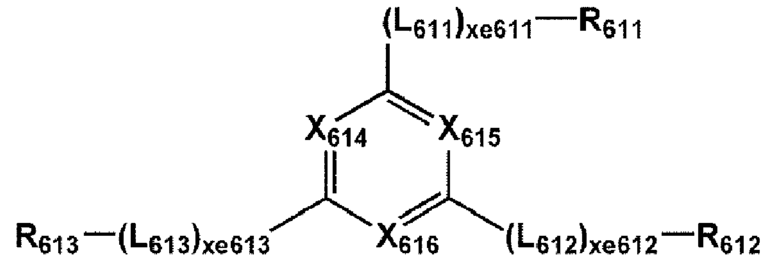

In Formula 601-1,

$X_{614}$ may be N or C($R_{614}$), $X_{615}$ may be N or C($R_{615}$), $X_{616}$ may be N or C($R_{616}$), at least one of $X_{614}$ to $X_{616}$ may be N,
$L_{611}$ to $L_{613}$ may each independently be the same as described in connection with $L_{601}$,
xe611 to xe613 may each independently be the same as described in connection with xe1,
$R_{611}$ to $R_{613}$ may each independently be the same as described in connection with $R_{601}$, and
$R_{614}$ to $R_{616}$ may each independently be hydrogen, deuterium, -F, -Cl, -Br, -I, a hydroxyl group, a cyano group, a nitro group, an amidino group, a hydrazino group, a hydrazono group, a $C_1$-$C_{20}$ alkyl group, a $C_1$-$C_{20}$ alkoxy group, a phenyl group, a biphenyl group, a terphenyl group, or a naphthyl group.

In one or more embodiments, xe1 and xe611 to xe613 in Formulae 601 and 601-1 may each independently be 0, 1, or 2.

In one or more embodiments, $R_{601}$ and $R_{611}$ to $R_{613}$ in Formulae 601 and 601-1 may each independently be a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, or an azacarbazolyl group, each unsubstituted or substituted with at least one of deuterium, -F, -Cl, -Br, -I, a hydroxyl group, a cyano group, a nitro group, an amidino group, a hydrazine group, a hydrazone group, a $C_1$-$C_{20}$ alkyl group, a $C_1$-$C_{20}$ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group,

an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, or an azacarbazolyl group; or

$-S(=O)_2(Q_{601})$ or $-P(=O)(Q_{601})(Q_{602})$,
wherein $Q_{601}$ and $Q_{602}$ are the same as described above.

The electron transport region may include at least one of Compounds ET1 to ET36, but embodiments of the present disclosure are not limited thereto:

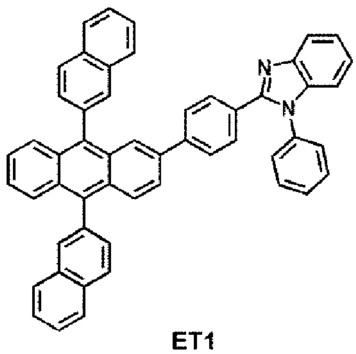

ET1

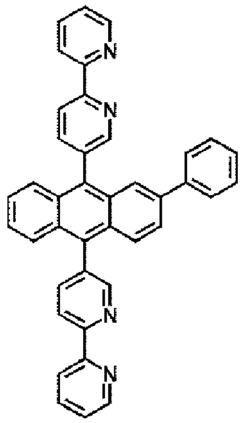

ET2

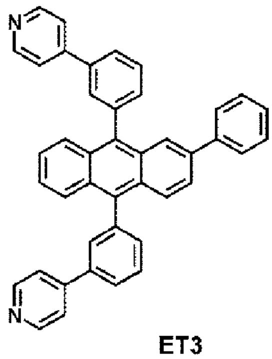

ET3

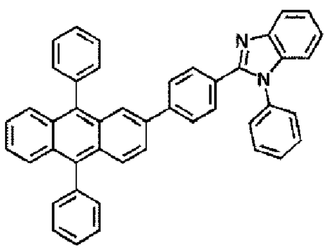

ET4

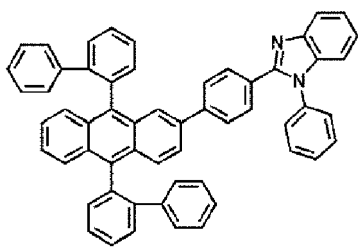

ET5

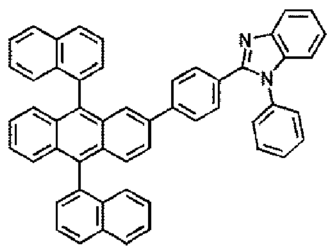

ET6

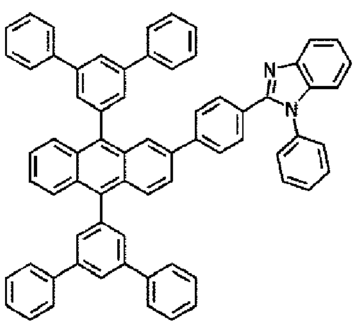

ET7

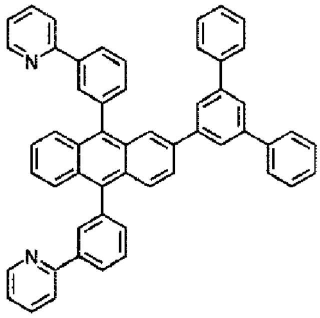

ET8

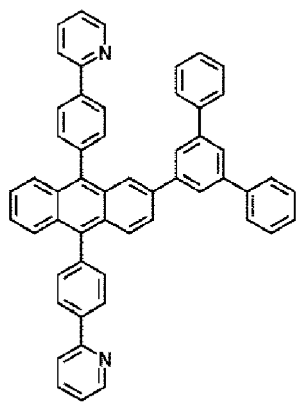

ET9

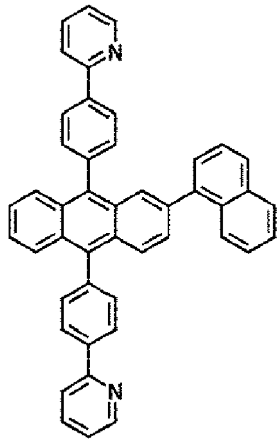

ET10

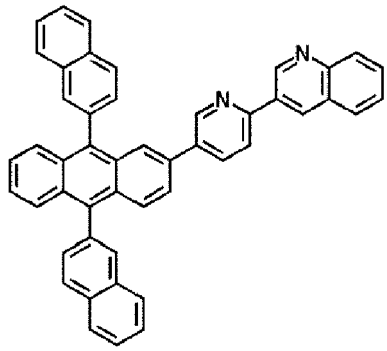

ET11

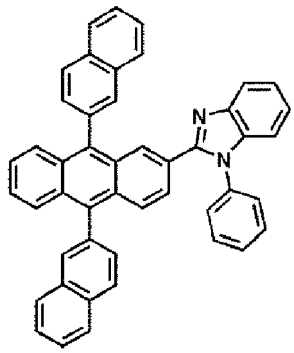

ET12

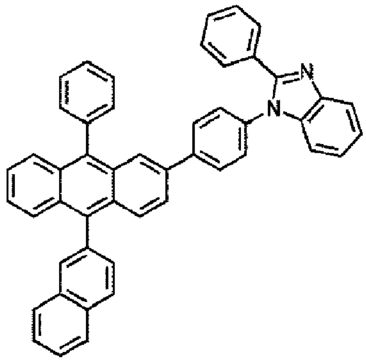

ET13

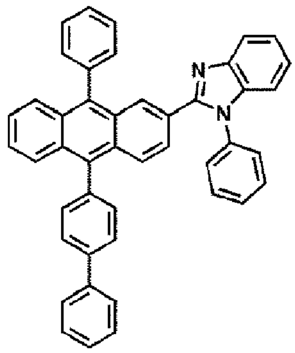

ET14

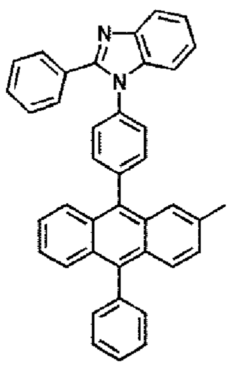

ET15

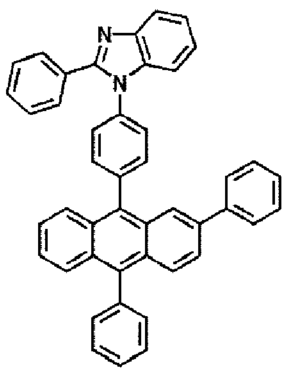

ET16

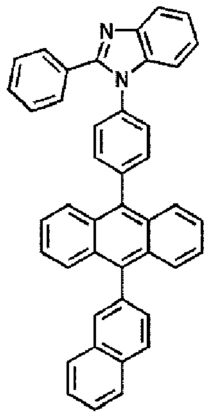

ET17

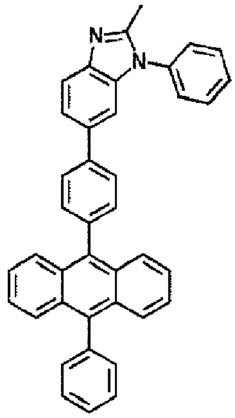

ET18

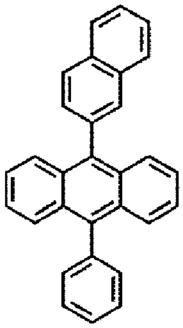

**ET19**

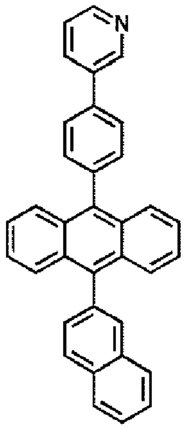

**ET20**

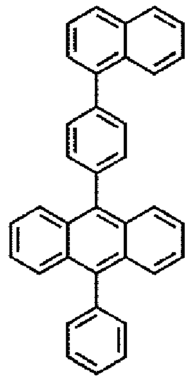

**ET21**

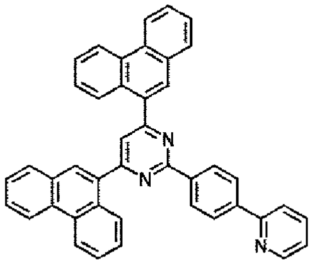

ET22

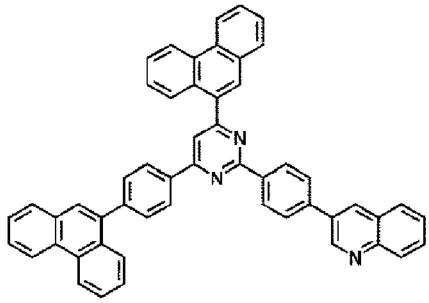

ET23

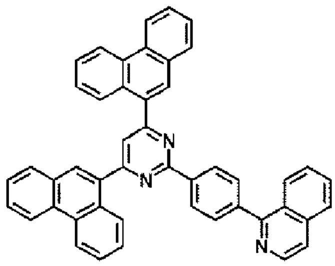

ET24

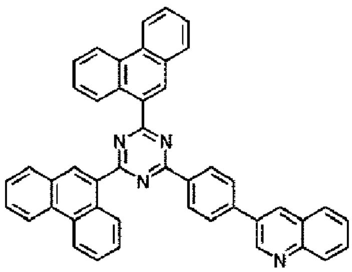

ET25

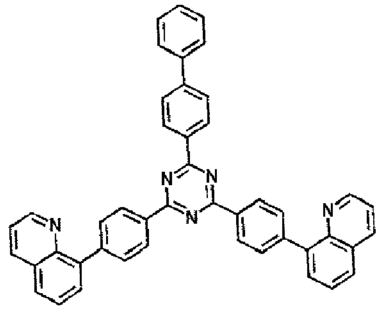

ET26

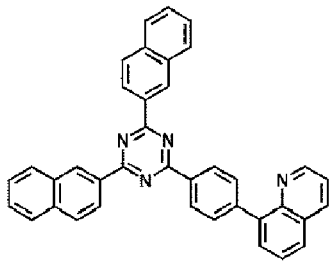

ET27

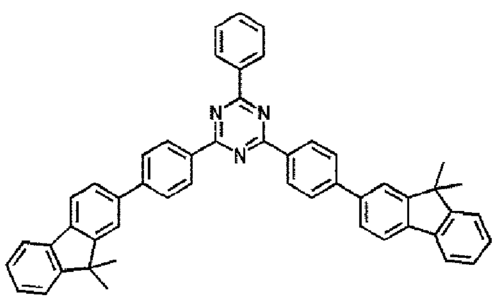

ET28

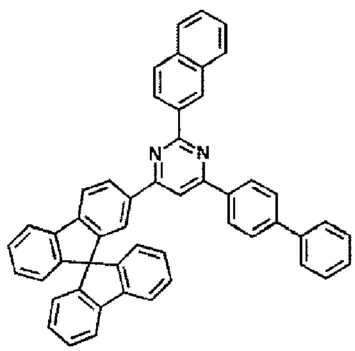

ET29

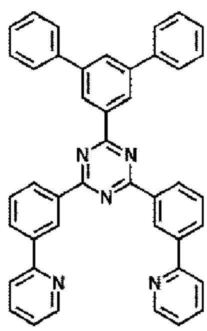

ET30

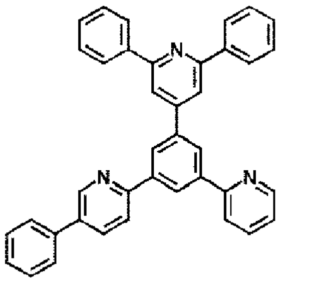

ET31

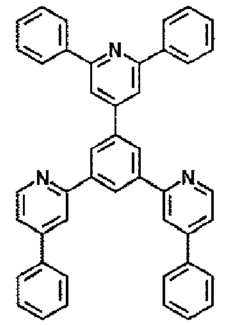

ET32

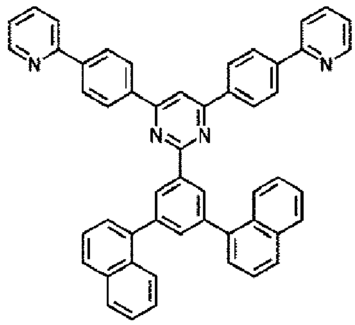

ET33

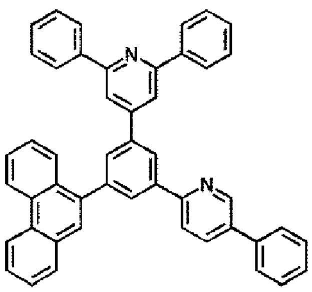

ET34

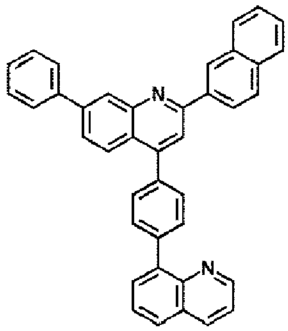

ET35

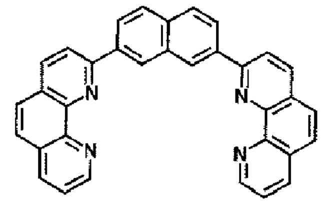

ET36

In one or more embodiments, the electron transport region may include at least one of 2,9-dimethyl-4,7-diphenyl-1,10-phenanthroline (BCP), 4,7-dphenyl-1,10-phenanthroline (Bphen), tris(8-hydroxyquinolinato)aluminum ($Alq_3$), bis(2-methyl-8-quinolinolato-N1,08)-(1,1'-biphenyl-4-olato)aluminum (BAlq), 3-(biphenyl-4-yl)-5-(4-tert-butylphenyl)-4-phenyl-4H-1,2,4-triazole (TAZ), or 4-(naphthalen-1-yl)-3,5-diphenyl-4H-1,2,4-triazole (NTAZ):

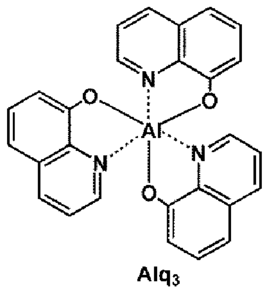

$Alq_3$

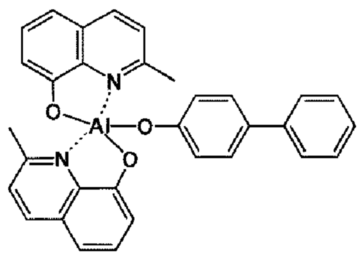

BAlq

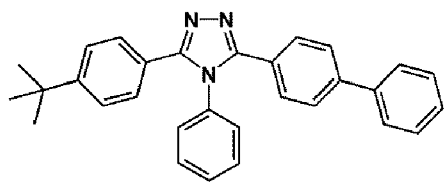

TAZ

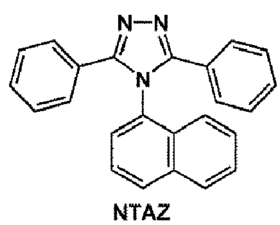

NTAZ

Thicknesses of the buffer layer, the hole-blocking layer, and the electron control layer may each independently be in the range of about 20 Å to about 1,000 Å, for example, about 30 Å to about 300 Å. When the thicknesses of the buffer layer, the hole-blocking layer, and the electron control layer are within these ranges, excellent hole blocking characteristics or excellent electron control characteristics may be obtained without a substantial increase in driving voltage.

A thickness of the electron transport layer may be in the range of about 100 Å to about 1,000 Å, for example, about 150 Å to about 500 Å. When the thickness of the electron transport layer is within the range described above, the

electron transport layer may have satisfactory electron-transporting characteristics without a substantial increase in driving voltage.

The electron transport region 17 (for example, the electron transport layer in the electron transport region) may further include, in addition to the materials described above, a metal-containing material.

The metal-containing material may include at least one of an alkali metal complex or an alkaline earth-metal complex. The alkali metal complex may include a metal ion that is a Li ion, a Na ion, a K ion, a Rb ion, or a Cs ion, and the alkaline earth-metal complex may include a metal ion that is a Be ion, a Mg ion, a Ca ion, a Sr ion, or a Ba ion. A ligand coordinated with the metal ion of the alkali metal complex or the metal ion of the alkaline earth-metal complex may be a hydroxy quinoline, a hydroxy isoquinoline, a hydroxy benzoquinoline, a hydroxy acridine, a hydroxy phenanthridine, a hydroxy phenyloxazole, a hydroxy phenylthiazole, a hydroxy diphenyloxadiazole, a hydroxy diphenylthiadiazole, a hydroxy phenylpyridine, a hydroxy phenylbenzimidazole, a hydroxy phenylbenzothiazole, a bipyridine, a phenanthroline, or a cyclopentadiene, but embodiments of the present disclosure are not limited thereto.

In one or more embodiments, the metal-containing material may include a Li complex. The Li complex may include, for example, Compound ET-D1 (lithium quinolate, LiQ) or ET-D2:

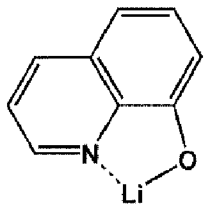

ET-D1

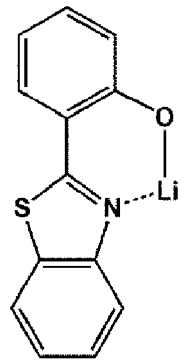

ET-D2

The electron transport region 17 may include an electron injection layer that facilitates the injection of electrons from the second electrode 19. The electron injection layer may directly contact the second electrode 19.

The electron injection layer may have i) a single-layered structure including a single layer including a single material, ii) a single-layered structure including a single layer including a plurality of different materials, or iii) a multi-layered structure having a plurality of layers including a plurality of different materials.

The electron injection layer may include an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or a combination thereof.

The alkali metal may be Li, Na, K, Rb, or Cs. In one or more embodiments, the alkali metal may be Li, Na, or Cs. In one or more embodiments, the alkali metal may be Li or Cs, but embodiments of the present disclosure are not limited thereto.

The alkaline earth metal may be Mg, Ca, Sr, or Ba.

The rare earth metal may be Sc, Y, Ce, Tb, Yb, or Gd.

The alkali metal compound, the alkaline earth-metal compound, and the rare earth metal compound may be an oxide or a halide (for example, fluorides, chlorides, bromides, or iodides) of the alkali metal, the alkaline earth-metal, or the rare earth metal.

The alkali metal compound may be an alkali metal oxide, such as $Li_2O$, $Cs_2O$, or $K_2O$, or an alkali metal halide, such as LiF, NaF, CsF, KF, LiI, NaI, CsI, or KI. In one or more embodiments, the alkali metal compound may be LiF, $Li_2O$, NaF, LiI, NaI, CsI, or KI, but embodiments of the present disclosure are not limited thereto.

The alkaline earth-metal compound may be an alkaline earth-metal oxide, such as BaO, SrO, CaO, $Ba_xSr_{1-x}O$ ($0<x<1$), or $Ba_xCa_{1-x}O$ ($0<x<1$). In one or more embodiments, the alkaline earth-metal compound may be BaO, SrO, or CaO, but embodiments of the present disclosure are not limited thereto.

The rare earth metal compound may be $YbF_3$, $ScF_3$, $Sc_2O_3$, $Y_2O_3$, $Ce_2O_3$, $GdF_3$, or $TbF_3$. In one or more embodiments, the rare earth metal compound may be $YbF_3$, $ScF_3$, $TbF_3$, $YbI_3$, $ScI_3$, or $TbI_3$, but embodiments of the present disclosure are not limited thereto.

The alkali metal complex, the alkaline earth-metal complex, and the rare earth metal complex may include an ion of alkali metal, alkaline earth-metal, and/or rare earth metal as described above, and a ligand coordinated with a metal ion of the alkali metal complex, the alkaline earth-metal complex, and/or the rare earth metal complex may be hydroxy quinoline, hydroxy isoquinoline, hydroxy benzoquinoline, hydroxy acridine, hydroxy phenanthridine, hydroxy

phenyloxazole, hydroxy phenylthiazole, hydroxy diphenyloxadiazole, hydroxy diphenylthiadiazole, hydroxy phenylpyridine, hydroxy phenylbenzimidazole, hydroxy phenylbenzothiazole, bipyridine, phenanthroline, or cyclopentadiene, but embodiments of the present disclosure are not limited thereto.

The electron injection layer may consist of an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or a combination thereof, as described above. In one or more embodiments, the electron injection layer may further include an organic material. When the electron injection layer further includes an organic material, an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or a combination thereof, may be homogeneously or non-homogeneously dispersed in a matrix including the organic material.

A thickness of the electron injection layer may be in a range of about 1 Å to about 100 Å, and, for example, about 3 Å to about 90 Å. When the thickness of the electron injection layer is within the range described above, the electron injection layer may have satisfactory electron injection characteristics without a substantial increase in driving voltage.

Second electrode 19

The second electrode 19 is located on the organic layer 10A having such a structure. The second electrode 19 may be a cathode which is an electron injection electrode, and in this regard, a material for forming the second electrode 19 may be a metal, an alloy, an electrically conductive compound, or a combination thereof, which have a relatively low work function.

The second electrode 19 may include at least one of lithium (Li), silver (Ag), magnesium (Mg), aluminum (Al), aluminum-lithium (Al-Li), calcium (Ca), magnesium-indium (Mg-In), magnesium-silver (Mg-Ag), ITO, or IZO, but embodiments of the present disclosure are not limited thereto. The second electrode 19 may be a transmissive electrode, a semi-transmissive electrode, or a reflective electrode.

The second electrode 19 may have a single-layered structure having a single layer or a multi-layered structure including two or more layers.

Hereinbefore, the organic light-emitting device has been described with reference to FIG. 1, but embodiments of the present disclosure are not limited thereto.

Description of FIG. 2

FIG. 2 is a schematic cross-sectional view of an organic light-emitting device 100 according to another embodiment.

The organic light-emitting device 100 of FIG. 2 includes a first electrode 110, a second electrode 190 facing the first electrode 110, and a first light-emitting unit 151 and a second light-emitting unit 152 between the first electrode 110 and the second electrode 190. A charge generation layer 141 is located between the first emission unit 151 and the second emission unit 152, and the charge generation layer 141 may include an n-type charge generation layer 141-N and a p-type charge generation layer 141-P. The charge generation layer 141 is a layer that generates charge and supplies the charge to neighboring emission units, and any known material may be used therefor.

The first emission unit 151 may include a first emission layer 151-EM, and the second emission unit 152 may include a second emission layer 152-EM. The maximum emission wavelength of light emitted from the first emission unit 151 may be different from the maximum emission wavelength of light emitted from the second emission unit 152. For example, the mixed light of the light emitted from the first emission unit 151 and the light emitted from the second emission unit 152 may be white light, but embodiments of the present disclosure are not limited thereto.

The hole transport region 120 is located between the first emission unit 151 and the first electrode 110, and the second emission unit 152 may include the first hole transport region 121 located on the side of the first electrode 110 .

An electron transport region 170 is located between the second emission unit 152 and the second electrode 190, and the first emission unit 151 may include a first electron transport region 171 located between the charge generation layer 141 and the first emission layer 151-EM.

The first emission layer 151-EM includes the composition described above.

For example, the first emission layer 151-EM may include a host, a dopant, and a sensitizer, the sensitizer may include the first compound of the composition, the dopant may include the second compound of the composition, and the host may include the third compound of the composition.

The second emission layer 152-EM includes the above composition.

For example, the second emission layer 152-EM may include a host, a dopant, and a sensitizer, the sensitizer may include the first compound of the composition, the dopant may include the second compound of the composition,

and the host may include the third compound of the composition.

The first electrode 110 and the second electrode 190 illustrated in FIG. 2 may be the same as described in connection with the first electrode 11 and the second electrode 19 illustrated in FIG. 1.

The first emission layer 151-EM and the second emission layer 152-EM illustrated in FIG. 2 are each the same as described in connection with the emission layer 15 illustrated in FIG. 2.

The hole transport region 120 and the first hole transport region 121 illustrated in FIG. 2 are each the same as described in connection with the hole transport region 12 illustrated in FIG. 1.

The electron transport region 170 and the first electron transport region 171 illustrated in FIG. 2 are each the same as described in connection with the electron transport region 17 illustrated in FIG. 1.

As described above, referring to FIG. 2, an organic light-emitting device in which each of the first light-emitting unit 151 and the second light-emitting unit 152 includes an emission layer including a host, a dopant, and a sensitizer, has been described. However, the organic light-emitting device may have various other forms. For example, one of the first light-emitting unit 151 and the second light-emitting unit 152 of the organic light-emitting device 100 of FIG. 2 may be replaced with any known light-emitting unit, or may include three or more light-emitting units.

Description of FIG. 3

FIG. 3 is a schematic cross-sectional view of an organic light-emitting device 200 according to another embodiment.

The organic light-emitting device 200 includes a first electrode 210, a second electrode 290 facing the first electrode 210, and a first emission layer 251 and a second emission layer 252 which are stacked between the first electrode 210 and the second electrode 290.

The maximum emission wavelength of light emitted from the first emission layer 251 may be different from the maximum emission wavelength of light emitted from the second emission layer 252. For example, the mixed light of the light emitted from the first emission layer 251 and the light emitted from the second emission layer 252 may be white light, but embodiments of the present disclosure are not limited thereto.

In one or more embodiments, a hole transport region 220 may be located between the first emission layer 251 and the first electrode 210, and an electron transport region 270 may be located between the second emission layer 252 and the second electrode 290.

The first emission layer 251 includes the composition described above.

For example, the first emission layer 251 may include a host, a dopant, and a sensitizer, the sensitizer may include the first compound of the composition, the dopant may include the second compound of the composition, and the host may include the third compound of the composition.

The second emission layer 252 includes the composition described above.

For example, the second emission layer 252 may include a host, a dopant, and a sensitizer, the sensitizer may include the first compound of the composition, the dopant may include the second compound of the composition, and the host may include the third compound of the composition.

The first electrode 210, the hole transport region 220, and the second electrode 290 illustrated in FIG. 3 are respectively the same as described in connection with the first electrode 11, the hole transport region 12, and the second electrode 19 illustrated in FIG. 1.

The first emission layer 251 and the second emission layer 252 illustrated in FIG. 3 are each the same as described in connection with the emission layer 15 illustrated in FIG. 1.

The electron transport region 270 illustrated in FIG. 3 may be the same as described in connection with the electron transport region 17 in FIG. 1.

As described above, referring to FIG. 3, an organic light-emitting device, in which each of the first emission layer 251 and the second emission layer 252 includes a host, a dopant, and a sensitizer, has been described. However, the organic light-emitting device may have various other forms. For example, one of the first emission layer 251 and the second emission layer 252 of the organic light-emitting device 200 of FIG. 3 may be replaced with any known emission layer, or an interlayer may be additionally located between neighboring emission layers.

Explanation of terms

The term "$C_1$-$C_{60}$ alkyl group" as used herein refers to a linear or branched saturated aliphatic hydrocarbon monovalent group having 1 to 60 carbon atoms, and examples thereof include a methyl group, an ethyl group, a propyl group, an isobutyl group, a sec-butyl group, a tert-butyl group, a pentyl group, an isoamyl group, and a hexyl group. The term "$C_1$-$C_{60}$ alkylene group" as used herein refers to a divalent group having the same structure as the $C_1$-$C_{60}$ alkyl group.

The term "$C_1$-$C_{60}$ alkoxy group" as used herein refers to a monovalent group represented by $-OA_{101}$ (wherein $A_{101}$ is a $C_1$-$C_{60}$ alkyl group), and examples thereof include a methoxy group, an ethoxy group, and an isopropyloxy

group. The term "$C_1$-$C_{60}$ alkylthio group " as used herein refers to a monovalent group represented by - $SA_{101}$ (wherein $A_{101}$ is a $C_1$-$C_{60}$ alkyl group).

The term "$C_2$-$C_{60}$ alkenyl group" as used herein refers to a hydrocarbon group formed by substituting at least one carbon-carbon double bond in the middle or at the terminus of the $C_2$-$C_{60}$ alkyl group, and examples thereof include an ethenyl group, a propenyl group, and a butenyl group. The term "$C_2$-$C_{60}$ alkenylene group" as used herein refers to a divalent group having the same structure as the $C_2$-$C_{60}$ alkenyl group.

The term "$C_2$-$C_{60}$ alkynyl group" as used herein refers to a hydrocarbon group formed by substituting at least one carbon-carbon triple bond in the middle or at the terminus of the $C_2$-$C_{60}$ alkyl group, and examples thereof include an ethynyl group, and a propynyl group. The term "$C_2$-$C_{60}$ alkynylene group" as used herein refers to a divalent group having the same structure as the $C_2$-$C_{60}$ alkynyl group.

The term "$C_3$-$C_{10}$ cycloalkyl group" as used herein refers to a monovalent saturated hydrocarbon monocyclic group having 3 to 10 carbon atoms, and examples thereof include a cyclopropyl group, a cyclobutyl group, a cyclopentyl group, a cyclohexyl group, and a cycloheptyl group. The term "$C_3$-$C_{10}$ cycloalkylene group" as used herein refers to a divalent group having the same structure as the $C_3$-$C_{10}$ cycloalkyl group.

The term "$C_1$-$C_{10}$ heterocycloalkyl group" as used herein refers to a monovalent saturated monocyclic group having at least one heteroatom that is N, O, P, Si, or S as a ring-forming atom and 1 to 10 carbon atoms, and non-limiting examples thereof include a tetrahydrofuranyl group, and a tetrahydrothiophenyl group. The term "$C_1$-$C_{10}$ heterocycloalkylene group" as used herein refers to a divalent group having the same structure as the $C_1$-$C_{10}$ heterocycloalkyl group.

The term "$C_3$-$C_{10}$ cycloalkenyl group" as used herein refers to a monovalent monocyclic group that has 3 to 10 carbon atoms and at least one carbon-carbon double bond in the ring thereof and no aromaticity, and non-limiting examples thereof include a cyclopentenyl group, a cyclohexenyl group, and a cycloheptenyl group. The term "$C_3$-$C_{10}$ cycloalkenylene group" as used herein refers to a divalent group having the same structure as the $C_3$-$C_{10}$ cycloalkenyl group.

The term "$C_1$-$C_{10}$ heterocycloalkenyl group" as used herein refers to a monovalent monocyclic group that has at least one heteroatom that is N, O, P, Si, or S as a ring-forming atom, 1 to 10 carbon atoms, and at least one double bond in its ring. Examples of the $C_1$-$C_{10}$ heterocycloalkenyl group are a 2,3-dihydrofuranyl group, and a 2,3-dihydrothiophenyl group. The term "$C_1$-$C_{10}$ heterocycloalkenylene group" as used herein refers to a divalent group having the same structure as the $C_1$-$C_{10}$ heterocycloalkenyl group.

The term "$C_6$-$C_{60}$ aryl group" as used herein refers to a monovalent group having a carbocyclic aromatic system having 6 to 60 carbon atoms, and the term "$C_6$-$C_{60}$ arylene group" as used herein refers to a divalent group having a carbocyclic aromatic system having 6 to 60 carbon atoms. Examples of the $C_6$-$C_{60}$ aryl group include a phenyl group, a naphthyl group, an anthracenyl group, a phenanthrenyl group, a pyrenyl group, and a chrysenyl group. When the $C_6$-$C_{60}$ aryl group and the $C_6$-$C_{60}$ arylene group each include two or more rings, the rings may be fused to each other.

The term "$C_7$-$C_{60}$ alkyl aryl group" as used herein refers to a $C_{6-60}$ aryl group that is substituted with a $C_{1-60}$ alkyl group. The term "$C_7$-$C_{60}$ aryl alkyl group" as used herein refers to a $C_{1-60}$ alkyl group that is substituted with a $C_{6-60}$ aryl group.

The term "$C_1$-$C_{60}$ heteroaryl group" as used herein refers to a monovalent group having a heterocarbocyclic aromatic system that has at least one heteroatom that is N, O, P, Si, or S as a ring-forming atom, and 1 to 60 carbon atoms. The term "$C_1$-$C_{60}$ heteroarylene group" as used herein refers to a divalent group having a carbocyclic aromatic system that has at least one heteroatom that is N, O, P, Si, or S as a ring-forming atom, and 1 to 60 carbon atoms. Examples of the $C_1$-$C_{60}$ heteroaryl group include a pyridinyl group, a pyrimidinyl group, a pyrazinyl group, a pyridazinyl group, a triazinyl group, a quinolinyl group, and an isoquinolinyl group. When the $C_6$-$C_{60}$ heteroaryl group and the $C_6$-$C_{60}$ heteroarylene group each include two or more rings, the rings may be fused to each other.

The term "$C_2$-$C_{60}$ alkyl heteroaryl group" as used herein refers to a $C_{1-60}$ heteroaryl group that is substituted with a $C_{1-60}$ alkyl group. The term "$C_2$-$C_{60}$ heteroaryl alkyl group" as used herein refers to a $C_{1-60}$ alkyl group that is substituted with a $C_{1-60}$ heteroaryl group.

The term "$C_6$-$C_{60}$ aryloxy group" as used herein indicates -$OA_{102}$ (wherein $A_{102}$ is the $C_6$-$C_{60}$ aryl group), and the term "$C_6$-$C_{60}$ arylthio group" as used herein indicates -$SA_{103}$ (wherein $A_{103}$ is the $C_6$-$C_{60}$ aryl group). The term "$C_1$-$C_{60}$ heteroaryloxy group" as used herein indicates -$OA_{104}$ (wherein $A_{104}$ is the $C_1$-$C_{60}$ heteroaryl group), and the term "$C_6$-$C_{60}$ heteroarylthio group" as used herein indicates -$SA_{105}$ (wherein $A_{105}$ is the C1-C60 heteroaryl group).

The term "monovalent non-aromatic condensed polycyclic group" used herein refers to a monovalent group in which two or more rings are condensed with each other, only carbon is used as a ring-forming atom (for example, the number of carbon atoms may be 8 to 60) and the whole molecule is a non-aromaticity group. Examples of the monovalent non-aromatic condensed polycyclic group include a fluorenyl group. The term "divalent non-aromatic condensed polycyclic group" as used herein refers to a divalent group having the same structure as a monovalent non-aromatic condensed polycyclic group.

The term "monovalent non-aromatic condensed heteropolycyclic group" as used herein refers to a monovalent

group having two or more rings condensed to each other, a heteroatom that is N, O, P, Si, or S, other than carbon atoms(for example, having 1 to 60 carbon atoms), as a ring-forming atom, and no aromaticity in its entire molecular structure. Examples of the monovalent non-aromatic condensed heteropolycyclic group include a carbazolyl group. The term "divalent non-aromatic heterocondensed polycyclic group" as used herein refers to a divalent group having the same structure as a monovalent non-aromatic heterocondensed polycyclic group.

The term "$C_5$-$C_{30}$ carbocyclic group" as used herein refers to a saturated or unsaturated cyclic group having, as a ring-forming atom, 5 to 30 carbon atoms only. The $C_5$-$C_{30}$ carbocyclic group may be a monocyclic group or a polycyclic group, and may be a monovalent, divalent, trivalent, tetravalent, pentavalent, or hexavalent group, depending on the formula structure.

The term "$C_1$-$C_{30}$ heterocyclic group" as used herein refers to a saturated or unsaturated cyclic group having, as a ring-forming atom, at least one heteroatom that is N, O, P, Si, or S other than 1 to 30 carbon atoms. The $C_1$-$C_{30}$ heterocyclic group may be a monocyclic group or a polycyclic group, and may be a monovalent, divalent, trivalent, tetravalent, pentavalent, or hexavalent group, depending on the formula structure.

At least one substituent of the substituted $C_5$-$C_{60}$ carbocyclic group, the substituted $C_1$-$C_{60}$ heterocyclic group, the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_1$-$C_{60}$ alkylthio group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_7$-$C_{60}$ alkyl aryl group, the substituted $C_7$-$C_{60}$ aryl alkyl group, the substituted $C_6$-$C_{60}$ aryloxy group, the substituted $C_6$-$C_{60}$ arylthio group, the substituted $C_1$-$C_{60}$ heteroaryl group, the substituted $C_2$-$C_{60}$ alkyl heteroaryl group, the substituted $C_2$-$C_{60}$ heteroaryl alkyl group, the substituted $C_1$-$C_{60}$ heteroaryloxy group, the substituted $C_1$-$C_{60}$ heteroarylthio group, the substituted monovalent non-aromatic condensed polycyclic group, and the substituted monovalent non-aromatic condensed heteropolycyclic group may be:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;

a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, $-N(Q_{11})(Q_{12})$, $-Si(Q_{13})(Q_{14})(Q_{15})$, $-B(Q_{16})(Q_{17})$, $-P(Q_{18})(Q_{19})$-, - $P(=S)(Q_{18})(Q_{19})$, or $-P(=O)(Q_{18})(Q_{19})$;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, a $C_1$-$C_{60}$ heteroaryloxy group, a $C_1$-$C_{60}$ heteroarylthio group, or a monovalent non-aromatic condensed heteropolycyclic group;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a $C_1$-$C_{60}$ heteroaryloxy group, a $C_1$-$C_{60}$ heteroarylthio group, a monovalent non-aromatic condensed polycyclic group, or a monovalent non-aromatic condensed heteropolycyclic group, each substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_1$-$C_{60}$ alkylthio group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alky aryl group, a $C_7$-$C_{60}$ aryl alkyl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a $C_2$-$C_{60}$ heteroaryl alkyl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, $-N(Q_{21})(Q_{22})$, - $Si(Q_{23})(Q_{24})(Q_{25})$, -B(Q26)(Q27), -P(Q28)(Q29), -P(=S)(Q28)(Q29), or $-P(=O)(Q_{28})(Q_{29})$; or

$-N(Q_{31})(Q_{32})$, $-Si(Q_{33})(Q_{34})(Q_{35})$, -B(Q36)(Q37), P(Q28)(Q29), - P(=S)(Q28)(Q29), or $-P(=O)(Q_{38})(Q_{39})$,

wherein $Q_1$ to $Q_9$, $Q_{11}$ to $Q_{19}$, $Q_{21}$ to $Q_{29}$ and $Q_{31}$ to $Q_{39}$ may each independently be hydrogen, deuterium, -F, -Cl, -Br, -I, a hydroxyl group, a cyano group, a nitro group, an amino group, an amidino group, a hydrazine group, a

hydrazone group, a carboxylic acid group or a salt thereof, a sulfonic acid group or a salt thereof, a phosphoric acid group a $C_1$-$C_{60}$ alkylthio group, or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryl group substituted with at least one of a $C_1$-$C_{60}$ alkyl group or a $C_6$-$C_{60}$ aryl group, a $C_6$-$C_{60}$ aryloxy group, a $C_6$-$C_{60}$ arylthio group, a $C_1$-$C_{60}$ heteroaryl group, a $C_1$-$C_{60}$ heteroaryloxy group, a $C_1$-$C_{60}$ heteroarylthio group, a monovalent non-aromatic condensed polycyclic group, or a monovalent non-aromatic condensed heteropolycyclic group.

The term "room temperature" used herein refers to a temperature of about 25 °C.

The terms "a biphenyl group, a terphenyl group, and a quaterphenyl group" as used herein respectively refer to monovalent groups in which two, three, or four phenyl groups which are linked together via a single bond.

The terms "a cyano-containing phenyl group, a cyano-containing biphenyl group, a cyano-containing terphenyl group, and a cyano-containing quaterphenyl group" as used herein respectively refer to a phenyl group, a biphenyl group, a terphenyl group, and a quaterphenyl group, each of which is substituted with at least one cyano group. In "a cyano-containing phenyl group, a cyano-containing biphenyl group, a cyano-containing terphenyl group, and a cyano-containing quaterphenyl group", a cyano group may be substituted to any position of the corresponding group, and the "cyano-containing phenyl group, the cyano-containing biphenyl group, the cyano-containing terphenyl group, and the cyano-containing quaterphenyl group" may further include substituents other than a cyano group. For example, a phenyl group substituted with a cyano group, and a phenyl group substituted with a cyano group and a methyl group may all belong to "a cyano-containing phenyl group."

Hereinafter, a compound and an organic light-emitting device according to one or more exemplary embodiments are described in further detail with reference to Synthesis Examples and Examples. However, the organic light-emitting device is not limited thereto. The wording "'B' was used instead of 'A'" used in describing Synthesis Examples means that an amount of 'A' used was identical to an amount of 'B' used, in terms of a molar equivalent.

EXAMPLES

Example 1-1

An ITO glass substrate was cut to a size of 50 millimeters (mm) × 50 mm × 0.5 mm and then, sonicated in acetone isopropyl alcohol and deionized (DI) water, each for 15 minutes, and then, cleaned by exposure to ultraviolet (UV) light and ozone for 30 minutes.

Subsequently, F6-TCNNQ was deposited on the ITO electrode (anode) on the glass substrate to form a hole injection layer having a thickness of 100 Å, and HT1 was deposited on the hole injection layer to form a hole transport layer having a thickness of 1260 Å, thereby completing the manufacture of a hole transport region.

Compound H-H104 (hereinafter referred to as H-H1 ((first host), Compound 57 of Group HE4 (hereinafter referred to as H-E1)(second host), Compound SP001(sensitizer)(where the weight ratio of the first host, the second host, and the sensitizer is 45:45:10), and FD1(dopant)(where the amount of the dopant is 0.1 weight percent (wt%) based on the total weight of the first host, the second host, the sensitizer, and the dopant) were co-deposited on the hole transport region to form an emission layer having a thickness of 400 Å.

Compound ET17 and Liq were co-deposited at the weight ratio of 5:5 on the emission layer to form an electron transport layer having a thickness of 360 Å, and then, LiQ was deposited on the electron transport layer to form an electron injection layer having a thickness of 5 Å, and Al was deposited on the electron injection layer to form a cathode having a thickness of 800 Å, thereby completing the manufacture of an organic light-emitting device.

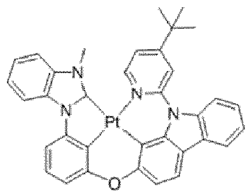

SP001

Examples 1-2 to 1-3, Examples 2-1 to 2-3, Example 3-1, Comparative Example 1, Comparative Examples 1-1 to 1-3, Comparative Example 2, Comparative Examples 2-1 to 2-3, Comparative Example 3, and Comparative Example 3-1

Organic light-emitting devices were manufactured in the same manner as in Example 1-1, except that, in forming

an emission layer, for use as a sensitizer and a dopant, the compounds and amounts shown in Table 7 were used.

Table 7

| | First host | Second host | Sensitizer | Dopant | Dopant amount (wt%) |
|---|---|---|---|---|---|
| Comparative Example 1 | H-H1 | H-E1 | SP001 | - | - |
| Example 1-1 | H-H1 | H-E1 | SP001 | FD1 | 0.1 |
| Example 1-2 | H-H1 | H-E1 | SP001 | FD1 | 0.5 |
| Example 1-3 | H-H1 | H-E1 | SP001 | FD1 | 1.5 |
| Comparative Example 1-1 | H-H1 | H-E1 | SP001 | FD-A | 0.1 |
| Comparative Example 1-2 | H-H1 | H-E1 | SP001 | FD-A | 0.5 |
| Comparative Example 1-3 | H-H1 | H-E1 | SP001 | FD-A | 1.5 |
| Comparative Example 2 | H-H1 | H-E1 | SP002 | - | - |
| Example 2-1 | H-H1 | H-E1 | SP002 | FD1 | 0.1 |
| Example 2-2 | H-H1 | H-E1 | SP002 | FD1 | 0.5 |
| Example 2-3 | H-H1 | H-E1 | SP002 | FD1 | 1.5 |
| Comparative Example 2-1 | H-H1 | H-E1 | SP002 | FD-A | 0.1 |
| Comparative Example 2-2 | H-H1 | H-E1 | SP002 | FD-A | 0.5 |
| Comparative Example 2-3 | H-H1 | H-E1 | SP002 | FD-A | 1.5 |
| Comparative Example 3 | H-H1 | H-E1 | SP003 | - | - |
| Example 3-1 | H-H1 | H-E1 | SP003 | FD2 | 1.5 |
| Comparative Example 3-1 | H-H1 | H-E1 | SP003 | FD-B | 1.5 |

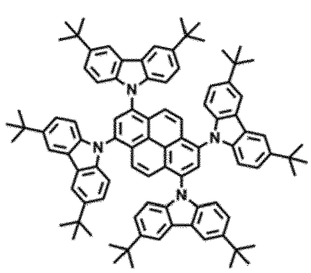

FD1

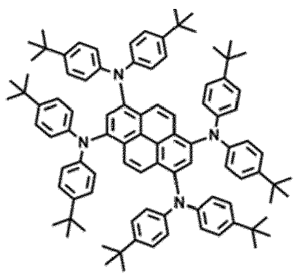

FD2

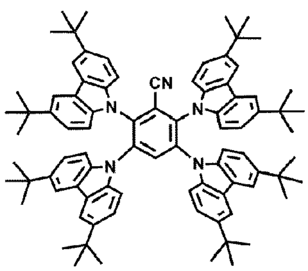

SP002

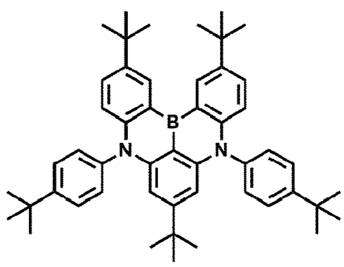

SP003

FD-A

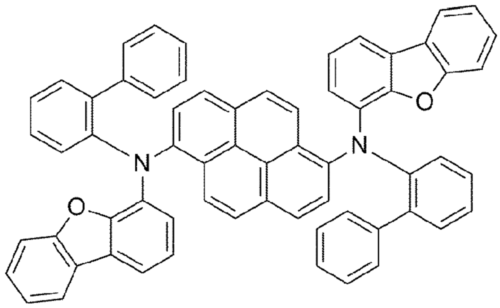

FD-B

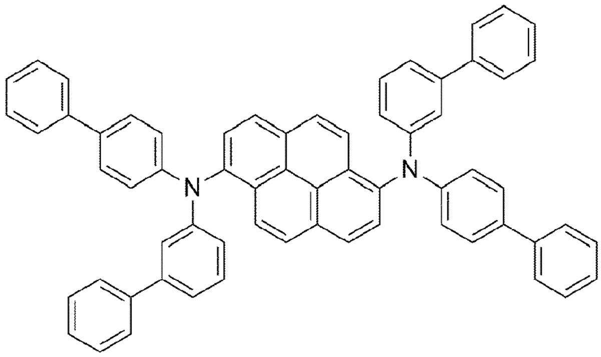

Evaluation Example 1: $\Delta E_{ST}$ of compounds SP002 and SP003

The S1 and T1 energy levels of the compounds SP002 and SP003 were measured according to the method described in Table 8, and the results are shown in Table 9.

Table 8

| | |
|---|---|
| T1 Energy Level Assessment Method | Put a mixture of toluene and each compound (dissolve each compound to a concentration of 1×10-4M in 3 mL of toluene) into a quartz cell, add liquid nitrogen (77 K), and use a photoluminescence measuring instrument to detect photoluminescence. The onset T1 energy level is calculated by measuring the spectrum and comparing it with the normal room temperature photoluminescence spectrum and analyzing only the peak observed only at low temperature. |
| S1 Energy Level Assessment Method | The onset S1 energy level was calculated by measuring the photoluminescence spectrum of a mixture of toluene and each compound (diluted to a concentration of 1x10-4M) at room temperature using a photoluminescence measuring instrument and analyzing the observed peak. |

Table 9

| Compound | $\Delta E_{ST}$ (peak reference) | $\Delta E_{ST}$ (onset reference) |
|---|---|---|
| SP002 | 0.11 eV | 0.15 eV |
| SP003 | 0.329 eV | 0.4 eV |

Evaluation Example 2: Measurement of OLED lifespan and external quantum efficiency

(1) For each of the organic light-emitting devices manufactured according to Comparative Example 1, Examples 1-1 to 1-3, and Comparative Examples 1-1 to 1-3, external quantum efficiency (EQE) and lifespan were evaluated. Results thereof are shown in FIGS. 4 to 7.
First, referring to FIGS. 4 and 5, in the case of Examples 1-1 to 1-3, some of the triplet excitons generated in SP001, which is a phosphorescent sensitizer, were lost by FD1, and the efficiency was slightly reduced compared to Comparative Example 1. However, the lifespan thereof was significantly increased.
On the other hand, referring to FIGS. 6 and 7, in the case of Comparative Examples 1-1 to 1-3, even compared to Examples 1-1 to 1-3 in which the same amount of dopant was included, the reduction in external quantum efficiency was remarkable, and rather, the lifespan was significantly reduced compared to Comparative Example 1. This is because triplet excitons generated in SP001 through dexter energy transfer are transferred to FD-A, and thus, do not contribute to light emission, and deteriorate due to non-radiative decay.

(2) For each of the organic light-emitting devices manufactured according to Comparative Example 2, Examples 2-1 to 2-3, and Comparative Examples 2-1 to 2-3, external quantum efficiency (EQE) and lifespan were evaluated. Results thereof are shown in FIGS. 8 to 11.

First, referring to FIGS. 8 and 9, in the case of Examples 2-1 to 2-3, some of the triplet excitons generated in SP002, which is a TADF sensitizer, was lost by FD1, and the efficiency was slightly reduced compared to Comparative Example 2. However, the lifespan thereof was significantly increased.
On the other hand, referring to FIGS. 10 and 11, in the case of Comparative Examples 2-1 to 2-3, even compared to Examples 2-1 to 2-3 in which the same amount of dopant was included, the reduction in external quantum efficiency was remarkable, and rather, the lifespan was significantly reduced compared to Comparative Example 1. This is because triplet excitons generated in SP002 through dexter energy transfer are transferred to FD-A, and thus, do not contribute to light emission, and deteriorate due to non-radiative decay.
(3) For each of the organic light-emitting devices manufactured according to Comparative Example 3, Example 3-1, and Comparative Example 3-1, external quantum efficiency (EQE) and lifespan were evaluated. Results thereof are shown in FIGS. 12 to 15.

First, referring to FIGS. 12 and 13, in the case of Example 3-1, although some of the triplet excitons generated in SP003, a TADF sensitizer, were lost by FD2, the maximum external quantum efficiency ($EQE_{max}$, %) was increased compared to Comparative Example 3. Furthermore, instead of minimizing TADF emission, FD2 fluorescence emission may occur, leading to a decrease in the lifespan of excitons. Accordingly, the efficiency roll-off is greatly improved, and at 1000 candela per square meter ($cd/m^2$), which is the actual luminance, EQE was rather increased compared to Comparative Example 3, and as described above, the lifespan was significantly increased.
On the other hand, referring to FIGS. 14 and 15, in the case of Comparative Example 3-1, even compared to Example 3-1 in which the same amount of dopant was included, the external quantum efficiency was significantly decreased, and the increase in the lifespan compared to Comparative Example 3 was negligible compared to Example 3-1. This is because triplet excitons generated in SP002 through dexter energy transfer are transferred to FD-A, and thus, do not contribute to light emission, and deteriorate due to non-radiative decay.

Evaluation Example 3: HOD measurement

(1) For Example 1-1, Comparative Example 1, and Comparative Example 1-1, a hole only device (HOD) was fabricated without stacking an electron transport layer and an electron injection layer on an emission layer.
For such a HOD device, a relative HOD value was measured by the method described herein. Results thereof are shown in FIG. 16.
(2) For Example 2-1, Comparative Example 2, and Comparative Example 2-1, a HOD was fabricated in the same manner as in (1), and for such HOD devices, relative HOD values of the HOD devices were measured by the method substrate in this specification. The values are shown in FIG. 17.

Organic light-emitting devices according to one or more embodiments of the present disclosure can have high efficiency and a long lifespan.
It should be understood that the exemplary embodiments described herein should be considered in a descriptive sense only and not for purposes of limitation. Descriptions of features or aspects within each exemplary embodiment should typically be considered as available for other similar features or aspects in other exemplary embodiments. While one or more exemplary embodiments have been described with reference to the figures, it will be understood by those of ordinary skill in the art that various changes in form and details may be made therein without departing from the scope as defined by the following claims.

**Claims**

**1.** A composition comprising a first compound, a second compound, and a third compound, wherein

the first compound, the second compound, and the third compound are different from each other,
the first compound satisfies one of Condition 1 and Condition 2, and
the second compound includes a compound represented by Formula 1:

Condition 1
the first compound contains a transition metal
Condition 2

the difference between a triplet energy level of the first compound and a singlet energy level of the first compound is 0.4 eV or less, and the first compound emits delayed fluorescence

Formula 1

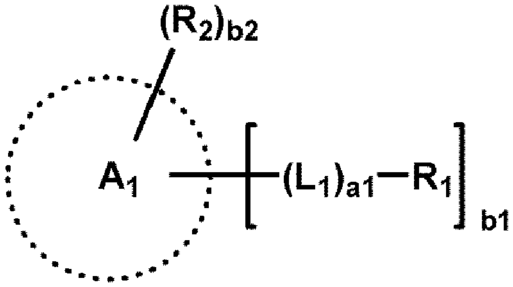

ring $A_1$ in Formula 1 is a condensed cyclic group in which 3 or more cyclic groups are condensed with each other, and the 3 or more cyclic groups are each a $C_5$-$C_{30}$ carbocyclic group or a $C_1$-$C_{30}$ heterocyclic group,
$L_1$ in Formula 1 is a single bond, a $C_5$-$C_{30}$ carbocyclic group that is unsubstituted or substituted with at least one $R_{10a}$, or a $C_1$-$C_{30}$ heterocyclic group that is unsubstituted or substituted with at least one $R_{10a}$,
a1 in Formula 1 is an integer from 1 to 5,
b1 in Formula 1 is an integer from 3 to 10, and
$R_1$ in Formula 1 is a group represented by Formula 2 or Formula 3,

Formula 2

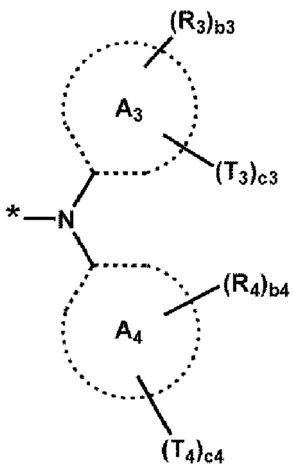

Formula 3

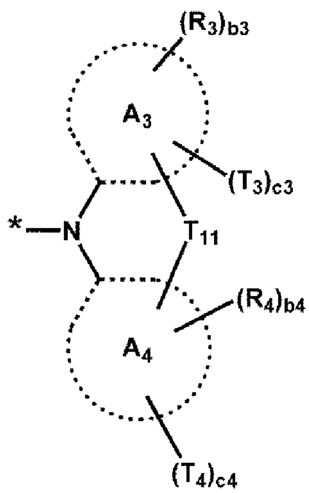

ring $A_3$ and ring $A_4$ in Formulae 2 and 3 are each independently a $C_5$-$C_{30}$ carbocyclic group or a $C_1$-$C_{30}$ heterocyclic group,
$T_3$ and $T_4$ in Formulae 2 and 3 are each independently a group represented by *-C($R_5$)($R_6$)($R_7$) or *-Si($R_5$)($R_6$)($R_7$),
c3 and c4 in Formulae 2 and 3 are each independently an integer from 0 to 10, wherein, when c3 is 2 or more, two or more of $T_3$(s) are identical to or different from each other, and when c4 is 2 or more, two or more of $T_4$(s) are identical to or different from each other,
$T_{11}$ in Formula 3 is a single bond, O, S, N($R_8$), C($R_8$)($R_9$), or Si($R_8$)($R_9$),
$R_2$ to $R_9$ and $R_{10a}$ in Formulae 1 to 3 are each independently hydrogen, deuterium, -F, -Cl, -Br, -I, -$SF_5$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted

or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkyl heteroaryl group, a substituted or unsubstituted $C_2$-$C_{60}$ heteroaryl alkyl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_3)(Q_4)(Q_5)$, $-Ge(Q_3)(Q_4)(Q_5)$, $-B(Q_6)(Q_7)$, $-P(=O)(Q_8)(Q_9)$, $-P(=S)(Q_8)(Q_9)$, or $-P(Q_8)(Q_9)$,
b2 to b4 in Formulae 1 to 3 are each independently an integer from 0 to 10, wherein, when b2 is 2 or more, two or more of $R_2$(s) are identical to or different from each other, when b3 is 2 or more, two or more of $R_3$(s) are identical to or different from each other, and when b4 is 2 or more, two or more of $R_4$(s) are identical to or different from each other, and
* indicates a binding site to a neighboring atom,
at least one substituent of the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_1$-$C_{60}$ alkylthio group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_7$-$C_{60}$ alkyl aryl group, the substituted $C_7$-$C_{60}$ aryl alkyl group, the substituted $C_6$-$C_{60}$ aryloxy group, the substituted $C_6$-$C_{60}$ arylthio group, the substituted $C_1$-$C_{60}$ heteroaryl group, the substituted $C_2$-$C_{60}$ alkyl heteroaryl group, the substituted $C_2$-$C_{60}$ heteroaryl alkyl group, the substituted $C_1$-$C_{60}$ heteroaryloxy group, the substituted $C_1$-$C_{60}$ heteroarylthio group, the substituted monovalent non-aromatic condensed polycyclic group, and the substituted monovalent non-aromatic condensed heteropolycyclic group is:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;
a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, $-N(Q_{11})(Q_{12})$, $-Si(Q_{13})(Q_{14})(Q_{18})$, $-Ge(Q_{13})(Q_{14})(Q_{18})$, $-B(Q_{16})(Q_{17})$, $-P(=O)(Q_{18})(Q_{19})$, $-P(=S)(Q_8)(Q_9)$, $-P(Q_{18})(Q_{19})$, or a combination thereof;
a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a monovalent non-aromatic condensed polycyclic group, or a monovalent non-aromatic condensed heteropolycyclic group, each unsubstituted or substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_1$-$C_{60}$ alkylthio group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alky aryl group, a $C_7$-$C_{60}$ aryl alkyl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a $C_2$-$C_{60}$ heteroaryl alkyl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, $-N(Q_{21})(Q_{22})$,

-Si($Q_{23}$)($Q_{24}$)($Q_{25}$), -Ge($Q_{23}$)($Q_{24}$)($Q_{25}$), -B(Q26)(Q27), - P(=O)($Q_{28}$)($Q_{29}$), -P(=S)($Q_8$)($Q_9$), -P($Q_{28}$)($Q_{29}$), or a combination thereof;

-N($Q_{31}$)($Q_{32}$), -Si($Q_{33}$)($Q_{34}$)($Q_{35}$), -Ge($Q_{33}$)($Q_{34}$)($Q_{35}$), -B(Q36)(Q37), - P(=O)($Q_{38}$)($Q_{39}$), -P(=S)($Q_8$)($Q_9$), or -P($Q_{38}$)($Q_{39}$); or

a combination thereof,

wherein $Q_1$ to $Q_9$, $Q_{11}$ to $Q_{19}$, $Q_{21}$ to $Q_{29}$, and $Q_{31}$ to $Q_{39}$ are each independently: hydrogen; deuterium; -F; -Cl; -Br; -I; a hydroxyl group; a cyano group; a nitro group; an amidino group; a hydrazine group; a hydrazone group; a carboxylic acid group or a salt thereof; a sulfonic acid group or a salt thereof; a phosphoric acid group or a salt thereof; a $C_1$-$C_{60}$ alkyl group which is unsubstituted or substituted with at least one of deuterium, a $C_1$-$C_{60}$ alkyl group, a $C_6$-$C_{60}$ aryl group, or a combination thereof; a $C_2$-$C_{60}$ alkenyl group; a $C_2$-$C_{60}$ alkynyl group; a $C_1$-$C_{60}$ alkoxy group; a $C_1$-$C_{60}$ alkylthio group; a $C_3$-$C_{10}$ cycloalkyl group; a $C_1$-$C_{10}$ heterocycloalkyl group; a $C_3$-$C_{10}$ cycloalkenyl group; a $C_1$-$C_{10}$ heterocycloalkenyl group; a $C_6$-$C_{60}$ aryl group which is unsubstituted or substituted with at least one of deuterium, a $C_1$-$C_{60}$ alkyl group, a $C_6$-$C_{60}$ aryl group, or a combination thereof; a $C_6$-$C_{60}$ aryloxy group; a $C_6$-$C_{60}$ arylthio group; a $C_1$-$C_{60}$ heteroaryl group; a $C_2$-$C_{60}$ alkyl heteroaryl group; a $C_2$-$C_{60}$ heteroaryl alkyl group; a $C_1$-$C_{60}$ heteroaryloxy group; a $C_1$-$C_{60}$ heteroarylthio group; a monovalent non-aromatic condensed polycyclic group; or a monovalent non-aromatic condensed heteropolycyclic group.

**2.** The composition of claim 1, wherein ring $A_1$ in Formula 1 is an anthracene group, a phenalene group, a phenanthrene group, a tetracene group, a pyrene group, a chrysene group, a triphenylene group, a pentacene group, a perylene group, a fluoranthene group, a fluorene group, an acridine group, a phenanthridine group, a phenazine group, a phenoxazine group, a phenothiazine group, a xanthene group, a carbazole group, a dibenzofuran group, or a dibenzothiophene group.

**3.** The composition of claims 1 or 2, wherein the compound represented by Formula 1 is represented by one of Formulae 1-1 to 1-4:

Formula 1-1

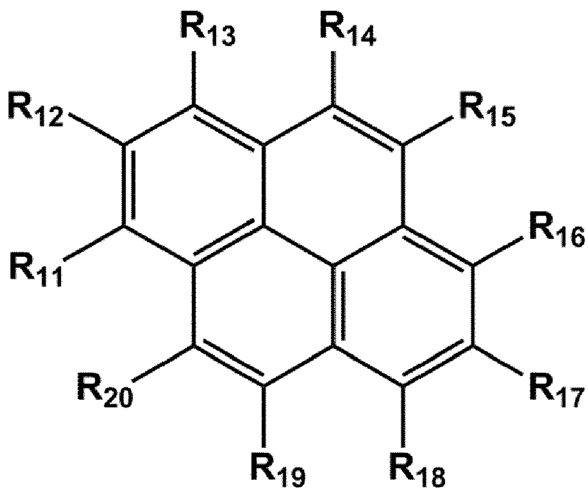

Formula 1-2

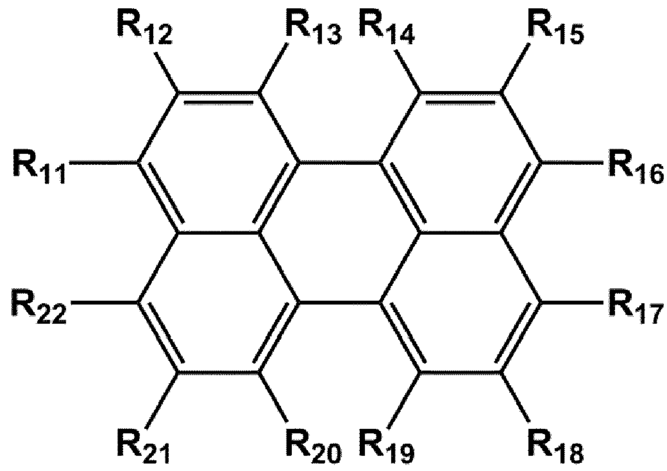

Formula 1-3

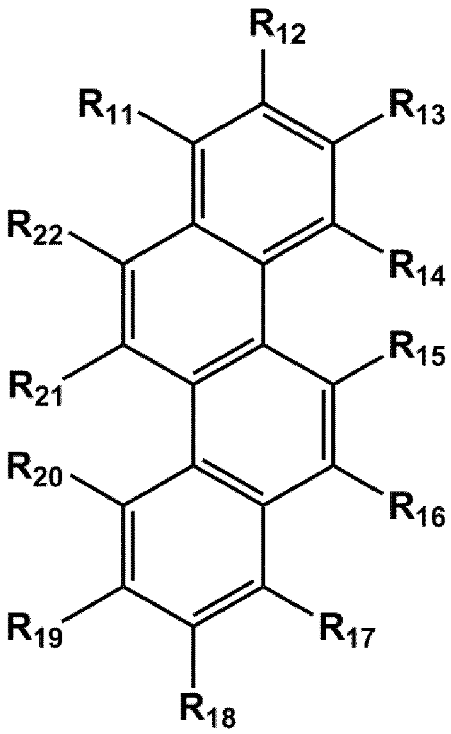

Formula 1-4

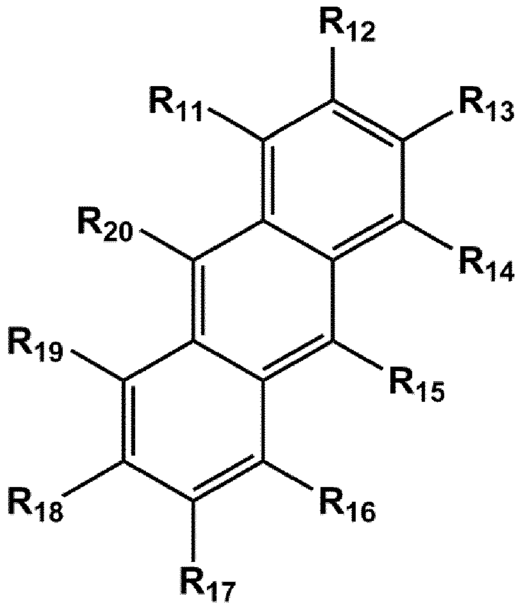

wherein, in Formulae 1-1 to 1-4,

$R_{11}$ to $R_{22}$ are each independently a group represented by *-$(L_1)_{a1}$-$R_1$, hydrogen, deuterium, -F, -Cl, -Br, -I, -$SF_5$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted

$C_2$-$C_{60}$ alkyl heteroaryl group, a substituted or unsubstituted $C_2$-$C_{60}$ heteroaryl alkyl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -N($Q_1$)($Q_2$), -Si($Q_3$)($Q_4$)($Q_5$), -Ge($Q_3$)($Q_4$)($Q_5$), -B($Q_6$)($Q_7$), -P(=O)($Q_8$)($Q_9$), - P(=S)($Q_8$)($Q_9$), or -P($Q_8$)($Q_9$),
at least three of $R_{11}$ to $R_{20}$ in Formulae 1-1 and 1-4 are each independently a group represented by *-($L_1$)$_{a1}$-$R_1$,
at least three of $R_{11}$ to $R_{22}$ in Formulae 1-2 and 1-3 are each independently a group represented by *-($L_1$)$_{a1}$-$R_1$,
* indicates a binding site to a neighboring atom, and
the substituents of the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_1$-$C_{60}$ alkylthio group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_7$-$C_{60}$ alkyl aryl group, the substituted $C_7$-$C_{60}$ aryl alkyl group, the substituted $C_6$-$C_{60}$ aryloxy group, the substituted $C_6$-$C_{60}$ arylthio group, the substituted $C_1$-$C_{60}$ heteroaryl group, the substituted $C_2$-$C_{60}$ alkyl heteroaryl group, the substituted $C_2$-$C_{60}$ heteroaryl alkyl group, the substituted $C_1$-$C_{60}$ heteroaryloxy group, the substituted $C_1$-$C_{60}$ heteroarylthio group, the substituted monovalent non-aromatic condensed polycyclic group, and the substituted monovalent non-aromatic condensed heteropolycyclic group are as described in claim 1;
preferably wherein

i) three or more of $R_{11}$, $R_{13}$, $R_{16}$, and $R_{18}$ in Formula 1-1 are each independently a group represented by *-($L_1$)$_{a1}$-$R_1$,
ii) three or more of $R_{12}$, $R_{15}$, $R_{18}$, and $R_{21}$ in Formula 1-2 are each independently a group represented by *-($L_1$)$_{a1}$-$R_1$,
iii) three or more of $R_{13}$, $R_{16}$, $R_{19}$, and $R_{22}$ in Formula 1-3 are each independently a group represented by *-($L_1$)$_{a1}$-$R_1$, and
iv) three or more of $R_{12}$, $R_{15}$, $R_{17}$, and $R_{20}$ in Formula 1-4 are each independently a group represented by *-($L_1$)$_{a1}$-$R_1$, and

wherein * indicates a binding site to a neighboring atom.

**4.** The composition of any of claims 1-3, wherein the sum of c3 and c4 in Formulae 2 and 3 is 1 or greater; and/or

wherein $R_5$ to $R_7$ in Formulae 2 and 3 are each independently -$CH_3$, -$CD_3$, - $CD_2H$, -$CDH_2$, a phenyl group, or a group represented by one of Formulae 9-1 to 9-19:

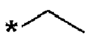
9-1

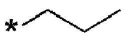
9-2

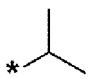
9-3

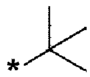
9-4

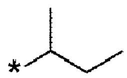
9-5

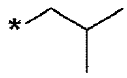
9-6

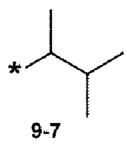
9-7

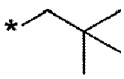
9-8

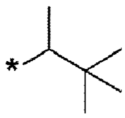
9-9

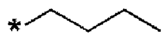
9-10

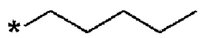
9-11

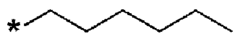
9-12

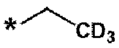
9-13

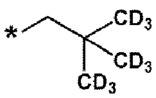
9-14

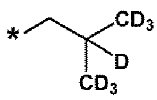
9-15

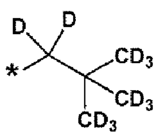
9-16

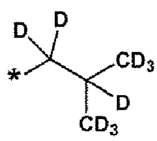
9-17

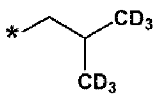
9-18

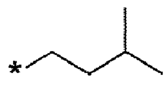

9-19

wherein * in Formulae 9-1 to 9-19 indicates a binding site to a neighboring atom; and/or wherein $R_1$ of Formula 1 is a group represented by one of Formulae 2-1 to 2-42 or 3-1 to 3-36:

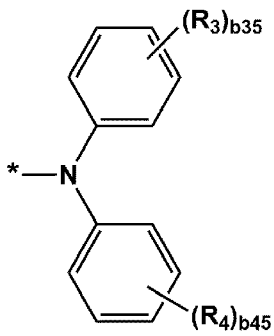

2-1

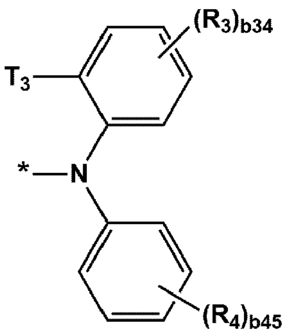

2-2

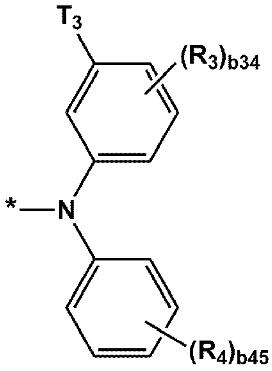

2-3

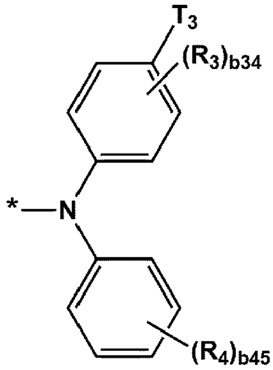

2-4

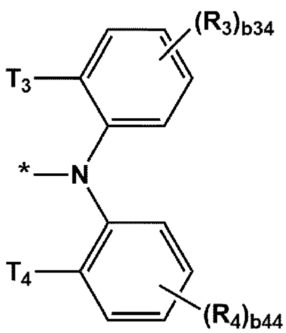

2-5

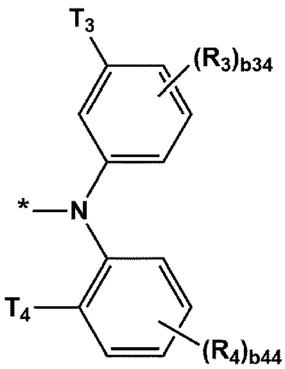

2-6

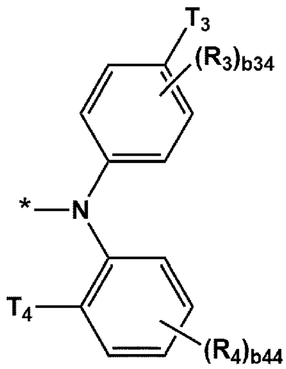

2-7

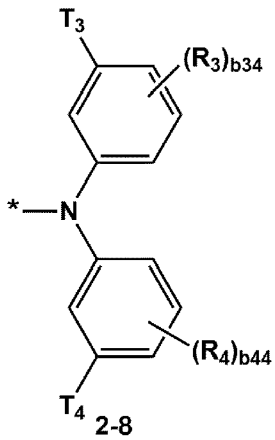

2-8

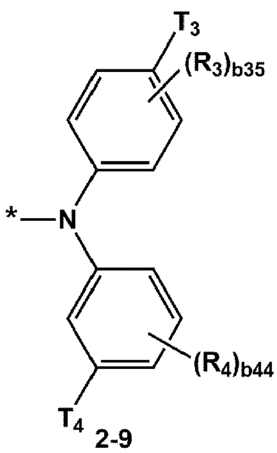

2-9

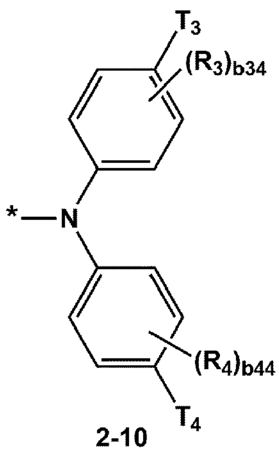

2-10

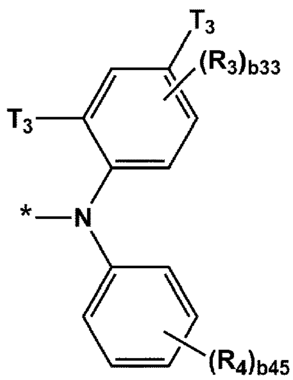

2-11

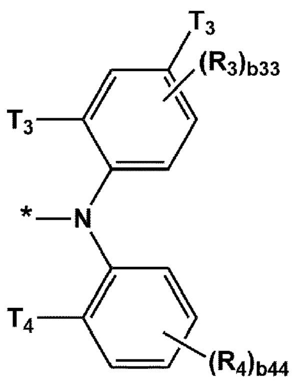

2-12

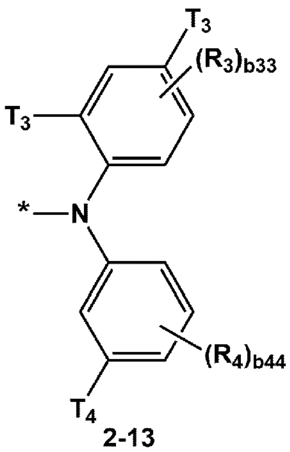
2-13

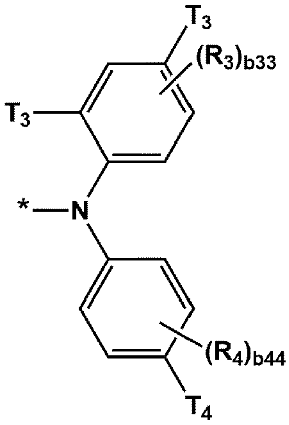
2-14

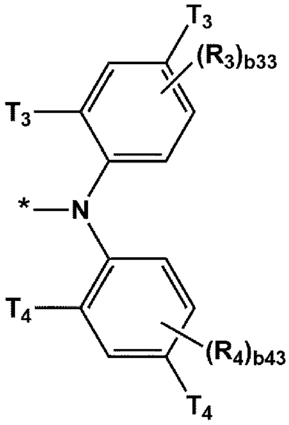
2-15

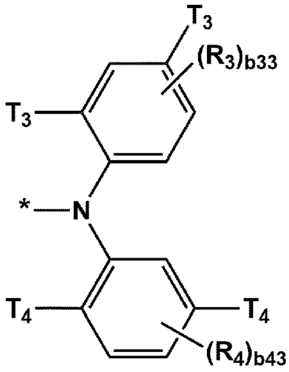
2-16

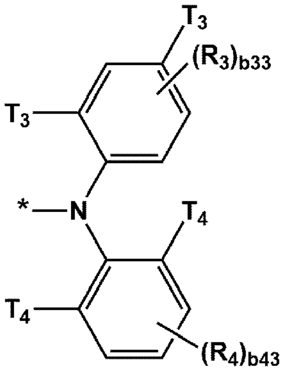
2-17

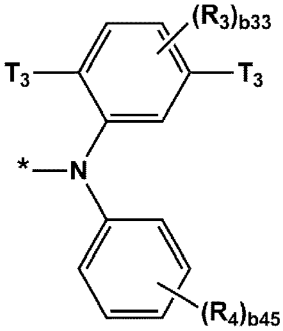
2-18

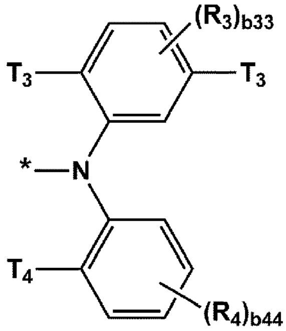
2-19

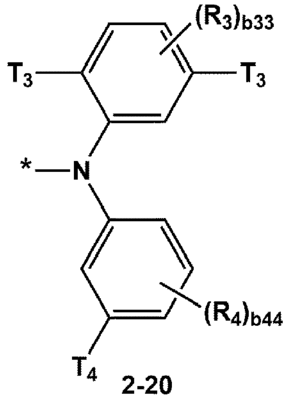
2-20

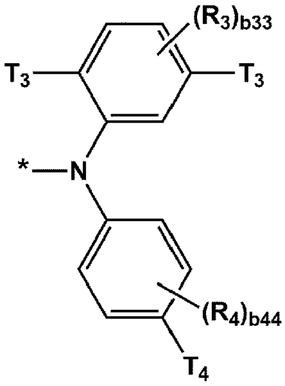
2-21

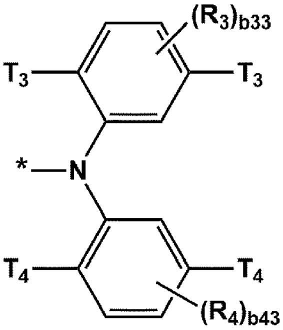
2-22

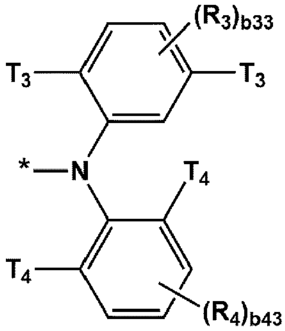
2-23

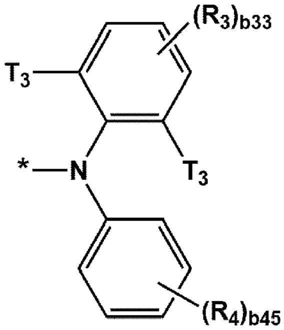
2-24

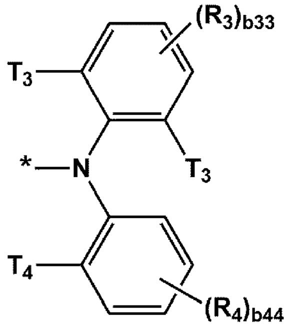

**2-25**

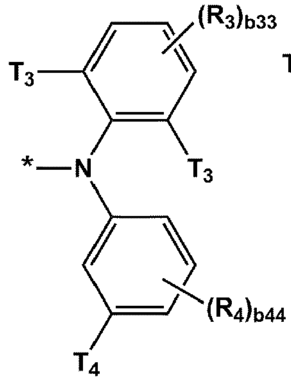

**2-26**

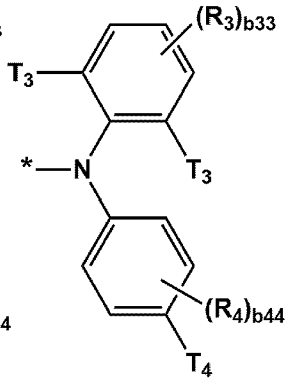

**2-27**

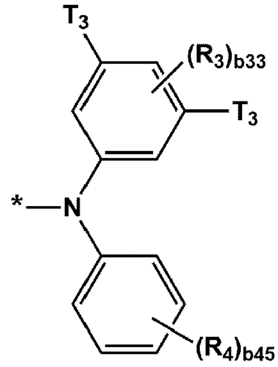

**2-28**

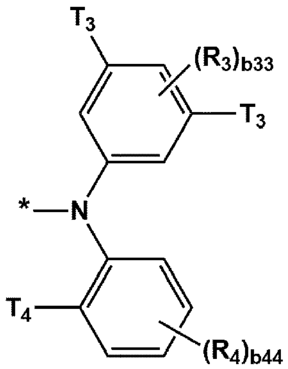

**2-29**

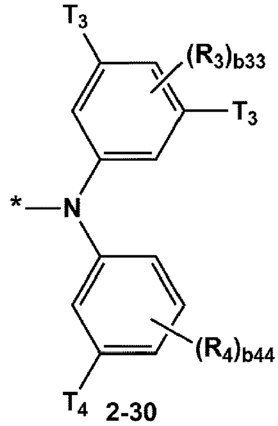

**2-30**

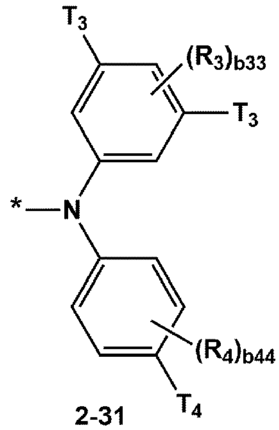

**2-31**

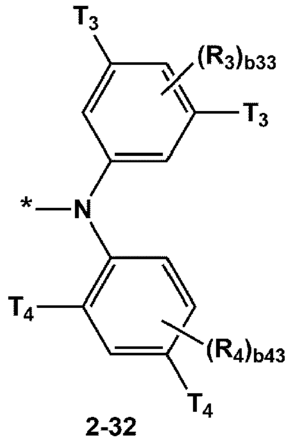

**2-32**

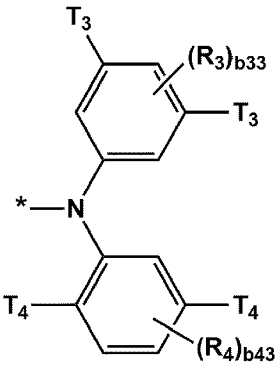

**2-33**

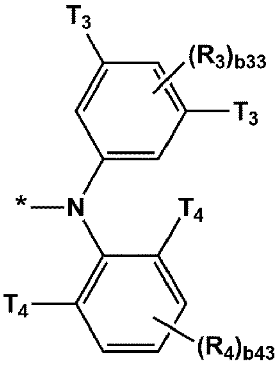

**2-34**

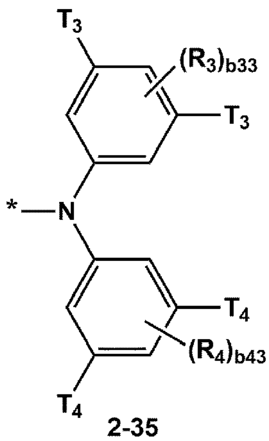

**2-35**

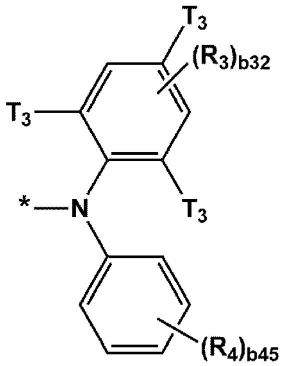

**2-36**

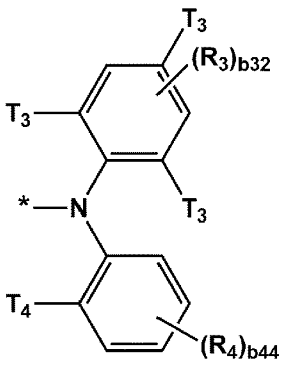

2-37

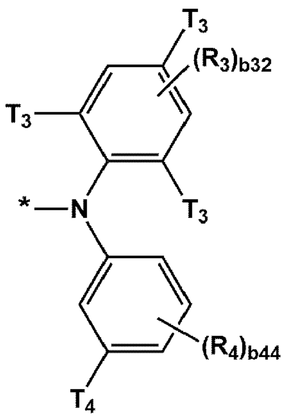

2-38

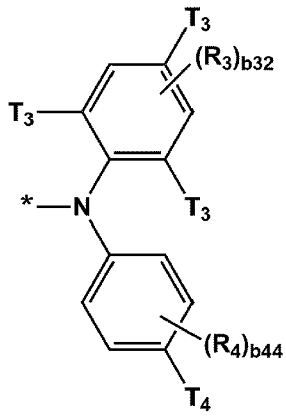

2-39

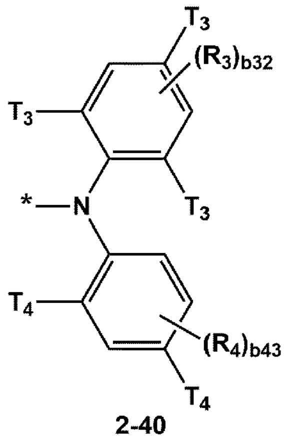

2-40

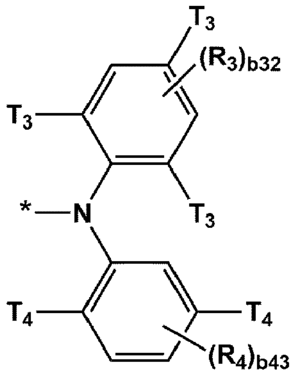

2-41

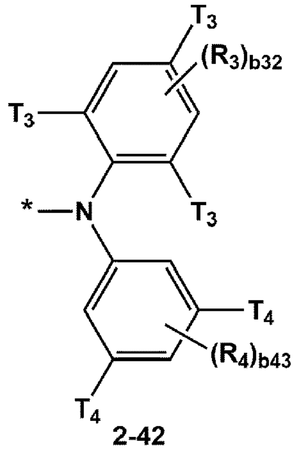

2-42

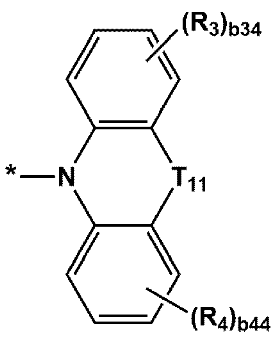

3-1

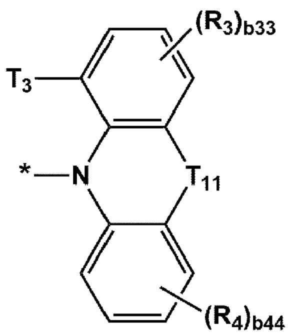

3-2

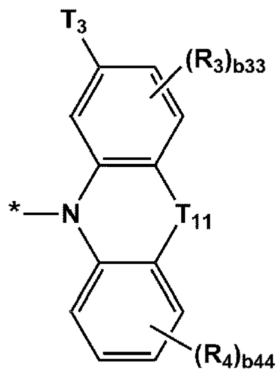

3-3

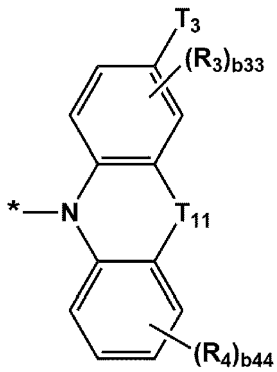

3-4

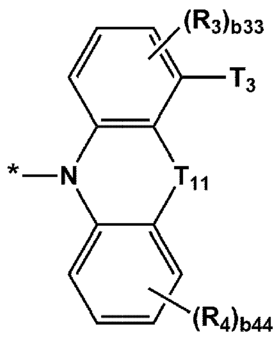

3-5

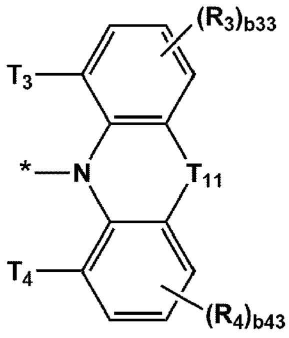

3-6

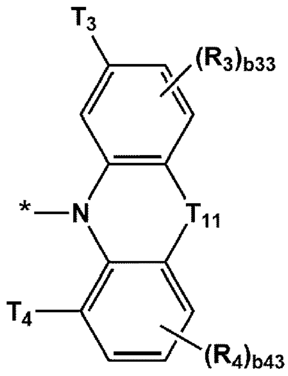

3-7

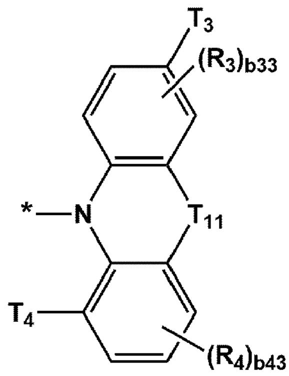

3-8

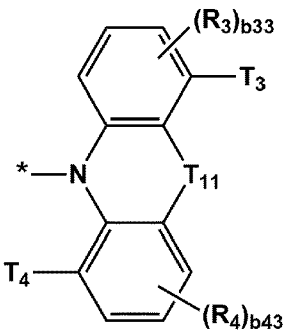

3-9

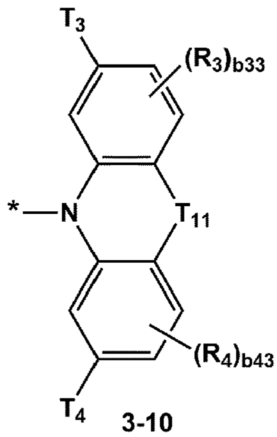

3-10

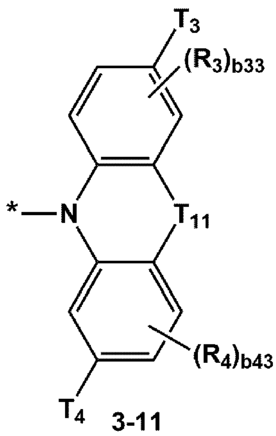

3-11

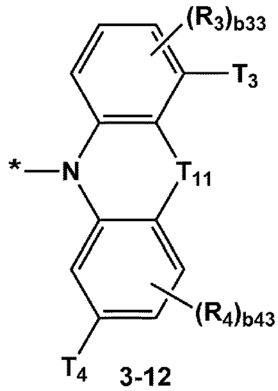

3-12

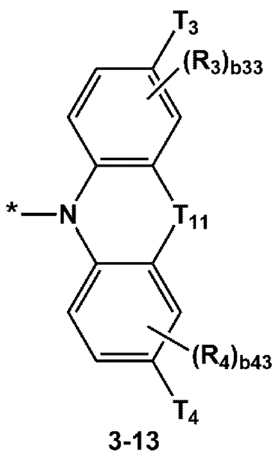

3-13

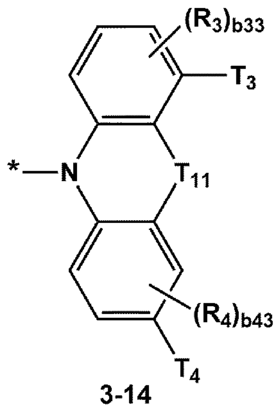

3-14

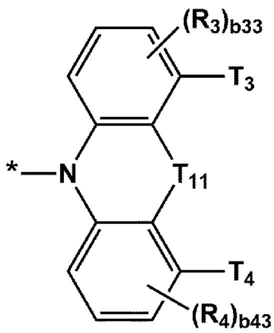

3-15

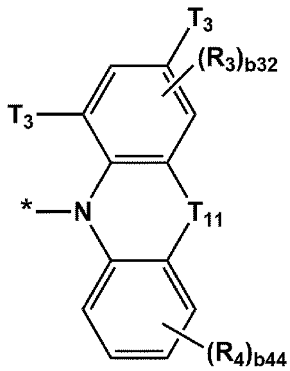

3-16

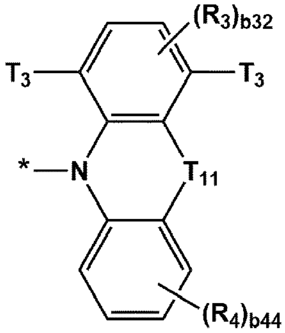

3-17

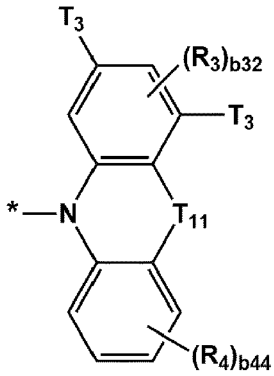

3-18

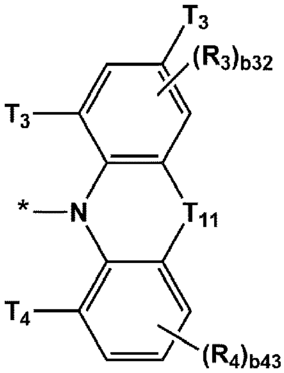

3-19

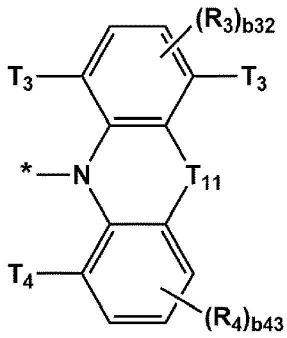

3-20

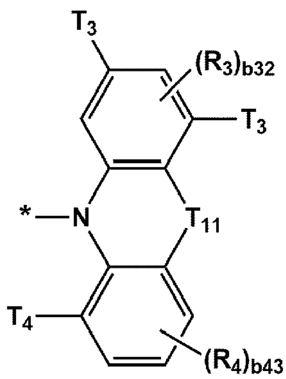

3-21

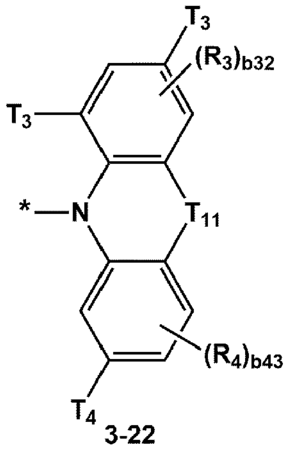

3-22

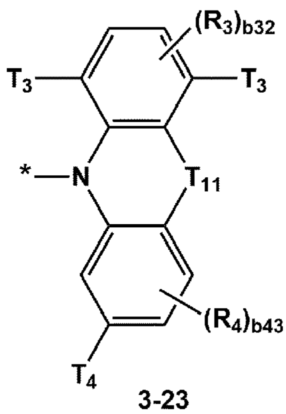

3-23

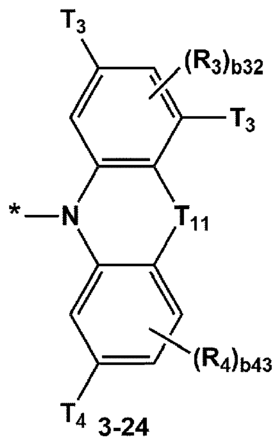

3-24

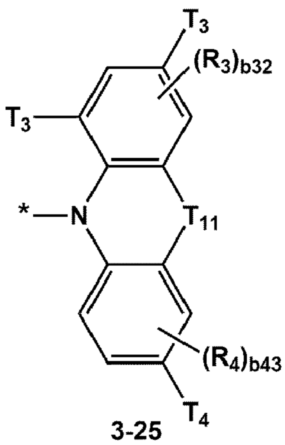

3-25

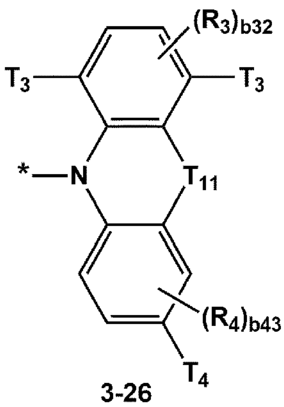

3-26

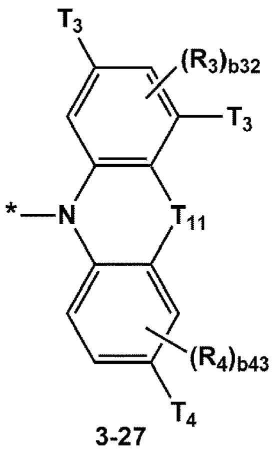

3-27

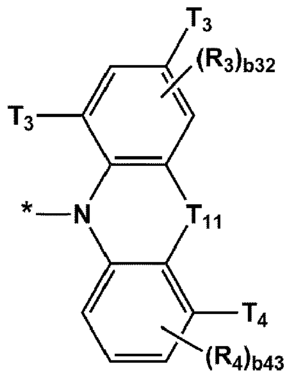

3-28

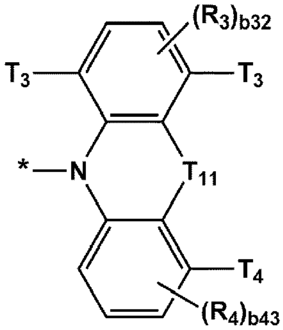
3-29

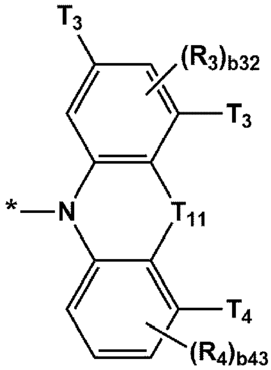
3-30

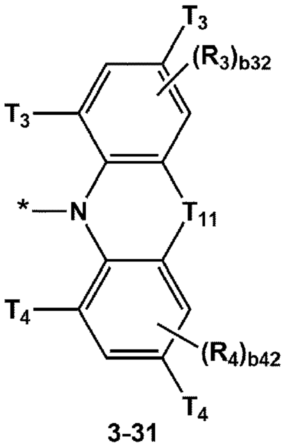
3-31

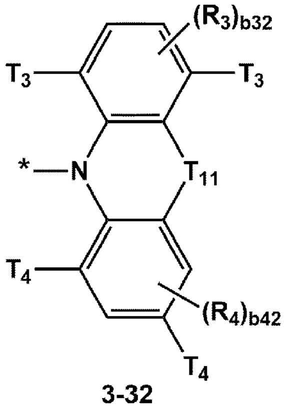
3-32

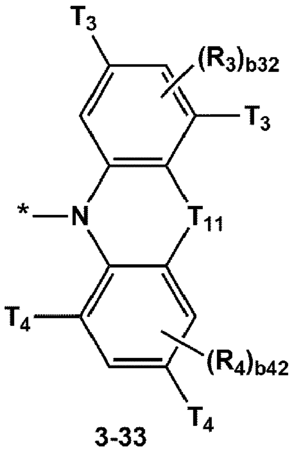
3-33

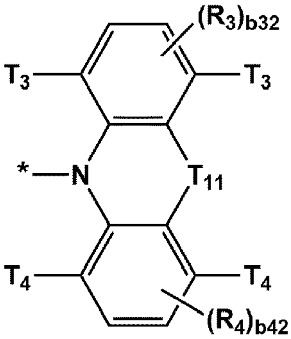
3-34

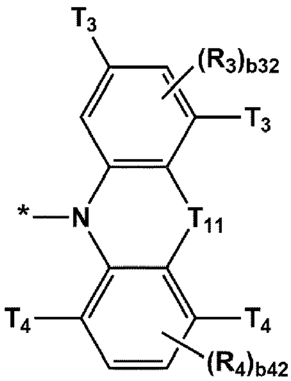
3-35

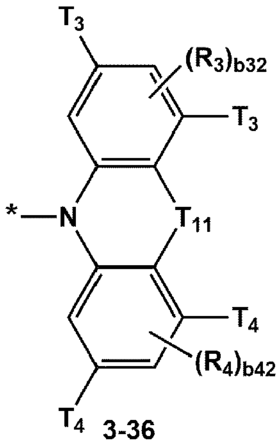
3-36

wherein, in Formulae 2-1 to 2-42 and 3-1 to 3-36,
$T_3$, $T_4$, $T_{11}$, $R_3$, and $R_4$ are as described in claim 1,
b32 and b42 are each independently an integer from 0 to 2,
b33 and b43 are each independently an integer from 0 to 3,
b34 and b44 are each independently an integer from 0 to 4,
b35 and b45 are each independently an integer from 0 to 5, and
* indicates a binding site to a neighboring atom.

**5.** The composition of any of claims 1-4, wherein the second compound is at least one of Compounds FD1 to FD12:

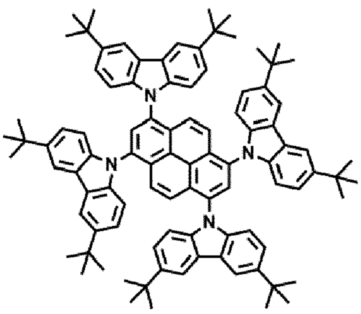
FD1

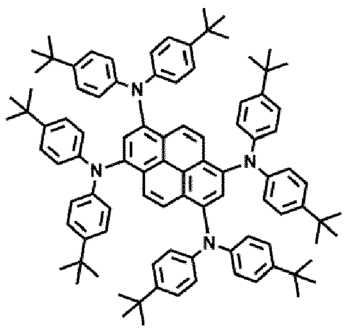
FD2

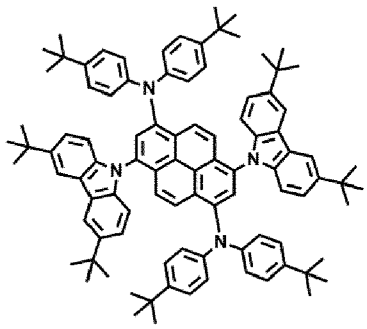
FD3

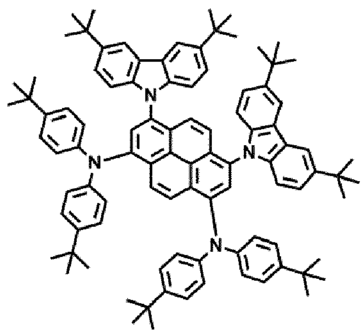
FD4

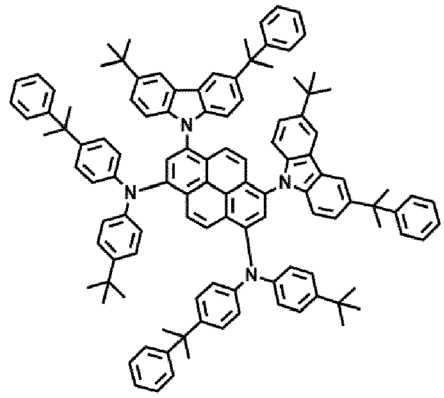
FD5

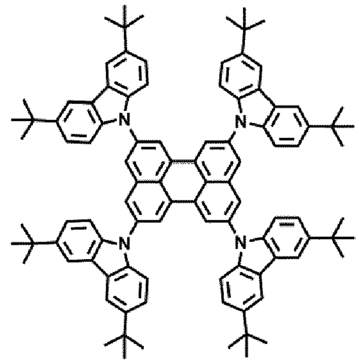
FD6

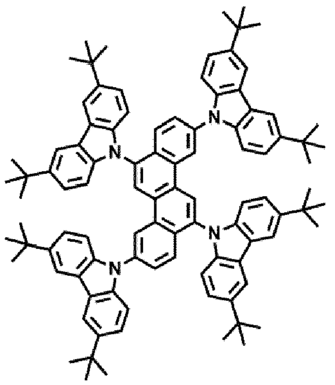
FD7

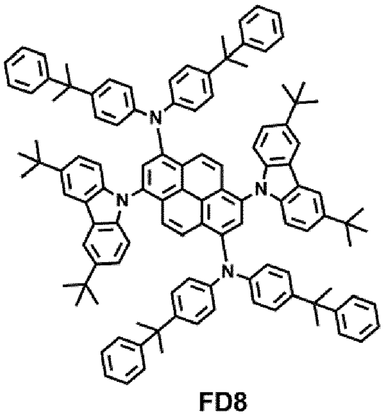
FD8

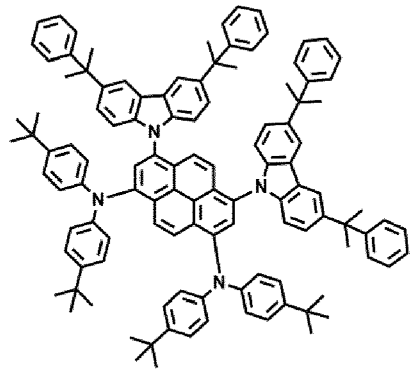
FD9

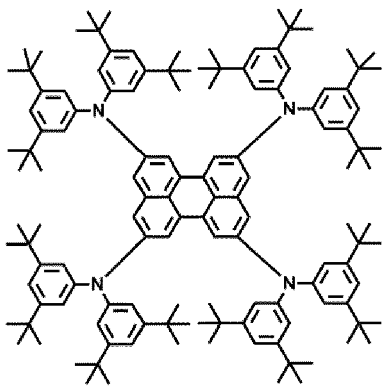
FD10

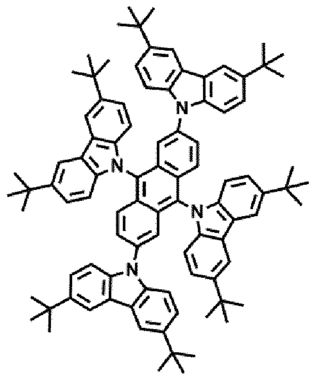
FD11

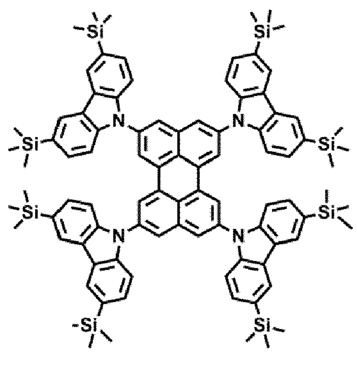
FD12

.

**6.** The composition of any of claims 1-5, wherein the first compound comprises an organometallic compound represented by Formula 101:

Formula 101 $M_{11}(L_{11})n_{11}(L_{12})_{n12}$

wherein, in Formula 101,

$M_{11}$ is a first-row transition metal of the Periodic Table of Elements, a second-row transition metal of the Periodic Table of Elements, or a third-row transition metal of the Periodic Table of Elements;
$L_{11}$ is a ligand represented by one of Formulae 101-1 to 101-4;
$L_{12}$ is a monodentate ligand and a bidentate ligand;

n11 is 1,
n12 is 0, 1, or 2;

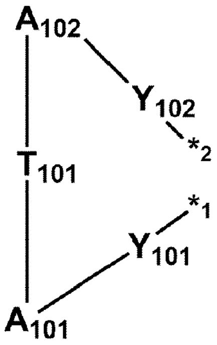

101-1

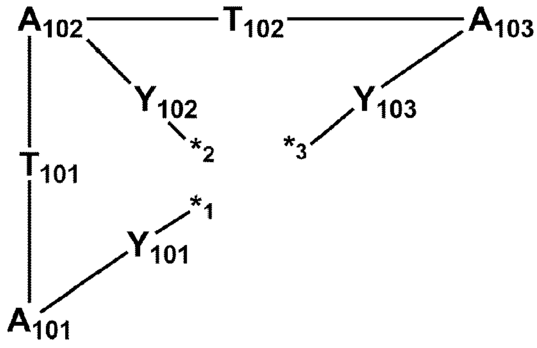

101-2

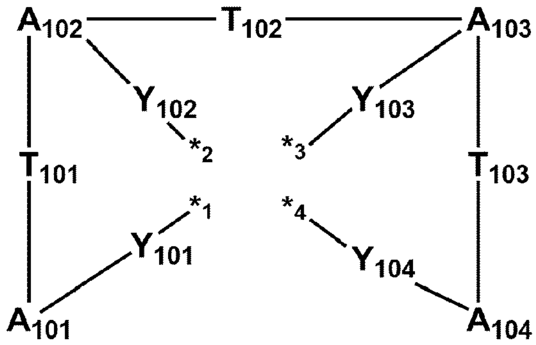

101-3

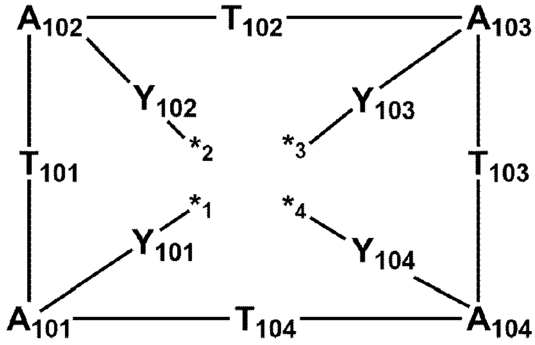

101-4

wherein, in Formulae 101-1 to 101-4,
$A_{101}$ to $A_{104}$ are each independently a substituted or unsubstituted $C_5$-$C_{30}$ carbocyclic group, a substituted or unsubstituted $C_1$-$C_{30}$ heterocyclic group, or a noncyclic group,
$Y_{101}$ to $Y_{104}$ are each independently a chemical bond, O, S, $N(R_{91})$, $B(R_{91})$, $P(R_{91})$, or $C(R_{91})(R_{92})$,
$T_{101}$ to $T_{104}$ are each independently a single bond, a double bond, $N(R_{93})$', $B(R_{93})$, $P(R_{93})$, $C(R_{93})(R_{94})$, $Si(R_{93})(R_{94})$, $Ge(R_{93})(R_{94})$, S, Se, O-, C(=O), S(=O), $S(=O)_2$, $-C(R_{93})=$, $=C(R_{93})-$, $C(R_{93})=C(R_{94})$, C(=S), or C=C, a substituent of the substituted $C_5$-$C_{30}$ carbocyclic group, a substituent of substituted $C_1$-$C_{30}$ heterocyclic group, and $R_{91}$ to $R_{94}$ are each independently hydrogen, deuterium, -F, -Cl, -Br, -I, $-SF_5$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkyl heteroaryl group, a substituted or unsubstituted $C_2$-$C_{60}$ heteroaryl alkyl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, $-C(=O)(Q_{41})$, $-S(=O)(Q_{41})$, $-S(=O)_2(Q_{41})$, $-N(Q_{42})(Q_{43})$, $-B(Q_{42})(Q_{43})$, - $Si(Q_{44})(Q_{45})(Q_{46})$, $-Ge(Q_{44})(Q_{45})(Q_{46})$, $-P(=O)(Q_{47})(Q_{48})$, -P(=S)(Q47)(Q48), or - $P(Q_{47})(Q_{48})$, wherein each of the substituent of the substituted $C_5$-$C_{30}$ carbocyclic group and the substituent of substituted $C_1$-$C_{30}$ heterocyclic group is not hydrogen,

$*_1$, $*_2$, $*_3$, and $*_4$ each indicate a binding site to $M_{11}$, and
$Q_{41}$ to $Q_{48}$ are each independently hydrogen; deuterium; -F; -Cl; -Br; -I; a hydroxyl group; a cyano group; a nitro group; an amidino group; a hydrazine group; a hydrazone group; a $C_1$-$C_{60}$ alkyl group; a $C_2$-$C_{60}$ alkenyl group; a $C_2$-$C_{60}$ alkynyl group; a $C_1$-$C_{60}$ alkoxy group; a $C_1$-$C_{60}$ alkylthio group; a $C_7$-$C_{60}$ aryl alkyl group; a $C_3$-$C_{10}$ cycloalkyl group; a $C_1$-$C_{10}$ heterocycloalkyl group; a $C_3$-$C_{10}$ cycloalkenyl group; a $C_1$-$C_{10}$ heterocycloalkenyl group; a $C_6$-$C_{60}$ aryl group; a $C_7$-$C_{60}$ alkyl aryl group; a $C_6$-$C_{60}$ aryloxy group; a $C_6$-$C_{60}$ arylthio group; a $C_1$-$C_{60}$ heteroaryl group; a $C_2$-$C_{60}$ alkyl heteroaryl group; a $C_2$-$C_{60}$ heteroaryl alkyl group; a $C_1$-$C_{60}$ heteroaryloxy group; a $C_1$-$C_{60}$ heteroarylthio group; a monovalent non-aromatic condensed polycyclic group; a monovalent non-aromatic condensed heteropolycyclic group; a $C_1$-$C_{60}$ alkyl group substituted with at least one of deuterium, -F, a cyano group, a $C_1$-$C_{60}$ alkyl group, or a $C_6$-$C_{60}$ aryl group; or a $C_6$-$C_{60}$ aryl group substituted with at least one of deuterium, -F, a cyano group, a $C_1$-$C_{60}$ alkyl group, or a $C_6$-$C_{60}$ aryl group.

**7.** The composition of any of claims 1-6, wherein the first compound includes a thermally activated delayed fluorescence emitter represented by one of Formula 201 or 202:

Formula 201

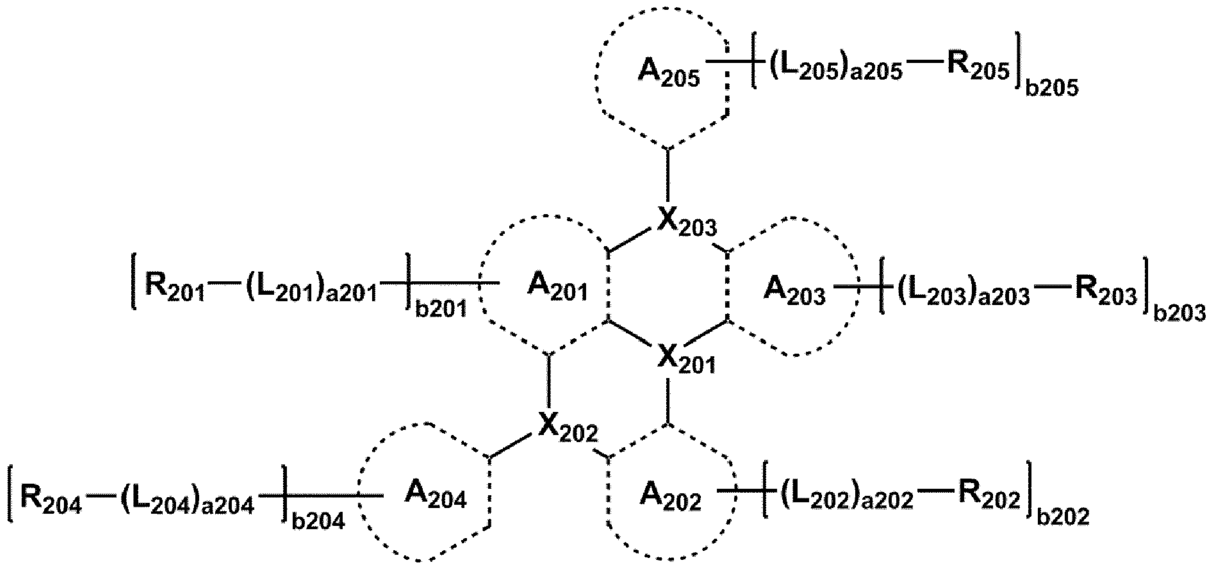

wherein, in Formula 201,

$X_{201}$ to $X_{203}$ are each independently B or N,
$A_{201}$ to $A_{205}$ are each independently a substituted or unsubstituted $C_5$-$C_{30}$ carbocyclic group or a substituted or unsubstituted $C_1$-$C_{30}$ heterocyclic group,
$L_{201}$ to $L_{205}$ are each independently a single bond, a $C_5$-$C_{30}$ carbocyclic group that is unsubstituted or substituted with at least one $R_{200a}$, or a $C_1$-$C_{30}$ heterocyclic group that is unsubstituted or substituted with at least one $R_{200a}$,
a201 to a205 are each independently an integer from 1 to 5,
$R_{201}$ to $R_{205}$ and $R_{200a}$ are each independently hydrogen, deuterium, -F, -Cl, - Br, -I, $-SF_5$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkyl heteroaryl group, a substituted or unsubstituted $C_2$-$C_{60}$ heteroaryl alkyl group, a substituted or unsubstituted $C_1$-$C_{60}$

heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -N($Q_{51}$)($Q_{52}$), - Si(Q53)(Q54)(Q55), -Ge($Q_{53}$)($Q_{54}$)($Q_{55}$), -B(Q56)(Q57), -P(=O)($Q_{58}$)($Q_{59}$), - P(=S)($Q_{58}$)($Q_{59}$), or -P($Q_{58}$)($Q_{59}$),

b201 to b205 are each independently an integer from 0 to 10, wherein, when b201 is 2 or more, two or more of $R_{201}$(s) are identical to or different from each other, when b202 is 2 or more, two or more of $R_{202}$(s) are identical to or different from each other, when b203 is 2 or more, two or more of $R_{203}$(s) are identical to or different from each other, when b204 is 2 or more, two or more of $R_{204}$(s) are identical to or different from each other, and when b205 is 2 or more, two or more of $R_{205}$(s) are identical to or different from each other,

Formula 202

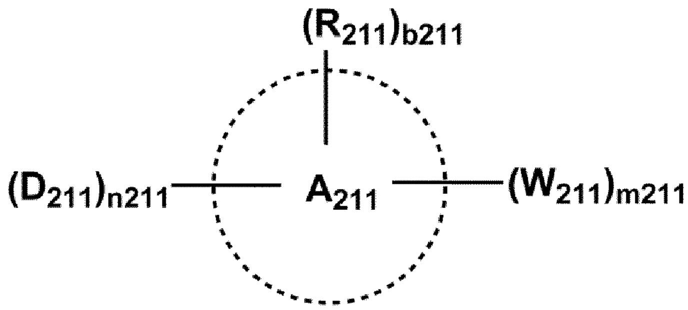

wherein, in Formula 202,

$A_{211}$ is a substituted or unsubstituted $C_5$-$C_{30}$ carbocyclic group or a substituted or unsubstituted $C_1$-$C_{30}$ heterocyclic group,

W211 is an acceptor group,

D211 is a donor group,

m211 is an integer from 1 to 4, and n211 is an integer from 1 to 4;

$R_{211}$ is hydrogen, deuterium, -F, -Cl, -Br, -I, -$SF_5$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_1$-$C_{60}$ alkylthio group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_7$-$C_{60}$ alkyl aryl group, a substituted or unsubstituted $C_7$-$C_{60}$ aryl alkyl group, a substituted or unsubstituted $C_6$-$C_{60}$ aryloxy group, a substituted or unsubstituted $C_6$-$C_{60}$ arylthio group, a substituted or unsubstituted $C_1$-$C_{60}$ heteroaryl group, a substituted or unsubstituted $C_2$-$C_{60}$ alkyl heteroaryl group, a substituted or unsubstituted $C_2$-$C_{60}$ heteroaryl alkyl group, a substituted or unsubstituted $C_1$-$C_{60}$ heteroaryloxy group, a substituted or unsubstituted $C_1$-$C_{60}$ heteroarylthio group, a substituted or unsubstituted monovalent non-aromatic condensed polycyclic group, a substituted or unsubstituted monovalent non-aromatic condensed heteropolycyclic group, -N($Q_{51}$)($Q_{52}$), -Si($Q_{53}$)($Q_{54}$)($Q_{55}$), - Ge(Q53)(Q54)(Q55), -B(Q56)(Q57), -P(=O)($Q_{58}$)($Q_{59}$), -P(=S)($Q_{58}$)($Q_{59}$), or -P($Q_{58}$)($Q_{59}$), and a plurality of $R_{211}$(s) are optionally bonded to form a substituted or unsubstituted $C_5$-$C_{30}$ carbocyclic group or a substituted or unsubstituted $C_1$-$C_{30}$ heterocyclic group,

at least one substituent of the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_1$-$C_{60}$ alkylthio group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_7$-$C_{60}$ alkyl aryl group, the substituted $C_7$-$C_{60}$ aryl alkyl group, the substituted $C_6$-$C_{60}$ aryloxy group, the substituted $C_6$-$C_{60}$ arylthio group, the substituted $C_1$-$C_{60}$ heteroaryl group, the substituted $C_2$-$C_{60}$ alkyl heteroaryl group, the substituted $C_2$-$C_{60}$ heteroaryl alkyl group, the substituted $C_1$-$C_{60}$ heteroaryloxy group, the substituted $C_1$-$C_{60}$ heteroarylthio group, the substituted monovalent non-aromatic condensed polycyclic group, and the substituted monovalent non-aromatic condensed heteropolycyclic group is:

deuterium, -F, -Cl, -Br, -I, -$CD_3$, -$CD_2H$, -$CDH_2$, -$CF_3$, -$CF_2H$, -$CFH_2$, a hydroxyl group, a cyano group, a nitro group, an amino group, an amidino group, a hydrazine group, a hydrazone group, a carboxylic acid

group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;

a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10\ h}$eterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, $-N(Q_{61})(Q_{62})$, $-Si(Q_{63})(Q_{64})(Q_{65})$, - Ge(Q63)(Q64)(Q65), -B(Q66)(Q67), $-P(=O)(Q_{68})(Q_{69})$, $-P(=S)(Q_{68})(Q_{69})$, $-P(Q_{68})(Q_{69})$, or a combination thereof;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_1$-$C_{60}$ heteroaryl group, a $C_1$-$C_{60}$ heteroaryloxy group, a $C_1$-$C_{60}$ heteroarylthio group, a monovalent non-aromatic condensed polycyclic group, or a monovalent non-aromatic condensed heteropolycyclic group, each unsubstituted or substituted with at least one of deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_1$-$C_{60}$ alkylthio group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_7$-$C_{60}$ alky aryl group, a $C_7$-$C_{60}$ aryl alkyl group, a $C_6$-$C_{60}$ aryloxy group, a $C_6$-$C_{60}$ arylthio group, a $C_1$-$C_{60}$ heteroaryl group, a $C_2$-$C_{60}$ alkyl heteroaryl group, a $C_2$-$C_{60}$ heteroaryl alkyl group, a $C_1$-$C_{60}$ heteroaryloxy group, a $C_1$-$C_{60}$ heteroarylthio group, a monovalent non-aromatic condensed polycyclic group, a monovalent non-aromatic condensed heteropolycyclic group, $-N(Q_{71})(Q_{72})$, $-Si(Q_{73})(Q_{74})(Q_{75})$, $-Ge(Q_{73})(Q_{74})(Q_{75})$, -B(Q76)(Q77), - $P(=O)(Q_{78})(Q_{79})$, $-P(=S)(Q_{78})(Q_{79})$, $-P(Q_{78})(Q_{79})$, or a combination thereof;

$-N(Q_{81})(Q_{82})$, $-Si(Q_{83})(Q_{84})(Q_{85})$, $-Ge(Q_{83})(Q_{84})(Q_{85})$, -B(Q86)(Q87), - $P(=O)(Q_{88})(Q_{89})$, $-P(=O)(Q_{78})(Q_{79})$, or $-P(Q_{88})(Q_{89})$; or

a combination thereof,

wherein $Q_{51}$ to $Q_{59}$, $Q_{61}$ to $Q_{69}$, $Q_{71}$ to $Q_{79}$, and $Q_{81}$ to $Q_{89}$ are each independently: hydrogen; deuterium; -F; -Cl; -Br; -I; a hydroxyl group; a cyano group; a nitro group; an amidino group; a hydrazine group; a hydrazone group; a carboxylic acid group or a salt thereof; a sulfonic acid group or a salt thereof; a phosphoric acid group or a salt thereof; a $C_1$-$C_{60}$ alkyl group which is unsubstituted or substituted with at least one of deuterium, a $C_1$-$C_{60}$ alkyl group, a $C_6$-$C_{60}$ aryl group, or a combination thereof; a $C_2$-$C_{60}$ alkenyl group; a $C_2$-$C_{60}$ alkynyl group; a $C_1$-$C_{60}$ alkoxy group; a $C_1$-$C_{60}$ alkylthio group; a $C_3$-$C_{10}$ cycloalkyl group; a $C_1$-$C_{10}$ heterocycloalkyl group; a $C_3$-$C_{10}$ cycloalkenyl group; a $C_1$-$C_{10}$ heterocycloalkenyl group; a $C_6$-$C_{60}$ aryl group which is unsubstituted or substituted with at least one of deuterium, a $C_1$-$C_{60}$ alkyl group, a $C_6$-$C_{60}$ aryl group, or a combination thereof; a $C_6$-$C_{60}$ aryloxy group; a $C_6$-$C_{60}$ arylthio group; a $C_1$-$C_{60}$ heteroaryl group; a $C_2$-$C_{60}$ alkyl heteroaryl group; a $C_2$-$C_{60}$ heteroaryl alkyl group; a $C_1$-$C_{60}$ heteroaryloxy group; a $C_1$-$C_{60}$ heteroarylthio group; a monovalent non-aromatic condensed polycyclic group; or a monovalent non-aromatic condensed heteropolycyclic group;

preferably wherein $W_{211}$ is a substituted or unsubstituted $\pi$ electron-deficient nitrogen-free cyclic group, and $D_{211}$ is:

-F, a cyano group, or a $\pi$ electron-deficient nitrogen-containing cyclic group;

a $C_1$-$C_{60}$ alkyl group, an $\pi$-electron deficient nitrogen-containing cyclic group, or an $\pi$ electron-deficient nitrogen-free cyclic group, each substituted with at least one of -F or a cyano group; or

an $\pi$-electron deficient nitrogen-containing cyclic group substituted with at least one of deuterium, a $C_1$-$C_{60}$ alkyl group, an $\pi$ -electron deficient nitrogen-containing cyclic group, or an $\pi$ electron-deficient nitrogen-free cyclic group,

wherein the $\pi$ electron-deficient nitrogen-free cyclic group is a phenyl group, a heptalene group, an indene group, a naphthalene group, an azulene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, an isoindole group, an indole group, a furan group, a thiophene group, a benzofuran

group, a benzothiophene group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, a triindolobenzene group, or a condensed cyclic group of two or more π electron-deficient nitrogen-free cyclic groups, and
wherein the π electron-deficient nitrogen-containing cyclic group is a cyclic group having at least one -N= moiety, and is:

an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, an azacarbazole group, or a benzimidazolobenzimidazole group; or
a condensed cyclic group in which two or more π electron-efficient nitrogen-containing cyclic groups are condensed with each other.

**8.** The composition of any of claims 1-7, wherein the third compound comprises a bipolar compound, an electron-transporting compound, a hole-transporting compound, or a combination thereof,

the electron-transporting compound comprises at least one electron-transporting moiety,
the hole-transporting compound does not comprise the electron-transporting moiety, and
the electron-transporting moiety is a cyano group, a π electron-deficient nitrogen-containing cyclic group, or a group represented by one of the following Formulae:

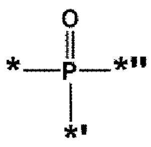 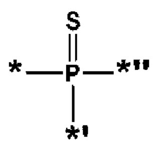 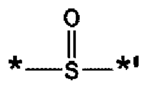 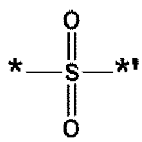

wherein *, *', and *" in the formulae above are each a binding site to a neighboring atom;
preferably wherein
the electron-transporting compound includes at least one π electron-deficient nitrogen-free cyclic group and at least one electron-transporting moiety,
the hole-transporting compound includes at least one π electron-deficient nitrogen-free cyclic group, and does not include an electron-transporting moiety, and
the electron-transporting moiety is a cyano group or a π electron-deficient nitrogen-containing cyclic group.

**9.** The composition of claim 8, wherein

the electron-transporting compound comprises i) at least one of a cyano group, a pyrimidine group, a pyrazine group, or a triazine group, or ii) a triphenylene group, and
the hole-transporting compound comprises a carbazole group.

**10.** The composition of any of claims 1-9, wherein the composition satisfies Condition 3:

$$\text{Condition 3} \qquad E_{HOMO_C1} - 0.4\ eV \leq E_{HOMO_C2} \leq E_{HOMO_C1} + 0.1\ eV$$

wherein, in Condition 3, $E_{HOMO_C1}$ is a highest occupied molecular orbital (HOMO) energy level value of the first compound, $E_{HOMO_C2}$ is a HOMO energy level value of the second compound, and eV is electron volts; and/or wherein the composition satisfies Condition 4:

Condition 4

$$R(HOD)_H / R(HOD)_0 \leq 1.07$$

wherein, in Condition 4,

$R(HOD)_H$ is a relative HOD value of the composition comprising the first compound, the second compound, and the third compound, and
$R(HOD)_0$ is a relative HOD value of a composition comprising the first compound and the third compound.

**11.** An organic light-emitting device comprising:

a first electrode;
a second electrode; and
an organic layer located between the first electrode and the second electrode,
wherein the organic layer comprises an emission layer, and

wherein the organic layer comprises the composition of any of claims 1-10.

**12.** The organic light-emitting device of claim 11, wherein the emission layer comprises the composition;

preferably wherein the emission layer comprises a sensitizer, a dopant, and a host,
the sensitizer comprises the first compound of the composition,
the dopant comprises the second compound of the composition, and
the host comprises the third compound of the composition.

**13.** The organic light-emitting device of claim 12, wherein the emission layer emits blue light.

**14.** An organic light-emitting device, comprising:

a first electrode;
a second electrode;
m light-emitting units located between the first electrode and the second electrode and comprising at least one emission layer; and
m-1 charge generation layers located between neighboring two light-emitting units of the m light-emitting units, and comprising an n-type charge generation layer and a p-type charge generation layer,
wherein m is an integer of 2 or greater,
a maximum emission wavelength of light emitted from at least one light-emitting unit of the m light-emitting units is different from a maximum emission wavelength of light emitted from at least one light-emitting unit of the other light-emitting units of the m light-emitting units, and
the at least one emission layer comprises the composition of any of claims 1-10.

**15.** An organic light-emitting device, comprising:

a first electrode;
a second electrode; and
m emission layers located between the first electrode and the second electrode, wherein,
m is an integer of 2 or more,
a maximum emission wavelength of light emitted from at least one emission layer of the m emission layers is different from a maximum emission wavelength of light emitted from at least one emission layer of the other emission layers of the m emission layers, and
the at least one emission layer comprises the composition of any of claims 1-10.

FIG. 1

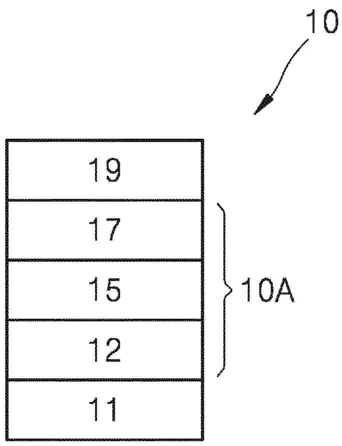


FIG. 2

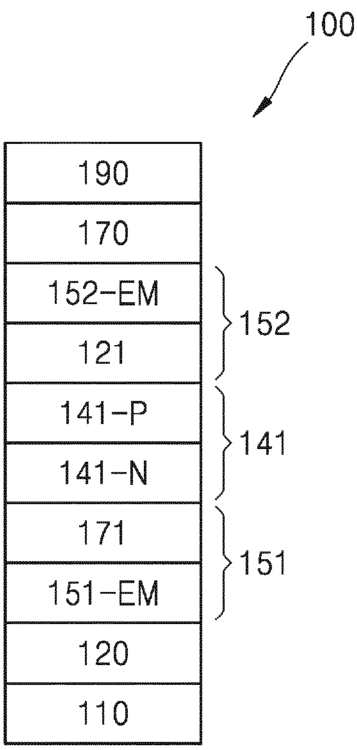


FIG. 3

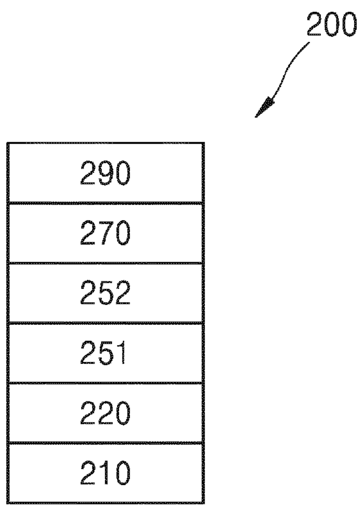

FIG. 4

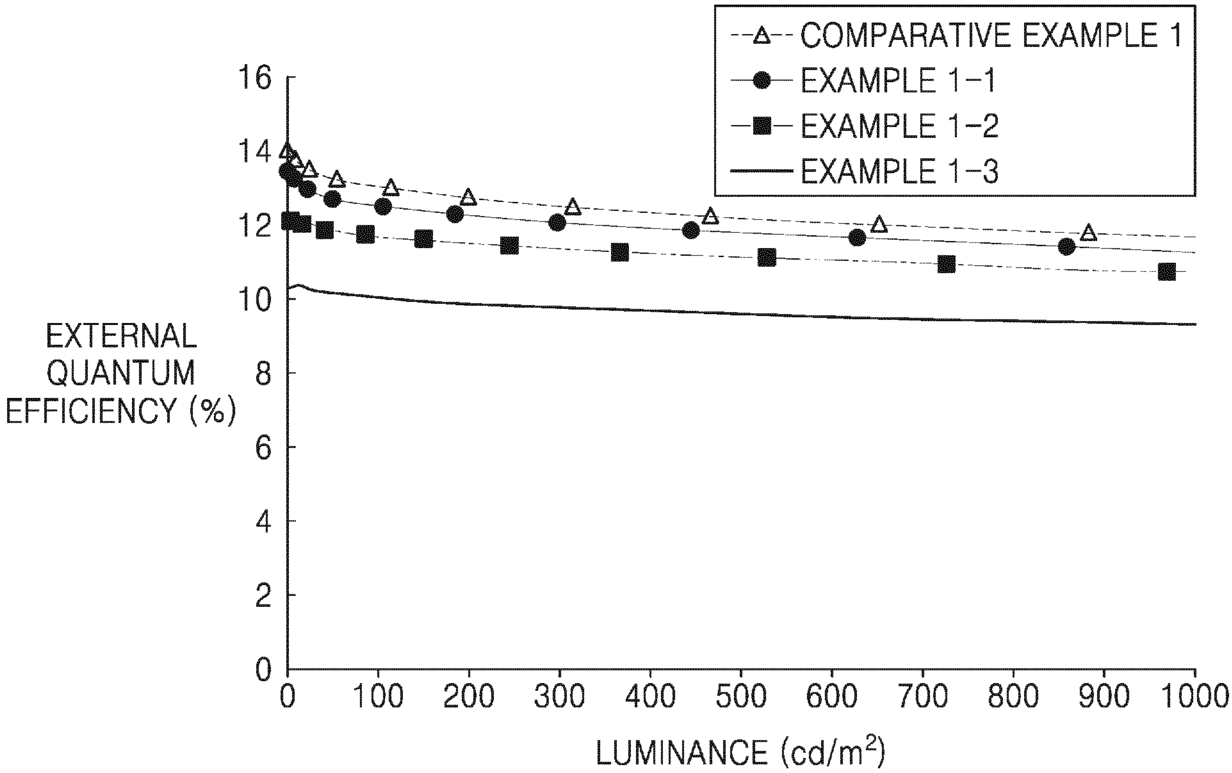
COMPARATIVE EXAMPLE 1
EXAMPLE 1-1
EXAMPLE 1-2
EXAMPLE 1-3
EXTERNAL QUANTUM EFFICIENCY (%)
LUMINANCE (cd/m2)
0
2
4
6
8
10
12
14
16
100
200
300
400
500
600
700
800
900
1000

FIG. 5

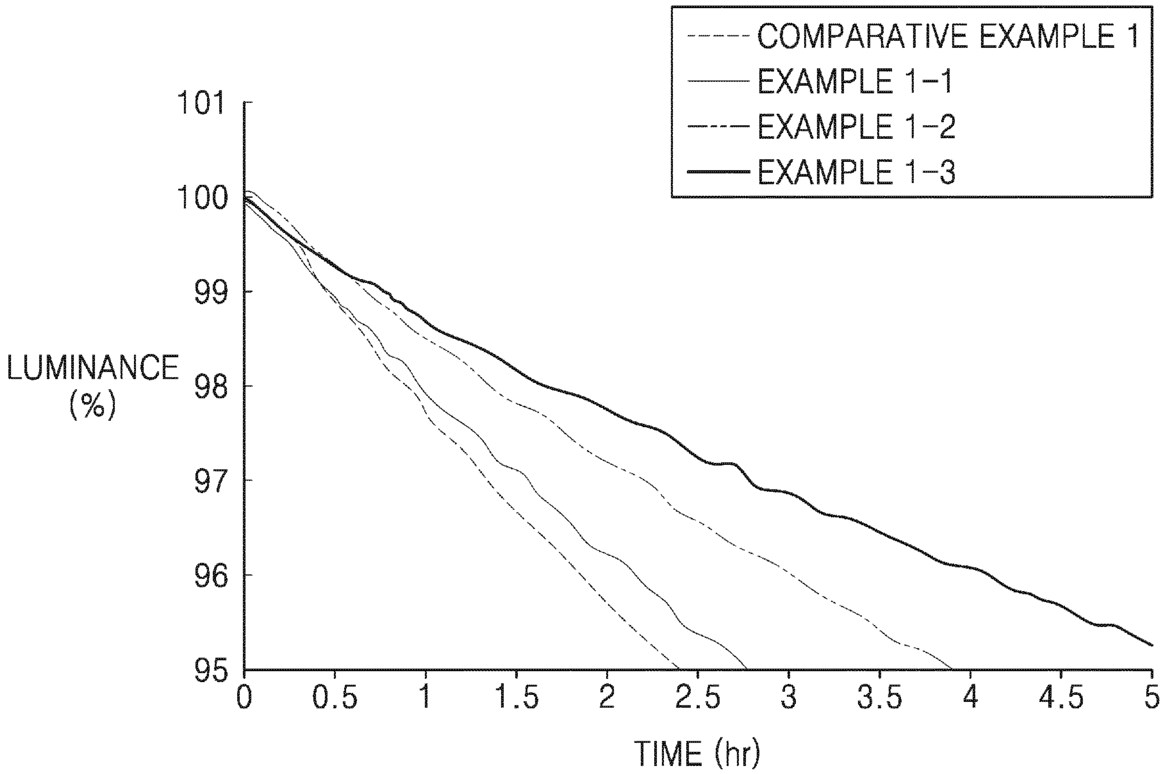
COMPARATIVE EXAMPLE 1
EXAMPLE 1-1
EXAMPLE 1-2
EXAMPLE 1-3
LUMINANCE (%)
TIME (hr)
95
96
97
98
99
100
101
0
0.5
1
1.5
2
2.5
3
3.5
4
4.5
5

FIG. 6

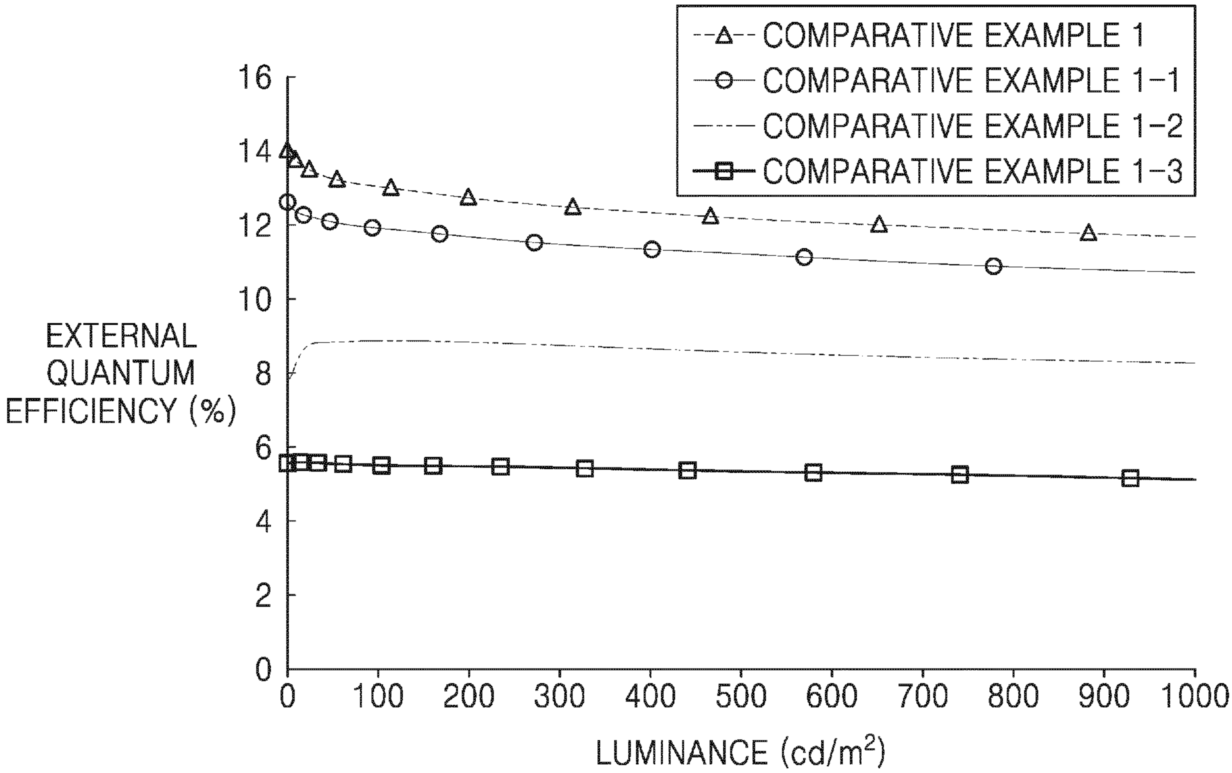
COMPARATIVE EXAMPLE 1
COMPARATIVE EXAMPLE 1-1
COMPARATIVE EXAMPLE 1-2
COMPARATIVE EXAMPLE 1-3
EXTERNAL QUANTUM EFFICIENCY (%)
0
2
4
6
8
10
12
14
16
0
100
200
300
400
500
600
700
800
900
1000
LUMINANCE (cd/m2)

FIG. 7

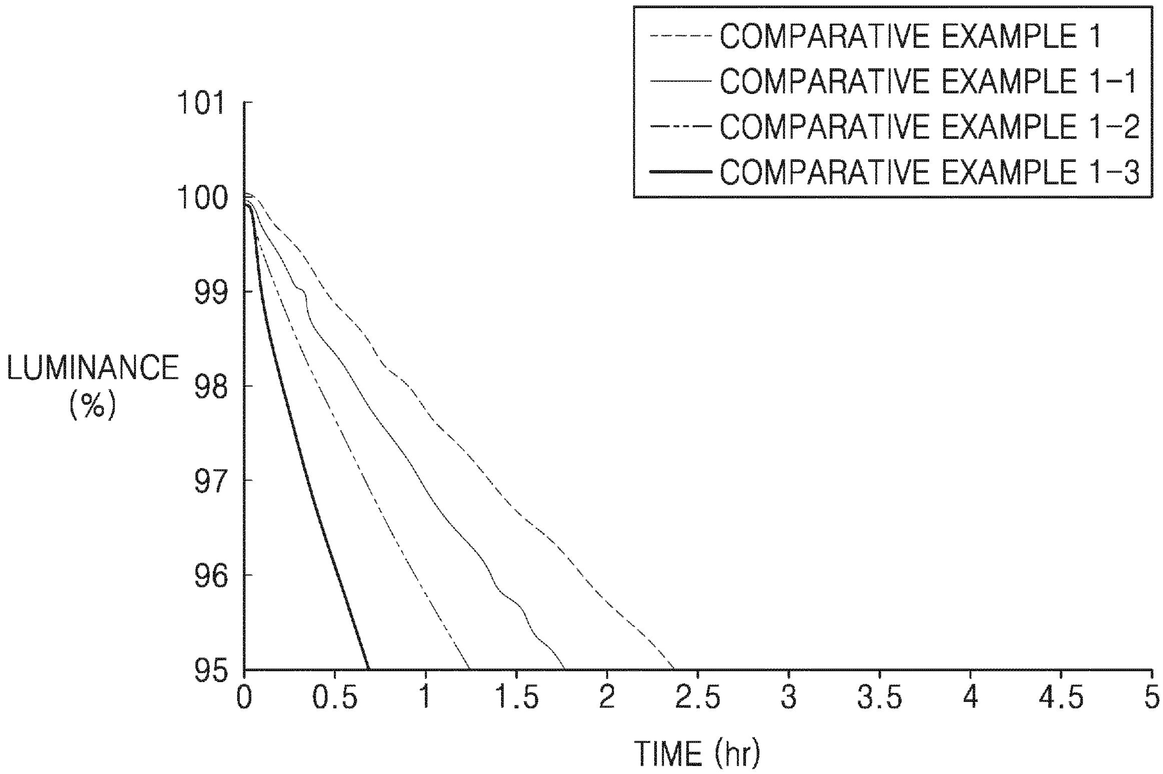
COMPARATIVE EXAMPLE 1
COMPARATIVE EXAMPLE 1-1
COMPARATIVE EXAMPLE 1-2
COMPARATIVE EXAMPLE 1-3
LUMINANCE (%)
95
96
97
98
99
100
101
0
0.5
1
1.5
2
2.5
3
3.5
4
4.5
5
TIME (hr)

FIG. 8

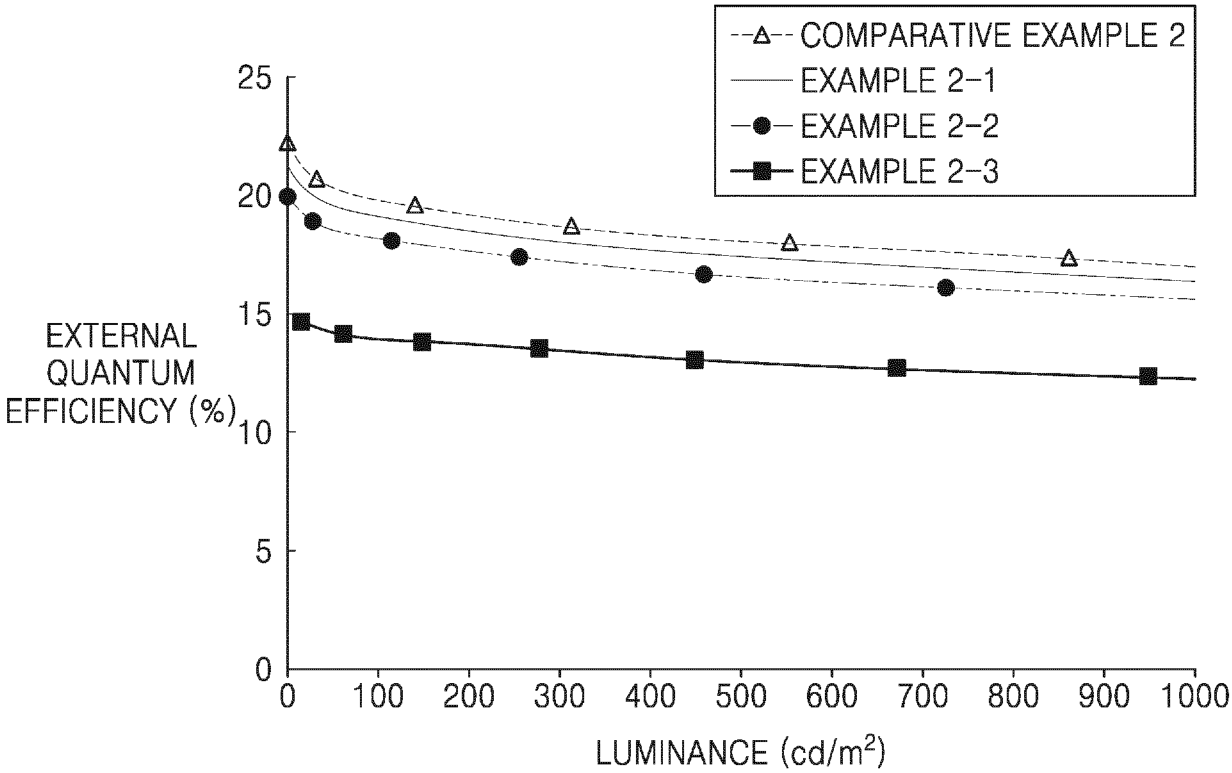
COMPARATIVE EXAMPLE 2
EXAMPLE 2-1
EXAMPLE 2-2
EXAMPLE 2-3
EXTERNAL QUANTUM EFFICIENCY (%)
LUMINANCE (cd/m2)
0
5
10
15
20
25
0
100
200
300
400
500
600
700
800
900
1000

FIG. 9

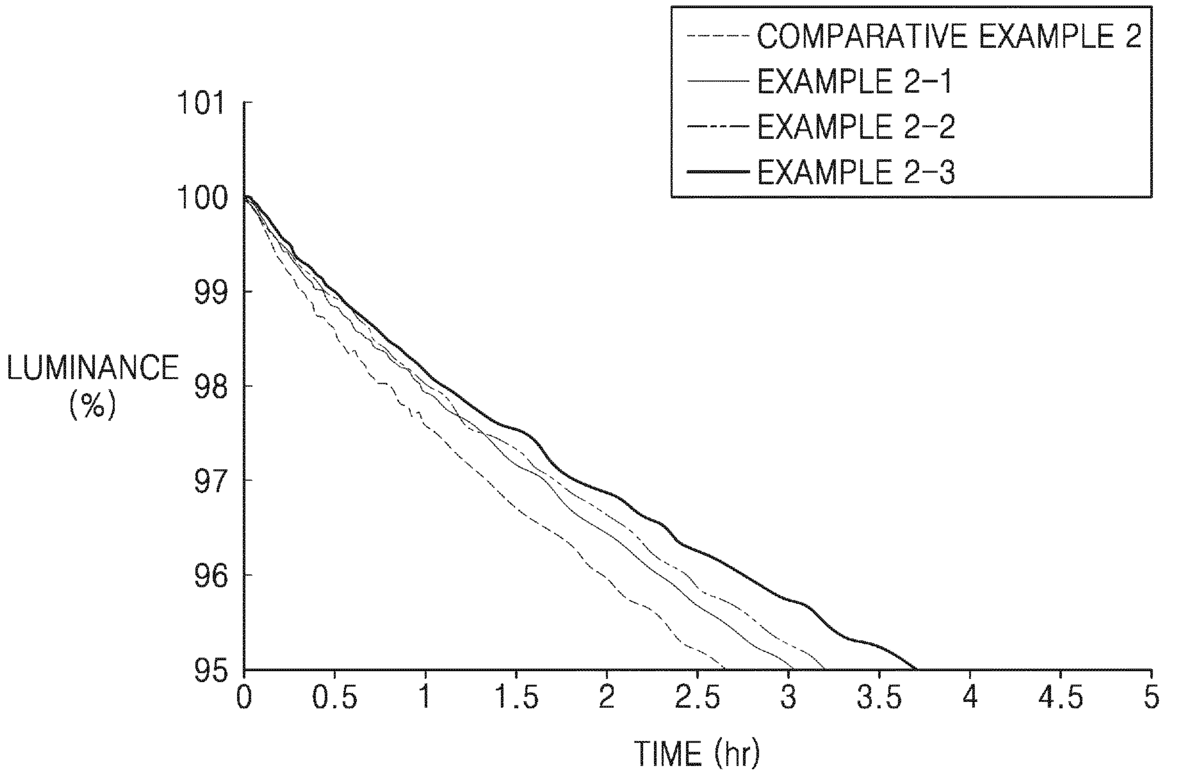
COMPARATIVE EXAMPLE 2
EXAMPLE 2-1
EXAMPLE 2-2
EXAMPLE 2-3
LUMINANCE (%)
TIME (hr)
95
96
97
98
99
100
101
0
0.5
1
1.5
2
2.5
3
3.5
4
4.5
5

FIG. 10

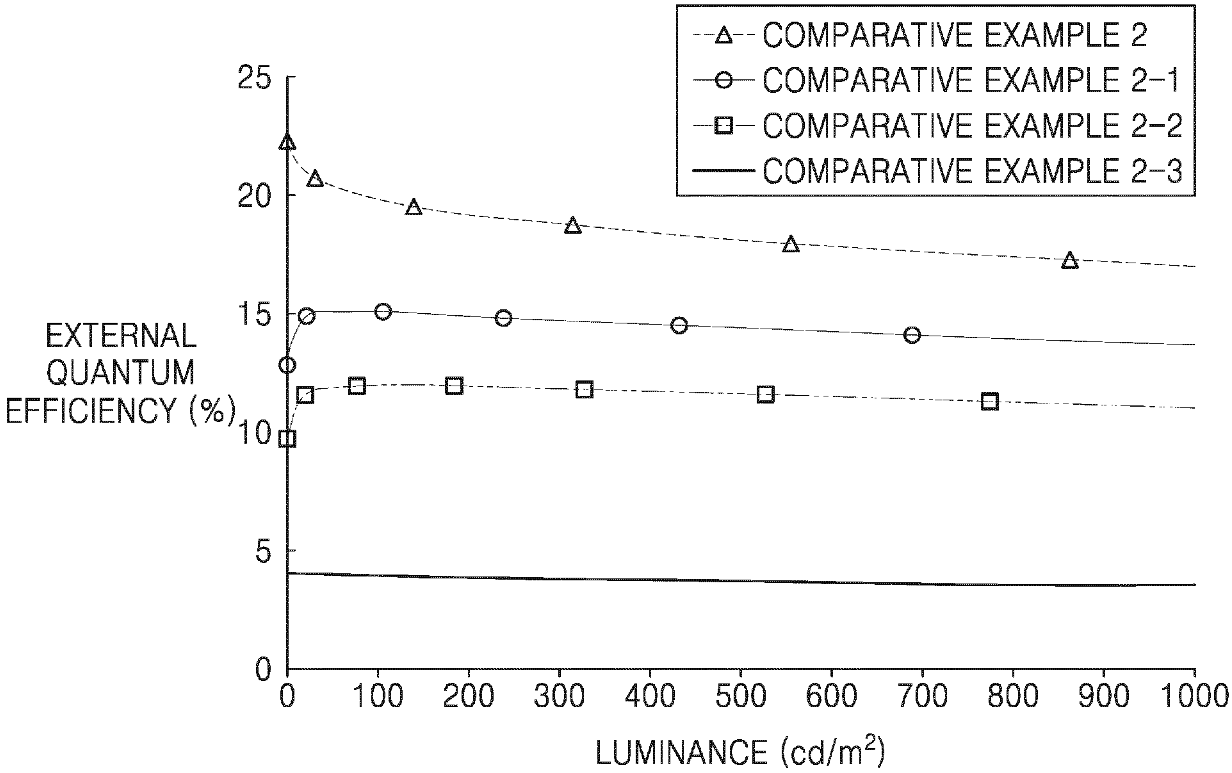
COMPARATIVE EXAMPLE 2
COMPARATIVE EXAMPLE 2-1
COMPARATIVE EXAMPLE 2-2
COMPARATIVE EXAMPLE 2-3
EXTERNAL QUANTUM EFFICIENCY (%)
25
20
15
10
5
0
0 100 200 300 400 500 600 700 800 900 1000
LUMINANCE (cd/m2)

FIG. 11

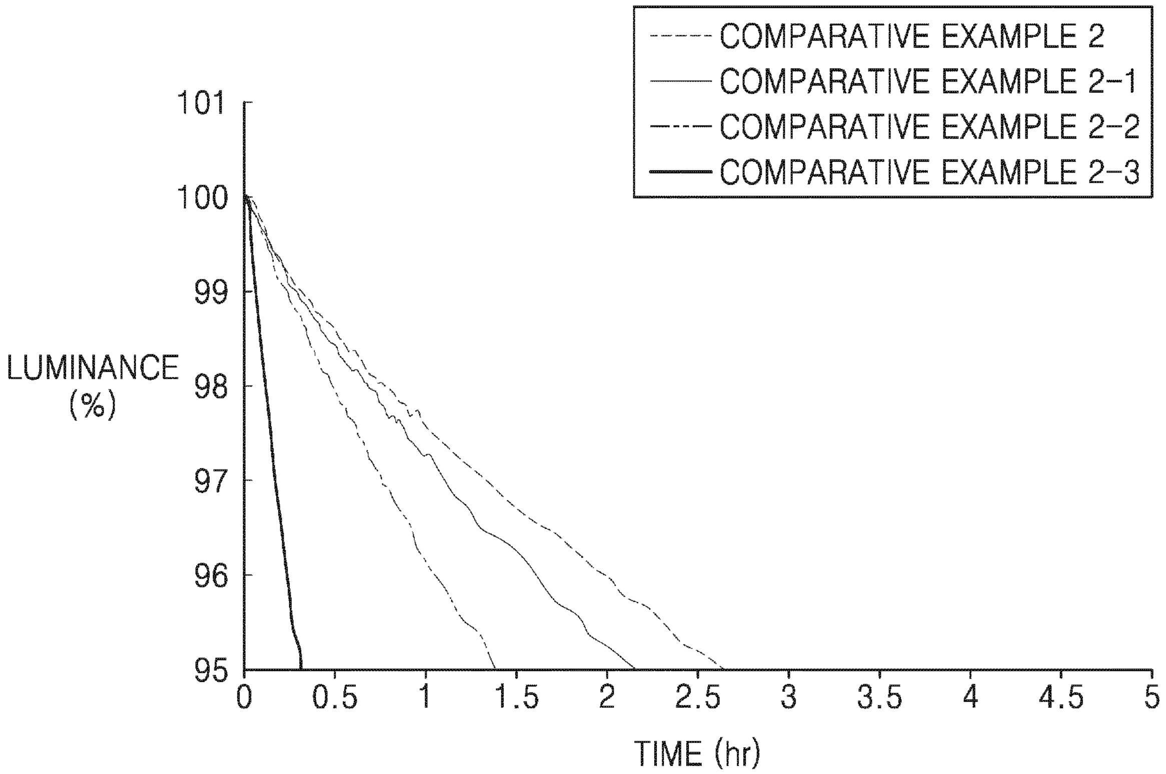
COMPARATIVE EXAMPLE 2
COMPARATIVE EXAMPLE 2-1
COMPARATIVE EXAMPLE 2-2
COMPARATIVE EXAMPLE 2-3
LUMINANCE (%)
101
100
99
98
97
96
95
0 0.5 1 1.5 2 2.5 3 3.5 4 4.5 5
TIME (hr)

FIG. 12

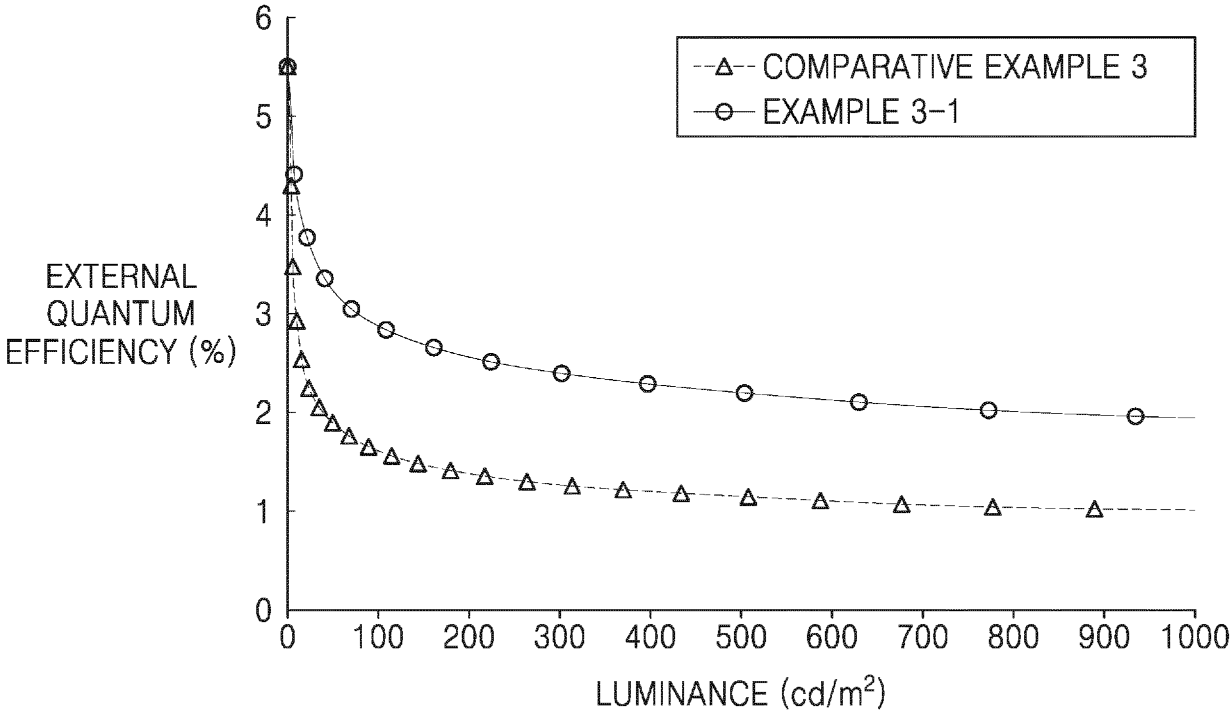
COMPARATIVE EXAMPLE 3
EXAMPLE 3-1
EXTERNAL QUANTUM EFFICIENCY (%)
LUMINANCE (cd/m²)
0
1
2
3
4
5
6
0
100
200
300
400
500
600
700
800
900
1000

FIG. 13

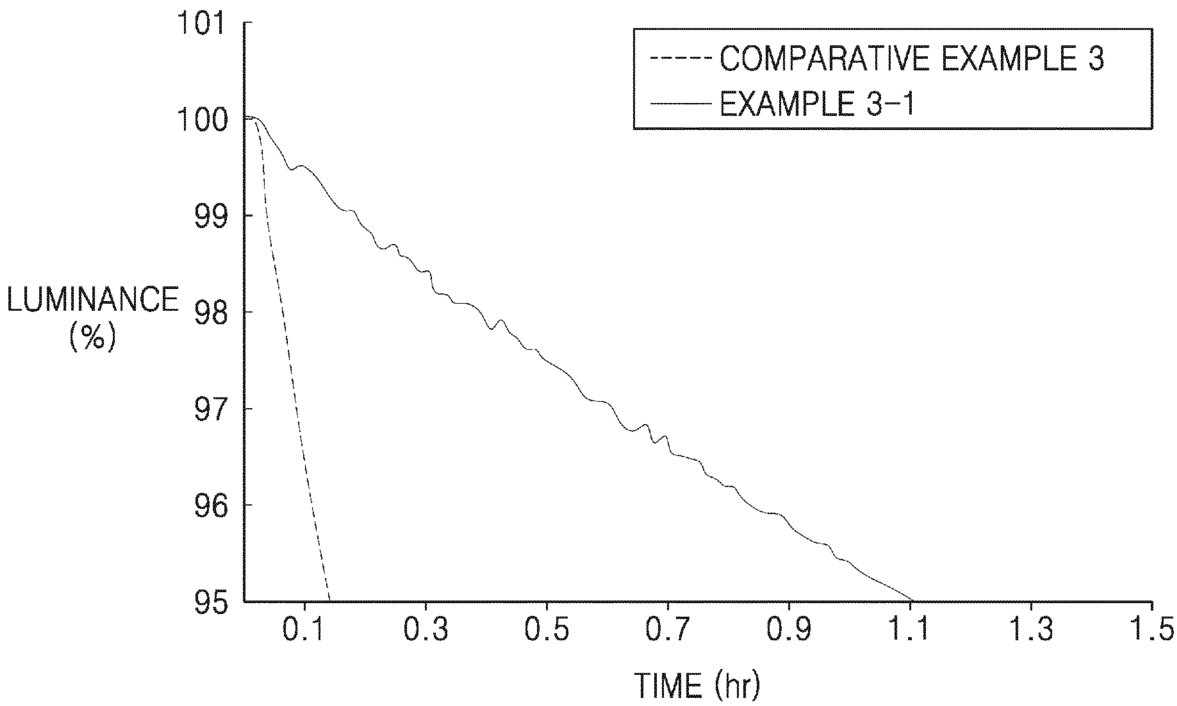
COMPARATIVE EXAMPLE 3
EXAMPLE 3-1
LUMINANCE (%)
TIME (hr)
95
96
97
98
99
100
101
0.1
0.3
0.5
0.7
0.9
1.1
1.3
1.5

FIG. 14

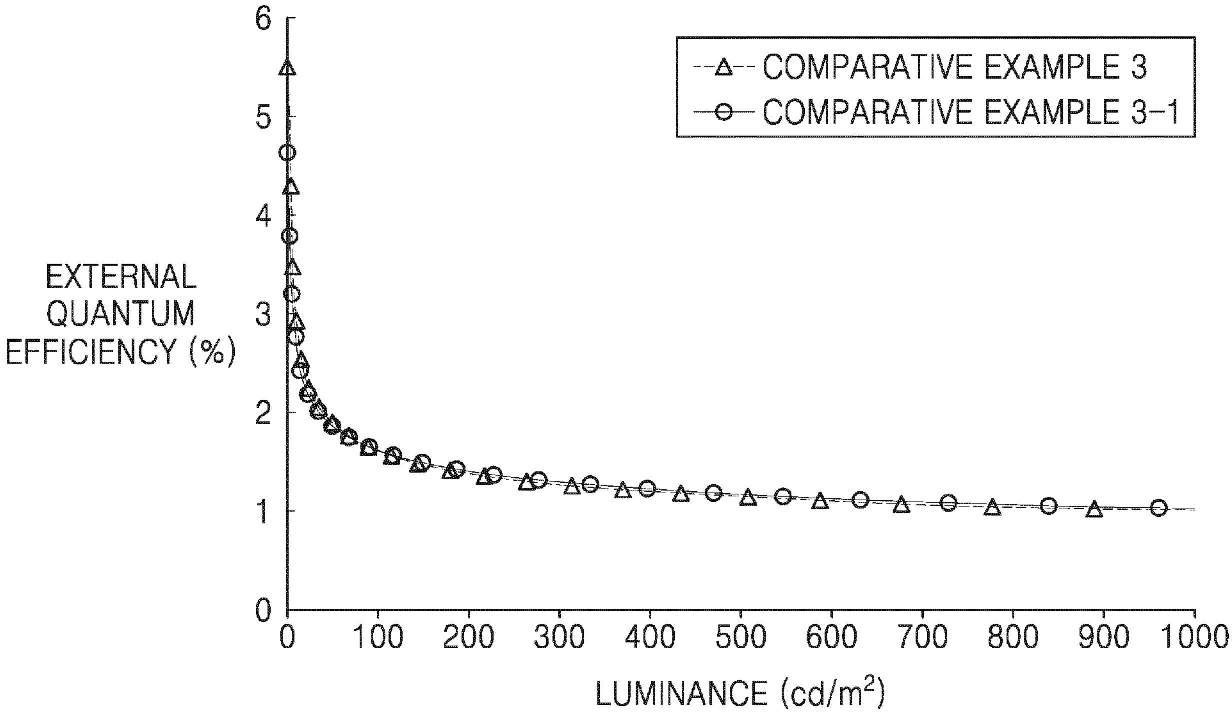
COMPARATIVE EXAMPLE 3
COMPARATIVE EXAMPLE 3-1
EXTERNAL QUANTUM EFFICIENCY (%)
LUMINANCE (cd/m2)
0 1 2 3 4 5 6
0 100 200 300 400 500 600 700 800 900 1000

FIG. 15

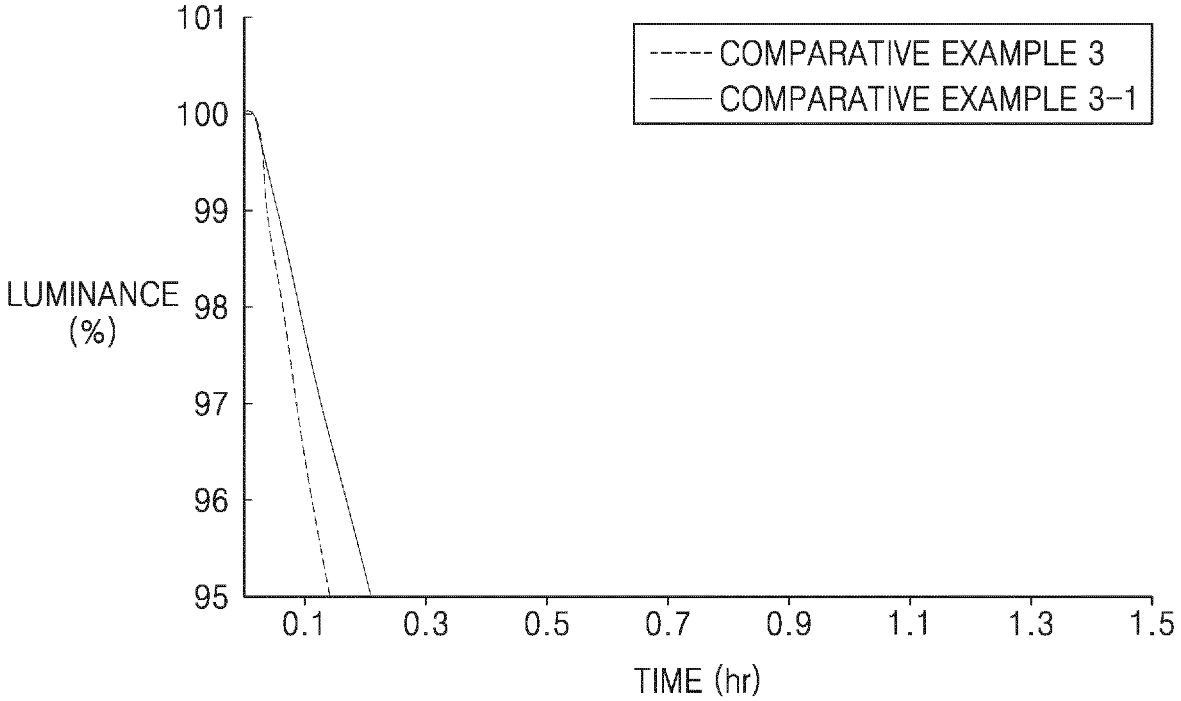
COMPARATIVE EXAMPLE 3
COMPARATIVE EXAMPLE 3-1
LUMINANCE (%)
TIME (hr)
95 96 97 98 99 100 101
0.1 0.3 0.5 0.7 0.9 1.1 1.3 1.5

FIG. 16

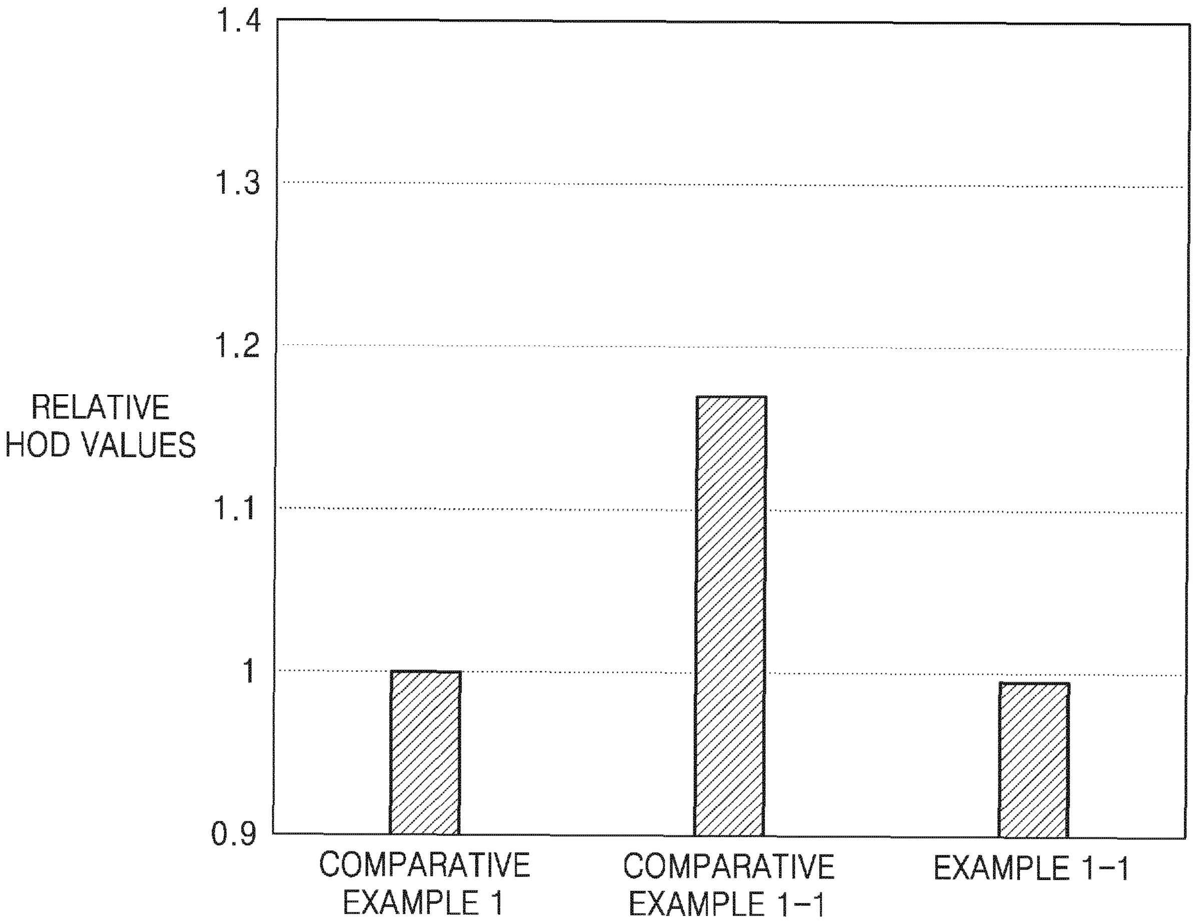
1.4
1.3
1.2
1.1
1
0.9
RELATIVE HOD VALUES
COMPARATIVE EXAMPLE 1
COMPARATIVE EXAMPLE 1-1
EXAMPLE 1-1

FIG. 17

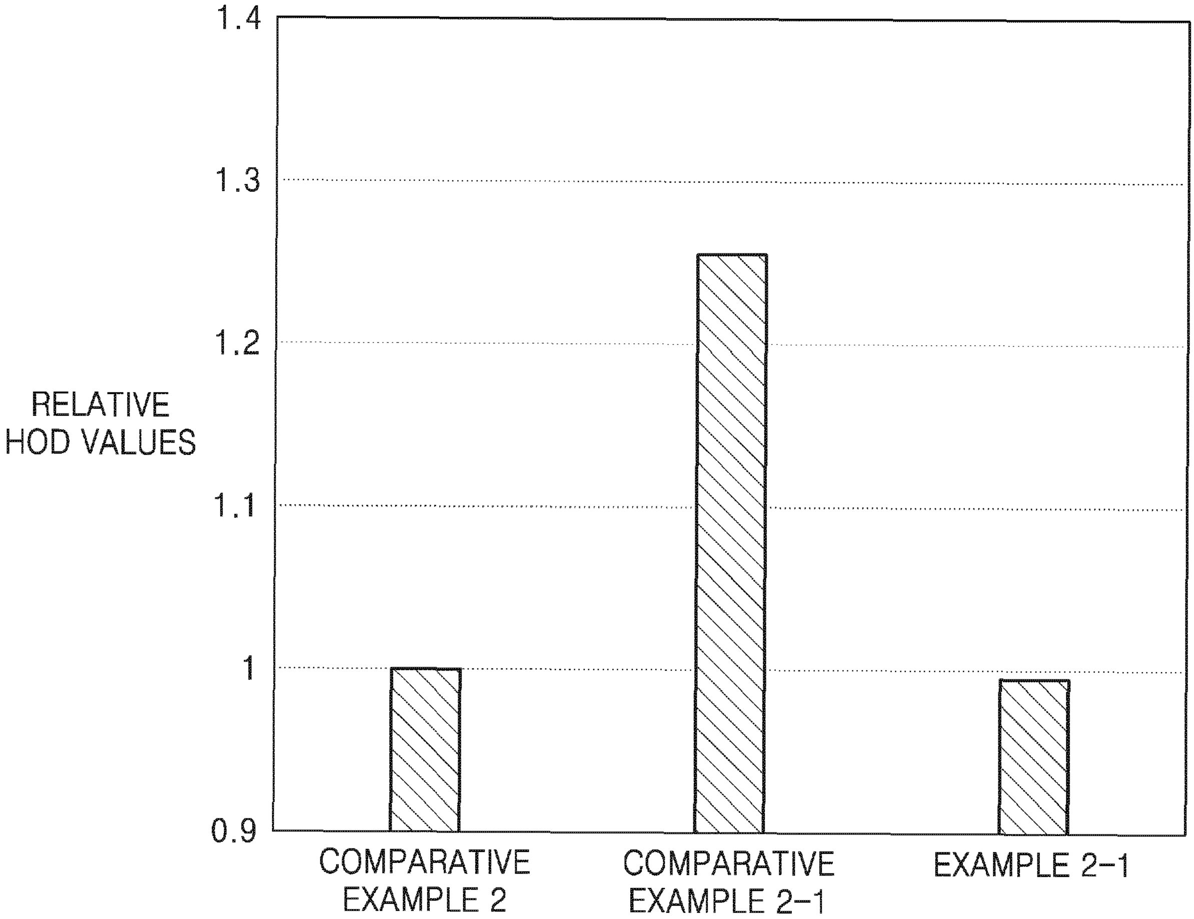
1.4
1.3
1.2
RELATIVE HOD VALUES
1.1
1
0.9
COMPARATIVE EXAMPLE 2
COMPARATIVE EXAMPLE 2-1
EXAMPLE 2-1